# EUROPEAN PATENT SPECIFICATION

(11) **EP 3 064 585 B1**
(45) Date of publication and mention of the grant of the patent: **05.02.2020**
(21) Application number: 16151694.3
(22) Date of filing: 12.12.2013
(51) Int. Cl.: C12N 15/63

(54) **ENGINEERING AND OPTIMIZATION OF IMPROVED SYSTEMS, METHODS AND ENZYME COMPOSITIONS FOR SEQUENCE MANIPULATION**
TECHNISCHE BEARBEITUNG UND OPTIMIERUNG VON VERBESSERTEN SYSTEMEN, VERFAHREN UND ENZYMZUSAMMENSETZUNGEN ZUR SEQUENZENMANIPULATION
FABRICATION ET OPTIMISATION DE SYSTÈMES, PROCÉDÉS ET COMPOSITIONS D'ENZYME AMÉLIORÉS POUR LA MANIPULATION DE SÉQUENCES

(30) Priority: 12.12.2012 US 201261736527 P; 02.01.2013 US 201361748427 P; 30.01.2013 US 201361758468 P; 25.02.2013 US 201361769046 P; 15.03.2013 US 201361802174 P; 15.03.2013 US 201361791409 P; 28.03.2013 US 201361806375 P; 20.04.2013 US 201361814263 P; 06.05.2013 US 201361819803 P; 28.05.2013 US 201361828130 P; 17.06.2013 US 201361835931 P; 17.06.2013 US 201361836101 P
(43) Date of publication of application: 07.09.2016
(62) Divisional of application: 13814364.9
(73) Proprietor: The Broad Institute, Inc., Cambridge, MA 02142 (US); Massachusetts Institute of Technology, Cambridge MA 02142 (US); President and Fellows of Harvard College, Cambridge, MA 02138 (US)
(72) Inventor: ZHANG, Feng, Cambridge, MA 02139 (US); RAN, Fei, Boston, MA 02116 (US); SHALEM, Ophir, Albany, CA 94706 (US)
(74) Representative: Icely, Dominic Michael

(56) References cited:
- M. JINEK ET AL: "A Programmable Dual-RNA-Guided DNA Endonuclease in Adaptive Bacterial Immunity", SCIENCE, vol. 337, no. 6096, 17 August 2012 (2012-08-17), pages 816-821, XP055067740, ISSN: 0036-8075, DOI: 10.1126/science.1225829
- G. GASIUNAS ET AL: "PNAS Plus: Cas9-crRNA ribonucleoprotein complex mediates specific DNA cleavage for adaptive immunity in bacteria", PROCEEDINGS OF THE NATIONAL ACADEMY OF SCIENCES, vol. 109, no. 39, 25 September 2012 (2012-09-25), pages E2579-E2586, XP055068588, ISSN: 0027-8424, DOI: 10.1073/pnas.1208507109
- JIANG WENYAN ET AL: "RNA-guided editing of bacterial genomes using CRISPR-Cas systems", NATURE BIOTECHNOLOGY,, vol. 31, no. 3, 29 January 2013 (2013-01-29), pages 233-239, XP002699849,
- BLAKE WIEDENHEFT ET AL: "RNA-guided genetic silencing systems in bacteria and archaea", NATURE, vol. 482, no. 7385, 15 February 2012 (2012-02-15), pages 331-338, XP055116249, ISSN: 0028-0836, DOI: 10.1038/nature10886
- ELITZA DELTCHEVA ET AL: "CRISPR RNA maturation by trans-encoded small RNA and host factor RNase III", NATURE, vol. 471, no. 7340, 31 March 2011 (2011-03-31), pages 602-607, XP055068535, ISSN: 0028-0836, DOI: 10.1038/nature09886

## Description

This application claims priority to US provisional patent application 61/836,101 entitled ENGINEERING AND OPTIMIZATION OF IMPROVED SYSTEMS, METHODS AND ENZYME COMPOSITIONS FOR SEQUENCE MANIPULATION filed on June 17, 2013. This application also claims priority to US provisional patent applications 61/758,468; 61/769,046; 61/802,174; 61/806,375; 61/814,263; 61/819,803 and 61/828,130 each entitled ENGINEERING AND OPTIMIZATION OF SYSTEMS, METHODS AND COMPOSITIONS FOR SEQUENCE MANIPULATION, filed on January 30, 2013; February 25, 2013; March 15, 2013; March 28, 2013; April 20, 2013; May 6, 2013 and May 28, 2013, respectively. This application also claims priority to US provisional patent applications 61/736,527 and 61/748,427, both entitled SYSTEMS METHODS AND COMPOSITIONS FOR SEQUENCE MANIPULATION filed on December 12, 2012 and January 2, 2013, respectively. Priority is also claimed to US provisional patent applications 61/791,409 and 61/835,931 filed on March 15, 2013 and June 17, 2013 respectively.

Reference is made to US provisional patent applications 61/836,127, 61/835,936, 61/836,080, 61/836,123, and 61/835,973 each filed June 17, 2013.

### FIELD OF THE INVENTION

The present invention generally relates to the engineering and optimization of systems, methods and compositions used for the control of gene expression involving sequence targeting, such as genome perturbation or gene-editing, that relate to Clustered Regularly Interspaced Short Palindromic Repeats (CRISPR) and components thereof.

### STATEMENT AS TO FEDERALLY SPONSORED RESEARCH

This invention was made with government support under the NIH Pioneer Award (1DP1MH100706) awarded by the National Institutes of Health. The government has certain rights in the invention.

### BACKGROUND OF THE INVENTION

Recent advances in genome sequencing techniques and analysis methods have significantly accelerated the ability to catalog and map genetic factors associated with a diverse range of biological functions and diseases. Precise genome targeting technologies are needed to enable systematic reverse engineering of causal genetic variations by allowing selective perturbation of individual genetic elements, as well as to advance synthetic biology, biotechnological, and medical applications. Although genome-editing techniques such as designer zinc fingers, transcription activator-like effectors (TALEs), or homing meganucleases are available for producing targeted genome perturbations, there remains a need for new genome engineering technologies that are affordable, easy to set up, scalable, and amenable to targeting multiple positions within the eukaryotic genome.

### SUMMARY OF THE INVENTION

The CRISPR/Cas or the CRISPR-Cas system (both terms are used interchangeably throughout this application) does not require the generation of customized proteins to target specific sequences but rather a single Cas enzyme can be programmed by a short RNA molecule to recognize a specific DNA target, in other words the Cas enzyme can be recruited to a specific DNA target using said short RNA molecule. Adding the CRISPR-Cas system to the repertoire of genome sequencing techniques and analysis methods may significantly simplify the methodology and accelerate the ability to catalog and map genetic factors associated with a diverse range of biological functions and diseases. To utilize the CRISPR-Cas system effectively for genome editing without deleterious effects, it is critical to understand aspects of engineering and optimization of these genome engineering tools, which are aspects of the claimed invention.

Accordingly, there exists a pressing need for alternative and robust systems and techniques for sequence targeting with a wide array of applications. Aspects of this invention address this need and provide related advantages. An exemplary CRISPR complex comprises a CRISPR enzyme complexed with a guide sequence hybridized to a target sequence within the target polynucleotide, wherein the CRISPR enzyme is a Cas ortholog, e.g. a Cas9 ortholog, of a genus which includes but is not limited to *Corynebacter, Sutterella, Legionella, Treponema, Filifactor, Eubacterium, Streptococcus, Lactobacillus, Mycoplasma, Bacteroides, Flaviivola, Flavobacterium, Sphaerochaeta, Azospirillum, Gluconacetobacter, Neisseria, Roseburia, Parvibaculum, Staphylococcus, Nitratifractor, Mycoplasma* and *Campylobacter.* The guide sequence is linked to a tracr mate sequence, which in turn hybridizes to a tracr sequence.

In one aspect, the invention provides methods for using one or more elements of a CRISPR system. The CRISPR complex of the invention provides an effective means for modifying a target polynucleotide. The CRISPR complex of the invention has a wide variety of utilities including modifying (e.g., deleting, inserting, translocating, inactivating, activating, repressing, altering methylation, transferring specific moieties) a target polynucleotide in a multiplicity of cell types. As such the CRISPR complex of the invention has a broad spectrum of applications in, e.g., gene or genome editing, gene regulation, gene therapy, drug discovery, drug screening, disease diagnosis, and prognosis. In preferred aspects of the invention, the CRISPR complex comprises a Cas enzyme, preferably a Cas9 ortholog, of a genus which includes but is not limited to *Corynebacter, Sutterella, Legionella, Treponema, Filifactor, Eubacterium, Streptococcus, Lactobacillus, Mycoplasma, Bacteroides, Flaviivola, Flavobacterium, Sphaerochaeta, Azospirillum, Gluconacetobacter, Neisseria, Roseburia, Parvibaculum, Staphylococcus, Nitratifractor, Mycoplasma* and *Campylobacter.*

Aspects of the invention relate to CRISPR enzymes having optimized function. With regard to the CRISPR enzyme being a Cas enzyme, preferred embodiments of the invention relate to Cas9 orthologs having improved target specificity in a CRISPR-Cas9 system. This may be accomplished by approaches that include but are not limited to designing and preparing guide RNAs having optimal activity, selecting Cas9 enzymes of a specific length, truncating the Cas9 enzyme making it smaller in length than the corresponding wild-type Cas9 enzyme by truncating the nucleic acid molecules coding therefor and generating chimeric Cas9 enzymes wherein different parts of the enzyme are swapped or exchanged between different orthologs to arrive at chimeric enzymes having tailored specificity. Aspects of the invention also relate to methods of improving the target specificity of a Cas9 ortholog enzyme or of designing a CRISPR-Cas9 system comprising designing or preparing guide RNAs having optimal activity and/or selecting or preparing a Cas9 ortholog enzyme having a smaller size or length than the corresponding wild-type Cas9 whereby packaging a nucleic acid coding therefor into a delivery vector is advanced as there is less coding sequence therefor in the delivery vector than for the corresponding wild-type Cas9 and/or generating chimeric Cas9 enzymes.

Also provided are uses of the present sequences, vectors, enzymes or systems, in medicine. Also provided are the same for use in gene or genome editing. Also provided is use of the same in the manufacture of a medicament for gene or genome editing, for instance treatment by gene or genome editing. Also provided are the present sequences, vectors, enzymes or systems for use in therapy.

In an additional aspect of the invention, a CRISPR enzyme, e.g. a Cas9 enzyme may comprise one or more mutations and may be used as a generic DNA binding protein with or without fusion to or being operably linked to a functional domain. The mutations may be artificially introduced mutations and may include but are not limited to one or more mutations in a catalytic domain. Examples of catalytic domains with reference to a Cas9 enzyme may include but are not limited to RuvC I, RuvC II, RuvC III and HNH domains. Preferred examples of suitable mutations are the catalytic residue(s) in the N-term RuvC I domain of Cas9 or the catalytic residue(s) in the internal HNH domain. In some embodiments, the Cas9 is (or is derived from) the *Streptococcus pyogenes* Cas9 (SpCas9). In such embodiments, preferred mutations are at any or all of positions 10, 762, 840, 854, 863 and/or 986 of SpCas9 or corresponding positions in other Cas9 orthologs with reference to the position numbering of SpCas9 (which may be ascertained for instance by standard sequence comparison tools, e.g. ClustalW or MegAlign by Lasergene 10 suite). In particular, any or all of the following mutations are preferred in SpCas9: D10A, E762A, H840A, N854A, N863A and/or D986A; as well as conservative substitution for any of the replacement amino acids is also envisaged. The same mutations (or conservative substitutions of these mutations) at corresponding positions with reference to the position numbering of SpCas9 in other Cas9 orthologs are also preferred. Particularly preferred are D10 and H840 in SpCas9. However, in other Cas9s, residues corresponding to SpCas9 D10 and H840 are also preferred. These are advantageous as when singly mutated they provide nickase activity and when both mutations are present the Cas9 is converted into a catalytically null mutant which is useful for generic DNA binding. Further mutations have been identified and characterized. Other aspects of the invention relate to the mutated Cas 9 enzyme being fused to or operably linked to domains which include but are not limited to a transcriptional activator, transcriptional repressor, a recombinase, a transposase, a histone remodeler, a DNA methyltransferase, a cryptochrome, a light inducible/controllable domain or a chemically inducible/controllable domain.

A further aspect of the invention provides for chimeric Cas9 proteins and methods of generating chimeric Cas9 proteins. Chimeric Cas9 proteins are proteins that comprise fragments that originate from different Cas9 orthologs. For instance, the N-terminal of a first Cas9 ortholog may be fused with the C-terminal of a second Cas9 ortholog to generate a resultant Cas9 chimeric protein. These chimeric Cas9 proteins may have a higher specificity or a higher efficiency than the original specificity or efficiency of either of the individual Cas9 enzymes from which the chimeric protein was generated. These chimeric proteins may also comprise one or more mutations or may be linked to one or more functional domains. Therefore, aspects of the invention relate to a chimeric Cas enzyme wherein the enzyme comprises one or more fragments from a first Cas ortholog and one or more fragments from a second Cas ortholog. In a embodiment of the invention the one or more fragments of the first or second Cas ortholog are from the C- or N-terminal of the first or second Cas ortholog. In a further embodiment the first or second Cas ortholog is selected from a genus belonging to the group consisting of *Corynebacter, Sutterella, Legionella, Treponema, Filifactor, Eubacterium, Streptococcus, Lactobacillus, Mycoplasma, Bacteroides, Flaviivola, Flavobacterium, Sphaerochaeta, Azospirillum, Gluconacetobacter, Neisseria, Roseburia, Parvibaculum, Staphylococcus, Nitratifractor, Mycoplasma* and *Campylobacter.*

In a further embodiment, the invention provides for methods to generate mutant components of the CRISPR complex comprising a Cas enzyme, e.g Cas9 ortholog. The mutant components may include but are not limited to mutant tracrRNA and tracr mate sequences or mutant chimeric guide sequences that allow for enhancing performance of these RNAs in cells. Use of the present composition or the enzyme in the preparation of a medicament for modification of a target sequence is also provided.

The invention in yet a further aspect provides compositions and methods related to a non-naturally occurring or engineered composition comprising :
A) -
   I. a CRISPR-Cas system chimeric RNA (chiRNA) polynucleotide sequence, wherein the polynucleotide sequence comprises:
      (a) a guide sequence capable of hybridizing to a target sequence in a eukaryotic cell,
      (b) a tracr mate sequence, and
      (c) a tracr sequence, and
   II. a polynucleotide sequence encoding a CRISPR enzyme comprising at least one or more nuclear localization sequences,
   wherein (a), (b) and (c) are arranged in a 5' to 3' orientation,
   wherein when transcribed, the tracr mate sequence hybridizes to the tracr sequence and the guide sequence directs sequence-specific binding of a CRISPR complex to the target sequence, and wherein the CRISPR complex comprises the CRISPR enzyme complexed with (1) the guide sequence that is hybridized to the target sequence, and (2) the tracr mate sequence that is hybridized to the tracr sequence and the polynucleotide sequence encoding a CRISPR enzyme is DNA or RNA,
   or
(B)
   I. polynucleotides comprising:
      (a) a guide sequence capable of hybridizing to a target sequence in a prokaryotic cell, and
      (b) at least one or more tracr mate sequences,
   II. a polynucleotide sequence encoding a CRISPR enzyme, and
   III. a polynucleotide sequence comprising a tracr sequence,
   wherein when transcribed, the tracr mate sequence hybridizes to the tracr sequence and the guide sequence directs sequence-specific binding of a CRISPR complex to the target sequence, and
   wherein the CRISPR complex comprises the CRISPR enzyme complexed with (1) the guide sequence that is hybridized to the target sequence, and (2) the tracr mate sequence that is hybridized to the tracr sequence, and the polynucleotide sequence encoding a CRISPR enzyme is DNA or RNA, and
   wherein the CRISPR enzyme is a Cas9 ortholog of a genus belonging to the group consisting of Corynebacter, Sutterella, Legionella, Treponema, Filifactor, Eubacterium, Streptococcus, Lactobacillus, Mycoplasma, Bacteroides, Flaviivola, Flavobacterium, Sphaerochaeta, Azospirillum, Gluconacetobacter, Neisseria, Roseburia, Parvibaculum, Staphylococcus, Nitratifractor, Mycoplasma and Campylobacter.

The invention in yet a further aspect provides: (A) A non-naturally occurring or engineered composition comprising a vector system comprising one or more vectors comprising: I. a first regulatory element operably linked to a CRISPR-Cas system chimeric RNA (chiRNA) polynucleotide sequence, wherein the polynucleotide sequence comprises (a) a guide sequence capable of hybridizing to a target sequence in a eukaryotic cell, (b) a tracr mate sequence, and (c) a tracr sequence, and II. a second regulatory element operably linked to an enzyme-coding sequence encoding a CRISPR enzyme comprising at least one or more nuclear localization sequences, wherein (a), (b) and (c) are arranged in a 5' to 3' orientation, wherein components I and II are located on the same or different vectors of the system, wherein when transcribed, the tracr mate sequence hybridizes to the tracr sequence and the guide sequence directs sequence-specific binding of a CRISPR complex to the target sequence, and wherein the CRISPR complex comprises the CRISPR enzyme complexed with (1) the guide sequence that is hybridized to the target sequence, and (2) the tracr mate sequence that is hybridized to the tracr sequence, wherein the CRISPR enzyme is a Cas9 ortholog of a genus belonging to the group consisting of *Corynebacter, Sutterella, Legionella, Treponema, Filifactor, Eubacterium, Streptococcus, Lactobacillus, Mycoplasma, Bacteroides, Flaviivola, Flavobacterium, Sphaerochaeta, Azospirillum, Gluconacetobacter, Neisseria, Roseburia, Parvibaculum, Staphylococcus, Nitratifractor, Mycoplasma* and *Campylobacter* or (B) a non-naturally occurring or engineered composition comprising a vector system comprising one or more vectors comprising I. a first regulatory element operably linked to (a) a guide sequence capable of hybridizing to a target sequence in a prokaryotic cell, and (b) at least one or more tracr mate sequences, II. a second regulatory element operably linked to an enzyme-coding sequence encoding a CRISPR enzyme, and III. a third regulatory element operably linked to a tracr sequence, wherein components I, II and III are located on the same or different vectors of the system, wherein when transcribed, the tracr mate sequence hybridizes to the tracr sequence and the guide sequence directs sequence-specific binding of a CRISPR complex to the target sequence, and wherein the CRISPR complex comprises the CRISPR enzyme complexed with (1) the guide sequence that is hybridized to the target sequence, and (2) the tracr mate sequence that is hybridized to the tracr sequence, wherein the CRISPR enzyme is a Cas9 ortholog of a genus belonging to the group consisting of *Corynebacter, Sutterella, Legionella, Treponema, Filifactor, Eubacterium, Streptococcus, Lactobacillus, Mycoplasma, Bacteroides, Flaviivola, Flavobacterium, Sphaerochaeta, Azospirillum, Gluconacetobacter, Neisseria, Roseburia, Parvibaculum, Staphylococcus, Nitratifractor, Mycoplasma* and *Campylobacter,* and wherein: at least one of the following criteria applies.

The criteria are as follows and it will be appreciated that any number of these may apply, preferably 1 or more, preferably 2 or more, and preferably 3 or more, 4 or more, or 5 or more, or all may apply:
- the CRISPR enzyme having a specific size is selected and has a length of at least 500 amino acids, at least 800-899 amino acids, at least 900-999 amino acids, at least 1000-1099 amino acids, at least 1100-1199 amino acids, at least 1200-1299 amino acids, at least 1300-1399 amino acids, at least 1400-1499 amino acids, at least 1500-1599 amino acids, at least 1600-1699 amino acids or at least 2000 amino acids;
- and/or the CRISPR enzyme is truncated in comparison to the corresponding wild type CRISPR enzyme;
- and/or the CRISPR enzyme is a nuclease directing cleavage of both strands at the location of the target sequence, or the CRISPR enzyme is a nickase directing cleavage of one strand at the location of the target sequence;
- and/or the guide sequence comprises at least 10, at least 15 or at least 20 nucleotides;
- and/or the CRISPR enzyme is codon-optimized or codon-optimized for expression in a eukaryotic cell;
- and/or the CRISPR enzyme comprises one or more mutations;
- and/or the CRISPR enzyme comprises a chimeric CRISPR enzyme;
- and/or the CRISPR enzyme has one or more other attributes herein discussed.

In some embodiments, the CRISPR enzyme is truncated in comparison to a wild type CRISPR enzyme or the CRISPR enzyme is comprised of at least 500 amino acids, at least 800-899 amino acids, at least 900-999 amino acids, at least 1000-1099 amino acids, at least 1100-1199 amino acids, at least 1200-1299 amino acids, at least 1300-1399 amino acids, at least 1400-1499 amino acids, at least 1500-1599 amino acids, at least 1600-1699 amino acids or at least 2000 amino acids. In preferred embodiments the CRISPR enzyme is a Cas enzyme, e.g. a Cas9 ortholog.

In some embodiments, the CRISPR enzyme is a nuclease directing cleavage of both strands at the location of the target sequence, or the CRISPR enzyme is a nickase directing cleavage of one strand at the location of the target sequence. In further embodiments, the CRISPR enzyme is a catalytically null mutant that is a generic DNA binding protein. In preferred embodiments the CRISPR enzyme is a Cas enzyme, e.g. a Cas9 ortholog.

In some embodiments, the guide sequence comprises at least fifteen nucleotides. In some embodiments, the CRISPR enzyme is codon-optimized or codon-optimized for expression in a eukaryotic cell. In some embodiments, the CRISPR enzyme comprises one or more mutations. In some embodiments, the CRISPR enzyme comprises a chimeric CRISPR enzyme. In some embodiments, the CRISPR enzyme has one or more other attributes herein discussed. In preferred embodiments the CRISPR enzyme is a Cas enzyme, e.g. a Cas9 ortholog.

In certain embodiments, the CRISPR enzyme comprises one or more mutations. The one or more mutations may be in a particular domain of the enzyme. In a preferred embodiment, the one or more mutations may be in a catalytic domain. In a further preferred embodiment the catalytic domain is a RuvC I, RuvC II, RuvC III or HNH domain. In a more preferred embodiment, the one or more mutations is in a RuvC1 or HNH domain of the CRISPR enzyme. In a further preferred embodiment the CRISPR enzyme is a Cas enzyme, e.g. a Cas9 ortholog and the mutation may be at one or positions that include but are not limited to positions that correspond to D10A, E762A, H840A, N854A, N863A or D986A with reference to the position numbering of SpCas9 and/or is a mutation as otherwise discussed herein. In some embodiments, the CRISPR enzyme has one or more mutations in a particular domain of the enzyme, wherein when transcribed, the tracr mate sequence hybridizes to the tracr sequence and the guide sequence directs sequence-specific binding of a CRISPR complex to the target sequence, and wherein the enzyme further comprises a functional domain. The functional domain may include but is not limited to transcriptional activator, transcriptional repressor, a recombinase, a transposase, a histone remodeler, a DNA methyltransferase, a cryptochrome, a light inducible/controllable domain or a chemically inducible/controllable domain.

In some embodiments, the functional domain is the transcriptional activator domain VP64. In some embodiments, the functional domain is the transcriptional repressor domain KRAB. In some embodiments, the transcriptional repressor domain is SID, or concatemers of SID (i.e. SID4X). In some embodiments, an epigenetic modifying enzyme is provided, e.g a histone modifying protein or an epigenetic chromatin modifying protein. In some embodiments, an activator domain is provided, which may be the P65 activator domain.

A further aspect of the invention comprehends methods of modifying two or more genomic loci of interest. In a preferred embodiment of the invention two or more genomic loci are differentially modulated by utilizing one or more CRISPR enzymes, e.g. two or more Cas9 orthologs, each ortholog being operably linked to one or more functional domain. In one aspect, the invention provides for a method of modifying two or more genomic loci in a eukaryotic cell. Therefore, aspects of the invention provide for a method of modulating the expression of two or more genomic loci of interest in an organism comprising delivering a non-naturally occurring or engineered composition comprising a vector system comprising one or more vectors comprising
I. a first regulatory element operably linked to a first CRISPR-Cas system chimeric RNA (chiRNA) polynucleotide sequence, wherein the first polynucleotide sequence comprises
   (i) a first guide sequence capable of hybridizing to a first target sequence at a first genomic locus in a cell of the organism,
   (ii) a first tracr mate sequence, and
   (iii) a first tracr sequence, and
II. a second regulatory element operably linked to a second CRISPR-Cas system chimeric RNA (chiRNA) polynucleotide sequence, wherein the second polynucleotide sequence comprises
   (i) a second guide sequence capable of hybridizing to a second target sequence at a second genomic locus in the cell of the organism,
   (ii) a second tracr mate sequence, and
   (iii) a second tracr sequence, and
III. a third regulatory element operably linked to an enzyme-coding sequence encoding a first CRISPR enzyme comprising at least one or more nuclear localization sequences and operably linked to a first functional domain,
IV. a fourth regulatory element operably linked to an enzyme-coding sequence encoding a second CRISPR enzyme comprising at least one or more nuclear localization sequences and operably linked to a second functional domain, wherein (i), (ii) and (iii) in I and II are arranged in a 5' to 3' orientation, wherein components I, II, III and IV are located on the same or different vectors of the system, wherein when transcribed, each tracr mate sequences hybridizes to its corresponding tracr sequence and the first and second guide sequences direct sequence-specific binding of the first and second CRISPR complex to the first and second target sequence, wherein the CRISPR complex comprises the CRISPR enzyme complexed with (1) the guide sequence that is hybridized to the target sequence, and (2) the tracr mate sequence that is hybridized to the tracr sequence and wherein expression of the CRISPR enzyme provides manipulation of the target sequence, wherein the first and second CRISPR enzyme each comprise two or more mutations, wherein the first and second CRISPR enzyme is a Cas9 ortholog of a genus belonging to the group consisting of *Corynebacter, Sutterella, Legionella, Treponema, Filifactor, Eubacterium, Streptococcus, Lactobacillus, Mycoplasma, Bacteroides, Flaviivola, Flavobacterium, Sphaerochaeta, Azospirillum, Gluconacetobacter, Neisseria, Roseburia, Parvibaculum, Staphylococcus, Nitratifractor, Mycoplasma* and *Campylobacter,* and wherein the first genomic locus is modulated by the activity of the first functional domain and the second genomic locus is modulated by the activity of the second functional domain. In a further embodiment the first functional domain is selected from the group consisting of a transcriptional activator, transcriptional repressor, a recombinase, a transposase, a histone remodeler, a DNA methyltransferase, a cryptochrome and a light inducible/controllable domain or a chemically inducible/controllable domain. In a further embodiment the second functional domain is selected from the group consisting of a transcriptional activator, transcriptional repressor, a recombinase, a transposase, a histone remodeler, a DNA methyltransferase, a cryptochrome and a light inducible/controllable domain or a chemically inducible/controllable domain. In preferred embodiments the first or second CRISPR enzyme is a Sutterella wadsworthensis Cas9, a Filifactor alocis Cas9, a Lactobacillus johnsonii Cas9, a Campylobacter lari Cas9, a Corynebacter diptheriae Cas9, a Parvibaculum lavamentivorans Cas9, a Mycoplasma gallisepticum Cas9, a Staphylococcus aureus subsubspecies Aureus Cas9, a Legionella pneumophila Paris Cas9, a Treponema denticola Cas9, a Staphylococcus pseudintermedius Cas9, a Neisseria cinerea Cas9.

In some embodiments, the CRISPR enzyme is a type I, II or III CRISPR enzyme, preferably a type II CRISPR enzyme. This type II CRISPR enzyme may be any Cas enzyme. A Cas enzyme may be identified as Cas9 as this can refer to the general class of enzymes that share homology to the biggest nuclease with multiple nuclease domains from the type II CRISPR system. Most preferably, the Cas9 enzyme is from, or is derived from, SpCas9 or *Staphylococcus aureus* subsubspecies Aureus SaCas9. By derived, it is meant that the derived enzyme is largely based, in the sense of having a high degree of sequence homology with, a wildtype enzyme, but that it has been mutated (modified) in some way as described herein.

It will be appreciated that the terms Cas and CRISPR enzyme are generally used herein interchangeably, unless otherwise apparent. As mentioned above, many of the residue numberings used herein refer to the Cas9 enzyme from the type II CRISPR locus in *Streptotoccus pyogenes.* However, it will be appreciated that this invention includes many more Cas9s from other species of microbes such as those belonging to the genus *Corynebacter, Sutterella, Legionella, Treponema, Filifactor, Eubacterium, Streptococcus, Lactobacillus, Mycoplasma, Bacteroides, Flaviivola, Flavobacterium, Sphaerochaeta, Azospirillum, Gluconacetobacter, Neisseria, Roseburia, Parvibaculum, Staphylococcus, Nitratifractor, Mycoplasma* or *Campylobacter,* such as SpCas9, SaCas9, St1Cas9, St3Cas9 and so forth, wherein St is *Streptococcus thermophilus.*

An example of a codon optimized sequence, in this instance optimized for humans (i.e. being optimized for expression in humans) is provided herein, see the SaCas9 human codon optimized sequence. Whilst this is preferred, it will be appreciated that other examples are possible and codon optimization for a host species is known.

Further aspects of the invention relate to improved cleavage specificity, optimized tracr sequence, optimized chimeric guide RNA, co-fold structure of tracrRNA and tracr mate sequence, stabilizing secondary structures of tracr RNA, tracrRNA with shortened region of base pairing, tracrRNA with fused RNA elements, simplified cloning and delivery, reduced toxicity and/or inducible systems. Another aspect of the invention relates to the stabilization of chimeric RNA, and/or guide sequence and or a portion thereof of CRISPR complexes wherein the CRISPR enzyme is a CRISPR ortholog, wherein the chimeric RNA, and/or guide sequence and or a portion thereof is stabilized by synthetic or chemically modified nucleotides (e.g. LNA/BNA: thiol-modification, 2'/3'-OH crosslink modification), is modified to be degradation/hydrolysis resistant and to which elements of structural stability have been added.

The invention further comprehends in certain embodiments a method of modifying an organism or a non-human organism by manipulation of a target sequence in a genomic locus of interest comprising delivering a non-naturally occurring or engineered composition comprising a vector system comprising one or more vectors operably encoding a composition herein discussed for expression thereof. Preferably, the vector is a viral vector, such as a lenti- or baculo- or preferably adeno-viral/adeno-associated viral vectors, but other means of delivery are known (such as yeast systems, microvesicles, gene guns/means of attaching vectors to gold nanoparticles) and are provided.

Various means of delivery are described herein, and further discussed in this section.

Viral delivery: The CRISPR enzyme, for instance a Cas9, and/or any of the present RNAs, for instance a guide RNA, can be delivered using adeno associated virus (AAV), lentivirus, adenovirus or other viral vector types, or combinations thereof. Cas9 and one or more guide RNAs can be packaged into one or more viral vectors. In some embodiments, the viral vector is delivered to the tissue of interest by, for example, an intramuscular injection, while othertimes the viral delivery is via intravenous, transdermal, intranasal, oral, mucosal, or other delivery methods. Such delivery may be either via a single dose, or multiple doses. One skilled in the art understands that the actual dosage to be delivered herein may vary greatly depending upon a variety of factors, such as the vector chosen, the target cell, organism, or tissue, the general condition of the subject to be treated, the degree of transformation/modification sought, the administration route, the administration mode, the type of transformation/modification sought, etc.

Such a dosage may further contain, for example, a carrier (water, saline, ethanol, glycerol, lactose, sucrose, calcium phosphate, gelatin, dextran, agar, pectin, peanut oil, sesame oil, etc.), a diluent, a pharmaceutically-acceptable carrier (e.g., phosphate-buffered saline), a pharmaceutically-acceptable excipient, an adjuvant to enhance antigenicity, an immunostimulatory compound or molecule, and/or other compounds known in the art. The adjuvant herein may contain a suspension of minerals (alum, aluminum hydroxide, aluminum phosphate) on which antigen is adsorbed; or water-in-oil emulsion in which antigen solution is emulsified in oil (MF-59, Freund's incomplete adjuvant), sometimes with the inclusion of killed mycobacteria (Freund's complete adjuvant) to further enhance antigenicity (inhibits degradation of antigen and/or causes influx of macrophages). Adjuvants also include immunostimulatory molecules, such as cytokines, costimulatory molecules, and for example, immunostimulatory DNA or RNA molecules, such as CpG oligonucleotides. Such a dosage formulation is readily ascertainable by one skilled in the art. The dosage may further contain one or more pharmaceutically acceptable salts such as, for example, a mineral acid salt such as a hydrochloride, a hydrobromide, a phosphate, a sulfate, etc.; and the salts of organic acids such as acetates, propionates, malonates, benzoates, etc. Additionally, auxiliary substances, such as wetting or emulsifying agents, pH buffering substances, gels or gelling materials, flavorings, colorants, microspheres, polymers, suspension agents, etc. may also be present. In addition, one or more other conventional pharmaceutical ingredients, such as preservatives, humectants, suspending agents, surfactants, antioxidants, anticaking agents, fillers, chelating agents, coating agents, chemical stabilizers, etc. may also be present, especially if the dosage form is a reconstitutable form. Suitable exemplary ingredients include microcrystalline cellulose, carboxymethylcellulose sodium, polysorbate 80, phenylethyl alcohol, chlorobutanol, potassium sorbate, sorbic acid, sulfur dioxide, propyl gallate, the parabens, ethyl vanillin, glycerin, phenol, parachlorophenol, gelatin, albumin and a combination thereof. A thorough discussion of pharmaceutically acceptable excipients is available in REMINGTON'S PHARMACEUTICAL SCIENCES (Mack Pub. Co., N.J. 1991).

In an embodiment herein the delivery is via an adenovirus, which may be at a single booster dose containing at least 1 x 10⁵ particles (also referred to as particle units, pu) of adenoviral vector. In an embodiment herein, the dose preferably is at least about 1 x 10⁶ particles (for example, about 1 x 10⁶-1 x 10¹² particles), more preferably at least about 1 x 10⁷ particles, more preferably at least about 1 x 10⁸ particles (e.g., about 1 x 10⁸-1 x 10¹¹ particles or about 1 x 10⁸-1 x 10¹² particles), and most preferably at least about 1 x 10⁰ particles (e.g., about 1* x 10⁹-1 x 10¹⁰ particles or about 1 x 10⁹-1 x 10¹² particles), or even at least about 1 x 10¹⁰ particles (e.g., about 1 x 10¹⁰-1 x 10¹² particles) of the adenoviral vector. Alternatively, the dose comprises no more than about 1 x 10¹⁴ particles, preferably no more than about 1 x 10¹³ particles, even more preferably no more than about 1 x 10¹² particles, even more preferably no more than about 1 x 10¹¹ particles, and most preferably no more than about 1 x 10¹⁰ particles (e.g., no more than about 1 x 10⁹ articles). Thus, the dose may contain a single dose of adenoviral vector with, for example, about 1 x 10⁶ particle units (pu), about 2 x 10⁶ pu, about 4 x 10⁶ pu, about 1 x 10⁷ pu, about 2 x 10⁷ pu, about 4 x 10⁷ pu, about 1 x 10⁸ pu, about 2 x 10⁸ pu, about 4 x 10⁸ pu, about 1 x 10⁹ pu, about 2 x 10⁹ pu, about 4 x 10⁹ pu, about 1 x 10¹⁰ pu, about 2 x 10¹⁰ pu, about 4 x 10¹⁰ pu, about 1 x 10¹¹ pu, about 2 x 10¹¹ pu, about 4 x 10¹¹ pu, about 1 x 10¹² pu, about 2 x 10¹² pu, or about 4 x 10¹² pu of adenoviral vector. See, for example, the adenoviral vectors in U.S. Patent No. 8,454,972 B2 to Nabel, et. al., granted on June 4, 2013; and the dosages at col 29, lines 36-58 thereof. In an embodiment herein, the adenovirus is delivered via multiple doses.

In an embodiment herein, the delivery is via an AAV. A therapeutically effective dosage for in vivo delivery of the AAV to a human is believed to be in the range of from about 20 to about 50 ml of saline solution containing from about 1 x 10¹⁰ to about 1 x 10¹⁰ functional AAV/ml solution. The dosage may be adjusted to balance the therapeutic benefit against any side effects. In an embodiment herein, the AAV dose is generally in the range of concentrations of from about 1 x 10⁵ to 1 x 10⁵⁰ genomes AAV, from about 1 x 10⁸ to 1 x 10²⁰ genomes AAV, from about 1 x 10¹⁰ to about 1 x 10¹⁶ genomes, or about 1 x 10¹¹ to about 1 x 10¹⁶ genomes AAV. A human dosage may be about 1 x 10¹³ genomes AAV. Such concentrations may be delivered in from about 0.001 ml to about 100 ml, about 0.05 to about 50 ml, or about 10 to about 25 ml of a carrier solution. Other effective dosages can be readily established by one of ordinary skill in the art through routine trials establishing dose response curves. See, for example, U.S. Patent No. 8,404,658 B2 to Hajjar, et al., granted on March 26, 2013, at col. 27, lines 45-60.

In an embodiment herein the delivery is via a plasmid. In such plasmid compositions, the dosage should be a sufficient amount of plasmid to elicit a response. For instance, suitable quantities of plasmid DNA in plasmid compositions can be from about 0.1 to about 2 mg, or from about 1 µg to about 10 µg.

The doses herein are based on an average 70 kg individual. The frequency of administration is within the ambit of the medical or veterinary practitioner (e.g., physician, veterinarian), or scientist skilled in the art.

The viral vectors can be injected into the tissue of interest. For cell-type specific genome modification, the expression of Cas9 can be driven by a cell-type specific promoter. For example, liver-specific expression might use the Albumin promoter and neuron-specific expression might use the Synapsin I promoter.

RNA delivery: The CRISPR enzyme, for instance a Cas9, and/or any of the present RNAs, for instance a guide RNA, can also be delivered in the form of RNA. Cas9 mRNA can be generated using in vitro transcription. For example, Cas9 mRNA can be synthesized using a PCR cassette containing the following elements: T7_promoter-kozak sequence (GCCACC)-Cas9-3' UTR from beta globin-polyA tail (a string of 120 or more adenines). The cassette can be used for transcription by T7 polymerase. Guide RNAs can also be transcribed using in vitro transcription from a cassette containing T7_promoter-GG-guide RNA sequence.

To enhance expression and reduce toxicity, the CRISPR enzyme and/or guide RNA can be modified using pseudo-U or 5-Methyl-C.

CRISPR enzyme mRNA and guide RNA may be delivered simultaneously using nanoparticles or lipid envelopes.

For example, Su X, Fricke J, Kavanagh DG, Irvine DJ ("In vitro and in vivo mRNA delivery using lipid-enveloped pH-responsive polymer nanoparticles" Mol Pharm. 2011 Jun 6;8(3):774-87. doi: 10.1021/mp100390w. Epub 2011 Apr 1) describes biodegradable core-shell structured nanoparticles with a poly(β-amino ester) (PBAE) core enveloped by a phospholipid bilayer shell. These were developed for in vivo mRNA delivery. The pH-responsive PBAE component was chosen to promote endosome disruption, while the lipid surface layer was selected to minimize toxicity of the polycation core. Such are, therefore, preferred for delivering RNA of the present invention.

Furthermore, Michael S D Kormann et al. ("Expression of therapeutic proteins after delivery of chemically modified mRNA in mice: Nature Biotechnology, Volume:29,Pages: 154-157 (2011) Published online 09 January 2011) describes the use of lipid envelopes to deliver RNA. Use of lipid envelopes is also preferred in the present invention.

mRNA delivery methods are especially promising for liver delivery currently.

CRISPR enzyme mRNA and guideRNA might also be delivered separately. CRISPR enzyme mRNA can be delivered prior to the guide RNA to give time for CRISPR enzyme to be expressed. CRISPR enzyme mRNA might be administered 1-12 hours (preferably around 2-6 hours) prior to the administration of guideRNA.

Alternatively, CRISPR enzyme mRNA and guide RNA can be administered together. Advantageously, a second booster dose of guide RNA can be administered 1-12 hours (preferably around 2-6 hours) after the initial administration of CRISPR enzyme mRNA + guideRNA.

Additional administrations of CRISPR enzyme mRNA and/or guide RNA might be useful to achieve the most efficient levels of genome modification.

For minimization of toxicity and off-target effects, it will be important to control the concentration of CRISPR enzyme mRNA and guide RNA delivered. Optimal concentrations of CRISPR enzyme mRNA and guide RNA can be determined by testing different concentrations in a cellular or animal model and using deep sequencing to analyze the extent of modification at potential off-target genomic loci. For example, for the guide sequence targeting 5'-GAGTCCGAGCAGAAGAAGAA-3' in the EMX1 gene of the human genome, deep sequencing can be used to assess the level of modification at the following two off-target loci, 1: 5'-GAGTCCTAGCAGGAGAAGAA-3' and 2: 5'-GAGTCTAAGCAGAAGAAGAA-3'. The concentration that gives the highest level of on-target modification while minimizing the level of off-target modification should be chosen for in vivo delivery.

Alternatively, to minimize the level of toxicity and off-target effects, CRISPR enzyme nickase mRNA (for example S. pyogenes Cas9 with the D10A mutation) can be delivered with a pair of guide RNAs targeting a site of interest. The two guide RNAs need to be spaced as follows. Guide sequences in red (single underline) and blue (double underline) respectively (these examples are based on the PAM requirement for Streptococcus pyogenes Cas9).

| Overhang length (bp) | Guide RNA design (guide sequence and PAM color coded) |
|---|---|
| 14 | |
| | |
| 13 | |
| | |
| 12 | |
| | |
| 11 | |
| | |
| 10 | |
| | |
| 9 | |
| 8 | |
| | |
| 7 | |
| | |
| 6 | |
| | |
| 5 | |
| | |
| 4 | |
| | |
| 3 | |
| | |
| 2 | |
| | |
| 1 | |
| | |
| blunt | |
| | |
| 1 | |
| | |
| 2 | |
| | |
| 3 | |
| | |
| 4 | |
| | |
| 5 | |
| | |
| 6 | |
| | |
| 7 | |
| | |
| 8 | |
| | |
| 12 | |
| | |
| 13 | |
| | |
| 14 | |
| | |
| 15 | |
| | |
| 16 | |
| | |
| 17 | |

Further interrogation of the system has given Applicants evidence of the 5' overhang (see, e.g., Ran et al., Cell. 2013 Sep 12;154(6):1380-9 and US Provisional Patent Application Serial No. 61/871,301 filed August 28, 2013). Applicants have further identified parameters that relate to efficient cleavage by the Cas9 nickase mutant when combined with two guide RNAs and these parameters include but are not limited to the length of the 5' overhang. In embodiments of the invention the 5' overhang is at most 200 base pairs, preferably at most 100 base pairs, or more preferably at most 50 base pairs. In embodiments of the invention the 5' overhang is at least 26 base pairs, preferably at least 30 base pairs or more preferably 34-50 base pairs or 1-34 base pairs. In other preferred methods of the invention the first guide sequence directing cleavage of one strand of the DNA duplex near the first target sequence and the second guide sequence directing cleavage of other strand near the second target sequence results in a blunt cut or a 3' overhang. In embodiments of the invention the 3' overhang is at most 150, 100 or 25 base pairs or at least 15, 10 or 1 base pairs. In preferred embodiments the 3' overhang is 1-100 base pairs.

Aspects of the invention relate to the expression of the gene product being decreased or a template polynucleotide being further introduced into the DNA molecule encoding the gene product or an intervening sequence being excised precisely by allowing the two 5' overhangs to reanneal and ligate or the activity or function of the gene product being altered or the expression of the gene product being increased. In an embodiment of the invention, the gene product is a protein.

Only sgRNA pairs creating 5' overhangs with less than 8bp overlap between the guide sequences (offset greater than -8 bp) were able to mediate detectable indel formation. Importantly, each guide used in these assays is able to efficiently induce indels when paired with wildtype Cas9, indicating that the relative positions of the guide pairs are the most important parameters in predicting double nicking activity.

Since Cas9n and Cas9H840A nick opposite strands of DNA, substitution of Cas9n with Cas9H840A with a given sgRNA pair should result in the inversion of the overhang type. For example, a pair of sgRNAs that will generate a 5' overhang with Cas9n should in principle generate the corresponding 3' overhang instead. Therefore, sgRNA pairs that lead to the generation of a 3' overhang with Cas9n might be used with Cas9H840A to generate a 5' overhang. Unexpectedly, Applicants tested Cas9H840A with a set of sgRNA pairs designed to generate both 5' and 3' overhangs (offset range from -278 to +58 bp), but were unable to observe indel formation. Further work may be needed to identify the necessary design rules for sgRNA pairing to allow double nicking by Cas9H840A.

Additional delivery options for the brain include encapsulation of CRISPR enzyme and guide RNA in the form of either DNA or RNA into liposomes and conjugating to molecular Trojan horses for trans-blood brain barrier (BBB) delivery. Molecular Trojan horses have been shown to be effective for delivery of B-gal expression vectors into the brain of non-human primates. The same approach can be used to delivery vectors containing CRISPR enzyme and guide RNA. For instance, Xia CF and Boado RJ, Pardridge WM ("Antibody-mediated targeting of siRNA via the human insulin receptor using avidin-biotin technology." Mol Pharm. 2009 May-Jun;6(3):747-51. doi: 10.1021/mp800194) describes how delivery of short interfering RNA (siRNA) to cells in culture, and in vivo, is possible with combined use of a receptor-specific monoclonal antibody (mAb) and avidin-biotin technology. The authors also report that because the bond between the targeting mAb and the siRNA is stable with avidin-biotin technology, and RNAi effects at distant sites such as brain are observed in vivo following an intravenous administration of the targeted siRNA.

Zhang Y, Schlachetzki F, Pardridge WM. ("Global non-viral gene transfer to the primate brain following intravenous administration." Mol Ther. 2003 Jan;7(1):11-8.) describe how expression plasmids encoding reporters such as luciferase were encapsulated in the interior of an "artificial virus" comprised of an 85 nm pegylated immunoliposome, which was targeted to the rhesus monkey brain in vivo with a monoclonal antibody (MAb) to the human insulin receptor (HIR). The HIRMAb enables the liposome carrying the exogenous gene to undergo transcytosis across the blood-brain barrier and endocytosis across the neuronal plasma membrane following intravenous injection. The level of luciferase gene expression in the brain was 50-fold higher in the rhesus monkey as compared to the rat. Widespread neuronal expression of the beta-galactosidase gene in primate brain was demonstrated by both histochemistry and confocal microscopy. The authors indicate that this approach makes feasible reversible adult transgenics in 24 hours. Accordingly, the use of immunoliposome is preferred. These may be used in conjuction with antibodies to target specific tissues or cell surface proteins.

Other means of delivery or RNA are also preferred, such as via nanoparticles >Cho, S., Goldberg, M., Son, S., Xu, Q., Yang, F., Mei, Y., Bogatyrev, S., Langer, R. and Anderson, D., Lipid-like nanoparticles for small interfering RNA delivery to endothelial cells, Advanced Functional Materials, 19: 3112-3118, 2010) or exosomes (Schroeder, A., Levins, C., Cortez, C., Langer, R., and Anderson, D., Lipid-based nanotherapeutics for siRNA delivery, Journal of Internal Medicine, 267: 9-21, 2010, PMID: 20059641). Indeed, exozomes have been shown to be particularly useful in delivery siRNA, a system with some parallels to the CRISPR system. For instance, El-Andaloussi S, et al. ("Exosome-mediated delivery of siRNA in vitro and in vivo." Nat Protoc. 2012 Dec;7(12):2112-26. doi: 10.1038/nprot.2012.131. Epub 2012 Nov 15.) describe how exosomes are promising tools for drug delivery across different biological barriers and can be harnessed for delivery of siRNA in vitro and in vivo. Their approach is to generate targeted exosomes through transfection of an expression vector, comprising an exosomal protein fused with a peptide ligand. The exosomes are then purified and characterized from transfected cell supernatant, then siRNA is loaded into the exosomes.

One aspect of manipulation of a target sequence also refers to the epigenetic manipulation of a target sequence. This may be of the chromatin state of a target sequence, such as by modification of the methylation state of the target sequence (i.e. addition or removal of methylation or methylation patterns or CpG islands), histone modification, increasing or reducing accessibility to the target sequence, or by promoting or reducing 3D folding.

A vector may mean not only a viral or yeast system (for instance, where the nucleic acids of interest may be operably linked to and under the control of (in terms of expression, such as to ultimately provide a processed RNA) a promoter, but also direct delivery of nucleic acids into a host cell.

The invention also comprehends, in certain embodiments, a method of treating or inhibiting a condition caused by a defect in a target sequence in a genomic locus of interest in a subject or a non-human subject in need thereof comprising modifying the subject or a non-human subject by manipulation of the target sequence and wherein the condition is susceptible to treatment or inhibition by manipulation of the target sequence comprising providing treatment comprising: delivering a non-naturally occurring or engineered composition comprising a vector system comprising one or more vectors comprising operably encoding a composition herein discussed for expression thereof, wherein the target sequence is manipulated by the composition when expressed.

In certain embodiments of the herein methods, the methods can include inducing expression, which can be inducing expression of the CRISPR enzyme and/or inducing expression of the guide, tracr or tracr mate sequences. In certain embodiments of the herein methods, the organism or subject is a eukaryote or a non-human eukaryote. In certain embodiments of the herein methods, the organism or subject is a plant. In certain embodiments of the herein methods, the organism or subject is a mammal or a non-human mammal. In certain embodiments of the herein methods, the organism or subject is algae.

While in herein methods the vector may be a viral vector and this is advantageously an AAV, other viral vectors as herein discussed can be employed. For example, baculoviruses may be used for expression in insect cells. These insect cells may, in turn be useful for producing large quantities of further vectors, such as AAV vectors adapted for delivery of the present invention.

Also envisaged is a method of delivering the present CRISPR enzyme comprising delivering to a cell mRNA encoding the CRISPR enzyme. It will be appreciated that the CRISPR enzyme is truncated, is of a specific size as decribed herein, is a nuclease or nickase or generic DNA binding protein, is codon-optimized, comprises one or more mutations, and/or comprises a chimeric CRISPR enzyme, or the other options as herein discussed.

Also envisaged is a method of preparing an vector for delivery of the compositions or the present CRISPR enzymes of the invention and for use in the present methods.

AAV viral vectors are preferred. Thus, in a further aspect, there is provided a method of preparing an AAV viral vector, comprising transfecting plasmid(s) containing or consisting essentially of nucleic acid molecule(s) coding for the AAV into AAV-infected cells, and supplying AAV rep and/or cap obligatory for replication and packaging of the AAV. In this regard, it will be appreciated that the CRISPR enzyme is truncated, comprised of less than one thousand amino acids or less than four thousand amino acids, is a nuclease or nickase, is codon-optimized comprises one or more mutations, and/or comprises a chimeric CRISPR enzyme, as herein discussed. In some embodiments the AAV rep and/or cap obligatory for replication and packaging of the AAV are supplied by transfecting the cells with helper plasmid(s) or helper virus(es). In some embodiments the helper virus is a poxvirus, adenovirus, herpesvirus or baculovirus. In some embodiments the poxvirus is a vaccinia virus. In some embodiments the cells are mammalian cells. And in some embodiments the cells are insect cells and the helper virus is baculovirus.

The invention further comprehends in certain embodiments a modified CRISPR enzyme. Differences from the wild type CRISPR enzyme can comprise: the modified CRISPR enzyme is truncated in comparison to a wild type CRISPR enzyme, or the CRISPR enzyme is of a specific size, e.g. at least 500 amino acids, at least 800-899 amino acids, at least 900-999 amino acids, at least 1000-1099 amino acids, at least 1100-1199 amino acids, at least 1200-1299 amino acids, at least 1300-1399 amino acids, at least 1400-1499 amino acids, at least 1500-1599 amino acids, at least 1600-1699 amino acids or at least 2000 amino acids; and/or the CRISPR enzyme is a nuclease directing cleavage of both strands at the location of the target sequence, or the CRISPR enzyme is a nickase directing cleavage of one strand at the location of the target sequence, or the CRISPR enzyme is a catalytic null mutant that functions as a DNA binding protein; and/or the CRISPR enzyme is codon-optimized or codon-optimized for expression in a eukaryotic cell, and/or the CRISPR enzyme comprises one or more mutations, and/or the CRISPR enzyme comprises a chimeric CRISPR enzyme and/or the CRISPR enzyme has one or more other attributes herein discussed. Accordingly, in certain embodiments, the CRISPR enzyme comprises one or more mutations in catalytic residues such as spCa9 D10A, E762A, H840A, N854A, N863A or D986A or those corresponding to them in other Cas enzymes (as described herein) with reference to the position numbering of SpCas9, and/or has one or more mutations is in a RuvC I, RuvC II, RuvC III, HNH or other domain described herein of the CRISPR enzyme and/or the CRISPR enzyme has one or more mutations in a catalytic domain, wherein when transcribed, a tracr mate sequence hybridizes to a tracr sequence and a guide sequence directs sequence-specific binding of a CRISPR complex to a target sequence, and wherein the enzyme further comprises a functional domain. The functional domain may include but is not limited to transcriptional activator, transcriptional repressor, a recombinase, a transposase, a histone remodeler, a DNA methyltransferase, a cryptochrome, a light inducible/controllable domain or a chemically inducible/controllable domain. The CRISPR enzyme in certain embodiments can have the functional domain be a transcriptional activator domain, e.g., VP64. In some embodiments, a transcription repressor domains is KRAB. In some embodiments, a transcription repressor domain is SID, or concatemers of SID (i.e. SID4X). In some embodiments, an epigenetic modifying enzyme is provided.

In preferred embodiments the CRSIPR enzyme is a Cas enzyme, e.g. a Cas9 ortholog.

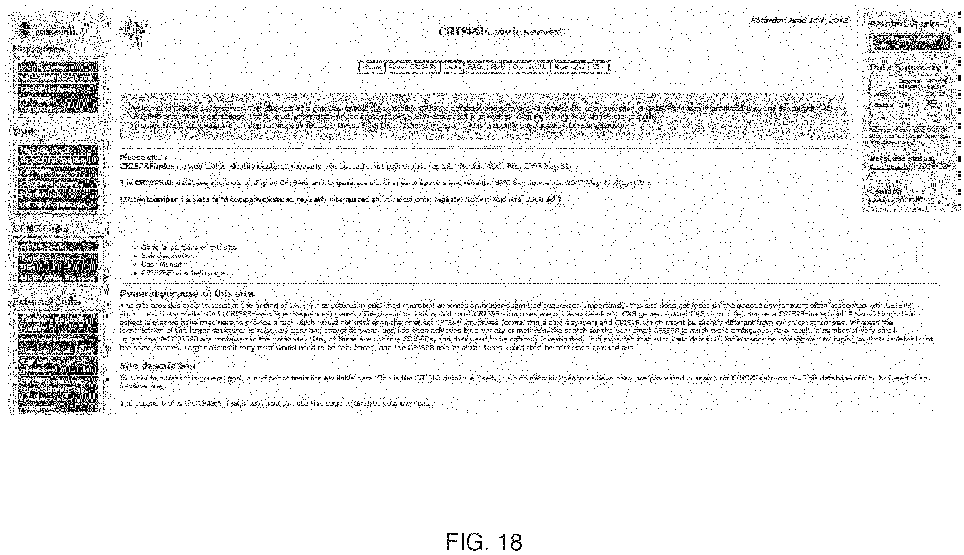

Aspects of the invention also comprehend identifying novel orthologs of CRISPR enzymes. Methods of identifying novel orthologs of CRISPR enzymes may involve identifying tracr sequences in genomes of interest. Identification of tracr sequences may relate to the following steps: Search for the direct repeats or tracr mate sequences in a database to identify a CRISPR region comprising a CRISPR enzyme, e.g. Figure 18. Search for homologous sequences in the CRISPR region flanking the CRISPR enzyme in both the sense and antisense directions. Look for transcriptional terminators and secondary structures. Identify any sequence that is not a direct repeat or a tracr mate sequence but has more than 50% identity to the direct repeat ot tracr mate sequence as a potential tracr sequence. Take the potential tracr sequence and analyze for transcriptional terminator sequences associated therewith.

The invention comprehends in certain embodiments use of a composition as described herein or a CRISPR enzyme in medicine. The invention further comprehends in certain embodiments a composition or CRISPR enzyme of the invention used in a method of the invention. The invention also comprehends, in certain embodiments, use of a composition or a CRISPR enzyme of the invention in, preferably *ex vivo,* gene or genome editing or, preferably an *ex vivo,* gene or genome editing method. The invention accordingly also comprehends in certain embodiments use of a composition according or a CRISPR enzyme of the invention in the manufacture of a medicament for *ex vivo* gene or genome editing or for use in a method as herein discussed.

In addition, the invention in certain embodiments comprehends a composition or a CRISPR enzyme of the invention, wherein the target sequence is flanked or followed, at its 3' end, by a PAM suitable for the CRISPR enzyme, typically a Cas and in particular a Cas9. This PAM sequence is specific for each Cas9 but may be readily determined by methods described herein.

For example, a suitable PAM is 5'-NRG or 5'-NNGRR for SpCas9 or SaCas9 enzymes (or derived enzymes), respectively. It will be appreciated that reference made herein to *Staphylococcus aureus* preferably includes *Staphylococcus aureus* subspecies *Aureus.*

Accordingly, it is an object of the invention to not encompass within the invention any previously known product, process of making the product, or method of using the product such that Applicants reserve the right and hereby disclose a disclaimer of any previously known product, process, or method. It is further noted that the invention does not intend to encompass within the scope of the invention any product, process, or making of the product or method of using the product, which does not meet the written description and enablement requirements of the USPTO (35 U.S.C. §112, first paragraph) or the EPO (Article 83 of the EPC), such that Applicants reserve the right and hereby disclose a disclaimer of any previously described product, process of making the product, or method of using the product.

It is noted that in this disclosure and particularly in the claims and/or paragraphs, terms such as "comprises", "comprised", "comprising" and the like can have the meaning attributed to it in U.S. Patent law; e.g., they can mean "includes", "included", "including", and the like; and that terms such as "consisting essentially of' and "consists essentially of' have the meaning ascribed to them in U.S. Patent law, e.g., they allow for elements not explicitly recited, but exclude elements that are found in the prior art or that affect a basic or novel characteristic of the invention.

These and other embodiments are disclosed or are obvious from and encompassed by, the following Detailed Description.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

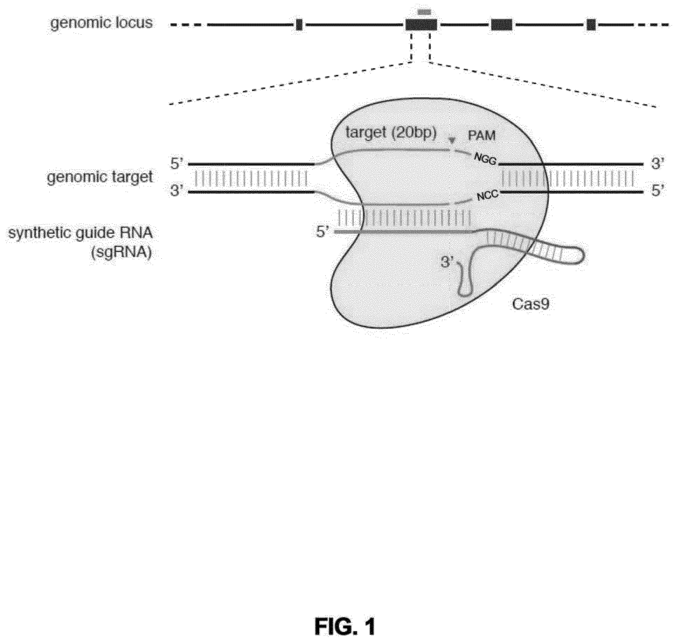

**Figure 1** shows a schematic of RNA-guided Cas9 nuclease. The Cas9 nuclease from Streptococcus pyogenes is targeted to genomic DNA by a synthetic guide RNA (sgRNA) consisting of a 20-nt guide sequence and a scaffold. The guide sequence base-pairs with the DNA target, directly upstream of a requisite 5'-NGG protospacer adjacent motif (PAM), and Cas9 mediates a double-stranded break (DSB) ∼3 bp upstream of the PAM (indicated by triangle).
**Figure 2A-F** shows an exemplary CRISPR system and a possible mechanism of action (A), an example adaptation for expression in eukaryotic cells, and results of tests assessing nuclear localization and CRISPR activity (B-F).
**Figure 3** shows a schematic representation of AAV in vivo delivery plasmids utilizing inverted terminal repeats (ITRs) sequences and guide chimeric RNAs to preferably aid delivery by AAV or AAV-associated systems.
**Figure 4A-D** shows a circular depiction of the phylogenetic analysis revealing five families of Cas9s, including three groups of large Cas9s (∼1400 amino acids) and two of small Cas9s (∼1100 amino acids).
**Figure 5A-F** shows a liner depiction of the phylogenetic analysis revealing five families of Cas9s, including three groups of large Cas9s (∼1400 amino acids) and two of small Cas9s (∼1100 amino acids).
**Figure 6** shows a graph representing the length distribution of Cas9 orthologs.
**Figure 7** shows a representation of the sequence logos of the PAMs of the Cas9 orthologs as reverse complements.
**Figure 8A-J** shows 18 chimeric RNA structures that preserved the sequence and secondary structures of the tracr mate:tracr sequence duplex while shortening the region of base-pairing and fusing the two RNA elements through an artificial loop.
**Figure 9A-O** shows a list of the human codon optimized Cas9 sequences to pair with the chimeric guide RNAs provided in Figures 8A-J.
**Figure 10A-M** shows sequences where the mutation points are located within the SpCas9 gene.
**Figure 11** shows Type II CRISPR loci in different organisms.
**Figure 12** shows guide RNA sequences corresponding to CRISPR loci in different organisms.
**Figure 13A-II** shows a table listing Cas9 orthologs and their corresponding PAM sequences.
**Figure 14** shows that the PAM for Staphylococcus aureus subspecies Aureus Cas9 is NNGRR.
**Figure 15A-D** shows single and multiple vector designs for SaCas9.
**Figrue 16** shows a vector design and gel images for Cas9 orthologs and respective sgRNAs being used to cleave two candidate targets present in a pUC19-based library.
**Figure 17** shows in vitro cleavage by SpCas9, St3Cas9, Sp_St3 chimera and St3_Sp chimera. The PAMs for St3Cas9 and St3_Sp chimeric Cas9 are NGG.
**Figure 18** shows an image of the CRISPRs web server.
**Figure 19A-L** shows a multiple sequence alignment for 12 Cas9 orthologs. Two catalytic residues are highlighted. The first residue highlighted is the catalytic Asp residue in the RuvCI domain, and the second residue highlighted is the catalytic His residue in the HNH domain. Mutation of one or the other residue into Ala can convert Cas9 into a nickase. Mutation of both residues converts Cas9 into a catalytically null mutant - useful for generic DNA binding.

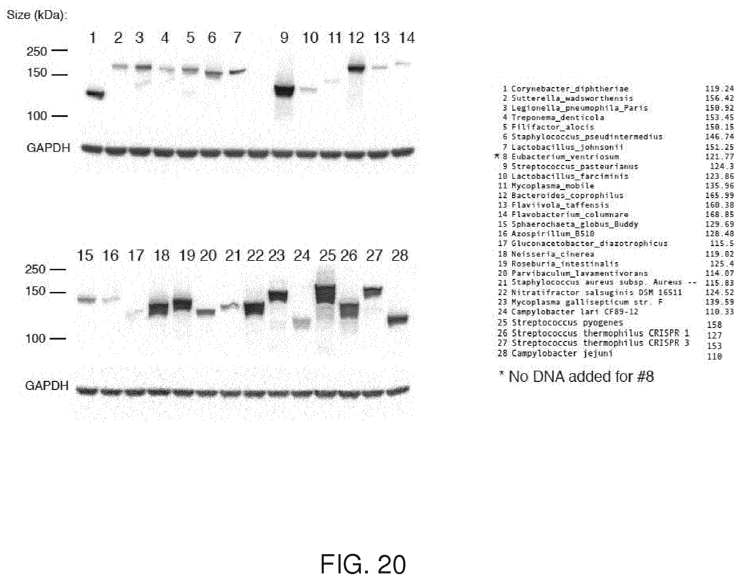

**Figure 20** shows a western blot showing that Cas9 orthologs are expressed in HEK 293FT cells; DNA plasmids encoding Cas9 orthologs are transfected into HEK 293FT cells and cell lysates are harvested for Western blot. No DNA is transfected for Cas9 ortholog #8.
**Figure 21** shows in vitro cleavage of candidate targets on pUC19 plasmid by 10 Cas9 orthologs. Consensus PAMs are predicted by sequence logos (Figure 7), based on which candidate targets on pUC19 are chosen. 7 of 9 new Cas9 orthologs tested have successfully cleaved at least one pUC19 target. SpCas9 can also cleave NGA in vitro.
**Figure 22** shows a Representative Surveyor Gel showing genomic cleavage by SaCas9.
**Figure 23** shows Genome Cleavage Efficiency of PAM Sequences (All targets).
**Figure 24** shows Genome Cleavage Efficiency of PAM Sequences (Cleaved targets)
**Figure 25** shows Genome Cleavage Efficiency of PAM Sequences (All targets, discard low-efficiency and orphan targets).
**Figure 26** shows Genome Cleavage Efficiency of PAM Sequences (Cleaved targets, discard low-efficiency and orphan targets).
**Figure 27** shows a Sequence Logo for Working Cleaved Spacers & PAMs (New endogenous genome test showing that T is not required).

The figures herein are for illustrative purposes only and are not necessarily drawn to scale.

### DETAILED DESCRIPTION OF THE INVENTION

The invention relates to the engineering and optimization of systems, methods and compositions used for the control of gene expression involving sequence targeting, such as genome perturbation or gene-editing, that relate to the CRISPR-Cas system and components thereof. In advantageous embodiments, the CRISPR enzyme is a Cas enzyme, e.g. a Cas9 ortholog.

The invention uses nucleic acids to bind target DNA sequences. This is advantageous as nucleic acids are much easier and cheaper to produce than, for example, peptides, and the specificity can be varied according to the length of the stretch where homology is sought. Complex 3-D positioning of multiple fingers, for example is not required.

The terms "polynucleotide", "nucleotide", "nucleotide sequence", "nucleic acid" and "oligonucleotide" are used interchangeably. They refer to a polymeric form of nucleotides of any length, either deoxyribonucleotides or ribonucleotides, or analogs thereof. Polynucleotides may have any three dimensional structure, and may perform any function, known or unknown. The following are non-limiting examples of polynucleotides: coding or non-coding regions of a gene or gene fragment, loci (locus) defined from linkage analysis, exons, introns, messenger RNA (mRNA), transfer RNA, ribosomal RNA, short interfering RNA (siRNA), short-hairpin RNA (shRNA), micro-RNA (miRNA), ribozymes, cDNA, recombinant polynucleotides, branched polynucleotides, plasmids, vectors, isolated DNA of any sequence, isolated RNA of any sequence, nucleic acid probes, and primers. The term also encompasses nucleic-acid-like structures with synthetic backbones, see, e.g., Eckstein, 1991; Baserga et al., 1992; Milligan, 1993; WO 97/03211; WO 96/39154; Mata, 1997; Strauss-Soukup, 1997; and Samstag, 1996. A polynucleotide may comprise one or more modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified after polymerization, such as by conjugation with a labeling component.

In aspects of the invention the terms "chimeric RNA", "chimeric guide RNA", "guide RNA", "single guide RNA" and "synthetic guide RNA" are used interchangeably and refer to the polynucleotide sequence comprising the guide sequence, the tracr sequence and the tracr mate sequence. The term "guide sequence" refers to the about 20bp sequence within the guide RNA that specifies the target site and may be used interchangeably with the terms "guide" or "spacer". The term "tracr mate sequence" may also be used interchangeably with the term "direct repeat(s)" .

As used herein the term "wild type" is a term of the art understood by skilled persons and means the typical form of an organism, strain, gene or characteristic as it occurs in nature as distinguished from mutant or variant forms.

As used herein the term "variant" should be taken to mean the exhibition of qualities that have a pattern that deviates from what occurs in nature.

The terms "non-naturally occurring" or "engineered" are used interchangeably and indicate the involvement of the hand of man. The terms, when referring to nucleic acid molecules or polypeptides mean that the nucleic acid molecule or the polypeptide is at least substantially free from at least one other component with which they are naturally associated in nature and as found in nature.

"Complementarity" refers to the ability of a nucleic acid to form hydrogen bond(s) with another nucleic acid sequence by either traditional Watson-Crick base pairing or other non-traditional types. A percent complementarity indicates the percentage of residues in a nucleic acid molecule which can form hydrogen bonds (e.g., Watson-Crick base pairing) with a second nucleic acid sequence (e.g., 5, 6, 7, 8, 9, 10 out of 10 being 50%, 60%, 70%, 80%, 90%, and 100% complementary). "Perfectly complementary" means that all the contiguous residues of a nucleic acid sequence will hydrogen bond with the same number of contiguous residues in a second nucleic acid sequence. "Substantially complementary" as used herein refers to a degree of complementarity that is at least 60%, 65%, 70%, 75%, 80%, 85%, 90%, 95%, 97%, 98%, 99%, or 100% over a region of 8, 9, 10, 11, 12, 13, 14, 15, 16, 17, 18, 19, 20, 21, 22, 23, 24, 25, 30, 35, 40, 45, 50, or more nucleotides, or refers to two nucleic acids that hybridize under stringent conditions.

As used herein, "stringent conditions" for hybridization refer to conditions under which a nucleic acid having complementarity to a target sequence predominantly hybridizes with the target sequence, and substantially does not hybridize to non-target sequences. Stringent conditions are generally sequence-dependent, and vary depending on a number of factors. In general, the longer the sequence, the higher the temperature at which the sequence specifically hybridizes to its target sequence. Non-limiting examples of stringent conditions are described in detail in Tijssen (1993), Laboratory Techniques In Biochemistry And Molecular Biology-Hybridization With Nucleic Acid Probes Part I, Second Chapter "Overview of principles of hybridization and the strategy of nucleic acid probe assay", Elsevier, N.Y. Where reference is made to a polynucleotide sequence, then complementary or partially complementary sequences are also envisaged. These are preferably capable of hybridising to the reference sequence under highly stringent conditions. Generally, in order to maximize the hybridization rate, relatively low-stringency hybridization conditions are selected: about 20 to 25° C lower than the thermal melting point (Tm). The Tm is the temperature at which 50% of specific target sequence hybridizes to a perfectly complementary probe in solution at a defined ionic strength and pH. Generally, in order to require at least about 85% nucleotide complementarity of hybridized sequences, highly stringent washing conditions are selected to be about 5 to 15° C lower than the Tm . In order to require at least about 70% nucleotide complementarity of hybridized sequences, moderately-stringent washing conditions are selected to be about 15 to 30° C.. lower than the Tm . Highly permissive (very low stringency) washing conditions may be as low as 50° C below the Tm , allowing a high level of mis-matching between hybridized sequences. Those skilled in the art will recognize that other physical and chemical parameters in the hybridization and wash stages can also be altered to affect the outcome of a detectable hybridization signal from a specific level of homology between target and probe sequences. Preferred highly stringent conditions comprise incubation in 50% formamide, 5×SSC, and 1% SDS at 42° C, or incubation in 5×SSC and 1% SDS at 65° C, with wash in 0.2×SSC and 0.1% SDS at 65° C.

"Hybridization" refers to a reaction in which one or more polynucleotides react to form a complex that is stabilized via hydrogen bonding between the bases of the nucleotide residues. The hydrogen bonding may occur by Watson Crick base pairing, Hoogstein binding, or in any other sequence specific manner. The complex may comprise two strands forming a duplex structure, three or more strands forming a multi stranded complex, a single self-hybridizing strand, or any combination of these. A hybridization reaction may constitute a step in a more extensive process, such as the initiation of PCR, or the cleavage of a polynucleotide by an enzyme. A sequence capable of hybridizing with a given sequence is referred to as the "complement" of the given sequence.

As used herein, the term "genomic locus" or "locus" (plural loci) is the specific location of a gene or DNA sequence on a chromosome. A "gene" refers to stretches of DNA or RNA that encode a polypeptide or an RNA chain that has functional role to play in an organism and hence is the molecular unit of heredity in living organisms. For the purpose of this invention it may be considered that genes include regions which regulate the production of the gene product, whether or not such regulatory sequences are adjacent to coding and/or transcribed sequences. Accordingly, a gene includes, but is not necessarily limited to, promoter sequences, terminators, translational regulatory sequences such as ribosome binding sites and internal ribosome entry sites, enhancers, silencers, insulators, boundary elements, replication origins, matrix attachment sites and locus control regions.

As used herein, "expression of a genomic locus" or "gene expression" is the process by which information from a gene is used in the synthesis of a functional gene product. The products of gene expression are often proteins, but in non-protein coding genes such as rRNA genes or tRNA genes, the product is functional RNA. The process of gene expression is used by all known life - eukaryotes (including multicellular organisms), prokaryotes (bacteria and archaea) and viruses to generate functional products to survive. As used herein "expression" of a gene or nucleic acid encompasses not only cellular gene expression, but also the transcription and translation of nucleic acid(s) in cloning systems and in any other context. As used herein, "expression" also refers to the process by which a polynucleotide is transcribed from a DNA template (such as into and mRNA or other RNA transcript) and/or the process by which a transcribed mRNA is subsequently translated into peptides, polypeptides, or proteins. Transcripts and encoded polypeptides may be collectively referred to as "gene product." If the polynucleotide is derived from genomic DNA, expression may include splicing of the mRNA in a eukaryotic cell.

The terms "polypeptide", "peptide" and "protein" are used interchangeably herein to refer to polymers of amino acids of any length. The polymer may be linear or branched, it may comprise modified amino acids, and it may be interrupted by non amino acids. The terms also encompass an amino acid polymer that has been modified; for example, disulfide bond formation, glycosylation, lipidation, acetylation, phosphorylation, or any other manipulation, such as conjugation with a labeling component. As used herein the term "amino acid" includes natural and/or unnatural or synthetic amino acids, including glycine and both the D or L optical isomers, and amino acid analogs and peptidomimetics.

As used herein, the term "domain" or "protein domain" refers to a part of a protein sequence that may exist and function independently of the rest of the protein chain.

As described in aspects of the invention, sequence identity is related to sequence homology. Homology comparisons may be conducted by eye, or more usually, with the aid of readily available sequence comparison programs. These commercially available computer programs may calculate percent (%) homology between two or more sequences and may also calculate the sequence identity shared by two or more amino acid or nucleic acid sequences. Sequence homologies may be generated by any of a number of computer programs known in the art, for example BLAST or FASTA, etc. A suitable computer program for carrying out such an alignment is the GCG Wisconsin Bestfit package (University of Wisconsin, U.S.A; Devereux et al., 1984, Nucleic Acids Research 12:387). Examples of other software than may perform sequence comparisons include, but are not limited to, the BLAST package (see Ausubel et al., 1999 ibid - Chapter 18), FASTA (Atschul et al., 1990, J. Mol. Biol., 403-410) and the GENEWORKS suite of comparison tools. Both BLAST and FASTA are available for offline and online searching (see Ausubel et al., 1999 ibid, pages 7-58 to 7-60). However it is preferred to use the GCG Bestfit program.

% homology may be calculated over contiguous sequences, i.e., one sequence is aligned with the other sequence and each amino acid or nucleotide in one sequence is directly compared with the corresponding amino acid or nucleotide in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion may cause the following amino acid residues to be put out of alignment, thus potentially resulting in a large reduction in % homology when a global alignment is performed. Consequently, most sequence comparison methods are designed to produce optimal alignments that take into consideration possible insertions and deletions without unduly penalizing the overall homology or identity score. This is achieved by inserting "gaps" in the sequence alignment to try to maximize local homology or identity.

However, these more complex methods assign "gap penalties" to each gap that occurs in the alignment so that, for the same number of identical amino acids, a sequence alignment with as few gaps as possible - reflecting higher relatedness between the two compared sequences - may achieve a higher score than one with many gaps. "Affinity gap costs" are typically used that charge a relatively high cost for the existence of a gap and a smaller penalty for each subsequent residue in the gap. This is the most commonly used gap scoring system. High gap penalties may, of course, produce optimized alignments with fewer gaps. Most alignment programs allow the gap penalties to be modified. However, it is preferred to use the default values when using such software for sequence comparisons. For example, when using the GCG Wisconsin Bestfit package the default gap penalty for amino acid sequences is -12 for a gap and -4 for each extension.

Calculation of maximum % homology therefore first requires the production of an optimal alignment, taking into consideration gap penalties. A suitable computer program for carrying out such an alignment is the GCG Wisconsin Bestfit package (Devereux et al., 1984 Nuc. Acids Research 12 p387). Examples of other software that may perform sequence comparisons include, but are not limited to, the BLAST package (see Ausubel et al., 1999 Short Protocols in Molecular Biology, 4th Ed. - Chapter 18), FASTA (Altschul et al., 1990 J. Mol. Biol. 403-410) and the GENEWORKS suite of comparison tools. Both BLAST and FASTA are available for offline and online searching (see Ausubel et al., 1999, Short Protocols in Molecular Biology, pages 7-58 to 7-60). However, for some applications, it is preferred to use the GCG Bestfit program. A new tool, called BLAST 2 Sequences is also available for comparing protein and nucleotide sequences (see FEMS Microbiol Lett. 1999 174(2): 247-50; FEMS Microbiol Lett. 1999 177(1): 187-8 and the website of the National Center for Biotechnology information at the website of the National Institutes for Health).

Although the final % homology may be measured in terms of identity, the alignment process itself is typically not based on an all-or-nothing pair comparison. Instead, a scaled similarity score matrix is generally used that assigns scores to each pair-wise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table, if supplied (see user manual for further details). For some applications, it is preferred to use the public default values for the GCG package, or in the case of other software, the default matrix, such as BLOSUM62.

Alternatively, percentage homologies may be calculated using the multiple alignment feature in DNASISTM (Hitachi Software), based on an algorithm, analogous to CLUSTAL (Higgins DG & Sharp PM (1988), Gene 73(1), 237-244). Once the software has produced an optimal alignment, it is possible to calculate % homology, preferably % sequence identity. The software typically does this as part of the sequence comparison and generates a numerical result.

The sequences may also have deletions, insertions or substitutions of amino acid residues which produce a silent change and result in a functionally equivalent substance. Deliberate amino acid substitutions may be made on the basis of similarity in amino acid properties (such as polarity, charge, solubility, hydrophobicity, hydrophilicity, and/or the amphipathic nature of the residues) and it is therefore useful to group amino acids together in functional groups. Amino acids may be grouped together based on the properties of their side chains alone. However, it is more useful to include mutation data as well. The sets of amino acids thus derived are likely to be conserved for structural reasons. These sets may be described in the form of a Venn diagram (Livingstone C.D. and Barton G.J. (1993) "Protein sequence alignments: a strategy for the hierarchical analysis of residue conservation" Comput. Appl. Biosci. 9: 745-756) (Taylor W.R. (1986) "The classification of amino acid conservation" J. Theor. Biol. 119; 205-218). Conservative substitutions may be made, for example according to the table below which describes a generally accepted Venn diagram grouping of amino acids.

| Set | | Sub-set | |
|---|---|---|---|
| Hydrophobic | F W Y H K M I L V A G C | Aromatic | F W Y H |
| | | Aliphatic | I L V |
| Polar | W Y H K R E D C S T N Q | Charged | H K R E D |
| | | Positively charged | H K R |
| | | Negatively charged | E D |
| Small | V C A G S P T N D | Tiny | A G S |

Embodiments of the invention include sequences (both polynucleotide or polypeptide) which may comprise homologous substitution (substitution and replacement are both used herein to mean the interchange of an existing amino acid residue or nucleotide, with an alternative residue or nucleotide) that may occur i.e., like-for-like substitution in the case of amino acids such as basic for basic, acidic for acidic, polar for polar, etc. Non-homologous substitution may also occur i.e., from one class of residue to another or alternatively involving the inclusion of unnatural amino acids such as ornithine (hereinafter referred to as Z), diaminobutyric acid ornithine (hereinafter referred to as B), norleucine ornithine (hereinafter referred to as O), pyridylalanine, thienylalanine, naphthylalanine and phenylglycine.

Variant amino acid sequences may include suitable spacer groups that may be inserted between any two amino acid residues of the sequence including alkyl groups such as methyl, ethyl or propyl groups in addition to amino acid spacers such as glycine or β-alanine residues. A further form of variation, which involves the presence of one or more amino acid residues in peptoid form, may be well understood by those skilled in the art. For the avoidance of doubt, "the peptoid form" is used to refer to variant amino acid residues wherein the α-carbon substituent group is on the residue's nitrogen atom rather than the α-carbon. Processes for preparing peptides in the peptoid form are known in the art, for example Simon RJ et al., PNAS (1992) 89(20), 9367-9371 and Horwell DC, Trends Biotechnol. (1995) 13(4), 132-134.

The practice of the present invention employs, unless otherwise indicated, conventional techniques of immunology, biochemistry, chemistry, molecular biology, microbiology, cell biology, genomics and recombinant DNA, which are within the skill of the art. See Sambrook, Fritsch and Maniatis, MOLECULAR CLONING: A LABORATORY MANUAL, 2nd edition (1989); CURRENT PROTOCOLS IN MOLECULAR BIOLOGY (F. M. Ausubel, et al. eds., (1987)); the series METHODS IN ENZYMOLOGY (Academic Press, Inc.): PCR 2: A PRACTICAL APPROACH (M.J. MacPherson, B.D. Hames and G.R. Taylor eds. (1995)), Harlow and Lane, eds. (1988) ANTIBODIES, A LABORATORY MANUAL, and ANIMAL CELL CULTURE (R.I. Freshney, ed. (1987)).

In one aspect, the invention provides for vectors that are used in the engineering and optimization of CRISPR-Cas systems.

As used herein, a "vector" is a tool that allows or facilitates the transfer of an entity from one environment to another. It is a replicon, such as a plasmid, phage, or cosmid, into which another DNA segment may be inserted so as to bring about the replication of the inserted segment. Generally, a vector is capable of replication when associated with the proper control elements. In general, the term "vector" refers to a nucleic acid molecule capable of transporting another nucleic acid to which it has been linked. Vectors include, but are not limited to, nucleic acid molecules that are single-stranded, double-stranded, or partially double-stranded; nucleic acid molecules that comprise one or more free ends, no free ends (e.g. circular); nucleic acid molecules that comprise DNA, RNA, or both; and other varieties of polynucleotides known in the art. One type of vector is a "plasmid," which refers to a circular double stranded DNA loop into which additional DNA segments can be inserted, such as by standard molecular cloning techniques. Another type of vector is a viral vector, wherein virally-derived DNA or RNA sequences are present in the vector for packaging into a virus (e.g. retroviruses, replication defective retroviruses, adenoviruses, replication defective adenoviruses, and adeno-associated viruses). Viral vectors also include polynucleotides carried by a virus for transfection into a host cell. Certain vectors are capable of autonomous replication in a host cell into which they are introduced (e.g. bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively-linked. Such vectors are referred to herein as "expression vectors." Common expression vectors of utility in recombinant DNA techniques are often in the form of plasmids. Further discussion of vectors is provided herein.

Recombinant expression vectors can comprise a nucleic acid of the invention in a form suitable for expression of the nucleic acid in a host cell, which means that the recombinant expression vectors include one or more regulatory elements, which may be selected on the basis of the host cells to be used for expression, that is operatively-linked to the nucleic acid sequence to be expressed. Within a recombinant expression vector, "operably linked" is intended to mean that the nucleotide sequence of interest is linked to the regulatory element(s) in a manner that allows for expression of the nucleotide sequence (e.g. in an in vitro transcription/translation system or in a host cell when the vector is introduced into the host cell). With regards to recombination and cloning methods, mention is made of U.S. patent application US 2004-0171156 A1.

Aspects and embodiments of the invention relate to bicistronic vectors for chimeric RNA and Cas9 or a Cas9 ortholog. In some embodiments, the Cas9 is driven by the CBh promoter. In some embodiments, the chimeric RNA is driven by a U6 promoter. Preferably, the CBh and U6 are used together in the sense that the Cas9 is driven by the CBh promoter and the chimeric RNA is driven by a U6 promoter. In some embodiments, the chimeric guide RNA consists of a 20bp guide sequence (Ns) joined to the tracr sequence (running from the first "U" of the lower strand to the end of the transcript), which is truncated at various positions as indicated. The guide and tracr sequences are preferably separated by the tracr-mate sequence. A preferred example of a tracr-mate sequence is GUUUUAGAGCUA. This is preferably followed by a loop sequence. The loop is preferably GAAA, but it is not limited to this sequence or indeed to being only 4bp in length. Indeeed, preferred loop forming sequences for use in hairpin structures are four nucleotides in length, and most preferably have the sequence GAAA. However, longer or shorter loop sequences may be used, as may alternative sequences. The sequences preferably include a nucleotide triplet (for example, AAA), and an additional nucleotide (for example C or G). Examples of loop forming sequences include CAAA and AAAG. Throughout this application, chimeric RNA may also be called single guide, or synthetic guide RNA (sgRNA).

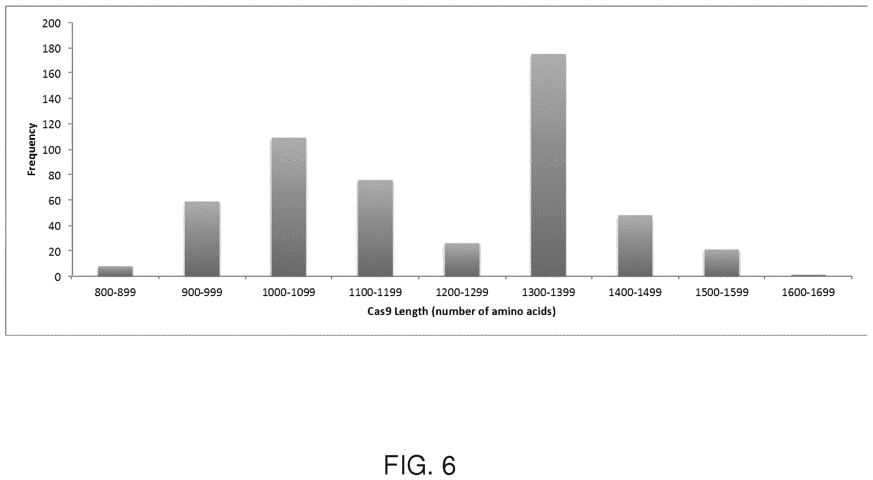

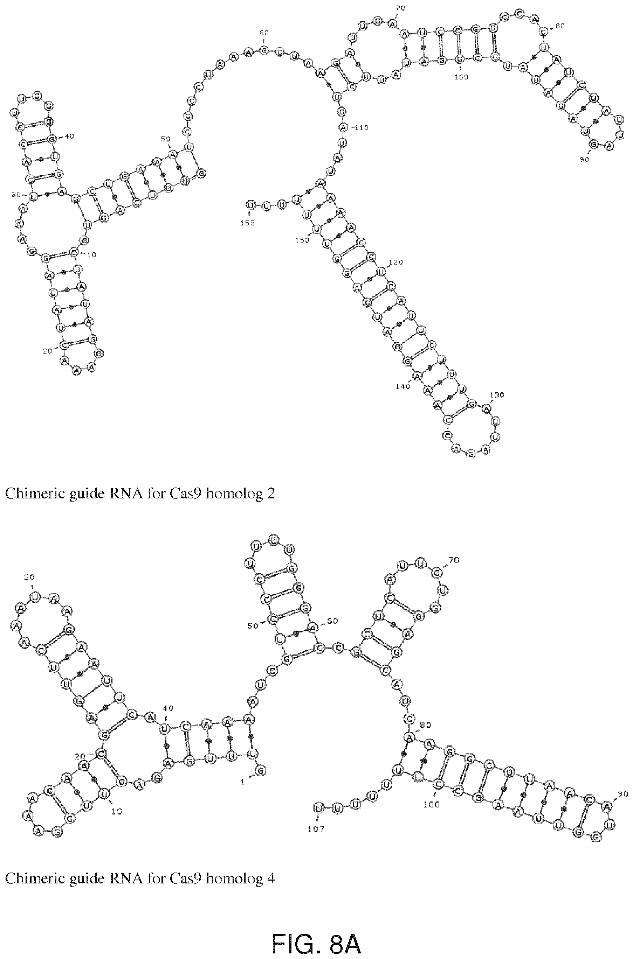

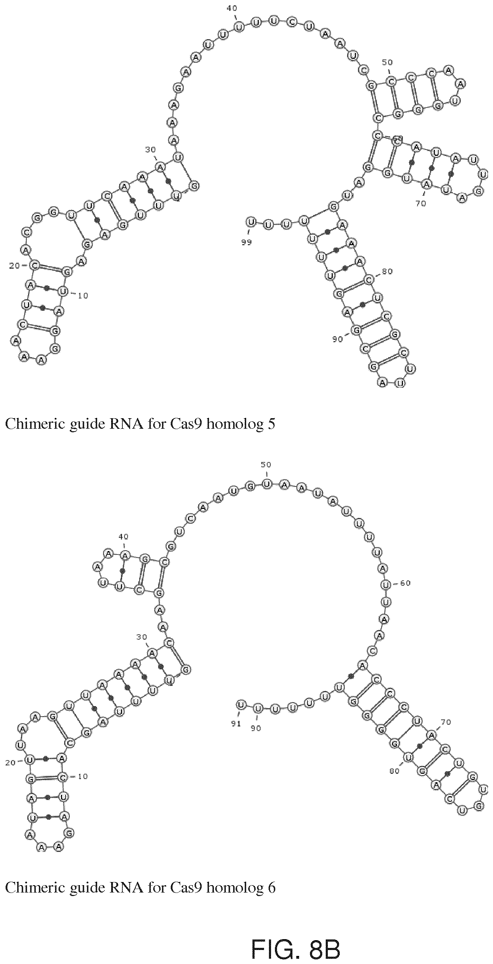

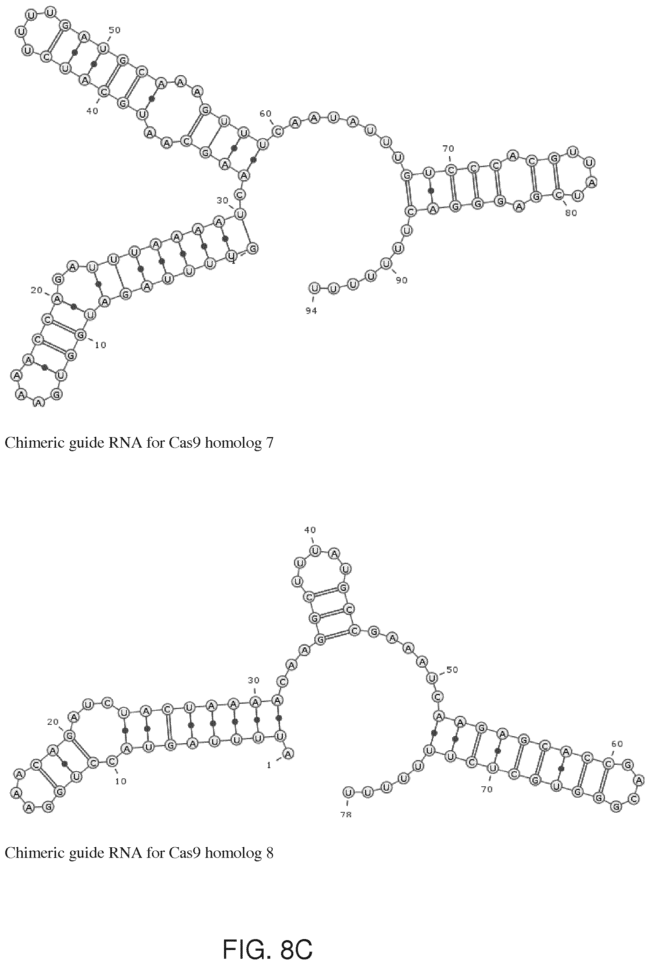

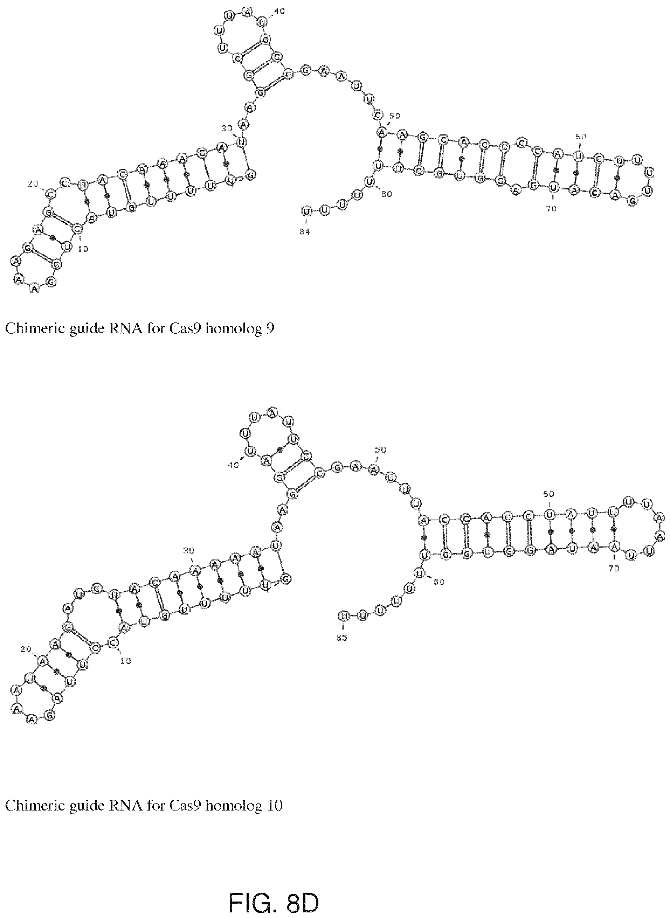

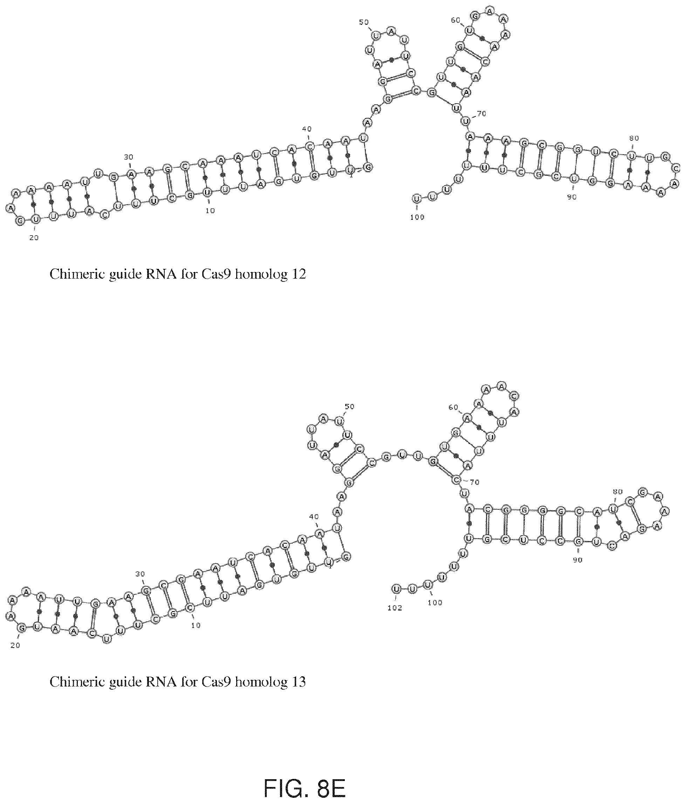

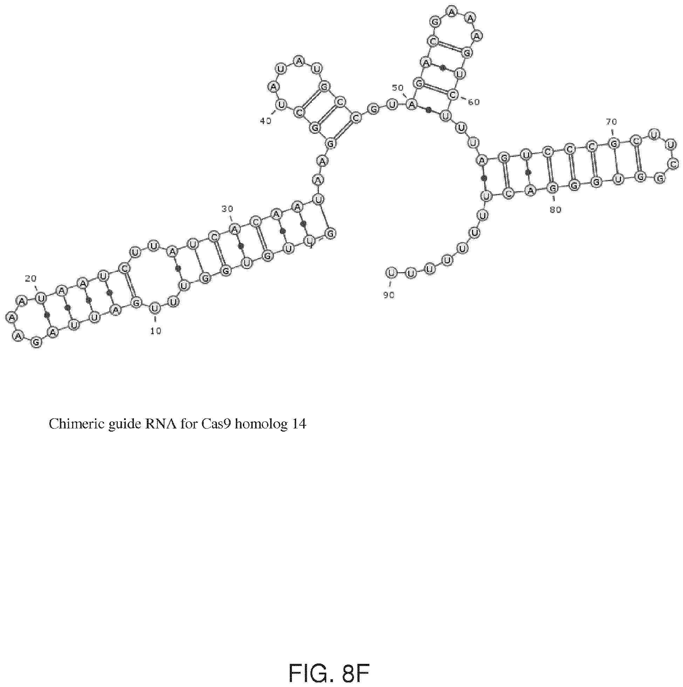

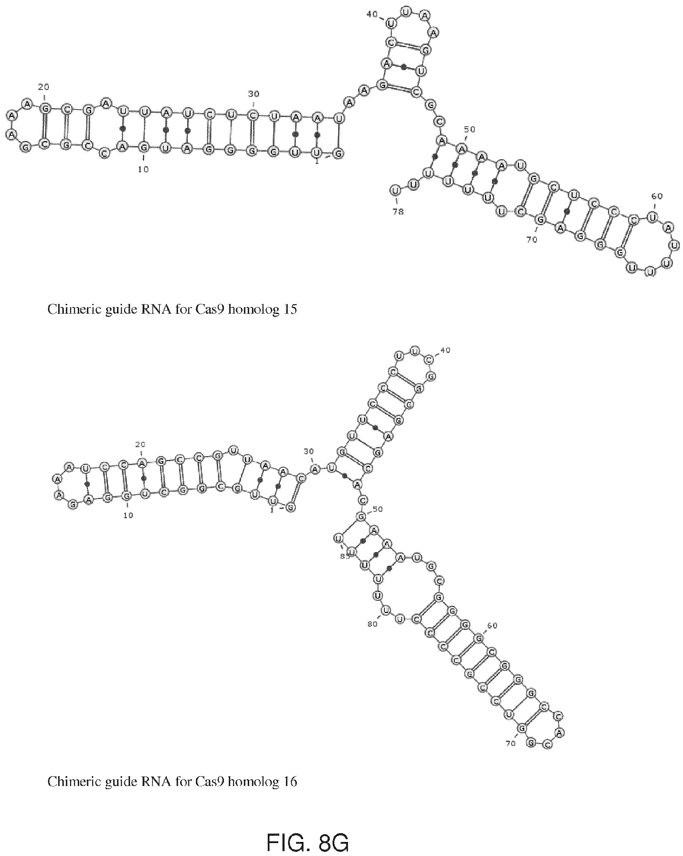

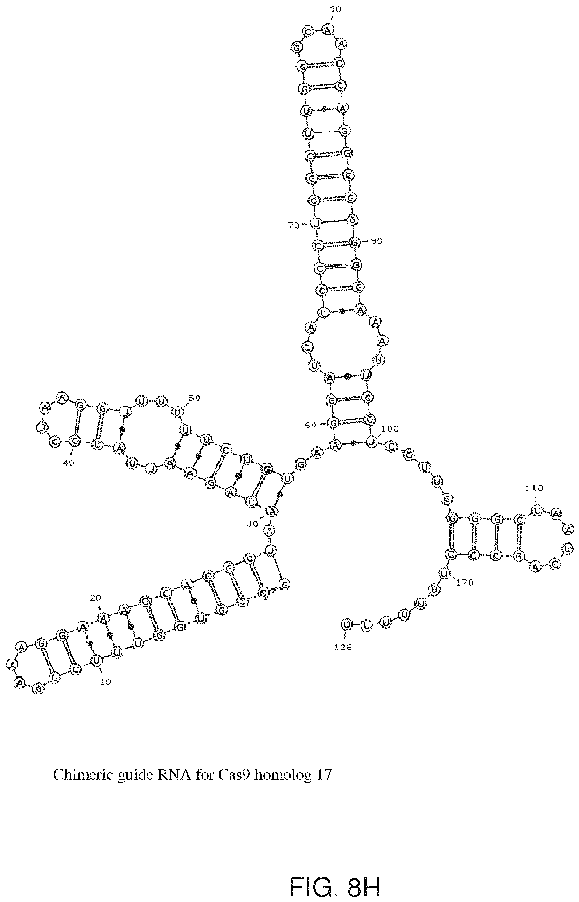

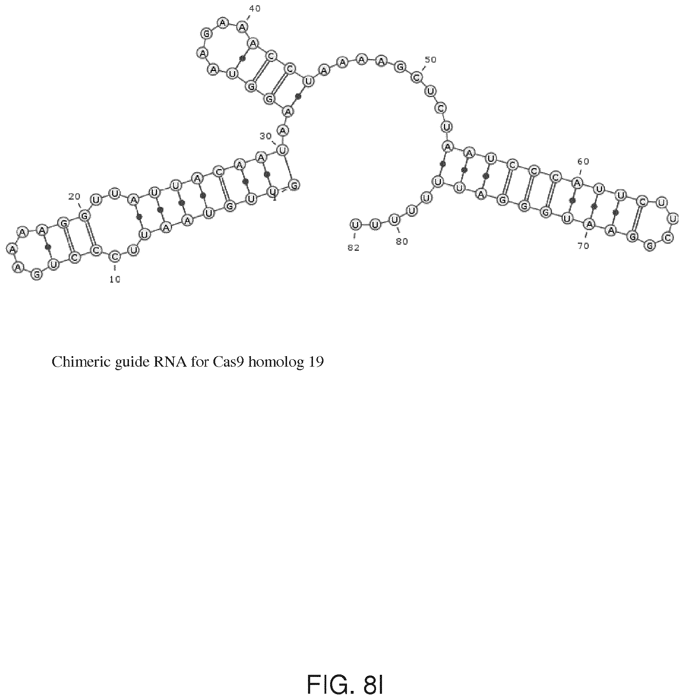

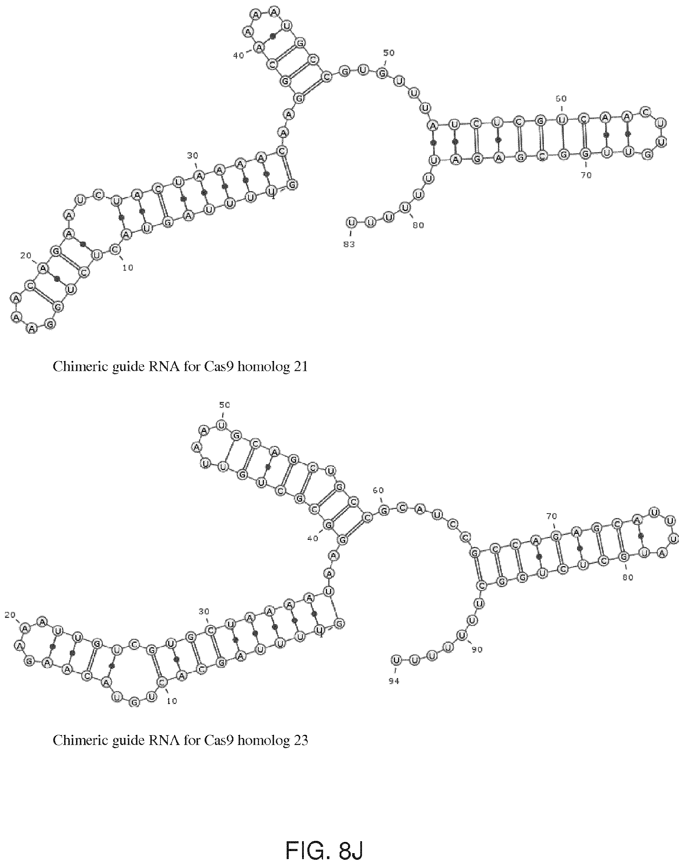

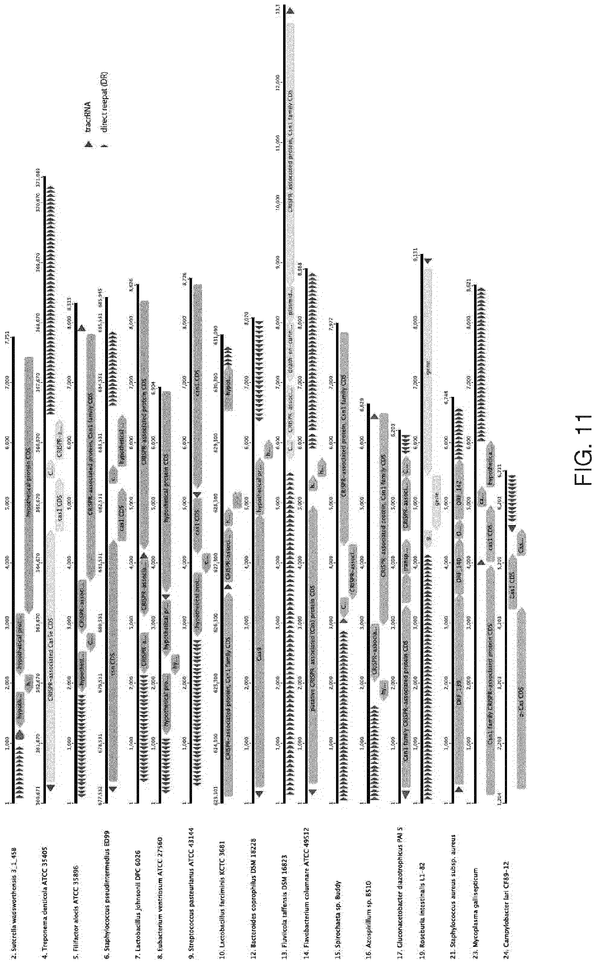

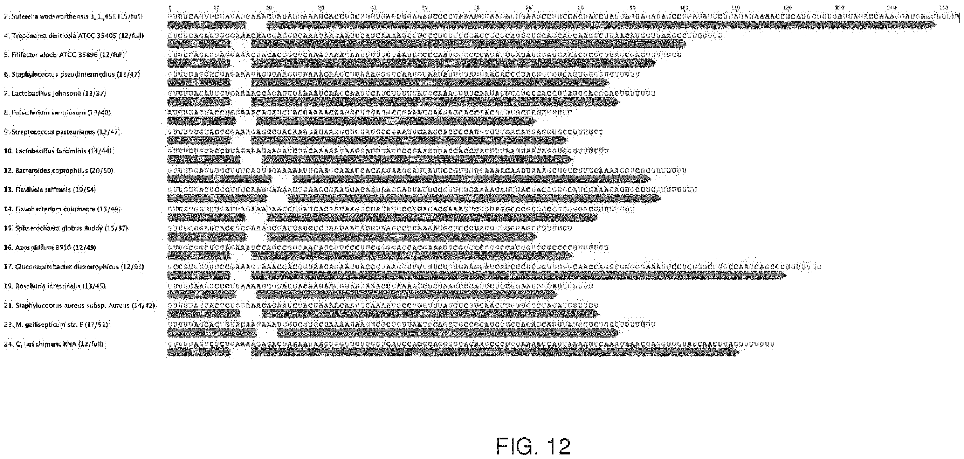

For instance, as described in specific detail in Example 2, chimeric guide RNAs may be designed as shown in Figure 8. The CRISPR loci in some of these families is depicted in Figure 11. The corresponding guide RNA sequences are shown in Figure 12. We analyzed the genomic DNA sequence within ∼2kb of the Cas9 proteins and identified direct repeats ranging from 35bp to 50bp, with intervening spacers ranging from 29bp to 35bp. Based on the direct repeat sequence, we searched for tracrRNA candidate sequences with the following criteria: outside the crRNA array but containing high degree of homology to direct repeats (as required for direct repeat:tracrRNA base-pairing; custom computational analysis), outside the coding regions of the protein components, containing Rho-independent transcriptional termination signals ∼60bp-120bp downstream from region of homology from with direct repeats, and co-folding with direct repeat to form a duplex, followed by two or more hairpin structures in the distal end of tracrRNA sequence. Based on these prediction criteria, we selected an initial set of 18 Cas9 proteins and their uniquely associated direct repeats and tracrRNAs distributed across all five Cas9 families. Applicants further generated a set of 18 chimeric RNA structures that preserved the sequence and secondary structures of the native direct repeat:tracrRNA duplex while shortening the region of base-pairing and fusing the two RNA elements through an artificial loop (Figures 6A-J).

The term "regulatory element" is intended to include promoters, enhancers, internal ribosomal entry sites (IRES), and other expression control elements (e.g. transcription termination signals, such as polyadenylation signals and poly-U sequences). Such regulatory elements are described, for example, in Goeddel, GENE EXPRESSION TECHNOLOGY: METHODS IN ENZYMOLOGY 185, Academic Press, San Diego, Calif. (1990). Regulatory elements include those that direct constitutive expression of a nucleotide sequence in many types of host cell and those that direct expression of the nucleotide sequence only in certain host cells (e.g., tissue-specific regulatory sequences). A tissue-specific promoter may direct expression primarily in a desired tissue of interest, such as muscle, neuron, bone, skin, blood, specific organs (e.g. liver, pancreas), or particular cell types (e.g. lymphocytes). Regulatory elements may also direct expression in a temporal-dependent manner, such as in a cell-cycle dependent or developmental stage-dependent manner, which may or may not also be tissue or cell-type specific. In some embodiments, a vector comprises one or more pol III promoter (e.g. 1, 2, 3, 4, 5, or more pol III promoters), one or more pol II promoters (e.g. 1, 2, 3, 4, 5, or more pol II promoters), one or more pol I promoters (e.g. 1, 2, 3, 4, 5, or more pol I promoters), or combinations thereof. Examples of pol III promoters include, but are not limited to, U6 and H1 promoters. Examples of pol II promoters include, but are not limited to, the retroviral Rous sarcoma virus (RSV) LTR promoter (optionally with the RSV enhancer), the cytomegalovirus (CMV) promoter (optionally with the CMV enhancer) [see, e.g., Boshart et al, Cell, 41:521-530 (1985)], the SV40 promoter, the dihydrofolate reductase promoter, the β-actin promoter, the phosphoglycerol kinase (PGK) promoter, and the EF1α promoter. Also encompassed by the term "regulatory element" are enhancer elements, such as WPRE; CMV enhancers; the R-U5' segment in LTR of HTLV-I (Mol. Cell. Biol., Vol. 8(1), p. 466-472, 1988); SV40 enhancer; and the intron sequence between exons 2 and 3 of rabbit β-globin (Proc. Natl. Acad. Sci. USA., Vol. 78(3), p. 1527-31, 1981). It will be appreciated by those skilled in the art that the design of the expression vector can depend on such factors as the choice of the host cell to be transformed, the level of expression desired, etc. A vector can be introduced into host cells to thereby produce transcripts, proteins, or peptides, including fusion proteins or peptides, encoded by nucleic acids as described herein (e.g., clustered regularly interspersed short palindromic repeats (CRISPR) transcripts, proteins, enzymes, mutant forms thereof, fusion proteins thereof, etc.). With regards to regulatory sequences, mention is made of U.S. patent application 10/491,026. With regards to promoters, mention is made of PCT publication WO 2011/028929 and U.S. application 12/511,940.

Vectors can be designed for expression of CRISPR transcripts (e.g. nucleic acid transcripts, proteins, or enzymes) in prokaryotic or eukaryotic cells. For example, CRISPR transcripts can be expressed in bacterial cells such as Escherichia coli, insect cells (using baculovirus expression vectors), yeast cells, or mammalian cells. Suitable host cells are discussed further in Goeddel, GENE EXPRESSION TECHNOLOGY: METHODS IN ENZYMOLOGY 185, Academic Press, San Diego, Calif. (1990). Alternatively, the recombinant expression vector can be transcribed and translated in vitro, for example using T7 promoter regulatory sequences and T7 polymerase.

Vectors may be introduced and propagated in a prokaryote or prokaryotic cell. In some embodiments, a prokaryote is used to amplify copies of a vector to be introduced into a eukaryotic cell or as an intermediate vector in the production of a vector to be introduced into a eukaryotic cell (e.g. amplifying a plasmid as part of a viral vector packaging system). In some embodiments, a prokaryote is used to amplify copies of a vector and express one or more nucleic acids, such as to provide a source of one or more proteins for delivery to a host cell or host organism. Expression of proteins in prokaryotes is most often carried out in Escherichia coli with vectors containing constitutive or inducible promoters directing the expression of either fusion or non-fusion proteins. Fusion vectors add a number of amino acids to a protein encoded therein, such as to the amino terminus of the recombinant protein. Such fusion vectors may serve one or more purposes, such as: (i) to increase expression of recombinant protein; (ii) to increase the solubility of the recombinant protein; and (iii) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Example fusion expression vectors include pGEX (Pharmacia Biotech Inc; Smith and Johnson, 1988. Gene 67: 31-40), pMAL (New England Biolabs, Beverly, Mass.) and pRIT5 (Pharmacia, Piscataway, N.J.) that fuse glutathione S-transferase (GST), maltose E binding protein, or protein A, respectively, to the target recombinant protein.

Examples of suitable inducible non-fusion E. coli expression vectors include pTrc (Amrann et al., (1988) Gene 69:301-315) and pET 11d (Studier et al., GENE EXPRESSION TECHNOLOGY: METHODS IN ENZYMOLOGY 185, Academic Press, San Diego, Calif. (1990)60-89).

In some embodiments, a vector is a yeast expression vector. Examples of vectors for expression in yeast Saccharomyces cerevisae include pYepSec1 (Baldari, et al., 1987. EMBO J. 6: 229-234), pMFa (Kuijan and Herskowitz, 1982. Cell 30: 933-943), pJRY88 (Schultz et al., 1987. Gene 54: 113-123), pYES2 (Invitrogen Corporation, San Diego, Calif.), and picZ (InVitrogen Corp, San Diego, Calif.).

In some embodiments, a vector drives protein expression in insect cells using baculovirus expression vectors. Baculovirus vectors available for expression of proteins in cultured insect cells (e.g., SF9 cells) include the pAc series (Smith, et al., 1983. Mol. Cell. Biol. 3: 2156-2165) and the pVL series (Lucklow and Summers, 1989. Virology 170: 31-39).

In some embodiments, a vector is capable of driving expression of one or more sequences in mammalian cells using a mammalian expression vector. Examples of mammalian expression vectors include pCDM8 (Seed, 1987. Nature 329: 840) and pMT2PC (Kaufman, et al., 1987. EMBO J. 6: 187-195). When used in mammalian cells, the expression vector's control functions are typically provided by one or more regulatory elements. For example, commonly used promoters are derived from polyoma, adenovirus 2, cytomegalovirus, simian virus 40, and others disclosed herein and known in the art. For other suitable expression systems for both prokaryotic and eukaryotic cells see, e.g., Chapters 16 and 17 of Sambrook, et al., MOLECULAR CLONING: A LABORATORY MANUAL. 2nd ed., Cold Spring Harbor Laboratory, Cold Spring Harbor Laboratory Press, Cold Spring Harbor, N.Y., 1989.

In some embodiments, the recombinant mammalian expression vector is capable of directing expression of the nucleic acid preferentially in a particular cell type (e.g., tissue-specific regulatory elements are used to express the nucleic acid). Tissue-specific regulatory elements are known in the art. Non-limiting examples of suitable tissue-specific promoters include the albumin promoter (liver-specific; Pinkert, et al., 1987. Genes Dev. 1: 268-277), lymphoid-specific promoters (Calame and Eaton, 1988. Adv. Immunol. 43: 235-275), in particular promoters of T cell receptors (Winoto and Baltimore, 1989. EMBO J. 8: 729-733) and immunoglobulins (Baneiji, et al., 1983. Cell 33: 729-740; Queen and Baltimore, 1983. Cell 33: 741-748), neuron-specific promoters (e.g., the neurofilament promoter; Byrne and Ruddle, 1989. Proc. Natl. Acad. Sci. USA 86: 5473-5477), pancreas-specific promoters (Edlund, et al., 1985. Science 230: 912-916), and mammary gland-specific promoters (e.g., milk whey promoter; U.S. Pat. No. 4,873,316 and European Application Publication No. 264,166). Developmentally-regulated promoters are also encompassed, e.g., the murine hox promoters (Kessel and Gruss, 1990. Science 249: 374-379) and the α-fetoprotein promoter (Campes and Tilghman, 1989. Genes Dev. 3: 537-546). With regards to these prokaryotic and eukaryotic vectors, mention is made of U.S. Patent 6,750,059. Other embodiments of the invention may relate to the use of viral vectors, with regards to which mention is made of U.S. Patent application 13/092,085. Tissue-specific regulatory elements are known in the art and in this regard, mention is made of U.S. Patent 7,776,321.

In some embodiments, a regulatory element is operably linked to one or more elements of a CRISPR system so as to drive expression of the one or more elements of the CRISPR system. In general, CRISPRs (Clustered Regularly Interspaced Short Palindromic Repeats), also known as SPIDRs (SPacer Interspersed Direct Repeats), constitute a family of DNA loci that are usually specific to a particular bacterial species. The CRISPR locus comprises a distinct class of interspersed short sequence repeats (SSRs) that were recognized in E. coli (Ishino et al., J. Bacteriol., 169:5429-5433 [1987]; and Nakata et al., J. Bacteriol., 171:3553-3556 [1989]), and associated genes. Similar interspersed SSRs have been identified in Haloferax mediterranei, Streptococcus pyogenes, Anabaena, and Mycobacterium tuberculosis (See, Groenen et al., Mol. Microbiol., 10:1057-1065 [1993]; Hoe et al., Emerg. Infect. Dis., 5:254-263 [1999]; Masepohl et al., Biochim. Biophys. Acta 1307:26-30 [1996]; and Mojica et al., Mol. Microbiol., 17:85-93 [1995]). The CRISPR loci typically differ from other SSRs by the structure of the repeats, which have been termed short regularly spaced repeats (SRSRs) (Janssen et al., OMICS J. Integ. Biol., 6:23-33 [2002]; and Mojica et al., Mol. Microbiol., 36:244-246 [2000]). In general, the repeats are short elements that occur in clusters that are regularly spaced by unique intervening sequences with a substantially constant length (Mojica et al., [2000], supra). Although the repeat sequences are highly conserved between strains, the number of interspersed repeats and the sequences of the spacer regions typically differ from strain to strain (van Embden et al., J. Bacteriol., 182:2393-2401 [2000]). CRISPR loci have been identified in more than 40 prokaryotes (See e.g., Jansen et al., Mol. Microbiol., 43:1565-1575 [2002]; and Mojica et al., [2005]) including, but not limited to Aeropyrum, Pyrobaculum, Sulfolobus, Archaeoglobus, Halocarcula, Methanobacterium, Methanococcus, Methanosarcina, Methanopyrus, Pyrococcus, Picrophilus, Thermoplasma, Corynebacterium, Mycobacterium, Streptomyces, Aquifex, Porphyromonas, Chlorobium, Thermus, Bacillus, Listeria, Staphylococcus, Clostridium, Thermoanaerobacter, Mycoplasma, Fusobacterium, Azarcus, Chromobacterium, Neisseria, Nitrosomonas, Desulfovibrio, Geobacter, Myxococcus, Campylobacter, Wolinella, Acinetobacter, Erwinia, Escherichia, Legionella, Methylococcus, Pasteurella, Photobacterium, Salmonella, Xanthomonas, Yersinia, Treponema, and Thermotoga.

In general, "CRISPR system" refers collectively to transcripts and other elements involved in the expression of or directing the activity of CRISPR-associated ("Cas") genes, including sequences encoding a Cas gene, a tracr (trans-activating CRISPR) sequence (e.g. tracrRNA or an active partial tracrRNA), a tracr-mate sequence (encompassing a "direct repeat" and a tracrRNA-processed partial direct repeat in the context of an endogenous CRISPR system), a guide sequence (also referred to as a "spacer" in the context of an endogenous CRISPR system), or other sequences and transcripts from a CRISPR locus. In some embodiments, one or more elements of a CRISPR system is derived from a type I, type II, or type III CRISPR system. In some embodiments, one or more elements of a CRISPR system is derived from a particular organism comprising an endogenous CRISPR system, such as Streptococcus pyogenes. In general, a CRISPR system is characterized by elements that promote the formation of a CRISPR complex at the site of a target sequence (also referred to as a protospacer in the context of an endogenous CRISPR system). In the context of formation of a CRISPR complex, "target sequence" refers to a sequence to which a guide sequence is designed to have complementarity, where hybridization between a target sequence and a guide sequence promotes the formation of a CRISPR complex. A target sequence may comprise any polynucleotide, such as DNA or RNA polynucleotides. In some embodiments, a target sequence is located in the nucleus or cytoplasm of a cell.

In preferred embodiments of the invention, the CRISPR system is a type II CRISPR system and the Cas enzyme is Cas9, which catalyzes DNA cleavage. Enzymatic action by Cas9 derived from Streptococcus pyogenes or any closely related Cas9 generates double stranded breaks at target site sequences which hybridize to 20 nucleotides of the guide sequence and that have a protospacer-adjacent motif (PAM) sequence NGG /NRG (for example, as discussed elsewhere, a suitable PAM is 5'-NRG or 5'-NNGRR for SpCas9 or SaCas9 enzymes (or derived enzymes), respectively) following the 20 nucleotides of the target sequence. CRISPR activity through Cas9 for site-specific DNA recognition and cleavage is defined by the guide sequence, the tracr sequence that hybridizes in part to the guide sequence and the PAM sequence. More aspects of the CRISPR system are described in Karginov and Hannon, The CRISPR system: small RNA-guided defense in bacteria and archae, Mole Cell 2010, January 15; 37(1): 7.

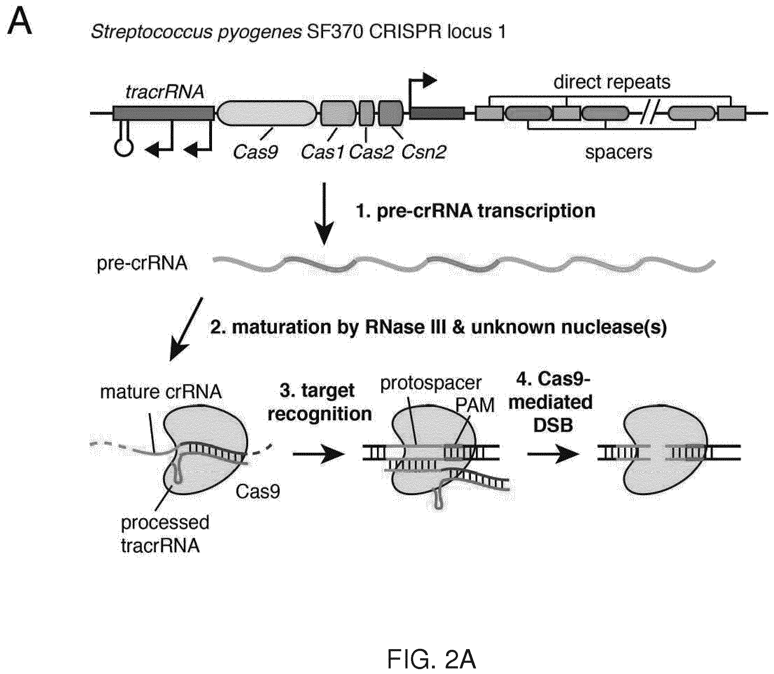

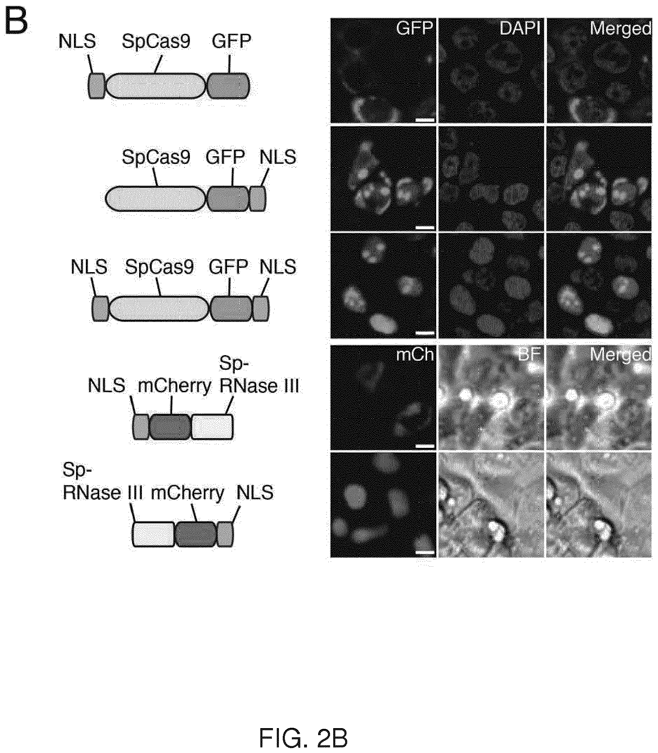

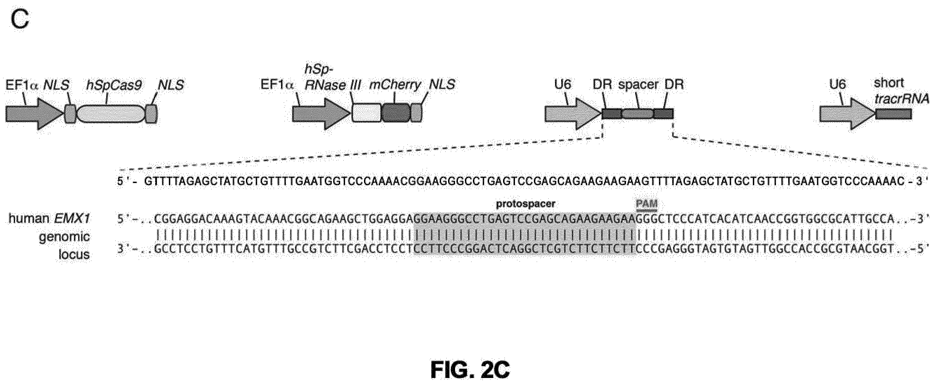

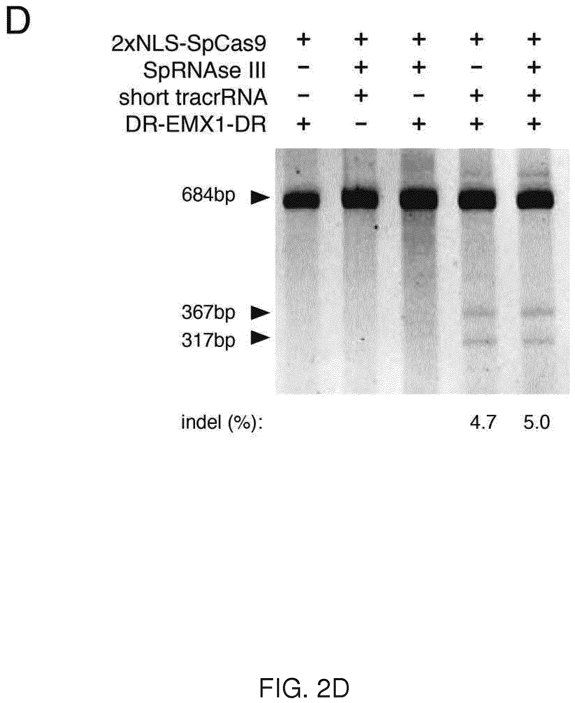

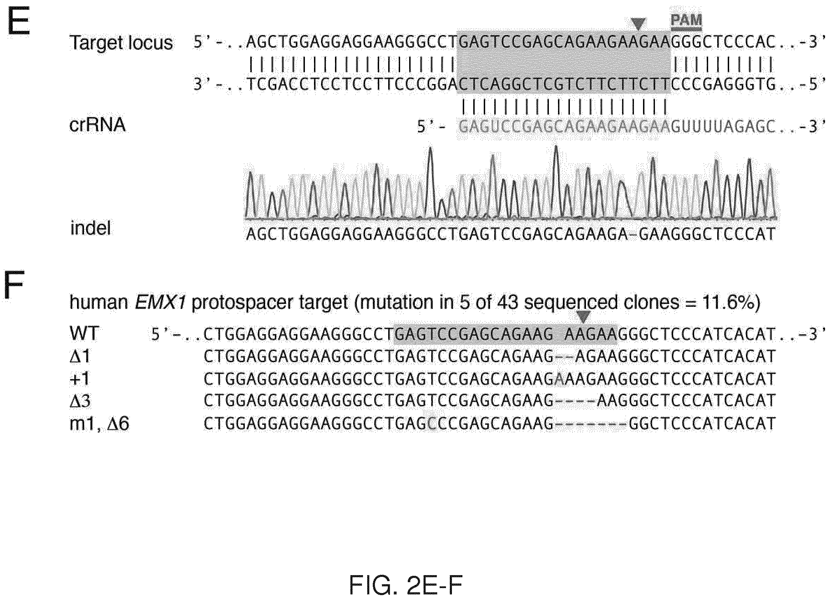

The type II CRISPR locus from Streptooccus pyogenes SF370 contains a cluster of four genes Cas9, Cas1, Cas2, and Csn1, as well as two non-coding RNA elements, tracrRNA and a characteristic array of repetitive sequences (direct repeats) interspaced by short stretches of non-repetitive sequences (spacers, about 30bp each). In this system, targeted DNA double-strand break (DSB) is generated in four sequential steps (Figure 2A). First, two non-coding RNAs, the pre-crRNA array and tracrRNA, are transcribed from the CRISPR locus. Second, tracrRNA hybridizes to the direct repeats of pre-crRNA, which is then processed into mature crRNAs containing individual spacer sequences. Third, the mature crRNA:tracrRNA complex directs Cas9 to the DNA target consisting of the protospacer and the corresponding PAM via heteroduplex formation between the spacer region of the crRNA and the protospacer DNA. Finally, Cas9 mediates cleavage of target DNA upstream of PAM to create a DSB within the protospacer (Figure 2A). Figure 2B demonstrates the nuclear localization of the codon optimized Cas9. To promote precise transcriptional initiation, the RNA polymerase III-based U6 promoter was selected to drive the expression of tracrRNA (Figure 2C). Similarly, a U6 promoter-based construct was developed to express a pre-crRNA array consisting of a single spacer flanked by two direct repeats (DRs, also encompassed by the term "tracr-mate sequences"; Figure 2C). The initial spacer was designed to target a 33-base-pair (bp) target site (30-bp protospacer plus a 3-bp CRISPR motif (PAM) sequence satisfying the NGG recognition motif of Cas9) in the human EMX1 locus (Figure 2C), a key gene in the development of the cerebral cortex.

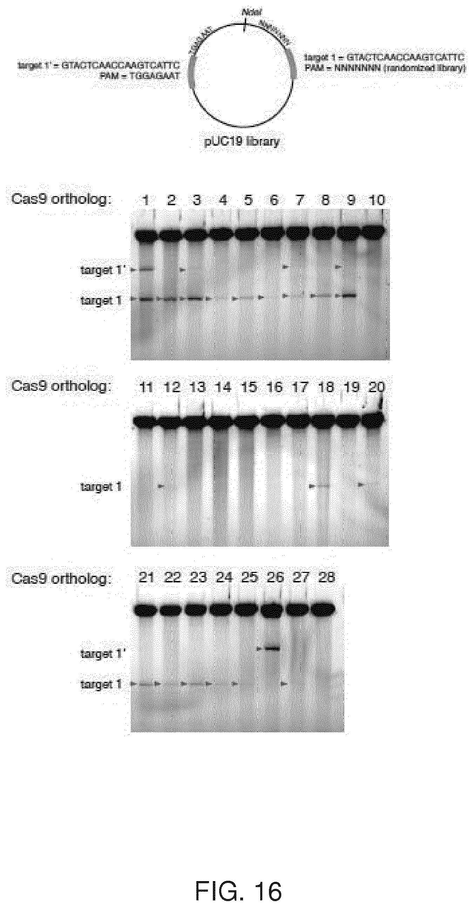

Figure 16 shows Cas9 orthologs and respective sgRNAs are used to cleave two candidate targets present in a pUC19-based library. Target 1 is followed by a randomized PAM containing 7 degenerate bases (5'-NNNNNNN-3'), and target 1', which contains the same target sequence as target 1, is followed by a fixed PAM (5'-TGGAGAAT-3'). The sgRNA of each Cas9 ortholog contains the guide sequence against target 1 or target 1'. Gel images show successful cleavage by 20 Cas9 orthologs, indicating that these sgRNA designs are functional with their respective Cas9 enzymes.

In some embodiments, direct repeats or tracr mate sequences are either downloaded from the CRISPRs database or identified in silico by searching for repetitive motifs that are 1. found in a 2kb window of genomic sequence flanking the type II CRISPR locus, 2. span from 20 to 50 bp, and 3. interspaced by 20 to 50 bp. In some embodiments, 2 of these criteria may be used, for instance 1 and 2, 2 and 3, or 1 and 3. In some embodiments, all 3 criteria may be used.

In some embodiments candidate tracrRNA are subsequently predicted by 1. sequence homology to direct repeats (motif search in Geneious with up to 18-bp mismatches), 2. presence of a predicted Rho-independent transcriptional terminator in direction of transcription, and 3. stable hairpin secondary structure between tracrRNA and direct repeat. In some embodiments, 2 of these criteria may be used, for instance 1 and 2, 2 and 3, or 1 and 3. In some embodiments, all 3 criteria may be used. In some embodiments, chimeric synthetic guide RNAs (sgRNAs) designs incorporate at least 8 bp of duplex structure between the direct repeat and tracrRNA.

Several aspects of the CRISPR system can be further improved to increase the efficiency and versatility of CRISPR targeting. Optimal Cas9 activity may depend on the availability of free Mg2+ at levels higher than that present in the mammalian nucleus (see e.g. Jinek et al., 2012, Science, 337:816), and the preference for an NGG/NRG motif immediately downstream of the protospacer restricts the ability to target on average every 12-bp in the human genome.

Typically, in the context of an endogenous CRISPR system, formation of a CRISPR complex (comprising a guide sequence hybridized to a target sequence and complexed with one or more Cas proteins) results in cleavage of one or both strands in or near (e.g. within 1, 2, 3, 4, 5, 6, 7, 8, 9, 10, 20, 50, or more base pairs from) the target sequence. Without wishing to be bound by theory, the tracr sequence, which may comprise or consist of all or a portion of a wild-type tracr sequence (e.g. about or more than about 20, 26, 32, 45, 48, 54, 63, 67, 85, or more nucleotides of a wild-type tracr sequence), may also form part of a CRISPR complex, such as by hybridization along at least a portion of the tracr sequence to all or a portion of a tracr mate sequence that is operably linked to the guide sequence. In some embodiments, one or more vectors driving expression of one or more elements of a CRISPR system are introduced into a host cell such that expression of the elements of the CRISPR system direct formation of a CRISPR complex at one or more target sites. For example, a Cas enzyme, a guide sequence linked to a tracr-mate sequence, and a tracr sequence could each be operably linked to separate regulatory elements on separate vectors. Alternatively, two or more of the elements expressed from the same or different regulatory elements, may be combined in a single vector, with one or more additional vectors providing any components of the CRISPR system not included in the first vector. CRISPR system elements that are combined in a single vector may be arranged in any suitable orientation, such as one element located 5' with respect to ("upstream" of) or 3' with respect to ("downstream" of) a second element. The coding sequence of one element may be located on the same or opposite strand of the coding sequence of a second element, and oriented in the same or opposite direction. In some embodiments, a single promoter drives expression of a transcript encoding a CRISPR enzyme and one or more of the guide sequence, tracr mate sequence (optionally operably linked to the guide sequence), and a tracr sequence embedded within one or more intron sequences (e.g. each in a different intron, two or more in at least one intron, or all in a single intron). In some embodiments, the CRISPR enzyme, guide sequence, tracr mate sequence, and tracr sequence are operably linked to and expressed from the same promoter.

In some embodiments, a vector comprises one or more insertion sites, such as a restriction endonuclease recognition sequence (also referred to as a "cloning site"). In some embodiments, one or more insertion sites (e.g. about or more than about 1, 2, 3, 4, 5, 6, 7, 8, 9, 10, or more insertion sites) are located upstream and/or downstream of one or more sequence elements of one or more vectors. In some embodiments, a vector comprises an insertion site upstream of a tracr mate sequence, and optionally downstream of a regulatory element operably linked to the tracr mate sequence, such that following insertion of a guide sequence into the insertion site and upon expression the guide sequence directs sequence-specific binding of a CRISPR complex to a target sequence in a eukaryotic cell. In some embodiments, a vector comprises two or more insertion sites, each insertion site being located between two tracr mate sequences so as to allow insertion of a guide sequence at each site. In such an arrangement, the two or more guide sequences may comprise two or more copies of a single guide sequence, two or more different guide sequences, or combinations of these. When multiple different guide sequences are used, a single expression construct may be used to target CRISPR activity to multiple different, corresponding target sequences within a cell. For example, a single vector may comprise about or more than about 1, 2, 3, 4, 5, 6, 7, 8, 9, 10, 15, 20, or more guide sequences. In some embodiments, about or more than about 1, 2, 3, 4, 5, 6, 7, 8, 9, 10, or more such guide-sequence-containing vectors may be provided, and optionally delivered to a cell.

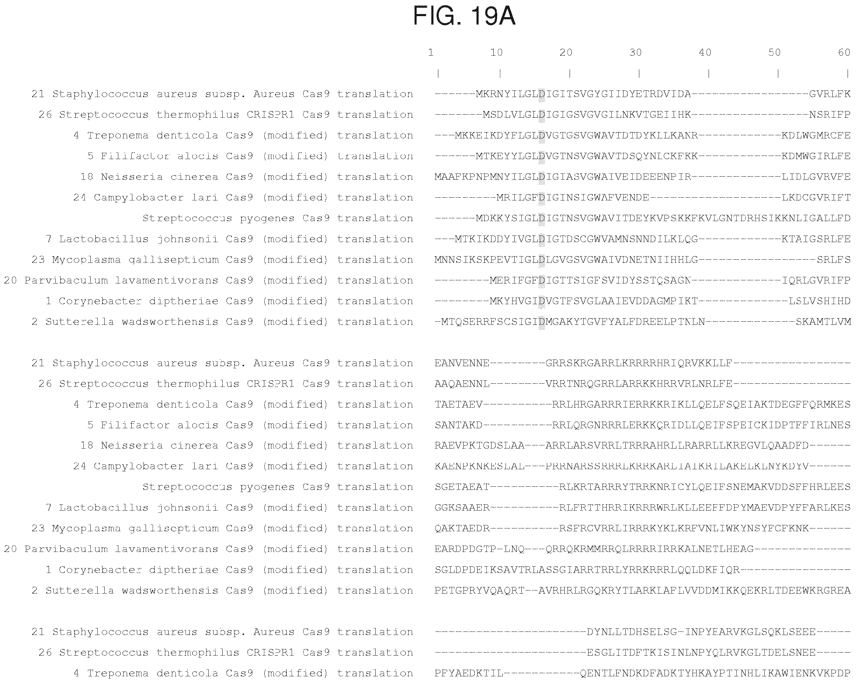

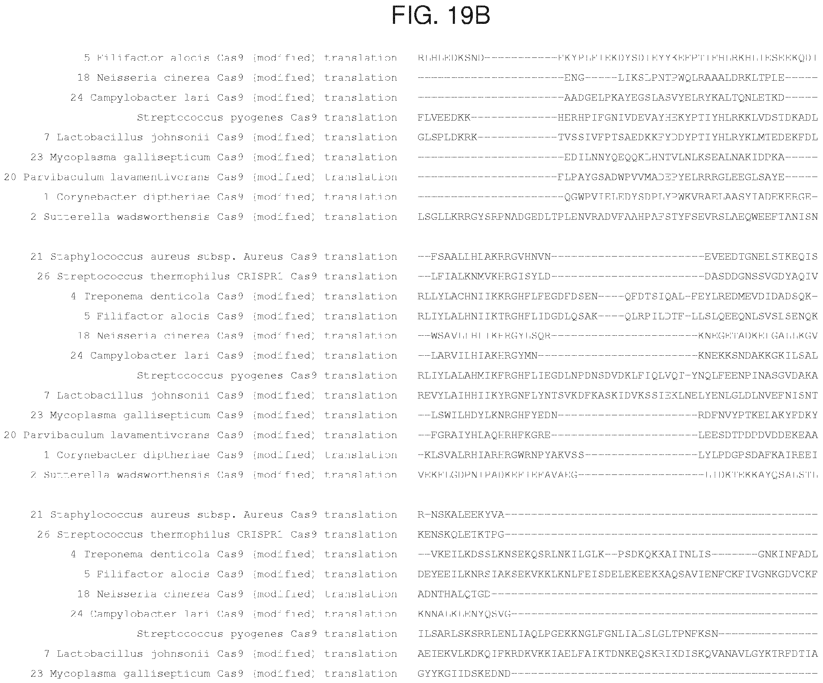

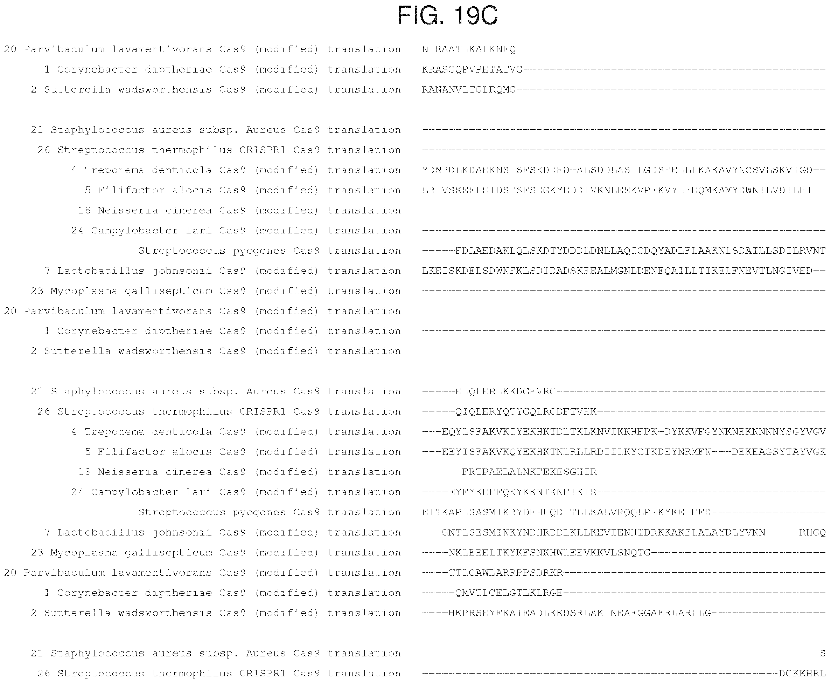

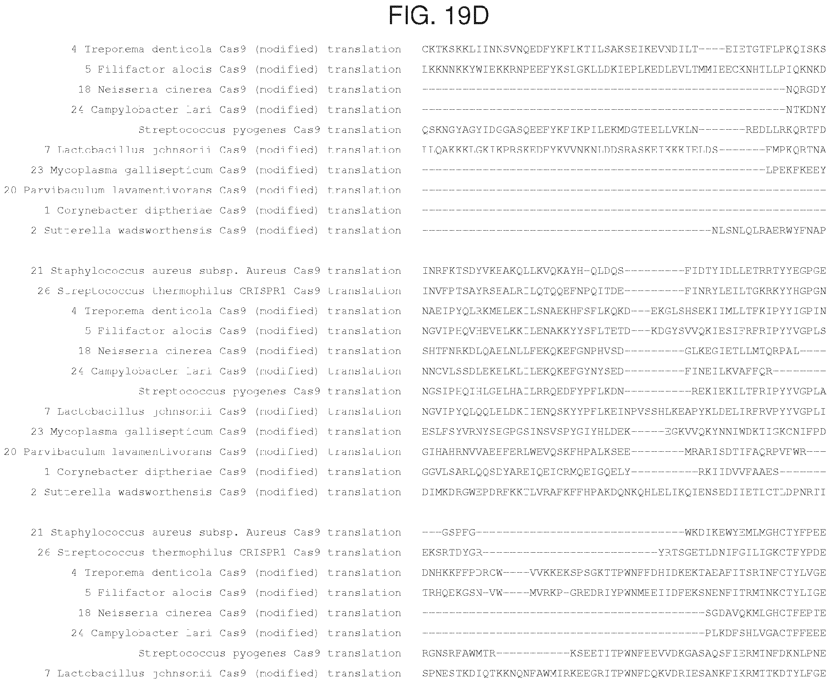

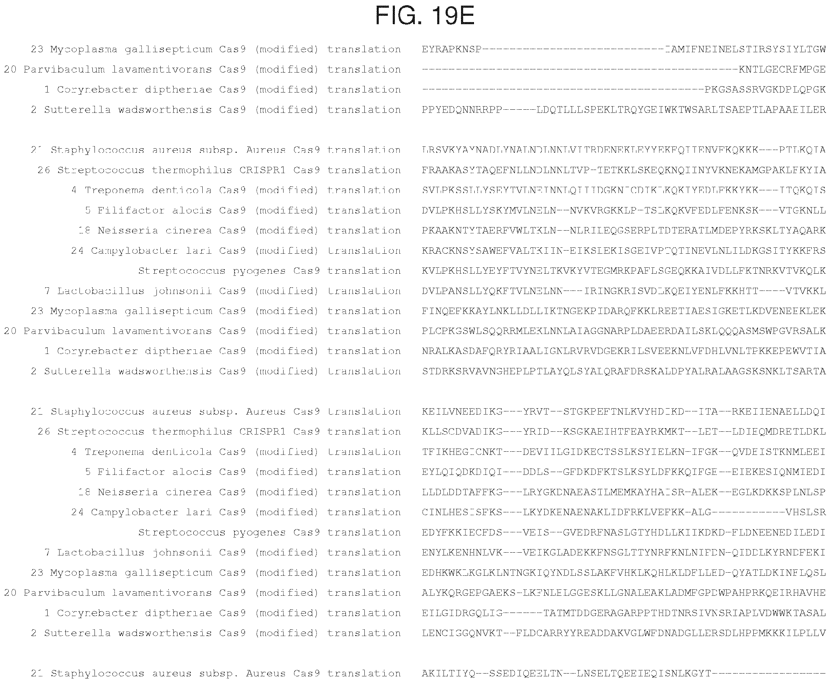

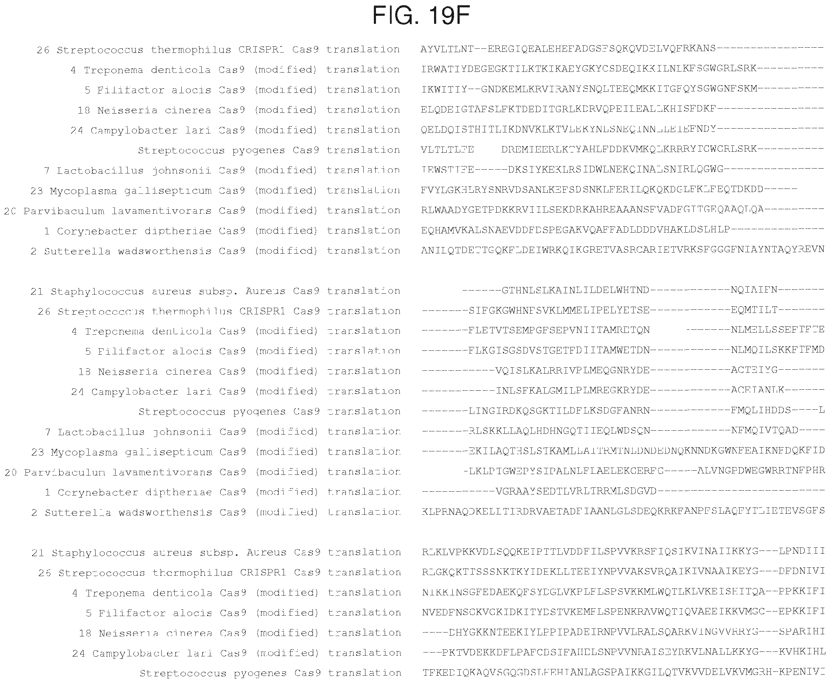

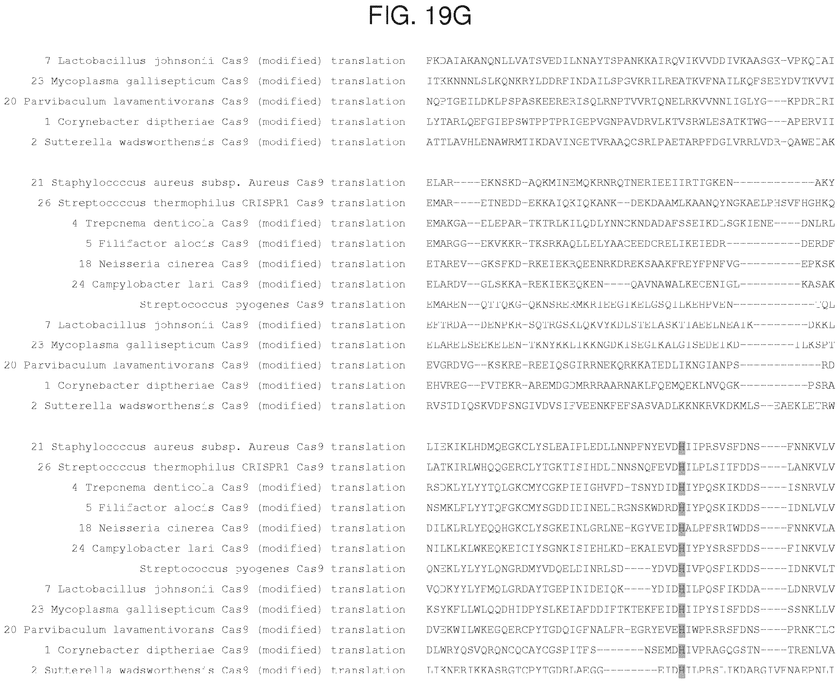

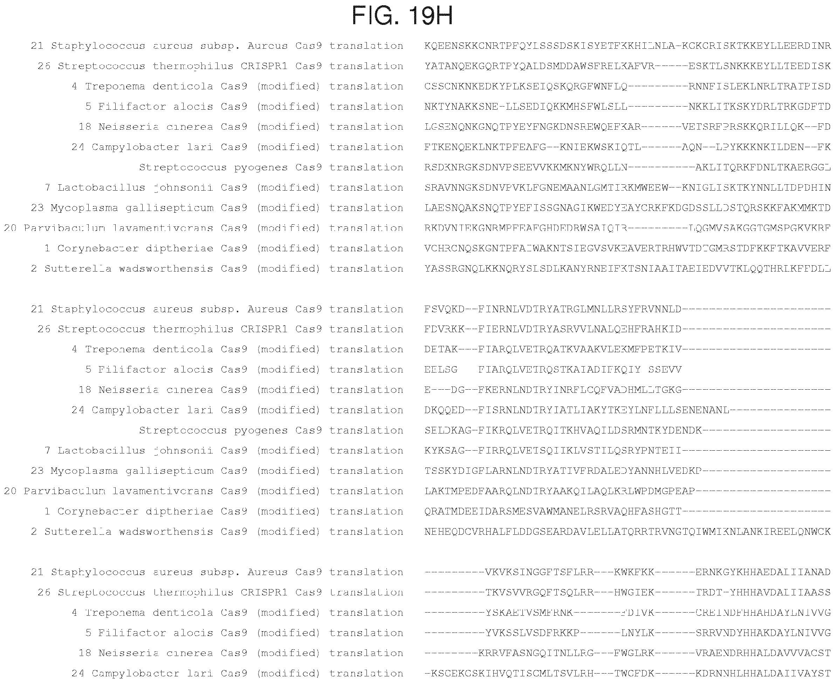

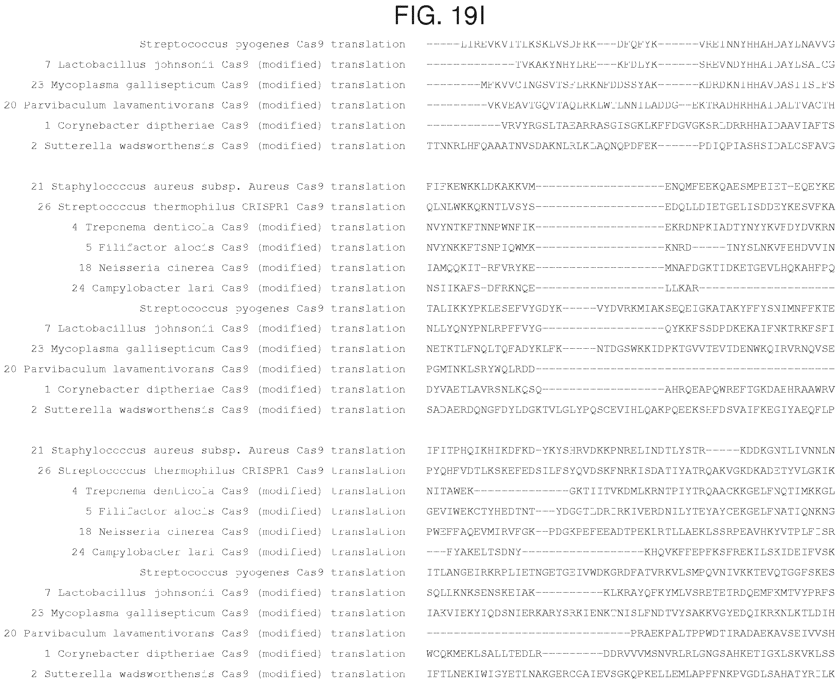

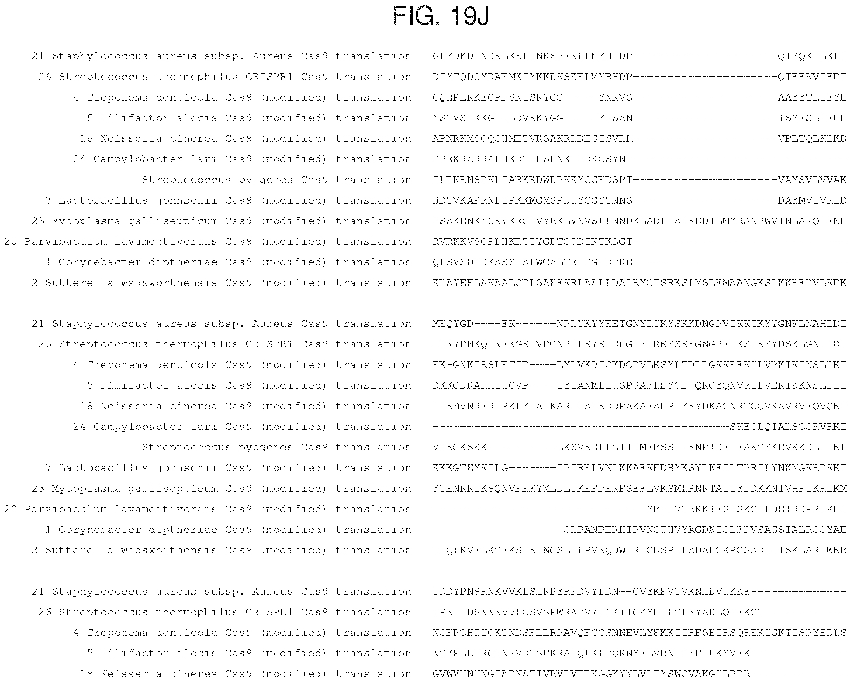

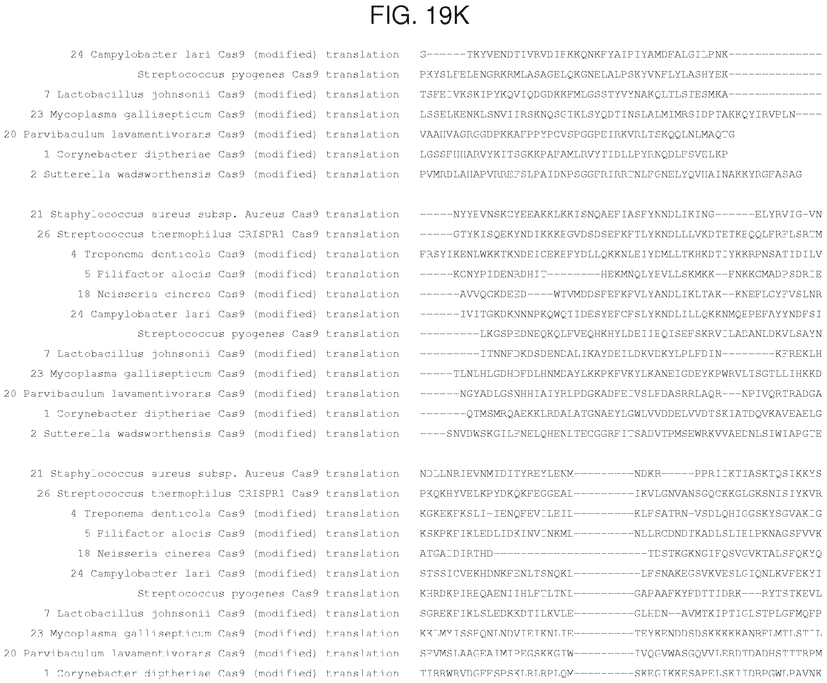

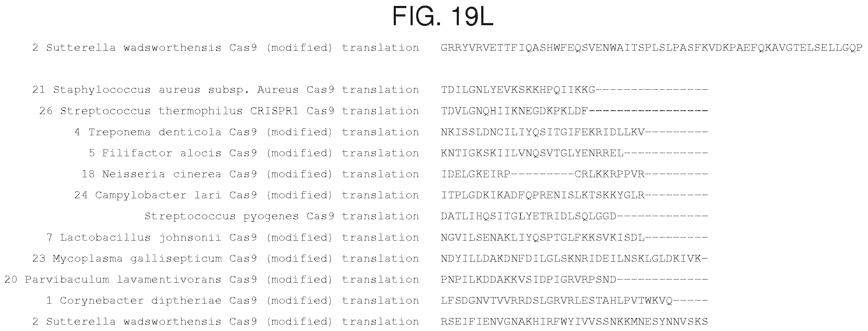

In some embodiments, a vector comprises a regulatory element operably linked to an enzyme-coding sequence encoding a CRISPR enzyme, such as a Cas protein. Non-limiting examples of Cas proteins include Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, homologues thereof, or modified versions thereof. In some embodiments, the unmodified CRISPR enzyme has DNA cleavage activity, such as Cas9. In some embodiments, the CRISPR enzyme directs cleavage of one or both strands at the location of a target sequence, such as within the target sequence and/or within the complement of the target sequence. In some embodiments, the CRISPR enzyme directs cleavage of one or both strands within about 1, 2, 3, 4, 5, 6, 7, 8, 9, 10, 15, 20, 25, 50, 100, 200, 500, or more base pairs from the first or last nucleotide of a target sequence. In some embodiments, a vector encodes a CRISPR enzyme that is mutated with respect to a corresponding wild-type enzyme such that the mutated CRISPR enzyme lacks the ability to cleave one or both strands of a target polynucleotide containing a target sequence. For example, an aspartate-to-alanine substitution (D10A) in the RuvC I catalytic domain of Cas9 from S. pyogenes converts Cas9 from a nuclease that cleaves both strands to a nickase (cleaves a single strand). Other examples of mutations that render Cas9 a nickase include, without limitation, H840A, N854A, and N863A in SpCas9. As discusssed herein, corresponding postions may be conserved in other Cas9s, i.e. in Cas9 orthologs from or derived from other bacterial species, with reference to the position numbering of SpCas9. (Figure 19) shows a multiple sequence alignment of 12 Cas9 orthologs and indicates the conserved catalytic Asp residue in the RuvC I domain and the conserved catalytic His residue in the HNH domain. Mutation of one or the other residue into Ala may convert the Cas9 ortholog into a nickase. Mutation of both residues may convert the Cas9 ortholog into a catalytically null mutant - useful for generic DNA binding. As a further example, two or more catalytic domains of Cas9 (RuvC I, RuvC II, and RuvC III or the HNH domain) may be mutated to produce a mutated Cas9 ortholog substantially lacking all DNA cleavage activity. In some embodiments, a D10A mutation is combined with one or more of H840A, N854A, or N863A mutations to produce a Cas9 enzyme substantially lacking all DNA cleavage activity. In some embodiments, a CRISPR enzyme is considered to substantially lack all DNA cleavage activity when the DNA cleavage activity of the mutated enzyme is less than about 25%, 10%, 5%, 1%, 0.1%, 0.01%, or lower with respect to its non-mutated form.

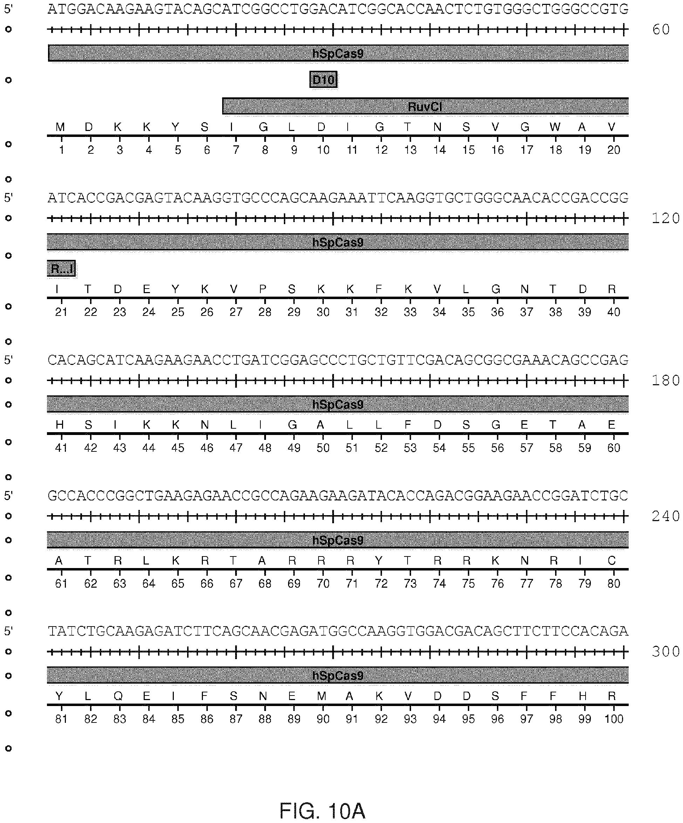

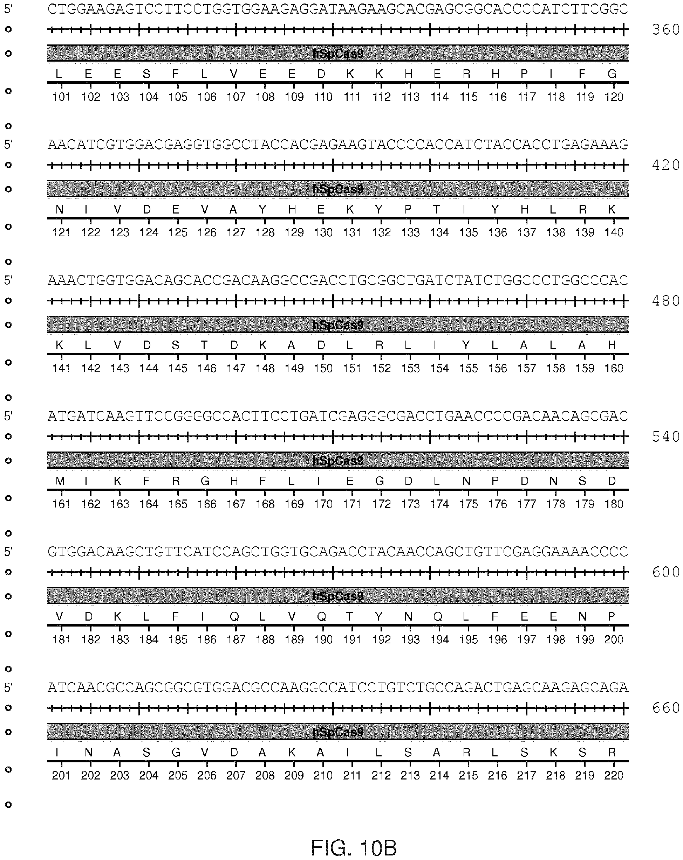

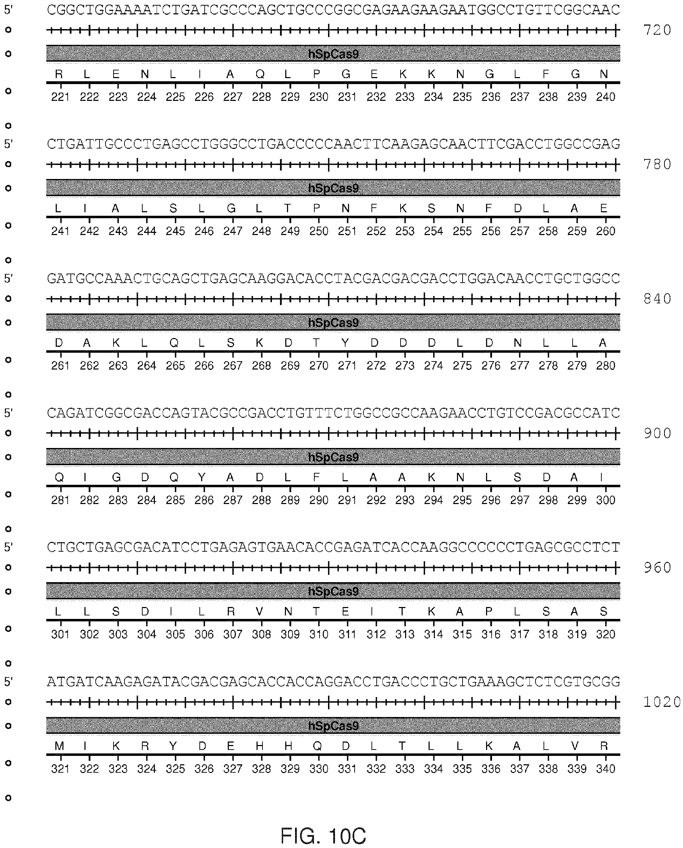

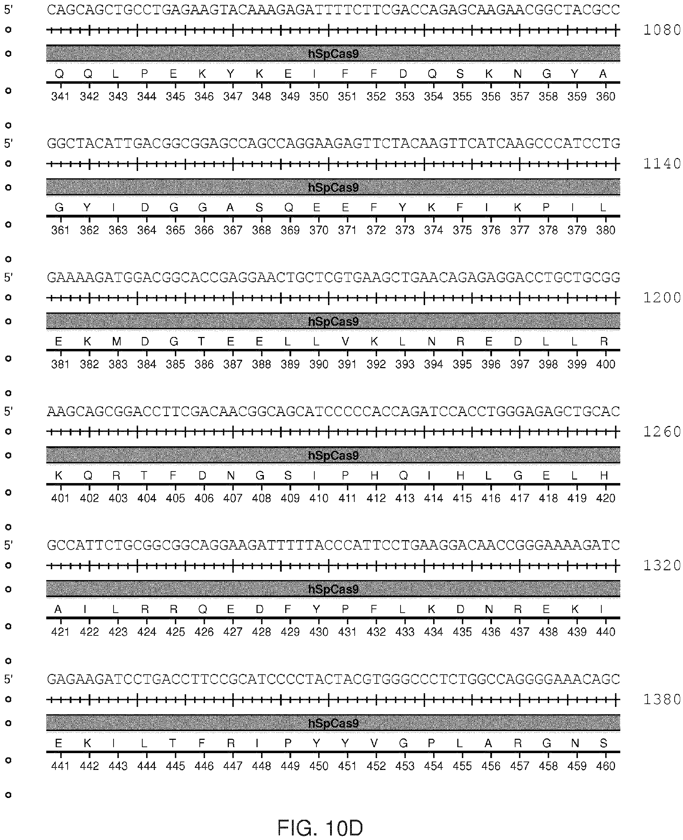

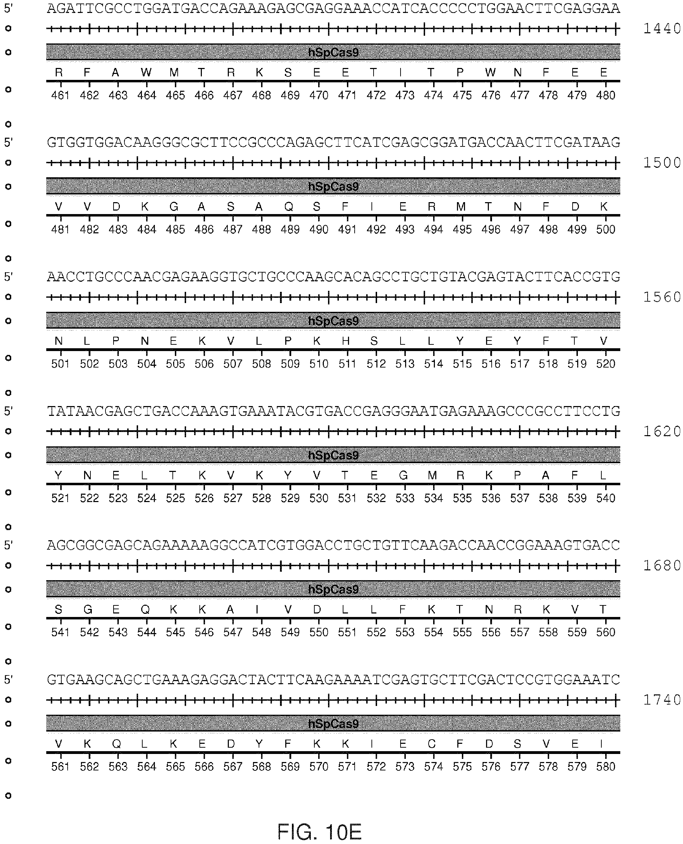

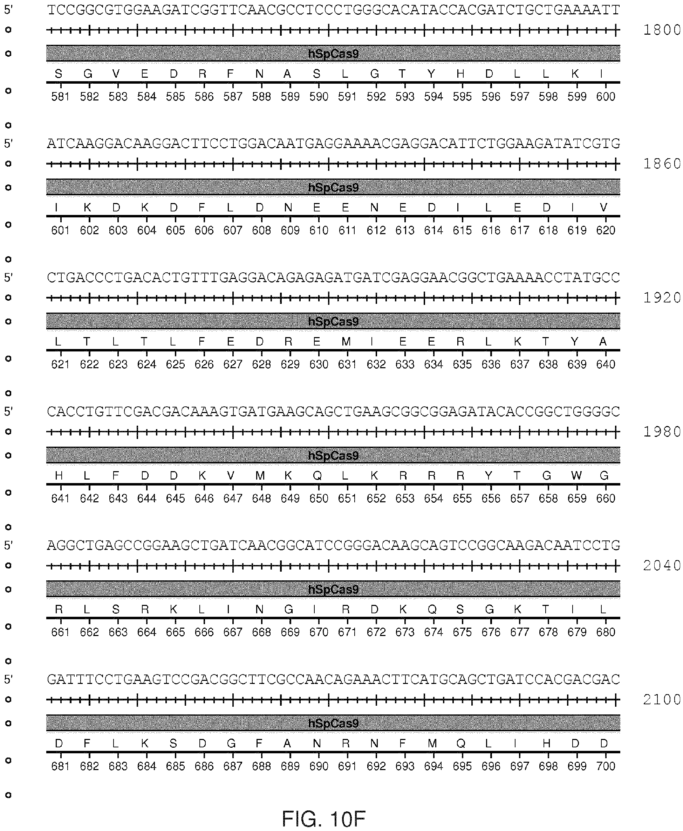

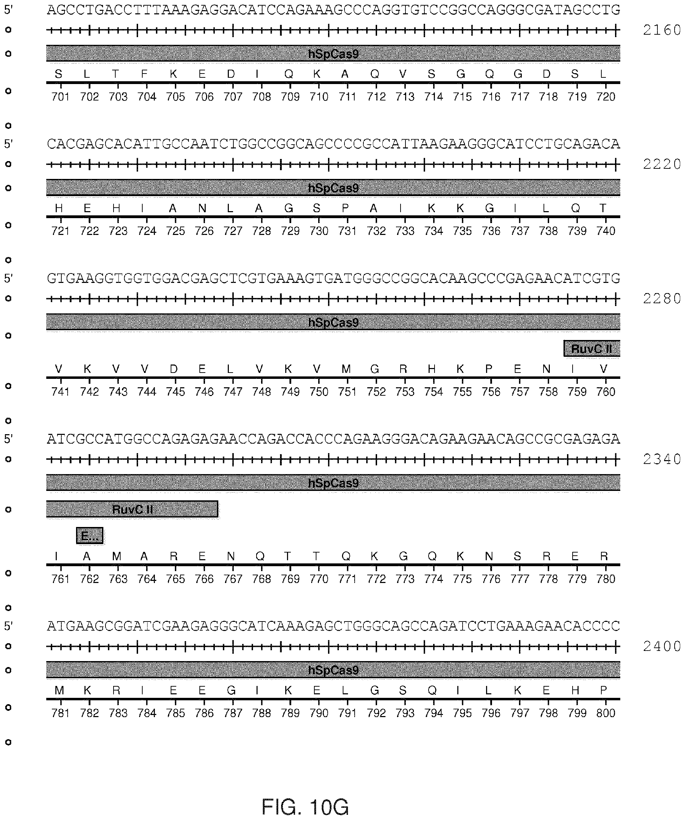

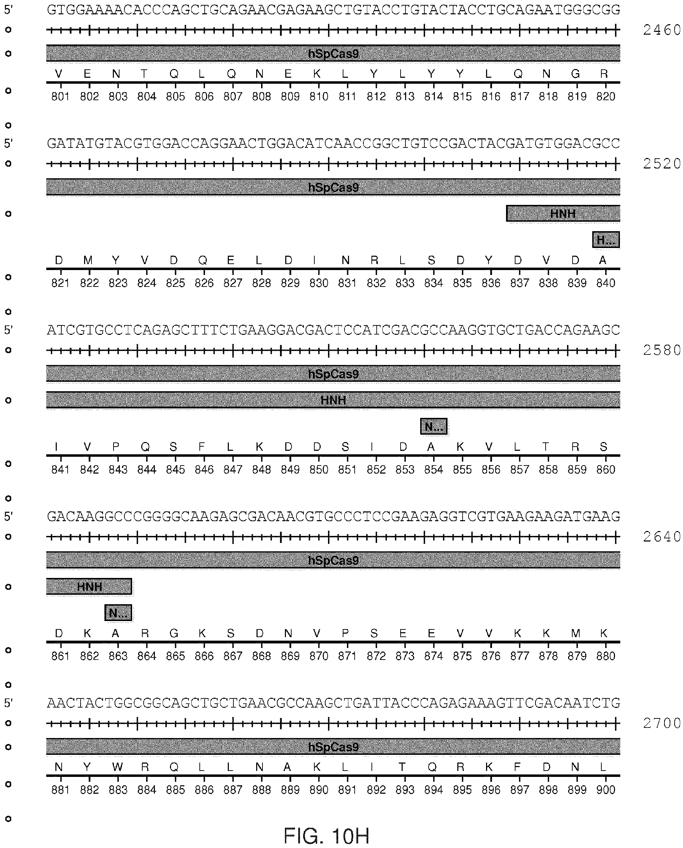

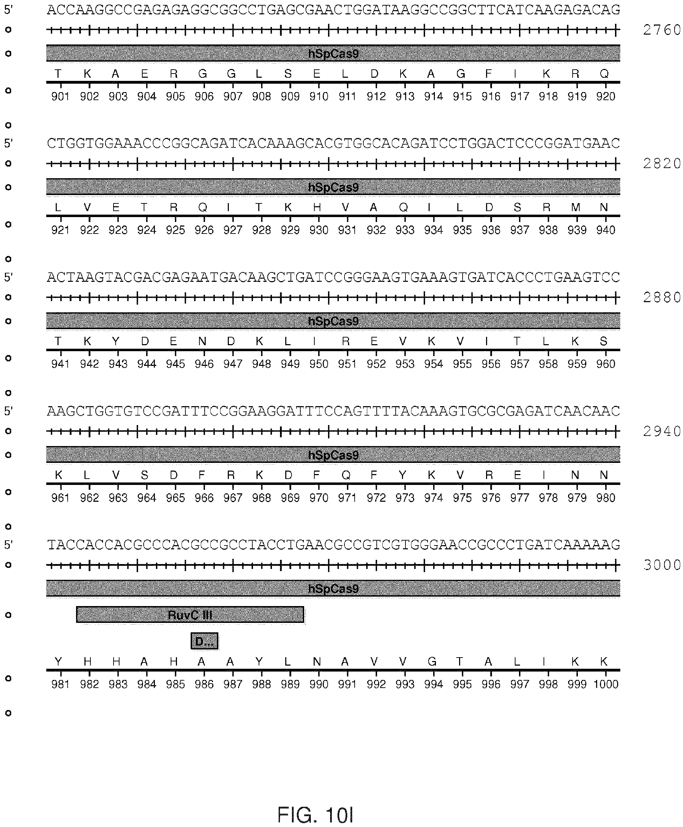

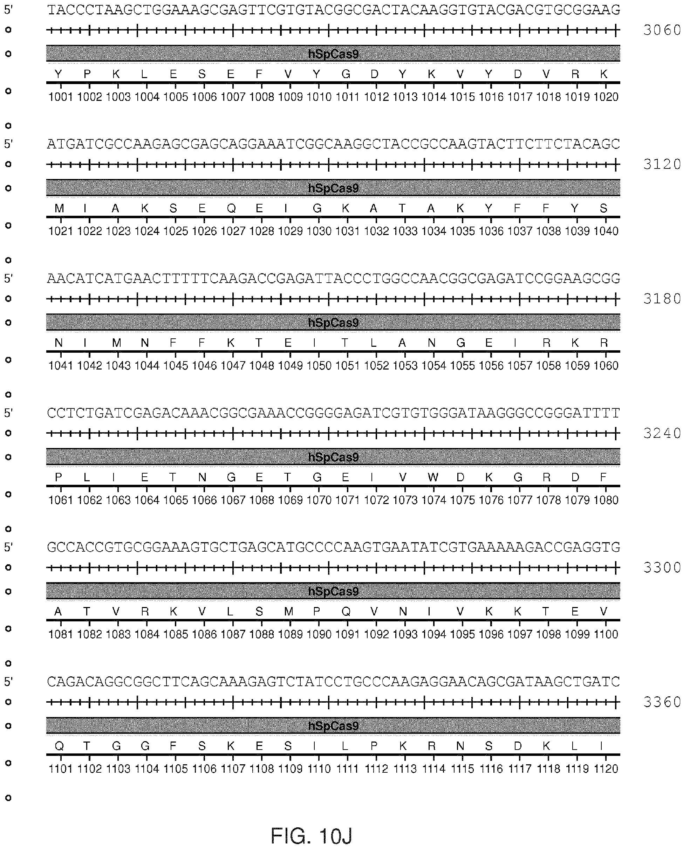

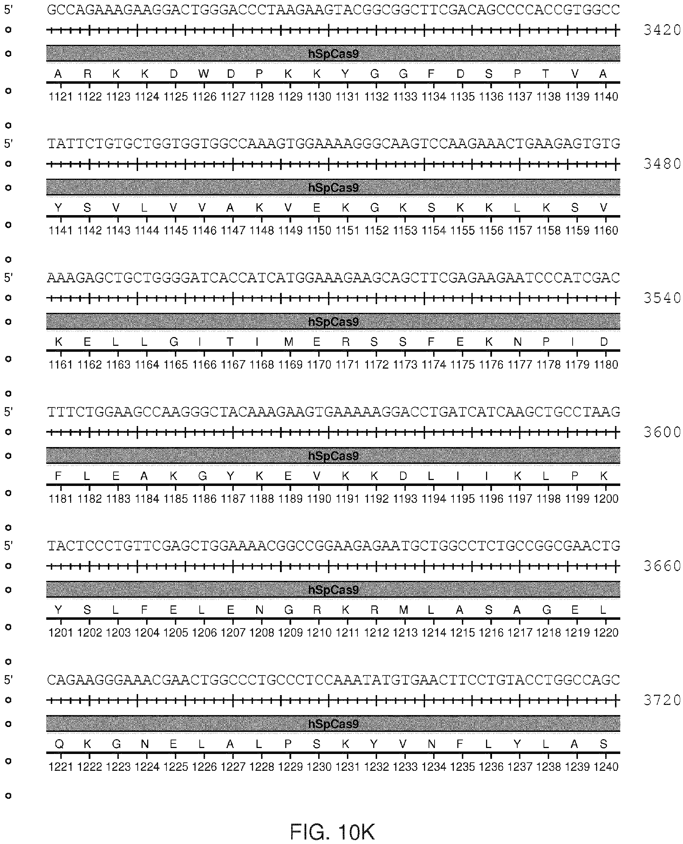

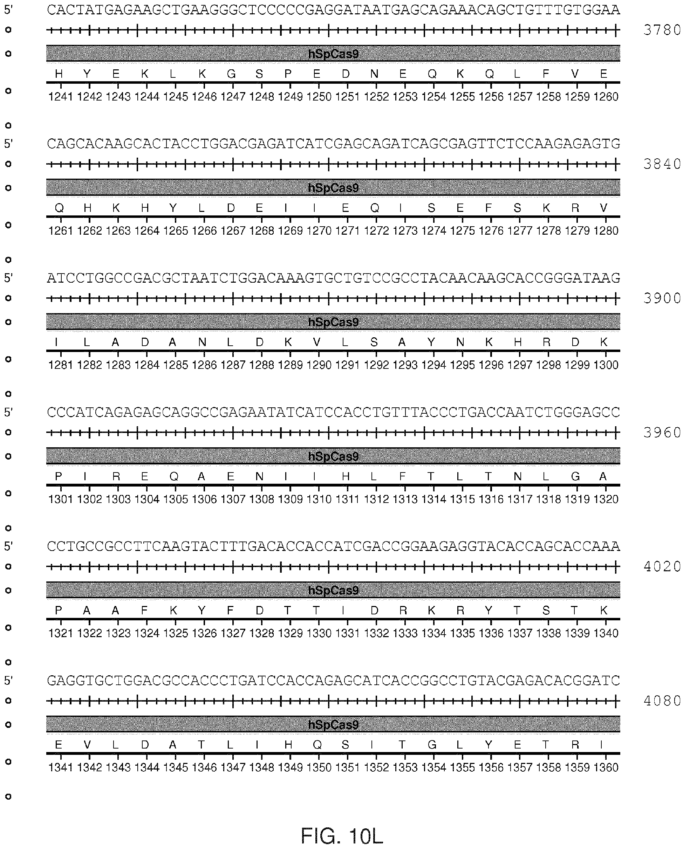

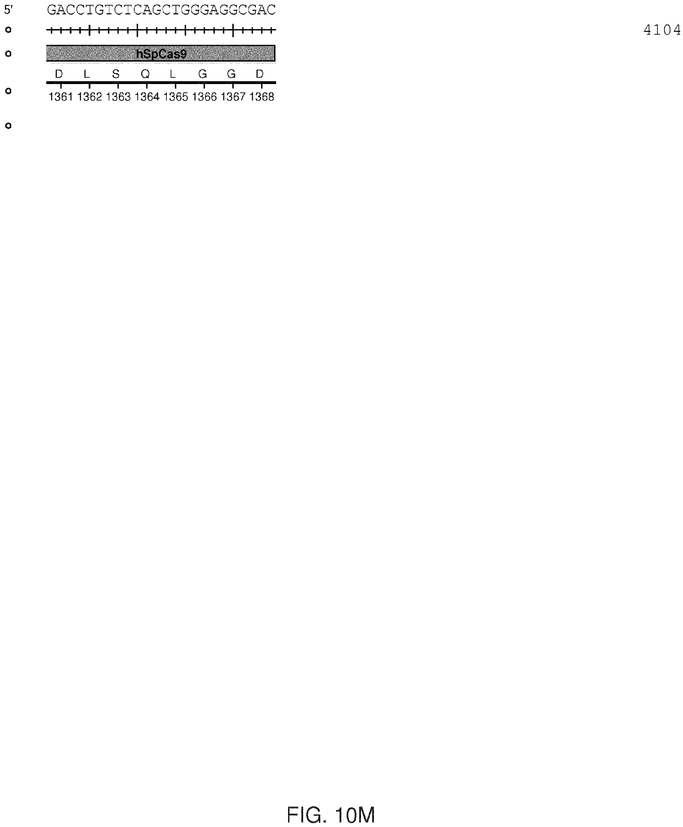

An aspartate-to-alanine substitution (D10A) in the RuvC I catalytic domain of SpCas9 was engineered to convert the nuclease into a nickase (see e.g. Sapranauskas et al., 2011, Nucleic Acis Research, 39: 9275; Gasiunas et al., 2012, Proc. Natl. Acad. Sci. USA, 109:E2579), such that nicked genomic DNA undergoes the high-fidelity homology-directed repair (HDR). Applicants used SURVEYOR assay to confirm that SpCas9n does not generate indels at the EMX1 protospacer target. It was seen that co-expression of EMX1-targeting chimeric crRNA (having the tracrRNA component as well) with SpCas9 produced indels in the target site, whereas co-expression with SpCas9n did not (n=3). Moreover, sequencing of 327 amplicons did not detect any indels induced by SpCas9n. The same locus was selected to test CRISPR-mediated HR by co-transfecting HEK 293FT cells with the chimeric RNA targeting EMX1, hSpCas9 or hSpCas9n, as well as a HR template to introduce a pair of restriction sites (HindIII and NheI) near the protospacer. SpCas9 and SpCas9n indeed catalyzed integration of the HR template into the EMX1 locus. PCR amplification of the target region followed by restriction digest with HindIII revealed cleavage products corresponding to expected fragment sizes, with SpCas9 and SpCas9n mediating similar levels of HR efficiencies. Applicants further verified HR using Sanger sequencing of genomic amplicons and demonstrated the utility of CRISPR for facilitating targeted gene insertion in the mammalian genome. Given the 14-bp (12-bp from the spacer and 2-bp from the PAM) target specificity of the wild type SpCas9, the availability of a nickase can significantly reduce the likelihood of off-target modifications, since single strand breaks are not substrates for the error-prone NHEJ pathway. Figure 10A-M provides a scheme indicating positions of mutations in SpCas9 and Cas9 orthologs typically share the general organization of 3-4 RuvC domains and a HNH domain. The 5' most RuvC domain cleaves the non-complementary strand, and the HNH domain cleaves the complementary strand.

The catalytic residue in the 5' RuvC domain is identified through homology comparison of the Cas9 of interest with other Cas9 orthologs (from S. pyogenes type II CRISPR locus, S. thermophilus CRISPR locus 1, S. thermophilus CRISPR locus 3, and Franciscilla novicida type II CRISPR locus), and the conserved Asp residue is mutated to alanine to convert Cas9 into a complementary-strand nicking enzyme. Similarly, the conserved His and Asn residues in the HNH domains are mutated to Alanine to convert Cas9 into a non-complementary-strand nicking enzyme.

In some embodiments, an enzyme coding sequence encoding a CRISPR enzyme is codon optimized for expression in particular cells, such as eukaryotic cells. The eukaryotic cells may be those of or derived from a particular organism, such as a mammal, including but not limited to human, mouse, rat, rabbit, dog, or non-human mammal including non-human primate. In some embodiments, processes for modifying the germ line genetic identity of human beings and/or processes for modifying the genetic identity of animals which are likely to cause them suffering without any substantial medical benefit to man or animal, and also animals resulting from such processes, may be excluded.

In general, codon optimization refers to a process of modifying a nucleic acid sequence for enhanced expression in the host cells of interest by replacing at least one codon (e.g. about or more than about 1, 2, 3, 4, 5, 10, 15, 20, 25, 50, or more codons) of the native sequence with codons that are more frequently or most frequently used in the genes of that host cell while maintaining the native amino acid sequence. Various species exhibit particular bias for certain codons of a particular amino acid. Codon bias (differences in codon usage between organisms) often correlates with the efficiency of translation of messenger RNA (mRNA), which is in turn believed to be dependent on, among other things, the properties of the codons being translated and the availability of particular transfer RNA (tRNA) molecules. The predominance of selected tRNAs in a cell is generally a reflection of the codons used most frequently in peptide synthesis. Accordingly, genes can be tailored for optimal gene expression in a given organism based on codon optimization. Codon usage tables are readily available, for example, at the "Codon Usage Database" available at www.kazusa.orjp/codon/ (visited Jul. 9, 2002), and these tables can be adapted in a number of ways. See Nakamura, Y., et al. "Codon usage tabulated from the international DNA sequence databases: status for the year 2000" Nucl. Acids Res. 28:292 (2000). Computer algorithms for codon optimizing a particular sequence for expression in a particular host cell are also available, such as Gene Forge (Aptagen; Jacobus, PA), are also available. In some embodiments, one or more codons (e.g. 1, 2, 3, 4, 5, 10, 15, 20, 25, 50, or more, or all codons) in a sequence encoding a CRISPR enzyme correspond to the most frequently used codon for a particular amino acid.

In some embodiments, a vector encodes a CRISPR enzyme comprising one or more nuclear localization sequences (NLSs), such as about or more than about 1, 2, 3, 4, 5, 6, 7, 8, 9, 10, or more NLSs. In some embodiments, the CRISPR enzyme comprises about or more than about 1, 2, 3, 4, 5, 6, 7, 8, 9, 10, or more NLSs at or near the amino-terminus, about or more than about 1, 2, 3, 4, 5, 6, 7, 8, 9, 10, or more NLSs at or near the carboxy-terminus, or a combination of these (e.g. one or more NLS at the amino-terminus and one or more NLS at the carboxy terminus). When more than one NLS is present, each may be selected independently of the others, such that a single NLS may be present in more than one copy and/or in combination with one or more other NLSs present in one or more copies. In a preferred embodiment of the invention, the CRISPR enzyme comprises at most 6 NLSs. In some embodiments, an NLS is considered near the N- or C-terminus when the nearest amino acid of the NLS is within about 1, 2, 3, 4, 5, 10, 15, 20, 25, 30, 40, 50, or more amino acids along the polypeptide chain from the Nor C-terminus. Non-limiting examples of NLSs include an NLS sequence derived from: the NLS of the SV40 virus large T-antigen, having the amino acid sequence PKKKRKV; the NLS from nucleoplasmin (e.g. the nucleoplasmin bipartite NLS with the sequence KRPAATKKAGQAKKKK); the c-myc NLS having the amino acid sequence PAAKRVKLD or RQRRNELKRSP; the hRNPA1 M9 NLS having the sequence NQSSNFGPMKGGNFGGRSSGPYGGGGQYFAKPRNQGGY; the sequence RMRIZFKNKGKDTAELRRRRVEVSVELRKAKKDEQILKRRNV of the IBB domain from importin-alpha; the sequences VSRKRPRP and PPKKARED of the myoma T protein; the sequence POPKKKPL of human p53; the sequence SALIKKKKKMAP of mouse c-abl IV; the sequences DRLRR and PKQKKRK of the influenza virus NS1; the sequence RKLKKKIKKL of the Hepatitis virus delta antigen; the sequence REKKKFLKRR of the mouse Mx1 protein; the sequence KRKGDEVDGVDEVAKKKSKK of the human poly(ADP-ribose) polymerase; and the sequence RKCLQAGMNLEARKTKK of the steroid hormone receptors (human) glucocorticoid.

In general, the one or more NLSs are of sufficient strength to drive accumulation of the CRISPR enzyme in a detectable amount in the nucleus of a eukaryotic cell. In general, strength of nuclear localization activity may derive from the number of NLSs in the CRISPR enzyme, the particular NLS(s) used, or a combination of these factors. Detection of accumulation in the nucleus may be performed by any suitable technique. For example, a detectable marker may be fused to the CRISPR enzyme, such that location within a cell may be visualized, such as in combination with a means for detecting the location of the nucleus (e.g. a stain specific for the nucleus such as DAPI). Cell nuclei may also be isolated from cells, the contents of which may then be analyzed by any suitable process for detecting protein, such as immunohistochemistry, Western blot, or enzyme activity assay. Accumulation in the nucleus may also be determined indirectly, such as by an assay for the effect of CRISPR complex formation (e.g. assay for DNA cleavage or mutation at the target sequence, or assay for altered gene expression activity affected by CRISPR complex formation and/or CRISPR enzyme activity), as compared to a control no exposed to the CRISPR enzyme or complex, or exposed to a CRISPR enzyme lacking the one or more NLSs.

In general, a guide sequence is any polynucleotide sequence having sufficient complementarity with a target polynucleotide sequence to hybridize with the target sequence and direct sequence-specific binding of a CRISPR complex to the target sequence. In some embodiments, the degree of complementarity between a guide sequence and its corresponding target sequence, when optimally aligned using a suitable alignment algorithm, is about or more than about 50%, 60%, 75%, 80%, 85%, 90%, 95%, 97.5%, 99%, or more. Optimal alignment may be determined with the use of any suitable algorithm for aligning sequences, non-limiting example of which include the Smith-Waterman algorithm, the Needleman-Wunsch algorithm, algorithms based on the Burrows-Wheeler Transform (e.g. the Burrows Wheeler Aligner), ClustalW, Clustal X, BLAT, Novoalign (Novocraft Technologies; available at www.novocraft.com), ELAND (Illumina, San Diego, CA), SOAP (available at soap.genomics.org.cn), and Maq (available at maq.sourceforge.net). In some embodiments, a guide sequence is about or more than about 5, 10, 11, 12, 13, 14, 15, 16, 17, 18, 19, 20, 21, 22, 23, 24, 25, 26, 27, 28, 29, 30, 35, 40, 45, 50, 75, or more nucleotides in length. In some embodiments, a guide sequence is less than about 75, 50, 45, 40, 35, 30, 25, 20, 15, 12, or fewer nucleotides in length. The ability of a guide sequence to direct sequence-specific binding of a CRISPR complex to a target sequence may be assessed by any suitable assay. For example, the components of a CRISPR system sufficient to form a CRISPR complex, including the guide sequence to be tested, may be provided to a host cell having the corresponding target sequence, such as by transfection with vectors encoding the components of the CRISPR sequence, followed by an assessment of preferential cleavage within the target sequence, such as by Surveyor assay as described herein. Similarly, cleavage of a target polynucleotide sequence may be evaluated in a test tube by providing the target sequence, components of a CRISPR complex, including the guide sequence to be tested and a control guide sequence different from the test guide sequence, and comparing binding or rate of cleavage at the target sequence between the test and control guide sequence reactions. Other assays are possible, and will occur to those skilled in the art.

Multiplexed Nickase: Aspects of optimization and the teachings of Cas9 detailed in this application may also be used to generate Cas9 nickases. Cas9 nickases may be advantageously used in combination with pairs of guide RNAs to generate DNA double strand breaks with defined overhangs. When two pairs of guide RNAs are used, it is possible to excise an intervening DNA fragment. If an exogenous piece of DNA is cleaved by the two pairs of guide RNAs to generate compatible overhangs with the genomic DNA, then the exogenous DNA fragment may be ligated into the genomic DNA to replace the excised fragment. For example, this may be used to remove trinucleotide repeat expansion in the huntingtin (HTT) gene to treat Huntington's Disease.

Cas9 and its chimeric guide RNA, or combination of tracrRNA and crRNA, can be delivered either as DNA or RNA. Delivery of Cas9 and guide RNA both as RNA (normal or containing base or backbone modifications) molecules can be used to reduce the amount of time that Cas9 protein persist in the cell. This may reduce the level of off-target cleavage activity in the target cell. Since delivery of Cas9 as mRNA takes time to be translated into protein, it might be advantageous to deliver the guide RNA several hours following the delivery of Cas9 mRNA, to maximize the level of guide RNA available for interaction with Cas9 protein.

In situations where guide RNA amount is limiting, it may be desirable to introduce Cas9 as mRNA and guide RNA in the form of a DNA expression cassette with a promoter driving the expression of the guide RNA. This way the amount of guide RNA available will be amplified via transcription.

A variety of delivery systems can be introduced to introduce Cas9 (DNA or RNA) and guide RNA (DNA or RNA) into the host cell. These include the use of liposomes, viral vectors, electroporation, nanoparticles , nanowires (Shalek et al., Nano Letters, 2012), exosomes. Molecular trojan horses liposomes (Pardridge et al., Cold Spring Harb Protoc; 2010; doi:10.1101/pdb.prot5407) may be used to deliver Cas9 and guide RNA across the blood brain barrier.

Discussion of guide RNAs for orthologs: In some embodiments, a guide sequence is selected to reduce the degree secondary structure within the guide RNA. In some embodiments, about or less than about 75%, 50%, 40%, 30%, 25%, 20%, 15%, 10%, 5%, 1%, or fewer of the nucleotides of the guide RNA participate in self-complementary base pairing when optimally folded. Optimal folding may be determined by any suitable polynucleotide folding algorithm. Some programs are based on calculating the minimal Gibbs free energy. An example of one such algorithm is mFold, as described by Zuker and Stiegler (Nucleic Acids Res. 9 (1981), 133-148). Another example folding algorithm is the online webserver RNAfold, developed at Institute for Theoretical Chemistry at the University of Vienna, using the centroid structure prediction algorithm (see e.g. A.R. Gruber et al., 2008, Cell 106(1): 23-24; and PA Carr and GM Church, 2009, Nature Biotechnology 27(12): 1151-62). A method of optimizing the guide RNA of a Cas9 ortholog comprises breaking up polyU tracts in the guide RNA. PolyU tracts that may be broken up may comprise a series of 4, 5, 6, 7, 8, 9 or 10 Us.

In general, a tracr mate sequence includes any sequence that has sufficient complementarity with a tracr sequence to promote one or more of: (1) excision of a guide sequence flanked by tracr mate sequences in a cell containing the corresponding tracr sequence; and (2) formation of a CRISPR complex at a target sequence, wherein the CRISPR complex comprises the tracr mate sequence hybridized to the tracr sequence. In general, degree of complementarity is with reference to the optimal alignment of the tracr mate sequence and tracr sequence, along the length of the shorter of the two sequences. Optimal alignment may be determined by any suitable alignment algorithm, and may further account for secondary structures, such as self-complementarity within either the tracr sequence or tracr mate sequence. In some embodiments, the degree of complementarity between the tracr sequence and tracr mate sequence along the length of the shorter of the two when optimally aligned is about or more than about 25%, 30%, 40%, 50%, 60%, 70%, 80%, 90%, 95%, 97.5%, 99%, or higher. In some embodiments, the tracr sequence is about or more than about 5, 6, 7, 8, 9, 10, 11, 12, 13, 14, 15, 16, 17, 18, 19, 20, 25, 30, 40, 50, or more nucleotides in length. In some embodiments, the tracr sequence and tracr mate sequence are contained within a single transcript, such that hybridization between the two produces a transcript having a secondary structure, such as a hairpin. In an embodiment of the invention, the transcript or transcribed polynucleotide sequence has at least two or more hairpins. In preferred embodiments, the transcript has two, three, four or five hairpins. In a further embodiment of the invention, the transcript has at most five hairpins. In a hairpin structure the portion of the sequence 5' of the final "N" and upstream of the loop corresponds to the tracr mate sequence, and the portion of the sequence 3' of the loop corresponds to the tracr sequence. Further non-limiting examples of single polynucleotides comprising a guide sequence, a tracr mate sequence, and a tracr sequence are as follows (listed 5' to 3'), where "N" represents a base of a guide sequence, the first block of lower case letters represent the tracr mate sequence, and the second block of lower case letters represent the tracr sequence, and the final poly-T sequence represents the transcription terminator: (1) NNNNNNNNNNNNNNNNNNNNgtttttgtactctcaagatttaGAAAtaaatcttgcagaagctacaaagataaggctt catgccgaaatcaacaccctgtcattttatggcagggtgttttcgttatttaaTTTTTT; (2) NNNNNNNNNNNNNNNNNNNNgtttttgtactctcaGAAAtgcagaagctacaaagataaggcttcatgccgaaatca acaccctgtcattttatggcagggtgttttcgttatttaaTTTTTT; (3) NNNNNNNNNNNNNNNNNNNNgtttttgtactctcaGAAAtgcagaagctacaaagataaggcttcatgccgaaatca acaccctgtcattttatggcagggtgtTTTTTT; (4) NNNNNNNNNNNNNNNNNNNNgttttagagctaGAAAtagcaagttaaaataaggctagtccgttatcaacttgaaaa agtggcaccgagtcggtgcTTTTTT; (5) NNNNNNNNNNNNNNNNNNNNgttttagagctaGAAATAGcaagttaaaataaggctagtccgttatcaacttgaa aaagtgTTTTTTT; and (6) NNNNNNNNNNNNNNNNNNNNgttttagagctagAAATAGcaagttaaaataaggctagtccgttatcaTTTTT TTT. In some embodiments, sequences (1) to (3) are used in combination with Cas9 from S. thermophilus CRISPR1. In some embodiments, sequences (4) to (6) are used in combination with Cas9 from S. pyogenes. In some embodiments, the tracr sequence is a separate transcript from a transcript comprising the tracr mate sequence.

Discussion of tracr mates for orthologs: In some embodiments, a recombination template is also provided. A recombination template may be a component of another vector as described herein, contained in a separate vector, or provided as a separate polynucleotide. In some embodiments, a recombination template is designed to serve as a template in homologous recombination, such as within or near a target sequence nicked or cleaved by a CRISPR enzyme as a part of a CRISPR complex. A template polynucleotide may be of any suitable length, such as about or more than about 10, 15, 20, 25, 50, 75, 100, 150, 200, 500, 1000, or more nucleotides in length. In some embodiments, the template polynucleotide is complementary to a portion of a polynucleotide comprising the target sequence. When optimally aligned, a template polynucleotide might overlap with one or more nucleotides of a target sequences (e.g. about or more than about 1, 5, 10, 15, 20, or more nucleotides). In some embodiments, when a template sequence and a polynucleotide comprising a target sequence are optimally aligned, the nearest nucleotide of the template polynucleotide is within about 1, 5, 10, 15, 20, 25, 50, 75, 100, 200, 300, 400, 500, 1000, 5000, 10000, or more nucleotides from the target sequence.

In some embodiments, the CRISPR enzyme is part of a fusion protein comprising one or more heterologous protein domains (e.g. about or more than about 1, 2, 3, 4, 5, 6, 7, 8, 9, 10, or more domains in addition to the CRISPR enzyme). A CRISPR enzyme fusion protein may comprise any additional protein sequence, and optionally a linker sequence between any two domains. Examples of protein domains that may be fused to a CRISPR enzyme include, without limitation, epitope tags, reporter gene sequences, and protein domains having one or more of the following activities: methylase activity, demethylase activity, transcription activation activity, transcription repression activity, transcription release factor activity, histone modification activity, RNA cleavage activity and nucleic acid binding activity. Non-limiting examples of epitope tags include histidine (His) tags, V5 tags, FLAG tags, influenza hemagglutinin (HA) tags, Myc tags, VSV-G tags, and thioredoxin (Trx) tags. Examples of reporter genes include, but are not limited to, glutathione-S-transferase (GST), horseradish peroxidase (HRP), chloramphenicol acetyltransferase (CAT) beta-galactosidase, beta-glucuronidase, luciferase, green fluorescent protein (GFP), HcRed, DsRed, cyan fluorescent protein (CFP), yellow fluorescent protein (YFP), and auto fluorescent proteins including blue fluorescent protein (BFP). A CRISPR enzyme may be fused to a gene sequence encoding a protein or a fragment of a protein that bind DNA molecules or bind other cellular molecules, including but not limited to maltose binding protein (MBP), S-tag, Lex A DNA binding domain (DBD) fusions, GAL4 DNA binding domain fusions, and herpes simplex virus (HSV) BP16 protein fusions. Additional domains that may form part of a fusion protein comprising a CRISPR enzyme are described in US20110059502. In some embodiments, a tagged CRISPR enzyme is used to identify the location of a target sequence.

In some embodiments, a CRISPR enzyme may form a component of an inducible system. The inducible nature of the system would allow for spatiotemporal control of gene editing or gene expression using a form of energy. The form of energy may include but is not limited to electromagnetic radiation, sound energy, chemical energy and thermal energy. Examples of inducible system include tetracycline inducible promoters (Tet-On or Tet-Off), small molecule two-hybrid transcription activations systems (FKBP, ABA, etc), or light inducible systems (Phytochrome, LOV domains, or cryptochorome). In one embodiment, the CRISPR enzyme may be a part of a Light Inducible Transcriptional Effector (LITE) to direct changes in transcriptional activity in a sequence-specific manner. The components of a light may include a CRISPR enzyme, a light-responsive cytochrome heterodimer (e.g. from Arabidopsis thaliana), and a transcriptional activation/repression domain. Further examples of inducible DNA binding proteins and methods for their use are provided in US 61/736465 and US 61/721,283.

In some aspects, the invention provides methods comprising delivering one or more polynucleotides, such as or one or more vectors as described herein, one or more transcripts thereof, and/or one or proteins transcribed therefrom, to a host cell. In some aspects, the invention further provides cells produced by such methods, and animals comprising or produced from such cells. In some embodiments, a CRISPR enzyme in combination with (and optionally complexed with) a guide sequence is delivered to a cell. Conventional viral and non-viral based gene transfer methods can be used to introduce nucleic acids in mammalian cells or target tissues. Such methods can be used to administer nucleic acids encoding components of a CRISPR system to cells in culture, or in a host organism. Non-viral vector delivery systems include DNA plasmids, RNA (e.g. a transcript of a vector described herein), naked nucleic acid, and nucleic acid complexed with a delivery vehicle, such as a liposome. Viral vector delivery systems include DNA and RNA viruses, which have either episomal or integrated genomes after delivery to the cell. For a review of gene therapy procedures, see Anderson, Science 256:808-813 (1992); Nabel & Felgner, TIBTECH 11:211-217 (1993); Mitani & Caskey, TIBTECH 11:162-166 (1993); Dillon, TIBTECH 11:167-175 (1993); Miller, Nature 357:455-460 (1992); Van Brunt, Biotechnology 6(10):1149-1154 (1988); Vigne, Restorative Neurology and Neuroscience 8:35-36 (1995); Kremer & Perricaudet, British Medical Bulletin 51(1):31-44 (1995); Haddada et al., in Current Topics in Microbiology and Immunology Doerfler and Böhm (eds) (1995); and Yu et al., Gene Therapy 1:13-26 (1994).

Methods of non-viral delivery of nucleic acids include lipofection, microinjection, biolistics, virosomes, liposomes, immunoliposomes, polycation or lipid:nucleic acid conjugates, naked DNA, artificial virions, and agent-enhanced uptake of DNA. Lipofection is described in e.g., U.S. Pat. Nos. 5,049,386, 4,946,787; and 4,897,355) and lipofection reagents are sold commercially (e.g., Transfectam™ and Lipofectin™). Cationic and neutral lipids that are suitable for efficient receptor-recognition lipofection of polynucleotides include those of Felgner, WO 91/17424; WO 91/16024. Delivery can be to cells (e.g. in vitro or ex vivo administration) or target tissues (e.g. in vivo administration).

The preparation of lipid:nucleic acid complexes, including targeted liposomes such as immunolipid complexes, is well known to one of skill in the art (see, e.g., Crystal, Science 270:404-410 (1995); Blaese et al., Cancer Gene Ther. 2:291-297 (1995); Behr et al., Bioconjugate Chem. 5:382-389 (1994); Remy et al., Bioconjugate Chem. 5:647-654 (1994); Gao et al., Gene Therapy 2:710-722 (1995); Ahmad et al., Cancer Res. 52:4817-4820 (1992); U.S. Pat. Nos. 4,186,183, 4,217,344, 4,235,871, 4,261,975, 4,485,054, 4,501,728, 4,774,085, 4,837,028, and 4,946,787).

The use of RNA or DNA viral based systems for the delivery of nucleic acids take advantage of highly evolved processes for targeting a virus to specific cells in the body and trafficking the viral payload to the nucleus. Viral vectors can be administered directly to patients (in vivo) or they can be used to treat cells in vitro, and the modified cells may optionally be administered to patients (ex vivo). Conventional viral based systems could include retroviral, lentivirus, adenoviral, adeno-associated and herpes simplex virus vectors for gene transfer. Integration in the host genome is possible with the retrovirus, lentivirus, and adeno-associated virus gene transfer methods, often resulting in long term expression of the inserted transgene. Additionally, high transduction efficiencies have been observed in many different cell types and target tissues.

The tropism of a retrovirus can be altered by incorporating foreign envelope proteins, expanding the potential target population of target cells. Lentiviral vectors are retroviral vectors that are able to transduce or infect non-dividing cells and typically produce high viral titers. Selection of a retroviral gene transfer system would therefore depend on the target tissue. Retroviral vectors are comprised of cis-acting long terminal repeats with packaging capacity for up to 6-10 kb of foreign sequence. The minimum cis-acting LTRs are sufficient for replication and packaging of the vectors, which are then used to integrate the therapeutic gene into the target cell to provide permanent transgene expression. Widely used retroviral vectors include those based upon murine leukemia virus (MuLV), gibbon ape leukemia virus (GaLV), Simian Immuno deficiency virus (SIV), human immuno deficiency virus (HIV), and combinations thereof (see, e.g., Buchscher et al., J. Virol. 66:2731-2739 (1992); Johann et al., J. Virol. 66:1635-1640 (1992); Sommnerfelt et al., Virol. 176:58-59 (1990); Wilson et al., J. Virol. 63:2374-2378 (1989); Miller et al., J. Virol. 65:2220-2224 (1991); PCT/US94/05700).

In applications where transient expression is preferred, adenoviral based systems may be used. Adenoviral based vectors are capable of very high transduction efficiency in many cell types and do not require cell division. With such vectors, high titer and levels of expression have been obtained. This vector can be produced in large quantities in a relatively simple system.

Adeno-associated virus ("AAV") vectors may also be used to transduce cells with target nucleic acids, e.g., in the in vitro production of nucleic acids and peptides, and for in vivo and ex vivo gene therapy procedures (see, e.g., West et al., Virology 160:38-47 (1987); U.S. Pat. No. 4,797,368; WO 93/24641; Kotin, Human Gene Therapy 5:793-801 (1994); Muzyczka, J. Clin. Invest. 94:1351 (1994). Construction of recombinant AAV vectors are described in a number of publications, including U.S. Pat. No. 5,173,414; Tratschin et al., Mol. Cell. Biol. 5:3251-3260 (1985); Tratschin, et al., Mol. Cell. Biol. 4:2072-2081 (1984); Hermonat & Muzyczka, PNAS 81:6466-6470 (1984); and Samulski et al., J. Virol. 63:03822-3828 (1989).

Packaging cells are typically used to form virus particles that are capable of infecting a host cell. Such cells include 293 cells, which package adenovirus, and ψ2 cells or PA317 cells, which package retrovirus. Viral vectors used in gene therapy are usually generated by producer a cell line that packages a nucleic acid vector into a viral particle. The vectors typically contain the minimal viral sequences required for packaging and subsequent integration into a host, other viral sequences being replaced by an expression cassette for the polynucleotide(s) to be expressed. The missing viral functions are typically supplied in trans by the packaging cell line. For example, AAV vectors used in gene therapy typically only possess ITR sequences from the AAV genome which are required for packaging and integration into the host genome. Viral DNA is packaged in a cell line, which contains a helper plasmid encoding the other AAV genes, namely rep and cap, but lacking ITR sequences. The cell line may also infected with adenovirus as a helper. The helper virus promotes replication of the AAV vector and expression of AAV genes from the helper plasmid. The helper plasmid is not packaged in significant amounts due to a lack of ITR sequences. Contamination with adenovirus can be reduced by, e.g., heat treatment to which adenovirus is more sensitive than AAV. Additional methods for the delivery of nucleic acids to cells are known to those skilled in the art. See, for example, US20030087817.

Accordingly, AAV is considered an ideal candidate for use as a transducing vector. Such AAV transducing vectors can comprise sufficient cis-acting functions to replicate in the presence of adenovirus or herpesvirus or poxvirus (e.g., vaccinia virus) helper functions provided in trans. Recombinant AAV (rAAV) can be used to carry exogenous genes into cells of a variety of lineages. In these vectors, the AAV cap and/or rep genes are deleted from the viral genome and replaced with a DNA segment of choice. Current AAV vectors may accommodate up to 4300 bases of inserted DNA.

There are a number of ways to produce rAAV, and the invention provides rAAV and methods for preparing rAAV. For example, plasmid(s) containing or consisting essentially of the desired viral construct are transfected into AAV-infected cells. In addition, a second or additional helper plasmid is cotransfected into these cells to provide the AAV rep and/or cap genes which are obligatory for replication and packaging of the recombinant viral construct. Under these conditions, the rep and/or cap proteins of AAV act in trans to stimulate replication and packaging of the rAAV construct. Two to Three days after transfection, rAAV is harvested. Traditionally rAAV is harvested from the cells along with adenovirus. The contaminating adenovirus is then inactivated by heat treatment. In the instant invention, rAAV is advantageously harvested not from the cells themselves, but from cell supernatant. Accordingly, in an initial aspect the invention provides for preparing rAAV, and in addition to the foregoing, rAAV can be prepared by a method that comprises or consists essentially of: infecting susceptible cells with a rAAV containing exogenous DNA including DNA for expression, and helper virus (e.g., adenovirus, herpesvirus, poxvirus such as vaccinia virus) wherein the rAAV lacks functioning cap and/or rep (and the helper virus (e.g., adenovirus, herpesvirus, poxvirus such as vaccinia virus) provides the cap and/or rev function that the rAAV lacks); or infecting susceptible cells with a rAAV containing exogenous DNA including DNA for expression, wherein the recombinant lacks functioning cap and/or rep, and transfecting said cells with a plasmid supplying cap and/or rep function that the rAAV lacks; or infecting susceptible cells with a rAAV containing exogenous DNA including DNA for expression, wherein the recombinant lacks functioning cap and/or rep, wherein said cells supply cap and/or rep function that the recombinant lacks; or transfecting the susceptible cells with an AAV lacking functioning cap and/or rep and plasmids for inserting exogenous DNA into the recombinant so that the exogenous DNA is expressed by the recombinant and for supplying rep and/or cap functions whereby transfection results in an rAAV containing the exogenous DNA including DNA for expression that lacks functioning cap and/or rep.

The rAAV can be from an AAV as herein described, and advantageously can be an rAAV1, rAAV2, AAV5 or rAAV having hybrid or capsid which may comprise AAV1, AAV2, AAV5 or any combination thereof. One can select the AAV of the rAAV with regard to the cells to be targeted by the rAAV; e.g., one can select AAV serotypes 1, 2, 5 or a hybrid or capsid AAV1, AAV2, AAV5 or any combination thereof for targeting brain or neuronal cells; and one can select AAV4 for targeting cardiac tissue, AAV8 for targeting of liver tissue.

In addition to 293 cells, other cells that can be used in the practice of the invention and the relative infectivity of certain AAV serotypes in vitro as to these cells (see Grimm, D. et al, J. Virol. 82: 5887-5911 (2008)) are as follows:

| Cell Line | AAV-1 | AAV-2 | AAV-3 | AAV-4 | AAV-5 | AAV-6 | AAV-8 | AAV-9 |
|---|---|---|---|---|---|---|---|---|
| Huh-7 | 13 | 100 | 2.5 | 0.0 | 0.1 | 10 | 0.7 | 0.0 |
| HEK293 | 25 | 100 | 2.5 | 0.1 | 0.1 | 5 | 0.7 | 0.1 |
| HeLa | 3 | 100 | 2.0 | 0.1 | 6.7 | 1 | 0.2 | 0.1 |
| HepG2 | 3 | 100 | 16.7 | 0.3 | 1.7 | 5 | 0.3 | ND |
| Hep1A | 20 | 100 | 0.2 | 1.0 | 0.1 | 1 | 0.2 | 0.0 |
| 911 | 17 | 100 | 11 | 0.2 | 0.1 | 17 | 0.1 | ND |
| CHO | 100 | 100 | 14 | 1.4 | 333 | 50 | 10 | 1.0 |
| COS | 33 | 100 | 33 | 3.3 | 5.0 | 14 | 2.0 | 0.5 |
| MeWo | 10 | 100 | 20 | 0.3 | 6.7 | 10 | 1.0 | 0.2 |
| NIH3T3 | 10 | 100 | 2.9 | 2.9 | 0.3 | 10 | 0.3 | ND |
| A549 | 14 | 100 | 20 | ND | 0.5 | 10 | 0.5 | 0.1 |
| HT1180 | 20 | 100 | 10 | 0.1 | 0.3 | 33 | 0.5 | 0.1 |
| Monocytes | 1111 | 100 | ND | ND | 125 | 1429 | ND | ND |
| Immature DC | 2500 | 100 | ND | ND | 222 | 2857 | ND | ND |
| Mature DC | 2222 | 100 | ND | ND | 333 | 3333 | ND | ND |

The invention provides rAAV that contains or consists essentially of an exogenous nucleic acid molecule encoding a CRISPR (Clustered Regularly Interspaced Short Palindromic Repeats) system, e.g., a plurality of cassettes comprising or consisting a first cassette comprising or consisting essentially of a promoter, a nucleic acid molecule encoding a CRISPR-associated (Cas) protein (putative nuclease or helicase proteins), e.g., Cas9 and a terminator, and a two, or more, advantageously up to the packaging size limit of the vector, e.g., in total (including the first cassette) five, cassettes comprising or consisting essentially of a promoter, nucleic acid molecule encoding guide RNA (gRNA) and a terminator (e.g., each cassette schematically represented as Promoter-gRNA1-terminator, Promoter-gRNA2-terminator ... Promoter-gRNA(N)-terminator (where N is a number that can be inserted that is at an upper limit of the packaging size limit of the vector), or two or more individual rAAVs, each containing one or more than one cassette of a CRISPR system, e.g., a first rAAV containing the first cassette comprising or consisting essentially of a promoter, a nucleic acid molecule encoding Cas, e.g., Cas9 and a terminator, and a second rAAV containing a plurality, four, cassettes comprising or consisting essentially of a promoter, nucleic acid molecule encoding guide RNA (gRNA) and a terminator (e.g., each cassette schematically represented as Promoter-gRNAl-terminator, Promoter-gRNA2-terminator ... Promoter-gRNA(N)-terminator (where N is a number that can be inserted that is at an upper limit of the packaging size limit of the vector). As rAAV is a DNA virus, the nucleic acid molecules in the herein discussion concerning AAV or rAAV are advantageously DNA. The promoter is in some embodiments advantageously human Synapsin I promoter (hSyn).

Two ways to package Cas9 coding nucleic acid molecules, e.g., DNA, into viral vectors to mediate genome modification in vivo are preferred:
To achieve NHEJ-mediated gene knockout:
   Single virus vector:
      Vector containing two or more expression cassettes:
      Promoter-Cas9 coding nucleic acid molecule -terminator
      Promoter-gRNA1-terminator
      Promoter-gRNA2-terminator
      Promoter-gRNA(N)-terminator (up to size limit of vector)
   Double virus vector:
      Vector 1 containing one expression cassette for driving the expression of Cas9
         Promoter-Cas9 coding nucleic acid molecule-terminator
      Vector 2 containing one more expression cassettes for driving the expression of one or more guideRNAs
      Promoter-gRNA1-terminator
      Promoter-gRNA(N)-terminator (up to size limit of vector)

To mediate homology-directed repair. In addition to the single and double virus vector approaches described above, an additional vector is used to deliver a homology-direct repair template.

Promoter used to drive Cas9 coding nucleic acid molecule expression can include: AAV ITR can serve as a promoter: this is advantageous for eliminating the need for an additional promoter element (which can take up space in the vector). The additional space freed up can be used to drive the expression of additional elements (gRNA, etc). Also, ITR activity is relatively weaker, so can be used to reduce toxicity due to over expression of Cas9.

For ubiquitous expression, can use promoters: CMV, CAG, CBh, PGK, SV40, Ferritin heavy or light chains, etc.

For brain expression, can use promoters: SynapsinI for all neurons, CaMKIIalpha for excitatory neurons, GAD67 or GAD65 or VGAT for GABAergic neurons, etc.
For liver expression, can use Albumin promoter
For lung expression, can use SP-B
For endothelial cells, can use ICAM
For hematopoietic cells can use IFNbeta or CD45
For Osteoblasts can use OG-2
Promoter used to drive guide RNA can include:
Pol III promoters such as U6 or HI
Use of Pol II promoter and intronic cassettes to express gRNA

As to AAV, the AAV can be AAV1, AAV2, AAV5 or any combination thereof. One can select the AAV of the AAV with regard to the cells to be targeted; e.g., one can select AAV serotypes 1, 2, 5 or a hybrid or capsid AAV1, AAV2, AAV5 or any combination thereof for targeting brain or neuronal cells; and one can select AAV4 for targeting cardiac tissue. AAV8 is useful for delivery to the liver. The above promoters and vectors are preferred individually.

Additional methods for the delivery of nucleic acids to cells are known to those skilled in the art. See, for example, US20030087817.

In some embodiments, a host cell is transiently or non-transiently transfected with one or more vectors described herein. In some embodiments, a cell is transfected as it naturally occurs in a subject. In some embodiments, a cell that is transfected is taken from a subject. In some embodiments, the cell is derived from cells taken from a subject, such as a cell line. A wide variety of cell lines for tissue culture are known in the art. Examples of cell lines include, but are not limited to, C8161, CCRF-CEM, MOLT, mIMCD-3, NHDF, HeLa-S3, Huh1, Huh4, Huh7, HUVEC, HASMC, HEKn, HEKa, MiaPaCell, Panc1, PC-3, TF1, CTLL-2, C1R, Rat6, CV1, RPTE, A10, T24, J82, A375, ARH-77, Calu1, SW480, SW620, SKOV3, SK-UT, CaCo2, P388D1, SEM-K2, WEHI-231, HB56, TIB55, Jurkat, J45.01, LRMB, Bcl-1, BC-3, IC21, DLD2, Raw264.7, NRK, NRK-52E, MRC5, MEF, Hep G2, HeLa B, HeLa T4, COS, COS-1, COS-6, COS-M6A, BS-C-1 monkey kidney epithelial, BALB/ 3T3 mouse embryo fibroblast, 3T3 Swiss, 3T3-L1, 132-d5 human fetal fibroblasts; 10.1 mouse fibroblasts, 293-T, 3T3, 721, 9L, A2780, A2780ADR, A2780cis, A172, A20, A253, A431, A-549, ALC, B16, B35, BCP-1 cells, BEAS-2B, bEnd.3, BHK-21, BR 293, BxPC3, C3H-10T1/2, C6/36, Cal-27, CHO, CHO-7, CHO-IR, CHO-K1, CHO-K2, CHO-T, CHO Dhfr -/-, COR-L23, COR-L23/CPR, COR-L23/5010, COR-L23/R23, COS-7, COV-434, CML T1, CMT, CT26, D17, DH82, DU145, DuCaP, EL4, EM2, EM3, EMT6/AR1, EMT6/AR10.0, FM3, H1299, H69, HB54, HB55, HCA2, HEK-293, HeLa, Hepalclc7, HL-60, HMEC, HT-29, Jurkat, JY cells, K562 cells, Ku812, KCL22, KG1, KYO1, LNCap, Ma-Mel 1-48, MC-38, MCF-7, MCF-10A, MDA-MB-231, MDA-MB-468, MDA-MB-435, MDCK II, MDCK II, MOR/0.2R, MONO-MAC 6, MTD-1A, MyEnd, NCI-H69/CPR, NCI-H69/LX10, NCI-H69/LX20, NCI-H69/LX4, NIH-3T3, NALM-1, NW-145, OPCN / OPCT cell lines, Peer, PNT-1A / PNT 2, RenCa, RIN-5F, RMA/RMAS, Saos-2 cells, Sf-9, SkBr3, T2, T-47D, T84, THP1 cell line, U373, U87, U937, VCaP, Vero cells, WM39, WT-49, X63, YAC-1, YAR, and transgenic varieties thereof. Cell lines are available from a variety of sources known to those with skill in the art (see, e.g., the American Type Culture Collection (ATCC) (Manassus, Va.)). In some embodiments, a cell transfected with one or more vectors described herein is used to establish a new cell line comprising one or more vector-derived sequences. In some embodiments, a cell transiently transfected with the components of a CRISPR system as described herein (such as by transient transfection of one or more vectors, or transfection with RNA), and modified through the activity of a CRISPR complex, is used to establish a new cell line comprising cells containing the modification but lacking any other exogenous sequence. In some embodiments, cells transiently or non-transiently transfected with one or more vectors described herein, or cell lines derived from such cells are used in assessing one or more test compounds.

In some embodiments, one or more vectors described herein are used to produce a non-human transgenic animal or transgenic plant. In some embodiments, the transgenic animal is a mammal, such as a mouse, rat, or rabbit. Methods for producing transgenic plants and animals are known in the art, and generally begin with a method of cell transfection, such as described herein.

With recent advances in crop genomics, the ability to use CRISPR-Cas systems to perform efficient and cost effective gene editing and manipulation will allow the rapid selection and comparison of single and and multiplexed genetic manipulations to transform such genomes for improved production and enhanced traits. In this regard reference is made to US patents and publications: US Patent No. 6,603,061 - Agrobacterium-Mediated Plant Transformation Method; US Patent No. 7,868,149 - Plant Genome Sequences and Uses Thereof and US 2009/0100536 - Transgenic Plants with Enhanced Agronomic Traits. In the practice of the invention, the contents and disclosure of Morrell et al "Crop genomics:advances and applications" Nat Rev Genet. 2011 Dec 29;13(2):85-96 are also cited herein. In an advantageous embodiment of the invention, the CRSIPR/Cas9 system is used to engineer microalgae (Example 7). Accordingly, reference herein to animal cells may also apply, mutatis mutandis, to plant cells unless otherwise apparent.

In one aspect, the invention provides for methods of modifying a target polynucleotide in a eukaryotic cell, which may be in vivo, ex vivo or in vitro. In some embodiments, the method comprises sampling a cell or population of cells from a human or non-human animal or plant (including micro-algae), and modifying the cell or cells. Culturing may occur at any stage ex vivo. The cell or cells may even be re-introduced into the non-human animal or plant (including micro-algae). For re-introduced cells it is particularly preferred that the cells are stem cells.

In some embodiments, the method comprises allowing a CRISPR complex to bind to the target polynucleotide to effect cleavage of said target polynucleotide thereby modifying the target polynucleotide, wherein the CRISPR complex comprises a CRISPR enzyme complexed with a guide sequence hybridized to a target sequence within said target polynucleotide, wherein said guide sequence is linked to a tracr mate sequence which in turn hybridizes to a tracr sequence.

In one aspect, the invention provides a method of modifying expression of a polynucleotide in a eukaryotic cell. In some embodiments, the method comprises allowing a CRISPR complex to bind to the polynucleotide such that said binding results in increased or decreased expression of said polynucleotide; wherein the CRISPR complex comprises a CRISPR enzyme complexed with a guide sequence hybridized to a target sequence within said polynucleotide, wherein said guide sequence is linked to a tracr mate sequence which in turn hybridizes to a tracr sequence. Similar considerations apply as above for methods of modifying a target polynucleotide. In fact, these sampling, culturing and re-introduction options apply across the aspects of the present invention.

In one aspect, the invention provides kits containing any one or more of the elements disclosed in the above methods and compositions. Elements may provide individually or in combinations, and may provided in any suitable container, such as a vial, a bottle, or a tube. In some embodiments, the kit includes instructions in one or more languages, for example in more than one language.

In some embodiments, a kit comprises one or more reagents for use in a process utilizing one or more of the elements described herein. Reagents may be provided in any suitable container. For example, a kit may provide one or more reaction or storage buffers. Reagents may be provided in a form that is usable in a particular assay, or in a form that requires addition of one or more other components before use (e.g. in concentrate or lyophilized form). A buffer can be any buffer, including but not limited to a sodium carbonate buffer, a sodium bicarbonate buffer, a borate buffer, a Tris buffer, a MOPS buffer, a HEPES buffer, and combinations thereof. In some embodiments, the buffer is alkaline. In some embodiments, the buffer has a pH from about 7 to about 10. In some embodiments, the kit comprises one or more oligonucleotides corresponding to a guide sequence for insertion into a vector so as to operably link the guide sequence and a regulatory element. In some embodiments, the kit comprises a homologous recombination template polynucleotide.

In one aspect, the invention provides methods for using one or more elements of a CRISPR system. The CRISPR complex of the invention provides an effective means for modifying a target polynucleotide. The CRISPR complex of the invention has a wide variety of utility including modifying (e.g., deleting, inserting, translocating, inactivating, activating) a target polynucleotide in a multiplicity of cell types. As such the CRISPR complex of the invention has a broad spectrum of applications in, e.g., gene therapy, drug screening, disease diagnosis, and prognosis. An exemplary CRISPR complex comprises a CRISPR enzyme complexed with a guide sequence hybridized to a target sequence within the target polynucleotide. The guide sequence is linked to a tracr mate sequence, which in turn hybridizes to a tracr sequence.

In one embodiment, this invention provides a method of cleaving a target polynucleotide. The method comprises modifying a target polynucleotide using a CRISPR complex that binds to the target polynucleotide and effect cleavage of said target polynucleotide. Typically, the CRISPR complex of the invention, when introduced into a cell, creates a break (e.g., a single or a double strand break) in the genome sequence. For example, the method can be used to cleave a disease gene in a cell.

The break created by the CRISPR complex can be repaired by a repair processes such as the error prone non-homologous end joining (NHEJ) pathway or the high fidelity homology-directed repair (HDR). During these repair processes, an exogenous polynucleotide template can be introduced into the genome sequence. In some methods, the HDR process is used to modify a genome sequence. For example, an exogenous polynucleotide template comprising a sequence to be integrated flanked by an upstream sequence and a downstream sequence is introduced into a cell. The upstream and downstream sequences share sequence similarity with either side of the site of integration in the chromosome.

Where desired, a donor polynucleotide can be DNA, e.g., a DNA plasmid, a bacterial artificial chromosome (BAC), a yeast artificial chromosome (YAC), a viral vector, a linear piece of DNA, a PCR fragment, a naked nucleic acid, or a nucleic acid complexed with a delivery vehicle such as a liposome or poloxamer.

The exogenous polynucleotide template comprises a sequence to be integrated (e.g, a mutated gene). The sequence for integration may be a sequence endogenous or exogenous to the cell. Examples of a sequence to be integrated include polynucleotides encoding a protein or a non-coding RNA (e.g., a microRNA). Thus, the sequence for integration may be operably linked to an appropriate control sequence or sequences. Alternatively, the sequence to be integrated may provide a regulatory function.

The upstream and downstream sequences in the exogenous polynucleotide template are selected to promote recombination between the chromosomal sequence of interest and the donor polynucleotide. The upstream sequence is a nucleic acid sequence that shares sequence similarity with the genome sequence upstream of the targeted site for integration. Similarly, the downstream sequence is a nucleic acid sequence that shares sequence similarity with the chromosomal sequence downstream of the targeted site of integration. The upstream and downstream sequences in the exogenous polynucleotide template can have 75%, 80%, 85%, 90%, 95%, or 100% sequence identity with the targeted genome sequence. Preferably, the upstream and downstream sequences in the exogenous polynucleotide template have about 95%, 96%, 97%, 98%, 99%, or 100% sequence identity with the targeted genome sequence. In some methods, the upstream and downstream sequences in the exogenous polynucleotide template have about 99% or 100% sequence identity with the targeted genome sequence.

An upstream or downstream sequence may comprise from about 20 bp to about 2500 bp, for example, about 50, 100, 200, 300, 400, 500, 600, 700, 800, 900, 1000, 1100, 1200, 1300, 1400, 1500, 1600, 1700, 1800, 1900, 2000, 2100, 2200, 2300, 2400, or 2500 bp. In some methods, the exemplary upstream or downstream sequence has about 200 bp to about 2000 bp, about 600 bp to about 1000 bp, or more particularly about 700 bp to about 1000 bp.

In some methods, the exogenous polynucleotide template may further comprise a marker. Such a marker may make it easy to screen for targeted integrations. Examples of suitable markers include restriction sites, fluorescent proteins, or selectable markers. The exogenous polynucleotide template of the invention can be constructed using recombinant techniques (see, for example, Sambrook et al., 2001 and Ausubel et al., 1996).

In an exemplary method for modifying a target polynucleotide by integrating an exogenous polynucleotide template, a double stranded break is introduced into the genome sequence by the CRISPR complex, the break is repaired via homologous recombination an exogenous polynucleotide template such that the template is integrated into the genome. The presence of a double-stranded break facilitates integration of the template.

In other embodiments, this invention provides a method of modifying expression of a polynucleotide in a eukaryotic cell. The method comprises increasing or decreasing expression of a target polynucleotide by using a CRISPR complex that binds to the polynucleotide.

In some methods, a target polynucleotide can be inactivated to effect the modification of the expression in a cell. For example, upon the binding of a CRISPR complex to a target sequence in a cell, the target polynucleotide is inactivated such that the sequence is not transcribed, the coded protein is not produced, or the sequence does not function as the wild-type sequence does. For example, a protein or microRNA coding sequence may be inactivated such that the protein is not produced.

In some methods, a control sequence can be inactivated such that it no longer functions as a control sequence. As used herein, "control sequence" refers to any nucleic acid sequence that effects the transcription, translation, or accessibility of a nucleic acid sequence. Examples of a control sequence include, a promoter, a transcription terminator, and an enhancer are control sequences.

The inactivated target sequence may include a deletion mutation (i.e., deletion of one or more nucleotides), an insertion mutation (i.e., insertion of one or more nucleotides), or a nonsense mutation (i.e., substitution of a single nucleotide for another nucleotide such that a stop codon is introduced). In some methods, the inactivation of a target sequence results in "knockout" of the target sequence.

An altered expression of one or more genome sequences associated with a signaling biochemical pathway can be determined by assaying for a difference in the mRNA levels of the corresponding genes between the test model cell and a control cell, when they are contacted with a candidate agent. Alternatively, the differential expression of the sequences associated with a signaling biochemical pathway is determined by detecting a difference in the level of the encoded polypeptide or gene product.

To assay for an agent-induced alteration in the level of mRNA transcripts or corresponding polynucleotides, nucleic acid contained in a sample is first extracted according to standard methods in the art. For instance, mRNA can be isolated using various lytic enzymes or chemical solutions according to the procedures set forth in Sambrook et al. (1989), or extracted by nucleic-acid-binding resins following the accompanying instructions provided by the manufacturers. The mRNA contained in the extracted nucleic acid sample is then detected by amplification procedures or conventional hybridization assays (e.g. Northern blot analysis) according to methods widely known in the art or based on the methods exemplified herein.

For purpose of this invention, amplification means any method employing a primer and a polymerase capable of replicating a target sequence with reasonable fidelity. Amplification may be carried out by natural or recombinant DNA polymerases such as TaqGold™, T7 DNA polymerase, Klenow fragment of E.coli DNA polymerase, and reverse transcriptase. A preferred amplification method is PCR. In particular, the isolated RNA can be subjected to a reverse transcription assay that is coupled with a quantitative polymerase chain reaction (RT-PCR) in order to quantify the expression level of a sequence associated with a signaling biochemical pathway.

Detection of the gene expression level can be conducted in real time in an amplification assay. In one aspect, the amplified products can be directly visualized with fluorescent DNA-binding agents including but not limited to DNA intercalators and DNA groove binders. Because the amount of the intercalators incorporated into the double-stranded DNA molecules is typically proportional to the amount of the amplified DNA products, one can conveniently determine the amount of the amplified products by quantifying the fluorescence of the intercalated dye using conventional optical systems in the art. DNA-binding dye suitable for this application include SYBR green, SYBR blue, DAPI, propidium iodine, Hoeste, SYBR gold, ethidium bromide, acridines, proflavine, acridine orange, acriflavine, fluorcoumanin, ellipticine, daunomycin, chloroquine, distamycin D, chromomycin, homidium, mithramycin, ruthenium polypyridyls, anthramycin, and the like.

In another aspect, other fluorescent labels such as sequence specific probes can be employed in the amplification reaction to facilitate the detection and quantification of the amplified products. Probe-based quantitative amplification relies on the sequence-specific detection of a desired amplified product. It utilizes fluorescent, target-specific probes (e.g., TaqMan® probes) resulting in increased specificity and sensitivity. Methods for performing probe-based quantitative amplification are well established in the art and are taught in U.S. Patent No. 5,210,015.

In yet another aspect, conventional hybridization assays using hybridization probes that share sequence homology with sequences associated with a signaling biochemical pathway can be performed. Typically, probes are allowed to form stable complexes with the sequences associated with a signaling biochemical pathway contained within the biological sample derived from the test subject in a hybridization reaction. It will be appreciated by one of skill in the art that where antisense is used as the probe nucleic acid, the target polynucleotides provided in the sample are chosen to be complementary to sequences of the antisense nucleic acids. Conversely, where the nucleotide probe is a sense nucleic acid, the target polynucleotide is selected to be complementary to sequences of the sense nucleic acid.

Hybridization can be performed under conditions of various stringency, for instance as described herein. Suitable hybridization conditions for the practice of the present invention are such that the recognition interaction between the probe and sequences associated with a signaling biochemical pathway is both sufficiently specific and sufficiently stable. Conditions that increase the stringency of a hybridization reaction are widely known and published in the art. See, for example, (Sambrook, et al., (1989); Nonradioactive In Situ Hybridization Application Manual, Boehringer Mannheim, second edition). The hybridization assay can be formed using probes immobilized on any solid support, including but are not limited to nitrocellulose, glass, silicon, and a variety of gene arrays. A preferred hybridization assay is conducted on high-density gene chips as described in U.S. Patent No. 5,445,934.

For a convenient detection of the probe-target complexes formed during the hybridization assay, the nucleotide probes are conjugated to a detectable label. Detectable labels suitable for use in the present invention include any composition detectable by photochemical, biochemical, spectroscopic, immunochemical, electrical, optical or chemical means. A wide variety of appropriate detectable labels are known in the art, which include fluorescent or chemiluminescent labels, radioactive isotope labels, enzymatic or other ligands. In preferred embodiments, one will likely desire to employ a fluorescent label or an enzyme tag, such as digoxigenin, β-galactosidase, urease, alkaline phosphatase or peroxidase, avidin/biotin complex.

The detection methods used to detect or quantify the hybridization intensity will typically depend upon the label selected above. For example, radiolabels may be detected using photographic film or a phosphoimager. Fluorescent markers may be detected and quantified using a photodetector to detect emitted light. Enzymatic labels are typically detected by providing the enzyme with a substrate and measuring the reaction product produced by the action of the enzyme on the substrate; and finally colorimetric labels are detected by simply visualizing the colored label.

An agent-induced change in expression of sequences associated with a signaling biochemical pathway can also be determined by examining the corresponding gene products. Determining the protein level typically involves a) contacting the protein contained in a biological sample with an agent that specifically bind to a protein associated with a signaling biochemical pathway; and (b) identifying any agent:protein complex so formed. In one aspect of this embodiment, the agent that specifically binds a protein associated with a signaling biochemical pathway is an antibody, preferably a monoclonal antibody.

The reaction is performed by contacting the agent with a sample of the proteins associated with a signaling biochemical pathway derived from the test samples under conditions that will allow a complex to form between the agent and the proteins associated with a signaling biochemical pathway. The formation of the complex can be detected directly or indirectly according to standard procedures in the art. In the direct detection method, the agents are supplied with a detectable label and unreacted agents may be removed from the complex; the amount of remaining label thereby indicating the amount of complex formed. For such method, it is preferable to select labels that remain attached to the agents even during stringent washing conditions. It is preferable that the label does not interfere with the binding reaction. In the alternative, an indirect detection procedure may use an agent that contains a label introduced either chemically or enzymatically. A desirable label generally does not interfere with binding or the stability of the resulting agent:polypeptide complex. However, the label is typically designed to be accessible to an antibody for an effective binding and hence generating a detectable signal.

A wide variety of labels suitable for detecting protein levels are known in the art. Non-limiting examples include radioisotopes, enzymes, colloidal metals, fluorescent compounds, bioluminescent compounds, and chemiluminescent compounds.

The amount of agent:polypeptide complexes formed during the binding reaction can be quantified by standard quantitative assays. As illustrated above, the formation of agent:polypeptide complex can be measured directly by the amount of label remained at the site of binding. In an alternative, the protein associated with a signaling biochemical pathway is tested for its ability to compete with a labeled analog for binding sites on the specific agent. In this competitive assay, the amount of label captured is inversely proportional to the amount of protein sequences associated with a signaling biochemical pathway present in a test sample.

A number of techniques for protein analysis based on the general principles outlined above are available in the art. They include but are not limited to radioimmunoassays, ELISA (enzyme linked immunoradiometric assays), "sandwich" immunoassays, immunoradiometric assays, in situ immunoassays (using e.g., colloidal gold, enzyme or radioisotope labels), western blot analysis, immunoprecipitation assays, immunofluorescent assays, and SDS-PAGE.

Antibodies that specifically recognize or bind to proteins associated with a signaling biochemical pathway are preferable for conducting the aforementioned protein analyses. Where desired, antibodies that recognize a specific type of post-translational modifications (e.g., signaling biochemical pathway inducible modifications) can be used. Post-translational modifications include but are not limited to glycosylation, lipidation, acetylation, and phosphorylation. These antibodies may be purchased from commercial vendors. For example, anti-phosphotyrosine antibodies that specifically recognize tyrosine-phosphorylated proteins are available from a number of vendors including Invitrogen and Perkin Elmer. Anti-phosphotyrosine antibodies are particularly useful in detecting proteins that are differentially phosphorylated on their tyrosine residues in response to an ER stress. Such proteins include but are not limited to eukaryotic translation initiation factor 2 alpha (eIF-2α). Alternatively, these antibodies can be generated using conventional polyclonal or monoclonal antibody technologies by immunizing a host animal or an antibody-producing cell with a target protein that exhibits the desired post-translational modification.

In practicing the subject method, it may be desirable to discern the expression pattern of an protein associated with a signaling biochemical pathway in different bodily tissue, in different cell types, and/or in different subcellular structures. These studies can be performed with the use of tissue-specific, cell-specific or subcellular structure specific antibodies capable of binding to protein markers that are preferentially expressed in certain tissues, cell types, or subcellular structures.

An altered expression of a gene associated with a signaling biochemical pathway can also be determined by examining a change in activity of the gene product relative to a control cell. The assay for an agent-induced change in the activity of a protein associated with a signaling biochemical pathway will dependent on the biological activity and/or the signal transduction pathway that is under investigation. For example, where the protein is a kinase, a change in its ability to phosphorylate the downstream substrate(s) can be determined by a variety of assays known in the art. Representative assays include but are not limited to immunoblotting and immunoprecipitation with antibodies such as anti-phosphotyrosine antibodies that recognize phosphorylated proteins. In addition, kinase activity can be detected by high throughput chemiluminescent assays such as AlphaScreen™ (available from Perkin Elmer) and eTag™ assay (Chan-Hui, et al. (2003) Clinical Immunology 111: 162-174).

Where the protein associated with a signaling biochemical pathway is part of a signaling cascade leading to a fluctuation of intracellular pH condition, pH sensitive molecules such as fluorescent pH dyes can be used as the reporter molecules. In another example where the protein associated with a signaling biochemical pathway is an ion channel, fluctuations in membrane potential and/or intracellular ion concentration can be monitored. A number of commercial kits and high-throughput devices are particularly suited for a rapid and robust screening for modulators of ion channels. Representative instruments include FLIPRTM (Molecular Devices, Inc.) and VIPR (Aurora Biosciences). These instruments are capable of detecting reactions in over 1000 sample wells of a microplate simultaneously, and providing real-time measurement and functional data within a second or even a minisecond.

In practicing any of the methods disclosed herein, a suitable vector can be introduced to a cell or an embryo via one or more methods known in the art, including without limitation, microinjection, electroporation, sonoporation, biolistics, calcium phosphate-mediated transfection, cationic transfection, liposome transfection, dendrimer transfection, heat shock transfection, nucleofection transfection, magnetofection, lipofection, impalefection, optical transfection, proprietary agent-enhanced uptake of nucleic acids, and delivery via liposomes, immunoliposomes, virosomes, or artificial virions. In some methods, the vector is introduced into an embryo by microinjection. The vector or vectors may be microinjected into the nucleus or the cytoplasm of the embryo. In some methods, the vector or vectors may be introduced into a cell by nucleofection.

The target polynucleotide of a CRISPR complex can be any polynucleotide endogenous or exogenous to the eukaryotic cell. For example, the target polynucleotide can be a polynucleotide residing in the nucleus of the eukaryotic cell. The target polynucleotide can be a sequence coding a gene product (e.g., a protein) or a non-coding sequence (e.g., a regulatory polynucleotide or a junk DNA).

Examples of target polynucleotides include a sequence associated with a signaling biochemical pathway, e.g., a signaling biochemical pathway-associated gene or polynucleotide. Examples of target polynucleotides include a disease associated gene or polynucleotide. A "disease-associated" gene or polynucleotide refers to any gene or polynucleotide which is yielding transcription or translation products at an abnormal level or in an abnormal form in cells derived from a disease-affected tissues compared with tissues or cells of a non disease control. It may be a gene that becomes expressed at an abnormally high level; it may be a gene that becomes expressed at an abnormally low level, where the altered expression correlates with the occurrence and/or progression of the disease. A disease-associated gene also refers to a gene possessing mutation(s) or genetic variation that is directly responsible or is in linkage disequilibrium with a gene(s) that is responsible for the etiology of a disease. The transcribed or translated products may be known or unknown, and may be at a normal or abnormal level.

The target polynucleotide of a CRISPR complex can be any polynucleotide endogenous or exogenous to the eukaryotic cell. For example, the target polynucleotide can be a polynucleotide residing in the nucleus of the eukaryotic cell. The target polynucleotide can be a sequence coding a gene product (e.g., a protein) or a non-coding sequence (e.g., a regulatory polynucleotide or a junk DNA).

The target polynucleotide of a CRISPR complex may include a number of disease-associated genes and polynucleotides as well as signaling biochemical pathway-associated genes and polynucleotides as listed in US provisional patent applications 61/736,527 and 61/748,427 having Broad reference BI-2011/008/WSGR Docket No. 44063-701.101 and BI-2011/008/WSGR Docket No. 44063-701.102 respectively, both entitled SYSTEMS METHODS AND COMPOSITIONS FOR SEQUENCE MANIPULATION filed on December 12, 2012 and January 2, 2013, respectively.

Examples of target polynucleotides include a sequence associated with a signaling biochemical pathway, e.g., a signaling biochemical pathway-associated gene or polynucleotide. Examples of target polynucleotides include a disease associated gene or polynucleotide. A "disease-associated" gene or polynucleotide refers to any gene or polynucleotide which is yielding transcription or translation products at an abnormal level or in an abnormal form in cells derived from a disease-affected tissues compared with tissues or cells of a non disease control. It may be a gene that becomes expressed at an abnormally high level; it may be a gene that becomes expressed at an abnormally low level, where the altered expression correlates with the occurrence and/or progression of the disease. A disease-associated gene also refers to a gene possessing mutation(s) or genetic variation that is directly responsible or is in linkage disequilibrium with a gene(s) that is responsible for the etiology of a disease. The transcribed or translated products may be known or unknown, and may be at a normal or abnormal level.

Examples of disease-associated genes and polynucleotides are available from McKusick-Nathans Institute of Genetic Medicine, Johns Hopkins University (Baltimore, Md.) and National Center for Biotechnology Information, National Library of Medicine (Bethesda, Md.), available on the World Wide Web.

Examples of disease-associated genes and polynucleotides are listed in Tables A and B. Disease specific information is available from McKusick-Nathans Institute of Genetic Medicine, Johns Hopkins University (Baltimore, Md.) and National Center for Biotechnology Information, National Library of Medicine (Bethesda, Md.), available on the World Wide Web. Examples of signaling biochemical pathway-associated genes and polynucleotides are listed in Table C.

Mutations in these genes and pathways can result in production of improper proteins or proteins in improper amounts which affect function. Further examples of genes, diseases and proteins are from US Provisional applications 61/736,527 filed on December 12, 2012 and 61/748,427 filed on January 2, 2013. Such genes, proteins and pathways may be the target polynucleotide of a CRISPR complex.

**Table A**

| DISEASE/DISORDERS | GENE(S) |
|---|---|
| Neoplasia | PTEN; ATM; ATR; EGFR; ERBB2; ERBB3; ERBB4; |
| | Notch1; Notch2; Notch3; Notch4; AKT; AKT2; AKT3; HIF; |
| | HIF1a; HIF3a; Met; HRG; Bcl2; PPAR alpha; PPAR |
| | gamma; WT1 (Wilms Tumor); FGF Receptor Family |
| | members (5 members: 1, 2, 3, 4, 5); CDKN2a; APC; RB |
| | (retinoblastoma); MEN1; VHL; BRCA1; BRCA2; AR |
| | (Androgen Receptor); TSG101; IGF; IGF Receptor; Igf1 (4 |
| | variants); Igf2 (3 variants); Igf 1 Receptor; Igf 2 Receptor; |
| | Bax; Bcl2; caspases family (9 members: |
| | 1, 2, 3, 4, 6, 7, 8, 9, 12); Kras; Apc |
| Age-related Macular | Abcr; Ccl2; Cc2; cp (ceruloplasmin); Timp3; cathepsinD; |
| Degeneration | Vldlr; Ccr2 |
| Schizophrenia | Neuregulin1 (Nrg1); Erb4 (receptor for Neuregulin); |
| | Complexin1 (Cplx1); Tph1 Tryptophan hydroxylase; Tph2 |
| | Tryptophan hydroxylase 2; Neurexin 1; GSK3; GSK3a; |
| | GSK3b |
| Disorders | 5-HTT (Slc6a4); COMT; DRD (Drd1a); SLC6A3; DAOA; |
| | DTNBP1; Dao (Dao1) |
| Trinucleotide Repeat | HTT (Huntington's Dx); SBMA/SMAX1/AR (Kennedy's |
| Disorders | Dx); FXN/X25 (Friedrich's Ataxia); ATX3 (Machado- |
| | Joseph's Dx); ATXN1 and ATXN2 (spinocerebellar |
| | ataxias); DMPK (myotonic dystrophy); Atrophin-1 and Atnl |
| | (DRPLA Dx); CBP (Creb-BP - global instability); VLDLR |
| | (Alzheimer's); Atxn7; Atxn10 |
| Fragile X Syndrome | FMR2; FXR1; FXR2; mGLUR5 |
| Secretase Related | APH-1 (alpha and beta); Presenilin (Psen1); nicastrin |
| Disorders | (Ncstn); PEN-2 |
| Others | Nos1; Parp1; Nat1; Nat2 |
| Prion - related disorders | Prp |
| ALS | SOD1; ALS2; STEX; FUS; TARDBP; VEGF (VEGF-a; VEGF-b; VEGF-c) |
| Drug addiction | Prkce (alcohol); Drd2; Drd4; ABAT (alcohol); GRIA2; |
| | Grm5; Grin1; Htrlb; Grin2a; Drd3; Pdyn; Gria1 (alcohol) |
| Autism | Mecp2; BZRAP1; MDGA2; Sema5A; Neurexin 1; Fragile X |
| | (FMR2 (AFF2); FXR1; FXR2; Mglur5) |
| Alzheimer's Disease | E1; CHIP; UCH; UBB; Tau; LRP; PICALM; Clusterin; PS1; |
| | SORL1; CR1; Vldlr; Uba1; Uba3; CHIP28 (Aqp1, |
| | Aquaporin 1); Uchl1; Uchl3; APP |
| Inflammation | IL-10; IL-1 (IL-1a; IL-1b); IL-13; IL-17 (IL-17a (CTLA8); IL- |
| | 17b; IL-17c; IL-17d; IL-17f); II-23; Cx3cr1; ptpn22; TNFa; |
| | NOD2/CARD15 for IBD; IL-6; IL-12 (IL-12a; IL-12b); |
| | CTLA4; Cx3cl1 |
| Parkinson's Disease | x-Synuclein; DJ-1; LRRK2; Parkin; PINK1 |

**Table B:**

| | |
|---|---|
| Blood and coagulation diseases and disorders | Anemia (CDAN1, CDA1, RPS19, DBA, PKLR, PK1, NT5C3, UMPH1, PSN1, RHAG, RH50A, NRAMP2, SPTB, ALAS2, ANH1, ASB, ABCB7, ABC7, ASAT); Bare lymphocyte syndrome (TAPBP, TPSN, TAP2, ABCB3, PSF2, RING11, MHC2TA, C2TA, RFX5, RFXAP, RFX5), Bleeding disorders (TBXA2R, P2RX1, P2X1); Factor H and factor H-like 1 (HF1, CFH, HUS); Factor V and factor VIII (MCFD2); Factor VII deficiency (F7); Factor X deficiency (F10); Factor XI deficiency (F11); Factor XII deficiency (F12, HAF); Factor XIIIA deficiency (F13A1, F13A); Factor XIIIB deficiency (F13B); Fanconi anemia (FANCA, FACA, FA1, FA, FAA, FAAP95, FAAP90, FLJ34064, FANCB, FANCC, FACC, BRCA2, FANCD1, FANCD2, FANCD, FACD, FAD, FANCE, FACE, FANCF, XRCC9, FANCG, BRIP1, BACH1, FANCJ, PHF9, FANCL, FANCM, KIAA1596); Hemophagocytic lymphohistiocytosis disorders (PRF1, HPLH2, UNC13D, MUNC13-4, HPLH3, HLH3, FHL3); Hemophilia A (F8, F8C, HEMA); Hemophilia B (F9, HEMB), Hemorrhagic disorders (PI, ATT, F5); Leukocyde deficiencies and disorders (ITGB2, CD18, LCAMB, LAD, EIF2B1, EIF2BA, EIF2B2, EIF2B3, EIF2B5, LVWM, CACH, CLE, EIF2B4); Sickle cell anemia (HBB); Thalassemia (HBA2, HBB, HBD, LCRB, HBA1). |
| | |
| Cell dysregulation and oncology diseases and disorders | B-cell non-Hodgkin lymphoma (BCL7A, BCL7); Leukemia (TAL1, TCL5, SCL, TAL2, FLT3, NBS1, NBS, ZNFN1A1, IK1, LYF1, HOXD4, HOX4B, BCR, CML, PHL, ALL, ARNT, KRAS2, RASK2, GMPS, AF10, ARHGEF12, LARG, KIAA0382, CALM, CLTH, CEBPA, CEBP, CHIC2, BTL, FLT3, KIT, PBT, LPP, NPM1, NUP214, D9S46E, CAN, CAIN, RUNX1, CBFA2, AML1, WHSC1L1, NSD3, FLT3, AF1Q, NPM1, NUMA1, ZNF145, PLZF, PML, MYL, STAT5B, AF10, CALM, CLTH, ARL11, ARLTS1, P2RX7, P2X7, BCR, CML, PHL, ALL, GRAF, NF1, VRNF, WSS, NFNS, PTPN11, PTP2C, SHP2, NS1, BCL2, CCND1, PRAD1, BCL1, TCRA, GATA1, GF1, ERYF1, NFE1, ABL1, NQO1, DIA4, NMOR1, NUP214, D9S46E, CAN, CAIN). |
| | |
| Inflammation and immune related diseases and disorders | AIDS (KIR3DL1, NKAT3, NKB1, AMB11, KIR3DS1, IFNG, CXCL12, SDF1); Autoimmune lymphoproliferative syndrome (TNFRSF6, APT1, FAS, CD95, ALPS1A); Combined immunodeficiency, (IL2RG, SCIDX1, SCIDX, IMD4); HIV-1 (CCL5, SCYA5, D17S136E, TCP228), HIV susceptibility or infection (IL10, CSIF, CMKBR2, CCR2, CMKBR5, CCCKR5 (CCR5)); Immunodeficiencies (CD3E, CD3G, AICDA, AID, HIGM2, TNFRSF5, CD40, UNG, DGU, HIGM4, TNFSF5, CD40LG, HIGM1, IGM, FOXP3, IPEX, AIID, XPID, PIDX, TNFRSF14B, TACI); Inflammation (IL-10, IL-1 (IL-1a, IL-1b), IL-13, IL-17 (IL-17a (CTLA8), IL-17b, IL-17c, IL-17d, IL-17f), II-23, Cx3cr1, ptpn22, TNFa, NOD2/CARD15 for IBD, IL-6, IL-12 (IL-12a, IL-12b), CTLA4, Cx3cl1); Severe combined immunodeficiencies (SCIDs)(JAK3, JAKL, DCLRE1C, ARTEMIS, SCIDA, RAG1, RAG2, ADA, PTPRC, CD45, LCA, IL7R, CD3D, T3D, IL2RG, SCIDX1, SCIDX, IMD4). |
| | |
| Metabolic, liver, kidney and protein diseases and disorders | Amyloid neuropathy (TTR, PALB); Amyloidosis (APOA1, APP, AAA, CVAP, AD1, GSN, FGA, LYZ, TTR, PALB); Cirrhosis (KRT18, KRT8, CIRH1A, NAIC, TEX292, KIAA1988); Cystic fibrosis (CFTR, ABCC7, CF, MRP7); Glycogen storage diseases (SLC2A2, GLUT2, G6PC, G6PT, G6PT1, GAA, LAMP2, LAMPB, AGL, GDE, GBE1, GYS2, PYGL, PFKM); Hepatic adenoma, 142330 (TCF1, HNF1A, MODY3), Hepatic failure, early onset, and neurologic disorder (SCOD1, SCO1), Hepatic lipase deficiency (LIPC), Hepatoblastoma, cancer and carcinomas (CTNNB1, PDGFRL, PDGRL, PRLTS, AXIN1, AXIN, CTNNB1, TP53, P53, LFS1, IGF2R, MPRI, MET, CASP8, MCH5; Medullary cystic kidney disease (UMOD, HNFJ, FJHN, MCKD2, ADMCKD2); Phenylketonuria (PAH, PKU1, QDPR, DHPR, PTS); Polycystic kidney and hepatic disease (FCYT, PKHD1, ARPKD, PKD1, PKD2, PKD4, PKDTS, PRKCSH, G19P1, PCLD, SEC63). |
| | |
| Muscular / Skeletal diseases and disorders | Becker muscular dystrophy (DMD, BMD, MYF6), Duchenne Muscular Dystrophy (DMD, BMD); Emery-Dreifuss muscular dystrophy (LMNA, LMN1, EMD2, FPLD, CMD1A, HGPS, LGMD1B, LMNA, LMN1, EMD2, FPLD, CMD1A); Facioscapulohumeral muscular dystrophy (FSHMD1A, FSHD1A); Muscular dystrophy (FKRP, MDC1C, LGMD2I, LAMA2, LAMM, LARGE, KIAA0609, MDC1D, FCMD, TTID, MYOT, CAPN3, CANP3, DYSF, LGMD2B, SGCG, LGMD2C, DMDA1, SCG3, SGCA, ADL, DAG2, LGMD2D, DMDA2, SGCB, LGMD2E, SGCD, SGD, LGMD2F, CMD1L, TCAP, LGMD2G, CMD1N, TRIM32, HT2A, LGMD2H, FKRP, MDC1C, LGMD2I, TTN, CMD1G, TMD, LGMD2J, POMT1, CAV3, LGMD1C, SEPN1, SELN, RSMD1, PLEC1, PLTN, EBS1); Osteopetrosis (LRP5, BMND1, LRP7, LR3, OPPG, VBCH2, CLCN7, CLC7, OPTA2, OSTM1, GL, TCIRG1, TIRC7, OC116, OPTB1); Muscular atrophy (VAPB, VAPC, ALS8, SMN1, SMA1, SMA2, SMA3, SMA4, BSCL2, SPG17, GARS, SMAD1, CMT2D, HEXB, IGHMBP2, SMUBP2, CATF1, SMARD1). |
| | |
| Neurological and neuronal diseases and disorders | ALS (SOD1, ALS2, STEX, FUS, TARDBP, VEGF (VEGF-a, VEGF-b, VEGF-c); Alzheimer disease (APP, AAA, CVAP, AD1, APOE, AD2, PSEN2, AD4, STM2, APBB2, FE65L1, NOS3, PLAU, URK, ACE, DCP1, ACE1, MPO, PACIP1, PAXIP1L, PTIP, A2M, BLMH, BMH, PSEN1, AD3); Autism (Mecp2, BZRAP1, MDGA2, Sema5A, Neurexin 1, GLO1, MECP2, RTT, PPMX, MRX16, MRX79, NLGN3, NLGN4, KIAA1260, AUTSX2); Fragile X Syndrome (FMR2, FXR1, FXR2, mGLUR5); Huntington's disease and disease like disorders (HD, IT15, PRNP, PRIP, JPH3, JP3, HDL2, TBP, SCA17); Parkinson disease (NR4A2, NURR1, NOT, TINUR, SNCAIP, TBP, SCA17, SNCA, NACP, PARK1, PARK4, DJ1, PARK7, LRRK2, PARK8, PINK1, PARK6, UCHL1, PARK5, SNCA, NACP, PARK1, PARK4, PRKN, PARK2, PDJ, DBH, NDUFV2); Rett syndrome (MECP2, RTT, PPMX, MRX16, MRX79, CDKL5, STK9, MECP2, RTT, PPMX, MRX16, MRX79, x-Synuclein, DJ-1); Schizophrenia (Neuregulin1 (Nrg1), Erb4 (receptor for Neuregulin), Complexin1 (Cplx1), Tph1 Tryptophan hydroxylase, Tph2, Tryptophan hydroxylase 2, Neurexin 1, GSK3, GSK3a, GSK3b, 5-HTT (Slc6a4), COMT, DRD (Drdla), SLC6A3, DAOA, DTNBP1, Dao (Dao1)); Secretase Related Disorders (APH-1 (alpha and beta), Presenilin (Psen1), nicastrin, (Ncstn), PEN-2, Nos1, Parp1, Nat1, Nat2); Trinucleotide Repeat Disorders (HTT (Huntington's Dx), SBMA/SMAX1/AR (Kennedy's Dx), FXN/X25 (Friedrich's Ataxia), ATX3 (Machado-Joseph's Dx), ATXN1 and ATXN2 (spinocerebellar ataxias), DMPK (myotonic dystrophy), Atrophin-1 and Atn1 (DRPLA Dx), CBP (Creb-BP - global instability), VLDLR (Alzheimer's), Atxn7, Atxn10). |
| | |
| Occular diseases and disorders | Age-related macular degeneration (Abcr, Ccl2, Cc2, cp (ceruloplasmin), Timp3, cathepsinD, Vldlr, Ccr2); Cataract (CRYAA, CRYA1, CRYBB2, CRYB2, PITX3, BFSP2, CP49, CP47, CRYAA, CRYA1, PAX6, AN2, MGDA, CRYBA1, CRYB1, CRYGC, CRYG3, CCL, LIM2, MP19, CRYGD, CRYG4, BFSP2, CP49, CP47, HSF4, CTM, HSF4, CTM, MIP, AQP0, CRYAB, CRYA2, CTPP2, CRYBB1, CRYGD, CRYG4, CRYBB2, CRYB2, CRYGC, CRYG3, CCL, CRYAA, CRYA1, GJA8, CX50, CAE1, GJA3, CX46, CZP3, CAE3, CCM1, CAM, KRIT1); Corneal clouding and dystrophy (APOA1, TGFBI, CSD2, CDGG1, CSD, BIGH3, CDG2, TACSTD2, TROP2, M1S1, VSX1, RINX, PPCD, PPD, KTCN, COL8A2, FECD, PPCD2, PIP5K3, CFD); Cornea plana congenital (KERA, CNA2); Glaucoma (MYOC, TIGR, GLC1A, JOAG, GPOA, OPTN, GLC1E, FIP2, HYPL, NRP, CYP1B1, GLC3A, OPA1, NTG, NPG, CYP1B1, GLC3A); Leber congenital amaurosis (CRB1, RP12, CRX, CORD2, CRD, RPGRIP1, LCA6, CORD9, RPE65, RP20, AIPL1, LCA4, GUCY2D, GUC2D, LCA1, CORD6, RDH12, LCA3); Macular dystrophy (ELOVL4, ADMD, STGD2, STGD3, RDS, RP7, PRPH2, PRPH, AVMD, AOFMD, VMD2). |

**Table C:**

| CELLULAR FUNCTION | GENES |
|---|---|
| PI3K/AKT Signaling | PRKCE; ITGAM; ITGA5; IRAK1; PRKAA2; EIF2AK2; |
| | PTEN; EIF4E; PRKCZ; GRK6; MAPK1; TSC1; PLK1; |
| | AKT2; IKBKB; PIK3CA; CDK8; CDKN1B; NFKB2; BCL2; |
| | PIK3CB; PPP2R1A; MAPK8; BCL2L1; MAPK3; TSC2; |
| | ITGA1; KRAS; EIF4EBP1; RELA; PRKCD; NOS3; |
| | PRKAA1; MAPK9; CDK2; PPP2CA; PIM1; ITGB7; |
| | YWHAZ; ILK; TP53; RAF1; IKBKG; RELB; DYRK1A; |
| | CDKN1A; ITGB1; MAP2K2; JAK1; AKT1; JAK2; PIK3R1; |
| | CHUK; PDPK1; PPP2R5C; CTNNB1; MAP2K1; NFKB1; |
| | PAK3; ITGB3; CCND1; GSK3A; FRAP1; SFN; ITGA2; |
| | TTK; CSNK1A1; BRAF; GSK3B; AKT3; FOXO1; SGK; |
| | HSP90AA1; RPS6KB1 |
| ERK/MAPK Signaling | PRKCE; ITGAM; ITGA5; HSPB1; IRAK1; PRKAA2; |
| | EIF2AK2; RAC1; RAP1A; TLN1; EIF4E; ELK1; GRK6; |
| | MAPK1; RAC2; PLK1; AKT2; PIK3CA; CDK8; CREB1; |
| | PRKCI; PTK2; FOS; RPS6KA4; PIK3CB; PPP2R1A; |
| | PIK3C3; MAPK8; MAPK3; ITGA1; ETS1; KRAS; MYCN; |
| | EIF4EBP1; PPARG; PRKCD; PRKAA1; MAPK9; SRC; |
| | CDK2; PPP2CA; PIM1; PIK3C2A; ITGB7; YWHAZ; |
| | PPP1CC; KSR1; PXN; RAF1; FYN; DYRK1A; ITGB1; |
| | MAP2K2; PAK4; PIK3R1; STAT3; PPP2R5C; MAP2K1; |
| | PAK3; ITGB3; ESR1; ITGA2; MYC; TTK; CSNK1A1; |
| | CRKL; BRAF; ATF4; PRKCA; SRF; STAT1; SGK |
| Glucocorticoid Receptor | RAC1; TAF4B; EP300; SMAD2; TRAF6; PCAF; ELK1; |
| Signaling | MAPK1; SMAD3; AKT2; IKBKB; NCOR2; UBE2I; |
| | PIK3CA; CREB1; FOS; HSPA5; NFKB2; BCL2; |
| | MAP3K14; STAT5B; PIK3CB; PIK3C3; MAPK8; BCL2L1; |
| | MAPK3; TSC22D3; MAPK10; NRIP1; KRAS; MAPK13; |
| | RELA; STAT5A; MAPK9; NOS2A; PBX1; NR3C1; |
| | PIK3C2A; CDKN1C; TRAF2; SERPINE1; NCOA3; |
| | MAPK14; TNF; RAF1; IKBKG; MAP3K7; CREBBP; |
| | CDKN1A; MAP2K2; JAK1; IL8; NCOA2; AKT1; JAK2; |
| | PIK3R1; CHUK; STAT3; MAP2K1; NFKB1; TGFBR1; |
| | ESR1; SMAD4; CEBPB; JUN; AR; AKT3; CCL2; MMP1; |
| | STAT1; IL6; HSP90AA1 |
| Axonal Guidance Signaling | PRKCE; ITGAM; ROCK1; ITGA5; CXCR4; ADAM12; |
| | IGF1; RAC1; RAP1A; EIF4E; PRKCZ; NRP1; NTRK2; |
| | ARHGEF7; SMO; ROCK2; MAPK1; PGF; RAC2; |
| | PTPN11; GNAS; AKT2; PIK3CA; ERBB2; PRKCI; PTK2; |
| | CFL1; GNAQ; PIK3CB; CXCL12; PIK3C3; WNT11; |
| | PRKD1; GNB2L1; ABL1; MAPK3; ITGA1; KRAS; RHOA; |
| | PRKCD; PIK3C2A; ITGB7; GLI2; PXN; VASP; RAF1; |
| | FYN; ITGB1; MAP2K2; PAK4; ADAM17; AKT1; PIK3R1; |
| | GLI1; WNT5A; ADAM10; MAP2K1; PAK3; ITGB3; |
| | CDC42; VEGFA; ITGA2; EPHA8; CRKL; RND1; GSK3B; |
| | AKT3; PRKCA |
| Ephrin Receptor Signaling | PRKCE; ITGAM; ROCK1; ITGA5; CXCR4; IRAKI; |
| | PRKAA2; EIF2AK2; RAC1; RAP1A; GRK6; ROCK2; |
| | MAPK1; PGF; RAC2; PTPN11; GNAS; PLK1; AKT2; |
| | DOK1; CDK8; CREB1; PTK2; CFL1; GNAQ; MAP3K14; |
| | CXCL12; MAPK8; GNB2L1; ABL1; MAPK3; ITGA1; |
| | KRAS; RHOA; PRKCD; PRKAA1; MAPK9; SRC; CDK2; |
| | PIM1; ITGB7; PXN; RAF1; FYN; DYRK1A; ITGB1; |
| | MAP2K2; PAK4; AKT1; JAK2; STAT3; ADAM10; |
| | MAP2K1; PAK3; ITGB3; CDC42; VEGFA; ITGA2; |
| | EPHA8; TTK; CSNK1A1; CRKL; BRAF; PTPN13; ATF4; |
| | AKT3; SGK |
| Actin Cytoskeleton | ACTN4; PRKCE; ITGAM; ROCK1; ITGA5; IRAK1; |
| Signaling | PRKAA2; EIF2AK2; RAC1; INS; ARHGEF7; GRK6; |
| | ROCK2; MAPK1; RAC2; PLK1; AKT2; PIK3CA; CDK8; |
| | PTK2; CFL1; PIK3CB; MYH9; DIAPH1; PIK3C3; MAPK8; |
| | F2R; MAPK3; SLC9A1; ITGA1; KRAS; RHOA; PRKCD; |
| | PRKAA1; MAPK9; CDK2; PIM1; PIK3C2A; ITGB7; |
| | PPP1CC; PXN; VIL2; RAF1; GSN; DYRK1A; ITGB1; |
| | MAP2K2; PAK4; PIP5K1A; PIK3R1; MAP2K1; PAK3; |
| | ITGB3; CDC42; APC; ITGA2; TTK; CSNK1A1; CRKL; |
| | BRAF; VAV3; SGK |
| Huntington's Disease | PRKCE; IGF1; EP300; RCOR1; PRKCZ; HDAC4; TGM2; |
| Signaling | MAPK1; CAPNS1; AKT2; EGFR; NCOR2; SP1; CAPN2; |
| | PIK3CA; HDAC5; CREB1; PRKCI; HSPA5; REST; |
| | GNAQ; PIK3CB; PIK3C3; MAPK8; IGF1R; PRKD1; |
| | GNB2L1; BCL2L1; CAPN1; MAPK3; CASP8; HDAC2; |
| | HDAC7A; PRKCD; HDAC11; MAPK9; HDAC9; PIK3C2A; |
| | HDAC3; TP53; CASP9; CREBBP; AKT1; PIK3R1; |
| | PDPK1; CASP1; APAF1; FRAP1; CASP2; JUN; BAX; |
| | ATF4; AKT3; PRKCA; CLTC; SGK; HDAC6; CASP3 |
| Apoptosis Signaling | PRKCE; ROCK1; BID; IRAKI; PRKAA2; EIF2AK2; BAK1; |
| | BIRC4; GRK6; MAPK1; CAPNS1; PLK1; AKT2; IKBKB; |
| | CAPN2; CDK8; FAS; NFKB2; BCL2; MAP3K14; MAPK8; |
| | BCL2L1; CAPN1; MAPK3; CASP8; KRAS; RELA; |
| | PRKCD; PRKAA1; MAPK9; CDK2; PIM1; TP53; TNF; |
| | RAF1; IKBKG; RELB; CASP9; DYRK1A; MAP2K2; |
| | CHUK; APAF1; MAP2K1; NFKB1; PAK3; LMNA; CASP2; |
| | BIRC2; TTK; CSNK1A1; BRAF; BAX; PRKCA; SGK; |
| | CASP3; BIRC3; PARP1 |
| B Cell Receptor Signaling | RAC1; PTEN; LYN; ELK1; MAPK1; RAC2; PTPN11; |
| | AKT2; IKBKB; PIK3CA; CREB1; SYK; NFKB2; CAMK2A; |
| | MAP3K14; PIK3CB; PIK3C3; MAPK8; BCL2L1; ABL1; |
| | MAPK3; ETS1; KRAS; MAPK13; RELA; PTPN6; MAPK9; |
| | EGR1; PIK3C2A; BTK; MAPK14; RAF1; IKBKG; RELB; |
| | MAP3K7; MAP2K2; AKT1; PIK3R1; CHUK; MAP2K1; |
| | NFKB1; CDC42; GSK3A; FRAP1; BCL6; BCL10; JUN; |
| | GSK3B; ATF4; AKT3; VAV3; RPS6KB1 |
| Leukocyte Extravasation | ACTN4; CD44; PRKCE; ITGAM; ROCK1; CXCR4; CYBA; |
| Signaling | RAC1; RAP1A; PRKCZ; ROCK2; RAC2; PTPN11; |
| | MMP14; PIK3CA; PRKCI; PTK2; PIK3CB; CXCL12; |
| | PIK3C3; MAPK8; PRKD1; ABL1; MAPK10; CYBB; |
| | MAPK13; RHOA; PRKCD; MAPK9; SRC; PIK3C2A; BTK; |
| | MAPK14; NOX1; PXN; VIL2; VASP; ITGB1; MAP2K2; |
| | CTNND1; PIK3R1; CTNNB1; CLDN1; CDC42; F11R; ITK; |
| | CRKL; VAV3; CTTN; PRKCA; MMP1; MMP9 |
| Integrin Signaling | ACTN4; ITGAM; ROCK1; ITGA5; RAC1; PTEN; RAP1A; |
| | TLN1; ARHGEF7; MAPK1; RAC2; CAPNS1; AKT2; |
| | CAPN2; PIK3CA; PTK2; PIK3CB; PIK3C3; MAPK8; |
| | CAV1; CAPN1; ABL1; MAPK3; ITGA1; KRAS; RHOA; |
| | SRC; PIK3C2A; ITGB7; PPP1CC; ILK; PXN; VASP; |
| | RAF1; FYN; ITGB1; MAP2K2; PAK4; AKT1; PIK3R1; |
| | TNK2; MAP2K1; PAK3; ITGB3; CDC42; RND3; ITGA2; |
| | CRKL; BRAF; GSK3B; AKT3 |
| Acute Phase Response | IRAKI; SOD2; MYD88; TRAF6; ELK1; MAPK1; PTPN11; |
| Signaling | AKT2; IKBKB; PIK3CA; FOS; NFKB2; MAP3K14; |
| | PIK3CB; MAPK8; RIPK1; MAPK3; IL6ST; KRAS; |
| | MAPK13; IL6R; RELA; SOCS1; MAPK9; FTL; NR3C1; |
| | TRAF2; SERPINE1; MAPK14; TNF; RAF1; PDK1; |
| | IKBKG; RELB; MAP3K7; MAP2K2; AKT1; JAK2; PIK3R1; |
| | CHUK; STAT3; MAP2K1; NFKB1; FRAP1; CEBPB; JUN; |
| | AKT3; IL1R1; IL6 |
| PTEN Signaling | ITGAM; ITGA5; RAC1; PTEN; PRKCZ; BCL2L11; |
| | MAPK1; RAC2; AKT2; EGFR; IKBKB; CBL; PIK3CA; |
| | CDKN1B; PTK2; NFKB2; BCL2; PIK3CB; BCL2L1; |
| | MAPK3; ITGA1; KRAS; ITGB7; ILK; PDGFRB; INSR; |
| | RAF1; IKBKG; CASP9; CDKN1A; ITGB1; MAP2K2; |
| | AKT1; PIK3R1; CHUK; PDGFRA; PDPK1; MAP2K1; |
| | NFKB1; ITGB3; CDC42; CCND1; GSK3A; ITGA2; |
| | GSK3B; AKT3; FOXO1; CASP3; RPS6KB1 |
| p53 Signaling | PTEN; EP300; BBC3; PCAF; FASN; BRCA1; GADD45A; |
| | BIRC5; AKT2; PIK3CA; CHEK1; TP53INP1; BCL2; |
| | PIK3CB; PIK3C3; MAPK8; THBS1; ATR; BCL2L1; E2F1; |
| | PMAIP1; CHEK2; TNFRSF10B; TP73; RB1; HDAC9; |
| | CDK2; PIK3C2A; MAPK14; TP53; LRDD; CDKN1A; |
| | HIPK2; AKT1; PIK3R1; RRM2B; APAF1; CTNNB1; |
| | SIRT1; CCND1; PRKDC; ATM; SFN; CDKN2A; JUN; |
| | SNAI2; GSK3B; BAX; AKT3 |
| Aryl Hydrocarbon Receptor | HSPB1; EP300; FASN; TGM2; RXRA; MAPK1; NQO1; |
| Signaling | NCOR2; SP1; ARNT; CDKN1B; FOS; CHEK1; |
| | SMARCA4; NFKB2; MAPK8; ALDH1A1; ATR; E2F1; |
| | MAPK3; NRIP1; CHEK2; RELA; TP73; GSTP1; RB1; |
| | SRC; CDK2; AHR; NFE2L2; NCOA3; TP53; TNF; |
| | CDKN1A; NCOA2; APAF1; NFKB1; CCND1; ATM; ESR1; |
| | CDKN2A; MYC; JUN; ESR2; BAX; IL6; CYP1B1; |
| | HSP90AA1 |
| Xenobiotic Metabolism | PRKCE; EP300; PRKCZ; RXRA; MAPK1; NQO1; |
| Signaling | NCOR2; PIK3CA; ARNT; PRKCI; NFKB2; CAMK2A; |
| | PIK3CB; PPP2R1A; PIK3C3; MAPK8; PRKD1; |
| | ALDH1A1; MAPK3; NRIP1; KRAS; MAPK13; PRKCD; |
| | GSTP1; MAPK9; NOS2A; ABCB1; AHR; PPP2CA; FTL; |
| | NFE2L2; PIK3C2A; PPARGC1A; MAPK14; TNF; RAF1; |
| | CREBBP; MAP2K2; PIK3R1; PPP2R5C; MAP2K1; |
| | NFKB1; KEAP1; PRKCA; EIF2AK3; IL6; CYP1B1; |
| | HSP90AA1 |
| SAPK/JNK Signaling | PRKCE; IRAK1; PRKAA2; EIF2AK2; RAC1; ELK1; |
| | GRK6; MAPK1; GADD45A; RAC2; PLK1; AKT2; PIK3CA; |
| | FADD; CDK8; PIK3CB; PIK3C3; MAPK8; RIPK1; |
| | GNB2L1; IRS1; MAPK3; MAPK10; DAXX; KRAS; |
| | PRKCD; PRKAA1; MAPK9; CDK2; PIM1; PIK3C2A; |
| | TRAF2; TP53; LCK; MAP3K7; DYRK1A; MAP2K2; |
| | PIK3R1; MAP2K1; PAK3; CDC42; JUN; TTK; CSNK1A1; |
| | CRKL; BRAF; SGK |
| PPAr/RXR Signaling | PRKAA2; EP300; INS; SMAD2; TRAF6; PPARA; FASN; |
| | RXRA; MAPK1; SMAD3; GNAS; IKBKB; NCOR2; |
| | ABCA1; GNAQ; NFKB2; MAP3K14; STAT5B; MAPK8; |
| | IRS1; MAPK3; KRAS; RELA; PRKAA1; PPARGC1A; |
| | NCOA3; MAPK14; INSR; RAF1; IKBKG; RELB; MAP3K7; |
| | CREBBP; MAP2K2; JAK2; CHUK; MAP2K1; NFKB1; |
| | TGFBR1; SMAD4; JUN; IL1R1; PRKCA; IL6; HSP90AA1; |
| | ADIPOQ |
| NF-KB Signaling | IRAK1; EIF2AK2; EP300; INS; MYD88; PRKCZ; TRAF6; |
| | TBK1; AKT2; EGFR; IKBKB; PIK3CA; BTRC; NFKB2; |
| | MAP3K14; PIK3CB; PIK3C3; MAPK8; RIPK1; HDAC2; |
| | KRAS; RELA; PIK3C2A; TRAF2; TLR4; PDGFRB; TNF; |
| | INSR; LCK; IKBKG; RELB; MAP3K7; CREBBP; AKT1; |
| | PIK3R1; CHUK; PDGFRA; NFKB1; TLR2; BCL10; |
| | GSK3B; AKT3; TNFAIP3; IL1R1 |
| Neuregulin Signaling | ERBB4; PRKCE; ITGAM; ITGA5; PTEN; PRKCZ; ELK1; |
| | MAPK1; PTPN11; AKT2; EGFR; ERBB2; PRKCI; |
| | CDKN1B; STAT5B; PRKD1; MAPK3; ITGA1; KRAS; |
| | PRKCD; STAT5A; SRC; ITGB7; RAF1; ITGB1; MAP2K2; |
| | ADAM17; AKT1; PIK3R1; PDPK1; MAP2K1; ITGB3; |
| | EREG; FRAP1; PSEN1; ITGA2; MYC; NRG1; CRKL; |
| | AKT3; PRKCA; HSP90AA1; RPS6KB1 |
| Wnt & Beta catenin | CD44; EP300; LRP6; DVL3; CSNK1E; GJA1; SMO; |
| Signaling | AKT2; PIN1; CDH1; BTRC; GNAQ; MARK2; PPP2R1A; |
| | WNT11; SRC; DKK1; PPP2CA; SOX6; SFRP2; ILK; |
| | LEF1; SOX9; TP53; MAP3K7; CREBBP; TCF7L2; AKT1; |
| | PPP2R5C; WNT5A; LRP5; CTNNB1; TGFBR1; CCND1; |
| | GSK3A; DVL1; APC; CDKN2A; MYC; CSNK1A1; GSK3B; |
| | AKT3; SOX2 |
| Insulin Receptor Signaling | PTEN; INS; EIF4E; PTPN1; PRKCZ; MAPK1; TSC1; |
| | PTPN11; AKT2; CBL; PIK3CA; PRKCI; PIK3CB; PIK3C3; |
| | MAPK8; IRS1; MAPK3; TSC2; KRAS; EIF4EBP1; |
| | SLC2A4; PIK3C2A; PPP1CC; INSR; RAF1; FYN; |
| | MAP2K2; JAK1; AKT1; JAK2; PIK3R1; PDPK1; MAP2K1; |
| | GSK3A; FRAP1; CRKL; GSK3B; AKT3; FOXO1; SGK; |
| | RPS6KB1 |
| IL-6 Signaling | HSPB1; TRAF6; MAPKAPK2; ELK1; MAPK1; PTPN11; |
| | IKBKB; FOS; NFKB2; MAP3K14; MAPK8; MAPK3; |
| | MAPK10; IL6ST; KRAS; MAPK13; IL6R; RELA; SOCS1; |
| | MAPK9; ABCB1; TRAF2; MAPK14; TNF; RAF1; IKBKG; |
| | RELB; MAP3K7; MAP2K2; IL8; JAK2; CHUK; STAT3; |
| | MAP2K1; NFKB1; CEBPB; JUN; IL1R1; SRF; IL6 |
| Hepatic Cholestasis | PRKCE; IRAK1; INS; MYD88; PRKCZ; TRAF6; PPARA; |
| | RXRA; IKBKB; PRKCI; NFKB2; MAP3K14; MAPK8; |
| | PRKD1; MAPK10; RELA; PRKCD; MAPK9; ABCB1; |
| | TRAF2; TLR4; TNF; INSR; IKBKG; RELB; MAP3K7; IL8; |
| | CHUK; NR1H2; TJP2; NFKB1; ESR1; SREBF1; FGFR4; |
| | JUN; IL1R1; PRKCA; IL6 |
| IGF-1 Signaling | IGF1; PRKCZ; ELK1; MAPK1; PTPN11; NEDD4; AKT2; |
| | PIK3CA; PRKCI; PTK2; FOS; PIK3CB; PIK3C3; MAPK8; |
| | IGF1R; IRS1; MAPK3; IGFBP7; KRAS; PIK3C2A; |
| | YWHAZ; PXN; RAF1; CASP9; MAP2K2; AKT1; PIK3R1; |
| | PDPK1; MAP2K1; IGFBP2; SFN; JUN; CYR61; AKT3; |
| | FOXO1; SRF; CTGF; RPS6KB1 |
| NRF2-mediated Oxidative | PRKCE; EP300; SOD2; PRKCZ; MAPK1; SQSTM1; |
| Stress Response | NQO1; PIK3CA; PRKCI; FOS; PIK3CB; PIK3C3; MAPK8; |
| | PRKD1; MAPK3; KRAS; PRKCD; GSTP1; MAPK9; FTL; |
| | NFE2L2; PIK3C2A; MAPK14; RAF1; MAP3K7; CREBBP; |
| | MAP2K2; AKT1; PIK3R1; MAP2K1; PPIB; JUN; KEAP1; |
| | GSK3B; ATF4; PRKCA; EIF2AK3; HSP90AA1 |
| Hepatic Fibrosis/Hepatic | EDN1; IGF1; KDR; FLT1; SMAD2; FGFR1; MET; PGF; |
| Stellate Cell Activation | SMAD3; EGFR; FAS; CSF1; NFKB2; BCL2; MYH9; |
| | IGF1R; IL6R; RELA; TLR4; PDGFRB; TNF; RELB; IL8; |
| | PDGFRA; NFKB1; TGFBR1; SMAD4; VEGFA; BAX; |
| | IL1R1; CCL2; HGF; MMP1; STAT1; IL6; CTGF; MMP9 |
| PPAR Signaling | EP300; INS; TRAF6; PPARA; RXRA; MAPK1; IKBKB; |
| | NCOR2; FOS; NFKB2; MAP3K14; STAT5B; MAPK3; |
| | NRIP1; KRAS; PPARG; RELA; STAT5A; TRAF2; |
| | PPARGC1A; PDGFRB; TNF; INSR; RAF1; IKBKG; |
| | RELB; MAP3K7; CREBBP; MAP2K2; CHUK; PDGFRA; |
| | MAP2K1; NFKB1; JUN; IL1R1; HSP90AA1 |
| Fc Epsilon RI Signaling | PRKCE; RAC1; PRKCZ; LYN; MAPK1; RAC2; PTPN11; |
| | AKT2; PIK3CA; SYK; PRKCI; PIK3CB; PIK3C3; MAPK8; |
| | PRKD1; MAPK3; MAPK10; KRAS; MAPK13; PRKCD; |
| | MAPK9; PIK3C2A; BTK; MAPK14; TNF; RAF1; FYN; |
| | MAP2K2; AKT1; PIK3R1; PDPK1; MAP2K1; AKT3; |
| | VAV3; PRKCA |
| G-Protein Coupled | PRKCE; RAP1A; RGS16; MAPK1; GNAS; AKT2; IKBKB; |
| Receptor Signaling | PIK3CA; CREB1; GNAQ; NFKB2; CAMK2A; PIK3CB; |
| | PIK3C3; MAPK3; KRAS; RELA; SRC; PIK3C2A; RAF1; |
| | IKBKG; RELB; FYN; MAP2K2; AKT1; PIK3R1; CHUK; |
| | PDPK1; STAT3; MAP2K1; NFKB1; BRAF; ATF4; AKT3; |
| | PRKCA |
| Inositol Phosphate | PRKCE; IRAKI; PRKAA2; EIF2AK2; PTEN; GRK6; |
| Metabolism | MAPK1; PLK1; AKT2; PIK3CA; CDK8; PIK3CB; PIK3C3; |
| | MAPK8; MAPK3; PRKCD; PRKAA1; MAPK9; CDK2; |
| | PIM1; PIK3C2A; DYRK1A; MAP2K2; PIP5K1A; PIK3R1; |
| | MAP2K1; PAK3; ATM; TTK; CSNK1A1; BRAF; SGK |
| PDGF Signaling | EIF2AK2; ELK1; ABL2; MAPK1; PIK3CA; FOS; PIK3CB; |
| | PIK3C3; MAPK8; CAV1; ABL1; MAPK3; KRAS; SRC; |
| | PIK3C2A; PDGFRB; RAF1; MAP2K2; JAK1; JAK2; |
| | PIK3R1; PDGFRA; STAT3; SPHK1; MAP2K1; MYC; |
| | JUN; CRKL; PRKCA; SRF; STAT1; SPHK2 |
| VEGF Signaling | ACTN4; ROCK1; KDR; FLT1; ROCK2; MAPK1; PGF; |
| | AKT2; PIK3CA; ARNT; PTK2; BCL2; PIK3CB; PIK3C3; |
| | BCL2L1; MAPK3; KRAS; HIF1A; NOS3; PIK3C2A; PXN; |
| | RAF1; MAP2K2; ELAVL1; AKT1; PIK3R1; MAP2K1; SFN; |
| | VEGFA; AKT3; FOXO1; PRKCA |
| Natural Killer Cell Signaling | PRKCE; RAC1; PRKCZ; MAPK1; RAC2; PTPN11; |
| | KIR2DL3; AKT2; PIK3CA; SYK; PRKCI; PIK3CB; |
| | PIK3C3; PRKD1; MAPK3; KRAS; PRKCD; PTPN6; |
| | PIK3C2A; LCK; RAF1; FYN; MAP2K2; PAK4; AKT1; |
| | PIK3R1; MAP2K1; PAK3; AKT3; VAV3; PRKCA |
| Cell Cycle: G1/S | HDAC4; SMAD3; SUV39H1; HDAC5; CDKN1B; BTRC; |
| Checkpoint Regulation | ATR; ABL1; E2F1; HDAC2; HDAC7A; RB1; HDAC11; |
| | HDAC9; CDK2; E2F2; HDAC3; TP53; CDKN1A; CCND1; |
| | E2F4; ATM; RBL2; SMAD4; CDKN2A; MYC; NRG1; |
| | GSK3B; RBL1; HDAC6 |
| T Cell Receptor Signaling | RAC1; ELK1; MAPK1; IKBKB; CBL; PIK3CA; FOS; |
| | NFKB2; PIK3CB; PIK3C3; MAPK8; MAPK3; KRAS; |
| | RELA; PIK3C2A; BTK; LCK; RAF1; IKBKG; RELB; FYN; |
| | MAP2K2; PIK3R1; CHUK; MAP2K1; NFKB1; ITK; BCL10; |
| | JUN; VAV3 |
| Death Receptor Signaling | CRADD; HSPB1; BID; BIRC4; TBK1; IKBKB; FADD; |
| | FAS; NFKB2; BCL2; MAP3K14; MAPK8; RIPK1; CASP8; |
| | DAXX; TNFRSF10B; RELA; TRAF2; TNF; IKBKG; RELB; |
| | CASP9; CHUK; APAF1; NFKB1; CASP2; BIRC2; CASP3; |
| | BIRC3 |
| FGF Signaling | RAC1; FGFR1; MET; MAPKAPK2; MAPK1; PTPN11; |
| | AKT2; PIK3CA; CREB1; PIK3CB; PIK3C3; MAPK8; |
| | MAPK3; MAPK13; PTPN6; PIK3C2A; MAPK14; RAF1; |
| | AKT1; PIK3R1; STAT3; MAP2K1; FGFR4; CRKL; ATF4; |
| | AKT3; PRKCA; HGF |
| GM-CSF Signaling | LYN; ELK1; MAPK1; PTPN11; AKT2; PIK3CA; CAMK2A; |
| | STAT5B; PIK3CB; PIK3C3; GNB2L1; BCL2L1; MAPK3; |
| | ETS1; KRAS; RUNX1; PIM1; PIK3C2A; RAF1; MAP2K2; |
| | AKT1; JAK2; PIK3R1; STAT3; MAP2K1; CCND1; AKT3; |
| | STAT1 |
| Amyotrophic Lateral | BID; IGF1; RAC1; BIRC4; PGF; CAPNS1; CAPN2; |
| Sclerosis Signaling | PIK3CA; BCL2; PIK3CB; PIK3C3; BCL2L1; CAPN1; |
| | PIK3C2A; TP53; CASP9; PIK3R1; RAB5A; CASP1; |
| | APAF1; VEGFA; BIRC2; BAX; AKT3; CASP3; BIRC3 |
| JAK/Stat Signaling | PTPN1; MAPK1; PTPN11; AKT2; PIK3CA; STAT5B; |
| | PIK3CB; PIK3C3; MAPK3; KRAS; SOCS1; STAT5A; |
| | PTPN6; PIK3C2A; RAF1; CDKN1A; MAP2K2; JAK1; |
| | AKT1; JAK2; PIK3R1; STAT3; MAP2K1; FRAP1; AKT3; |
| | STAT1 |
| Nicotinate and Nicotinamide | PRKCE; IRAKI; PRKAA2; EIF2AK2; GRK6; MAPK1; |
| Metabolism | PLK1; AKT2; CDK8; MAPK8; MAPK3; PRKCD; PRKAA1; |
| | PBEF1; MAPK9; CDK2; PIM1; DYRK1A; MAP2K2; |
| | MAP2K1; PAK3; NT5E; TTK; CSNK1A1; BRAF; SGK |
| Chemokine Signaling | CXCR4; ROCK2; MAPK1; PTK2; FOS; CFL1; GNAQ; |
| | CAMK2A; CXCL12; MAPK8; MAPK3; KRAS; MAPK13; |
| | RHOA; CCR3; SRC; PPP1CC; MAPK14; NOX1; RAF1; |
| | MAP2K2; MAP2K1; JUN; CCL2; PRKCA |
| IL-2 Signaling | ELK1; MAPK1; PTPN11; AKT2; PIK3CA; SYK; FOS; |
| | STAT5B; PIK3CB; PIK3C3; MAPK8; MAPK3; KRAS; |
| | SOCS1; STAT5A; PIK3C2A; LCK; RAF1; MAP2K2; |
| | JAK1; AKT1; PIK3R1; MAP2K1; JUN; AKT3 |
| Synaptic Long Term | PRKCE; IGF1; PRKCZ; PRDX6; LYN; MAPK1; GNAS; |
| Depression | PRKCI; GNAQ; PPP2R1A; IGF1R; PRKD1; MAPK3; |
| | KRAS; GRN; PRKCD; NOS3; NOS2A; PPP2CA; |
| | YWHAZ; RAF1; MAP2K2; PPP2R5C; MAP2K1; PRKCA |
| Estrogen Receptor | TAF4B; EP300; CARM1; PCAF; MAPK1; NCOR2; |
| Signaling | SMARCA4; MAPK3; NRIP1; KRAS; SRC; NR3C1; |
| | HDAC3; PPARGC1A; RBM9; NCOA3; RAF1; CREBBP; |
| | MAP2K2; NCOA2; MAP2K1; PRKDC; ESR1; ESR2 |
| Protein Ubiquitination | TRAF6; SMURF1; BIRC4; BRCA1; UCHL1; NEDD4; |
| Pathway | CBL; UBE2I; BTRC; HSPA5; USP7; USP10; FBXW7; |
| | USP9X; STUB1; USP22; B2M; BIRC2; PARK2; USP8; |
| | USP1; VHL; HSP90AA1; BIRC3 |
| IL-10 Signaling | TRAF6; CCR1; ELK1; IKBKB; SP1; FOS; NFKB2; |
| | MAP3K14; MAPK8; MAPK13; RELA; MAPK14; TNF; |
| | IKBKG; RELB; MAP3K7; JAK1; CHUK; STAT3; NFKB1; |
| | JUN; IL1R1; IL6 |
| VDR/RXR Activation | PRKCE; EP300; PRKCZ; RXRA; GADD45A; HES1; |
| | NCOR2; SP1; PRKCI; CDKN1B; PRKD1; PRKCD; |
| | RUNX2; KLF4; YY1; NCOA3; CDKN1A; NCOA2; SPP1; |
| | LRP5; CEBPB; FOXO1; PRKCA |
| TGF-beta Signaling | EP300; SMAD2; SMURF1; MAPK1; SMAD3; SMAD1; |
| | FOS; MAPK8; MAPK3; KRAS; MAPK9; RUNX2; |
| | SERPINE1; RAF1; MAP3K7; CREBBP; MAP2K2; |
| | MAP2K1; TGFBR1; SMAD4; JUN; SMAD5 |
| Toll-like Receptor Signaling | IRAKI; EIF2AK2; MYD88; TRAF6; PPARA; ELK1; |
| | IKBKB; FOS; NFKB2; MAP3K14; MAPK8; MAPK13; |
| | RELA; TLR4; MAPK14; IKBKG; RELB; MAP3K7; CHUK; |
| | NFKB1; TLR2; JUN |
| p38 MAPK Signaling | HSPB1; IRAK1; TRAF6; MAPKAPK2; ELK1; FADD; FAS; |
| | CREB1; DDIT3; RPS6KA4; DAXX; MAPK13; TRAF2; |
| | MAPK14; TNF; MAP3K7; TGFBR1; MYC; ATF4; IL1R1; |
| | SRF; STAT1 |
| Neurotrophin/TRK Signaling | NTRK2; MAPK1; PTPN11; PIK3CA; CREB1; FOS; |
| | PIK3CB; PIK3C3; MAPK8; MAPK3; KRAS; PIK3C2A; |
| | RAF1; MAP2K2; AKT1; PIK3R1; PDPK1; MAP2K1; |
| | CDC42; JUN; ATF4 |
| FXR/RXR Activation | INS; PPARA; FASN; RXRA; AKT2; SDC1; MAPK8; |
| | APOB; MAPK10; PPARG; MTTP; MAPK9; PPARGC1A; |
| | TNF; CREBBP; AKT1; SREBF1; FGFR4; AKT3; FOXO1 |
| Synaptic Long Term | PRKCE; RAP1A; EP300; PRKCZ; MAPK1; CREB1; |
| Potentiation | PRKCI; GNAQ; CAMK2A; PRKD1; MAPK3; KRAS; |
| | PRKCD; PPP1CC; RAF1; CREBBP; MAP2K2; MAP2K1; |
| | ATF4; PRKCA |
| Calcium Signaling | RAP1A; EP300; HDAC4; MAPK1; HDAC5; CREB1; |
| | CAMK2A; MYH9; MAPK3; HDAC2; HDAC7A; HDAC11; |
| | HDAC9; HDAC3; CREBBP; CALR; CAMKK2; ATF4; |
| | HDAC6 |
| EGF Signaling | ELK1; MAPK1; EGFR; PIK3CA; FOS; PIK3CB; PIK3C3; |
| | MAPK8; MAPK3; PIK3C2A; RAF1; JAK1; PIK3R1; |
| | STAT3; MAP2K1; JUN; PRKCA; SRF; STAT1 |
| Hypoxia Signaling in the | EDN1; PTEN; EP300; NQO1; UBE2I; CREB1; ARNT; |
| Cardiovascular System | HIF1A; SLC2A4; NOS3; TP53; LDHA; AKT1; ATM; |
| | VEGFA; JUN; ATF4; VHL; HSP90AA1 |
| LPS/IL-1 Mediated Inhibition | IRAKI; MYD88; TRAF6; PPARA; RXRA; ABCA1; |
| of RXR Function | MAPK8; ALDH1A1; GSTP1; MAPK9; ABCB1; TRAF2; |
| | TLR4; TNF; MAP3K7; NR1H2; SREBF1; JUN; IL1R1 |
| LXR/RXR Activation | FASN; RXRA; NCOR2; ABCA1; NFKB2; IRF3; RELA; |
| | NOS2A; TLR4; TNF; RELB; LDLR; NR1H2; NFKB1; |
| | SREBF1; IL1R1; CCL2; IL6; MMP9 |
| Amyloid Processing | PRKCE; CSNK1E; MAPK1; CAPNS1; AKT2; CAPN2; |
| | CAPN1; MAPK3; MAPK13; MAPT; MAPK14; AKT1; |
| | PSEN1; CSNK1A1; GSK3B; AKT3; APP |
| IL-4 Signaling | AKT2; PIK3CA; PIK3CB; PIK3C3; IRS1; KRAS; SOCS1; |
| | PTPN6; NR3C1; PIK3C2A; JAK1; AKT1; JAK2; PIK3R1; |
| | FRAP1; AKT3; RPS6KB1 |
| Cell Cycle: G2/M DNA | EP300; PCAF; BRCA1; GADD45A; PLK1; BTRC; |
| Damage Checkpoint | CHEK1; ATR; CHEK2; YWHAZ; TP53; CDKN1A; |
| Regulation | PRKDC; ATM; SFN; CDKN2A |
| Nitric Oxide Signaling in the | KDR; FLT1; PGF; AKT2; PIK3CA; PIK3CB; PIK3C3; |
| Cardiovascular System | CAV1; PRKCD; NOS3; PIK3C2A; AKT1; PIK3R1; |
| | VEGFA; AKT3; HSP90AA1 |
| Purine Metabolism | NME2; SMARCA4; MYH9; RRM2; ADAR; EIF2AK4; |
| | PKM2; ENTPD1; RAD51; RRM2B; TJP2; RAD51C; |
| | NT5E; POLD1; NME1 |
| cAMP-mediated Signaling | RAP1A; MAPK1; GNAS; CREB1; CAMK2A; MAPK3; |
| | SRC; RAF1; MAP2K2; STAT3; MAP2K1; BRAF; ATF4 |
| Mitochondrial Dysfunction | SOD2; MAPK8; CASP8; MAPK10; MAPK9; CASP9; |
| | PARK7; PSEN1; PARK2; APP; CASP3 |
| Notch Signaling | HES1; JAG1; NUMB; NOTCH4; ADAM17; NOTCH2; |
| | PSEN1; NOTCH3; NOTCH1; DLL4 |
| Endoplasmic Reticulum | HSPA5; MAPK8; XBP1; TRAF2; ATF6; CASP9; ATF4; |
| Stress Pathway | EIF2AK3; CASP3 |
| Pyrimidine Metabolism | NME2; AICDA; RRM2; EIF2AK4; ENTPD1; RRM2B; |
| | NT5E; POLD1; NME1 |
| Parkinson's Signaling | UCHL1; MAPK8; MAPK13; MAPK14; CASP9; PARK7; |
| | PARK2; CASP3 |
| Cardiac & Beta Adrenergic | GNAS; GNAQ; PPP2R1A; GNB2L1; PPP2CA; PPP1CC; |
| Signaling | PPP2R5C |
| Glycolysis/Gluconeogene sis | HK2; GCK; GPI; ALDH1A1; PKM2; LDHA; HK1 |
| Interferon Signaling | IRF1; SOCS1; JAK1; JAK2; IFITM1; STAT1; IFIT3 |
| Sonic Hedgehog Signaling | ARRB2; SMO; GLI2; DYRK1A; GLI1; GSK3B; DYRK1B |
| Glycerophospholipid | PLD1; GRN; GPAM; YWHAZ; SPHK1; SPHK2 |
| Metabolism | |
| Phospholipid Degradation | PRDX6; PLD1; GRN; YWHAZ; SPHK1; SPHK2 |
| Tryptophan Metabolism | SIAH2; PRMT5; NEDD4; ALDH1A1; CYP1B1; SIAH1 |
| Lysine Degradation | SUV39H1; EHMT2; NSD1; SETD7; PPP2R5C |
| Nucleotide Excision Repair | ERCC5; ERCC4; XPA; XPC; ERCC1 |
| Pathway | |
| Starch and Sucrose | UCHL1; HK2; GCK; GPI; HK1 |
| Metabolism | |
| Aminosugars Metabolism | NQO1; HK2; GCK; HK1 |
| Arachidonic Acid | PRDX6; GRN; YWHAZ; CYP1B1 |
| Metabolism | |
| Circadian Rhythm Signaling | CSNK1E; CREB1; ATF4; NR1D1 |
| Coagulation System | BDKRB1; F2R; SERPINE1; F3 |
| Dopamine Receptor | PPP2R1A; PPP2CA; PPP1CC; PPP2R5C |
| Signaling | |
| Glutathione Metabolism | IDH2; GSTP1; ANPEP; IDH1 |
| Glycerolipid Metabolism | ALDH1A1; GPAM; SPHK1; SPHK2 |
| Linoleic Acid Metabolism | PRDX6; GRN; YWHAZ; CYP1B1 |
| Methionine Metabolism | DNMT1; DNMT3B; AHCY; DNMT3A |
| Pyruvate Metabolism | GLO1; ALDH1A1; PKM2; LDHA |
| Arginine and Proline | ALDH1A1; NOS3; NOS2A |
| Metabolism | |
| Eicosanoid Signaling | PRDX6; GRN; YWHAZ |
| Fructose and Mannose | HK2; GCK; HK1 |
| Metabolism | |
| Galactose Metabolism | HK2; GCK; HK1 |
| Stilbene, Coumarine and | PRDX6; PRDX1; TYR |
| Lignin Biosynthesis | |
| Antigen Presentation | CALR; B2M |
| Pathway | |
| Biosynthesis of Steroids | NQO1; DHCR7 |
| Butanoate Metabolism | ALDH1A1; NLGN1 |
| Citrate Cycle | IDH2; IDH1 |
| Fatty Acid Metabolism | ALDH1A1; CYP1B1 |
| Glycerophospholipid | PRDX6; CHKA |
| Metabolism | |
| Histidine Metabolism | PRMT5; ALDH1A1 |
| Inositol Metabolism | ERO1L; APEX1 |
| Metabolism of Xenobiotics | GSTP1; CYP1B1 |
| by Cytochrome p450 | |
| Methane Metabolism | PRDX6; PRDX1 |
| Phenylalanine Metabolism | PRDX6; PRDX1 |
| Propanoate Metabolism | ALDH1A1; LDHA |
| Selenoamino Acid | PRMT5; AHCY |
| Metabolism | |
| Sphingolipid Metabolism | SPHK1; SPHK2 |
| Aminophosphonate | PRMT5 |
| Metabolism | |
| Androgen and Estrogen | PRMT5 |
| Metabolism | |
| Ascorbate and Aldarate | ALDH1A1 |
| Metabolism | |
| Bile Acid Biosynthesis | ALDH1A1 |
| Cysteine Metabolism | LDHA |
| Fatty Acid Biosynthesis | FASN |
| Glutamate Receptor | GNB2L1 |
| Signaling | |
| NRF2-mediated Oxidative | PRDX1 |
| Stress Response | |
| Pentose Phosphate | GPI |
| Pathway | |
| Pentose and Glucuronate | UCHL1 |
| Interconversions | |
| Retinol Metabolism | ALDH1A1 |
| Riboflavin Metabolism | TYR |
| Tyrosine Metabolism | PRMT5, TYR |
| Ubiquinone Biosynthesis | PRMT5 |
| Valine, Leucine and | ALDH1A1 |
| Isoleucine Degradation | |
| Glycine, Serine and | CHKA |
| Threonine Metabolism | |
| Lysine Degradation | ALDH1A1 |
| Pain/Taste | TRPM5; TRPA1 |
| Pain | TRPM7; TRPC5; TRPC6; TRPC1; Cnr1; cnr2; Grk2; |
| | Trpa1; Pomc; Cgrp; Crf; Pka; Era; Nr2b; TRPM5; Prkaca; |
| | Prkacb; Prkar1a; Prkar2a |
| Mitochondrial Function | AIF; CytC; SMAC (Diablo); Aifm-1; Aifm-2 |
| Developmental Neurolo gy | BMP-4; Chordin (Chrd); Noggin (Nog); WNT (Wnt2; |
| | Wnt2b; Wnt3a; Wnt4; Wnt5a; Wnt6; Wnt7b; Wnt8b; |
| | Wnt9a; Wnt9b; Wnt10a; Wnt10b; Wnt16); beta-catenin; |
| | Dkk-1; Frizzled related proteins; Otx-2; Gbx2; FGF-8; |
| | Reelin; Dab1; unc-86 (Pou4f1 or Brn3a); Numb; Reln |

Embodiments of the invention also relate to methods and compositions related to knocking out genes, amplifying genes and repairing particular mutations associated with DNA repeat instability and neurological disorders (Robert D. Wells, Tetsuo Ashizawa, Genetic Instabilities and Neurological Diseases, Second Edition, Academic Press, Oct 13, 2011 - Medical). Specific aspects of tandem repeat sequences have been found to be responsible for more than twenty human diseases (New insights into repeat instability: role of RNA•DNA hybrids. McIvor EI, Polak U, Napierala M. RNA Biol. 2010 Sep-Oct;7(5):551-8). The CRISPR-Cas system may be harnessed to correct these defects of genomic instability.

A further aspect of the invention relates to utilizing the CRISPR-Cas system for correcting defects in the EMP2A and EMP2B genes that have been identified to be associated with Lafora disease. Lafora disease is an autosomal recessive condition which is characterized by progressive myoclonus epilepsy which may start as epileptic seizures in adolescence. A few cases of the disease may be caused by mutations in genes yet to be identified. The disease causes seizures, muscle spasms, difficulty walking, dementia, and eventually death. There is currently no therapy that has proven effective against disease progression. Other genetic abnormalities associated with epilepsy may also be targeted by the CRISPR-Cas sytem and the underlying genetics is further described in Genetics of Epilepsy and Genetic Epilepsies, edited by Giuliano Avanzini, Jeffrey L. Noebels, Mariani Foundation Paediatric Neurology:20; 2009).

In yet another aspect of the invention, the CRISPR-Cas system may be used to correct ocular defects that arise from several genetic mutations further described in Genetic Diseases of the Eye, Second Edition, edited by Elias I. Traboulsi, Oxford University Press, 2012.

Several further aspects of the invention relate to correcting defects associated with a wide range of genetic diseases which are further described on the website of the National Institutes of Health under the topic subsection Genetic Disorders (website at health.nih.gov/topic/GeneticDisorders). The genetic brain diseases may include but are not limited to Adrenoleukodystrophy, Agenesis of the Corpus Callosum, Aicardi Syndrome, Alpers' Disease, Alzheimer's Disease, Barth Syndrome, Batten Disease, CADASIL, Cerebellar Degeneration, Fabry's Disease, Gerstmann-Straussler-Scheinker Disease, Huntington's Disease and other Triplet Repeat Disorders, Leigh's Disease, Lesch-Nyhan Syndrome, Menkes Disease, Mitochondrial Myopathies and NINDS Colpocephaly. These diseases are further described on the website of the National Institutes of Health under the subsection Genetic Brain Disorders.

In some embodiments, the condition may be neoplasia. In some embodiments, where the condition is neoplasia, the genes to be targeted are any of those listed in Table A (in this case PTEN asn so forth). In some embodiments, the condition may be Age-related Macular Degeneration. In some embodiments, the condition may be a Schizophrenic Disorder. In some embodiments, the condition may be a Trinucleotide Repeat Disorder. In some embodiments, the condition may be Fragile X Syndrome. In some embodiments, the condition may be a Secretase Related Disorder. In some embodiments, the condition may be a Prion - related disorder. In some embodiments, the condition may be ALS. In some embodiments, the condition may be a drug addiction. In some embodiments, the condition may be Autism. In some embodiments, the condition may be Alzheimer's Disease. In some embodiments, the condition may be inflammation. In some embodiments, the condition may be Parkinson's Disease.

Examples of proteins associated with Parkinson's disease include but are not limited to α-synuclein, DJ-1, LRRK2, PINK1, Parkin, UCHL1, Synphilin-1, and NURR1.

Examples of addiction-related proteins may include ABAT for example.

Examples of inflammation-related proteins may include the monocyte chemoattractant protein-1 (MCP1) encoded by the Ccr2 gene, the C-C chemokine receptor type 5 (CCR5) encoded by the Ccr5 gene, the IgG receptor IIB (FCGR2b, also termed CD32) encoded by the Fcgr2b gene, or the Fc epsilon R1g (FCER1g) protein encoded by the Fcer1g gene, for example.

Examples of cardiovascular diseases associated proteins may include IL1B (interleukin 1, beta), XDH (xanthine dehydrogenase), TP53 (tumor protein p53), PTGIS (prostaglandin I2 (prostacyclin) synthase), MB (myoglobin), IL4 (interleukin 4), ANGPT1 (angiopoietin 1), ABCG8 (ATP-binding cassette, sub-family G (WHITE), member 8), or CTSK (cathepsin K), for example.

Examples of Alzheimer's disease associated proteins may include the very low density lipoprotein receptor protein (VLDLR) encoded by the VLDLR gene, the ubiquitin-like modifier activating enzyme 1 (UBA1) encoded by the UBA1 gene, or the NEDD8-activating enzyme E1 catalytic subunit protein (UBE1C) encoded by the UBA3 gene, for example.

Examples of proteins associated Autism Spectrum Disorder may include the benzodiazapine receptor (peripheral) associated protein 1 (BZRAP1) encoded by the BZRAP1 gene, the AF4/FMR2 family member 2 protein (AFF2) encoded by the AFF2 gene (also termed MFR2), the fragile X mental retardation autosomal homolog 1 protein (FXR1) encoded by the FXR1 gene, or the fragile X mental retardation autosomal homolog 2 protein (FXR2) encoded by the FXR2 gene, for example.

Examples of proteins associated Macular Degeneration may include the ATP-binding cassette, sub-family A (ABC1) member 4 protein (ABCA4) encoded by the ABCR gene, the apolipoprotein E protein (APOE) encoded by the APOE gene, or the chemokine (C-C motif) Ligand 2 protein (CCL2) encoded by the CCL2 gene, for example.

Examples of proteins associated Schizophrenia may include NRG1, ErbB4, CPLX1, TPH1, TPH2, NRXN1, GSK3A, BDNF, DISCI, GSK3B, and combinations thereof.

Examples of proteins involved in tumor suppression may include ATM (ataxia telangiectasia mutated), ATR (ataxia telangiectasia and Rad3 related), EGFR (epidermal growth factor receptor), ERBB2 (v-erb-b2 erythroblastic leukemia viral oncogene homolog 2), ERBB3 (v-erb-b2 erythroblastic leukemia viral oncogene homolog 3), ERBB4 (v-erb-b2 erythroblastic leukemia viral oncogene homolog 4), Notch 1, Notch2, Notch 3, or Notch 4, for example.

Examples of proteins associated with a secretase disorder may include PSENEN (presenilin enhancer 2 homolog (C. elegans)), CTSB (cathepsin B), PSEN1 (presenilin 1), APP (amyloid beta (A4) precursor protein), APH1B (anterior pharynx defective 1 homolog B (C. elegans)), PSEN2 (presenilin 2 (Alzheimer disease 4)), or BACE1 (beta-site APP-cleaving enzyme 1), for example.

Examples of proteins associated with Amyotrophic Lateral Sclerosis may include SOD1 (superoxide dismutase 1), ALS2 (amyotrophic lateral sclerosis 2), FUS (fused in sarcoma), TARDBP (TAR DNA binding protein), VAGFA (vascular endothelial growth factor A), VAGFB (vascular endothelial growth factor B), and VAGFC (vascular endothelial growth factor C), and any combination thereof.

Examples of proteins associated with prion diseases may include SOD1 (superoxide dismutase 1), ALS2 (amyotrophic lateral sclerosis 2), FUS (fused in sarcoma), TARDBP (TAR DNA binding protein), VAGFA (vascular endothelial growth factor A), VAGFB (vascular endothelial growth factor B), and VAGFC (vascular endothelial growth factor C), and any combination thereof.

Examples of proteins related to neurodegenerative conditions in prion disorders may include A2M (Alpha-2-Macroglobulin), AATF (Apoptosis antagonizing transcription factor), ACPP (Acid phosphatase prostate), ACTA2 (Actin alpha 2 smooth muscle aorta), ADAM22 (ADAM metallopeptidase domain), ADORA3 (Adenosine A3 receptor), or ADRA1D (Alpha-ID adrenergic receptor for Alpha-ID adrenoreceptor), for example.

Examples of proteins associated with Immunodeficiency may include A2M [alpha-2-macroglobulin]; AANAT [arylalkylamine N-acetyltransferase]; ABCA1 [ATP-binding cassette, sub-family A (ABC1), member 1]; ABCA2 [ATP-binding cassette, sub-family A (ABC1), member 2]; or ABCA3 [ATP-binding cassette, sub-family A (ABC1), member 3]; for example.

Examples of proteins associated with Trinucleotide Repeat Disorders include AR (androgen receptor), FMR1 (fragile X mental retardation 1), HTT (huntingtin), or DMPK (dystrophia myotonica-protein kinase), FXN (frataxin), ATXN2 (ataxin 2), for example.

Examples of proteins associated with Neurotransmission Disorders include SST (somatostatin), NOS1 (nitric oxide synthase 1 (neuronal)), ADRA2A (adrenergic, alpha-2A-, receptor), ADRA2C (adrenergic, alpha-2C-, receptor), TACR1 (tachykinin receptor 1), or HTR2c (5-hydroxytryptamine (serotonin) receptor 2C), for example.

Examples of neurodevelopmental-associated sequences include A2BP1 [ataxin 2-binding protein 1], AADAT [aminoadipate aminotransferase], AANAT [arylalkylamine N-acetyltransferase], ABAT [4-aminobutyrate aminotransferase], ABCA1 [ATP-binding cassette, sub-family A (ABC1), member 1], or ABCA13 [ATP-binding cassette, sub-family A (ABC1), member 13], for example.

Further examples of preferred conditions treatable with the present system include may be selected from: Aicardi-Goutières Syndrome; Alexander Disease; Allan-Herndon-Dudley Syndrome; POLG-Related Disorders; Alpha-Mannosidosis (Type II and III); Alström Syndrome; Angelman; Syndrome; Ataxia-Telangiectasia; Neuronal Ceroid-Lipofuscinoses; Beta-Thalassemia; Bilateral Optic Atrophy and (Infantile) Optic Atrophy Type 1; Retinoblastoma (bilateral); Canavan Disease; Cerebrooculofacioskeletal Syndrome 1 [COFS1]; Cerebrotendinous Xanthomatosis; Cornelia de Lange Syndrome; MAPT-Related Disorders; Genetic Prion Diseases; Dravet Syndrome; Early-Onset Familial Alzheimer Disease; Friedreich Ataxia [FRDA]; Fryns Syndrome; Fucosidosis; Fukuyama Congenital Muscular Dystrophy; Galactosialidosis; Gaucher Disease; Organic Acidemias; Hemophagocytic Lymphohistiocytosis; Hutchinson-Gilford Progeria Syndrome; Mucolipidosis II; Infantile Free Sialic Acid Storage Disease; PLA2G6-Associated Neurodegeneration; Jervell and Lange-Nielsen Syndrome; Junctional Epidermolysis Bullosa; Huntington Disease; Krabbe Disease (Infantile); Mitochondrial DNA-Associated Leigh Syndrome and NARP; Lesch-Nyhan Syndrome; LIS1-Associated Lissencephaly; Lowe Syndrome; Maple Syrup Urine Disease; MECP2 Duplication Syndrome; ATP7A-Related Copper Transport Disorders; LAMA2-Related Muscular Dystrophy; Arylsulfatase A Deficiency; Mucopolysaccharidosis Types I, II or III; Peroxisome Biogenesis Disorders, Zellweger Syndrome Spectrum; Neurodegeneration with Brain Iron Accumulation Disorders; Acid Sphingomyelinase Deficiency; Niemann-Pick Disease Type C; Glycine Encephalopathy; ARX-Related Disorders; Urea Cycle Disorders; COL1A1/2-Related Osteogenesis Imperfecta; Mitochondrial DNA Deletion Syndromes; PLP1-Related Disorders; Perry Syndrome; Phelan-McDermid Syndrome; Glycogen Storage Disease Type II (Pompe Disease) (Infantile); MAPT-Related Disorders; MECP2-Related Disorders; Rhizomelic Chondrodysplasia Punctata Type 1; Roberts Syndrome; Sandhoff Disease; Schindler Disease - Type 1; Adenosine Deaminase Deficiency; Smith-Lemli-Opitz Syndrome; Spinal Muscular Atrophy; Infantile-Onset Spinocerebellar Ataxia; Hexosaminidase A Deficiency; Thanatophoric Dysplasia Type 1; Collagen Type VI-Related Disorders; Usher Syndrome Type I; Congenital Muscular Dystrophy; Wolf-Hirschhorn Syndrome; Lysosomal Acid Lipase Deficiency; and Xeroderma Pigmentosum.

As will be apparent, it is envisaged that the present system can be used to target any polynucleotide sequence of interest. Some examples of conditions or diseases that might be usefully treated using the present system are included in the Tables above and examples of genes currently associated with those conditions are also provided there. However, the genes exemplified are not exhaustive.

### EXAMPLES

The following examples are given for the purpose of illustrating various embodiments of the invention and are not meant to limit the present invention in any fashion. The present examples, along with the methods described herein are presently representative of preferred embodiments, are exemplary, and are not intended as limitations on the scope of the invention.

### Example 1: Improvement of the Cas9 system for in vivo application

Applicants conducted a Metagenomic search for a Cas9 with small molecular weight. Most Cas9 orthologs are fairly large. Many known Cas9 orthologs are large and contain more than 1300 amino acids. For example the SpCas9 is around 1368aa long, which is too large to be easily packaged into viral vectors for delivery. A graph representing the length distribution of Cas9 homologs is shown in Figure 6: The graph is generated from sequences deposited in GenBank. Some of the sequences may have been mis-annotated and therefore the exact frequency for each length may not necessarily be accurate. Nevertheless it provides a glimpse at distribution of Cas9 proteins and suggest that there are shorter Cas9 homologs.

Through computational analysis, Applicants found that in the bacterial strain Campylobacter, there are two Cas9 proteins with less than 1000 amino acids. The sequence for one Cas9 from *Campylobacter jejuni* is presented below. At this length, CjCas9 can be easily packaged into AAV, lentiviruses, Adenoviruses, and other viral vectors for robust delivery into primary cells and in vivo animal models. In a preferred embodiment of the invention, the Cas9 protein from *S. aureus* is used.

Campylobacter jejuni Cas9 (CjCas9)

The putative tracrRNA element for this CjCas9 is:

The Direct Repeat sequence is:
GTTTTAGTCCCTTTTTAAATTTCTTTATGGTAAAAT

An example of a chimeric guideRNA for CjCas9 is:

### Example 2: Cas9 diversity and chimeric RNAs

The wild-type CRISPR-Cas system is an adaptive immune mechanism against invading exogenous DNA employed by diverse species across bacteria and archaea. The type II CRISPR-Cas system consists of a set of genes encoding proteins responsible for the "acquisition" of foreign DNA into the CRISPR locus, as well as a set of genes encoding the "execution" of the DNA cleavage mechanism; these include the DNA nuclease (Cas9), a noncoding transactivating cr-RNA (tracrRNA), and an array of foreign DNA-derived spacers flanked by direct repeats (crRNAs). Upon maturation by Cas9, the tracRNA and crRNA duplex guide the Cas9 nuclease to a target DNA sequence specified by the spacer guide sequences, and mediates double-stranded breaks in the DNA near a short sequence motif in the target DNA that is required for cleavage and specific to each CRISPR-Cas system. The type II CRISPR-Cas systems are found throughout the bacterial kingdom and highly diverse in Cas9 protein sequence and size, tracrRNA and crRNA direct repeat sequence, genome organization of these elements, and the motif requirement for target cleavage. One species may have multiple distinct CRISPR-Cas systems.

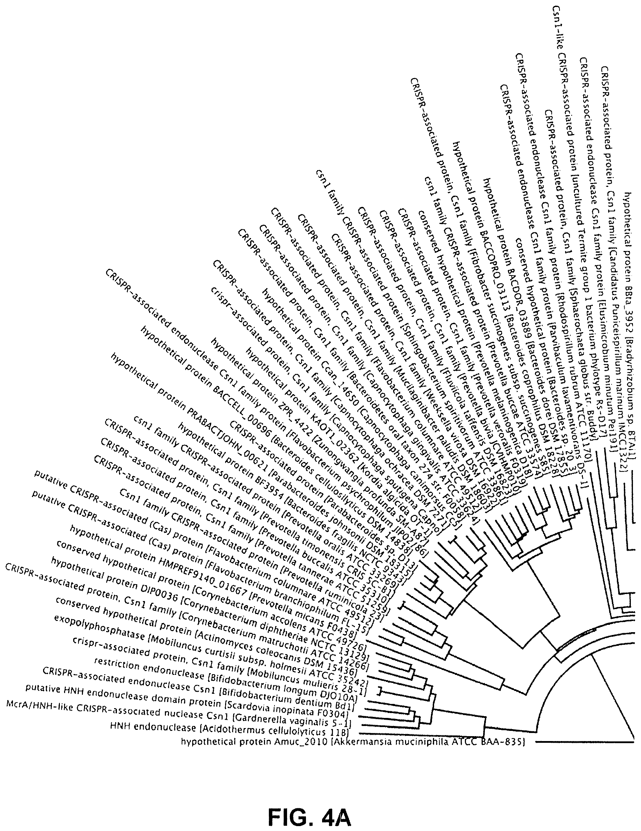

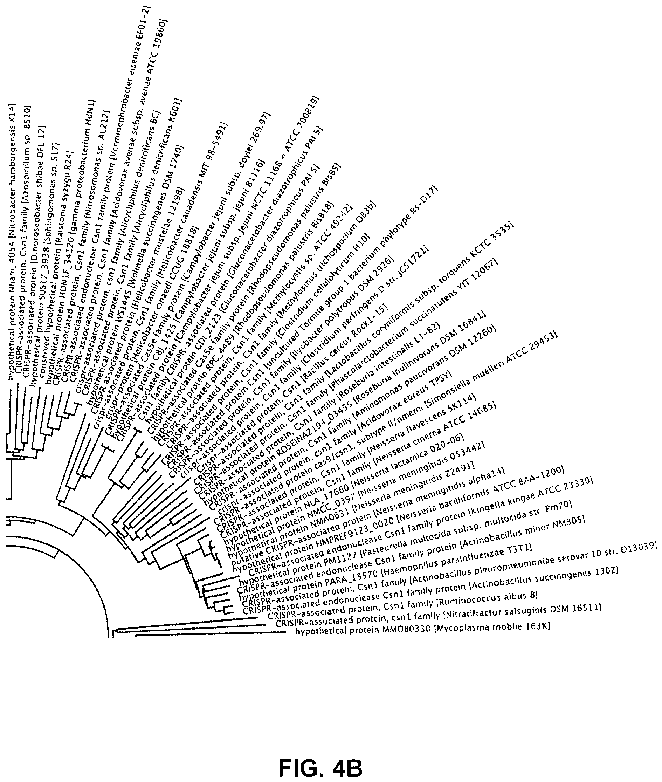

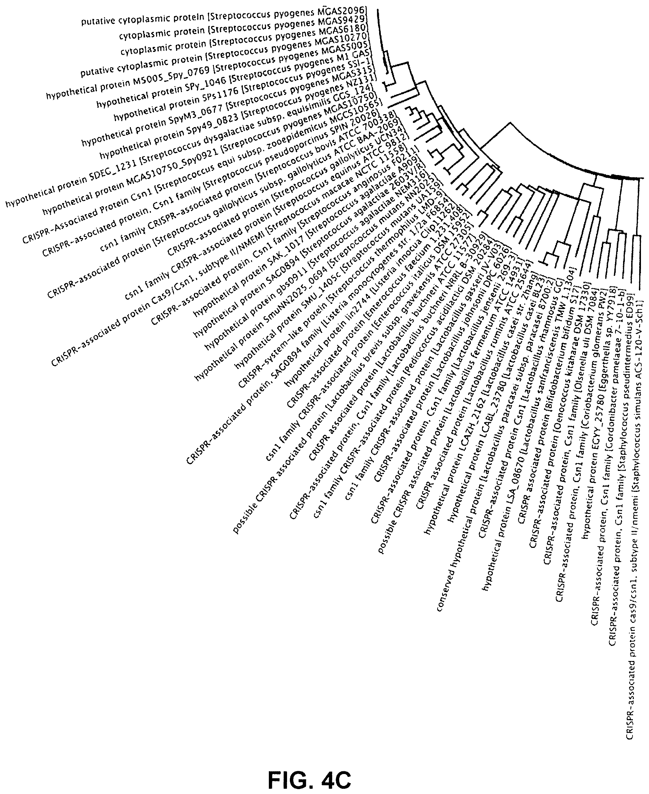

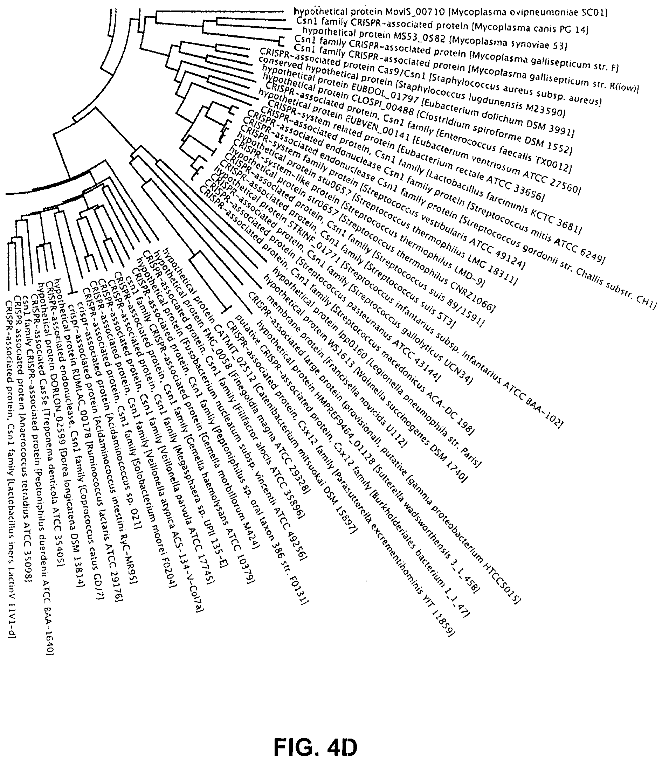

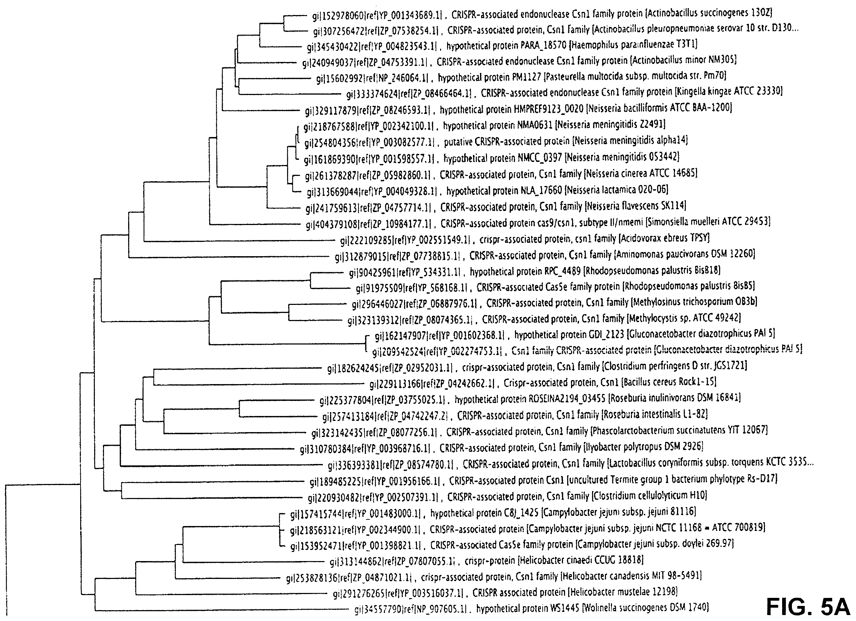

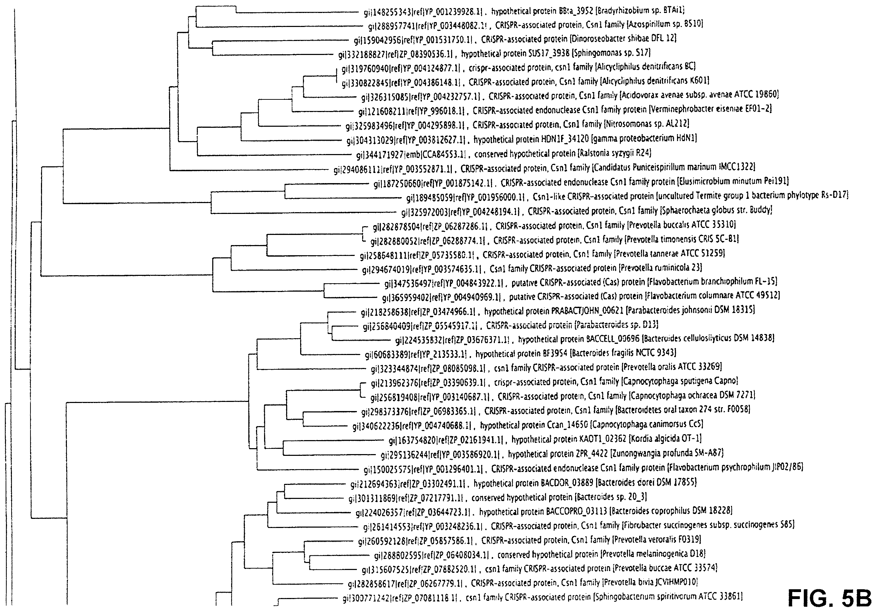

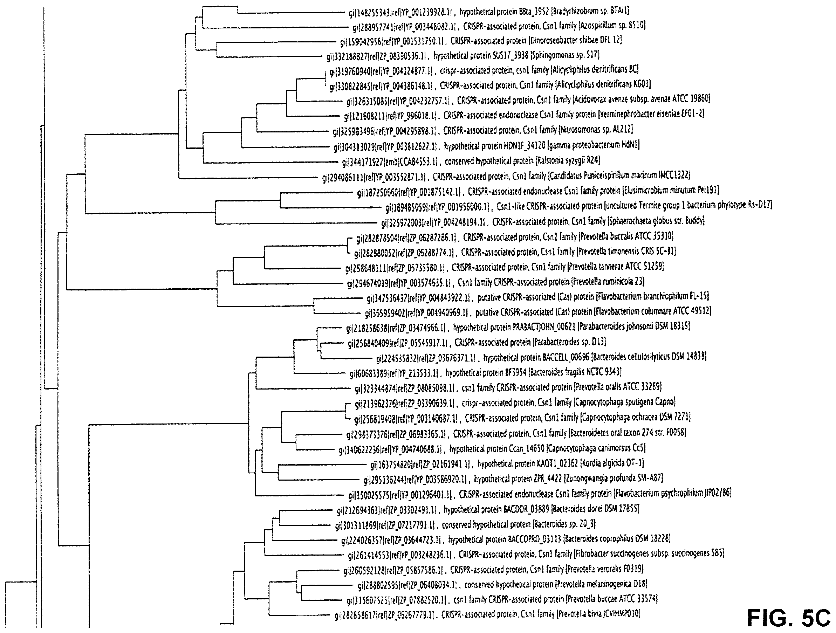

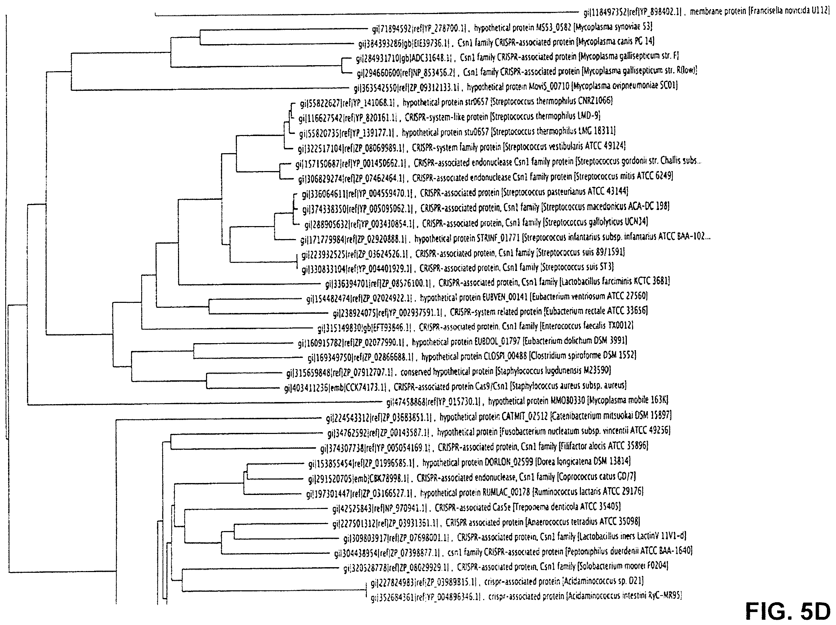

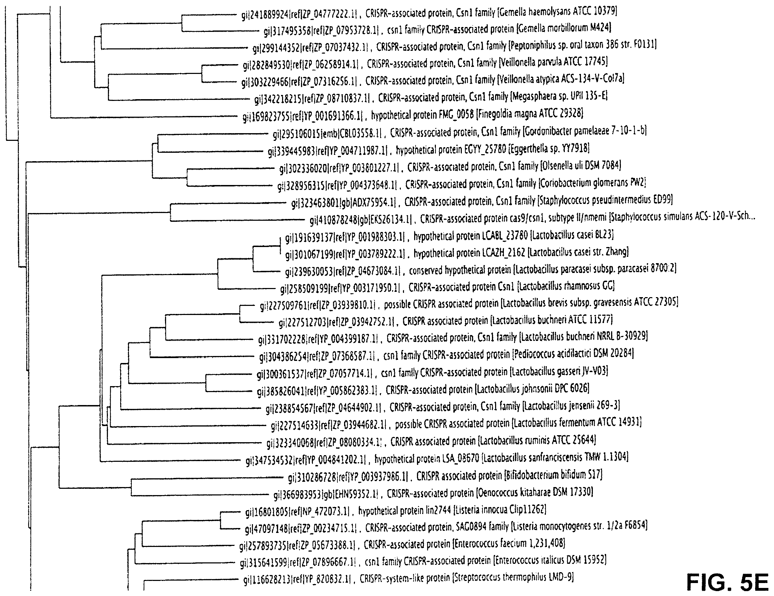

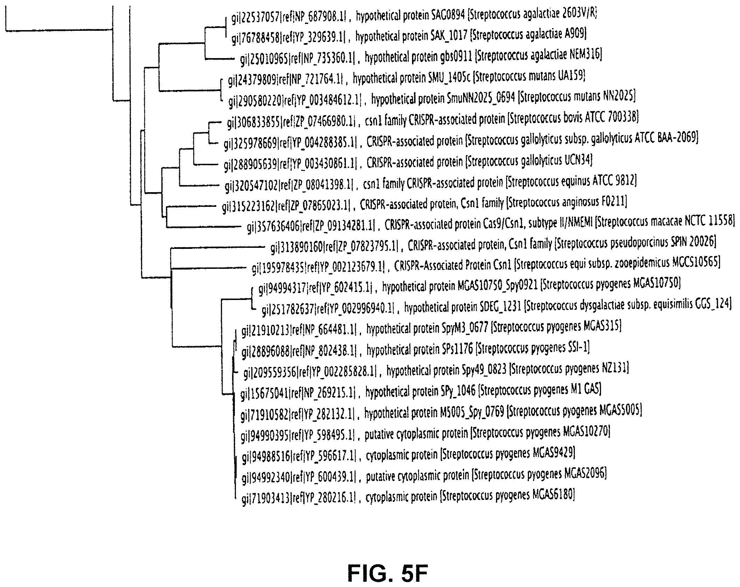

Applicants evaluated 207 putative Cas9s from bacterial species identified based on sequence homology to known Cas9s and structures orthologous to known subdomains, including the HNH endonuclease domain and the RuvC endonuclease domains [information from the Eugene Koonin and Kira Makarova]. Phylogenetic analysis based on the protein sequence conservation of this set revealed five families of Cas9s, including three groups of large Cas9s (∼1400 amino acids) and two of small Cas9s (∼1100 amino acids) (Figures 4 and 5A-F).

In some embodiments, the tracr mate sequences or the direct repeats are either downloaded from the CRISPRs database or identified in silico by searching for repetitive motifs that are 1. found in a 2kb window of genomic sequence flanking the type II CRISPR locus, 2. span from 20 to 50 bp, and 3. interspaced by 20 to 50 bp. In some embodiments, 2 of these criteria may be used, for instance 1 and 2, 2 and 3, or 1 and 3. In some embodiments, all 3 criteria may be used. In some embodiments candidate tracrRNA are subsequently predicted by 1. sequence homology to direct repeats (motif search in Geneious with up to 18-bp mismatches), 2. presence of a predicted Rho-independent transcriptional terminator in direction of transcription, and 3. stable hairpin secondary structure between tracrRNA and direct repeat. In some embodiments, 2 of these criteria may be used, for instance 1 and 2, 2 and 3, or 1 and 3. In some embodiments, all 3 criteria may be used. In some embodiments, chimeric synthetic guide RNAs (sgRNAs) designs incorporate at least 12 bp of duplex structure between the direct repeat and tracrRNA.

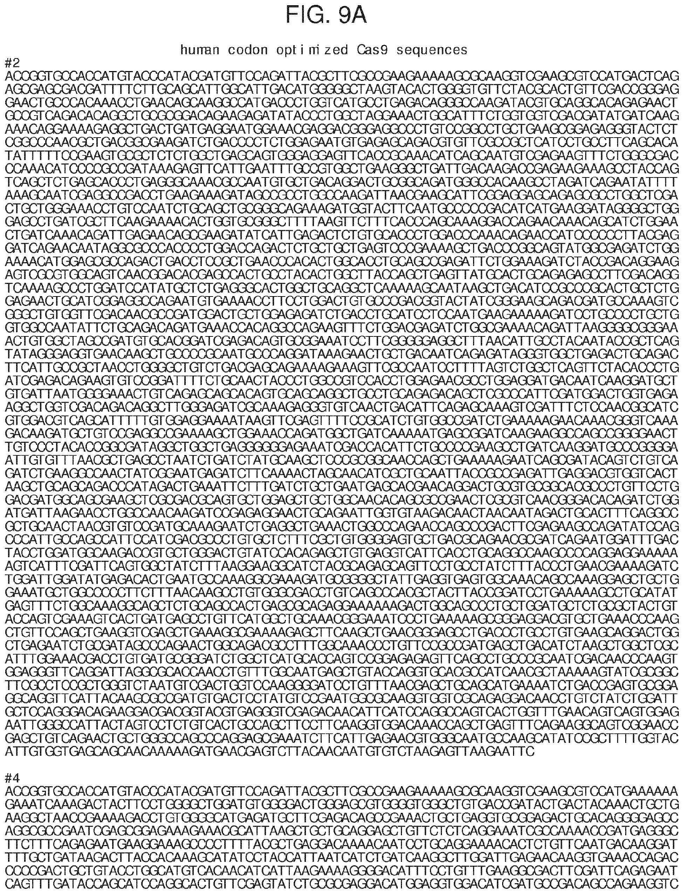

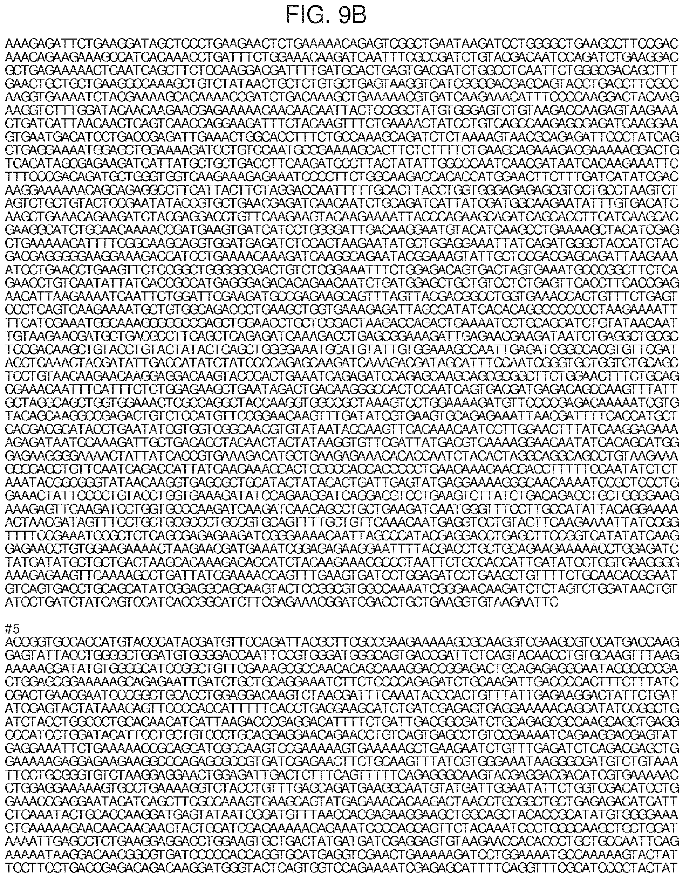

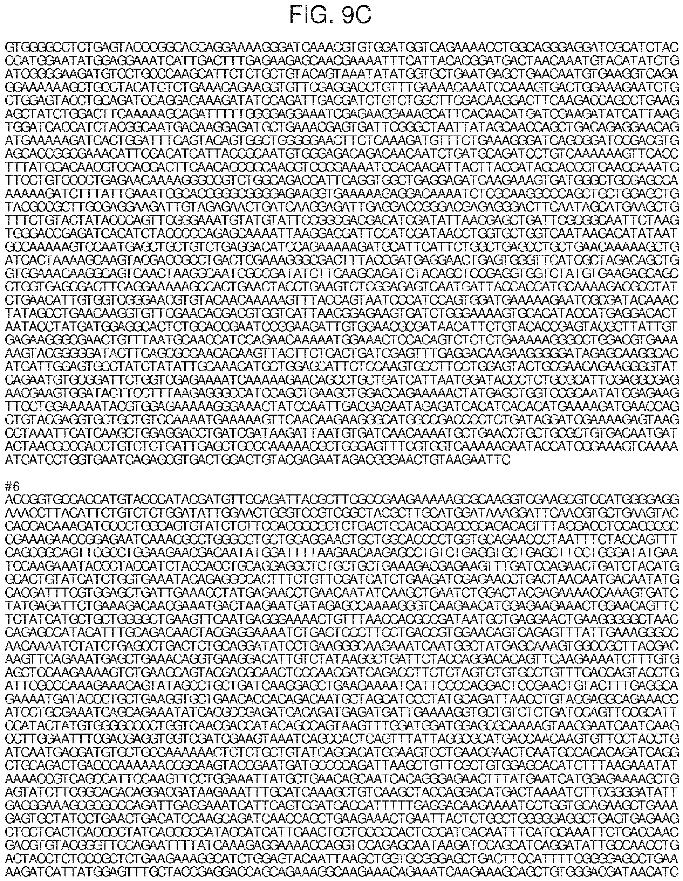

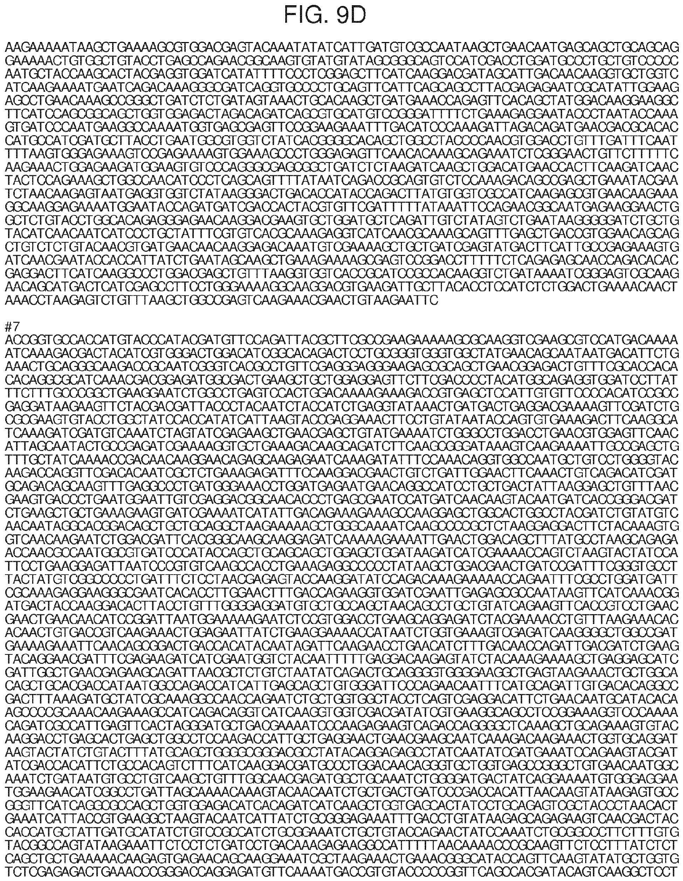

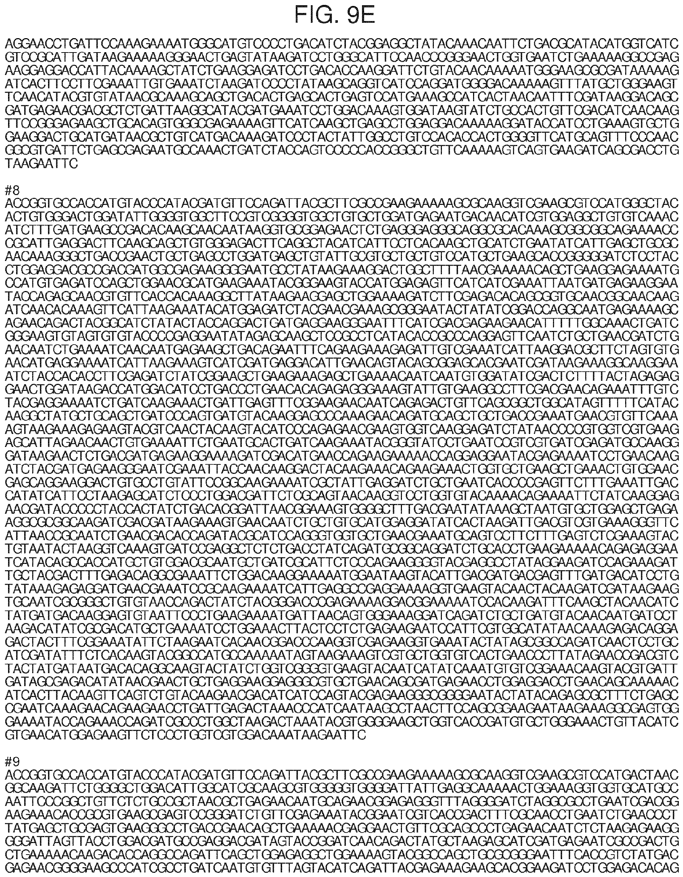

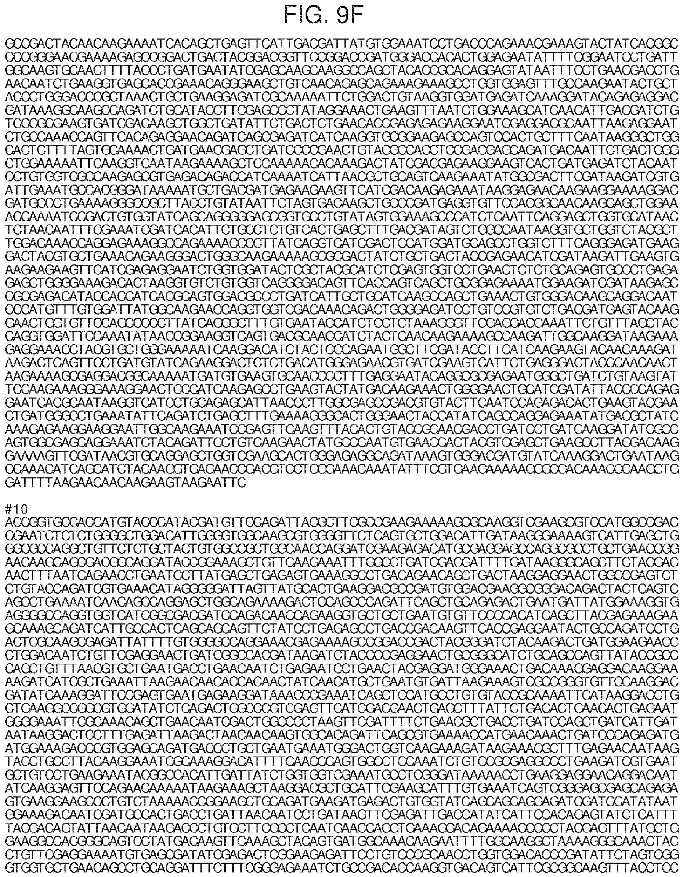

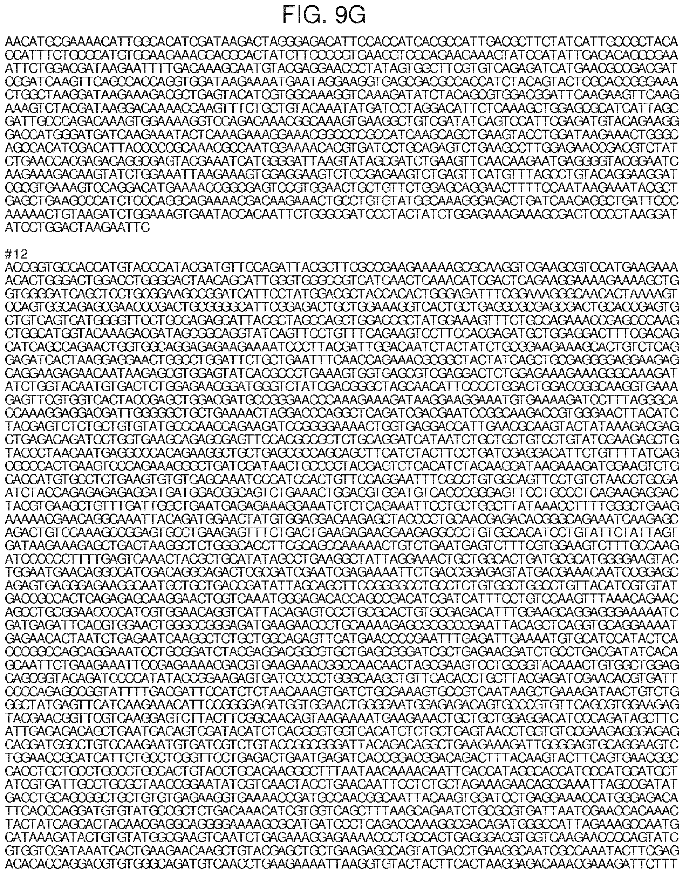

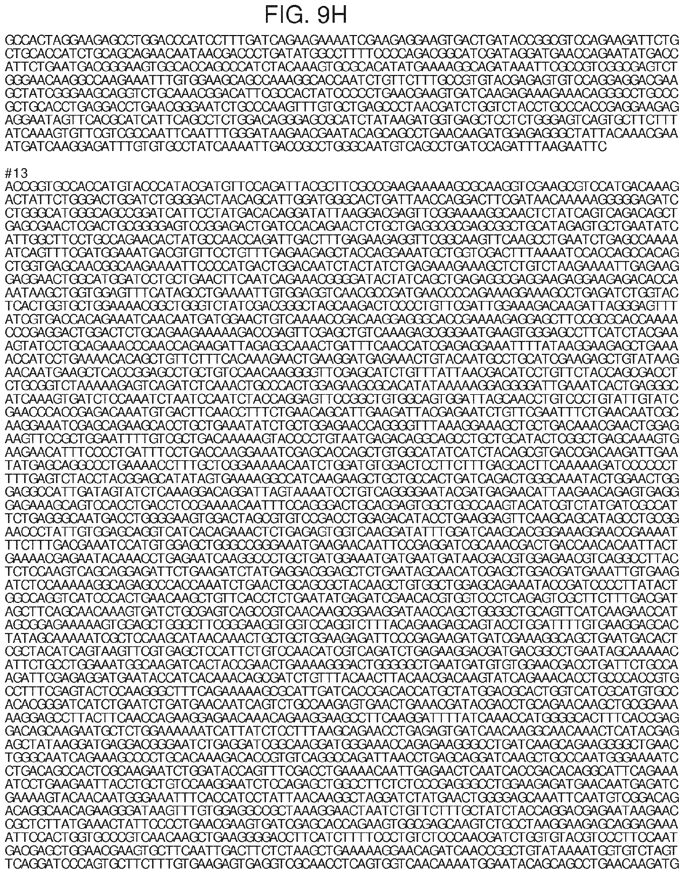

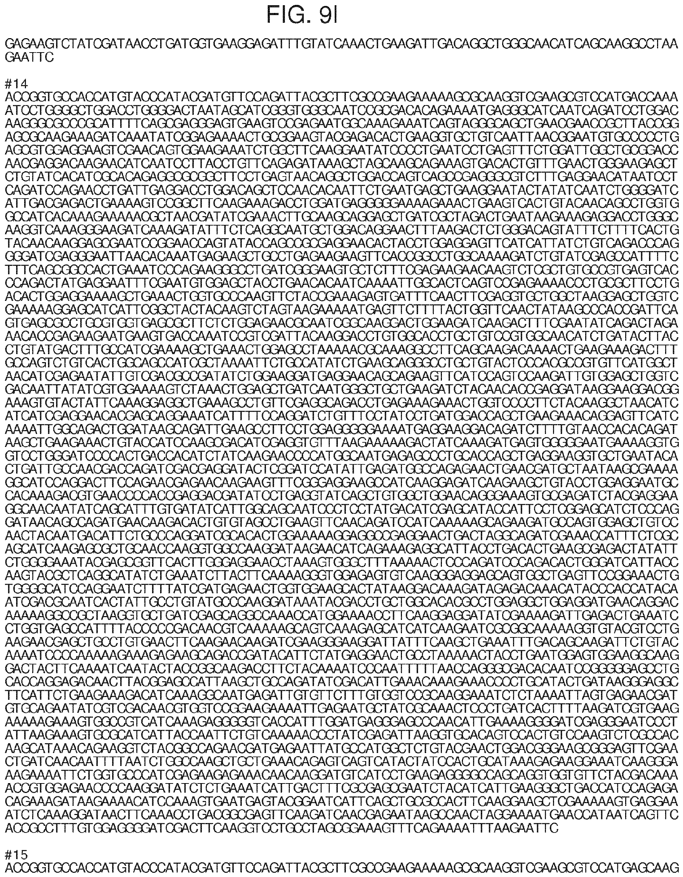

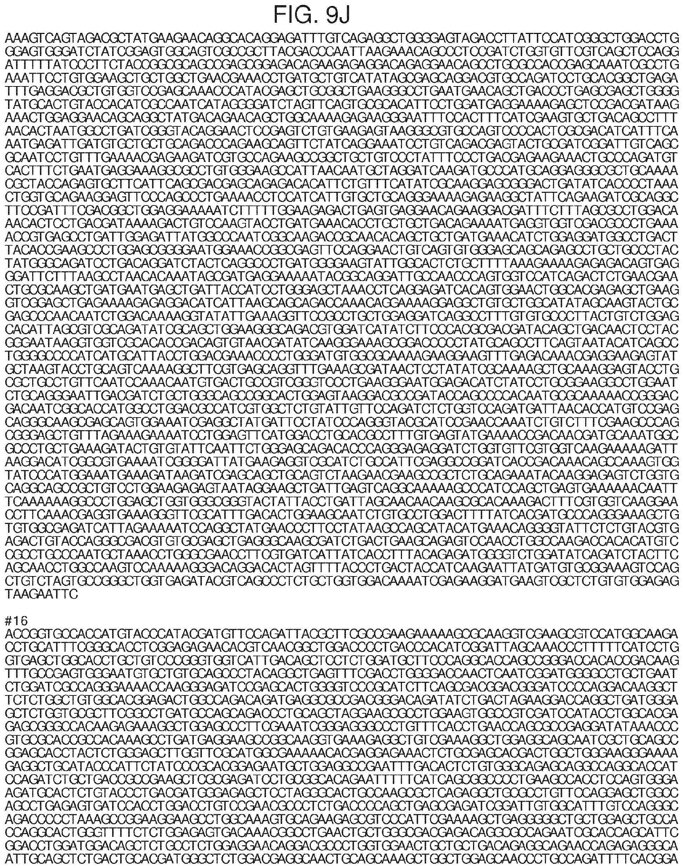

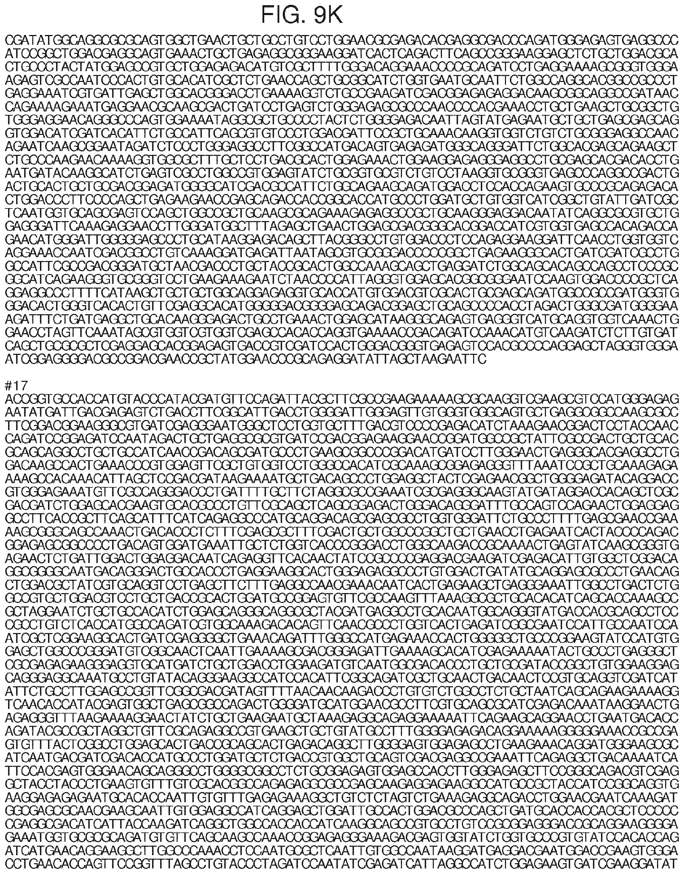

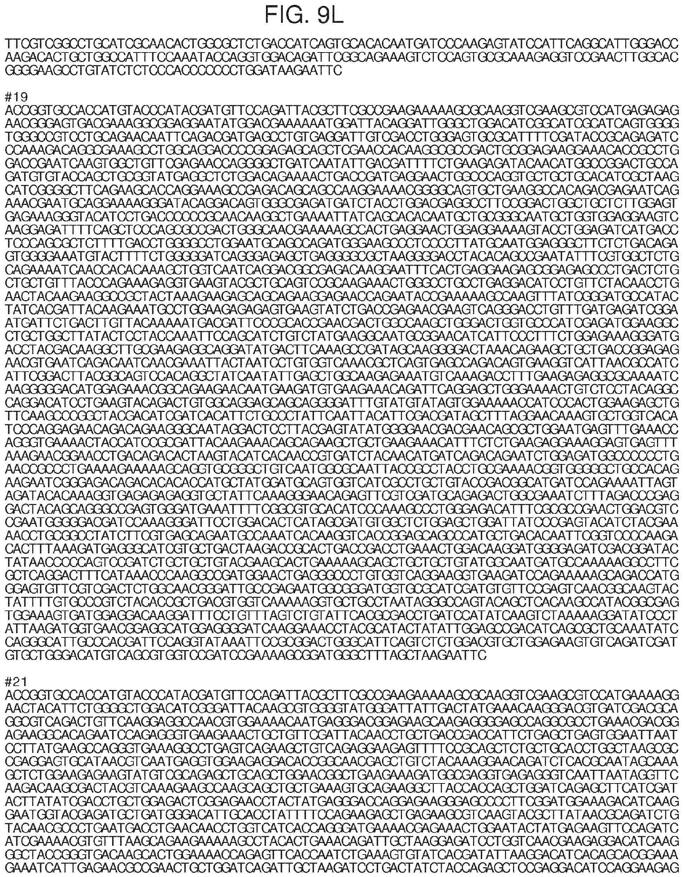

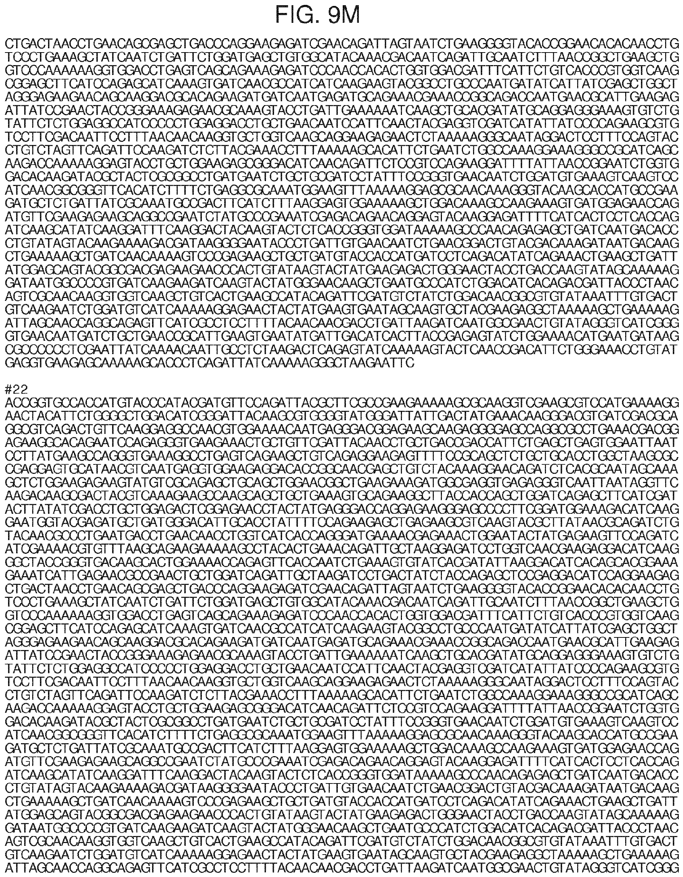

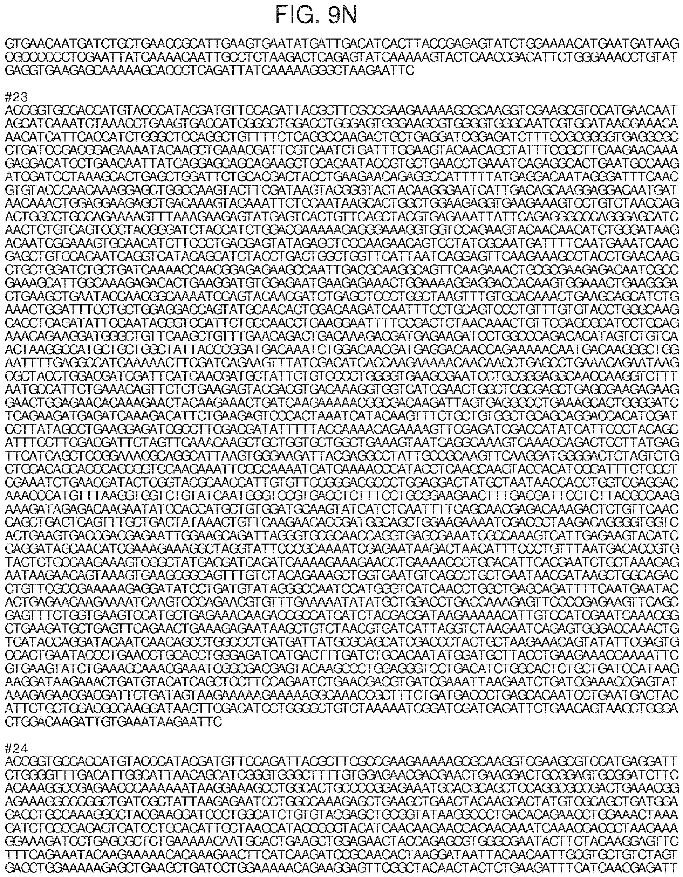

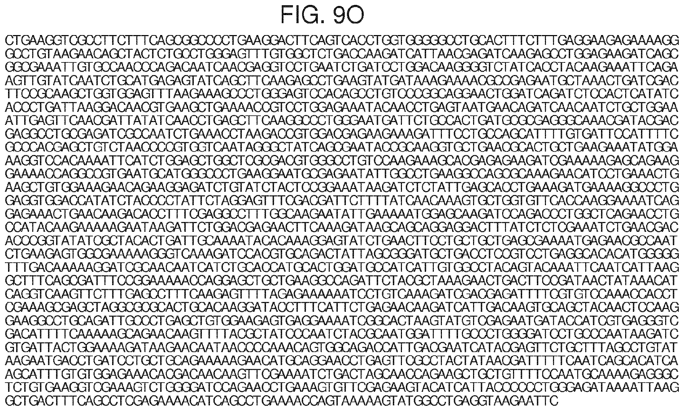

The list of the human codon optimized Cas9 ortholog sequences to pair with the chimeric RNAs provided in Figures 8A-J is provided in Figures 9A-O. Applicants have also shown that the Cas9 orthologs can cleave their targets in in vitro cleavage assays (Figure 16). The CRISPR loci in some of these families is depicted in Figure 11. The corresponding guide RNA sequences are shown in Figure 12. Applicants systematically analyzed the genomic DNA sequence within ∼2kb of the Cas9 proteins using custom computational analysis code and identified direct repeats ranging from 35bp to 50bp, with intervening spacers ranging from 29bp to 35bp. Based on the direct repeat sequence, Applicants computationally searched for tracrRNA candidate sequences with the following criteria: outside the crRNA array but containing high degree of homology to direct repeats (as required for direct repeat:tracrRNA base-pairing; custom computational analysis), outside the coding regions of the protein components, containing Rho-independent transcriptional termination signals ∼60bp-120bp downstream from region of homology from with direct repeats, and co-folding with direct repeat to form a duplex, followed by two or more hairpin structures in the distal end of tracrRNA sequence. Based on these prediction criteria, Applicants selected an initial set of 18 Cas9 proteins and their uniquely associated direct repeats and tracrRNAs distributed across all five Cas9 families. Applicants further generated a set of 18 chimeric RNA structures that preserved the sequence and secondary structures of the native direct repeat:tracrRNA duplex while shortening the region of base-pairing and fusing the two RNA elements through an artificial loop (Figures 8A-J).

### Example 3: Cas9 orthologs

Applicants have generated codon optimized Cas9 orthologs to advance expression in eukaryotic cells.

Example of a human codon optimized sequence (i.e. being optimized for expression in humans) sequence : SaCas9 : is provided below

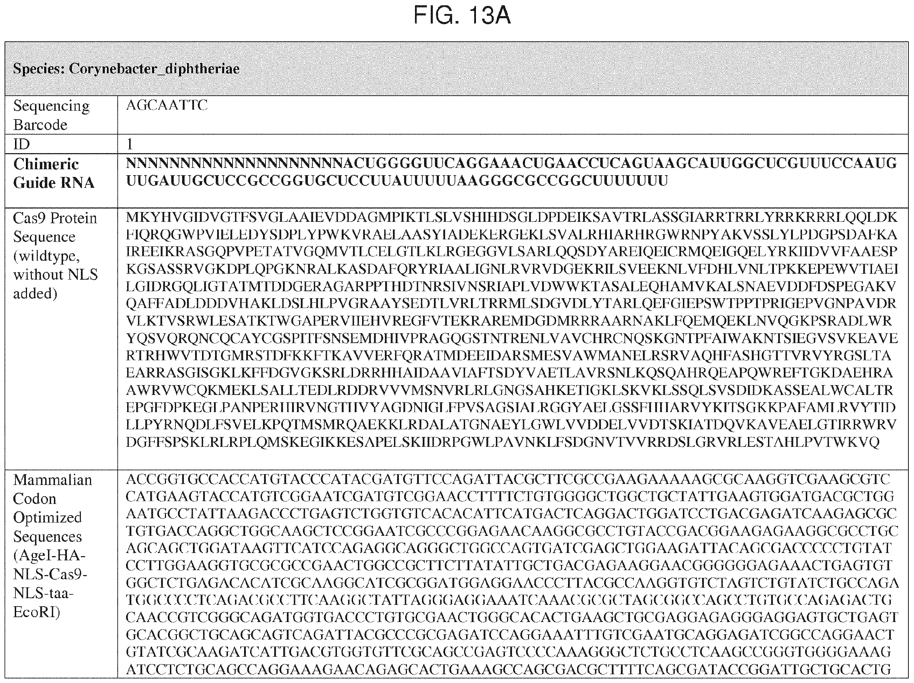

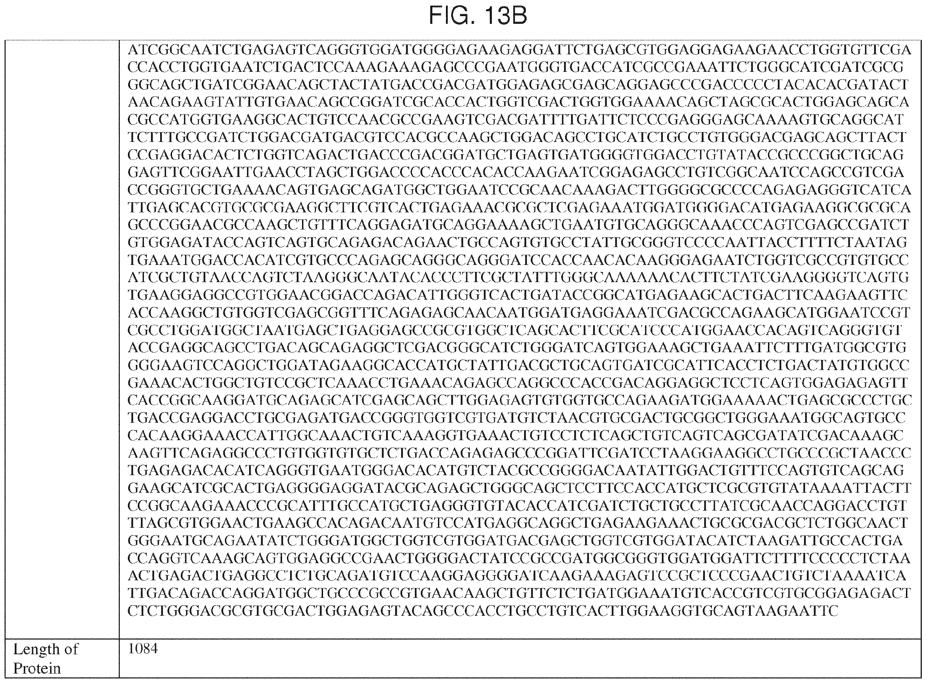

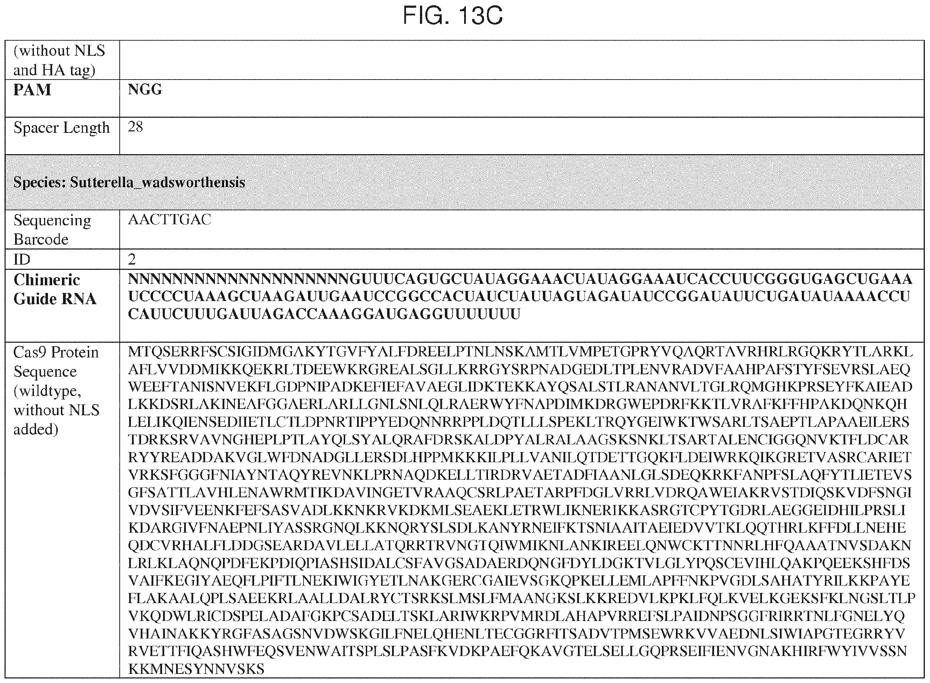

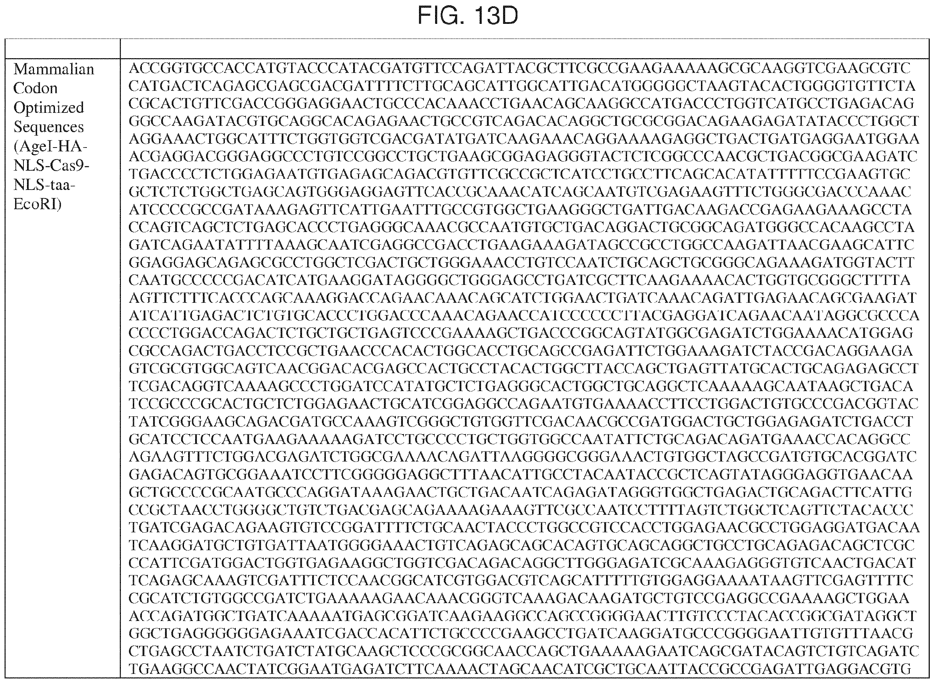

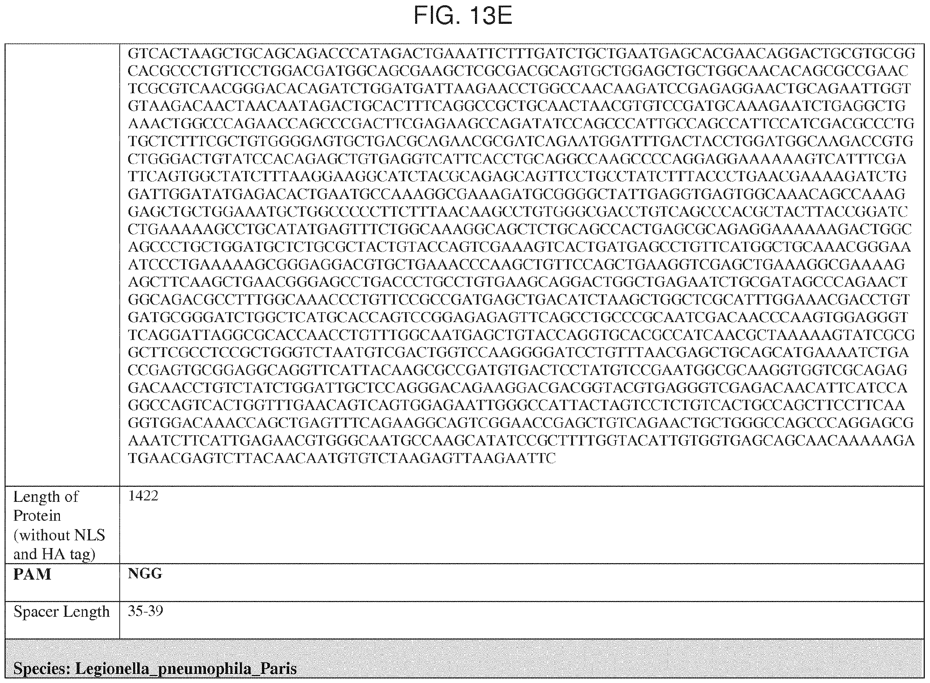

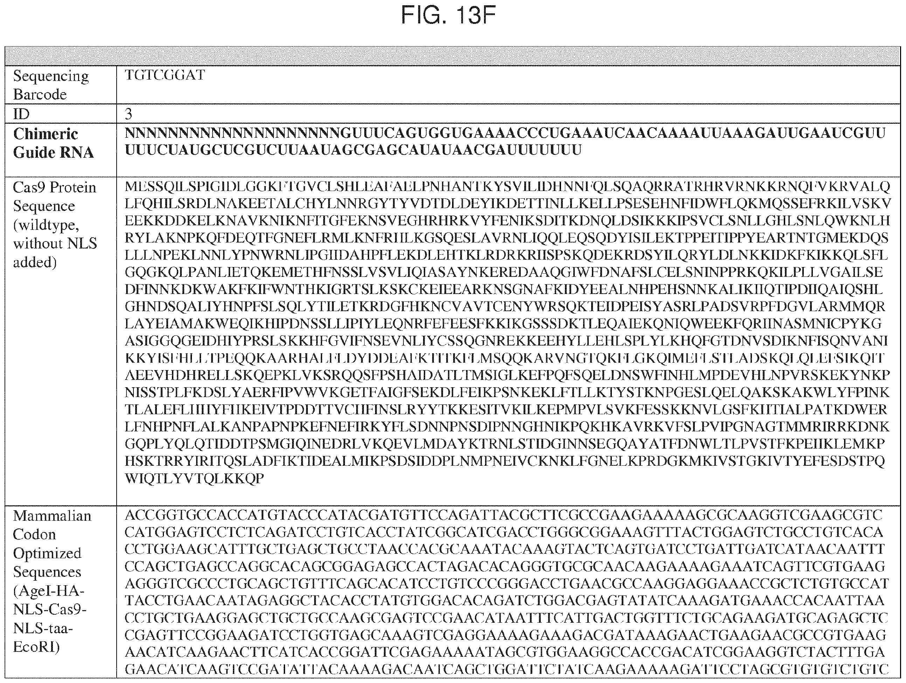

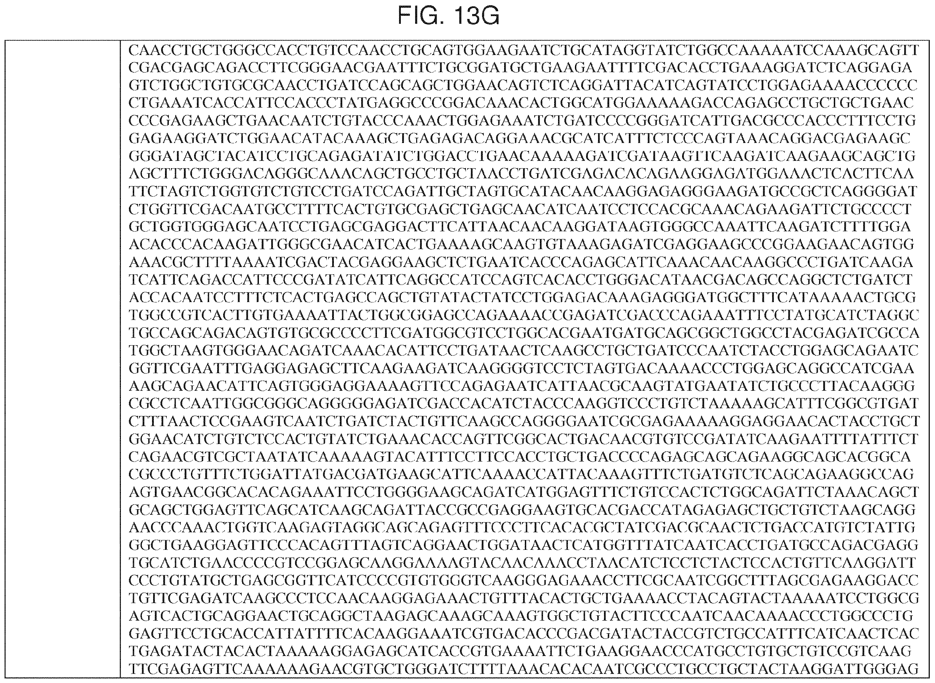

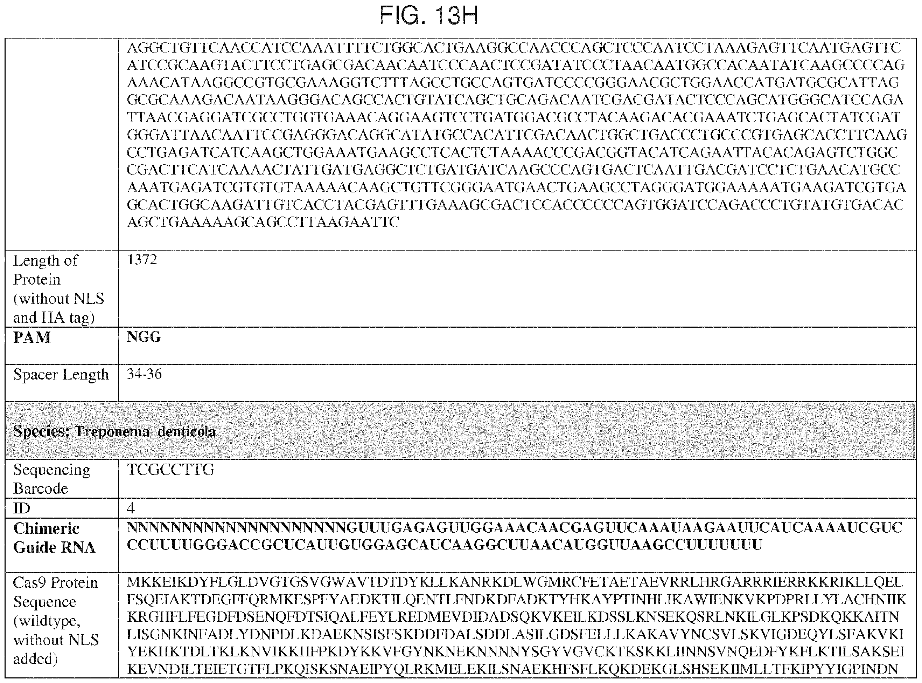

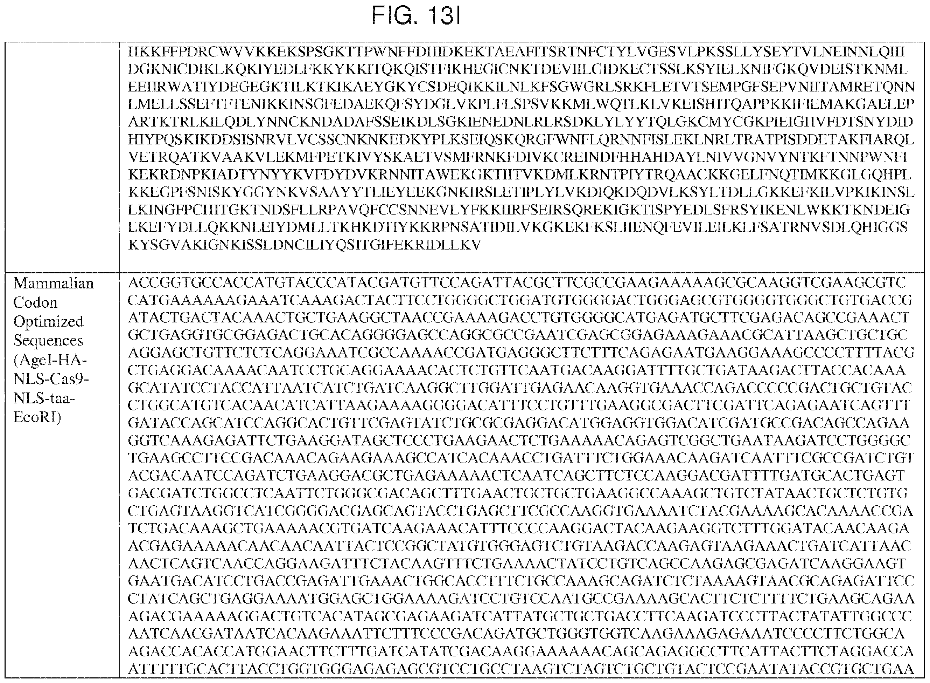

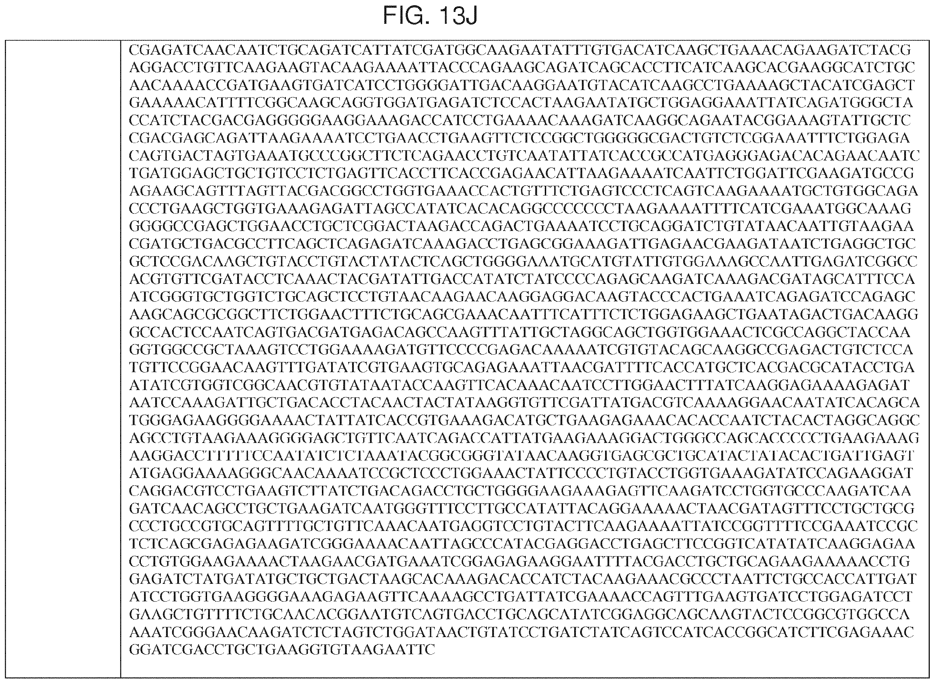

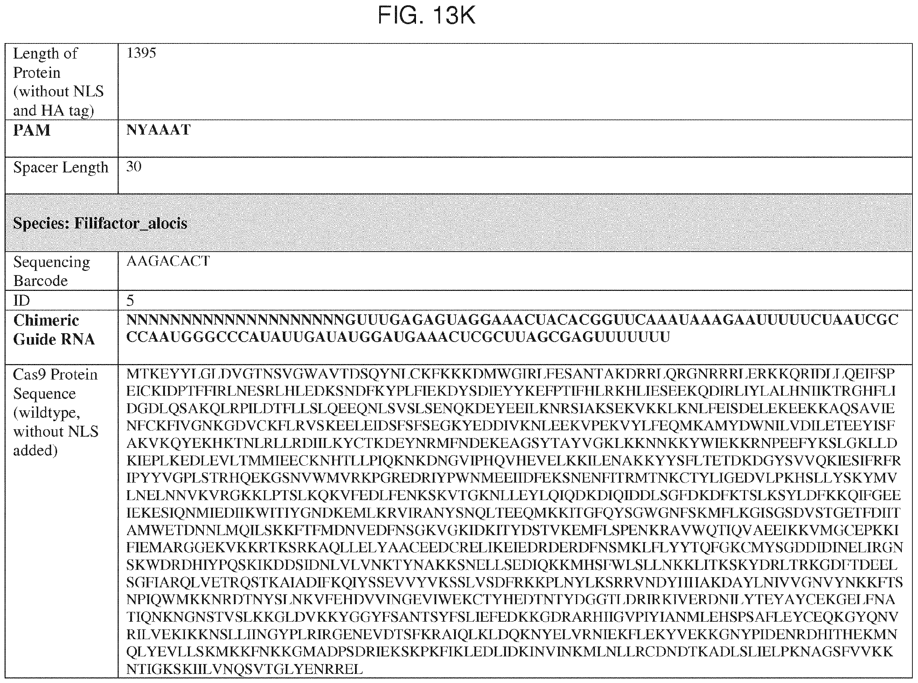

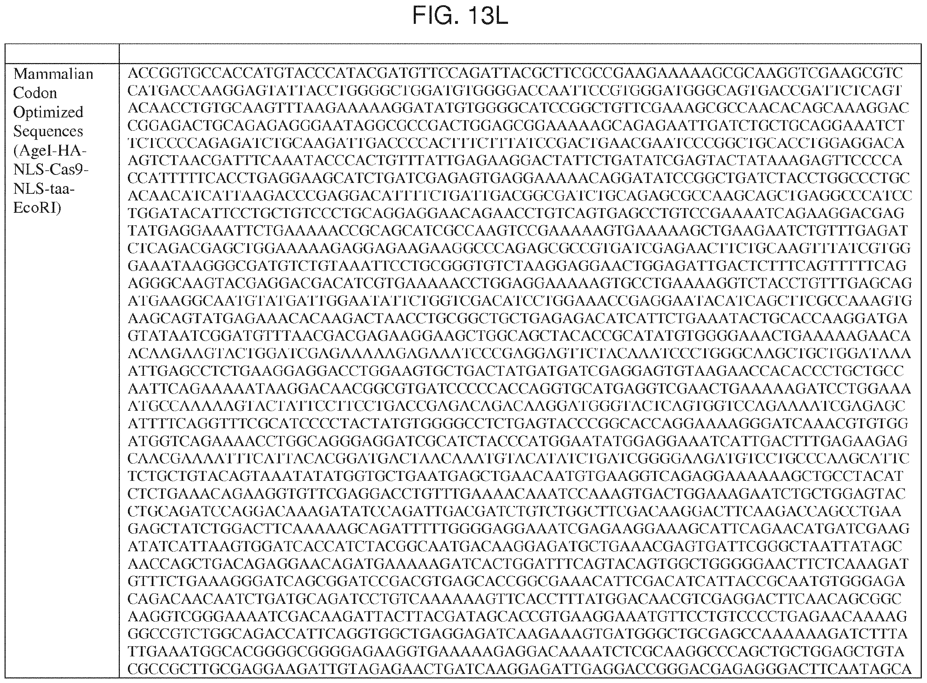

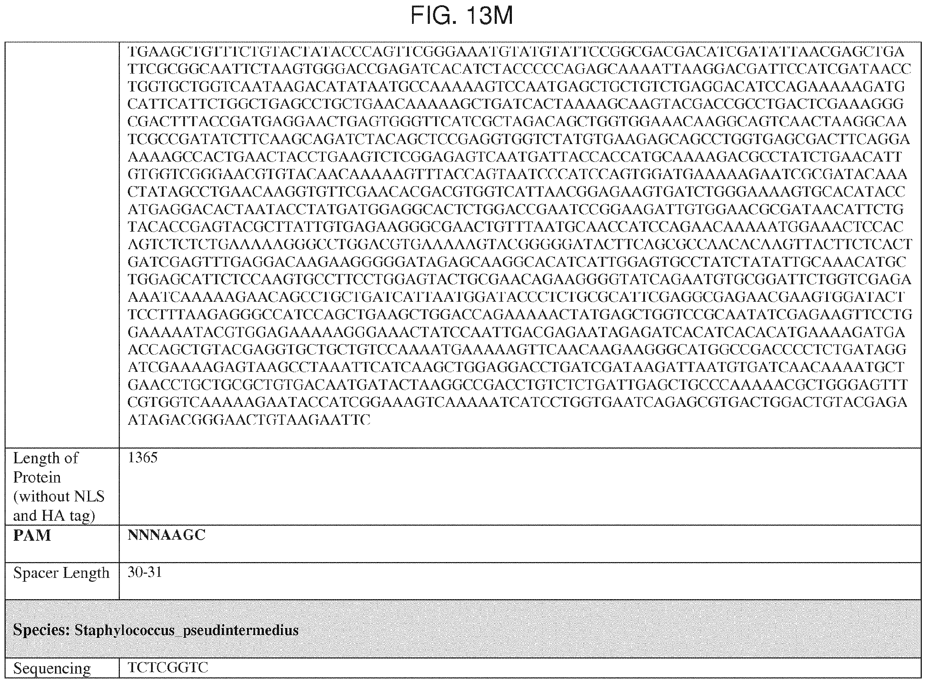

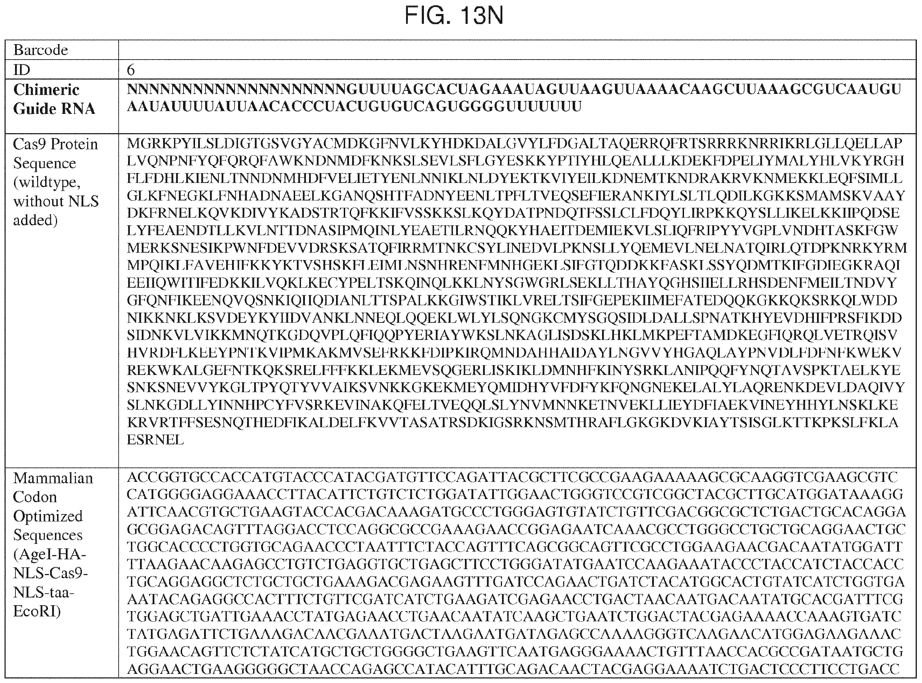

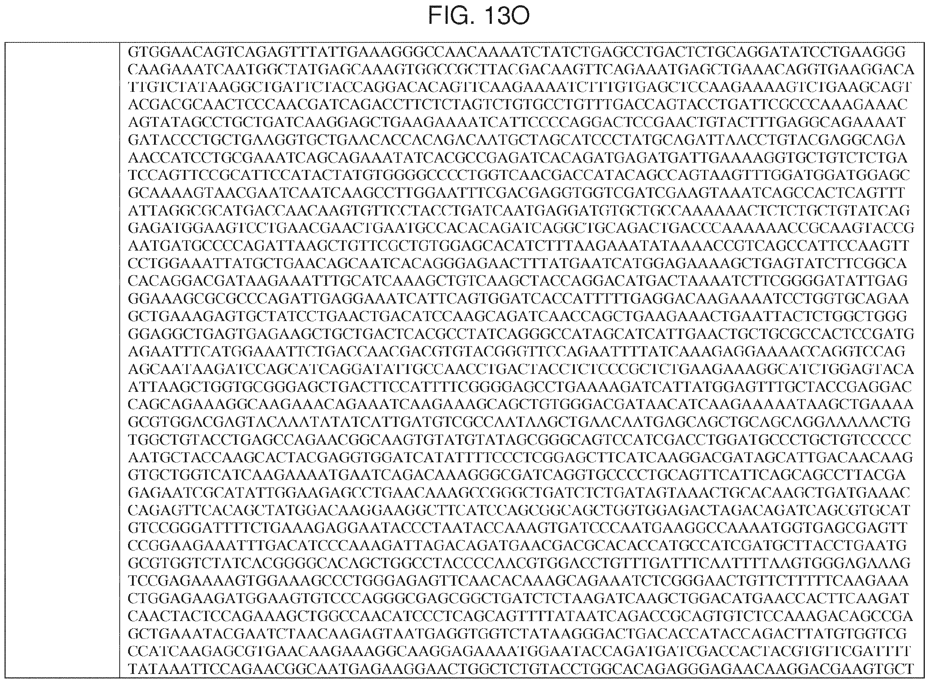

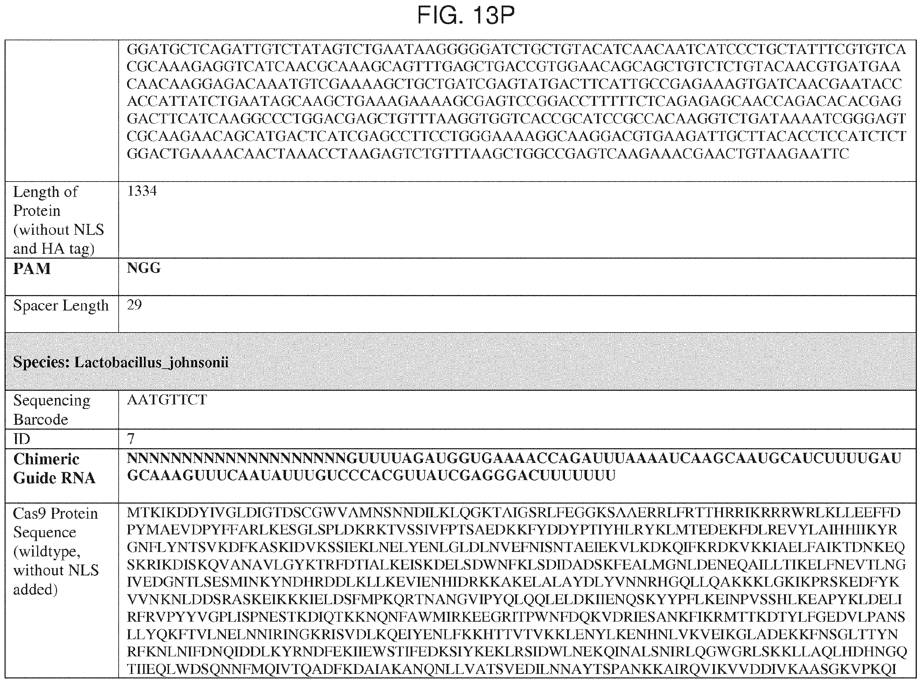

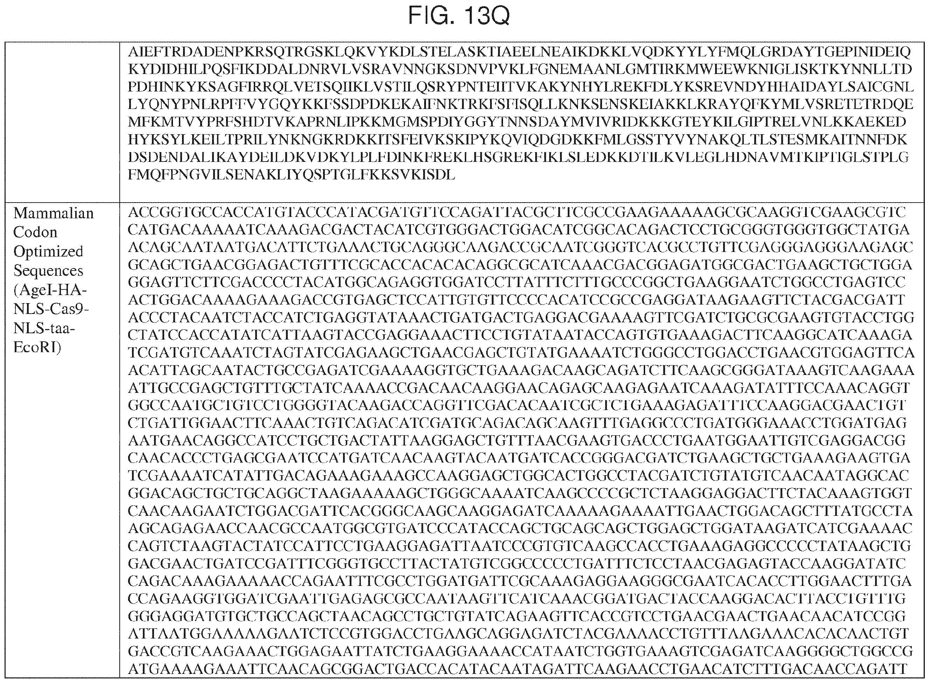

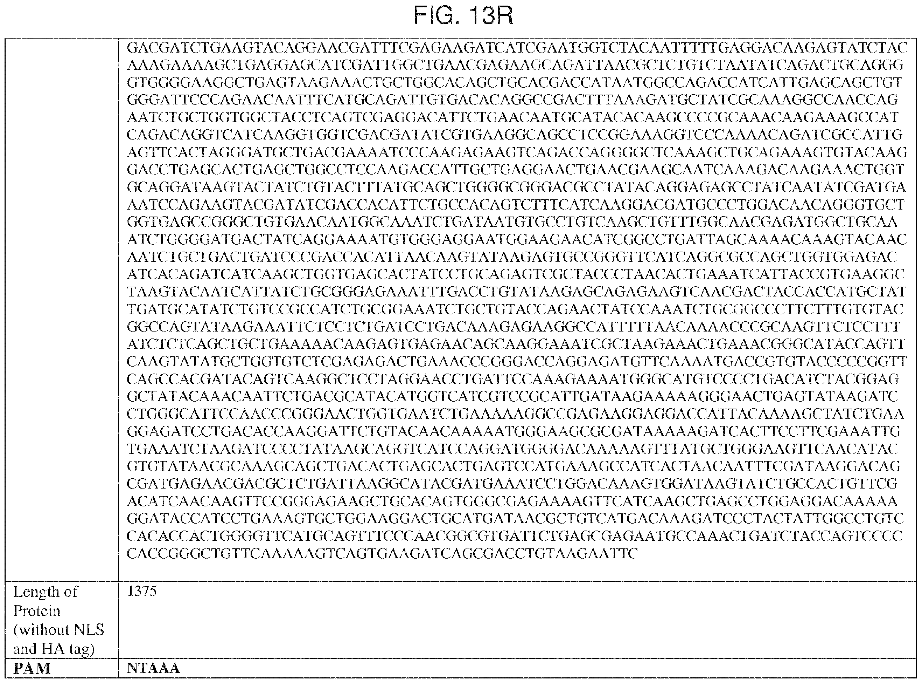

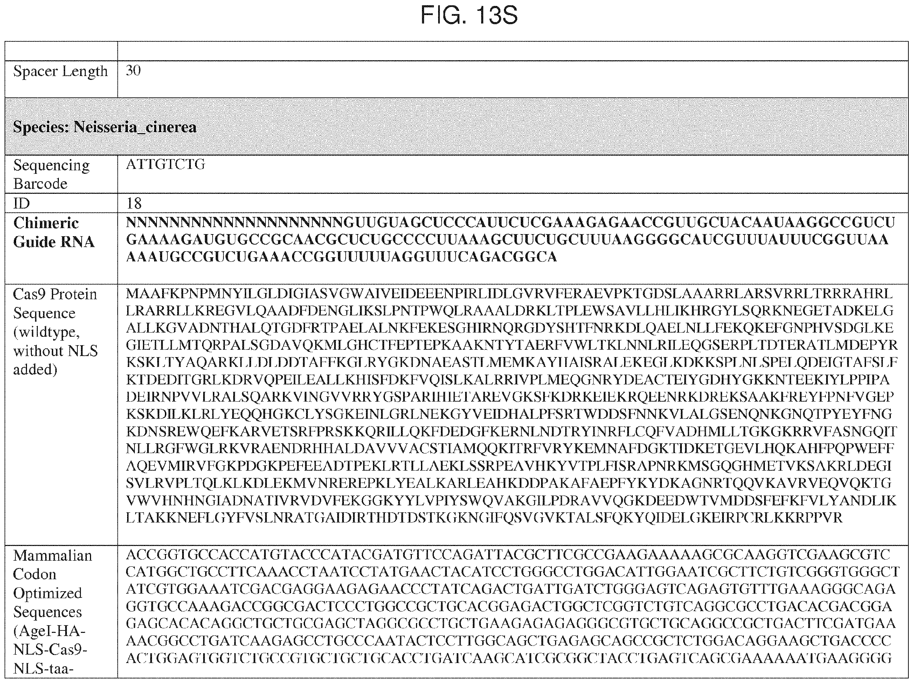

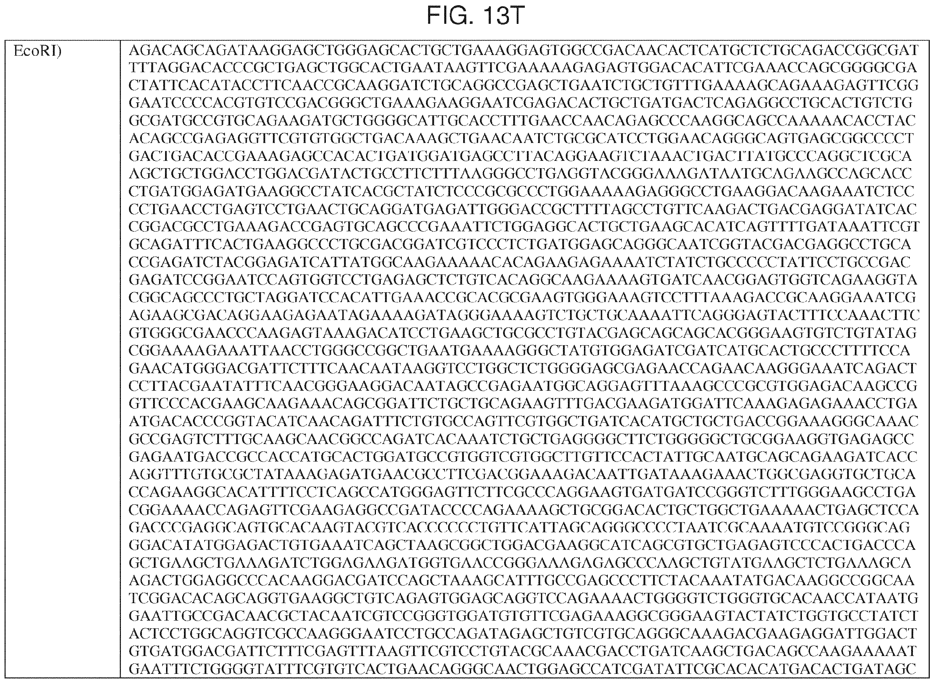

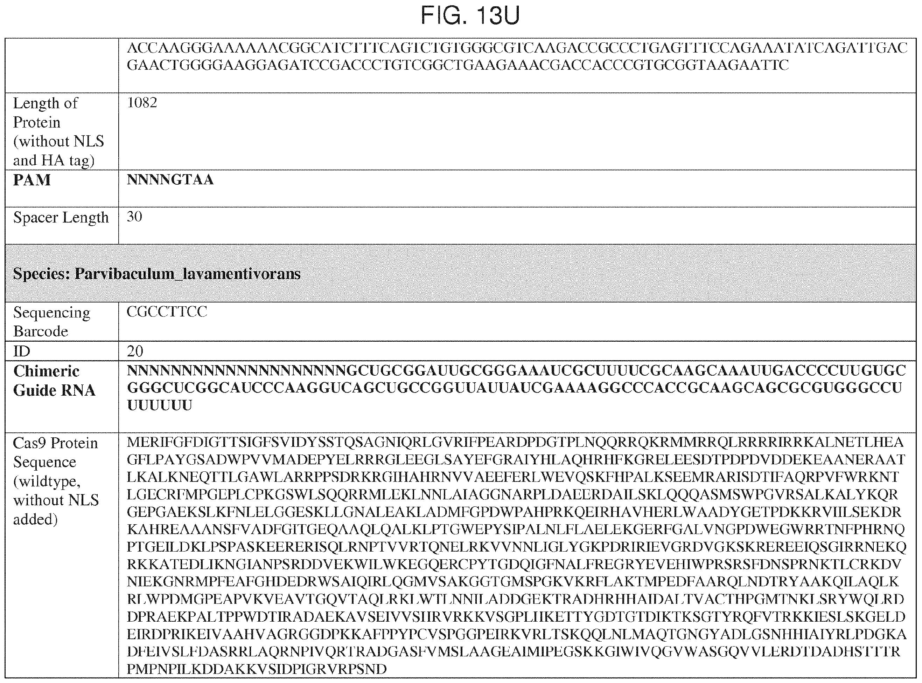

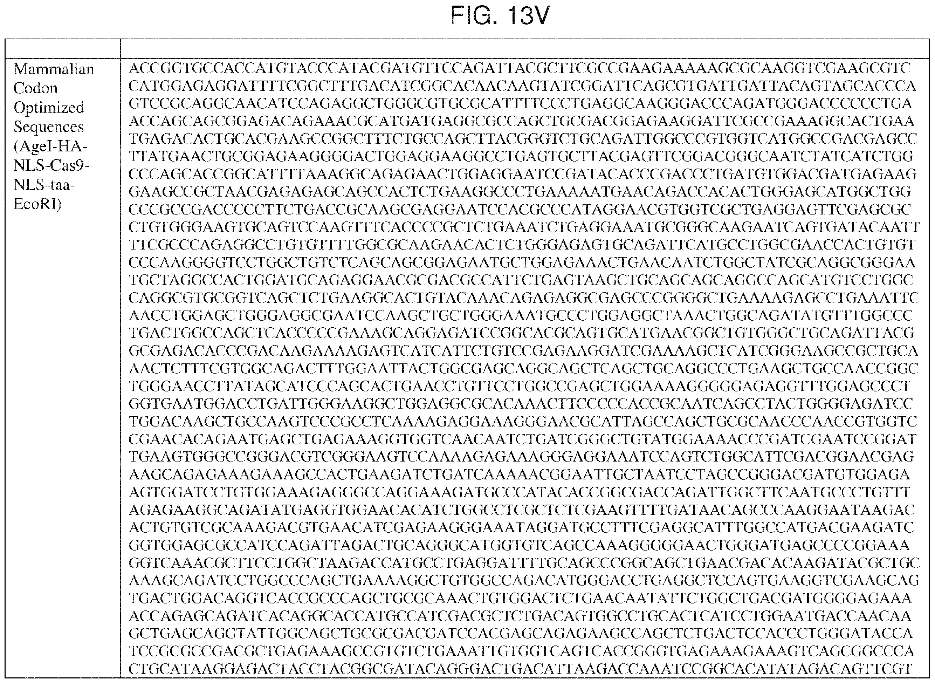

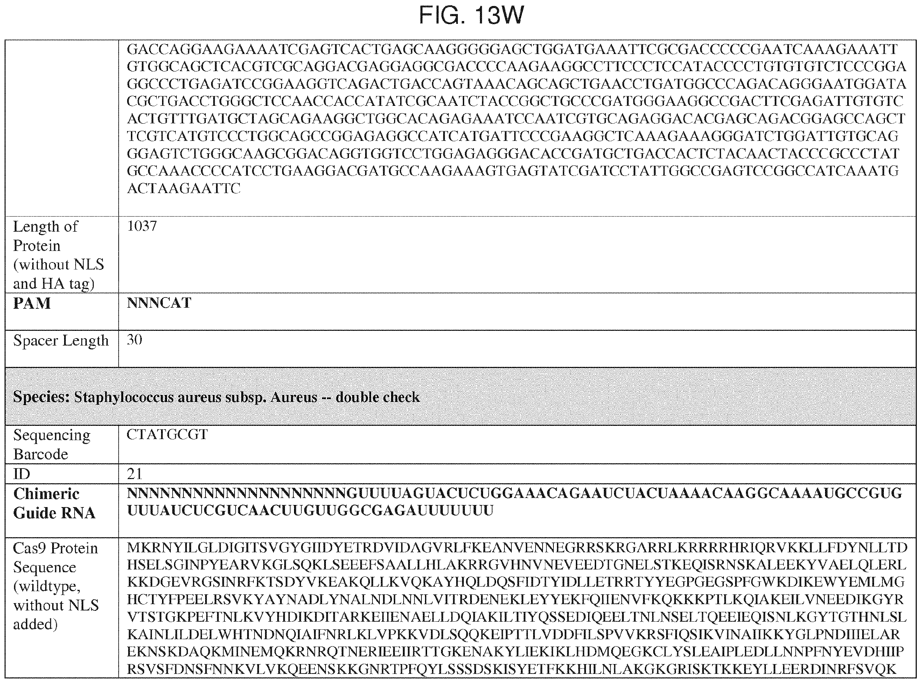

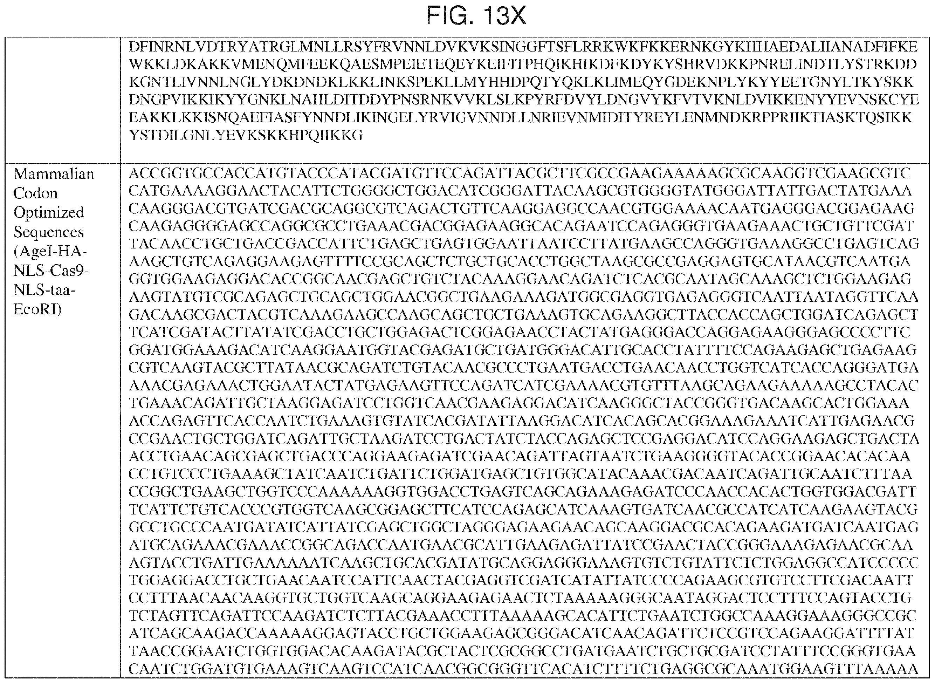

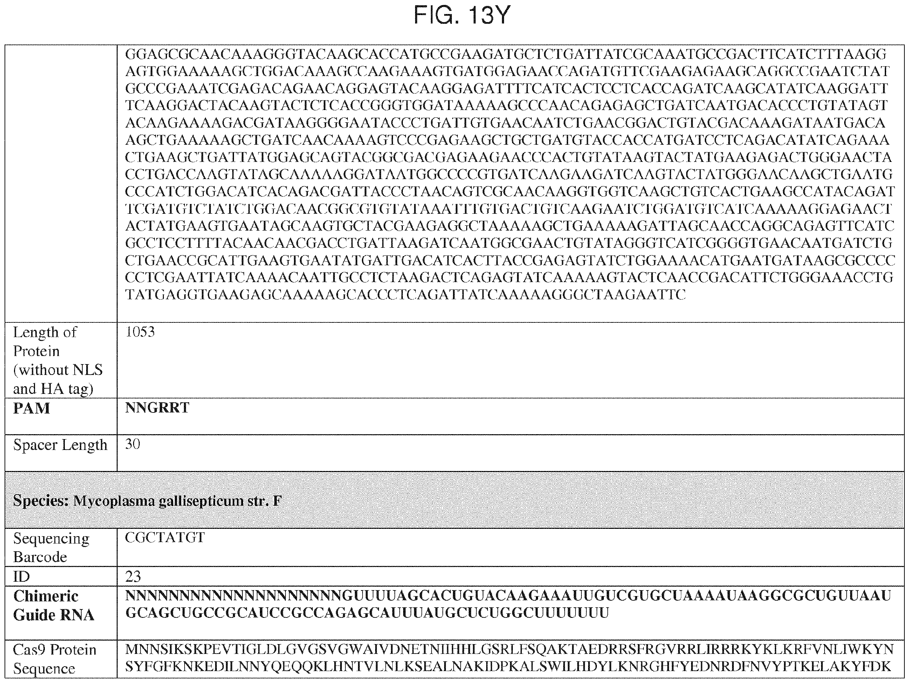

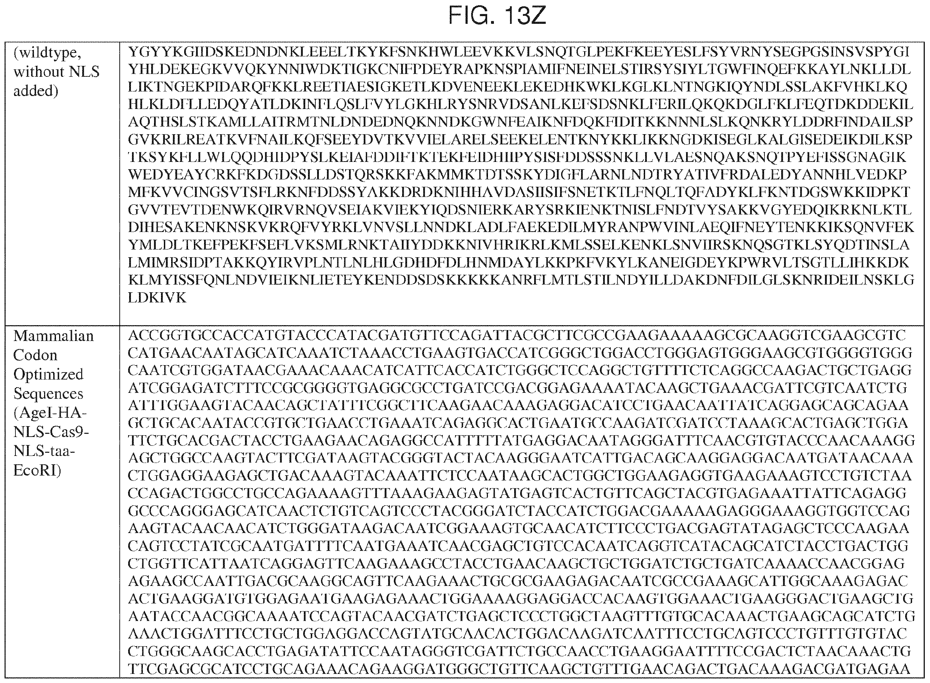

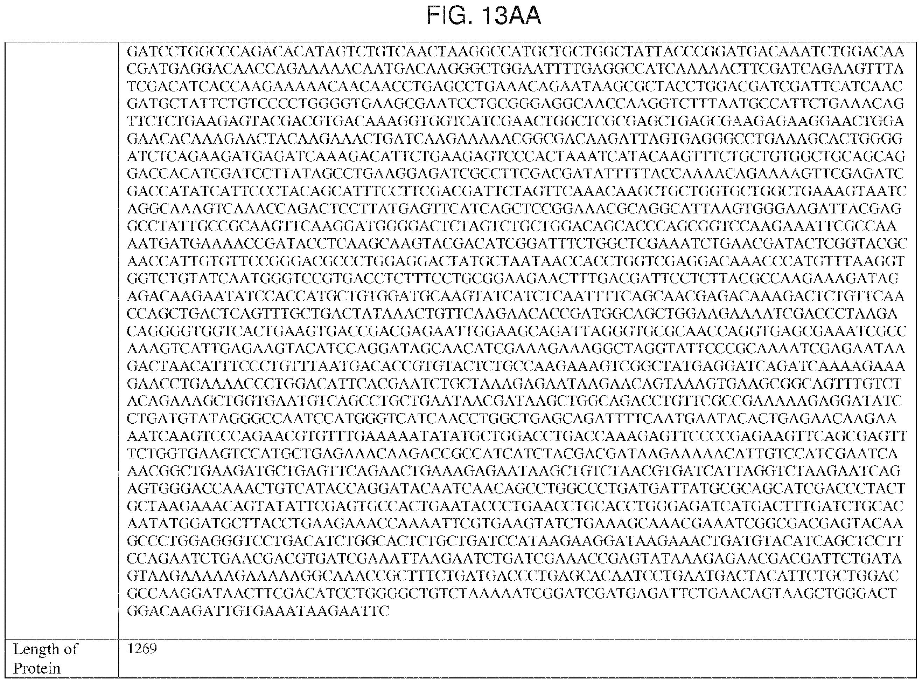

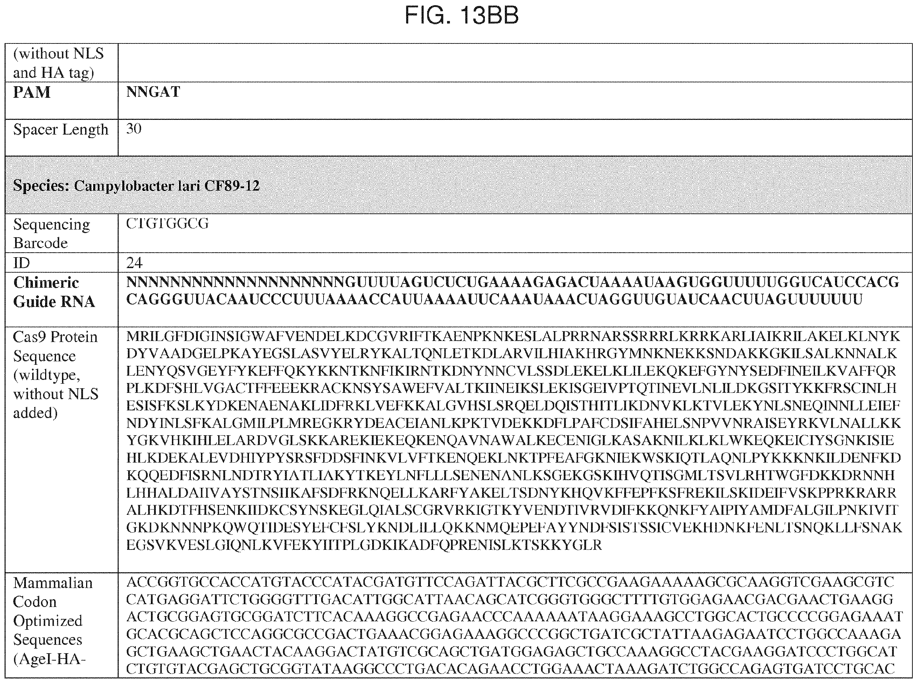

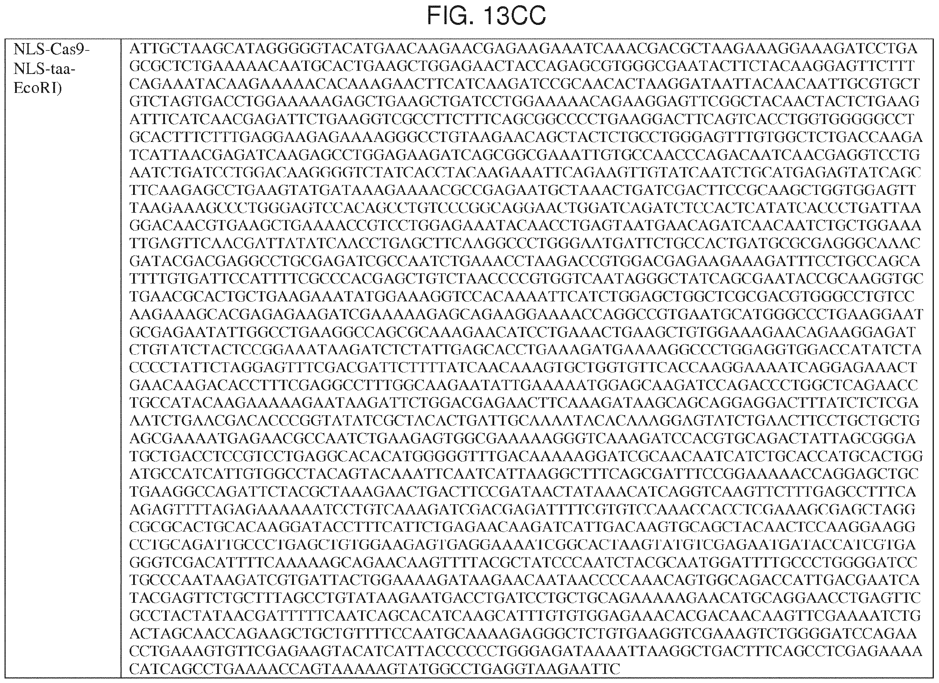

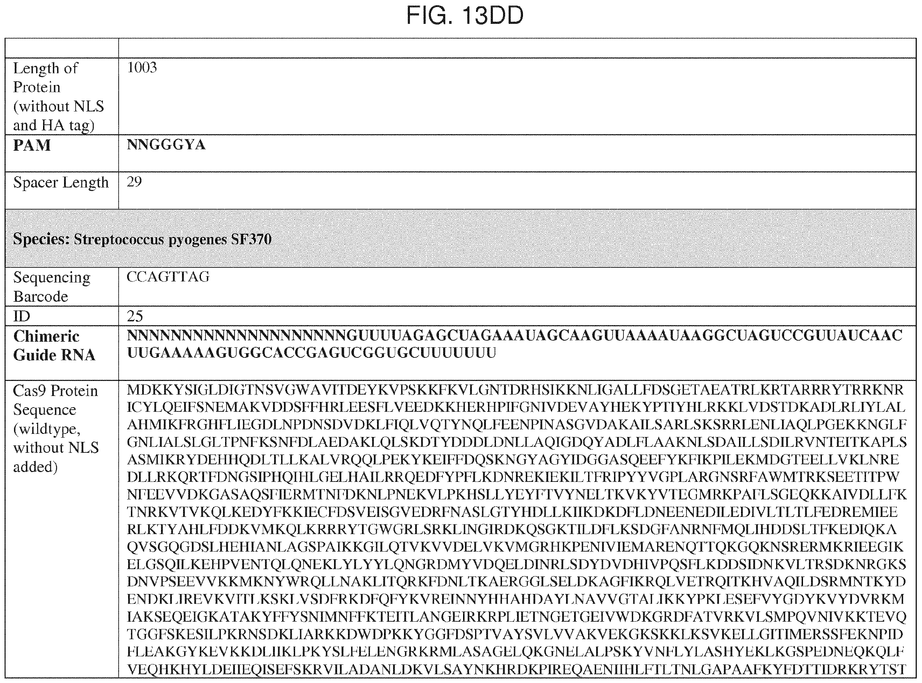

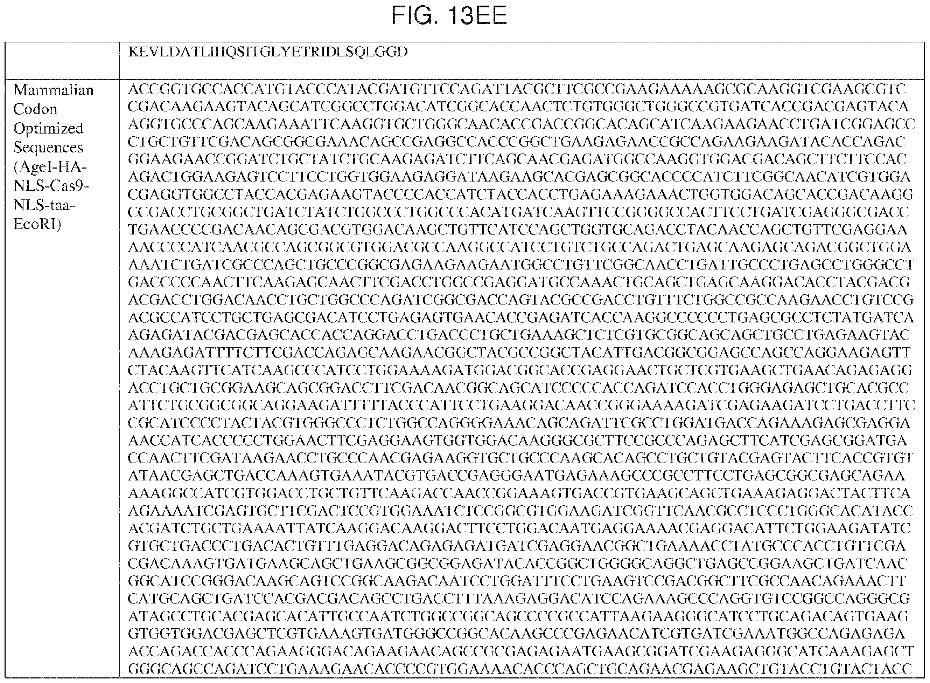

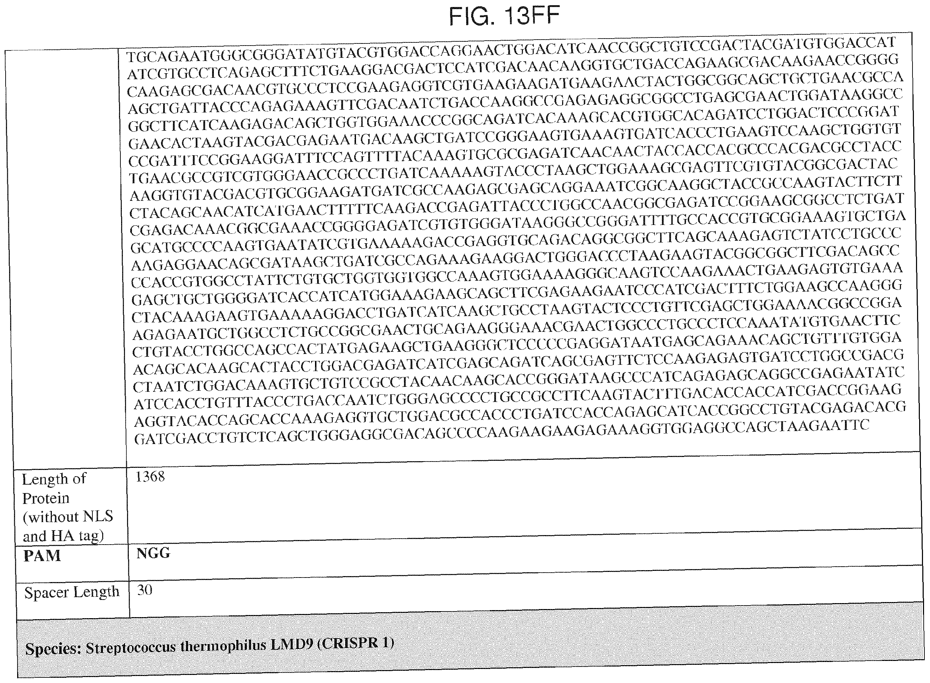

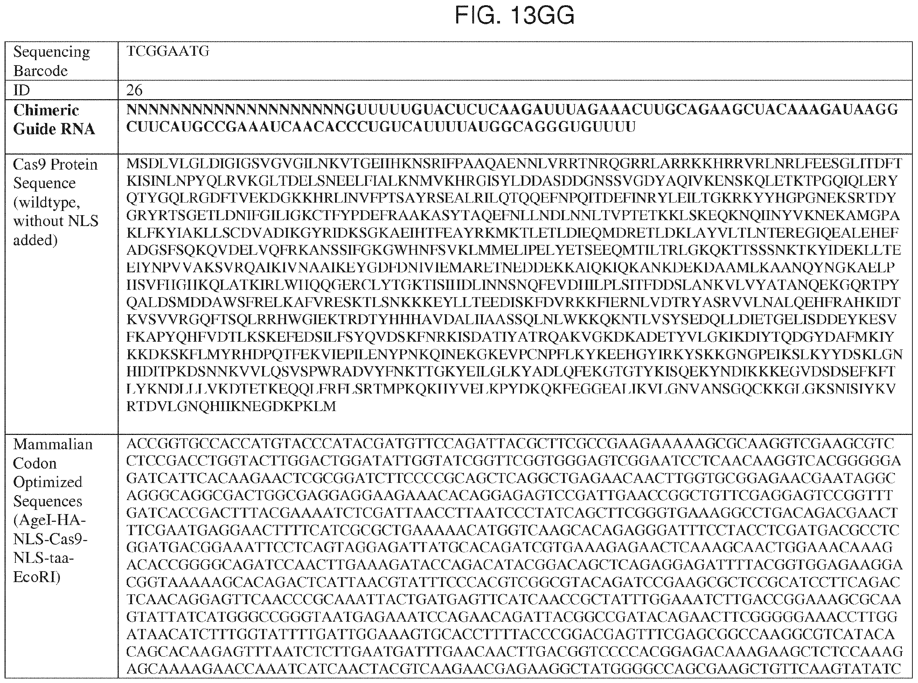

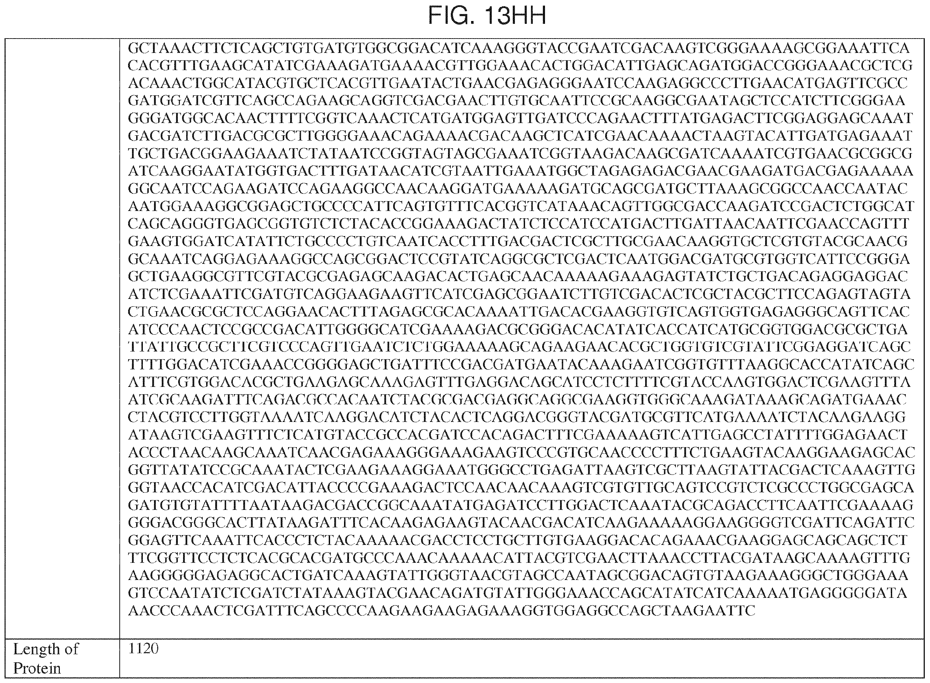

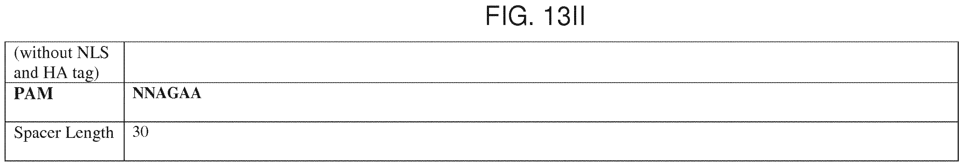

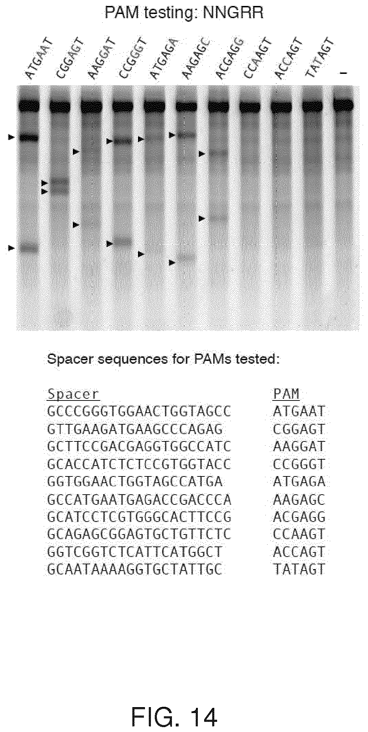

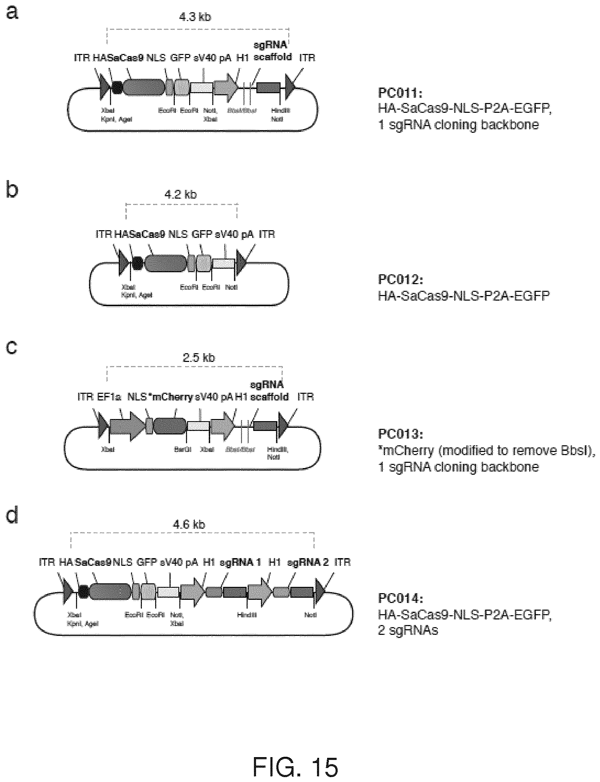

Applicants analyzed Cas9 orthologs to identify the relevant PAM sequences and the corresponding chimeric guide RNA as indicated in Figure 13A-II. This expanded set of PAMs provides broader targeting across the genome and also significantly increases the number of unique target sites and provides potential for identifying novel Cas9s with increased levels of specificity in the genome. Applicants determined the PAM for Staphylococcus aureus subspecies Aureus Cas9 to be NNGRR (Figure 14). Staphylococcus aureus subspecies Aureus Cas9 is also known as SaCas9. Figure 15a-d provides SaCas9 single or multiple vector designs.

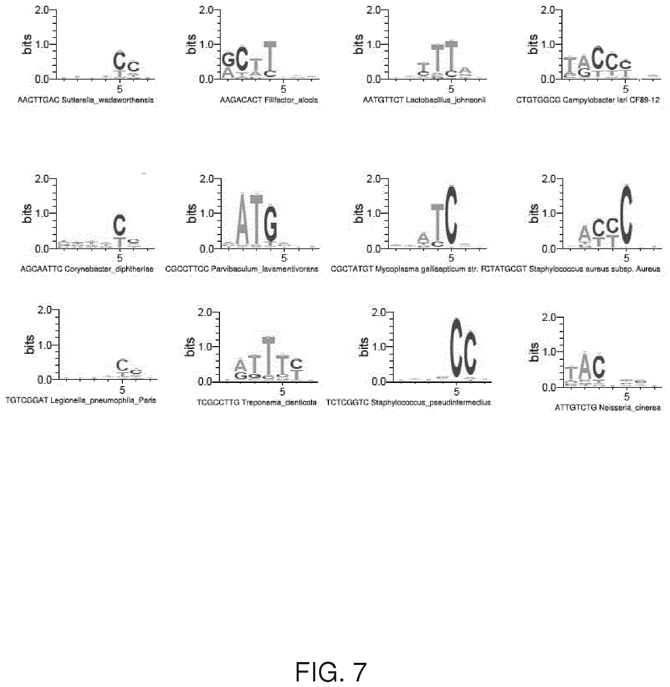

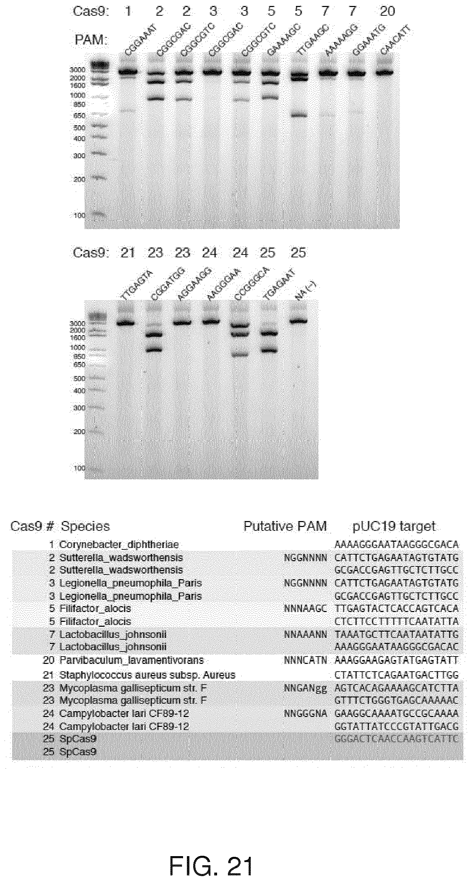

Figure 7 show sequence logos for putative PAMs as indicated by reverse complements. Cas9 orthologs and their respective sgRNAs were used to cleave a library of targets bearing a randomized PAM (7-bp sequence immediately 3' of the target sequence). Cleaved products were isolated and deep-sequenced to yield 7-bp candidate sequences that were permissive to cleavage for each Cas9 ortholog. For each Cas9 ortholog, consensus PAMs were determined by aligning all 7-bp candidate sequences. (Figures 7 and 21).

Further work examining the thermodynamics and *in vivo* stability of sgRNA-DNA duplexes will likely yield additional predictive power for off-target activity, while exploration of SpCas9 mutants and orthologs may yield novel variants with improved specificity. The specificity of Cas9 orthologs can be further evaluated by testing the ability of each Cas9 to tolerate mismatches between the guide RNA and its DNA target.

### Example 4: Cas9 mutations

In this example, Applicants show that the following mutations can convert SpCas9 into a nicking enzyme: D10A, E762A, H840A, N854A, N863A, D986A.

Applicants provide sequences showing where the mutation points are located within the SpCas9 gene (Figure 10A-M). Applicants also show that the nickases are still able to mediate homologous recombination. Furthermore, SpCas9 with these mutations (individually) reduce the level of double strand break. Cas9 orthologs all share the general organization of 3-4 RuvC domains and a HNH domain (Figure 19). The 5' most RuvC domain cleaves the non-complementary strand, and the HNH domain cleaves the complementary strand. All notations are in reference to the guide sequence.

The catalytic residue in the 5' RuvC domain is identified through homology comparison of the Cas9 of interest with other Cas9 orthologs (from S. pyogenes type II CRISPR locus, S. thermophilus CRISPR locus 1, S. thermophilus CRISPR locus 3, and Franciscilla novicida type II CRISPR locus), and the conserved Asp residue is mutated to alanine to convert Cas9 into a complementary-strand nicking enzyme. Similarly, the conserved His and Asn residues in the HNH domains are mutated to Alanine to convert Cas9 into a non-complementary-strand nicking enzyme.

### Example 5: Cas9 functional optimization

For enhanced function or to develop new functions, Applicants generate chimeric Cas9 proteins by combining fragments from different Cas9 orthologs.

For instance, Applicants fused the N-term of St1Cas9 (fragment from this protein is in bold) with C-term of SpCas9 (fragment from this protein is underlined).
>St1(N)Sp(C)Cas9
>Sp(N)St1(C)Cas9

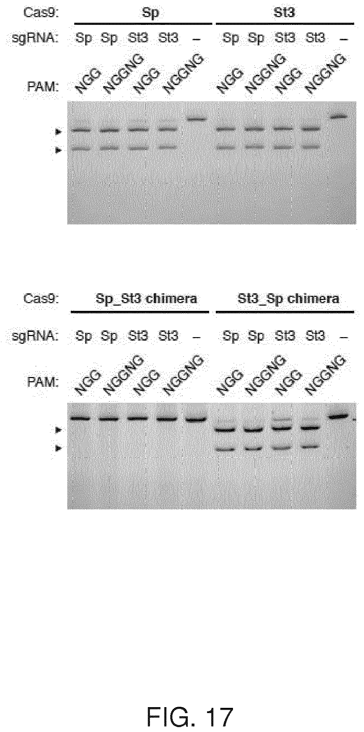

Applicants have also generated Sp_St3 chimeric proteins and have shown *in vitro* cleavage by SpCas9, St3Cas9, Sp_St3 chimera and St3_Sp chimera (Figure 17).

The benefit of making chimeric Cas9 include:
a. reduce toxicity
b. improve expression in eukaryotic cells
c. enhance specifity
d. reduce molecular weight of protein, make protein smaller by combining the smallest domains from different Cas9 homologs.
e. Altering the PAM sequence requirement

### Example 6: Cas9 delivery in vivo using AAV particles or vectors

### In vivo delivery - AAV method

AAV is advantagenous over other viral vectors for a couple of reasons:
∘ Low toxicity (this may be due to the purification method not requiring ultra centrifugation of cell particles that can activate the immune response)
∘ Low probability of causing insertional mutagenesis because it does not integrate into the host genome.

While certain current AAV vectors may accommodate up to 4300 bases of inserted DNA, as an upper limit or a packaging limit, AAV can have of 4.5 or 4.75 KB inserted DNA. This means that DNA encoding a Cas9 enzyme as well as a promoter and transcription terminator have to be all fit into the same viral vector. Constructs larger than 4.5 or 4.75 KB will lead to significantly reduced virus production. SpCas9 is quite large, the gene itself is over 4.1kb, which makes it difficult for packing into AAV. Therefore embdiments of the invention include utilizing orthologs of Cas9 that are shorter. For example:

| Species | Cas9 Size |
|---|---|
| Corynebacter diphtheria | 3252 |
| Eubacterium ventriosum | 3321 |
| Streptococcus pasteurianus | 3390 |
| Lactobacillus farciminis | 3378 |
| Sphaerochaeta globus | 3537 |
| Azospirillum B510 | 3504 |
| Gluconacetobacter diazotrophicus | 3150 |
| Neisseria cinerea | 3246 |
| Roseburia intestinalis | 3420 |
| Parvibaculum lavamentivorans | 3111 |
| Staphylococcus aureus | 3159 |
| Nitratifractor salsuginis DSM 16511 | 3396 |
| Campylobacter lari CF89-12 | 3009 |
| Streptococcus thermophilus LMD-9 | 3396 |

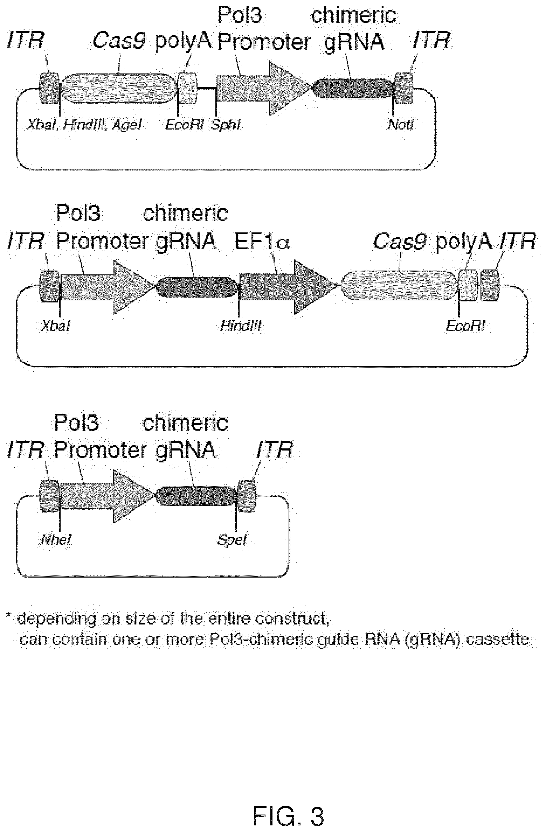

Figure 3 provides schematic representations of AAV vectors which may be used in methods and compositions of the invention. Packaging is discussed above.

### Example 7: Engineering of Microalgae using Cas9

Methods of delivering Cas9
Method 1: Applicants deliver Cas9 and guide RNA using a vector that expresses Cas9 under the control of a constitutive promoter such as Hsp70A-Rbc S2 or Beta2-tubulin.
Method 2: Applicants deliver Cas9 and T7 polymerase using vectors that expresses Cas9 and T7 polymerase under the control of a constitutive promoter such as Hsp70A-Rbc S2 or Beta2-tubulin. Guide RNA will be delivered using a vector containing T7 promoter driving the guide RNA.
Method 3: Applicants deliver Cas9 mRNA and in vitro transcribed guide RNA to algae cells. RNA can be in vitro transcribed. Cas9 mRNA will consist of the coding region for Cas9 as well as 3'UTR from Cop1 to ensure stabilization of the Cas9 mRNA.

For Homologous recombination, Applicants provide an additional homology directed repair template.

Sequence for a cassette driving the expression of Cas9 under the control of beta-2 tubulin promoter, followed by the 3' UTR of Cop1.

Sequence for a cassette driving the expression of T7 polymerase under the control of beta-2 tubulin promoter, followed by the 3' UTR of Cop1:

Sequence of guide RNA driven by the T7 promoter (T7 promoter, Ns represent targeting sequence):

### Gene delivery:

Chlamydomonas reinhardtii strain CC-124 and CC-125 from the Chlamydomonas Resource Center will be used for electroporation. Electroporation protocol follows standard recommended protocol from the GeneArt Chlamydomonas Engineering kit (website information at tools.invitrogen.com/content/sfs/manuals/geneart_chlamy_kits_man.pdf).

Also, Applicants generate a line of Chlamydomonas reinhardtii that expresses Cas9 constitutively. This can be done by using pChlamy1 (linearized using PvuI) and selecting for hygromycin resistant colonies. Sequence for pChlamy1 containing Cas9 is below. In this way to achieve gene knockout one simply needs to deliver RNA for the guideRNA. For homologous recombination Applicants deliver guideRNA as well as a linearized homologous recombination template.

pChlamy1-Cas9:

For all modified Chlamydomonas reinhardtii cells, Applicants use PCR, SURVEYOR nuclease assay, and DNA sequencing to verify successful modification.

### Example 8: SaCas9 and PAM recognition for in vivo applications

The project started as Applicants wanted to further explore the diversity of the type II CRISPR/Cas system following the identification of *Streptococcus pyogenes* (Sp) and *Streptococcus thermophiles* (St) CRISPR/Cas system as a functional genome engineering tool in mammalian cells.

By defining new functional type II CRISPR/Cas systems for application in mammalian cells, Applicants will potentially be able to find:
(1) CRISPR/Cas system with higher efficiency and/or specificity
(2) CRISPR/Cas system with different Protospacer Adjacent Motif (PAM) that allows the targeting of broader range of genomic loci
(3) CRISPR/Cas system with smaller size so Applicants could deliver them in vivo in a single vector with mammalian viral delivery system such as adeno-associated virus (AAV) vectors that has a packaging size limit (the current Sp or St system exceed this limit of 4.7kb)
   and other desirable traits.

### Identification and Design of Sa CRISPR/Cas System for in vivo application.

Applicants tested a new type II CRISPR/Cas system in *Staphylococcus aureus* (Sa) that works in vitro in dsDNA cleavage assay and identified a putative PAM of NNGRRT. The components of this system are a Cas9 protein from Sa, a guide CRISPR RNA with direct repeats (DR) from Sa that will form a functional guide RNA complex with tracrRNA from Sa. This three-component system is similar to all other type II CRISPR/Cas systems. Hence, Applicants designed a two-component system, where Applicants fused the Sa tracrRNA to the Sa guide CRISPR RNA via a short stem-loop to form a chimeric guide RNA, exactly as Applicants did with the *Streptococcus pyogenes* (Sp) CRISPR/Cas system. This chimeric guide RNA was able to support cleavage of dsDNA in vitro. Therefore, Applicants decided to clone the full two-component system: cas9 and the chimeric guide RNA, into an AAV vector to test its functionality in living organisms.

Applicants chose the AAV system because it is a non-integrating, ssDNA-based, non-immunogenic mammalian virus that has broad-spectrum of tropism in different tissues/organs depending on the serotype that has been shown to be safe for in vivo application and also support long-term expression of transgene in living organisms.

Design of the initial AAV vector has (1) CMV promoter driving SaCas9 protein with a single NLS and a HA epitope tag. (2) human U6 promoter driving the chimeric RNA. These are placed in between two Inverted Terminal Repeats (ITRs) from the most-well studied AAV serotype 2 that serve as the viral packaging signal.

The PAM sequence test on endogenous mammalian genome is as follows: SaCas9 target spacers were selected across multiple genes to cover different potential PAM sequences. Different spacers were cloned into U6-sgRNA (single-guide RNA) expression dsDNA cassette U6-sgRNA expression dsDNA cassette were co-transfected into mammalian cells lines (293FT for human targets, N2a and Hepa for mouse targets). 72 hours following transfection, all genomic DNA were extracted and subjected to surveyor nuclease assay. Run through TBE Page Gel to detect genomic cleavage. Quantify genomic DNA cleavage efficiency and plot.

### Summary of Genome Cleavage Efficiency and other Statistics on All Tested Targets

| SaCasθ PAM Sequences | Targets Count | Cleavaged Targets Count | Percentage of Cleaved Targets (%) | Cumulative Cleavage Efficiency (%) | Average Spacer GC Content (%) |
|---|---|---|---|---|---|
| GAAA | 1 | 1 | 100.0 | 5.4 | 65.0 |
| GAAC | 2 | 2 | 100.0 | 6.1 | 55.0 |
| GAAG | 8 | 8 | 100.0 | 47.1 | 65.0 |
| GAAT | 9 | 8 | 88.9 | 138.4 | 66.1 |
| GAGA | 3 | 3 | 100.0 | 17.5 | 63.3 |
| GAGC | 6 | 6 | 100.0 | 44.2 | 60.0 |
| GAGG | 12 | 12 | 100.0 | 93.3 | 58.8 |
| GAGT | 44 | 20 | 45.5 | 434.0 | 58.9 |
| GGAA | 2 | 2 | 100.0 | 4.7 | 50.0 |
| GGAC | 3 | 2 | 66.7 | 39.9 | 60.0 |
| GGAG | 12 | 9 | 75.0 | 36.9 | 59.6 |
| GGAT | 20 | 10 | 50.0 | 186.2 | 59.0 |
| GGGA | 7 | 5 | 71.4 | 39.1 | 63.6 |
| GGGC | 11 | 9 | 81.8 | 70.3 | 65.5 |
| GGGG | 8 | 5 | 62.5 | 53.3 | 70.0 |
| GGGT | 45 | 18 | 40.0 | 402.3 | 56.2 |
| **Grand Total** | **196** | **120** | **61.2** | **1618.6** | **59.4** |

### Summary of Genome Cleavage Efficiency and other Statistics on All Tested Targets (cleaned up)

| SaCas9 PAM Sequences | Targets Count | Cleavaged Targets Count | Percentage of Cleaved Targets (%) | Cumulative Cleavage | Average Cleavage Efficiency (%) | Average Spacer GC Content (%) |
|---|---|---|---|---|---|---|
| GAAA | 1 | 1 | 100.0 | 5.4 | 5.4 | 65.0 |
| GAAC | 2 | 2 | 100.0 | 6.1 | 3.0 | 55.0 |
| GAAG | 8 | 8 | 100.0 | 47.1 | 5.9 | 65.0 |
| QAAT | 4 | 4 | 100.0 | 68.4 | 17.1 | 65.0 |
| GAGA | 2 | 2 | 100.0 | 12.5 | 6.3 | 67.5 |
| GAGC | 5 | 5 | 100.0 | 39.2 | 7.8 | 61.0 |
| GAGG | 11 | 11 | 100.0 | 88.3 | 8.0 | 58.2 |
| GAGT | 13 | 10 | 76.9 | 199.0 | 15.3 | 56.2 |
| GGAA | 2 | 2 | 100.0 | 4.7 | 2.3 | 50.0 |
| GGAC | 3 | 2 | 66.7 | 39.9 | 13.3 | 60.0 |
| GGAG | 12 | 9 | 75.0 | 36.9 | 3.1 | 59.6 |
| GGAT | 13 | 9 | 6B.2 | 161.2 | 12.4 | 58.13 |
| GGGA | 7 | 5 | 71.4 | 39.1 | 5.6 | 63.6 |
| GGGC | 11 | 9 | 81.8 | 70.3 | 6.4 | 65.5 |
| GGGG | 3 | 5 | 62.5 | 53.3 | 6.7 | 70.0 |
| GGGT | 14 | 8 | 57.1 | 182.3 | 13.0 | 54.6 |
| **Grand Total** | **116** | **92** | **79.3** | **1053.6** | **9.1** | **60.5** |

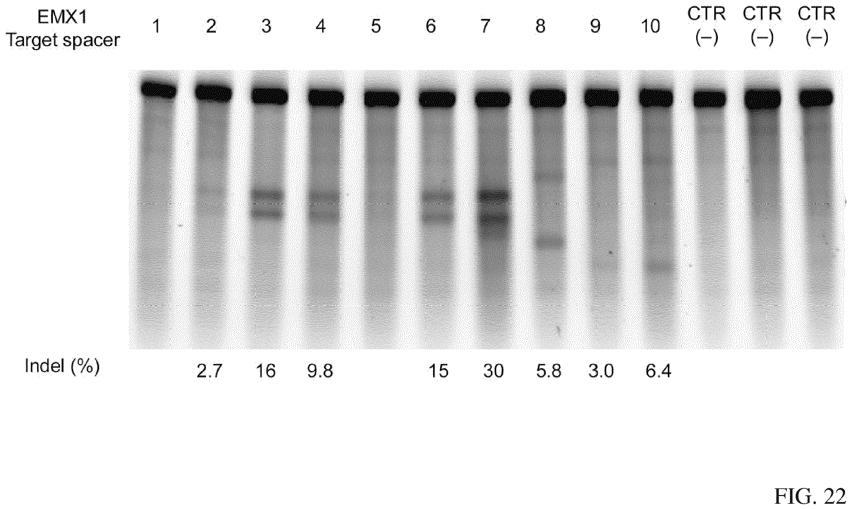

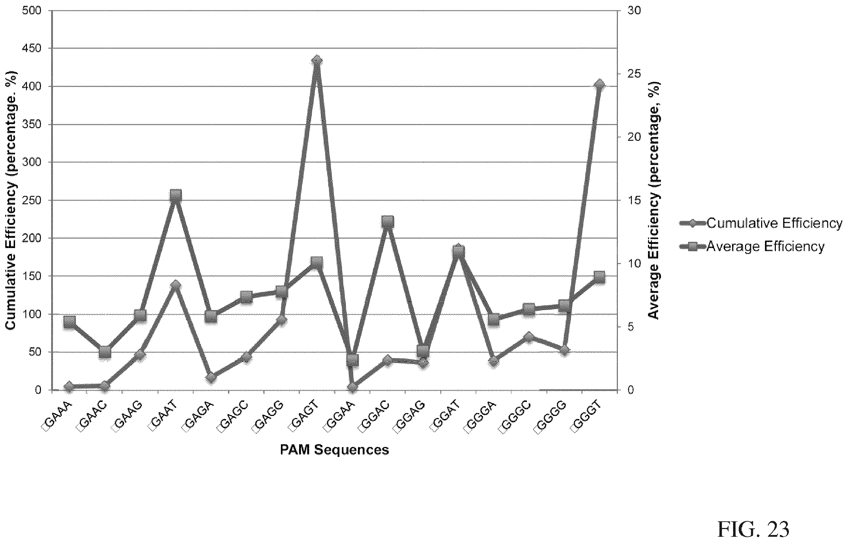

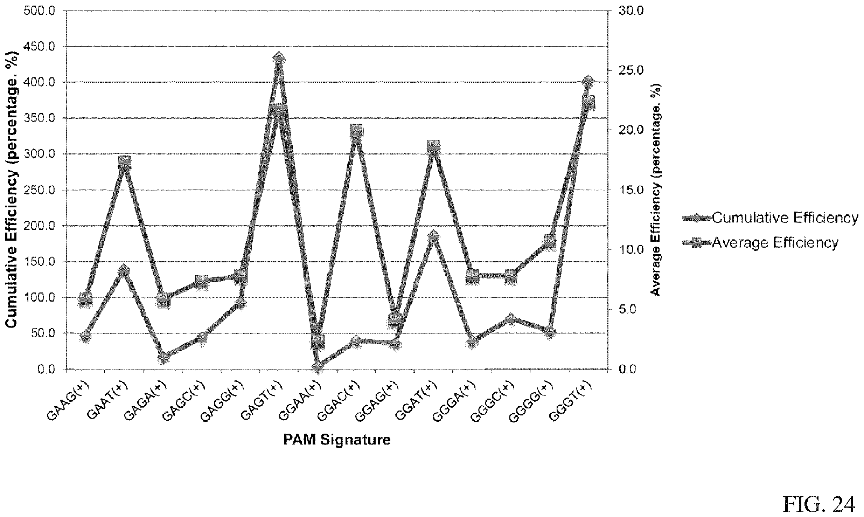

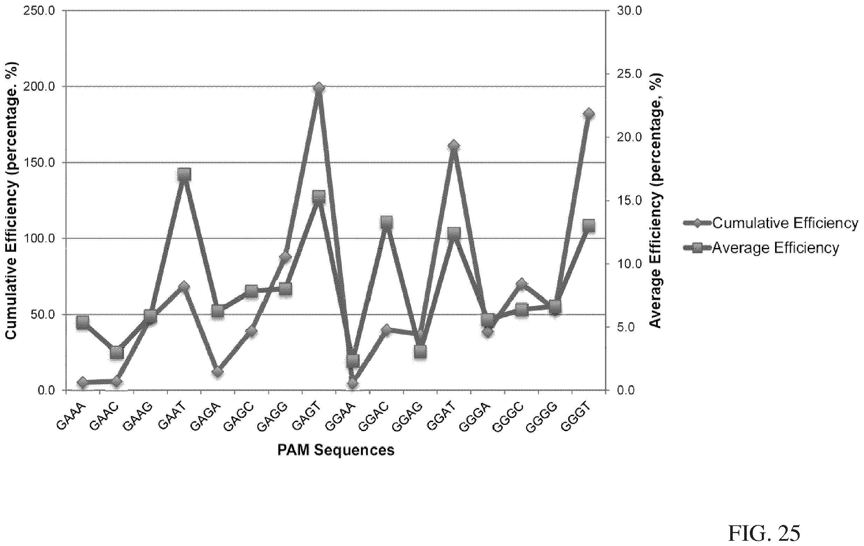

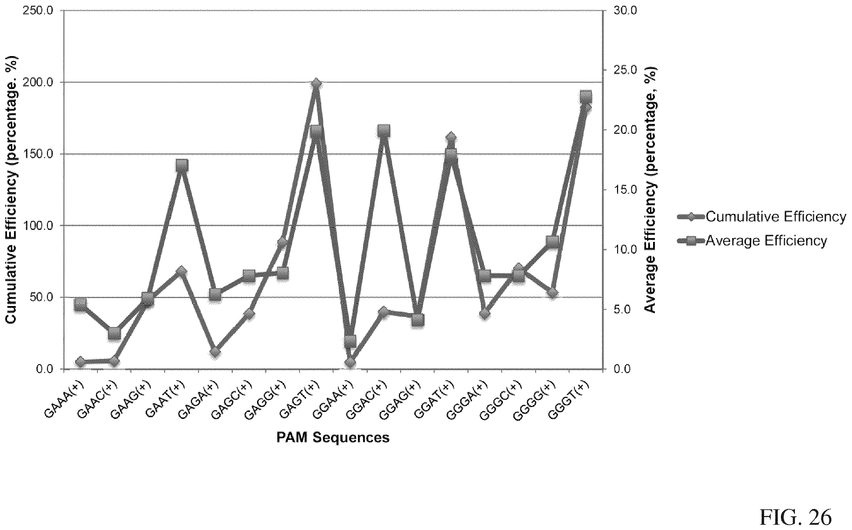

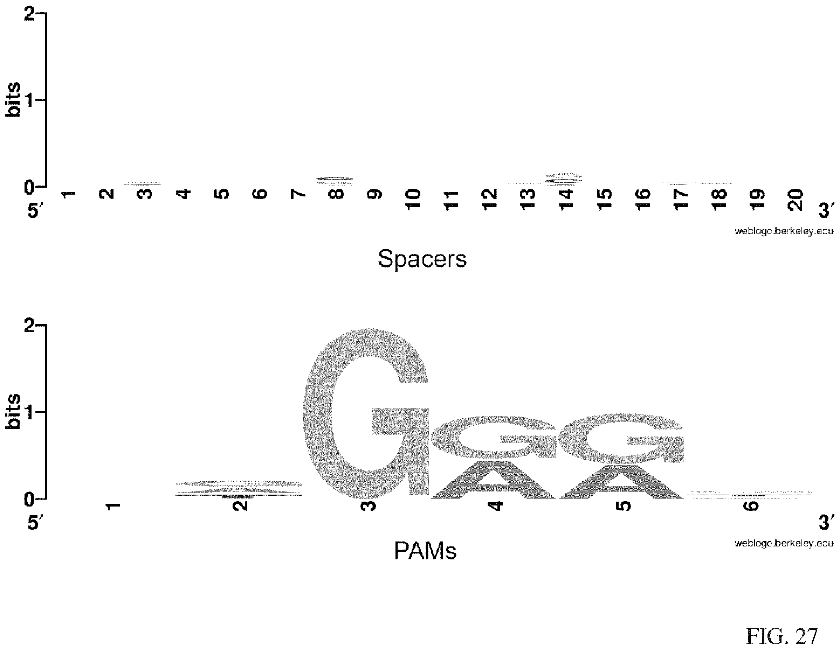

Results from the PAM test are shown in Figs. 22-27. A comprehensive test of over 100 targets identified that the PAM for SaCas9 could be described as NNGRR (but not the NNGRRT as indicated earlier).

PAM Test Summary: (1) NNGRR for general SaCas9 PAM - helpful for design new targets, (2) Testing double-nickase with new targets, (3) NNGRG may be a more potent PAM.

### REFERENCES

1. Cong, L. et al. Multiplex genome engineering using CRISPR/Cas systems. Science 339, 819-823 (2013)
2. Mali, P. et al. RNA-Guided Human Genome Engineering via Cas9. Science 339, 823-826 (2013).
3. Jinek, M. et al. RNA-programmed genome editing in human cells. eLife 2, e00471 (2013).
4. Cho, S.W., Kim, S., Kim, J.M. & Kim, J.S. Targeted genome engineering in human cells with the Cas9 RNA-guided endonuclease. Nat Biotechnol 31, 230-232 (2013).
5. Deltcheva, E. et al. CRISPR RNA maturation by trans-encoded small RNA and host factor RNase III. Nature 471, 602-607 (2011).
6. Jinek, M. et al. A programmable dual-RNA-guided DNA endonuclease in adaptive bacterial immunity. Science 337, 816-821 (2012).
7. Wang, H. et al. One-Step Generation of Mice Carrying Mutations in Multiple Genes by CRISPR/Cas-Mediated Genome Engineering. Cell 153, 910-918 (2013).
8. Guschin, D.Y. et al. A rapid and general assay for monitoring endogenous gene modification. Methods Mol Biol 649, 247-256 (2010).
9. Bogenhagen, D.F. & Brown, D.D. Nucleotide sequences in Xenopus 5S DNA required for transcription termination. Cell 24, 261-270 (1981).
10. Hwang, W.Y. et al. Efficient genome editing in zebrafish using a CRISPR-Cas system. Nat Biotechnol 31, 227-229 (2013).
11. Bultmann, S. et al. Targeted transcriptional activation of silent oct4 pluripotency gene by combining designer TALEs and inhibition of epigenetic modifiers. Nucleic Acids Res 40, 5368-5377 (2012).
12. Valton, J. et al. Overcoming transcription activator-like effector (TALE) DNA binding domain sensitivity to cytosine methylation. J Biol Chem 287, 38427-38432 (2012).
13. Christian, M. et al. Targeting DNA double-strand breaks with TAL effector nucleases. Genetics 186, 757-761 (2010).
14. Miller, J.C. et al. A TALE nuclease architecture for efficient genome editing. Nat Biotechnol 29, 143-148 (2011).
15. Mussolino, C. et al. A novel TALE nuclease scaffold enables high genome editing activity in combination with low toxicity. Nucleic acids research 39, 9283-9293 (2011).
16. Hsu, P.D. & Zhang, F. Dissecting neural function using targeted genome engineering technologies. ACS chemical neuroscience 3, 603-610 (2012).
17. Sanjana, N.E. et al. A transcription activator-like effector toolbox for genome engineering. Nature protocols 7, 171-192 (2012).
18. Porteus, M.H. & Baltimore, D. Chimeric nucleases stimulate gene targeting in human cells. Science 300, 763 (2003).
19. Miller, J.C. et al. An improved zinc-finger nuclease architecture for highly specific genome editing. Nat Biotechnol 25, 778-785 (2007).
20. Sander, J.D. et al. Selection-free zinc-finger-nuclease engineering by context-dependent assembly (CoDA). Nat Methods 8, 67-69 (2011).
21. Wood, A.J. et al. Targeted genome editing across species using ZFNs and TALENs. Science 333, 307 (2011).
22. Bobis-Wozowicz, S., Osiak, A., Rahman, S.H. & Cathomen, T. Targeted genome editing in pluripotent stem cells using zinc-finger nucleases. Methods 53, 339-346 (2011).
23. Jiang, W., Bikard, D., Cox, D., Zhang, F. & Marraffini, L.A. RNA-guided editing of bacterial genomes using CRISPR-Cas systems. Nat Biotechnol 31, 233-239 (2013).
24. Qi, L.S. et al. Repurposing CRISPR as an RNA-Guided Platform for Sequence-Specific Control of Gene Expression. Cell 152, 1173-1183 (2013).
25. Michaelis, L.M., Maud "Die kinetik der invertinwirkung.". Biochem. z (1913).
26. Mahfouz, M.M. et al. De novo-engineered transcription activator-like effector (TALE) hybrid nuclease with novel DNA binding specificity creates double-strand breaks. Proc Natl Acad Sci U S A 108, 2623-2628 (2011).
27. Wilson, E.B. Probable inference, the law of succession, and statistical inference. J Am Stat Assoc 22, 209-212 (1927).
28. Ding, Q. et al. A TALEN genome-editing system for generating human stem cell-based disease models. Cell Stem Cell 12, 238-251 (2013).
29. Soldner, F. et al. Generation of isogenic pluripotent stem cells differing exclusively at two early onset Parkinson point mutations. Cell 146, 318-331 (2011).
30. Carlson, D.F. et al. Efficient TALEN-mediated gene knockout in livestock. Proc Natl Acad Sci U S A 109, 17382-17387 (2012).
31. Geurts, A.M. et al. Knockout Rats via Embryo Microinjection of Zinc-Finger Nucleases. Science 325, 433-433 (2009).
32. Takasu, Y. et al. Targeted mutagenesis in the silkworm Bombyx mori using zinc finger nuclease mRNA injection. Insect Biochem Molec 40, 759-765 (2010).
33. Watanabe, T. et al. Non-transgenic genome modifications in a hemimetabolous insect using zinc-finger and TAL effector nucleases. Nat Commun 3 (2012).
34. Reyon, D. et al. FLASH assembly of TALENs for high-throughput genome editing. Nat Biotechnol 30, 460-465 (2012).
35. Boch, J. et al. Breaking the code of DNA binding specificity of TAL-type III effectors. Science 326, 1509-1512 (2009).
36. Moscou, M.J. & Bogdanove, A.J. A simple cipher governs DNA recognition by TAL effectors. Science 326, 1501 (2009).
37. Deveau, H., Garneau, J.E. & Moineau, S. CRISPR/Cas system and its role in phage-bacteria interactions. Annu Rev Microbiol 64, 475-493 (2010).
38. Horvath, P. & Barrangou, R. CRISPR/Cas, the immune system of bacteria and archaea. Science 327, 167-170 (2010).
39. Makarova, K.S. et al. Evolution and classification of the CRISPR-Cas systems. Nat Rev Microbiol 9, 467-477 (2011).
40. Bhaya, D., Davison, M. & Barrangou, R. CRISPR-Cas systems in bacteria and archaea: versatile small RNAs for adaptive defense and regulation. Annu Rev Genet 45, 273-297 (2011).
41. Garneau, J.E. et al. The CRISPR/Cas bacterial immune system cleaves bacteriophage and plasmid DNA. Nature 468, 67-71 (2010).
42. Gasiunas, G., Barrangou, R., Horvath, P. & Siksnys, V. Cas9-crRNA ribonucleoprotein complex mediates specific DNA cleavage for adaptive immunity in bacteria. Proc Natl Acad Sci U S A 109, E2579-2586 (2012).
43. Urnov, F.D., Rebar, E.J., Holmes, M.C., Zhang, H.S. & Gregory, P.D. Genome editing with engineered zinc finger nucleases. Nat Rev Genet 11, 636-646 (2010).
44. Perez, E.E. et al. Establishment of HIV-1 resistance in CD4(+) T cells by genome editing using zinc-finger nucleases. Nat Biotechnol 26, 808-816 (2008).
45. Chen, F.Q. et al. High-frequency genome editing using ssDNA oligonucleotides with zinc-finger nucleases. Nat Methods 8, 753-U796 (2011).
46. Bedell, V.M. et al. In vivo genome editing using a high-efficiency TALEN system. Nature 491, 114-U133 (2012).
47. Saleh-Gohari, N. & Helleday, T. Conservative homologous recombination preferentially repairs DNA double-strand breaks in the S phase of the cell cycle in human cells. Nucleic Acids Res 32, 3683-3688 (2004).
48. Sapranauskas, R. et al. The Streptococcus thermophilus CRISPR/Cas system provides immunity in Escherichia coli. Nucleic Acids Res 39, 9275-9282 (2011).
49. Shen, B. et al. Generation of gene-modified mice via Cas9/RNA-mediated gene targeting. Cell Res 23, 720-723 (2013).
50. Tuschl, T. Expanding small RNA interference. Nat Biotechnol 20, 446-448 (2002).
51. Smithies, O., Gregg, R.G., Boggs, S.S., Koralewski, M.A. & Kucherlapati, R.S. Insertion of DNA sequences into the human chromosomal beta-globin locus by homologous recombination. Nature 317, 230-234 (1985).
52. Thomas, K.R., Folger, K.R. & Capecchi, M.R. High frequency targeting of genes to specific sites in the mammalian genome. Cell 44, 419-428 (1986).
53. Hasty, P., Rivera-Perez, J. & Bradley, A. The length of homology required for gene targeting in embryonic stem cells. Mol CellBiol 11, 5586-5591 (1991).
54. Wu, S., Ying, G.X., Wu, Q. & Capecchi, M.R. A protocol for constructing gene targeting vectors: generating knockout mice for the cadherin family and beyond. Nat Protoc 3, 1056-1076 (2008).
55. Oliveira, T.Y. et al. Translocation capture sequencing: a method for high throughput mapping of chromosomal rearrangements. J Immunol Methods 375, 176-181 (2012).
56. Tremblay et al., Transcription Activator-Like Effector Proteins Induce the Expression of the Frataxin Gene; Human Gene Therapy. August 2012, 23(8): 883-890.
57. Shalek et al. Nanowire-mediated delivery enables functional interrogation of primary immune cells: application to the analysis of chronic lymphocytic leukemia. Nano Letters, 2012, Dec 12;12(12):6498-504.
58. Pardridge et al. Preparation of Trojan horse liposomes (THLs) for gene transfer across the blood-brain barrier; Cold Spring Harb Protoc; 2010; Apr; 2010 (4)
59. Plosker GL et al. Fluvastatin: a review of its pharmacology and use in the management of hypercholesterolaemia; Drugs 1996, 51(3):433-459).
60. Trapani et al. Potential role of nonstatin cholesterol lowering agents; IUBMB Life, Volume 63, Issue 11, pages 964-971, November 2011
61. Birch AM et al. DGAT1 inhibitors as anti-obesity and anti-diabetic agents; Current Opinion in Drug Discovery & Development, 2010, 13(4):489-496
62. Fuchs et al. Killing of leukemic cells with a BCR/ABL fusion gene by RNA interference (RNAi), Oncogene 2002, 21(37):5716-5724.
63. McManaman JL et al. Perilipin-2 Null Mice are Protected Against Diet-Induced Obesity, Adipose Inflammation and Fatty Liver Disease; The Journal of Lipid Research, jlr.M035063. First Published on February 12, 2013.
64. Tang J et al. Inhibition of SREBP by a Small Molecule, Betulin, Improves Hyperlipidemia and Insulin Resistance and Reduces Atherosclerotic Plaques; Cell Metabolism, Volume 13, Issue 1, 44-56, 5 January 2011.
65. Dumitrache et al. Trex2 enables spontaneous sister chromatid exchanges without facilitating DNA double-strand break repair; Genetics. 2011 August; 188(4): 787-797

While preferred embodiments of the present invention have been shown and described herein, it will be obvious to those skilled in the art that such embodiments are provided by way of example only.

## Claims

1. A non-naturally occurring or engineered composition comprising a vector system comprising one or more vectors comprising
I. a first regulatory element operably linked to a CRISPR-Cas system guide RNA comprising:
(a) a guide RNA polynucleotide capable of hybridizing to a target sequence in a eukaryotic cell,
(b) a tracr mate RNA polynucleotide, and
(c) a tracr RNA polynucleotide, and
II. a second regulatory element operably linked to a protein-coding sequence encoding a Cas9 protein comprising at least one or more nuclear localization sequences,
wherein (a), (b) and (c) are arranged in a 5' to 3' orientation,
wherein components I and II are located on the same or different vectors of the system,
wherein when transcribed, the tracr mate RNA polynucleotide hybridizes to the tracr RNA polynucleotide and the guide RNA polynucleotide directs sequence-specific binding of a CRISPR complex to the target sequence, and
wherein the CRISPR complex comprises the Cas9 protein complexed with (1) the guide RNA polynucleotide that is hybridized to the target sequence, and (2) the tracr mate RNA polynucleotide that is hybridized to the tracr RNA polynucleotide,
wherein the Cas9 protein is *Staphylococcus aureus* Cas9 (SaCas9).

2. A composition according to claim 1, wherein the Cas9 protein has one or more mutations in any domain of the protein.

3. A composition according to claim 2, wherein the Cas9 protein has one or more mutations in a catalytic domain.

4. The composition or protein according to claim 2 or 3, wherein the one or more mutations is in a RuvC I, RuvC II, RuvC III or HNH domain of the Cas9 protein.

5. The composition or protein according to claim 3 or 4, wherein the Cas9 protein comprises one or more mutations corresponding to the position numbering of SpCas9 at D10A, H840A, N854A, or N863A.

6. A composition according to any preceding claim, wherein the Cas9 protein further comprises a functional domain.

7. A composition according to claim 6, wherein the functional domain is a transcriptional activator domain.

8. A composition according to claim 7, wherein the transcriptional activator domain is VP64.

9. A composition according to claim 6, wherein the functional domain is a transcriptional repressor domain.

10. A composition according to claim 9, wherein the transcriptional repressor domain is KRAB, SID or SID4X.

11. A composition according to claim 6, wherein the functional domain is a recombinase, a transposase, a histone remodeler, a DNA methyltransferase, a cryptochrome and a light inducible/controllable domain or a chemically inducible/controllable domain.

12. A composition according to any one of the preceding claims, wherein the polynucleotide sequence encoding the Cas9 protein is codon optimized for expression in a eukaryotic cell.

13. A composition according to any one of the preceding claims, comprising two guide RNAs.

14. A composition according to any one of the preceding claims, comprising a vector system comprising one or more vectors, and wherein polynucleotide sequences comprising or encoding said components I and II are located on the same or different vectors of the system.

15. A composition according to claim 14, wherein the vector system comprises a single vector.

16. A composition according to claim 14, wherein the one or more vectors comprise one or more viral vectors.

17. A composition according to claim 16, wherein the one or more viral vectors comprise one or more retrovirus, lentivirus, adenovirus, adeno-associated virus or herpes simplex virus vectors.

18. A composition according to any one of claims 1 to 15, comprising a nanoparticle, liposome, exosome, yeast system, or microvesicle.

19. An *ex vivo* or *in vitro* host cell or cell line comprising the composition according to any one of the preceding claims, or progeny thereof.

20. An ex vivo or in vivo host cell, cell line or progeny thereof according to claim 19, which is a stem cell or stem cell line.

21. A method of modifying an organism by manipulation of one or more target sequences at genomic loci of interest comprising delivering to the organism the composition according to any one of claims 1-18, wherein the organism is a non-animal organism.

22. An *ex vivo* method of modifying a cell or an organism by manipulation of one or more target sequences at genomic loci of interest comprising delivering to the cell a composition according to any one of claims 1-18, wherein the method does not comprise a process for modifying the germ line genetic identity of a human being.

23. A method according to claim 21 or 22, wherein the organism is a plant or algae.

24. Use of the composition according to any one of claims 1-18 for *ex vivo* gene or genome editing, wherein the use does not comprise a process for modifying the germ line genetic identity of a human being.

25. Use of a composition according to any one of claims 1-18 for the preparation of a medicament.

26. A composition according to any one of claims 1-18, for use in correcting ocular defects that arise from genetic mutations.

## Patentansprüche

1. Nicht natürlich vorkommende oder manipulierte Zusammensetzung umfassend ein Vektorsystem umfassend einen oder mehrere Vektoren umfassend
I. ein erstes regulatorisches Element, das funktionsfähig an eine CRISPR-Cas-Systemführungs-RNA angeknüpft ist, umfassend
(a) ein Führung-RNA-Polynucleotid, das in der Lage ist, an eine Targetsequenz in einer eukaryotischen Zelle zu hybridisieren,
(b) ein tracr-Mate-RNA-Polynucleotid und
(c) ein tracr-RNA-Polynucleotid und
II. ein zweites regulatorisches Element, das funktionsfähig an eine proteincodierende Sequenz angeknüpft ist, die für ein Cas9-Protein codiert, das mindestens eine oder mehrere nukleare Lokalisationssequenzen umfasst,
wobei (a), (b) und (c) in einer 5' - bis 3'-Orientation angeordnet sind,
wobei die Komponenten I und II sich an demselben oder verschiedenen Vektoren des Systems befinden,
wobei, wenn es transkribiert wird, das tracr-Mate-RNA- Polynucleotid an das tracr-RNA-Polynucleotid hybridisiert und das Führung-RNA-Polynucleotid die sequenzspezifische Bindung eines CRISPR-Komplexes an die Targetsequenz richtet und
wobei der CRISPR-Komplex das Cas9-Protein umfasst, das mit (1) dem Führungs-RNA-Polynucleotid, das an die Targetsequenz hybridisiert ist, und (2) dem tracr-Mate-RNA-Polynucleotid, das an das tracr-RNA-Polynucleotid hybridisiert ist, komplexiert ist,
wobei das Cas9-Protein *Staphylococcus aureus* Cas9 (SaCas9) ist.

2. Zusammensetzung nach Anspruch 1, wobei das Cas9-Protein eine oder mehrere Mutationen in irgendeiner Domäne des Proteins aufweist.

3. Zusammensetzung nach Anspruch 2, wobei das Cas9-Protein eine oder mehrere Mutationen in einer katalytischen Domäne aufweist.

4. Zusammensetzung oder Protein nach Anspruch 2 oder 3, wobei eine oder mehrere Mutationen sich in einer RuvC I-, RuvC II-, RuvC III- oder HNH-Domäne des Cas9-Proteins befindet.

5. Zusammensetzung oder Protein nach Anspruch 3 oder 4, wobei das Cas9-Protein eine oder mehrere Mutationen umfasst, die der Positionsnummerierung von SpCas9 bei D10A, H840A, N854A oder N863A entspricht.

6. Zusammensetzung nach irgendeinem vorhergehenden Anspruch, wobei das Cas9-Protein ferner eine funktionelle Domäne umfasst.

7. Zusammensetzung nach Anspruch 6, wobei die funktionelle Domäne eine transkriptionelle Aktivatordomäne ist.

8. Zusammensetzung nach Anspruch 7, wobei die transkriptionelle Aktivatordomäne VP64 ist.

9. Zusammensetzung nach Anspruch 6, wobei die funktionelle Domäne eine transkriptionelle Repressordomäne ist.

10. Zusammensetzung nach Anspruch 9, wobei die transkriptionenelle Repressordomäne KRAB, SID oder SID4X ist.

11. Zusammensetzung nach Anspruch 6, wobei die funktionelle Domäne eine Rekombinase, eine Transposase, ein Histonumwandler, eine DNA-Methyltransferase, ein Cryptochrom und eine lichtinduzierbare/regulierbare Domäne oder eine chemisch induzierbare/regulierbare Domäne ist.

12. Zusammensetzung nach irgendeinem der vorhergehenden Ansprüche, wobei die Polynucleotidsequenz, die für das Cas9-Protein codiert, für die Expression in einer eukaryotischen Zelle codonoptimiert ist.

13. Zusammensetzung nach irgendeinem der vorhergehenden Ansprüche umfassend zwei Führung-RNA.

14. Zusammensetzung nach irgendeinem der vorhergehenden Ansprüche, umfassend ein Vektorsystem umfassend einen oder mehrere Vektoren und wobei die Polynucleotidsequenzen, die die Komponenten I und II umfassen oder dafür kodieren, sich an demselben oder verschiedenen Vektoren des Systems befinden.

15. Zusammensetzung nach Anspruch 14, wobei das Vektorsystem einen einzigen Vektor umfasst.

16. Zusammensetzung nach Anspruch 14, wobei der eine oder die mehreren Vektoren einen oder mehrere Virusvektoren umfasst/umfassen.

17. Zusammensetzung nach Anspruch 16, wobei der eine oder die mehreren Virusvektoren einen oder mehrere Retroviren, Lentiviren, Adenoviren, adenoassoziierte Viren oder Herpes simplex-Virusvektoren umfasst/umfassen.

18. Zusammensetzung nach irgendeinem der Ansprüche 1 bis 15, umfassend ein Nanoteilchen, Liposom, Exosom, Hefesystem oder Mikrovesikel.

19. Ex-Vivo- oder In-vitro-Wirtszelle oder -Zelllinie umfassend die Zusammensetzung nach irgendeinem der vorhergehenden Ansprüche oder einen Abkömmling davon.

20. Ex-Vivo- oder In-Vivo-Wirtszelle, Zelllinie oder Abkömmling davon nach Anspruch 19, die/der eine Stammzelle oder Stammzellenlinie ist.

21. Verfahren zum Modifizieren eines Organismus durch Manipulieren einer oder mehrerer Targetsequenzen an genomischen Loci, die von Interesse sind, umfassend das Liefern, an den Organismus, ich der Zusammensetzung nach irgendeinem der Ansprüche 1-18, wobei der Organismus ein nichttierischer Organismus ist.

22. Ex-Vivo-Verfahren zum Modifizieren einer Zelle oder eines Organismus durch Manipulieren einer oder mehrerer Targetsequenzen an genomischen Loci, die von Interesse sind, umfassend das Liefern, an die Zelle, einer Zusammensetzung nach irgendeinem der Ansprüche 1-18, wobei das Verfahren keinen Vorgang zum Modifizieren der genetischen Keimlinienidentität eines menschlichen Wesens umfasst.

23. Verfahren nach Anspruch 21 oder 22, wobei der Organismus eine Pflanze oder Alge ist.

24. Verwendung der Zusammensetzung nach irgendeinem der Ansprüche 1-18 zum Ex-Vivo-Gen- oder -Genomeditieren, wobei die Verwendung keinen Vorgang zum Modifizieren der genetischen Keimlinienidentität eines menschlichen Wesens umfasst.

25. Verwendung einer Zusammensetzung nach irgendeinem der Ansprüche 1-18 für die Herstellung eines Medikaments.

26. Zusammensetzung nach irgendeinem der Ansprüche 1-18 zur Verwendung zum Korrigieren von Augendefekten, die durch genetische Mutationen entstehen.

## Revendications

1. Composition non naturelle ou modifiée comprenant un système de vecteur comprenant un ou plusieurs vecteurs comprenant
I. un premier élément régulateur lié de manière fonctionnelle à un ARN guide de système CRISPR-Cas comprenant
(a) un polynucléotide d'ARN guide capable de s'hybrider avec une séquence cible dans une cellule eucaryote ;
(b) un polynucléotide d'ARN tracr mate, et
(c) un polynucléotide d'ARN tracr, et
II. un second élément régulateur lié de manière fonctionnelle à une séquence codant pour une protéine codant pour une protéine Cas9 comprenant au moins une ou plusieurs séquences de localisation nucléaire,
dans laquelle (a), (b) et (c) sont agencés dans une orientation 5' à 3',
dans laquelle les composants I et II sont situés sur des vecteurs identiques ou différents du système,
dans laquelle après transcription, le polynucléotide d'ARN tracr mate s'hybride avec le polynucléotide d'ARN tracr et le polynucléotide d'ARN guide dirige une liaison spécifique d'une séquence d'un complexe CRISPR à la séquence cible, et
dans laquelle le complexe CRISPR comprend la protéine Cas9 complexée avec (1) le polynucléotide d'ARN guide qui est hybridé avec la séquence cible, et (2) le polynucléotide d'ARN tracr mate qui est hybridé avec le polynucléotide d'ARN tracr, dans laquelle la protéine Cas9 est *Staphylococcus aureus* (SaCas9).

2. Composition selon la revendication 1, dans laquelle la protéine Cas9 a une ou plusieurs mutations dans un domaine quelconque de la protéine.

3. Composition selon la revendication 2, dans laquelle la protéine Cas9 a une ou plusieurs mutations dans un domaine catalytique.

4. Composition ou protéine selon la revendication 2 ou 3, dans laquelle les une ou les plusieurs mutations sont dans un domaine RuvC I, RuvC II, RuvC III ou HNH de la protéine Cas9.

5. Composition ou protéine selon la revendication 3 ou 4, dans laquelle la protéine Cas9 comprend une ou plusieurs mutations correspondant à la numérotation de position de SpCas9 en D10A, H840A, N854A, ou N863A.

6. Composition selon l'une quelconque des revendications précédentes, dans laquelle la protéine Cas9 comprend en outre un domaine fonctionnel.

7. Composition selon la revendication 6, dans laquelle le domaine fonctionnel est un domaine d'activation transcriptionnelle.

8. Composition selon la revendication 7, dans laquelle le domaine d'activation transcriptionnelle est VP64.

9. Composition selon la revendication 6, dans laquelle le domaine fonctionnel est un domaine de répression transcriptionnelle.

10. Composition selon la revendication 9, dans laquelle le domaine de répression transcriptionnelle est KRAB, SID ou SID4X.

11. Composition selon la revendication 6, dans laquelle le domaine fonctionnel est une recombinase, une transposase, un remodeleur d'histone, une ADN méthyltransférase, un cryptochrome et un domaine photo-inductible/commandable ou un domaine chimiquement inductible/commandable.

12. Composition selon l'une quelconque des revendications précédentes, dans laquelle la séquence polynucléotidique codant pour la protéine Cas9 est codon-optimisée pour une expression dans une cellule eucaryote.

13. Composition selon l'une quelconque des revendications précédentes, comprenant deux ARN guides.

14. Composition selon l'une quelconque des revendications précédentes, comprenant un système de vecteur comprenant un ou plusieurs vecteurs, et dans laquelle les séquences polynucléotidiques comprenant ou codant pour lesdits composants I et II sont situées sur des vecteurs identiques ou différents du système.

15. Composition selon la revendication 14, dans laquelle le système de vecteur comprend un vecteur unique.

16. Composition selon la revendication 14, dans laquelle les un ou plusieurs vecteurs comprennent un ou plusieurs vecteurs viraux.

17. Composition selon la revendication 16, dans laquelle les un ou plusieurs vecteurs viraux comprennent un ou plusieurs vecteurs rétroviraux, lentiviraux, viraux adéno-associés ou viraux herpès simplex.

18. Composition selon l'une quelconque des revendications 1 à 15, comprenant une nanoparticule, un liposome, un exosome, un système de levure ou une microvésicule.

19. Cellule hôte ou lignée cellulaire *ex vivo* ou *in vitro* comprenant la composition selon l'une quelconque des revendications précédentes, ou la descendance de celles-ci.

20. Cellule hôte, lignée cellulaire ou descendance de celles-ci *ex vivo* ou *in vitro* selon la revendication 19, qui est une cellule souche ou une lignée de cellules souches.

21. Procédé de modification d'un organisme par manipulation d'une ou plusieurs séquences cibles au niveau de loci génomiques d'intérêt, comprenant l'administration à l'organisme de la composition selon l'une quelconque des revendications 1 à 18, dans lequel l'organisme est un organisme non-animal.

22. Procédé *ex vivo* de modification d'une cellule d'un organisme par manipulation d'une ou de plusieurs séquences cibles au niveau de loci génomiques d'intérêt comprenant l'administration à la cellule d'une composition selon l'une quelconque des revendications 1 à 18, dans lequel le procédé ne comprend pas de processus de modification de l'identité génétique de la lignée germinale d'un être humain.

23. Procédé selon la revendication 21 ou 22, dans lequel l'organisme est une plante ou une algue.

24. Utilisation de la composition selon l'une quelconque des revendications 1 à 18 pour l'édition de gène ou de génome *ex vivo,* dans laquelle l'utilisation ne comprend pas de procédé de modification de l'identité génétique de la lignée germinale d'un être humain.

25. Utilisation de la composition selon l'une quelconque des revendications 1 à 18 pour la préparation d'un médicament.

26. Composition selon l'une quelconque des revendications 1 à 18 pour son utilisation dans la correction des troubles oculaires découlant de mutations génétiques.


### SEQUENCE LISTING

<110> THE BROAD INSTITUTE, INC. MASSACHUSETTS INSTITUTE OF TECHNOLOGY PRESIDENT AND FELLOWS OF HARVARD COLLEGE
<120> ENGINEERING AND OPTIMIZATION OF IMPROVED SYSTEMS, METHODS AND ENZYME COMPOSITIONS FOR SEQUENCE MANIPULATION
<130> 1199-1001EP Divl
<140> EP DIVISIONAL DERIVED FROM EP APPLN NO. 13814364.9 (PCT/US2013/074691)
   <141> 2013-12-12
<150> 61/836,101
   <151> 2013-06-17
<150> 61/835,931
   <151> 2013-06-17
<150> 61/828,130
   <151> 2013-05-28
<150> 61/819,803
   <151> 2013-05-06
<150> 61/814,263
   <151> 2013-04-20
<150> 61/806,375
   <151> 2013-03-28
<150> 61/802,174
   <151> 2013-03-15
<150> 61/791,409
   <151> 2013-03-15
<150> 61/769,046
   <151> 2013-02-25
<150> 61/758,468
   <151> 2013-01-30
<150> 61/748,427
   <151> 2013-01-02
<150> 61/736,527
   <151> 2012-12-12
<160> 228
<170> PatentIn version 3.5
<210> 1
   <211> 20
   <212> DNA
   <213> Homo sapiens
<400> 1
   gagtccgagc agaagaagaa 20
<210> 2
   <211> 20
   <212> DNA
   <213> Homo sapiens
<400> 2
   gagtcctagc aggagaagaa 20
<210> 3
   <211> 20
   <212> DNA
   <213> Homo sapiens
<400> 3
   gagtctaagc agaagaagaa 20
<210> 4
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, u, g, unknown or other
<220>
   <221> modified_base
   <222> (23)..(44)
   <223> a, c, u, g, unknown or other
<220>
   <221> modified_base
   <222> (47)..(60)
   <223> a, c, u, g, unknown or other
<400> 4
   nnnnnnnnnn nnnnnnnnnn ccnnnnnnnn nnnnnnnnnn nnnnggnnnn nnnnnnnnnn 60
<210> 5
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(14)
   <223> a, c, u, g, unknown or other
<220>
   <221> modified_base
   <222> (17)..(38)
   <223> a, c, u, g, unknown or other
<220>
   <221> modified_base
   <222> (41)..(60)
   <223> a, c, u, g, unknown or other
<400> 5
   nnnnnnnnnn nnnnccnnnn nnnnnnnnnn nnnnnnnngg nnnnnnnnnn nnnnnnnnnn 60
<210> 6
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, u, g, unknown or other
<220>
   <221> modified_base
   <222> (23)..(43)
   <223> a, c, u, g, unknown or other
<220>
   <221> modified_base
   <222> (46)..(60)
   <223> a, c, u, g, unknown or other
<400> 6
   nnnnnnnnnn nnnnnnnnnn ccnnnnnnnn nnnnnnnnnn nnnggnnnnn nnnnnnnnnn 60
<210> 7
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(15)
   <223> a, c, u, g, unknown or other
<220>
   <221> modified_base
   <222> (18)..(38)
   <223> a, c, u, g, unknown or other
<220>
   <221> modified_base
   <222> (41)..(60)
   <223> a, c, u, g, unknown or other
<400> 7
   nnnnnnnnnn nnnnnccnnn nnnnnnnnnn nnnnnnnngg nnnnnnnnnn nnnnnnnnnn 60
<210> 8
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, u, g, unknown or other
<220>
   <221> modified_base
   <222> (23)..(42)
   <223> a, c, u, g, unknown or other
<220>
   <221> modified_base
   <222> (45)..(60)
   <223> a, c, u, g, unknown or other
<400> 8
   nnnnnnnnnn nnnnnnnnnn ccnnnnnnnn nnnnnnnnnn nnggnnnnnn nnnnnnnnnn 60
<210> 9
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(15)
   <223> a, c, u, g, unknown or other
<220>
   <221> modified_base
   <222> (18)..(38)
   <223> a, c, u, g, unknown or other
<220>
   <221> modified_base
   <222> (41)..(60)
   <223> a, c, u, g, unknown or other
<400> 9
   nnnnnnnnnn nnnnnccnnn nnnnnnnnnn nnnnnnnngg nnnnnnnnnn nnnnnnnnnn 60
<210> 10
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, u, g, unknown or other
<220>
   <221> modified_base
   <222> (23)..(41)
   <223> a, c, u, g, unknown or other
<220>
   <221> modified_base
   <222> (44)..(60)
   <223> a, c, u, g, unknown or other
<400> 10
   nnnnnnnnnn nnnnnnnnnn ccnnnnnnnn nnnnnnnnnn nggnnnnnnn nnnnnnnnnn 60
<210> 11
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(17)
   <223> a, c, u, g, unknown or other
<220>
   <221> modified_base
   <222> (20)..(38)
   <223> a, c, u, g, unknown or other
<220>
   <221> modified_base
   <222> (41)..(60)
   <223> a, c, u, g, unknown or other
<400> 11
   nnnnnnnnnn nnnnnnnccn nnnnnnnnnn nnnnnnnngg nnnnnnnnnn nnnnnnnnnn 60
<210> 12
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, u, g, unknown or other
<220>
   <221> modified_base
   <222> (23)..(40)
   <223> a, c, u, g, unknown or other
<220>
   <221> modified_base
   <222> (43)..(60)
   <223> a, c, u, g, unknown or other
<400> 12
   nnnnnnnnnn nnnnnnnnnn ccnnnnnnnn nnnnnnnnnn ggnnnnnnnn nnnnnnnnnn 60
<210> 13
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(18)
   <223> a, c, u, g, unknown or other
<220>
   <221> modified_base
   <222> (21)..(38)
   <223> a, c, u, g, unknown or other
<220>
   <221> modified_base
   <222> (41)..(60)
   <223> a, c, u, g, unknown or other
<400> 13
   nnnnnnnnnn nnnnnnnncc nnnnnnnnnn nnnnnnnngg nnnnnnnnnn nnnnnnnnnn 60
<210> 14
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, u, g, unknown or other
<220>
   <221> modified_base
   <222> (23)..(39)
   <223> a, c, u, g, unknown or other
<220>
   <221> modified_base
   <222> (42)..(60)
   <223> a, c, u, g, unknown or other
<400> 14
   nnnnnnnnnn nnnnnnnnnn ccnnnnnnnn nnnnnnnnng gnnnnnnnnn nnnnnnnnnn 60
<210> 15
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(19)
   <223> a, c, u, g, unknown or other
<220>
   <221> modified_base
   <222> (22)..(38)
   <223> a, c, u, g, unknown or other
<220>
   <221> modified_base
   <222> (41)..(60)
   <223> a, c, u, g, unknown or other
<400> 15
   nnnnnnnnnn nnnnnnnnnc cnnnnnnnnn nnnnnnnngg nnnnnnnnnn nnnnnnnnnn 60
<210> 16
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, u, g, unknown or other
<220>
   <221> modified_base
   <222> (23)..(38)
   <223> a, c, u, g, unknown or other
<220>
   <221> modified_base
   <222> (41)..(60)
   <223> a, c, u, g, unknown or other
<400> 16
   nnnnnnnnnn nnnnnnnnnn ccnnnnnnnn nnnnnnnngg nnnnnnnnnn nnnnnnnnnn 60
<210> 17
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, u, g, unknown or other
<220>
   <221> modified_base
   <222> (23)..(38)
   <223> a, c, u, g, unknown or other
<220>
   <221> modified_base
   <222> (41)..(60)
   <223> a, c, u, g, unknown or other
<400> 17
   nnnnnnnnnn nnnnnnnnnn ccnnnnnnnn nnnnnnnngg nnnnnnnnnn nnnnnnnnnn 60
<210> 18
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, u, g, unknown or other
<220>
   <221> modified_base
   <222> (23)..(37)
   <223> a, c, u, g, unknown or other
<220>
   <221> modified_base
   <222> (40)..(60)
   <223> a, c, u, g, unknown or other
<400> 18
   nnnnnnnnnn nnnnnnnnnn ccnnnnnnnn nnnnnnnggn nnnnnnnnnn nnnnnnnnnn 60
<210> 19
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(21)
   <223> a, c, u, g, unknown or other
<220>
   <221> modified_base
   <222> (24)..(38)
   <223> a, c, u, g, unknown or other
<220>
   <221> modified_base
   <222> (41)..(60)
   <223> a, c, u, g, unknown or other
<400> 19
   nnnnnnnnnn nnnnnnnnnn nccnnnnnnn nnnnnnnngg nnnnnnnnnn nnnnnnnnnn 60
<210> 20
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, u, g, unknown or other
<220>
   <221> modified_base
   <222> (23).. (36)
   <223> a, c, u, g, unknown or other
<220>
   <221> modified_base
   <222> (39)..(60)
   <223> a, c, u, g, unknown or other
<400> 20
   nnnnnnnnnn nnnnnnnnnn ccnnnnnnnn nnnnnnggnn nnnnnnnnnn nnnnnnnnnn 60
<210> 21
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(22)
   <223> a, c, u, g, unknown or other
<220>
   <221> modified_base
   <222> (25)..(38)
   <223> a, c, u, g, unknown or other
<220>
   <221> modified_base
   <222> (41)..(60)
   <223> a, c, u, g, unknown or other
<400> 21
   nnnnnnnnnn nnnnnnnnnn nnccnnnnnn nnnnnnnngg nnnnnnnnnn nnnnnnnnnn 60
<210> 22
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, u, g, unknown or other
<220>
   <221> modified_base
   <222> (23)..(35)
   <223> a, c, u, g, unknown or other
<220>
   <221> modified_base
   <222> (38)..(60)
   <223> a, c, u, g, unknown or other
<400> 22
   nnnnnnnnnn nnnnnnnnnn ccnnnnnnnn nnnnnggnnn nnnnnnnnnn nnnnnnnnnn 60
<210> 23
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(23)
   <223> a, c, u, g, unknown or other
<220>
   <221> modified_base
   <222> (26)..(38)
   <223> a, c, u, g, unknown or other
<220>
   <221> modified_base
   <222> (41)..(60)
   <223> a, c, u, g, unknown or other
<400> 23
   nnnnnnnnnn nnnnnnnnnn nnnccnnnnn nnnnnnnngg nnnnnnnnnn nnnnnnnnnn 60
<210> 24
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, u, g, unknown or other
<220>
   <221> modified_base
   <222> (23)..(34)
   <223> a, c, u, g, unknown or other
<220>
   <221> modified_base
   <222> (37)..(60)
   <223> a, c, u, g, unknown or other
<400> 24
   nnnnnnnnnn nnnnnnnnnn ccnnnnnnnn nnnnggnnnn nnnnnnnnnn nnnnnnnnnn 60
<210> 25
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(24)
   <223> a, c, u, g, unknown or other
<220>
   <221> modified_base
   <222> (27)..(38)
   <223> a, c, u, g, unknown or other
<220>
   <221> modified_base
   <222> (41)..(60)
   <223> a, c, u, g, unknown or other
<400> 25
   nnnnnnnnnn nnnnnnnnnn nnnnccnnnn nnnnnnnngg nnnnnnnnnn nnnnnnnnnn 60
<210> 26
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, u, g, unknown or other
<220>
   <221> modified_base
   <222> (23)..(33)
   <223> a, c, u, g, unknown or other
<220>
   <221> modified_base
   <222> (36)..(60)
   <223> a, c, u, g, unknown or other
<400> 26
   nnnnnnnnnn nnnnnnnnnn ccnnnnnnnn nnnggnnnnn nnnnnnnnnn nnnnnnnnnn 60
<210> 27
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(25)
   <223> a, c, u, g, unknown or other
<220>
   <221> modified_base
   <222> (28)..(38)
   <223> a, c, u, g, unknown or other
<220>
   <221> modified_base
   <222> (41)..(60)
   <223> a, c, u, g, unknown or other
<400> 27
   nnnnnnnnnn nnnnnnnnnn nnnnnccnnn nnnnnnnngg nnnnnnnnnn nnnnnnnnnn 60
<210> 28
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, u, g, unknown or other
<220>
   <221> modified_base
   <222> (23)..(32)
   <223> a, c, u, g, unknown or other
<220>
   <221> modified_base
   <222> (35)..(60)
   <223> a, c, u, g, unknown or other
<400> 28
   nnnnnnnnnn nnnnnnnnnn ccnnnnnnnn nnggnnnnnn nnnnnnnnnn nnnnnnnnnn 60
<210> 29
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(26)
   <223> a, c, u, g, unknown or other
<220>
   <221> modified_base
   <222> (29)..(38)
   <223> a, c, u, g, unknown or other
<220>
   <221> modified_base
   <222> (41)..(60)
   <223> a, c, u, g, unknown or other
<400> 29
   nnnnnnnnnn nnnnnnnnnn nnnnnnccnn nnnnnnnngg nnnnnnnnnn nnnnnnnnnn 60
<210> 30
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, u, g, unknown or other
<220>
   <221> modified_base
   <222> (23)..(31)
   <223> a, c, u, g, unknown or other
<220>
   <221> modified_base
   <222> (34)..(60)
   <223> a, c, u, g, unknown or other
<400> 30
   nnnnnnnnnn nnnnnnnnnn ccnnnnnnnn nggnnnnnnn nnnnnnnnnn nnnnnnnnnn 60
<210> 31
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(27)
   <223> a, c, u, g, unknown or other
<220>
   <221> modified_base
   <222> (30)..(38)
   <223> a, c, u, g, unknown or other
<220>
   <221> modified_base
   <222> (41)..(60)
   <223> a, c, u, g, unknown or other
<400> 31
   nnnnnnnnnn nnnnnnnnnn nnnnnnnccn nnnnnnnngg nnnnnnnnnn nnnnnnnnnn 60
<210> 32
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, u, g, unknown or other
<220>
   <221> modified_base
   <222> (23)..(30)
   <223> a, c, u, g, unknown or other
<220>
   <221> modified_base
   <222> (33)..(60)
   <223> a, c, u, g, unknown or other
<400> 32
   nnnnnnnnnn nnnnnnnnnn ccnnnnnnnn ggnnnnnnnn nnnnnnnnnn nnnnnnnnnn 60
<210> 33
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(28)
   <223> a, c, u, g, unknown or other
<220>
   <221> modified_base
   <222> (31)..(38)
   <223> a, c, u, g, unknown or other
<220>
   <221> modified_base
   <222> (41)..(60)
   <223> a, c, u, g, unknown or other
<400> 33
   nnnnnnnnnn nnnnnnnnnn nnnnnnnncc nnnnnnnngg nnnnnnnnnn nnnnnnnnnn 60
<210> 34
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, u, g, unknown or other
<220>
   <221> modified_base
   <222> (23)..(29)
   <223> a, c, u, g, unknown or other
<220>
   <221> modified_base
   <222> (32)..(60)
   <223> a, c, u, g, unknown or other
<400> 34
   nnnnnnnnnn nnnnnnnnnn ccnnnnnnng gnnnnnnnnn nnnnnnnnnn nnnnnnnnnn 60
<210> 35
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(29)
   <223> a, c, u, g, unknown or other
<220>
   <221> modified_base
   <222> (32)..(38)
   <223> a, c, u, g, unknown or other
<220>
   <221> modified_base
   <222> (41)..(60)
   <223> a, c, u, g, unknown or other
<400> 35
   nnnnnnnnnn nnnnnnnnnn nnnnnnnnnc cnnnnnnngg nnnnnnnnnn nnnnnnnnnn 60
<210> 36
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, u, g, unknown or other
<220>
   <221> modified_base
   <222> (23)..(28)
   <223> a, c, u, g, unknown or other
<220>
   <221> modified_base
   <222> (31)..(60)
   <223> a, c, u, g, unknown or other
<400> 36
   nnnnnnnnnn nnnnnnnnnn ccnnnnnngg nnnnnnnnnn nnnnnnnnnn nnnnnnnnnn 60
<210> 37
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(30)
   <223> a, c, u, g, unknown or other
<220>
   <221> modified_base
   <222> (33)..(38)
   <223> a, c, u, g, unknown or other
<220>
   <221> modified_base
   <222> (41)..(60)
   <223> a, c, u, g, unknown or other
<400> 37
   nnnnnnnnnn nnnnnnnnnn nnnnnnnnnn ccnnnnnngg nnnnnnnnnn nnnnnnnnnn 60
<210> 38
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, u, g, unknown or other
<220>
   <221> modified_base
   <222> (23)..(27)
   <223> a, c, u, g, unknown or other
<220>
   <221> modified_base
   <222> (30)..(60)
   <223> a, c, u, g, unknown or other
<400> 38
   nnnnnnnnnn nnnnnnnnnn ccnnnnnggn nnnnnnnnnn nnnnnnnnnn nnnnnnnnnn 60
<210> 39
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(31)
   <223> a, c, u, g, unknown or other
<220>
   <221> modified_base
   <222> (34)..(38)
   <223> a, c, u, g, unknown or other
<220>
   <221> modified_base
   <222> (41)..(60)
   <223> a, c, u, g, unknown or other
<400> 39
   nnnnnnnnnn nnnnnnnnnn nnnnnnnnnn nccnnnnngg nnnnnnnnnn nnnnnnnnnn 60
<210> 40
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, u, g, unknown or other
<220>
   <221> modified_base
   <222> (23)..(26)
   <223> a, c, u, g, unknown or other
<220>
   <221> modified_base
   <222> (29)..(60)
   <223> a, c, u, g, unknown or other
<400> 40
   nnnnnnnnnn nnnnnnnnnn ccnnnnggnn nnnnnnnnnn nnnnnnnnnn nnnnnnnnnn 60
<210> 41
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(32)
   <223> a, c, u, g, unknown or other
<220>
   <221> modified_base
   <222> (35)..(38)
   <223> a, c, u, g, unknown or other
<220>
   <221> modified_base
   <222> (41)..(60)
   <223> a, c, u, g, unknown or other
<400> 41
   nnnnnnnnnn nnnnnnnnnn nnnnnnnnnn nnccnnnngg nnnnnnnnnn nnnnnnnnnn 60
<210> 42
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, u, g, unknown or other
<220>
   <221> modified_base
   <222> (23)..(25)
   <223> a, c, u, g, unknown or other
<220>
   <221> modified_base
   <222> (28)..(60)
   <223> a, c, u, g, unknown or other
<400> 42
   nnnnnnnnnn nnnnnnnnnn ccnnnggnnn nnnnnnnnnn nnnnnnnnnn nnnnnnnnnn 60
<210> 43
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(33)
   <223> a, c, u, g, unknown or other
<220>
   <221> modified_base
   <222> (36)..(38)
   <223> a, c, u, g, unknown or other
<220>
   <221> modified_base
   <222> (41)..(60)
   <223> a, c, u, g, unknown or other
<400> 43
   nnnnnnnnnn nnnnnnnnnn nnnnnnnnnn nnnccnnngg nnnnnnnnnn nnnnnnnnnn 60
<210> 44
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, u, g, unknown or other
<220>
   <221> modified_base
   <222> (23)..(24)
   <223> a, c, u, g, unknown or other
<220>
   <221> modified_base
   <222> (27)..(60)
   <223> a, c, u, g, unknown or other
<400> 44
   nnnnnnnnnn nnnnnnnnnn ccnnggnnnn nnnnnnnnnn nnnnnnnnnn nnnnnnnnnn 60
<210> 45
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(34)
   <223> a, c, u, g, unknown or other
<220>
   <221> modified_base
   <222> (37)..(38)
   <223> a, c, u, g, unknown or other
<220>
   <221> modified_base
   <222> (41)..(60)
   <223> a, c, u, g, unknown or other
<400> 45
   nnnnnnnnnn nnnnnnnnnn nnnnnnnnnn nnnnccnngg nnnnnnnnnn nnnnnnnnnn 60
<210> 46
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, u, g, unknown or other
<220>
   <221> modified_base
   <222> (23)..(23)
   <223> a, c, u, g, unknown or other
<220>
   <221> modified_base
   <222> (26)..(60)
   <223> a, c, u, g, unknown or other
<400> 46
   nnnnnnnnnn nnnnnnnnnn ccnggnnnnn nnnnnnnnnn nnnnnnnnnn nnnnnnnnnn 60
<210> 47
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(35)
   <223> a, c, u, g, unknown or other
<220>
   <221> modified_base
   <222> (38)..(38)
   <223> a, c, u, g, unknown or other
<220>
   <221> modified_base
   <222> (41)..(60)
   <223> a, c, u, g, unknown or other
<400> 47
   nnnnnnnnnn nnnnnnnnnn nnnnnnnnnn nnnnnccngg nnnnnnnnnn nnnnnnnnnn 60
<210> 48
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(21)
   <223> a, c, u, g, unknown or other
<220>
   <221> modified_base
   <222> (26)..(60)
   <223> a, c, u, g, unknown or other
<400> 48
   nnnnnnnnnn nnnnnnnnnn nccggnnnnn nnnnnnnnnn nnnnnnnnnn nnnnnnnnnn 60
<210> 49
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(35)
   <223> a, c, u, g, unknown or other
<220>
   <221> modified_base
   <222> (40)..(60)
   <223> a, c, u, g, unknown or other
<400> 49
   nnnnnnnnnn nnnnnnnnnn nnnnnnnnnn nnnnnccggn nnnnnnnnnn nnnnnnnnnn 60
<210> 50
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(23)
   <223> a, c, u, g, unknown or other
<220>
   <221> modified_base
   <222> (26)..(60)
   <223> a, c, u, g, unknown or other
<400> 50
   nnnnnnnnnn nnnnnnnnnn nnnggnnnnn nnnnnnnnnn nnnnnnnnnn nnnnnnnnnn 60
<210> 51
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(33)
   <223> a, c, u, g, unknown or other
<220>
   <221> modified_base
   <222> (38)..(60)
   <223> a, c, u, g, unknown or other
<400> 51
   nnnnnnnnnn nnnnnnnnnn nnnnnnnnnn nnnggccnnn nnnnnnnnnn nnnnnnnnnn 60
<210> 52
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(22)
   <223> a, c, u, g, unknown or other
<220>
   <221> modified_base
   <222> (26)..(60)
   <223> a, c, u, g, unknown or other
<400> 52
   nnnnnnnnnn nnnnnnnnnn nncggnnnnn nnnnnnnnnn nnnnnnnnnn nnnnnnnnnn 60
<210> 53
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(32)
   <223> a, c, u, g, unknown or other
<220>
   <221> modified_base
   <222> (35)..(35)
   <223> a, c, u, g, unknown or other
<220>
   <221> modified_base
   <222> (38)..(60)
   <223> a, c, u, g, unknown or other
<400> 53
   nnnnnnnnnn nnnnnnnnnn nnnnnnnnnn nnggnccnnn nnnnnnnnnn nnnnnnnnnn 60
<210> 54
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(31)
   <223> a, c, u, g, unknown or other
<220>
   <221> modified_base
   <222> (34)..(35)
   <223> a, c, u, g, unknown or other
<220>
   <221> modified_base
   <222> (38)..(60)
   <223> a, c, u, g, unknown or other
<400> 54
   nnnnnnnnnn nnnnnnnnnn nnnnnnnnnn nggnnccnnn nnnnnnnnnn nnnnnnnnnn 60
<210> 55
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(30)
   <223> a, c, u, g, unknown or other
<220>
   <221> modified_base
   <222> (33)..(35)
   <223> a, c, u, g, unknown or other
<220>
   <221> modified_base
   <222> (38)..(60)
   <223> a, c, u, g, unknown or other
<400> 55
   nnnnnnnnnn nnnnnnnnnn nnnnnnnnnn ggnnnccnnn nnnnnnnnnn nnnnnnnnnn 60
<210> 56
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(29)
   <223> a, c, u, g, unknown or other
<220>
   <221> modified_base
   <222> (32)..(35)
   <223> a, c, u, g, unknown or other
<220>
   <221> modified_base
   <222> (38)..(60)
   <223> a, c, u, g, unknown or other
<400> 56
   nnnnnnnnnn nnnnnnnnnn nnnnnnnnng gnnnnccnnn nnnnnnnnnn nnnnnnnnnn 60
<210> 57
   <211> 60
   <212> RNA
   <213> Artificial Sequence
<220>
   <221> source
   <223> /note="Description of Artificial Sequence: Synthetic oligonucleotide"
<220>
   <221> modified_base
   <222> (1)..(28)
   <223> a, c, u, g, unknown or other
<220>
   <221> modified_base
   <222> (31)..(35)
   <223> a, c, u, g, unknown or other
<220>
   <221> modified_base
   <222> (38)..(60)
   <223> a, c, u, g, unknown or other
<400> 57
   nnnnnnnnnn nnnnnnnnnn nnnnnnnngg nnnnnccnnn nnnnnnnnnn nnnnnnnnnn 60
<210> 58
   <211> 12
   <212> RNA
   <213> Artificial Sequence
<220>
   <221> source
   <223> /note="Description of Artificial Sequence: Synthetic oligonucleotide"
<400> 58
   guuuuagagc ua 12
<210> 59
   <211> 7
   <212> PRT
   <213> Simian virus 40
<400> 59
   Pro Lys Lys Lys Arg Lys Val
   1               5
<210> 60
   <211> 16
   <212> PRT
   <213> Unknown
<220>
   <221> source
   <223> /note="Description of Unknown: Nucleoplasmin bipartite NLS sequence"
<400> 60
   Lys Arg Pro Ala Ala Thr Lys Lys Ala Gly Gln Ala Lys Lys Lys Lys
   1 5 10 15
<210> 61
   <211> 9
   <212> PRT
   <213> Unknown
<220>
   <221> source
   <223> /note="Description of Unknown: C-myc NLS sequence"
<400> 61
   Pro Ala Ala Lys Arg Val Lys Leu Asp
   1 5
<210> 62
   <211> 11
   <212> PRT
   <213> Unknown
<220>
   <221> source
   <223> /note="Description of Unknown: C-myc NLS sequence"
<400> 62
   Arg Gln Arg Arg Asn Glu Leu Lys Arg Ser Pro
   1 5 10
<210> 63
   <211> 38
   <212> PRT
   <213> Homo sapiens
<400> 63
   Asn Gln Ser Ser Asn Phe Gly Pro Met Lys Gly Gly Asn Phe Gly Gly
   1 5 10 15
   Arg Ser Ser Gly Pro Tyr Gly Gly Gly Gly Gln Tyr Phe Ala Lys Pro
   20 25 30
   Arg Asn Gln Gly Gly Tyr
   35
<210> 64
   <211> 42
   <212> PRT
   <213> Unknown
<220>
   <221> source
   <223> /note="Description of Unknown: IBB domain from importin-alpha sequence"
<400> 64
   Arg Met Arg Ile Glx Phe Lys Asn Lys Gly Lys Asp Thr Ala Glu Leu
   1               5                   10                  15
   Arg Arg Arg Arg Val Glu Val Ser Val Glu Leu Arg Lys Ala Lys Lys
               20                  25                  30
   Asp Glu Gln Ile Leu Lys Arg Arg Asn Val
           35                  40
<210> 65
   <211> 8
   <212> PRT
   <213> Unknown
<220>
   <221> source
   <223> /note="Description of Unknown: Myoma T protein sequence"
<400> 65
   Val Ser Arg Lys Arg Pro Arg Pro
   1               5
<210> 66
   <211> 8
   <212> PRT
   <213> Unknown
<220>
   <221> source
   <223> /note="Description of Unknown: Myoma T protein sequence"
<400> 66
   Pro Pro Lys Lys Ala Arg Glu Asp
   1               5
<210> 67
   <211> 8
   <212> PRT
   <213> Homo sapiens
<400> 67
   Pro Gln Pro Lys Lys Lys Pro Leu
   1               5
<210> 68
   <211> 12
   <212> PRT
   <213> Mus musculus
<400> 68
   Ser Ala Leu Ile Lys Lys Lys Lys Lys Met Ala Pro
   1 5 10
<210> 69
   <211> 5
   <212> PRT
   <213> Influenza virus
<400> 69
   Asp Arg Leu Arg Arg
   1 5
<210> 70
   <211> 7
   <212> PRT
   <213> Influenza virus
<400> 70
   Pro Lys Gln Lys Lys Arg Lys
   1 5
<210> 71
   <211> 10
   <212> PRT
   <213> Hepatitus delta virus
<400> 71
   Arg Lys Leu Lys Lys Lys Ile Lys Lys Leu
   1 5 10
<210> 72
   <211> 10
   <212> PRT
   <213> Mus musculus
<400> 72
   Arg Glu Lys Lys Lys Phe Leu Lys Arg Arg
   1 5 10
<210> 73
   <211> 20
   <212> PRT
   <213> Homo sapiens
<400> 73
   Lys Arg Lys Gly Asp Glu Val Asp Gly Val Asp Glu Val Ala Lys Lys
   1 5 10 15
   Lys Ser Lys Lys
   20
<210> 74
   <211> 17
   <212> PRT
   <213> Homo sapiens
<400> 74
   Arg Lys Cys Leu Gln Ala Gly Met Asn Leu Glu Ala Arg Lys Thr Lys
   1               5                   10                  15
   Lys
<210> 75
   <211> 137
   <212> DNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, t, g, unknown or other
<400> 75
   nnnnnnnnnn nnnnnnnnnn gtttttgtac tctcaagatt tagaaataaa tcttgcagaa      60
   gctacaaaga taaggcttca tgccgaaatc aacaccctgt cattttatgg cagggtgttt     120
   tcgttattta atttttt                                                    137
<210> 76
   <211> 123
   <212> DNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, t, g, unknown or other
<400> 76
   nnnnnnnnnn nnnnnnnnnn gtttttgtac tctcagaaat gcagaagcta caaagataag      60
   gcttcatgcc gaaatcaaca ccctgtcatt ttatggcagg gtgttttcgt tatttaattt     120
   ttt                                                                   123
<210> 77
   <211> 110
   <212> DNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, t, g, unknown or other
<400> 77
   nnnnnnnnnn nnnnnnnnnn gtttttgtac tctcagaaat gcagaagcta caaagataag 60
   gcttcatgcc gaaatcaaca ccctgtcatt ttatggcagg gtgttttttt 110
<210> 78
   <211> 102
   <212> DNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, t, g, unknown or other
<400> 78
   nnnnnnnnnn nnnnnnnnnn gttttagagc tagaaatagc aagttaaaat aaggctagtc 60
   cgttatcaac ttgaaaaagt ggcaccgagt cggtgctttt tt 102
<210> 79
   <211> 88
   <212> DNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, t, g, unknown or other
<400> 79
   nnnnnnnnnn nnnnnnnnnn gttttagagc tagaaatagc aagttaaaat aaggctagtc 60
   cgttatcaac ttgaaaaagt gttttttt 88
<210> 80
   <211> 76
   <212> DNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, t, g, unknown or other
<400> 80
   nnnnnnnnnn nnnnnnnnnn gttttagagc tagaaatagc aagttaaaat aaggctagtc 60
   cgttatcatt tttttt 76
<210> 81
   <211> 984
   <212> PRT
   <213> Campylobacter jejuni
<400> 81
   Met Ala Arg Ile Leu Ala Phe Asp Ile Gly Ile Ser Ser Ile Gly Trp
   1               5                   10                  15
   Ala Phe Ser Glu Asn Asp Glu Leu Lys Asp Cys Gly Val Arg Ile Phe
               20                  25                  30
   Thr Lys Val Glu Asn Pro Lys Thr Gly Glu Ser Leu Ala Leu Pro Arg
           35                  40                  45
   Arg Leu Ala Arg Ser Ala Arg Lys Arg Leu Ala Arg Arg Lys Ala Arg
       50                  55                  60
   Leu Asn His Leu Lys His Leu Ile Ala Asn Glu Phe Lys Leu Asn Tyr
   65                  70                  75                  80
   Glu Asp Tyr Gln Ser Phe Asp Glu Ser Leu Ala Lys Ala Tyr Lys Gly
                   85                  90                  95
   Ser Leu Ile Ser Pro Tyr Glu Leu Arg Phe Arg Ala Leu Asn Glu Leu
               100                 105                 110
   Leu Ser Lys Gln Asp Phe Ala Arg Val Ile Leu His Ile Ala Lys Arg
           115                 120                 125
   Arg Gly Tyr Asp Asp Ile Lys Asn Ser Asp Asp Lys Glu Lys Gly Ala
   130 135 140
   Ile Leu Lys Ala Ile Lys Gln Asn Glu Glu Lys Leu Ala Asn Tyr Gln
   145 150 155 160
   Ser Val Gly Glu Tyr Leu Tyr Lys Glu Tyr Phe Gln Lys Phe Lys Glu
   165 170 175
   Asn Ser Lys Glu Phe Thr Asn Val Arg Asn Lys Lys Glu Ser Tyr Glu
   180 185 190
   Arg Cys Ile Ala Gln Ser Phe Leu Lys Asp Glu Leu Lys Leu Ile Phe
   195 200 205
   Lys Lys Gln Arg Glu Phe Gly Phe Ser Phe Ser Lys Lys Phe Glu Glu
   210 215 220
   Glu Val Leu Ser Val Ala Phe Tyr Lys Arg Ala Leu Lys Asp Phe Ser
   225 230 235 240
   His Leu Val Gly Asn Cys Ser Phe Phe Thr Asp Glu Lys Arg Ala Pro
   245 250 255
   Lys Asn Ser Pro Leu Ala Phe Met Phe Val Ala Leu Thr Arg Ile Ile
   260 265 270
   Asn Leu Leu Asn Asn Leu Lys Asn Thr Glu Gly Ile Leu Tyr Thr Lys
   275 280 285
   Asp Asp Leu Asn Ala Leu Leu Asn Glu Val Leu Lys Asn Gly Thr Leu
   290 295 300
   Thr Tyr Lys Gln Thr Lys Lys Leu Leu Gly Leu Ser Asp Asp Tyr Glu
   305 310 315 320
   Phe Lys Gly Glu Lys Gly Thr Tyr Phe Ile Glu Phe Lys Lys Tyr Lys
   325 330 335
   Glu Phe Ile Lys Ala Leu Gly Glu His Asn Leu Ser Gln Asp Asp Leu
   340 345 350
   Asn Glu Ile Ala Lys Asp Ile Thr Leu Ile Lys Asp Glu Ile Lys Leu
   355 360 365
   Lys Lys Ala Leu Ala Lys Tyr Asp Leu Asn Gln Asn Gln Ile Asp Ser
   370 375 380
   Leu Ser Lys Leu Glu Phe Lys Asp His Leu Asn Ile Ser Phe Lys Ala
   385 390 395 400
   Leu Lys Leu Val Thr Pro Leu Met Leu Glu Gly Lys Lys Tyr Asp Glu
   405 410 415
   Ala Cys Asn Glu Leu Asn Leu Lys Val Ala Ile Asn Glu Asp Lys Lys
   420 425 430
   Asp Phe Leu Pro Ala Phe Asn Glu Thr Tyr Tyr Lys Asp Glu Val Thr
   435 440 445
   Asn Pro Val Val Leu Arg Ala Ile Lys Glu Tyr Arg Lys Val Leu Asn
   450 455 460
   Ala Leu Leu Lys Lys Tyr Gly Lys Val His Lys Ile Asn Ile Glu Leu
   465 470 475 480
   Ala Arg Glu Val Gly Lys Asn His Ser Gln Arg Ala Lys Ile Glu Lys
   485 490 495
   Glu Gln Asn Glu Asn Tyr Lys Ala Lys Lys Asp Ala Glu Leu Glu Cys
   500 505 510
   Glu Lys Leu Gly Leu Lys Ile Asn Ser Lys Asn Ile Leu Lys Leu Arg
   515 520 525
   Leu Phe Lys Glu Gln Lys Glu Phe Cys Ala Tyr Ser Gly Glu Lys Ile
   530 535 540
   Lys Ile Ser Asp Leu Gln Asp Glu Lys Met Leu Glu Ile Asp His Ile
   545 550 555 560
   Tyr Pro Tyr Ser Arg Ser Phe Asp Asp Ser Tyr Met Asn Lys Val Leu
   565 570 575
   Val Phe Thr Lys Gln Asn Gln Glu Lys Leu Asn Gln Thr Pro Phe Glu
   580 585 590
   Ala Phe Gly Asn Asp Ser Ala Lys Trp Gln Lys Ile Glu Val Leu Ala
   595 600 605
   Lys Asn Leu Pro Thr Lys Lys Gln Lys Arg Ile Leu Asp Lys Asn Tyr
   610 615 620
   Lys Asp Lys Glu Gln Lys Asn Phe Lys Asp Arg Asn Leu Asn Asp Thr
   625 630 635 640
   Arg Tyr Ile Ala Arg Leu Val Leu Asn Tyr Thr Lys Asp Tyr Leu Asp
                   645                 650                 655
   Phe Leu Pro Leu Ser Asp Asp Glu Asn Thr Lys Leu Asn Asp Thr Gln
               660                 665                 670
   Lys Gly Ser Lys Val His Val Glu Ala Lys Ser Gly Met Leu Thr Ser
           675                 680                 685
   Ala Leu Arg His Thr Trp Gly Phe Ser Ala Lys Asp Arg Asn Asn His
       690                 695                 700
   Leu His His Ala Ile Asp Ala Val Ile Ile Ala Tyr Ala Asn Asn Ser
   705                 710                 715                 720
   Ile Val Lys Ala Phe Ser Asp Phe Lys Lys Glu Gln Glu Ser Asn Ser
                   725                 730                 735
   Ala Glu Leu Tyr Ala Lys Lys Ile Ser Glu Leu Asp Tyr Lys Asn Lys
               740                 745                 750
   Arg Lys Phe Phe Glu Pro Phe Ser Gly Phe Arg Gln Lys Val Leu Asp
           755                 760                 765
   Lys Ile Asp Glu Ile Phe Val Ser Lys Pro Glu Arg Lys Lys Pro Ser
       770                 775                 780
   Gly Ala Leu His Glu Glu Thr Phe Arg Lys Glu Glu Glu Phe Tyr Gln
   785                 790                 795                 800
   Ser Tyr Gly Gly Lys Glu Gly Val Leu Lys Ala Leu Glu Leu Gly Lys
                   805                 810                 815
   Ile Arg Lys Val Asn Gly Lys Ile Val Lys Asn Gly Asp Met Phe Arg
               820                 825                 830
   Val Asp Ile Phe Lys His Lys Lys Thr Asn Lys Phe Tyr Ala Val Pro
           835                 840                 845
   Ile Tyr Thr Met Asp Phe Ala Leu Lys Val Leu Pro Asn Lys Ala Val
       850                 855                 860
   Ala Arg Ser Lys Lys Gly Glu Ile Lys Asp Trp Ile Leu Met Asp Glu
   865                 870                 875                 880
   Asn Tyr Glu Phe Cys Phe Ser Leu Tyr Lys Asp Ser Leu Ile Leu Ile
                   885                 890                 895
   Gln Thr Lys Asp Met Gln Glu Pro Glu Phe Val Tyr Tyr Asn Ala Phe
   900 905 910
   Thr Ser Ser Thr Val Ser Leu Ile Val Ser Lys His Asp Asn Lys Phe
   915 920 925
   Glu Thr Leu Ser Lys Asn Gln Lys Ile Leu Phe Lys Asn Ala Asn Glu
   930 935 940
   Lys Glu Val Ile Ala Lys Ser Ile Gly Ile Gln Asn Leu Lys Val Phe
   945 950 955 960
   Glu Lys Tyr Ile Val Ser Ala Leu Gly Glu Val Thr Lys Ala Glu Phe
   965 970 975
   Arg Gln Arg Glu Asp Phe Lys Lys
   980
<210> 82
   <211> 91
   <212> DNA
   <213> Artificial Sequence
<220>
   <221> source
   <223> /note="Description of Artificial Sequence: Synthetic oligonucleotide"
<400> 82
   tataatctca taagaaattt aaaaagggac taaaataaag agtttgcggg actctgcggg 60
   gttacaatcc cctaaaaccg cttttaaaat t 91
<210> 83
   <211> 36
   <212> DNA
   <213> Artificial Sequence
<220>
   <221> source
   <223> /note="Description of Artificial Sequence: Synthetic oligonucleotide"
<400> 83
   gttttagtcc ctttttaaat ttctttatgg taaaat 36
<210> 84
   <211> 95
   <212> RNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, u, g, unknown or other
<400> 84
   nnnnnnnnnn nnnnnnnnnn guuuuagucc cgaaagggac uaaaauaaag aguuugcggg        60
   acucugcggg guuacaaucc ccuaaaaccg cuuuu                                    95
<210> 85
   <211> 3243
   <212> DNA
   <213> Artificial Sequence
<220>
   <221> source
   <223> /note="Description of Artificial Sequence: Synthetic polynucleotide"
<400> 85
   accggtgcca ccatgtaccc atacgatgtt ccagattacg cttcgccgaa gaaaaagcgc        60
   aaggtcgaag cgtccatgaa aaggaactac attctggggc tggacatcgg gattacaagc       120
   gtggggtatg ggattattga ctatgaaaca agggacgtga tcgacgcagg cgtcagactg       180
   ttcaaggagg ccaacgtgga aaacaatgag ggacggagaa gcaagagggg agccaggcgc       240
   ctgaaacgac ggagaaggca cagaatccag agggtgaaga aactgctgtt cgattacaac       300
   ctgctgaccg accattctga gctgagtgga attaatcctt atgaagccag ggtgaaaggc       360
   ctgagtcaga agctgtcaga ggaagagttt tccgcagctc tgctgcacct ggctaagcgc       420
   cgaggagtgc ataacgtcaa tgaggtggaa gaggacaccg gcaacgagct gtctacaaag       480
   gaacagatct cacgcaatag caaagctctg gaagagaagt atgtcgcaga gctgcagctg       540
   gaacggctga agaaagatgg cgaggtgaga gggtcaatta ataggttcaa gacaagcgac       600
   tacgtcaaag aagccaagca gctgctgaaa gtgcagaagg cttaccacca gctggatcag       660
   agcttcatcg atacttatat cgacctgctg gagactcgga gaacctacta tgagggacca       720
   ggagaaggga gccccttcgg atggaaagac atcaaggaat ggtacgagat gctgatggga       780
   cattgcacct attttccaga agagctgaga agcgtcaagt acgcttataa cgcagatctg       840
   tacaacgccc tgaatgacct gaacaacctg gtcatcacca gggatgaaaa cgagaaactg       900
   gaatactatg agaagttcca gatcatcgaa aacgtgttta agcagaagaa aaagcctaca       960
   ctgaaacaga ttgctaagga gatcctggtc aacgaagagg acatcaaggg ctaccgggtg      1020
   acaagcactg gaaaaccaga gttcaccaat ctgaaagtgt atcacgatat taaggacatc      1080
   acagcacgga aagaaatcat tgagaacgcc gaactgctgg atcagattgc taagatcctg      1140
   actatctacc agagctccga ggacatccag gaagagctga ctaacctgaa cagcgagctg 1200
   acccaggaag agatcgaaca gattagtaat ctgaaggggt acaccggaac acacaacctg 1260
   tccctgaaag ctatcaatct gattctggat gagctgtggc atacaaacga caatcagatt 1320
   gcaatcttta accggctgaa gctggtccca aaaaaggtgg acctgagtca gcagaaagag 1380
   atcccaacca cactggtgga cgatttcatt ctgtcacccg tggtcaagcg gagcttcatc 1440
   cagagcatca aagtgatcaa cgccatcatc aagaagtacg gcctgcccaa tgatatcatt 1500
   atcgagctgg ctagggagaa gaacagcaag gacgcacaga agatgatcaa tgagatgcag 1560
   aaacgaaacc ggcagaccaa tgaacgcatt gaagagatta tccgaactac cgggaaagag 1620
   aacgcaaagt acctgattga aaaaatcaag ctgcacgata tgcaggaggg aaagtgtctg 1680
   tattctctgg aggccatccc cctggaggac ctgctgaaca atccattcaa ctacgaggtc 1740
   gatcatatta tccccagaag cgtgtccttc gacaattcct ttaacaacaa ggtgctggtc 1800
   aagcaggaag agaactctaa aaagggcaat aggactcctt tccagtacct gtctagttca 1860
   gattccaaga tctcttacga aacctttaaa aagcacattc tgaatctggc caaaggaaag 1920
   ggccgcatca gcaagaccaa aaaggagtac ctgctggaag agcgggacat caacagattc 1980
   tccgtccaga aggattttat taaccggaat ctggtggaca caagatacgc tactcgcggc 2040
   ctgatgaatc tgctgcgatc ctatttccgg gtgaacaatc tggatgtgaa agtcaagtcc 2100
   atcaacggcg ggttcacatc ttttctgagg cgcaaatgga agtttaaaaa ggagcgcaac 2160
   aaagggtaca agcaccatgc cgaagatgct ctgattatcg caaatgccga cttcatcttt 2220
   aaggagtgga aaaagctgga caaagccaag aaagtgatgg agaaccagat gttcgaagag 2280
   aagcaggccg aatctatgcc cgaaatcgag acagaacagg agtacaagga gattttcatc 2340
   actcctcacc agatcaagca tatcaaggat ttcaaggact acaagtactc tcaccgggtg 2400
   gataaaaagc ccaacagaga gctgatcaat gacaccctgt atagtacaag aaaagacgat 2460
   aaggggaata ccctgattgt gaacaatctg aacggactgt acgacaaaga taatgacaag 2520
   ctgaaaaagc tgatcaacaa aagtcccgag aagctgctga tgtaccacca tgatcctcag 2580
   acatatcaga aactgaagct gattatggag cagtacggcg acgagaagaa cccactgtat 2640
   aagtactatg aagagactgg gaactacctg accaagtata gcaaaaagga taatggcccc 2700
   gtgatcaaga agatcaagta ctatgggaac aagctgaatg cccatctgga catcacagac 2760
   gattacccta acagtcgcaa caaggtggtc aagctgtcac tgaagccata cagattcgat 2820
   gtctatctgg acaacggcgt gtataaattt gtgactgtca agaatctgga tgtcatcaaa 2880
   aaggagaact actatgaagt gaatagcaag tgctacgaag aggctaaaaa gctgaaaaag 2940
   attagcaacc aggcagagtt catcgcctcc ttttacaaca acgacctgat taagatcaat 3000
   ggcgaactgt atagggtcat cggggtgaac aatgatctgc tgaaccgcat tgaagtgaat      3060
   atgattgaca tcacttaccg agagtatctg gaaaacatga atgataagcg cccccctcga      3120
   attatcaaaa caattgcctc taagactcag agtatcaaaa agtactcaac cgacattctg      3180
   ggaaacctgt atgaggtgaa gagcaaaaag caccctcaga ttatcaaaaa gggctaagaa      3240
   ttc                                                                    3243
<210> 86
   <211> 1115
   <212> PRT
   <213> Artificial Sequence
<220>
   <221> source
   <223> /note="Description of Artificial Sequence: Synthetic polypeptide"
<400> 86
   Met Ser Asp Leu Val Leu Gly Leu Asp Ile Gly Ile Gly Ser Val Gly
   1               5                   10                  15
   Val Gly Ile Leu Asn Lys Val Thr Gly Glu Ile Ile His Lys Asn Ser
               20                  25                  30
   Arg Ile Phe Pro Ala Ala Gln Ala Glu Asn Asn Leu Val Arg Arg Thr
           35                  40                  45
   Asn Arg Gln Gly Arg Arg Leu Ala Arg Arg Lys Lys His Arg Arg Val
       50                  55                  60
   Arg Leu Asn Arg Leu Phe Glu Glu Ser Gly Leu Ile Thr Asp Phe Thr
   65                  70                  75                  80
   Lys Ile Ser Ile Asn Leu Asn Pro Tyr Gln Leu Arg Val Lys Gly Leu
                   85                  90                  95
   Thr Asp Glu Leu Ser Asn Glu Glu Leu Phe Ile Ala Leu Lys Asn Met
               100                 105                 110
   Val Lys His Arg Gly Ile Ser Tyr Leu Asp Asp Ala Ser Asp Asp Gly
           115                 120                 125
   Asn Ser Ser Val Gly Asp Tyr Ala Gln Ile Val Lys Glu Asn Ser Lys
       130                 135                 140
   Gln Leu Glu Thr Lys Thr Pro Gly Gln Ile Gln Leu Glu Arg Tyr Gln
   145                 150                 155                 160
   Thr Tyr Gly Gln Leu Arg Gly Asp Phe Thr Val Glu Lys Asp Gly Lys
   165 170 175
   Lys His Arg Leu Ile Asn Val Phe Pro Thr Ser Ala Tyr Arg Ser Glu
   180 185 190
   Ala Leu Arg Ile Leu Gln Thr Gln Gln Glu Phe Asn Pro Gln Ile Thr
   195 200 205
   Asp Glu Phe Ile Asn Arg Tyr Leu Glu Ile Leu Thr Gly Lys Arg Lys
   210 215 220
   Tyr Tyr His Gly Pro Gly Asn Glu Lys Ser Arg Thr Asp Tyr Gly Arg
   225 230 235 240
   Tyr Arg Thr Ser Gly Glu Thr Leu Asp Asn Ile Phe Gly Ile Leu Ile
   245 250 255
   Gly Lys Cys Thr Phe Tyr Pro Asp Glu Phe Arg Ala Ala Lys Ala Ser
   260 265 270
   Tyr Thr Ala Gln Glu Phe Asn Leu Leu Asn Asp Leu Asn Asn Leu Thr
   275 280 285
   Val Pro Thr Glu Thr Lys Lys Leu Ser Lys Glu Gln Lys Asn Gln Ile
   290 295 300
   Ile Asn Tyr Val Lys Asn Glu Lys Ala Met Gly Pro Ala Lys Leu Phe
   305 310 315 320
   Lys Tyr Ile Ala Lys Leu Leu Ser Cys Asp Val Ala Asp Ile Lys Gly
   325 330 335
   Tyr Arg Ile Asp Lys Ser Gly Lys Ala Glu Ile His Thr Phe Glu Ala
   340 345 350
   Tyr Arg Lys Met Lys Thr Leu Glu Thr Leu Asp Ile Glu Gln Met Asp
   355 360 365
   Arg Glu Thr Leu Asp Lys Leu Ala Tyr Val Leu Thr Leu Asn Thr Glu
   370 375 380
   Arg Glu Gly Ile Gln Glu Ala Leu Glu His Glu Phe Ala Asp Gly Ser
   385 390 395 400
   Phe Ser Gln Lys Gln Val Asp Glu Leu Val Gln Phe Arg Lys Ala Asn
   405 410 415
   Ser Ser Ile Phe Gly Lys Gly Trp His Asn Phe Ser Val Lys Leu Met
               420                 425                 430
   Met Glu Leu Ile Pro Glu Leu Tyr Glu Thr Ser Glu Glu Gln Met Thr
           435                 440                 445
   Ile Leu Thr Arg Leu Gly Lys Gln Lys Thr Thr Ser Ser Ser Asn Lys
       450                 455                 460
   Thr Lys Tyr Ile Asp Glu Lys Leu Leu Thr Glu Glu Ile Tyr Asn Pro
   465                 470                 475                 480
   Val Val Ala Lys Ser Val Arg Gln Ala Ile Lys Ile Val Asn Ala Ala
                   485                 490                 495
   Ile Lys Glu Tyr Gly Asp Phe Asp Asn Ile Val Ile Glu Met Ala Arg
               500                 505                 510
   Glu Asn Gln Thr Thr Gln Lys Gly Gln Lys Asn Ser Arg Glu Arg Met
           515                 520                 525
   Lys Arg Ile Glu Glu Gly Ile Lys Glu Leu Gly Ser Gln Ile Leu Lys
       530                 535                 540
   Glu His Pro Val Glu Asn Thr Gln Leu Gln Asn Glu Lys Leu Tyr Leu
   545                 550                 555                 560
   Tyr Tyr Leu Gln Asn Gly Arg Asp Met Tyr Val Asp Gln Glu Leu Asp
                   565                 570                 575
   Ile Asn Arg Leu Ser Asp Tyr Asp Val Asp His Ile Val Pro Gln Ser
               580                 585                 590
   Phe Leu Lys Asp Asp Ser Ile Asp Asn Lys Val Leu Thr Arg Ser Asp
           595                 600                 605
   Lys Asn Arg Gly Lys Ser Asp Asn Val Pro Ser Glu Glu Val Val Lys
       610                 615                 620
   Lys Met Lys Asn Tyr Trp Arg Gln Leu Leu Asn Ala Lys Leu Ile Thr
   625                 630                 635                 640
   Gln Arg Lys Phe Asp Asn Leu Thr Lys Ala Glu Arg Gly Gly Leu Ser
                   645                 650                 655
   Glu Leu Asp Lys Ala Gly Phe Ile Lys Arg Gln Leu Val Glu Thr Arg
               660                 665                 670
   Gln Ile Thr Lys His Val Ala Gln Ile Leu Asp Ser Arg Met Asn Thr
           675                 680                 685
   Lys Tyr Asp Glu Asn Asp Lys Leu Ile Arg Glu Val Lys Val Ile Thr
       690                 695                 700
   Leu Lys Ser Lys Leu Val Ser Asp Phe Arg Lys Asp Phe Gln Phe Tyr
   705                 710                 715                 720
   Lys Val Arg Glu Ile Asn Asn Tyr His His Ala His Asp Ala Tyr Leu
                   725                 730                 735
   Asn Ala Val Val Gly Thr Ala Leu Ile Lys Lys Tyr Pro Lys Leu Glu
               740                 745                 750
   Ser Glu Phe Val Tyr Gly Asp Tyr Lys Val Tyr Asp Val Arg Lys Met
           755                 760                 765
   Ile Ala Lys Ser Glu Gln Glu Ile Gly Lys Ala Thr Ala Lys Tyr Phe
       770                 775                 780
   Phe Tyr Ser Asn Ile Met Asn Phe Phe Lys Thr Glu Ile Thr Leu Ala
   785                 790                 795                 800
   Asn Gly Glu Ile Arg Lys Arg Pro Leu Ile Glu Thr Asn Gly Glu Thr
                   805                 810                 815
   Gly Glu Ile Val Trp Asp Lys Gly Arg Asp Phe Ala Thr Val Arg Lys
               820                 825                 830
   Val Leu Ser Met Pro Gln Val Asn Ile Val Lys Lys Thr Glu Val Gln
           835                 840                 845
   Thr Gly Gly Phe Ser Lys Glu Ser Ile Leu Pro Lys Arg Asn Ser Asp
       850                 855                 860
   Lys Leu Ile Ala Arg Lys Lys Asp Trp Asp Pro Lys Lys Tyr Gly Gly
   865                 870                 875                 880
   Phe Asp Ser Pro Thr Val Ala Tyr Ser Val Leu Val Val Ala Lys Val
                   885                 890                 895
   Glu Lys Gly Lys Ser Lys Lys Leu Lys Ser Val Lys Glu Leu Leu Gly
               900                 905                 910
   Ile Thr Ile Met Glu Arg Ser Ser Phe Glu Lys Asn Pro Ile Asp Phe
           915                 920                 925
   Leu Glu Ala Lys Gly Tyr Lys Glu Val Lys Lys Asp Leu Ile Ile Lys
       930                 935                 940
   Leu Pro Lys Tyr Ser Leu Phe Glu Leu Glu Asn Gly Arg Lys Arg Met
   945                 950                 955                 960
   Leu Ala Ser Ala Gly Glu Leu Gln Lys Gly Asn Glu Leu Ala Leu Pro
                   965                 970                 975
   Ser Lys Tyr Val Asn Phe Leu Tyr Leu Ala Ser His Tyr Glu Lys Leu
               980                 985                 990
   Lys Gly Ser Pro Glu Asp Asn Glu  Gln Lys Gln Leu Phe  Val Glu Gln
           995                 1000                 1005
   His Lys  His Tyr Leu Asp Glu  Ile Ile Glu Gln Ile  Ser Glu Phe
       1010                 1015                 1020
   Ser Lys  Arg Val Ile Leu Ala  Asp Ala Asn Leu Asp  Lys Val Leu
       1025                 1030                 1035
   Ser Ala  Tyr Asn Lys His Arg  Asp Lys Pro Ile Arg  Glu Gln Ala
       1040                 1045                 1050
   Glu Asn  Ile Ile His Leu Phe  Thr Leu Thr Asn Leu  Gly Ala Pro
       1055                 1060                 1065
   Ala Ala  Phe Lys Tyr Phe Asp  Thr Thr Ile Asp Arg  Lys Arg Tyr
       1070                 1075                 1080
   Thr Ser  Thr Lys Glu Val Leu  Asp Ala Thr Leu Ile  His Gln Ser
       1085                 1090                 1095
   Ile Thr  Gly Leu Tyr Glu Thr  Arg Ile Asp Leu Ser  Gln Leu Gly
       1100                 1105                 1110
   Gly Asp
       1115
<210> 87
   <211> 1374
   <212> PRT
   <213> Artificial Sequence
<220>
   <221> source
   <223> /note="Description of Artificial Sequence: Synthetic polypeptide"
<400> 87
   Met Asp Lys Lys Tyr Ser Ile Gly Leu Asp Ile Gly Thr Asn Ser Val
   1               5                   10                  15
   Gly Trp Ala Val Ile Thr Asp Glu Tyr Lys Val Pro Ser Lys Lys Phe
               20                  25                  30
   Lys Val Leu Gly Asn Thr Asp Arg His Ser Ile Lys Lys Asn Leu Ile
           35                  40                  45
   Gly Ala Leu Leu Phe Asp Ser Gly Glu Thr Ala Glu Ala Thr Arg Leu
       50                  55                  60
   Lys Arg Thr Ala Arg Arg Arg Tyr Thr Arg Arg Lys Asn Arg Ile Cys
   65                  70                  75                  80
   Tyr Leu Gln Glu Ile Phe Ser Asn Glu Met Ala Lys Val Asp Asp Ser
                   85                  90                  95
   Phe Phe His Arg Leu Glu Glu Ser Phe Leu Val Glu Glu Asp Lys Lys
               100                 105                 110
   His Glu Arg His Pro Ile Phe Gly Asn Ile Val Asp Glu Val Ala Tyr
           115                 120                 125
   His Glu Lys Tyr Pro Thr Ile Tyr His Leu Arg Lys Lys Leu Val Asp
       130                 135                 140
   Ser Thr Asp Lys Ala Asp Leu Arg Leu Ile Tyr Leu Ala Leu Ala His
   145                 150                 155                 160
   Met Ile Lys Phe Arg Gly His Phe Leu Ile Glu Gly Asp Leu Asn Pro
                   165                 170                 175
   Asp Asn Ser Asp Val Asp Lys Leu Phe Ile Gln Leu Val Gln Thr Tyr
               180                 185                 190
   Asn Gln Leu Phe Glu Glu Asn Pro Ile Asn Ala Ser Gly Val Asp Ala
           195                 200                 205
   Lys Ala Ile Leu Ser Ala Arg Leu Ser Lys Ser Arg Arg Leu Glu Asn
       210                 215                 220
   Leu Ile Ala Gln Leu Pro Gly Glu Lys Lys Asn Gly Leu Phe Gly Asn
   225                 230                 235                 240
   Leu Ile Ala Leu Ser Leu Gly Leu Thr Pro Asn Phe Lys Ser Asn Phe
                   245                 250                 255
   Asp Leu Ala Glu Asp Ala Lys Leu Gln Leu Ser Lys Asp Thr Tyr Asp
               260                 265                 270
   Asp Asp Leu Asp Asn Leu Leu Ala Gln Ile Gly Asp Gln Tyr Ala Asp
           275                 280                 285
   Leu Phe Leu Ala Ala Lys Asn Leu Ser Asp Ala Ile Leu Leu Ser Asp
       290                 295                 300
   Ile Leu Arg Val Asn Thr Glu Ile Thr Lys Ala Pro Leu Ser Ala Ser
   305                 310                 315                 320
   Met Ile Lys Arg Tyr Asp Glu His His Gln Asp Leu Thr Leu Leu Lys
                   325                 330                 335
   Ala Leu Val Arg Gln Gln Leu Pro Glu Lys Tyr Lys Glu Ile Phe Phe
               340                 345                 350
   Asp Gln Ser Lys Asn Gly Tyr Ala Gly Tyr Ile Asp Gly Gly Ala Ser
           355                 360                 365
   Gln Glu Glu Phe Tyr Lys Phe Ile Lys Pro Ile Leu Glu Lys Met Asp
       370                 375                 380
   Gly Thr Glu Glu Leu Leu Val Lys Leu Asn Arg Glu Asp Leu Leu Arg
   385                 390                 395                 400
   Lys Gln Arg Thr Phe Asp Asn Gly Ser Ile Pro His Gln Ile His Leu
                   405                 410                 415
   Gly Glu Leu His Ala Ile Leu Arg Arg Gln Glu Asp Phe Tyr Pro Phe
               420                 425                 430
   Leu Lys Asp Asn Arg Glu Lys Ile Glu Lys Ile Leu Thr Phe Arg Ile
           435                 440                 445
   Pro Tyr Tyr Val Gly Pro Leu Ala Arg Gly Asn Ser Arg Phe Ala Trp
       450                 455                 460
   Met Thr Arg Lys Ser Glu Glu Thr Ile Thr Pro Trp Asn Phe Glu Glu
   465                 470                 475                 480
   Val Val Asp Lys Gly Ala Ser Ala Gln Ser Phe Ile Glu Arg Met Thr
                   485                 490                 495
   Asn Phe Asp Lys Asn Leu Pro Asn Glu Lys Val Leu Pro Lys His Ser
               500                 505                 510
   Leu Leu Tyr Glu Tyr Phe Thr Val Tyr Asn Glu Leu Thr Lys Val Lys
           515                 520                 525
   Tyr Val Thr Glu Gly Met Arg Lys Pro Ala Phe Leu Ser Gly Glu Gln
       530                 535                 540
   Lys Lys Ala Ile Val Asp Leu Leu Phe Lys Thr Asn Arg Lys Val Thr
   545                 550                 555                 560
   Val Lys Gln Leu Lys Glu Asp Tyr Phe Lys Lys Ile Glu Cys Phe Asp
                   565                 570                 575
   Ser Val Glu Ile Ser Gly Val Glu Asp Arg Phe Asn Ala Ser Leu Gly
               580                 585                 590
   Thr Tyr His Asp Leu Leu Lys Ile Ile Lys Asp Lys Asp Phe Leu Asp
           595                 600                 605
   Asn Glu Glu Asn Glu Asp Ile Leu Glu Asp Ile Val Leu Thr Leu Thr
       610                 615                 620
   Leu Phe Glu Asp Arg Glu Met Ile Glu Glu Arg Leu Lys Thr Tyr Ala
   625                 630                 635                 640
   His Leu Phe Asp Asp Lys Val Met Lys Gln Leu Lys Arg Arg Arg Tyr
                   645                 650                 655
   Thr Gly Trp Gly Arg Leu Ser Arg Lys Leu Ile Asn Gly Ile Arg Asp
               660                 665                 670
   Lys Gln Ser Gly Lys Thr Ile Leu Asp Phe Leu Lys Ser Asp Gly Phe
           675                 680                 685
   Ala Asn Arg Asn Phe Met Gln Leu Ile His Asp Asp Ser Leu Thr Phe
       690                 695                 700
   Lys Glu Asp Ile Gln Lys Ala Gln Val Ser Gly Gln Gly Asp Ser Leu
   705                 710                 715                 720
   His Glu His Ile Ala Asn Leu Ala Gly Ser Pro Ala Ile Lys Lys Gly
                   725                 730                 735
   Ile Leu Gln Thr Val Lys Val Val Asp Glu Leu Val Lys Val Met Gly
               740                 745                 750
   Arg His Lys Pro Glu Asn Ile Val Ile Glu Met Ala Arg Glu Thr Asn
   755 760 765
   Glu Asp Asp Glu Lys Lys Ala Ile Gln Lys Ile Gln Lys Ala Asn Lys
   770 775 780
   Asp Glu Lys Asp Ala Ala Met Leu Lys Ala Ala Asn Gln Tyr Asn Gly
   785 790 795 800
   Lys Ala Glu Leu Pro His Ser Val Phe His Gly His Lys Gln Leu Ala
   805 810 815
   Thr Lys Ile Arg Leu Trp His Gln Gln Gly Glu Arg Cys Leu Tyr Thr
   820 825 830
   Gly Lys Thr Ile Ser Ile His Asp Leu Ile Asn Asn Ser Asn Gln Phe
   835 840 845
   Glu Val Asp His Ile Leu Pro Leu Ser Ile Thr Phe Asp Asp Ser Leu
   850 855 860
   Ala Asn Lys Val Leu Val Tyr Ala Thr Ala Asn Gln Glu Lys Gly Gln
   865 870 875 880
   Arg Thr Pro Tyr Gln Ala Leu Asp Ser Met Asp Asp Ala Trp Ser Phe
   885 890 895
   Arg Glu Leu Lys Ala Phe Val Arg Glu Ser Lys Thr Leu Ser Asn Lys
   900 905 910
   Lys Lys Glu Tyr Leu Leu Thr Glu Glu Asp Ile Ser Lys Phe Asp Val
   915 920 925
   Arg Lys Lys Phe Ile Glu Arg Asn Leu Val Asp Thr Arg Tyr Ala Ser
   930 935 940
   Arg Val Val Leu Asn Ala Leu Gln Glu His Phe Arg Ala His Lys Ile
   945 950 955 960
   Asp Thr Lys Val Ser Val Val Arg Gly Gln Phe Thr Ser Gln Leu Arg
   965 970 975
   Arg His Trp Gly Ile Glu Lys Thr Arg Asp Thr Tyr His His His Ala
   980 985 990
   Val Asp Ala Leu Ile Ile Ala Ala Ser Ser Gln Leu Asn Leu Trp Lys
   995 1000 1005
   Lys Gln Lys Asn Thr Leu Val Ser Tyr Ser Glu Asp Gln Leu Leu
       1010                 1015                 1020
   Asp Ile Glu Thr Gly Glu Leu Ile Ser Asp Asp Glu Tyr Lys Glu
       1025                 1030                 1035
   Ser Val Phe Lys Ala Pro Tyr Gln His Phe Val Asp Thr Leu Lys
       1040                 1045                 1050
   Ser Lys Glu Phe Glu Asp Ser Ile Leu Phe Ser Tyr Gln Val Asp
       1055                 1060                 1065
   Ser Lys Phe Asn Arg Lys Ile Ser Asp Ala Thr Ile Tyr Ala Thr
       1070                 1075                 1080
   Arg Gln Ala Lys Val Gly Lys Asp Lys Ala Asp Glu Thr Tyr Val
       1085                 1090                 1095
   Leu Gly Lys Ile Lys Asp Ile Tyr Thr Gln Asp Gly Tyr Asp Ala
       1100                 1105                 1110
   Phe Met Lys Ile Tyr Lys Lys Asp Lys Ser Lys Phe Leu Met Tyr
       1115                 1120                 1125
   Arg His Asp Pro Gln Thr Phe Glu Lys Val Ile Glu Pro Ile Leu
       1130                 1135                 1140
   Glu Asn Tyr Pro Asn Lys Gln Ile Asn Glu Lys Gly Lys Glu Val
       1145                 1150                 1155
   Pro Cys Asn Pro Phe Leu Lys Tyr Lys Glu Glu His Gly Tyr Ile
       1160                 1165                 1170
   Arg Lys Tyr Ser Lys Lys Gly Asn Gly Pro Glu Ile Lys Ser Leu
       1175                 1180                 1185
   Lys Tyr Tyr Asp Ser Lys Leu Gly Asn His Ile Asp Ile Thr Pro
       1190                 1195                 1200
   Lys Asp Ser Asn Asn Lys Val Val Leu Gln Ser Val Ser Pro Trp
       1205                 1210                 1215
   Arg Ala Asp Val Tyr Phe Asn Lys Thr Thr Gly Lys Tyr Glu Ile
       1220                 1225                 1230
   Leu Gly Leu Lys Tyr Ala Asp Leu Gln Phe Glu Lys Gly Thr Gly
   1235 1240 1245
   Thr Tyr Lys Ile Ser Gln Glu Lys Tyr Asn Asp Ile Lys Lys Lys
   1250 1255 1260
   Glu Gly Val Asp Ser Asp Ser Glu Phe Lys Phe Thr Leu Tyr Lys
   1265 1270 1275
   Asn Asp Leu Leu Leu Val Lys Asp Thr Glu Thr Lys Glu Gln Gln
   1280 1285 1290
   Leu Phe Arg Phe Leu Ser Arg Thr Met Pro Lys Gln Lys His Tyr
   1295 1300 1305
   Val Glu Leu Lys Pro Tyr Asp Lys Gln Lys Phe Glu Gly Gly Glu
   1310 1315 1320
   Ala Leu Ile Lys Val Leu Gly Asn Val Ala Asn Ser Gly Gln Cys
   1325 1330 1335
   Lys Lys Gly Leu Gly Lys Ser Asn Ile Ser Ile Tyr Lys Val Arg
   1340 1345 1350
   Thr Asp Val Leu Gly Asn Gln His Ile Ile Lys Asn Glu Gly Asp
   1355 1360 1365
   Lys Pro Lys Leu Asp Phe
   1370
<210> 88
   <211> 4677
   <212> DNA
   <213> Artificial Sequence
<220>
   <221> source
   <223> /note="Description of Artificial Sequence: Synthetic polynucleotide"
<400> 88
   tctttcttgc gctatgacac ttccagcaaa aggtagggcg ggctgcgaga cggcttcccg 60
   gcgctgcatg caacaccgat gatgcttcga ccccccgaag ctccttcggg gctgcatggg 120
   cgctccgatg ccgctccagg gcgagcgctg tttaaatagc caggcccccg attgcaaaga 180
   cattatagcg agctaccaaa gccatattca aacacctaga tcactaccac ttctacacag 240
   gccactcgag cttgtgatcg cactccgcta agggggcgcc tcttcctctt cgtttcagtc 300
   acaacccgca aacatgtacc catacgatgt tccagattac gcttcgccga agaaaaagcg 360
   caaggtcgaa gcgtccgaca agaagtacag catcggcctg gacatcggca ccaactctgt 420
   gggctgggcc gtgatcaccg acgagtacaa ggtgcccagc aagaaattca aggtgctggg 480
   caacaccgac cggcacagca tcaagaagaa cctgatcgga gccctgctgt tcgacagcgg 540
   cgaaacagcc gaggccaccc ggctgaagag aaccgccaga agaagataca ccagacggaa 600
   gaaccggatc tgctatctgc aagagatctt cagcaacgag atggccaagg tggacgacag 660
   cttcttccac agactggaag agtccttcct ggtggaagag gataagaagc acgagcggca 720
   ccccatcttc ggcaacatcg tggacgaggt ggcctaccac gagaagtacc ccaccatcta 780
   ccacctgaga aagaaactgg tggacagcac cgacaaggcc gacctgcggc tgatctatct 840
   ggccctggcc cacatgatca agttccgggg ccacttcctg atcgagggcg acctgaaccc 900
   cgacaacagc gacgtggaca agctgttcat ccagctggtg cagacctaca accagctgtt 960
   cgaggaaaac cccatcaacg ccagcggcgt ggacgccaag gccatcctgt ctgccagact 1020
   gagcaagagc agacggctgg aaaatctgat cgcccagctg cccggcgaga agaagaatgg 1080
   cctgttcggc aacctgattg ccctgagcct gggcctgacc cccaacttca agagcaactt 1140
   cgacctggcc gaggatgcca aactgcagct gagcaaggac acctacgacg acgacctgga 1200
   caacctgctg gcccagatcg gcgaccagta cgccgacctg tttctggccg ccaagaacct 1260
   gtccgacgcc atcctgctga gcgacatcct gagagtgaac accgagatca ccaaggcccc 1320
   cctgagcgcc tctatgatca agagatacga cgagcaccac caggacctga ccctgctgaa 1380
   agctctcgtg cggcagcagc tgcctgagaa gtacaaagag attttcttcg accagagcaa 1440
   gaacggctac gccggctaca ttgacggcgg agccagccag gaagagttct acaagttcat 1500
   caagcccatc ctggaaaaga tggacggcac cgaggaactg ctcgtgaagc tgaacagaga 1560
   ggacctgctg cggaagcagc ggaccttcga caacggcagc atcccccacc agatccacct 1620
   gggagagctg cacgccattc tgcggcggca ggaagatttt tacccattcc tgaaggacaa 1680
   ccgggaaaag atcgagaaga tcctgacctt ccgcatcccc tactacgtgg gccctctggc 1740
   caggggaaac agcagattcg cctggatgac cagaaagagc gaggaaacca tcaccccctg 1800
   gaacttcgag gaagtggtgg acaagggcgc ttccgcccag agcttcatcg agcggatgac 1860
   caacttcgat aagaacctgc ccaacgagaa ggtgctgccc aagcacagcc tgctgtacga 1920
   gtacttcacc gtgtataacg agctgaccaa agtgaaatac gtgaccgagg gaatgagaaa 1980
   gcccgccttc ctgagcggcg agcagaaaaa ggccatcgtg gacctgctgt tcaagaccaa 2040
   ccggaaagtg accgtgaagc agctgaaaga ggactacttc aagaaaatcg agtgcttcga 2100
   ctccgtggaa atctccggcg tggaagatcg gttcaacgcc tccctgggca cataccacga 2160
   tctgctgaaa attatcaagg acaaggactt cctggacaat gaggaaaacg aggacattct 2220
   ggaagatatc gtgctgaccc tgacactgtt tgaggacaga gagatgatcg aggaacggct 2280
   gaaaacctat gcccacctgt tcgacgacaa agtgatgaag cagctgaagc ggcggagata 2340
   caccggctgg ggcaggctga gccggaagct gatcaacggc atccgggaca agcagtccgg 2400
   caagacaatc ctggatttcc tgaagtccga cggcttcgcc aacagaaact tcatgcagct 2460
   gatccacgac gacagcctga cctttaaaga ggacatccag aaagcccagg tgtccggcca 2520
   gggcgatagc ctgcacgagc acattgccaa tctggccggc agccccgcca ttaagaaggg 2580
   catcctgcag acagtgaagg tggtggacga gctcgtgaaa gtgatgggcc ggcacaagcc 2640
   cgagaacatc gtgatcgaaa tggccagaga gaaccagacc acccagaagg gacagaagaa 2700
   cagccgcgag agaatgaagc ggatcgaaga gggcatcaaa gagctgggca gccagatcct 2760
   gaaagaacac cccgtggaaa acacccagct gcagaacgag aagctgtacc tgtactacct 2820
   gcagaatggg cgggatatgt acgtggacca ggaactggac atcaaccggc tgtccgacta 2880
   cgatgtggac catatcgtgc ctcagagctt tctgaaggac gactccatcg acaacaaggt 2940
   gctgaccaga agcgacaaga accggggcaa gagcgacaac gtgccctccg aagaggtcgt 3000
   gaagaagatg aagaactact ggcggcagct gctgaacgcc aagctgatta cccagagaaa 3060
   gttcgacaat ctgaccaagg ccgagagagg cggcctgagc gaactggata aggccggctt 3120
   catcaagaga cagctggtgg aaacccggca gatcacaaag cacgtggcac agatcctgga 3180
   ctcccggatg aacactaagt acgacgagaa tgacaagctg atccgggaag tgaaagtgat 3240
   caccctgaag tccaagctgg tgtccgattt ccggaaggat ttccagtttt acaaagtgcg 3300
   cgagatcaac aactaccacc acgcccacga cgcctacctg aacgccgtcg tgggaaccgc 3360
   cctgatcaaa aagtacccta agctggaaag cgagttcgtg tacggcgact acaaggtgta 3420
   cgacgtgcgg aagatgatcg ccaagagcga gcaggaaatc ggcaaggcta ccgccaagta 3480
   cttcttctac agcaacatca tgaacttttt caagaccgag attaccctgg ccaacggcga 3540
   gatccggaag cggcctctga tcgagacaaa cggcgaaacc ggggagatcg tgtgggataa 3600
   gggccgggat tttgccaccg tgcggaaagt gctgagcatg ccccaagtga atatcgtgaa 3660
   aaagaccgag gtgcagacag gcggcttcag caaagagtct atcctgccca agaggaacag 3720
   cgataagctg atcgccagaa agaaggactg ggaccctaag aagtacggcg gcttcgacag 3780
   ccccaccgtg gcctattctg tgctggtggt ggccaaagtg gaaaagggca agtccaagaa 3840
   actgaagagt gtgaaagagc tgctggggat caccatcatg gaaagaagca gcttcgagaa 3900
   gaatcccatc gactttctgg aagccaaggg ctacaaagaa gtgaaaaagg acctgatcat 3960
   caagctgcct aagtactccc tgttcgagct ggaaaacggc cggaagagaa tgctggcctc 4020
   tgccggcgaa ctgcagaagg gaaacgaact ggccctgccc tccaaatatg tgaacttcct 4080
   gtacctggcc agccactatg agaagctgaa gggctccccc gaggataatg agcagaaaca 4140
   gctgtttgtg gaacagcaca agcactacct ggacgagatc atcgagcaga tcagcgagtt 4200
   ctccaagaga gtgatcctgg ccgacgctaa tctggacaaa gtgctgtccg cctacaacaa 4260
   gcaccgggat aagcccatca gagagcaggc cgagaatatc atccacctgt ttaccctgac 4320
   caatctggga gcccctgccg ccttcaagta ctttgacacc accatcgacc ggaagaggta 4380
   caccagcacc aaagaggtgc tggacgccac cctgatccac cagagcatca ccggcctgta 4440
   cgagacacgg atcgacctgt ctcagctggg aggcgacagc cccaagaaga agagaaaggt 4500
   ggaggccagc taaggatccg gcaagactgg ccccgcttgg caacgcaaca gtgagcccct 4560
   ccctagtgtg tttggggatg tgactatgta ttcgtgtgtt ggccaacggg tcaacccgaa 4620
   cagattgata cccgccttgg catttcctgt cagaatgtaa cgtcagttga tggtact 4677
<210> 89
   <211> 3150
   <212> DNA
   <213> Artificial Sequence
<220>
   <221> source
   <223> /note="Description of Artificial Sequence: Synthetic polynucleotide"
<400> 89
   tctttcttgc gctatgacac ttccagcaaa aggtagggcg ggctgcgaga cggcttcccg      60
   gcgctgcatg caacaccgat gatgcttcga ccccccgaag ctccttcggg gctgcatggg     120
   cgctccgatg ccgctccagg gcgagcgctg tttaaatagc caggcccccg attgcaaaga     180
   cattatagcg agctaccaaa gccatattca aacacctaga tcactaccac ttctacacag     240
   gccactcgag cttgtgatcg cactccgcta agggggcgcc tcttcctctt cgtttcagtc      300
   acaacccgca aacatgccta agaagaagag gaaggttaac acgattaaca tcgctaagaa     360
   cgacttctct gacatcgaac tggctgctat cccgttcaac actctggctg accattacgg     420
   tgagcgttta gctcgcgaac agttggccct tgagcatgag tcttacgaga tgggtgaagc     480
   acgcttccgc aagatgtttg agcgtcaact taaagctggt gaggttgcgg ataacgctgc     540
   cgccaagcct ctcatcacta ccctactccc taagatgatt gcacgcatca acgactggtt     600
   tgaggaagtg aaagctaagc gcggcaagcg cccgacagcc ttccagttcc tgcaagaaat     660
   caagccggaa gccgtagcgt acatcaccat taagaccact ctggcttgcc taaccagtgc     720
   tgacaataca accgttcagg ctgtagcaag cgcaatcggt cgggccattg aggacgaggc     780
   tcgcttcggt cgtatccgtg accttgaagc taagcacttc aagaaaaacg ttgaggaaca     840
   actcaacaag cgcgtagggc acgtctacaa gaaagcattt atgcaagttg tcgaggctga     900
   catgctctct aagggtctac tcggtggcga ggcgtggtct tcgtggcata aggaagactc     960
   tattcatgta ggagtacgct gcatcgagat gctcattgag tcaaccggaa tggttagctt    1020
   acaccgccaa aatgctggcg tagtaggtca agactctgag actatcgaac tcgcacctga 1080
   atacgctgag gctatcgcaa cccgtgcagg tgcgctggct ggcatctctc cgatgttcca 1140
   accttgcgta gttcctccta agccgtggac tggcattact ggtggtggct attgggctaa 1200
   cggtcgtcgt cctctggcgc tggtgcgtac tcacagtaag aaagcactga tgcgctacga 1260
   agacgtttac atgcctgagg tgtacaaagc gattaacatt gcgcaaaaca ccgcatggaa 1320
   aatcaacaag aaagtcctag cggtcgccaa cgtaatcacc aagtggaagc attgtccggt 1380
   cgaggacatc cctgcgattg agcgtgaaga actcccgatg aaaccggaag acatcgacat 1440
   gaatcctgag gctctcaccg cgtggaaacg tgctgccgct gctgtgtacc gcaaggacaa 1500
   ggctcgcaag tctcgccgta tcagccttga gttcatgctt gagcaagcca ataagtttgc 1560
   taaccataag gccatctggt tcccttacaa catggactgg cgcggtcgtg tttacgctgt 1620
   gtcaatgttc aacccgcaag gtaacgatat gaccaaagga ctgcttacgc tggcgaaagg 1680
   taaaccaatc ggtaaggaag gttactactg gctgaaaatc cacggtgcaa actgtgcggg 1740
   tgtcgacaag gttccgttcc ctgagcgcat caagttcatt gaggaaaacc acgagaacat 1800
   catggcttgc gctaagtctc cactggagaa cacttggtgg gctgagcaag attctccgtt 1860
   ctgcttcctt gcgttctgct ttgagtacgc tggggtacag caccacggcc tgagctataa 1920
   ctgctccctt ccgctggcgt ttgacgggtc ttgctctggc atccagcact tctccgcgat 1980
   gctccgagat gaggtaggtg gtcgcgcggt taacttgctt cctagtgaaa ccgttcagga 2040
   catctacggg attgttgcta agaaagtcaa cgagattcta caagcagacg caatcaatgg 2100
   gaccgataac gaagtagtta ccgtgaccga tgagaacact ggtgaaatct ctgagaaagt 2160
   caagctgggc actaaggcac tggctggtca atggctggct tacggtgtta ctcgcagtgt 2220
   gactaagcgt tcagtcatga cgctggctta cgggtccaaa gagttcggct tccgtcaaca 2280
   agtgctggaa gataccattc agccagctat tgattccggc aagggtctga tgttcactca 2340
   gccgaatcag gctgctggat acatggctaa gctgatttgg gaatctgtga gcgtgacggt 2400
   ggtagctgcg gttgaagcaa tgaactggct taagtctgct gctaagctgc tggctgctga 2460
   ggtcaaagat aagaagactg gagagattct tcgcaagcgt tgcgctgtgc attgggtaac 2520
   tcctgatggt ttccctgtgt ggcaggaata caagaagcct attcagacgc gcttgaacct 2580
   gatgttcctc ggtcagttcc gcttacagcc taccattaac accaacaaag atagcgagat 2640
   tgatgcacac aaacaggagt ctggtatcgc tcctaacttt gtacacagcc aagacggtag 2700
   ccaccttcgt aagactgtag tgtgggcaca cgagaagtac ggaatcgaat cttttgcact 2760
   gattcacgac tccttcggta cgattccggc tgacgctgcg aacctgttca aagcagtgcg 2820
   cgaaactatg gttgacacat atgagtcttg tgatgtactg gctgatttct acgaccagtt 2880
   cgctgaccag ttgcacgagt ctcaattgga caaaatgcca gcacttccgg ctaaaggtaa 2940
   cttgaacctc cgtgacatct tagagtcgga cttcgcgttc gcgtaaggat ccggcaagac 3000
   tggccccgct tggcaacgca acagtgagcc cctccctagt gtgtttgggg atgtgactat 3060
   gtattcgtgt gttggccaac gggtcaaccc gaacagattg atacccgcct tggcatttcc 3120
   tgtcagaatg taacgtcagt tgatggtact 3150
<210> 90
   <211> 125
   <212> DNA
   <213> Artificial Sequence
<220>
   <221> source
   <223> /note="Description of Artificial Sequence: Synthetic polynucleotide"
<220>
   <221> modified_base
   <222> (23)..(42)
   <223> a, c, t, g, unknown or other
<400> 90
   gaaattaata cgactcacta tannnnnnnn nnnnnnnnnn nngttttaga gctagaaata 60
   gcaagttaaa ataaggctag tccgttatca acttgaaaaa gtggcaccga gtcggtgctt 120
   ttttt 125
<210> 91
   <211> 8452
   <212> DNA
   <213> Artificial Sequence
<220>
   <221> source
   <223> /note="Description of Artificial Sequence: Synthetic polynucleotide"
<400> 91
<210> 92
   <211> 102
   <212> DNA
   <213> Artificial Sequence
<220>
   <221> source
   <223> /note="Description of Artificial Sequence: Synthetic polynucleotide"
<400> 92
   gttttagagc tatgctgttt tgaatggtcc caaaacggaa gggcctgagt ccgagcagaa 60
   gaagaagttt tagagctatg ctgttttgaa tggtcccaaa ac 102
<210> 93
   <211> 100
   <212> DNA
   <213> Homo sapiens
<400> 93
   cggaggacaa agtacaaacg gcagaagctg gaggaggaag ggcctgagtc cgagcagaag 60
   aagaagggct cccatcacat caaccggtgg cgcattgcca 100
<210> 94
   <211> 50
   <212> DNA
   <213> Homo sapiens
<400> 94
   agctggagga ggaagggcct gagtccgagc agaagaagaa gggctcccac 50
<210> 95
   <211> 30
   <212> RNA
   <213> Artificial Sequence
<220>
   <221> source
   <223> /note="Description of Artificial Sequence: Synthetic oligonucleotide"
<400> 95
   gaguccgagc agaagaagaa guuuuagagc 30
<210> 96
   <211> 49
   <212> DNA
   <213> Artificial Sequence
<220>
   <221> source
   <223> /note="Description of Artificial Sequence: Synthetic oligonucleotide"
<400> 96
   agctggagga ggaagggcct gagtccgagc agaagagaag ggctcccat 49
<210> 97
   <211> 53
   <212> DNA
   <213> Homo sapiens
<400> 97
   ctggaggagg aagggcctga gtccgagcag aagaagaagg gctcccatca cat 53
<210> 98
   <211> 52
   <212> DNA
   <213> Homo sapiens
<400> 98
   ctggaggagg aagggcctga gtccgagcag aagagaaggg ctcccatcac at 52
<210> 99
   <211> 54
   <212> DNA
   <213> Homo sapiens
<400> 99
   ctggaggagg aagggcctga gtccgagcag aagaaagaag ggctcccatc acat 54
<210> 100
   <211> 50
   <212> DNA
   <213> Homo sapiens
<400> 100
   ctggaggagg aagggcctga gtccgagcag aagaagggct cccatcacat 50
<210> 101
   <211> 47
   <212> DNA
   <213> Homo sapiens
<400> 101
   ctggaggagg aagggcctga gcccgagcag aagggctccc atcacat 47
<210> 102
   <211> 155
   <212> RNA
   <213> Artificial Sequence
<220>
   <221> source
   <223> /note="Description of Artificial Sequence: Synthetic polynucleotide"
<400> 102
   guuucagugc uauaggaaac uauaggaaau caccuucggg ugagcugaaa uccccuaaag      60
   cuaagauuga auccggccac uaucuauuag uagauauccg gauauucuga uauaaaaccu     120
   cauucuuuga uuagaccaaa ggaugagguu uuuuu                                155
<210> 103
   <211> 107
   <212> RNA
   <213> Artificial Sequence
<220>
   <221> source
   <223> /note="Description of Artificial Sequence: Synthetic polynucleotide"
<400> 103
   guuugagagu uggaaacaac gaguucaaau aagaauucau caaaaucguc ccuuuuggga      60
   ccgcucauug uggagcauca aggcuuaaca ugguuaagcc uuuuuuu                   107
<210> 104
   <211> 99
   <212> RNA
   <213> Artificial Sequence
<220>
   <221> source
   <223> /note="Description of Artificial Sequence: Synthetic oligonucleotide"
<400> 104
   guuugagagu aggaaacuac acgguucaaa uaaagaauuu uucuaaucgc ccaaugggcc      60
   cauauugaua uggaugaaac ucgcuuagcg aguuuuuuu                             99
<210> 105
   <211> 91
   <212> RNA
   <213> Artificial Sequence
<220>
   <221> source
   <223> /note="Description of Artificial Sequence: Synthetic oligonucleotide"
<400> 105
   guuuuagcac uagaaauagu uaaguuaaaa caagcuuaaa gcgucaaugu aauauuuuau 60
   uaacacccua cugugucagu gggguuuuuu u 91
<210> 106
   <211> 94
   <212> RNA
   <213> Artificial Sequence
<220>
   <221> source
   <223> /note="Description of Artificial Sequence: Synthetic oligonucleotide"
<400> 106
   guuuuagaug gugaaaacca gauuuaaaau caagcaaugc aucuuuugau gcaaaguuuc 60
   aauauuuguc ccacguuauc gagggacuuu uuuu 94
<210> 107
   <211> 78
   <212> RNA
   <213> Artificial Sequence
<220>
   <221> source
   <223> /note="Description of Artificial Sequence: Synthetic oligonucleotide"
<400> 107
   auuuuaguac cuggaaacag aucuacuaaa acaaggcuuu augccgaaau caagagcacc 60
   gacgggugcu cuuuuuuu 78
<210> 108
   <211> 84
   <212> RNA
   <213> Artificial Sequence
<220>
   <221> source
   <223> /note="Description of Artificial Sequence: Synthetic oligonucleotide"
<400> 108
   guuuuuguac ucgaaagagc cuacaaagau aaggcuuuau gccgaauuca agcaccccau 60
   guuuugacau gaggugcuuu uuuu 84
<210> 109
   <211> 85
   <212> RNA
   <213> Artificial Sequence
<220>
   <221> source
   <223> /note="Description of Artificial Sequence: Synthetic oligonucleotide"
<400> 109
   guuuuuguac cuuagaaaua agaucuacaa aaauaaggau uuauuccgaa uuuaccaccu 60
   auuuuaauua auaggugguu uuuuu 85
<210> 110
   <211> 100
   <212> RNA
   <213> Artificial Sequence
<220>
   <221> source
   <223> /note="Description of Artificial Sequence: Synthetic polynucleotide"
<400> 110
   guugugauuu gcuuucauuu gaaaaauuga agcaaaucac aauaaggauu auuccguugu 60
   gaaaacaauu aaagcggucu ugcaaaaggu cgcuuuuuuu 100
<210> 111
   <211> 102
   <212> RNA
   <213> Artificial Sequence
<220>
   <221> source
   <223> /note="Description of Artificial Sequence: Synthetic polynucleotide"
<400> 111
   guugugauuc gcuuucaaug aaaauugaag cgaaucacaa uaaggauuau uccguuguga 60
   aaacauuuac uacggggcau cgaaagacug ccucguuuuu uu 102
<210> 112
   <211> 90
   <212> RNA
   <213> Artificial Sequence
<220>
   <221> source
   <223> /note="Description of Artificial Sequence: Synthetic oligonucleotide"
<400> 112
   guugugguuu gauuagaaau aaucuuauca caauaaggcu auaugccgua gacgaaaguc 60
   uuuagucccg cuucgguggg acuuuuuuuu 90
<210> 113
   <211> 78
   <212> RNA
   <213> Artificial Sequence
<220>
   <221> source
   <223> /note="Description of Artificial Sequence: Synthetic oligonucleotide"
<400> 113
   guuggggaug accgcgaaag cgauuaucuc uaauaagacu uaagucgcaa aaugcucccu 60
   auuuugggag cuuuuuuu 78
<210> 114
   <211> 85
   <212> RNA
   <213> Artificial Sequence
<220>
   <221> source
   <223> /note="Description of Artificial Sequence: Synthetic oligonucleotide"
<400> 114
   guugcggcug gagaaaucca gccguuaaca uguucccuuc ggggagcacg aaaugcgggg 60
   cgggccacgg uccgccccuu uuuuu 85
<210> 115
   <211> 126
   <212> RNA
   <213> Artificial Sequence
<220>
   <221> source
   <223> /note="Description of Artificial Sequence: Synthetic polynucleotide"
<400> 115
   gccgugguuu ccgaaaggaa accacgguaa cagaauuacc guaagguuuu uucugugaag 60
   gaucaucccu cgcuugggca accaggcggg ggaaauuccu cguucgggcc aaucagcccu 120
   uuuuuu 126
<210> 116
   <211> 82
   <212> RNA
   <213> Artificial Sequence
<220>
   <221> source
   <223> /note="Description of Artificial Sequence: Synthetic oligonucleotide"
<400> 116
   guuguaauuc ccugaaaagg uuauuacaau aagguaagaa accuaaaagc ucuaauccca 60
   uucuucggaa ugggauuuuu uu 82
<210> 117
   <211> 83
   <212> RNA
   <213> Artificial Sequence
<220>
   <221> source
   <223> /note="Description of Artificial Sequence: Synthetic oligonucleotide"
<400> 117
   guuuuaguac ucuggaaaca gaaucuacua aaacaaggca aaaugccgug uuuaucucgu 60
   caacuuguug gcgagauuuu uuu 83
<210> 118
   <211> 94
   <212> RNA
   <213> Artificial Sequence
<220>
   <221> source
   <223> /note="Description of Artificial Sequence: Synthetic oligonucleotide"
<400> 118
   guuuuagcac uguacaagaa auugucgugc uaaaauaagg cgcuguuaau gcagcugccg 60
   cauccgccag agcauuuaug cucuggcuuu uuuu 94
<210> 119
   <211> 4350
   <212> DNA
   <213> Artificial Sequence
<220>
   <221> source
   <223> /note="Description of Artificial Sequence: Synthetic polynucleotide"
<400> 119
   accggtgcca ccatgtaccc atacgatgtt ccagattacg cttcgccgaa gaaaaagcgc      60
   aaggtcgaag cgtccatgac tcagagcgag cgacgatttt cttgcagcat tggcattgac     120
   atgggggcta agtacactgg ggtgttctac gcactgttcg accgggagga actgcccaca     180
   aacctgaaca gcaaggccat gaccctggtc atgcctgaga cagggccaag atacgtgcag     240
   gcacagagaa ctgccgtcag acacaggctg cgcggacaga agagatatac cctggctagg     300
   aaactggcat ttctggtggt cgacgatatg atcaagaaac aggaaaagag gctgactgat     360
   gaggaatgga aacgaggacg ggaggccctg tccggcctgc tgaagcggag agggtactct     420
   cggcccaacg ctgacggcga agatctgacc cctctggaga atgtgagagc agacgtgttc     480
   gccgctcatc ctgccttcag cacatatttt tccgaagtgc gctctctggc tgagcagtgg     540
   gaggagttca ccgcaaacat cagcaatgtc gagaagtttc tgggcgaccc aaacatcccc     600
   gccgataaag agttcattga atttgccgtg gctgaagggc tgattgacaa gaccgagaag     660
   aaagcctacc agtcagctct gagcaccctg agggcaaacg ccaatgtgct gacaggactg     720
   cggcagatgg gccacaagcc tagatcagaa tattttaaag caatcgaggc cgacctgaag     780
   aaagatagcc gcctggccaa gattaacgaa gcattcggag gagcagagcg cctggctcga     840
   ctgctgggaa acctgtccaa tctgcagctg cgggcagaaa gatggtactt caatgccccc     900
   gacatcatga aggatagggg ctgggagcct gatcgcttca agaaaacact ggtgcgggct     960
   tttaagttct ttcacccagc aaaggaccag aacaaacagc atctggaact gatcaaacag 1020
   attgagaaca gcgaagatat cattgagact ctgtgcaccc tggacccaaa cagaaccatc 1080
   cccccttacg aggatcagaa caataggcgc ccacccctgg accagactct gctgctgagt 1140
   cccgaaaagc tgacccggca gtatggcgag atctggaaaa catggagcgc cagactgacc 1200
   tccgctgaac ccacactggc acctgcagcc gagattctgg aaagatctac cgacaggaag 1260
   agtcgcgtgg cagtcaacgg acacgagcca ctgcctacac tggcttacca gctgagttat 1320
   gcactgcaga gagccttcga caggtcaaaa gccctggatc catatgctct gagggcactg 1380
   gctgcaggct caaaaagcaa taagctgaca tccgcccgca ctgctctgga gaactgcatc 1440
   ggaggccaga atgtgaaaac cttcctggac tgtgcccgac ggtactatcg ggaagcagac 1500
   gatgccaaag tcgggctgtg gttcgacaac gccgatggac tgctggagag atctgacctg 1560
   catcctccaa tgaagaaaaa gatcctgccc ctgctggtgg ccaatattct gcagacagat 1620
   gaaaccacag gccagaagtt tctggacgag atctggcgaa aacagattaa ggggcgggaa 1680
   actgtggcta gccgatgtgc acggatcgag acagtgcgga aatccttcgg gggaggcttt 1740
   aacattgcct acaataccgc tcagtatagg gaggtgaaca agctgccccg caatgcccag 1800
   gataaagaac tgctgacaat cagagatagg gtggctgaga ctgcagactt cattgccgct 1860
   aacctggggc tgtctgacga gcagaaaaga aagttcgcca atccttttag tctggctcag 1920
   ttctacaccc tgatcgagac agaagtgtcc ggattttctg caactaccct ggccgtccac 1980
   ctggagaacg cctggaggat gacaatcaag gatgctgtga ttaatgggga aactgtcaga 2040
   gcagcacagt gcagcaggct gcctgcagag acagctcgcc cattcgatgg actggtgaga 2100
   aggctggtcg acagacaggc ttgggagatc gcaaagaggg tgtcaactga cattcagagc 2160
   aaagtcgatt tctccaacgg catcgtggac gtcagcattt ttgtggagga aaataagttc 2220
   gagttttccg catctgtggc cgatctgaaa aagaacaaac gggtcaaaga caagatgctg 2280
   tccgaggccg aaaagctgga aaccagatgg ctgatcaaaa atgagcggat caagaaggcc 2340
   agccggggaa cttgtcccta caccggcgat aggctggctg aggggggaga aatcgaccac 2400
   attctgcccc gaagcctgat caaggatgcc cggggaattg tgtttaacgc tgagcctaat 2460
   ctgatctatg caagctcccg cggcaaccag ctgaaaaaga atcagcgata cagtctgtca 2520
   gatctgaagg ccaactatcg gaatgagatc ttcaaaacta gcaacatcgc tgcaattacc 2580
   gccgagattg aggacgtggt cactaagctg cagcagaccc atagactgaa attctttgat 2640
   ctgctgaatg agcacgaaca ggactgcgtg cggcacgccc tgttcctgga cgatggcagc 2700
   gaagctcgcg acgcagtgct ggagctgctg gcaacacagc gccgaactcg cgtcaacggg 2760
   acacagatct ggatgattaa gaacctggcc aacaagatcc gagaggaact gcagaattgg 2820
   tgtaagacaa ctaacaatag actgcacttt caggccgctg caactaacgt gtccgatgca 2880
   aagaatctga ggctgaaact ggcccagaac cagcccgact tcgagaagcc agatatccag 2940
   cccattgcca gccattccat cgacgccctg tgctctttcg ctgtggggag tgctgacgca 3000
   gaacgcgatc agaatggatt tgactacctg gatggcaaga ccgtgctggg actgtatcca 3060
   cagagctgtg aggtcattca cctgcaggcc aagccccagg aggaaaaaag tcatttcgat 3120
   tcagtggcta tctttaagga aggcatctac gcagagcagt tcctgcctat ctttaccctg 3180
   aacgaaaaga tctggattgg atatgagaca ctgaatgcca aaggcgaaag atgcggggct 3240
   attgaggtga gtggcaaaca gccaaaggag ctgctggaaa tgctggcccc cttctttaac 3300
   aagcctgtgg gcgacctgtc agcccacgct acttaccgga tcctgaaaaa gcctgcatat 3360
   gagtttctgg caaaggcagc tctgcagcca ctgagcgcag aggaaaaaag actggcagcc 3420
   ctgctggatg ctctgcgcta ctgtaccagt cgaaagtcac tgatgagcct gttcatggct 3480
   gcaaacggga aatccctgaa aaagcgggag gacgtgctga aacccaagct gttccagctg 3540
   aaggtcgagc tgaaaggcga aaagagcttc aagctgaacg ggagcctgac cctgcctgtg 3600
   aagcaggact ggctgagaat ctgcgatagc ccagaactgg cagacgcctt tggcaaaccc 3660
   tgttccgccg atgagctgac atctaagctg gctcgcattt ggaaacgacc tgtgatgcgg 3720
   gatctggctc atgcaccagt ccggagagag ttcagcctgc ccgcaatcga caacccaagt 3780
   ggagggttca ggattaggcg caccaacctg tttggcaatg agctgtacca ggtgcacgcc 3840
   atcaacgcta aaaagtatcg cggcttcgcc tccgctgggt ctaatgtcga ctggtccaag 3900
   gggatcctgt ttaacgagct gcagcatgaa aatctgaccg agtgcggagg caggttcatt 3960
   acaagcgccg atgtgactcc tatgtccgaa tggcgcaagg tggtcgcaga ggacaacctg 4020
   tctatctgga ttgctccagg gacagaagga cgacggtacg tgagggtcga gacaacattc 4080
   atccaggcca gtcactggtt tgaacagtca gtggagaatt gggccattac tagtcctctg 4140
   tcactgccag cttccttcaa ggtggacaaa ccagctgagt ttcagaaggc agtcggaacc 4200
   gagctgtcag aactgctggg ccagcccagg agcgaaatct tcattgagaa cgtgggcaat 4260
   gccaagcata tccgcttttg gtacattgtg gtgagcagca acaaaaagat gaacgagtct 4320
   tacaacaatg tgtctaagag ttaagaattc 4350
<210> 120
   <211> 4269
   <212> DNA
   <213> Artificial Sequence
<220>
   <221> source
   <223> /note="Description of Artificial Sequence: Synthetic polynucleotide"
<400> 120
   accggtgcca ccatgtaccc atacgatgtt ccagattacg cttcgccgaa gaaaaagcgc 60
   aaggtcgaag cgtccatgaa aaaagaaatc aaagactact tcctggggct ggatgtgggg 120
   actgggagcg tggggtgggc tgtgaccgat actgactaca aactgctgaa ggctaaccga 180
   aaagacctgt ggggcatgag atgcttcgag acagccgaaa ctgctgaggt gcggagactg 240
   cacaggggag ccaggcgccg aatcgagcgg agaaagaaac gcattaagct gctgcaggag 300
   ctgttctctc aggaaatcgc caaaaccgat gagggcttct ttcagagaat gaaggaaagc 360
   cccttttacg ctgaggacaa aacaatcctg caggaaaaca ctctgttcaa tgacaaggat 420
   tttgctgata agacttacca caaagcatat cctaccatta atcatctgat caaggcttgg 480
   attgagaaca aggtgaaacc agacccccga ctgctgtacc tggcatgtca caacatcatt 540
   aagaaaaggg gacatttcct gtttgaaggc gacttcgatt cagagaatca gtttgatacc 600
   agcatccagg cactgttcga gtatctgcgc gaggacatgg aggtggacat cgatgccgac 660
   agccagaagg tcaaagagat tctgaaggat agctccctga agaactctga aaaacagagt 720
   cggctgaata agatcctggg gctgaagcct tccgacaaac agaagaaagc catcacaaac 780
   ctgatttctg gaaacaagat caatttcgcc gatctgtacg acaatccaga tctgaaggac 840
   gctgagaaaa actcaatcag cttctccaag gacgattttg atgcactgag tgacgatctg 900
   gcctcaattc tgggcgacag ctttgaactg ctgctgaagg ccaaagctgt ctataactgc 960
   tctgtgctga gtaaggtcat cggggacgag cagtacctga gcttcgccaa ggtgaaaatc 1020
   tacgaaaagc acaaaaccga tctgacaaag ctgaaaaacg tgatcaagaa acatttcccc 1080
   aaggactaca agaaggtctt tggatacaac aagaacgaga aaaacaacaa caattactcc 1140
   ggctatgtgg gagtctgtaa gaccaagagt aagaaactga tcattaacaa ctcagtcaac 1200
   caggaagatt tctacaagtt tctgaaaact atcctgtcag ccaagagcga gatcaaggaa 1260
   gtgaatgaca tcctgaccga gattgaaact ggcacctttc tgccaaagca gatctctaaa 1320
   agtaacgcag agattcccta tcagctgagg aaaatggagc tggaaaagat cctgtccaat 1380
   gccgaaaagc acttctcttt tctgaagcag aaagacgaaa aaggactgtc acatagcgag 1440
   aagatcatta tgctgctgac cttcaagatc ccttactata ttggcccaat caacgataat 1500
   cacaagaaat tctttcccga cagatgctgg gtggtcaaga aagagaaatc cccttctggc 1560
   aagaccacac catggaactt ctttgatcat atcgacaagg aaaaaacagc agaggccttc 1620
   attacttcta ggaccaattt ttgcacttac ctggtgggag agagcgtcct gcctaagtct 1680
   agtctgctgt actccgaata taccgtgctg aacgagatca acaatctgca gatcattatc 1740
   gatggcaaga atatttgtga catcaagctg aaacagaaga tctacgagga cctgttcaag 1800
   aagtacaaga aaattaccca gaagcagatc agcaccttca tcaagcacga aggcatctgc 1860
   aacaaaaccg atgaagtgat catcctgggg attgacaagg aatgtacatc aagcctgaaa 1920
   agctacatcg agctgaaaaa cattttcggc aagcaggtgg atgagatctc cactaagaat 1980
   atgctggagg aaattatcag atgggctacc atctacgacg agggggaagg aaagaccatc 2040
   ctgaaaacaa agatcaaggc agaatacgga aagtattgct ccgacgagca gattaagaaa 2100
   atcctgaacc tgaagttctc cggctggggg cgactgtctc ggaaatttct ggagacagtg 2160
   actagtgaaa tgcccggctt ctcagaacct gtcaatatta tcaccgccat gagggagaca 2220
   cagaacaatc tgatggagct gctgtcctct gagttcacct tcaccgagaa cattaagaaa 2280
   atcaattctg gattcgaaga tgccgagaag cagtttagtt acgacggcct ggtgaaacca 2340
   ctgtttctga gtccctcagt caagaaaatg ctgtggcaga ccctgaagct ggtgaaagag 2400
   attagccata tcacacaggc cccccctaag aaaattttca tcgaaatggc aaagggggcc 2460
   gagctggaac ctgctcggac taagaccaga ctgaaaatcc tgcaggatct gtataacaat 2520
   tgtaagaacg atgctgacgc cttcagctca gagatcaaag acctgagcgg aaagattgag 2580
   aacgaagata atctgaggct gcgctccgac aagctgtacc tgtactatac tcagctgggg 2640
   aaatgcatgt attgtggaaa gccaattgag atcggccacg tgttcgatac ctcaaactac 2700
   gatattgacc atatctatcc ccagagcaag atcaaagacg atagcatttc caatcgggtg 2760
   ctggtctgca gctcctgtaa caagaacaag gaggacaagt acccactgaa atcagagatc 2820
   cagagcaagc agcgcggctt ctggaacttt ctgcagcgaa acaatttcat ttctctggag 2880
   aagctgaata gactgacaag ggccactcca atcagtgacg atgagacagc caagtttatt 2940
   gctaggcagc tggtggaaac tcgccaggct accaaggtgg ccgctaaagt cctggaaaag 3000
   atgttccccg agacaaaaat cgtgtacagc aaggccgaga ctgtctccat gttccggaac 3060
   aagtttgata tcgtgaagtg cagagaaatt aacgattttc accatgctca cgacgcatac 3120
   ctgaatatcg tggtcggcaa cgtgtataat accaagttca caaacaatcc ttggaacttt 3180
   atcaaggaga aaagagataa tccaaagatt gctgacacct acaactacta taaggtgttc 3240
   gattatgacg tcaaaaggaa caatatcaca gcatgggaga aggggaaaac tattatcacc 3300
   gtgaaagaca tgctgaagag aaacacacca atctacacta ggcaggcagc ctgtaagaaa 3360
   ggggagctgt tcaatcagac cattatgaag aaaggactgg gccagcaccc cctgaagaaa 3420
   gaaggacctt tttccaatat ctctaaatac ggcgggtata acaaggtgag cgctgcatac 3480
   tatacactga ttgagtatga ggaaaagggc aacaaaatcc gctccctgga aactattccc 3540
   ctgtacctgg tgaaagatat ccagaaggat caggacgtcc tgaagtctta tctgacagac 3600
   ctgctgggga agaaagagtt caagatcctg gtgcccaaga tcaagatcaa cagcctgctg 3660
   aagatcaatg ggtttccttg ccatattaca ggaaaaacta acgatagttt cctgctgcgc 3720
   cctgccgtgc agttttgctg ttcaaacaat gaggtcctgt acttcaagaa aattatccgg 3780
   ttttccgaaa tccgctctca gcgagagaag atcgggaaaa caattagccc atacgaggac 3840
   ctgagcttcc ggtcatatat caaggagaac ctgtggaaga aaactaagaa cgatgaaatc 3900
   ggagagaagg aattttacga cctgctgcag aagaaaaacc tggagatcta tgatatgctg 3960
   ctgactaagc acaaagacac catctacaag aaacgcccta attctgccac cattgatatc 4020
   ctggtgaagg ggaaagagaa gttcaaaagc ctgattatcg aaaaccagtt tgaagtgatc 4080
   ctggagatcc tgaagctgtt ttctgcaaca cggaatgtca gtgacctgca gcatatcgga 4140
   ggcagcaagt actccggcgt ggccaaaatc gggaacaaga tctctagtct ggataactgt 4200
   atcctgatct atcagtccat caccggcatc ttcgagaaac ggatcgacct gctgaaggtg 4260
   taagaattc 4269
<210> 121
   <211> 4179
   <212> DNA
   <213> Artificial Sequence
<220>
   <221> source
   <223> /note="Description of Artificial Sequence: Synthetic polynucleotide"
<400> 121
   accggtgcca ccatgtaccc atacgatgtt ccagattacg cttcgccgaa gaaaaagcgc 60
   aaggtcgaag cgtccatgac caaggagtat tacctggggc tggatgtggg gaccaattcc 120
   gtgggatggg cagtgaccga ttctcagtac aacctgtgca agtttaagaa aaaggatatg 180
   tggggcatcc ggctgttcga aagcgccaac acagcaaagg accggagact gcagagaggg 240
   aataggcgcc gactggagcg gaaaaagcag agaattgatc tgctgcagga aatcttctcc 300
   ccagagatct gcaagattga ccccactttc tttatccgac tgaacgaatc ccggctgcac 360
   ctggaggaca agtctaacga tttcaaatac ccactgttta ttgagaagga ctattctgat 420
   atcgagtact ataaagagtt ccccaccatt tttcacctga ggaagcatct gatcgagagt 480
   gaggaaaaac aggatatccg gctgatctac ctggccctgc acaacatcat taagacccga 540
   ggacattttc tgattgacgg cgatctgcag agcgccaagc agctgaggcc catcctggat 600
   acattcctgc tgtccctgca ggaggaacag aacctgtcag tgagcctgtc cgaaaatcag 660
   aaggacgagt atgaggaaat tctgaaaaac cgcagcatcg ccaagtccga aaaagtgaaa 720
   aagctgaaga atctgtttga gatctcagac gagctggaaa aagaggagaa gaaggcccag 780
   agcgccgtga tcgagaactt ctgcaagttt atcgtgggaa ataagggcga tgtctgtaaa 840
   ttcctgcggg tgtctaagga ggaactggag attgactctt tcagtttttc agagggcaag 900
   tacgaggacg acatcgtgaa aaacctggag gaaaaagtgc ctgaaaaggt ctacctgttt 960
   gagcagatga aggcaatgta tgattggaat attctggtcg acatcctgga aaccgaggaa 1020
   tacatcagct tcgccaaagt gaagcagtat gagaaacaca agactaacct gcggctgctg 1080
   agagacatca ttctgaaata ctgcaccaag gatgagtata atcggatgtt taacgacgag 1140
   aaggaagctg gcagctacac cgcatatgtg gggaaactga aaaagaacaa caagaagtac 1200
   tggatcgaga aaaagagaaa tcccgaggag ttctacaaat ccctgggcaa gctgctggat 1260
   aaaattgagc ctctgaagga ggacctggaa gtgctgacta tgatgatcga ggagtgtaag 1320
   aaccacaccc tgctgccaat tcagaaaaat aaggacaacg gcgtgatccc ccaccaggtg 1380
   catgaggtcg aactgaaaaa gatcctggaa aatgccaaaa agtactattc cttcctgacc 1440
   gagacagaca aggatgggta ctcagtggtc cagaaaatcg agagcatttt caggtttcgc 1500
   atcccctact atgtggggcc tctgagtacc cggcaccagg aaaagggatc aaacgtgtgg 1560
   atggtcagaa aacctggcag ggaggatcgc atctacccat ggaatatgga ggaaatcatt 1620
   gactttgaga agagcaacga aaatttcatt acacggatga ctaacaaatg tacatatctg 1680
   atcggggaag atgtcctgcc caagcattct ctgctgtaca gtaaatatat ggtgctgaat 1740
   gagctgaaca atgtgaaggt cagaggaaaa aagctgccta catctctgaa acagaaggtg 1800
   ttcgaggacc tgtttgaaaa caaatccaaa gtgactggaa agaatctgct ggagtacctg 1860
   cagatccagg acaaagatat ccagattgac gatctgtctg gcttcgacaa ggacttcaag 1920
   accagcctga agagctatct ggacttcaaa aagcagattt ttggggagga aatcgagaag 1980
   gaaagcattc agaacatgat cgaagatatc attaagtgga tcaccatcta cggcaatgac 2040
   aaggagatgc tgaaacgagt gattcgggct aattatagca accagctgac agaggaacag 2100
   atgaaaaaga tcactggatt tcagtacagt ggctggggga acttctcaaa gatgtttctg 2160
   aaagggatca gcggatccga cgtgagcacc ggcgaaacat tcgacatcat taccgcaatg 2220
   tgggagacag acaacaatct gatgcagatc ctgtcaaaaa agttcacctt tatggacaac 2280
   gtcgaggact tcaacagcgg caaggtcggg aaaatcgaca agattactta cgatagcacc 2340
   gtgaaggaaa tgttcctgtc ccctgagaac aaaagggccg tctggcagac cattcaggtg 2400
   gctgaggaga tcaagaaagt gatgggctgc gagccaaaaa agatctttat tgaaatggca 2460
   cggggcgggg agaaggtgaa aaagaggaca aaatctcgca aggcccagct gctggagctg 2520
   tacgccgctt gcgaggaaga ttgtagagaa ctgatcaagg agattgagga ccgggacgag 2580
   agggacttca atagcatgaa gctgtttctg tactataccc agttcgggaa atgtatgtat 2640
   tccggcgacg acatcgatat taacgagctg attcgcggca attctaagtg ggaccgagat 2700
   cacatctacc cccagagcaa aattaaggac gattccatcg ataacctggt gctggtcaat 2760
   aagacatata atgccaaaaa gtccaatgag ctgctgtctg aggacatcca gaaaaagatg 2820
   cattcattct ggctgagcct gctgaacaaa aagctgatca ctaaaagcaa gtacgaccgc 2880
   ctgactcgaa agggcgactt taccgatgag gaactgagtg ggttcatcgc tagacagctg 2940
   gtggaaacaa ggcagtcaac taaggcaatc gccgatatct tcaagcagat ctacagctcc 3000
   gaggtggtct atgtgaagag cagcctggtg agcgacttca ggaaaaagcc actgaactac 3060
   ctgaagtctc ggagagtcaa tgattaccac catgcaaaag acgcctatct gaacattgtg 3120
   gtcgggaacg tgtacaacaa aaagtttacc agtaatccca tccagtggat gaaaaagaat 3180
   cgcgatacaa actatagcct gaacaaggtg ttcgaacacg acgtggtcat taacggagaa 3240
   gtgatctggg aaaagtgcac ataccatgag gacactaata cctatgatgg aggcactctg 3300
   gaccgaatcc ggaagattgt ggaacgcgat aacattctgt acaccgagta cgcttattgt 3360
   gagaagggcg aactgtttaa tgcaaccatc cagaacaaaa atggaaactc cacagtctct 3420
   ctgaaaaagg gcctggacgt gaaaaagtac gggggatact tcagcgccaa cacaagttac 3480
   ttctcactga tcgagtttga ggacaagaag ggggatagag caaggcacat cattggagtg 3540
   cctatctata ttgcaaacat gctggagcat tctccaagtg ccttcctgga gtactgcgaa 3600
   cagaaggggt atcagaatgt gcggattctg gtcgagaaaa tcaaaaagaa cagcctgctg 3660
   atcattaatg gataccctct gcgcattcga ggcgagaacg aagtggatac ttcctttaag 3720
   agggccatcc agctgaagct ggaccagaaa aactatgagc tggtccgcaa tatcgagaag 3780
   ttcctggaaa aatacgtgga gaaaaaggga aactatccaa ttgacgagaa tagagatcac 3840
   atcacacatg aaaagatgaa ccagctgtac gaggtgctgc tgtccaaaat gaaaaagttc 3900
   aacaagaagg gcatggccga cccctctgat aggatcgaaa agagtaagcc taaattcatc 3960
   aagctggagg acctgatcga taagattaat gtgatcaaca aaatgctgaa cctgctgcgc 4020
   tgtgacaatg atactaaggc cgacctgtct ctgattgagc tgcccaaaaa cgctgggagt 4080
   ttcgtggtca aaaagaatac catcggaaag tcaaaaatca tcctggtgaa tcagagcgtg 4140
   actggactgt acgagaatag acgggaactg taagaattc 4179
<210> 122
   <211> 4086
   <212> DNA
   <213> Artificial Sequence
<220>
   <221> source
   <223> /note="Description of Artificial Sequence: Synthetic polynucleotide"
<400> 122
   accggtgcca ccatgtaccc atacgatgtt ccagattacg cttcgccgaa gaaaaagcgc        60
   aaggtcgaag cgtccatggg gaggaaacct tacattctgt ctctggatat tggaactggg       120
   tccgtcggct acgcttgcat ggataaagga ttcaacgtgc tgaagtacca cgacaaagat       180
   gccctgggag tgtatctgtt cgacggcgct ctgactgcac aggagcggag acagtttagg       240
   acctccaggc gccgaaagaa ccggagaatc aaacgcctgg gcctgctgca ggaactgctg 300
   gcacccctgg tgcagaaccc taatttctac cagtttcagc ggcagttcgc ctggaagaac 360
   gacaatatgg attttaagaa caagagcctg tctgaggtgc tgagcttcct gggatatgaa 420
   tccaagaaat accctaccat ctaccacctg caggaggctc tgctgctgaa agacgagaag 480
   tttgatccag aactgatcta catggcactg tatcatctgg tgaaatacag aggccacttt 540
   ctgttcgatc atctgaagat cgagaacctg actaacaatg acaatatgca cgatttcgtg 600
   gagctgattg aaacctatga gaacctgaac aatatcaagc tgaatctgga ctacgagaaa 660
   accaaagtga tctatgagat tctgaaagac aacgaaatga ctaagaatga tagagccaaa 720
   agggtcaaga acatggagaa gaaactggaa cagttctcta tcatgctgct ggggctgaag 780
   ttcaatgagg gaaaactgtt taaccacgcc gataatgctg aggaactgaa gggggctaac 840
   cagagccata catttgcaga caactacgag gaaaatctga ctcccttcct gaccgtggaa 900
   cagtcagagt ttattgaaag ggccaacaaa atctatctga gcctgactct gcaggatatc 960
   ctgaagggca agaaatcaat ggctatgagc aaagtggccg cttacgacaa gttcagaaat 1020
   gagctgaaac aggtgaagga cattgtctat aaggctgatt ctaccaggac acagttcaag 1080
   aaaatctttg tgagctccaa gaaaagtctg aagcagtacg acgcaactcc caacgatcag 1140
   accttctcta gtctgtgcct gtttgaccag tacctgattc gcccaaagaa acagtatagc 1200
   ctgctgatca aggagctgaa gaaaatcatt ccccaggact ccgaactgta ctttgaggca 1260
   gaaaatgata ccctgctgaa ggtgctgaac accacagaca atgctagcat ccctatgcag 1320
   attaacctgt acgaggcaga aaccatcctg cgaaatcagc agaaatatca cgccgagatc 1380
   acagatgaga tgattgaaaa ggtgctgtct ctgatccagt tccgcattcc atactatgtg 1440
   gggcccctgg tcaacgacca tacagccagt aagtttggat ggatggagcg caaaagtaac 1500
   gaatcaatca agccttggaa tttcgacgag gtggtcgatc gaagtaaatc agccactcag 1560
   tttattaggc gcatgaccaa caagtgttcc tacctgatca atgaggatgt gctgccaaaa 1620
   aactctctgc tgtatcagga gatggaagtc ctgaacgaac tgaatgccac acagatcagg 1680
   ctgcagactg acccaaaaaa ccgcaagtac cgaatgatgc cccagattaa gctgttcgct 1740
   gtggagcaca tctttaagaa atataaaacc gtcagccatt ccaagttcct ggaaattatg 1800
   ctgaacagca atcacaggga gaactttatg aatcatggag aaaagctgag tatcttcggc 1860
   acacaggacg ataagaaatt tgcatcaaag ctgtcaagct accaggacat gactaaaatc 1920
   ttcggggata ttgagggaaa gcgcgcccag attgaggaaa tcattcagtg gatcaccatt 1980
   tttgaggaca agaaaatcct ggtgcagaag ctgaaagagt gctatcctga actgacatcc 2040
   aagcagatca accagctgaa gaaactgaat tactctggct gggggaggct gagtgagaag 2100
   ctgctgactc acgcctatca gggccatagc atcattgaac tgctgcgcca ctccgatgag 2160
   aatttcatgg aaattctgac caacgacgtg tacgggttcc agaattttat caaagaggaa 2220
   aaccaggtcc agagcaataa gatccagcat caggatattg ccaacctgac tacctctccc 2280
   gctctgaaga aaggcatctg gagtacaatt aagctggtgc gggagctgac ttccattttc 2340
   ggggagcctg aaaagatcat tatggagttt gctaccgagg accagcagaa aggcaagaaa 2400
   cagaaatcaa gaaagcagct gtgggacgat aacatcaaga aaaataagct gaaaagcgtg 2460
   gacgagtaca aatatatcat tgatgtcgcc aataagctga acaatgagca gctgcagcag 2520
   gaaaaactgt ggctgtacct gagccagaac ggcaagtgta tgtatagcgg gcagtccatc 2580
   gacctggatg ccctgctgtc ccccaatgct accaagcact acgaggtgga tcatattttc 2640
   cctcggagct tcatcaagga cgatagcatt gacaacaagg tgctggtcat caagaaaatg 2700
   aatcagacaa agggcgatca ggtgcccctg cagttcattc agcagcctta cgagagaatc 2760
   gcatattgga agagcctgaa caaagccggg ctgatctctg atagtaaact gcacaagctg 2820
   atgaaaccag agttcacagc tatggacaag gaaggcttca tccagcggca gctggtggag 2880
   actagacaga tcagcgtgca tgtccgggat tttctgaaag aggaataccc taataccaaa 2940
   gtgatcccaa tgaaggccaa aatggtgagc gagttccgga agaaatttga catcccaaag 3000
   attagacaga tgaacgacgc acaccatgcc atcgatgctt acctgaatgg cgtggtctat 3060
   cacggggcac agctggccta ccccaacgtg gacctgtttg atttcaattt taagtgggag 3120
   aaagtccgag aaaagtggaa agccctggga gagttcaaca caaagcagaa atctcgggaa 3180
   ctgttctttt tcaagaaact ggagaagatg gaagtgtccc agggcgagcg gctgatctct 3240
   aagatcaagc tggacatgaa ccacttcaag atcaactact ccagaaagct ggccaacatc 3300
   cctcagcagt tttataatca gaccgcagtg tctccaaaga cagccgagct gaaatacgaa 3360
   tctaacaaga gtaatgaggt ggtctataag ggactgacac cataccagac ttatgtggtc 3420
   gccatcaaga gcgtgaacaa gaaaggcaag gagaaaatgg aataccagat gatcgaccac 3480
   tacgtgttcg atttttataa attccagaac ggcaatgaga aggaactggc tctgtacctg 3540
   gcacagaggg agaacaagga cgaagtgctg gatgctcaga ttgtctatag tctgaataag 3600
   ggggatctgc tgtacatcaa caatcatccc tgctatttcg tgtcacgcaa agaggtcatc 3660
   aacgcaaagc agtttgagct gaccgtggaa cagcagctgt ctctgtacaa cgtgatgaac 3720
   aacaaggaga caaatgtcga aaagctgctg atcgagtatg acttcattgc cgagaaagtg 3780
   atcaacgaat accaccatta tctgaatagc aagctgaaag aaaagcgagt ccggaccttt 3840
   ttctcagaga gcaaccagac acacgaggac ttcatcaagg ccctggacga gctgtttaag 3900
   gtggtcaccg catccgccac aaggtctgat aaaatcggga gtcgcaagaa cagcatgact 3960
   catcgagcct tcctgggaaa aggcaaggac gtgaagattg cttacacctc catctctgga 4020
   ctgaaaacaa ctaaacctaa gagtctgttt aagctggccg agtcaagaaa cgaactgtaa 4080
   gaattc 4086
<210> 123
   <211> 4209
   <212> DNA
   <213> Artificial Sequence
<220>
   <221> source
   <223> /note="Description of Artificial Sequence: Synthetic polynucleotide"
<400> 123
   accggtgcca ccatgtaccc atacgatgtt ccagattacg cttcgccgaa gaaaaagcgc      60
   aaggtcgaag cgtccatgac aaaaatcaaa gacgactaca tcgtgggact ggacatcggc     120
   acagactcct gcgggtgggt ggctatgaac agcaataatg acattctgaa actgcagggc     180
   aagaccgcaa tcgggtcacg cctgttcgag ggagggaaga gcgcagctga acggagactg     240
   tttcgcacca cacacaggcg catcaaacga cggagatggc gactgaagct gctggaggag     300
   ttcttcgacc cctacatggc agaggtggat ccttatttct ttgcccggct gaaggaatct     360
   ggcctgagtc cactggacaa aagaaagacc gtgagctcca ttgtgttccc cacatccgcc     420
   gaggataaga agttctacga cgattaccct acaatctacc atctgaggta taaactgatg     480
   actgaggacg aaaagttcga tctgcgcgaa gtgtacctgg ctatccacca tatcattaag     540
   taccgaggaa acttcctgta taataccagt gtgaaagact tcaaggcatc aaagatcgat     600
   gtcaaatcta gtatcgagaa gctgaacgag ctgtatgaaa atctgggcct ggacctgaac     660
   gtggagttca acattagcaa tactgccgag atcgaaaagg tgctgaaaga caagcagatc     720
   ttcaagcggg ataaagtcaa gaaaattgcc gagctgtttg ctatcaaaac cgacaacaag     780
   gaacagagca agagaatcaa agatatttcc aaacaggtgg ccaatgctgt cctggggtac     840
   aagaccaggt tcgacacaat cgctctgaaa gagatttcca aggacgaact gtctgattgg     900
   aacttcaaac tgtcagacat cgatgcagac agcaagtttg aggccctgat gggaaacctg     960
   gatgagaatg aacaggccat cctgctgact attaaggagc tgtttaacga agtgaccctg    1020
   aatggaattg tcgaggacgg caacaccctg agcgaatcca tgatcaacaa gtacaatgat    1080
   caccgggacg atctgaagct gctgaaagaa gtgatcgaaa atcatattga cagaaagaaa    1140
   gccaaggagc tggcactggc ctacgatctg tatgtcaaca ataggcacgg acagctgctg    1200
   caggctaaga aaaagctggg caaaatcaag ccccgctcta aggaggactt ctacaaagtg    1260
   gtcaacaaga atctggacga ttcacgggca agcaaggaga tcaaaaagaa aattgaactg    1320
   gacagcttta tgcctaagca gagaaccaac gccaatggcg tgatcccata ccagctgcag    1380
   cagctggagc tggataagat catcgaaaac cagtctaagt actatccatt cctgaaggag 1440
   attaatcccg tgtcaagcca cctgaaagag gccccctata agctggacga actgatccga 1500
   tttcgggtgc cttactatgt cggccccctg atttctccta acgagagtac caaggatatc 1560
   cagacaaaga aaaaccagaa tttcgcctgg atgattcgca aagaggaagg gcgaatcaca 1620
   ccttggaact ttgaccagaa ggtggatcga attgagagcg ccaataagtt catcaaacgg 1680
   atgactacca aggacactta cctgtttggg gaggatgtgc tgccagctaa cagcctgctg 1740
   tatcagaagt tcaccgtcct gaacgaactg aacaacatcc ggattaatgg aaaaagaatc 1800
   tccgtggacc tgaagcagga gatctacgaa aacctgttta agaaacacac aactgtgacc 1860
   gtcaagaaac tggagaatta tctgaaggaa aaccataatc tggtgaaagt cgagatcaag 1920
   gggctggccg atgaaaagaa attcaacagc ggactgacca catacaatag attcaagaac 1980
   ctgaacatct ttgacaacca gattgacgat ctgaagtaca ggaacgattt cgagaagatc 2040
   atcgaatggt ctacaatttt tgaggacaag agtatctaca aagaaaagct gaggagcatc 2100
   gattggctga acgagaagca gattaacgct ctgtctaata tcagactgca ggggtgggga 2160
   aggctgagta agaaactgct ggcacagctg cacgaccata atggccagac catcattgag 2220
   cagctgtggg attcccagaa caatttcatg cagattgtga cacaggccga ctttaaagat 2280
   gctatcgcaa aggccaacca gaatctgctg gtggctacct cagtcgagga cattctgaac 2340
   aatgcataca caagccccgc aaacaagaaa gccatcagac aggtcatcaa ggtggtcgac 2400
   gatatcgtga aggcagcctc cggaaaggtc ccaaaacaga tcgccattga gttcactagg 2460
   gatgctgacg aaaatcccaa gagaagtcag accaggggct caaagctgca gaaagtgtac 2520
   aaggacctga gcactgagct ggcctccaag accattgctg aggaactgaa cgaagcaatc 2580
   aaagacaaga aactggtgca ggataagtac tatctgtact ttatgcagct ggggcgggac 2640
   gcctatacag gagagcctat caatatcgat gaaatccaga agtacgatat cgaccacatt 2700
   ctgccacagt ctttcatcaa ggacgatgcc ctggacaaca gggtgctggt gagccgggct 2760
   gtgaacaatg gcaaatctga taatgtgcct gtcaagctgt ttggcaacga gatggctgca 2820
   aatctgggga tgactatcag gaaaatgtgg gaggaatgga agaacatcgg cctgattagc 2880
   aaaacaaagt acaacaatct gctgactgat cccgaccaca ttaacaagta taagagtgcc 2940
   gggttcatca ggcgccagct ggtggagaca tcacagatca tcaagctggt gagcactatc 3000
   ctgcagagtc gctaccctaa cactgaaatc attaccgtga aggctaagta caatcattat 3060
   ctgcgggaga aatttgacct gtataagagc agagaagtca acgactacca ccatgctatt 3120
   gatgcatatc tgtccgccat ctgcggaaat ctgctgtacc agaactatcc aaatctgcgg 3180
   cccttctttg tgtacggcca gtataagaaa ttctcctctg atcctgacaa agagaaggcc 3240
   atttttaaca aaacccgcaa gttctccttt atctctcagc tgctgaaaaa caagagtgag 3300
   aacagcaagg aaatcgctaa gaaactgaaa cgggcatacc agttcaagta tatgctggtg 3360
   tctcgagaga ctgaaacccg ggaccaggag atgttcaaaa tgaccgtgta cccccggttc 3420
   agccacgata cagtcaaggc tcctaggaac ctgattccaa agaaaatggg catgtcccct 3480
   gacatctacg gaggctatac aaacaattct gacgcataca tggtcatcgt ccgcattgat 3540
   aagaaaaagg gaactgagta taagatcctg ggcattccaa cccgggaact ggtgaatctg 3600
   aaaaaggccg agaaggagga ccattacaaa agctatctga aggagatcct gacaccaagg 3660
   attctgtaca acaaaaatgg gaagcgcgat aaaaagatca cttccttcga aattgtgaaa 3720
   tctaagatcc cctataagca ggtcatccag gatggggaca aaaagtttat gctgggaagt 3780
   tcaacatacg tgtataacgc aaagcagctg acactgagca ctgagtccat gaaagccatc 3840
   actaacaatt tcgataagga cagcgatgag aacgacgctc tgattaaggc atacgatgaa 3900
   atcctggaca aagtggataa gtatctgcca ctgttcgaca tcaacaagtt ccgggagaag 3960
   ctgcacagtg ggcgagaaaa gttcatcaag ctgagcctgg aggacaaaaa ggataccatc 4020
   ctgaaagtgc tggaaggact gcatgataac gctgtcatga caaagatccc tactattggc 4080
   ctgtccacac cactggggtt catgcagttt cccaacggcg tgattctgag cgagaatgcc 4140
   aaactgatct accagtcccc caccgggctg ttcaaaaagt cagtgaagat cagcgacctg 4200
   taagaattc 4209
<210> 124
   <211> 3405
   <212> DNA
   <213> Artificial Sequence
<220>
   <221> source
   <223> /note="Description of Artificial Sequence: Synthetic polynucleotide"
<400> 124
   accggtgcca ccatgtaccc atacgatgtt ccagattacg cttcgccgaa gaaaaagcgc      60
   aaggtcgaag cgtccatggg ctacactgtg ggactggata ttggggtggc ttccgtcggg     120
   gtggctgtgc tggatgagaa tgacaacatc gtggaggctg tgtcaaacat ctttgatgaa     180
   gccgacacaa gcaacaataa ggtgcggaga actctgaggg agggcaggcg cacaaagcgg     240
   cggcagaaaa cccgcattga ggacttcaag cagctgtggg agacttcagg ctacatcatt     300
   cctcacaagc tgcatctgaa tatcattgag ctgcgcaaca aagggctgac cgaactgctg     360
   agcctggatg agctgtattg cgtgctgctg tccatgctga agcaccgggg gatctcctac     420
   ctggaggacg ccgacgatgg cgagaagggg aatgcctata agaaaggact ggcttttaac     480
   gaaaaacagc tgaaggagaa aatgccatgt gagatccagc tggaacgcat gaagaaatac     540
   gggaagtacc atggagagtt catcatcgaa attaatgatg agaaggaata ccagagcaac 600
   gtgttcacca caaaggctta taagaaggag ctggaaaaga tcttcgagac acagcggtgc 660
   aacggcaaca agatcaacac aaagttcatt aagaaataca tggagatcta cgaacgaaag 720
   cgggaatact atatcggacc aggcaatgag aaaagcagaa cagactacgg catctatact 780
   accaggactg atgaggaagg gaatttcatc gacgagaaga acatttttgg caaactgatc 840
   gggaagtgta gtgtgtaccc cgaggaatat agagcaagct ccgcctcata caccgcccag 900
   gagttcaatc tgctgaacga tctgaacaat ctgaaaatca acaatgagaa gctgacagaa 960
   tttcagaaga aagagattgt cgaaatcatt aaggacgctt ctagtgtgaa catgaggaaa 1020
   atcattaaga aagtcatcga tgaggacatt gaacagtaca gcggagcacg aatcgataag 1080
   aaaggcaagg aaatctacca caccttcgag atctatcgga agctgaagaa agagctgaaa 1140
   acaatcaatg tggatatcga ctcttttact agagaggaac tggataagac catggacatc 1200
   ctgaccctga acacagagag ggaaagtatt gtgaaggcct tcgacgaaca gaaatttgtc 1260
   tacgaggaaa atctgatcaa gaaactgatt gagtttcgga agaacaatca gagactgttc 1320
   agcggctggc atagtttttc atacaaggct atgctgcagc tgatcccagt gatgtacaag 1380
   gagcccaaag aacagatgca gctgctgacc gaaatgaacg tgttcaaaag taagaaagag 1440
   aagtacgtca actacaagta catcccagag aacgaagtgg tcaaggagat ctataacccc 1500
   gtggtcgtga agagcattag aacaactgtg aaaattctga atgcactgat caagaaatac 1560
   gggtatcctg aatccgtcgt gatcgagatg ccaagggata agaactctga cgatgagaag 1620
   gaaaagatcg acatgaacca gaagaaaaac caggaggaat acgagaaaat cctgaacaag 1680
   atctacgatg agaagggaat cgaaattacc aacaaggact acaagaaaca gaagaaactg 1740
   gtgctgaagc tgaaactgtg gaacgagcag gaaggactgt gcctgtattc cggcaagaaa 1800
   atcgctattg aggatctgct gaatcacccc gagttctttg aaattgacca tatcattcct 1860
   aagagcatct ccctggacga ttctcgcagt aacaaggtcc tggtgtacaa aacagaaaat 1920
   tctatcaagg agaacgatac cccctaccac tatctgacac ggattaacgg aaagtggggc 1980
   tttgacgaat ataaagctaa tgtgctggag ctgagaaggc gcggcaagat cgacgataag 2040
   aaagtgaaca atctgctgtg catggaggat atcactaaga ttgacgtcgt gaaagggttc 2100
   attaaccgca atctgaacga caccagatac gcatccaggg tggtgctgaa cgaaatgcag 2160
   tccttctttg agtctcgaaa gtactgtaat actaaggtca aagtgatccg aggctctctg 2220
   acctatcaga tgcggcagga tctgcacctg aagaaaaaca gagaggaatc atacagccac 2280
   catgctgtgg acgcaatgct gatcgcattc tcccagaagg ggtacgaggc ctataggaag 2340
   atccagaaag attgctacga ctttgagaca ggcgaaattc tggacaagga aaaatggaat 2400
   aagtacattg acgatgacga gtttgatgac atcctgtata aagagaggat gaacgaaatc 2460
   cgcaagaaaa tcattgaggc cgaggaaaag gtgaagtaca actacaagat cgataagaag 2520
   tgcaatcgcg ggctgtgtaa ccagactatc tacgggaccc gagaaaagga cggaaaaatc 2580
   cacaagattt caagctacaa catctatgat gacaaggagt gtaattccct gaagaaaatg 2640
   attaacagtg ggaaaggatc agatctgctg atgtacaaca atgatcctaa gacatatcgc 2700
   gacatgctga aaatcctgga aacttactcc tctgagaaga atccattcgt ggcatataac 2760
   aaagagacag gagactactt tcggaaatat tctaagaatc acaacggacc caaggtcgag 2820
   aaggtgaaat actatagcgg ccagatcaac tcctgcatcg atatttctca caagtacggc 2880
   catgccaaaa atagtaagaa agtcgtgctg gtgtcactga acccttatag aaccgacgtc 2940
   tactatgata atgacacagg caagtactat ctggtcgggg tgaagtacaa tcatatcaaa 3000
   tgtgtcggaa acaagtacgt gattgatagc gagacatata acgaactgct gaggaaggag 3060
   ggcgtgctga acagcgatga gaacctggag gacctgaaca gcaaaaacat cacttacaag 3120
   ttcagtctgt acaagaacga catcatccag tacgagaagg gcggggaata ctatacagag 3180
   cgctttctga gccgaatcaa agaacagaag aacctgattg agactaaacc catcaataag 3240
   cctaacttcc agcggaagaa taagaaaggc gagtgggaaa ataccagaaa ccagatcgcc 3300
   ctggctaaga ctaaatacgt ggggaagctg gtcaccgatg tgctgggaaa ctgttacatc 3360
   gtgaacatgg agaagttctc cctggtcgtg gacaaataag aattc 3405
<210> 125
   <211> 3474
   <212> DNA
   <213> Artificial Sequence
<220>
   <221> source
   <223> /note="Description of Artificial Sequence: Synthetic polynucleotide"
<400> 125
   accggtgcca ccatgtaccc atacgatgtt ccagattacg cttcgccgaa gaaaaagcgc        60
   aaggtcgaag cgtccatgac taacggcaag attctggggc tggacattgg catcgcaagc       120
   gtgggggtgg ggattattga ggcaaaaact ggaaaggtgg tgcatgccaa ttcccggctg       180
   ttctctgccg ctaacgctga gaacaatgca gaacggagag ggtttagggg atctaggcgc       240
   ctgaatcgac ggaagaaaca ccgcgtgaag cgagtccggg atctgttcga gaaatacgga       300
   atcgtcaccg actttcgcaa cctgaatctg aacccttatg agctgcgagt gaagggcctg       360
   accgaacagc tgaaaaacga ggaactgttc gcagccctga gaacaatctc taagagaagg       420
   gggattagtt acctggacga tgccgaggac gatagtaccg gatcaacaga ctatgctaag       480
   agcatcgatg agaatcgccg actgctgaaa aacaagacac caggccagat tcagctggag       540
   aggctggaaa agtacggcca gctgcgcggg aatttcaccg tctatgacga gaacggggaa 600
   gcccatcgcc tgatcaatgt gtttagtaca tcagattacg agaaagaagc acggaagatc 660
   ctggagacac aggccgacta caacaagaaa atcacagctg agttcattga cgattatgtg 720
   gaaatcctga cccagaaacg aaagtactat cacggccccg ggaacgaaaa gagccggact 780
   gactacggac ggttccggac cgatgggacc acactggaga atattttcgg aatcctgatt 840
   ggcaagtgca acttttaccc tgatgaatat cgagcaagca aggccagcta caccgcacag 900
   gagtataatt tcctgaacga cctgaacaat ctgaaggtga gcaccgaaac agggaagctg 960
   tcaacagagc agaaagaaag cctggtggag tttgccaaga atactgctac cctgggaccc 1020
   gctaaactgc tgaaggagat cgcaaaaatt ctggactgta aggtggatga gatcaaagga 1080
   tacagagagg acgataaagg caagccagat ctgcatacct tcgagcccta taggaaactg 1140
   aagtttaatc tggaaagcat caacattgac gatctgtccc gcgaagtgat cgacaagctg 1200
   gctgatattc tgactctgaa caccgagaga gaaggaatcg aggacgcaat taagaggaat 1260
   ctgccaaacc agttcacaga ggaacagatc agcgagatca tcaaggtgcg gaagagccag 1320
   tccactgctt tcaataaggg ctggcactct tttagtgcaa aactgatgaa cgagctgatc 1380
   cccgaactgt acgccacctc cgacgagcag atgacaattc tgactcggct ggaaaaattc 1440
   aaggtcaata agaaaagctc caaaaacaca aagactatcg acgagaagga agtcactgat 1500
   gagatctaca atcctgtggt cgccaagagc gtgagacaga ccatcaaaat cattaacgct 1560
   gcagtcaaga aatatggcga cttcgataag atcgtgattg aaatgccacg ggataaaaat 1620
   gctgacgatg agaagaagtt catcgacaag agaaataagg agaacaagaa ggaaaaggac 1680
   gatgccctga aaagggccgc ttacctgtat aattctagtg acaagctgcc cgatgaggtg 1740
   ttccacggca acaagcagct ggaaaccaaa atccgactgt ggtatcagca gggggagcgg 1800
   tgcctgtata gtggaaagcc catctcaatt caggagctgg tgcataactc taacaatttc 1860
   gaaatcgatc acattctgcc tctgtcactg agctttgacg atagtctggc caataaggtg 1920
   ctggtctacg cttggacaaa ccaggagaaa ggccagaaaa ccccttatca ggtcatcgac 1980
   tccatggatg cagcctggtc tttcagggag atgaaggact acgtgctgaa acagaaggga 2040
   ctgggcaaga aaaagcgcga ctatctgctg actaccgaga acatcgataa gattgaagtg 2100
   aagaagaagt tcatcgagag gaatctggtg gatactcgct acgcatctcg agtggtcctg 2160
   aactctctgc agagtgccct gagagagctg gggaaagaca ctaaggtgtc tgtggtcagg 2220
   ggacagttca ccagtcagct gcggagaaaa tggaagatcg ataagagccg cgagacatac 2280
   caccatcacg cagtggacgc cctgatcatt gctgcatcaa gccagctgaa actgtgggag 2340
   aagcaggaca atcccatgtt tgtggattat ggcaagaacc aggtggtcga caaacagact 2400
   ggggagatcc tgtccgtgtc tgacgatgag tacaaggaac tggtgttcca gcccccttat 2460
   cagggctttg tgaataccat ctcctctaaa gggttcgagg acgaaattct gtttagctac 2520
   caggtggatt ccaaatataa ccggaaggtc agtgacgcaa ccatctactc aacaagaaaa 2580
   gccaagattg gcaaggataa gaaagaggaa acctacgtgc tgggaaaaat caaggacatc 2640
   tactcccaga atggcttcga taccttcatc aagaagtaca acaaagataa gactcagttc 2700
   ctgatgtatc agaaggactc tctgacatgg gagaacgtga tcgaagtcat tctgagggac 2760
   tacccaacaa ctaagaaaag cgaggacggc aaaaatgatg tgaagtgcaa cccctttgag 2820
   gaatacaggc gcgagaatgg gctgatctgt aagtattcca agaaagggaa aggaactccc 2880
   atcaagagcc tgaagtacta tgacaagaaa ctggggaact gcatcgatat taccccagag 2940
   gaatcacgca ataaggtcat cctgcagagc attaaccctt ggcgagccga cgtgtacttc 3000
   aatccagaga cactgaagta cgaactgatg ggcctgaaat attcagatct gagctttgaa 3060
   aagggcactg ggaactacca tatcagccag gagaaatatg acgctatcaa agagaaggaa 3120
   ggaattggca agaaatccga gttcaagttt acactgtacc gcaacgacct gatcctgatc 3180
   aaggatatcg ccagtggcga gcaggaaatc tacagattcc tgtcaagaac tatgcccaat 3240
   gtgaaccact acgtcgagct gaagccttac gacaaggaaa agttcgataa cgtgcaggag 3300
   ctggtcgaag cactgggaga ggcagataaa gtgggacgat gtatcaaagg actgaataag 3360
   ccaaacatca gcatctacaa ggtgagaacc gacgtcctgg gaaacaaata tttcgtgaag 3420
   aaaaagggcg acaaacccaa gctggatttt aagaacaaca agaagtaaga attc 3474
<210> 126
   <211> 3462
   <212> DNA
   <213> Artificial Sequence
<220>
   <221> source
   <223> /note="Description of Artificial Sequence: Synthetic polynucleotide"
<400> 126
   accggtgcca ccatgtaccc atacgatgtt ccagattacg cttcgccgaa gaaaaagcgc      60
   aaggtcgaag cgtccatggc cgaccgaatc tctctggggc tggacattgg ggtggcaagc     120
   gtggggttct cagtgctgga cattgataag ggaaaagtca ttgagctggg cgccaggctg     180
   ttctctgcta ctgtggccgc tggcaaccag gatcgaagag acatgcgagg agccaggcgc     240
   ctgctgaacc ggaacaagca gcgacggcag gataccggaa agctgttcaa gaaatttggc     300
   ctgatcgacg attttgataa gggcagcttc tacgacaact ttaatcagaa cctgaatcct     360
   tatgagctga gagtgaaagg cctgacagaa cagctgacta aggaggaact ggccgagtct     420
   ctgtaccaga tcgtgaaaca tagggggatt agttatgcac tgaaggacgc cgatgtggac    480
   gaaggcggga cagactactc agtcagcctg aaaatcaaca gccaggagct ggcagaaaag 540
   actccagccc agattcagct gcagagactg aatgattatg gaaaggtgag gggccaggtg 600
   gtcatcggcg acgatccaga caaccagaag gtgctgctga atgtgttccc cacatcagct 660
   tacgagaaag aagcaaagca gatcattgcc actcagcagc agttctatcc tgagagcctg 720
   accgacaagt tcaccgagga atactgccag atcctgactc gcaagcgaga ttattttgtg 780
   gggccaggaa acgagaaaag ccggaccgac tacgggatct acaagactga tggaagaacc 840
   ctggacaatc tgttcgagga actgatcggc cacgataaga tctaccccga ggaactgcgg 900
   gcatctgcag ccagttatac cgcccagctg tttaacgtgc tgaatgacct gaacaatctg 960
   agaatcctga actacgagga tgggaaactg acaaaggagg acaaggaaaa gatcatcgct 1020
   gaaattaaga acaacaccac aactatcaac atgctgaatg tgattaagaa agtcgccggg 1080
   tgttccaagg acgatatcaa aggattccga gtgaatgaga aggataaacc cgaaatcagc 1140
   tccatgcctg tgtaccgcaa aattcataag gacctgctga aggccggcgt ggatatctca 1200
   gactggcccg tcgagttcat cgacgaactg agctttattc tgacactgaa cactgagaat 1260
   ggggaaattc gcaaacagct gaacaatcga ctggccccta agttcgattt tctgaacgct 1320
   gacctgatcc agctgatcat tgataataag gactcctttg agattaagac taacaacaag 1380
   tggcacagat tcagcgtgaa aaccatgaac aaactgatcc cagagatgat ggaaagaccc 1440
   gtggagcaga tgaccctgct gaatgaaatg ggactggtca agaaagataa gaaacgcttt 1500
   gagaacaata agtacctgcc ttacaaggaa atcgcaaagg acattttcaa cccagtggcc 1560
   tccaaatctg tccgcgaggc cctgaagatc gtgaatgctg tcctgaagaa atacggccac 1620
   attgattatc tggtggtcga aatgcctcgg gataaaaacc tgaaggagga acaggacaat 1680
   atcaaggagt tccagaacaa aaataagaaa gctaaggacg ctgcattcga agcatttgtg 1740
   aaatcagtcg ggagcgagca gagagtgaag gaagccctgt ctaaaaaccg gaagctgcag 1800
   atgaagatga gactgtggta tcagcagcag gagatcgatc catataatgg aaagacaatc 1860
   gatgccactg acctgattaa caatcctgat aagttcgaga ttgaccatat cattccacag 1920
   agtatctcat tttacgacag tattaacaat aagaccctgt gcttcgcctc aatgaaccag 1980
   gtgaaaggac agaaaacccc ctacgagttt atgctggaag gccacgggca gtcctatgac 2040
   aagttcaaag ctacagtgat ggcaaacaag aattttggca aggctaaaag ggcaaactac 2100
   ctgttcgagg aaaatgtgag cgatatcgag actcggaaga gattcctgtc ccgcaacctg 2160
   gtggacaccc gatattctag tcgggtggtg ctgaacagcc tgcaggattt ctttcgggag 2220
   aaatctgccg acaccaaggt gacagtcatt cgcggcaagt ttacctccaa catgcgaaaa 2280
   cattggcaca tcgataagac tagggagaca ttccaccatc acgccattga cgcttctatc 2340
   attgccgcta caccatttct gcgcatgtgg aagaaaggag gcactatctt ccccgtgaag 2400
   gtcggagaag aaagtatcga tattgagaca ggcgaaattc tggacgataa gaattttgac 2460
   aaagcaatgt acgaggaacc ctatagtggc ttcgtgtcag agatcatgaa cgccgacgat 2520
   cggatcaagt tcagccacca ggtggataag aaaatgaata ggaaggtgag cgacgccacc 2580
   atctacagta ctcgcaccgg gaaactggct aaggataaga aagacgctga gtacatcgtg 2640
   gcaaaggtca aagatatcta cagcgtggac ggattcaaga agttcaagaa agtctacgat 2700
   aaggacaaaa ccaagtttct gctgtacaaa tatgatccta ggacattctc aaagctggag 2760
   cgcatcatta gcgattgccc agacaaagtg gaaaaggtcc agacaaacgg caaagtgaag 2820
   gctgtcgata tcagtccatt cgagatgtac agaagggacc atgggatgat caagaaatac 2880
   tcaaagaaag gaaacggccc cgccatcaag cagctgaagt acctggataa gaaactgggc 2940
   agccacatcg acattacccc cgcaaacgcc aatggaaaac acgtgatcct gcagagtctg 3000
   aagccttgga gaaccgacgt ctatctgaac cacgagacag gcgagtacga aatcatgggg 3060
   attaagtata gcgatctgaa gttcaacaag aatgaggggt acggaatcaa gaaagacaag 3120
   tatctggaaa ttaagaaagt ggaggaagtc tccgagaagt ctgagttcat gtttagcctg 3180
   tacaggaagg atcgcgtgaa agtccaggac atgaaaaccg gcgagtccgt ggaactgctg 3240
   ttctggagca ggaacttttc caataagaaa tacgctgagc tgaagcccat ctcccaggca 3300
   gaaaacgaca agaaactgcc tgtgtatggc aaagggagac tgatcaagag gctgattccc 3360
   aaaaactgta agatctggaa agtgaatacc acaattctgg gcgatcccta ctatctggag 3420
   aaagaaagcg actcccctaa ggatatcctg gactaagaat tc 3462
<210> 127
   <211> 4611
   <212> DNA
   <213> Artificial Sequence
<220>
   <221> source
   <223> /note="Description of Artificial Sequence: Synthetic polynucleotide"
<400> 127
   accggtgcca ccatgtaccc atacgatgtt ccagattacg cttcgccgaa gaaaaagcgc 60
   aaggtcgaag cgtccatgaa gaaaacactg ggactggacc tggggactaa cagcattggg 120
   tgggccgtca tcaactcaaa catcgactca gaaggaaaag aaaagctggt ggggatcagc 180
   tcctgcggaa gccggatcat tcctatggac gctaccacac tgggagattt cggaaagggc 240
   aacactaaaa gtccagtggc agagcgaacc cgactgcggg gcattcggag actgctggaa 300
   aggtcactgc tgaggcgcga gcgactgcac cgagtgctgt cagtcatggg gttcctgcca 360
   gagcattacg ctagccagct ggaccgctat ggaaagtttc tgccagaaac cgagcccaag 420
   ctggcatggt acaaagacga tagcggcagg tatcagttcc tgtttcagaa gtccttccac 480
   gagatgctgg aggactttcg acagcatcag ccagaactgg tggcaggaga gaagaaaatc 540
   ccttacgatt ggacaatcta ctatctgcgg aagaaagcac tgtctcagga gatcactaag 600
   gaggaactgg cctggattct gctgaatttc aaccagaaac gcggctacta tcagctgcga 660
   ggggaggaag agcaggaaga gaacaataag agcgtggagt atcacgccct gaaagtggtg 720
   agcgtcgagg actctggaga aagaaagggc aaagatatct ggtacaatgt gactctggag 780
   aacggatggg tctatcgacg ggctagcaac attcccctgg actggaccgg caaggtgaaa 840
   gagttcgtgg tcactaccga gctggacgat gccgggaacc caaagaaaga taaggaagga 900
   aatgtgaaaa gatcctttag ggcaccaaag gaggacgatt gggggctgct gaaaactagg 960
   acccaggctc agatcgacga atccggcaag accgtgggaa cttacatcta cgagtctctg 1020
   ctgtgtatgc ccaaccagaa gatccgggga aaactggtga ggaccattga acgcaagtac 1080
   tataaagacg agctgagaca gatcctggtg aagcagagcg agttccacgc cgctctgcag 1140
   gatcataatc tgctgctgtc ctgtatcgaa gagctgtacc ctaacaatga ggcccacaga 1200
   aggctgctga gcgccagcag cttcatctac ttcctgatcg aggacattct gttttatcag 1260
   cgcccactga agtcccagaa agggctgatc gataactgcc cctacgagtc tcacatctac 1320
   aaggataaga aagatggaag tctgcaccat gtgcctctga agtgtgtcag caaatcccat 1380
   ccactgttcc aggaatttcg cctgtggcag ttcctgtcta acctgcgaat ctaccagaga 1440
   gagaggatga tggacggcag tctgaaactg gacgtggatg tcacccggga gttcctgccc 1500
   tcagaagagg actacgtgaa gctgtttgat tggctgaatg agagaaagga aatctctcag 1560
   aaattcctgc tggcttataa accttttggg ctgaagaaaa acgaacaggc aaattacaga 1620
   tggaactatg tggaggacaa gagctacccc tgcaacgaga cacgggcaga aatcaagagc 1680
   agactgtcca aagccggagt gcctgaagag tttctgactg aagagaagga agaggccctg 1740
   tggcacatcc tgtattctat tagtgataag aaagagctga ctaaggctct gggcaccttc 1800
   gcagccaaaa actgtctgaa tgagtctttc gtggaagtct ttgccaagat cccccctttt 1860
   gagtcaaact acgctgcata tagcctgaag gctattagga aactgctggc actgatgcgc 1920
   atggggaagt actggaatga acaggccatc gacaggcaga ctcgcgatcg aatcgagaaa 1980
   attctgaccg gagagtatga cgaaacaatc cggagcagag tgagggagaa ggcaatgctg 2040
   ctgaccgata ttagcagctt ccggggcctg cctctgtggc tggcctgtta catcgtgtat 2100
   gaccgccact cagagagcaa ggaactggtc aaatgggaga caccagccga catcgatcat 2160
   ttcctgtcca agtttaaaca gaacagcctg cggaacccca tcgtggaaca ggtcattaca 2220
   gagtccctgc gcactgtgcg agacatttgg aagcaggagg gaaaaatcga tgagattcac 2280
   gtggaactgg gccgggagat gaagaaccct gcaaaagagc gcgcccgaat tacagctcag 2340
   gtgcaggaaa atgagaacac taatctgaga atcaaggctc tgctggcaga gttcatgaac 2400
   cccgaatttg agattgaaaa tgtgcatcca tactcacccg gccagcagga aatcctgcgg 2460
   atctacgagg acggcgtgct gagcgggatc gctgagaagg atctgcctga cgatatcaca 2520
   gcaattctga agaaattccg agaaaacgac gtgaagaaac ggccaacaac tagcgaagtc 2580
   ctgcggtaca aactgtggct ggagcagcgg tacagatccc catataccgg aagagtgatc 2640
   cccctgggca agctgttcac acctgcttac gagatcgaac acgtgattcc ccagagccgg 2700
   tattttgacg attccatctc taacaaagtg atctgcgaaa gtgccgtcaa taagctgaaa 2760
   gataactgtc tgggctatga gttcatcaag aaacattccg gggagatggt ggaactgggg 2820
   aatggagaga cagtgcccgt gttcagcgtg gaagagtacg aacggttcgt caaggagtct 2880
   tacttcggca acagtaagaa aatgaagaaa ctgctgctgg aggacatccc agatagcttc 2940
   attgagagac agctgaatga cagtcgatac atctcacggg tggtcacatc tctgctgagt 3000
   aacctggtgt gcgaagaggg agagcaggat ggcctgtcca agaatgtgat cgtctgtacc 3060
   ggcgggatta cagacaggct gaagaaagat tggggagtgc aggaagtctg gaaccgcatc 3120
   attctgcctc ggttcctgag actgaatgag atcaccggac ggacagactt tacaagtact 3180
   tcagtgaacg gccacctgct gcctgccctg ccactgtacc tgcagaaggg ctttaataag 3240
   aaaagaattg accataggca ccatgccatg gatgctatcg tgattgcctg cgctaaccgg 3300
   aatatcgtca actacctgaa caattcctct gctagaaaga acagcgaaat tagccgatat 3360
   gacctgcagc ggctgctgtg tgagaaggtg aaaaccgatg ccaacggcaa ttacaagtgg 3420
   atcctgagga aaccatggga gacattcacc caggatgtgt atgccgctct gacaaacatc 3480
   gtggtcagct ttaagcagaa tctgcgcgtg attaatcgaa ccacaaacta ctatcagcac 3540
   tacaacgagg caggggaaaa gcgcatgatc cctcagacca aaggcgacag atgggccatt 3600
   agaaagccaa tgcataaaga tactgtgtat ggcgaagtca atctgagaaa ggagaaaacc 3660
   ctgccactga gggacgtggt caagaacccc agtatcgtgg tcgataaatc actgaagaac 3720
   aagctgtacg agctgctgaa gagccagtat gacctgaagg caatcgccaa atacttcgag 3780
   acacaccagg acgtgtgggc agatgtcaac ctgaagaaaa ttaaggtgta ctacttcact 3840
   aaggagacaa acgaaagatt ctttgccact aggaagagcc tggacccatc ctttgatcag 3900
   aagaaaatcg aagaggaagt gactgatacc ggcgtccaga agattctgct gcaccatctg 3960
   cagcagaaca ataacgaccc tgatatggcc ttttccccag acggcatcga taggatgaac 4020
   cagaatatga ccattctgaa tgacgggaag tggcaccagc ccatctacaa agtgcgcaca 4080
   tatgaaaagg cagataaatt cgccgtcggc gagtctggga acaaggccaa gaaatttgtg 4140
   gaagcagcca aaggcaccaa tctgttcttt gccgtgtacg agagtgtcca ggaggacgaa 4200
   gctatcggga agcaggtctg caaacggaca ttcgccacta tccccctgaa cgaagtgatc 4260
   aagagaaaga aacagggcct gcccgctgca cctgaggacc tgaacgggaa tctgcccaag 4320
   tttgtgctga gccctaacga tctggtctac ctgcccaccg aggaagagag gaatagttca 4380
   cgcatcattc agcctctgga cagggagcgc atctataaga tggtgagctc ctctgggagt 4440
   cagtgcttct ttatcaaagt gttcgtcgcc aattcaattt gggataagaa cgaatacagc 4500
   agcctgaaca agatggagag ggctattaca aacgaaatga tcaaggagat ttgtgtgcct 4560
   atcaaaattg accgcctggg caatgtcagc ctgatccaga tttaagaatt c 4611
<210> 128
   <211> 4458
   <212> DNA
   <213> Artificial Sequence
<220>
   <221> source
   <223> /note="Description of Artificial Sequence: Synthetic polynucleotide"
<400> 128
   accggtgcca ccatgtaccc atacgatgtt ccagattacg cttcgccgaa gaaaaagcgc      60
   aaggtcgaag cgtccatgac aaagactatt ctgggactgg atctggggac taacagcatt     120
   ggatgggcac tgattaacca ggacttcgat aacaaaaagg gggagatcct gggcatgggc     180
   agccggatca ttcctatgac acaggatatt aaggacgagt tcggaaaagg caactctatc     240
   agtcagacag ctgagcgaac tcgactgcgg ggagtccgga gactgatcca cagaactctg     300
   ctgaggcgcg agcggctgca tagagtgctg aatatcattg gcttcctgcc agaacactat     360
   gccaaccaga ttgactttga gaagaggttc ggcaagttca agcctgaatc tgagccaaaa     420
   atcagtttcg atggaaatga cgtgttcctg tttgagaaga gctaccagga aatgctggtc     480
   gactttaaaa tccaccagcc acagctggtg agcaacggca agaaaattcc ccatgactgg     540
   acaatctact atctgagaaa gaaagctctg tctaagaaaa ttgagaagga ggaactggca     600
   tggatcctgc tgaacttcaa tcagaaacgg ggatactatc agctgagagg cgaggaagag     660
   gaagagacac caaataagct ggtggagttt catagcctga aaattgtgga ggtcaacgcc     720
   gatgaacccc agaaaggaaa gcctgagatc tggtactcac tggtgctgga aaacggctgg     780
   gtctatcgac gggctagcaa gactcccctg ttcgattgga aagacaagat tagggagttt     840
   atcgtgacca cagaaatcaa caatgatgga actgtcaaaa ccgacaagga gggcaccgaa     900
   aagaggagct tccgcgcacc aaaacccgag gactggactc tgcagaagaa aaagaccgag     960
   ttcgagctgt caaagagcgg gaatgaagtg ggagccttca tctacgaaag tatcctgcag    1020
   aaacccaacc agaagattag aggcaaactg atttcaacca tcgagaggaa attttataag    1080
   gaagagctga aaaccatcct gaaaacacag ctgttctttc acaaagaact gaaggatgag    1140
   aaactgtaca atgcctgcat cgaagagctg tataagaaca atgaagctca ccggagcctg 1200
   ctgtccaaca aggggttcga gcatctgttt attaacgaca tcctgttcta ccagcgacct 1260
   ctgcggtcta aaaagagtca gatctcaaac tgcccactgg agaagcgcac atataaaaag 1320
   gaggggattg aaatcactga gggcatcaaa gtgatctcca aatctaatcc aatctaccag 1380
   gagttccggc tgtggcagtg gattagcaac ctgtccctgt attgtatcga acccaccgag 1440
   acaaatgtga cttcaacctt tctgaacagc attgaagatt acgagaatct gttcgaattt 1500
   ctgaacaatc gcaaggaaat cgagcagaag cacctgctga aatatctgct ggagaaccag 1560
   gggtttaaag gaaagctgct gacaaacgaa ctggagaagt tccgctggaa ttttgtcgct 1620
   gacaaaaagt acccctgtaa tgagacaggc agcctgctgc atactcggct gagcaaagtg 1680
   aagaacattt cccctgattt cctgaccaag gaaatcgagc accagctgtg gcatatcatc 1740
   tacagcgtga ccgacaagat tgaatatgag caggccctga aaacctttgc tcggaaaaac 1800
   aatctggatg tggactcctt ctttgagcac ttcaaaaaga tccccccttt tgagtctacc 1860
   tacggagcat atagtgaaaa ggccatcaag aagctgctgc cactgatcag actgggcaaa 1920
   tactggaact gggaggccat tgatagtatc tcaaaggaca ggattagtaa aatcctgtca 1980
   ggggaatacg atgagaacat taagaacaga gtgagggaga aagcagtcca cctgacctcc 2040
   gaaaacaatt tccagggact gcaggagtgg ctggccaagt acatcgtcta tgatcgccat 2100
   tctgagggca atgacctggg gaagtggact agcgtgtccg acctggagac atacctgaag 2160
   gagttcaagc agcatagcct gcggaaccct attgtggagc aggtcatcac agaaactctg 2220
   agagtggtca aggatatttg gatcaagcac gggaaaggaa ccgaaaattt ctttgacgaa 2280
   atccatgtgg agctgggccg ggaaatgaag aacaattccg aggatcgcaa acgactgacc 2340
   aacacaatta ctgaaaacga gaatacaaac ctgagaatca aggccctgct gatggaaatg 2400
   atgaatgata acgacgtgga gaacgtcagg ccttactctc caagtcagca ggagattctg 2460
   aagatctatg aggacggagc tctgaatagc aacatcgagc tggacgatga aattgtgaag 2520
   atctccaaaa aggcagagcc caccaaatct gaactgcagc gctacaagct gtggctggag 2580
   cagaaatacc gatcccctta tactggccag gtcatcccac tgaacaagct gttcacctct 2640
   gaatatgaga tcgaacacgt ggtccctcag agtcgcttct ttgacgatag cttcagcaac 2700
   aaagtgatct gcgagtcagc cgtcaacaag cggaaggata accagctggg gctgcagttc 2760
   atcaagaacc atagcggaga aaaagtggag ctgggcttcg ggaaggtggt ccaggtcttt 2820
   acagaagagc agtacctgga ttttgtgaag gagcactata gcaaaaatcg ctccaagcat 2880
   aacaaactgc tgctggaaga gattcccgag aagatgatcg aaaggcagct gaatgacact 2940
   cgctacatca gtaagttcgt gagctccatt ctgtccaaca tcgtcagatc tgagaaggac 3000
   gatgacggcc tgaatagcaa aaacattctg cctggaaatg gcaagatcac taccgaactg 3060
   aaaagggact gggggctgaa tgatgtgtgg aacgacctga ttctgccaag attcgagagg 3120
   atgaatacca tcacaaacag cgatctgttt acaacttaca acgacaagta tcagaaacac 3180
   ctgcccaccg tgcctttcga gtactccaag ggctttcaga aaaagcgcat tgatcaccga 3240
   caccatgcta tggacgcact ggtcatcgca tgtgccacac gggatcatct gaatctgatg 3300
   aacaatcagt ctgccaagag tgaactgaaa cgatacgacc tgcagaacaa gctgcggaaa 3360
   aaggagcctt acttcaacca gaaggagaac aaacagaagg aagccttcaa ggattttatc 3420
   aaaccatggg gcactttcac cgaggacagc aagaatgctc tggaaaaaat cattatctcc 3480
   tttaagcaga acctgagagt gatcaacaag gcaacaaact catacgagag ctataaggat 3540
   gaggacggga atctgaggat cggcaaggat gggaaaccag agaagggcct gatcaagcag 3600
   aaggggctga actgggcaat cagaaagccc ctgcacaaag acaccgtgtc aggccagatt 3660
   aacctgagca ggatcaagct gcccaatggg aaaatcctga cagccactcg caagaatctg 3720
   gataccagtt tcgacctgaa aacaattgag aactcaatca ccgacacagg cattcagaaa 3780
   atcctgaaga attacctgct gtccaaggaa tctccagagc tggccttctc tcccgagggc 3840
   ctggaagaga tgaacaatga gatcgaaaag tacaacaatg ggaaatttca ccatcctatt 3900
   aacaaggcta ggatctatga actggggagc aaattcaatg tcggacagac aggcaacaga 3960
   agggataagt ttgtggaggc cgctaaagga actaatctgt tctttgctat ctaccaggac 4020
   gagaataaga accgctctta tgaaactatt cccctgaacg aagtgatcga gcaccagaag 4080
   tggcgagcaa gtctgcctaa ggaagagcag gagaaaattc cactggtgcc cgtcaacaag 4140
   ctgaagggga ccttcatctt ttccctgtct cccaacgatc tggtgtacgt cccttccaat 4200
   gacgagctgg aacgaagtgc ttcaattgac ttctctaagc tgaaaaagga acagatcaac 4260
   cggctgtata aaatggtgtc tagttcagga tcccagtgct tctttgtgaa gagtgaggtc 4320
   gcaacctcag tggtcaacaa aatggaatac agcagcctga acaagatgga gaagtctatc 4380
   gataacctga tggtgaagga gatttgtatc aaactgaaga ttgacaggct gggcaacatc 4440
   agcaaggcct aagaattc 4458
<210> 129
   <211> 4689
   <212> DNA
   <213> Artificial Sequence
<220>
   <221> source
   <223> /note="Description of Artificial Sequence: Synthetic polynucleotide"
<400> 129
   accggtgcca ccatgtaccc atacgatgtt ccagattacg cttcgccgaa gaaaaagcgc 60
   aaggtcgaag cgtccatgac caaaatcctg gggctggacc tggggactaa tagcatcggg 120
   tgggcaatcc gcgacacaga aaatgagggc atcaatcaga tcctggacaa gggcgcccgc 180
   attttcagcg agggagtgaa gtccgagaat ggcaaagaaa tcagtagggc agctgaacga 240
   accgcttacc ggagcgcaag aaagatcaaa tatcggagaa aactgcggaa gtacgagaca 300
   ctgaaggtgc tgtcaattaa cggaatgtgc cccctgagcg tggaggaagt cgaacagtgg 360
   aagaaatctg gcttcaagga atatcccctg aatcctgagt ttctggattg gctgcggacc 420
   aacgaggaca agaacatcaa tccttacctg ttcagagata aagctagcaa gcagaaagtg 480
   acactgtttg aactgggaag agctctgtat cacatcgcac agaggcgcgg cttcctgagt 540
   aacaggctgg accagtcagc cgagggcgtc tttgaggaac ataatcctca gatccagaac 600
   ctgattgagg acctggacag ctccaacaca attctgaatg agctgaagga atactatatc 660
   aatctgggga tcattgacga gactgaaaag tccggcttca agaaagacct ggatgagggg 720
   gaaaagaaac tgaagtcact gtacaacagc ctggtggcca tcacaaagaa aaacgctaac 780
   gatatcgaaa cttgcaagca ggagctgatc gctagactga ataagaaaga ggacctgggc 840
   aaggtcaaag ggaagatcaa agatatttct caggcaatgc tggacaggaa ctttaagact 900
   ctgggacagt atttcttttc actgtacaac aaggagcgaa tccggaacca gtataccagc 960
   cgcgaggaac actacctgga ggagttcatc attatctgtc agacccaggg gatcgaggga 1020
   attaacacaa atgagaagct gcctgagaag aagttcaccg gcctggcaaa agatctgtat 1080
   cgagccattt tctttcagcg gccactgaaa tcccagaagg gcctgatcgg gaagtgctct 1140
   ttcgagaaga acaagtctcg ctgtgccgtg agtcacccag actatgagga atttcgaatg 1200
   tggagctacc tgaacacaat caaaattggc actcagtccg agaaaaccct gcgcttcctg 1260
   acactggagg aaaagctgaa actggtgccc aagttctacc gaaagagtga tttcaacttc 1320
   gaggtgctgg ctaaggagct ggtcgaaaaa ggagcatcat tcggctacta caagtctagt 1380
   aagaaaaatg agttctttta ctggttcaac tataagccca ccgattcagt gagcgcctgc 1440
   gtggtgagcg cttctctgga gaacgcaatc ggcaaggact ggaagatcaa gactttcgaa 1500
   tatcagacta gaaacaccga gaagaatgaa gtgaccaaat ccgtcgatta caaggacctg 1560
   tggcacctgc tgtccgtggc aacatctgat acttacctgt atgactttgc catcgaaaag 1620
   ctgaaactgg agcctaaaaa cgcaaaggcc ttcagcaaga caaaactgaa gaaagacttt 1680
   gccagtctgt cactggcagc catcgctaaa attctgccat atctgaagca gggcctgctg 1740
   tactcccacg ccgtgttcat ggctaacatc gagaatattg tcgacgccga tatctggaag 1800
   gatgaggaac agcagaagtt catccagtcc aagattgtgg agctggtcga caattatatc 1860
   gtggaaaagt ctaaactgga gctgatcaat gggctgctga agatctacaa caccgaggat 1920
   aaggaaggac ggaaagtgta ctattcaaag gaggctgaaa gcctgttcga ggcagacctg 1980
   agaaagaaac tggtcccctt ctacaaggct aacatcatca tcgaggaaca cgagcaggaa 2040
   atcattttcc aggatctgtt tcctatcctg atggaccagc tgaagaaaca ggagttcatc 2100
   aaaattggca gactggataa gcagattgaa gccttcctgg agggggaaaa tgaggaagga 2160
   cagatctttt gtaaccacac agataagctg aagaaactgt accatccaag cgacatcgag 2220
   gtgtttaaga aaaagactat caaagatgag tgggggaatg aaaaggtggt cctgggatcc 2280
   ccactgacca catctatcaa gaaccccatg gcaatgagag ccctgcacca gctgaggaag 2340
   gtgctgaata cactgattgc caacgaccag atcgacgagg atactcggat ccatattgag 2400
   atggccagag aactgaacga tgctaataag cgaaaaggca tccaggactt ccagaacgag 2460
   aacaagaagt ttcgggagga agccatcaag gagatcaaga agctgtacct ggaggaatgc 2520
   cacaaagacg tgaaccccac cgaggacgat atcctgaggt atcagctgtg gctggaacag 2580
   ggaaagtgcg agatctacga ggaaggcaac aatatcagca tttgtgatat cattggcagc 2640
   aatccctcct atgacatcga gcataccatt cctcggagca tctcccagga taacagccag 2700
   atgaacaaga cactgtgtag cctgaagttc aacagatcca tcaaaaagca gaagatgcca 2760
   gtggagctgt ccaactacaa tgacattctg cccaggatcg cacactggaa aaaggaggcc 2820
   gaggaactga ctaggcagat cgaaaccatt tctcgcagca tcaagagcgc tgcaaccaag 2880
   gtggccaagg ataagaacat cagaaagagg cattacctga cactgaagcg agactatatt 2940
   ctggggaaat acgagcggtt cacttgggag gaacctaaag tgggctttaa aaactcccag 3000
   atcccagaca ctgggatcat taccaagtac gctcaggcat atctgaaatc ttacttcaaa 3060
   agggtggaga gtgtcaaggg aggagcagtg gctgagttcc ggaaactgtg gggcatccag 3120
   gaatctttta tcgatgagaa ctggtggaag cactataagg acaaagatag agacaaacat 3180
   acccaccata caatcgacgc aatcactatt gcctgtatgc ccaaggataa atacgacctg 3240
   ctggcacacg cctggaggct ggaggatgaa caggacaaaa aggccgctaa ggtgctgatc 3300
   gagcaggcca aaccatggaa aaccttcaag gaggatatcg aaaagattga gactgaaatc 3360
   ctggtgagcc attttacccc cgacaacgtc aaaaagcagt caaagagcat catcaagaat 3420
   cgcggcaaaa aggtgtacgt cctgaagaac gagctgcctg tgaacttcaa gaacaagatc 3480
   gaagggaagg attatttcaa gctgaaattt gacagcaaga ttctgtacaa aatccccaaa 3540
   aagaaagaga agcagaccga tacattctat gaggaactgc ctaaaaacta cctgaatgga 3600
   gtggaaggca aggactactt caaaatcaat actaccggca agaccttcta caaaatccca 3660
   atttttaacc agggcgacac aatccggggg agcctgcacc aggagacaac ttacggagcc 3720
   attaagctgc cagatatcga cattgaaaca aagaaacccc tgcatactga taagggaggc 3780
   ttcattctga agaaagacat caaaggcaat gagattgtgt tctttgtggt ccgcaaggaa 3840
   atctctaaaa ttagtgagaa cgatgtgcag aatatcgtcg acaacgtggt ccggaagaaa 3900
   attgagaatg ctatcgcaaa ctccctgatc acttttaaga tcgtgaagaa aaagaaagtg 3960
   gccgtcatca aagagggggt caccatttgg atgagggagc ccaacattga aaaggggatc 4020
   gagggaatcc ctattaagaa agtgcgcatc attaccaatt ctgtcaaaaa ccctatcgag 4080
   attaaggtgc acagtccact gtccaagtct cgccacaagc ataaacagaa ggtctacggc 4140
   cagaacgatg agaattatgc catggctctg tacgaactgg acgggaagcg ggagttcgaa 4200
   ctgatcaaca attttaatct ggccaagctg ctgaaacaga gtcagtcata ctatccactg 4260
   cataaagaga aggaaatcaa gggaaagaaa attctggtgc ccatcgagaa gagaaacaac 4320
   aaggatgtca tcctgaagag gggccagcag gtggtgttct acgacaaaac cgtggagaac 4380
   cccaaggata tctctgaaat cattgacttt cgcgagcgaa tctacatcat tgaagggctg 4440
   accatccaga gacagaaaga taagaaaaca tccaaagtga atgagtacgg aatcattcag 4500
   ctgcgccact tcaaggaagc tcgaaaaagt gaggaaatct caaaggataa cttcaaacct 4560
   gacggcgagt tcaagatcaa cgagaataag ccaactagga aaatgaacca taatcagttc 4620
   accgcctttg tggaggggat cgacttcaag gtcctgccta gcggaaagtt tcagaaaatt 4680
   taagaattc 4689
<210> 130
   <211> 3621
   <212> DNA
   <213> Artificial Sequence
<220>
   <221> source
   <223> /note="Description of Artificial Sequence: Synthetic polynucleotide"
<400> 130
   accggtgcca ccatgtaccc atacgatgtt ccagattacg cttcgccgaa gaaaaagcgc 60
   aaggtcgaag cgtccatgag caagaaagtc agtagacgct atgaagaaca ggcacaggag 120
   atttgtcaga ggctggggag tagaccttat tccatcgggc tggacctggg agtgggatct 180
   atcggagtgg cagtcgccgc ttacgaccca attaagaaac agccctccga tctggtgttc 240
   gtcagctcca ggatttttat cccttctacc ggcgcagccg agcggagaca gaagagagga 300
   cagaggaaca gcctgcgcca ccgagcaaat cgcctgaaat tcctgtggaa gctgctggct 360
   gaacgaaacc tgatgctgtc atatagcgag caggacgtgc cagatcctgc acggctgaga 420
   tttgaggacg ctgtggtccg agcaaaccca tacgagctgc ggctgaaggg cctgaatgaa 480
   cagctgaccc tgagcgagct ggggtatgca ctgtaccaca tcgccaatca taggggatct 540
   agttcagtgc gcacattcct ggatgaggaa aagagctccg acgataagaa actggaggaa 600
   cagcaggcta tgacagaaca gctggcaaaa gagaagggaa tttccacttt catcgaagtg 660
   ctgacagcct ttaacactaa tggcctgatc gggtacagga actccgagtc tgtgaagagt 720
   aagggcgtgc cagtccccac tcgcgacatc atttcaaatg agattgatgt gctgctgcag 780
   acccagaagc agttctatca ggaaatcctg tcagacgagt actgcgatcg gattgtcagc 840
   gcaatcctgt ttgaaaacga gaagatcgtg ccagaagccg gctgctgtcc ctatttccct 900
   gacgagaaga aactgcccag atgtcacttt ctgaatgagg aaaggcgcct gtgggaagcc 960
   attaacaatg ctaggatcaa gatgcccatg caggagggcg ctgcaaaacg ctaccagagt 1020
   gcttcattca gcgacgagca gagacacatt ctgtttcata tcgcaaggag cgggactgat 1080
   atcaccccta aactggtgca gaaggagttc ccagccctga aaacctccat cattgtgctg 1140
   cagggaaaag agaaggctat tcagaagatc gcaggcttcc gatttcgacg gctggaggaa 1200
   aaatcttttt ggaagagact gagtgaggaa cagaaggacg atttctttag cgcctggaca 1260
   aacactcctg acgataaaag actgtccaag tacctgatga aacacctgct gctgacagaa 1320
   aatgaggtgg tcgacgccct gaaaaccgtg agcctgattg gagattatgg cccaatcggc 1380
   aagaccgcaa cacagctgct gatgaaacat ctggaggatg gcctgactta caccgaagcc 1440
   ctggagcggg gaatggaaac cggcgagttc caggaactgt cagtgtggga gcagcagagc 1500
   ctgctgccct actatgggca gatcctgaca ggatctactc aggccctgat ggggaagtat 1560
   tggcactctg cttttaaaga aaagagagac agtgagggat tctttaagcc taacacaaat 1620
   agcgatgagg aaaaatacgg caggattgcc aacccagtgg tccatcagac tctgaacgaa 1680
   ctgcgcaagc tgatgaatga gctgattacc atcctgggag ctaaacctca ggagatcaca 1740
   gtggaactgg cacgagagct gaaggtcgga gctgagaaaa gagaggacat cattaagcag 1800
   cagaccaaac aggaaaagga ggctgtgctg gcatatagca agtactgcga gcccaacaat 1860
   ctggacaaaa ggtatattga aaggttccgc ctgctggagg atcaggcctt tgtgtgccct 1920
   tactgtctgg agcacattag cgtcgcagat atcgcagctg gaagggcaga cgtggatcat 1980
   atcttcccac gcgacgatac agctgacaac tcctacggga ataaggtggt cgcacaccga 2040
   cagtgtaacg atatcaaggg aaagcggacc ccctatgcag ccttcagtaa tacatcagcc 2100
   tggggcccca tcatgcatta cctggacgaa acccctggga tgtggcgcaa aagaaggaag 2160
   tttgagacaa acgaggaaga gtatgctaag tacctgcagt caaaaggctt cgtgagcagg 2220
   tttgaaagcg ataactccta tatcgcaaaa gctgcaaagg agtacctgcg ctgcctgttc 2280
   aatccaaaca atgtgactgc cgtcgggtcc ctgaagggaa tggagacatc tatcctgcgg 2340
   aaggcctgga atctgcaggg aattgacgat ctgctgggca gccggcactg gagtaaggac 2400
   gccgatacca gccccacaat gcgcaaaaac cgggacgaca atcggcacca tggcctggac 2460
   gccatcgtgg ctctgtattg ttccagatct ctggtccaga tgattaacac catgtccgag 2520
   cagggcaagc gagcagtgga aatcgaggct atgattccta tcccagggta cgcatccgaa 2580
   ccaaatctgt ctttcgaagc ccagcgggag ctgtttagaa agaaaatcct ggagttcatg 2640
   gacctgcacg cctttgtgag tatgaaaacc gacaacgatg caaatggcgc cctgctgaaa 2700
   gatactgtgt attcaattct gggagcagac acccagggag aggatctggt gttcgtggtc 2760
   aagaaaaaga ttaaggacat cggcgtgaaa atcggggatt atgaagaggt cgcatctgcc 2820
   attcgaggcc ggatcaccga caaacagcca aagtggtatc ccatggaaat gaaagataag 2880
   atcgagcagc tgcagtctaa gaacgaagcc gctctgcaga aatacaagga gagtctggtg 2940
   caggcagccg ctgtcctgga agagagtaat aggaagctga ttgagtcagg caaaaagccc 3000
   atccagctga gtgaaaaaac aatttcaaaa aaggccctgg agctggtggg cgggtactat 3060
   tacctgatta gcaacaacaa gcgcacaaag actttcgtgg tcaaggaacc ttcaaacgag 3120
   gtgaaagggt tcgcatttga cactggaagc aatctgtgcc tggactttta tcacgatgcc 3180
   cagggaaagc tgtgtggcga gatcattaga aaaatccagg ctatgaaccc ttcctataag 3240
   ccagcataca tgaaacaggg gtattctctg tacgtgagac tgtaccaggg cgacgtgtgc 3300
   gagctgaggg caagcgatct gactgaagca gagtccaacc tggccaagac cacacatgtc 3360
   cgcctgccca atgctaaacc tgggcgaacc ttcgtgatca ttatcacctt tacagagatg 3420
   gggtctggat atcagatcta cttcagcaac ctggccaagt ccaaaaaggg acaggacact 3480
   agttttaccc tgactaccat caagaattat gatgtgcgga aagtccagct gtctagtgcc 3540
   gggctggtga gatacgtcag ccctctgctg gtggacaaaa tcgagaagga tgaagtcgct 3600
   ctgtgtggag agtaagaatt c 3621
<210> 131
   <211> 3588
   <212> DNA
   <213> Artificial Sequence
<220>
   <221> source
   <223> /note="Description of Artificial Sequence: Synthetic polynucleotide"
<400> 131
   accggtgcca ccatgtaccc atacgatgtt ccagattacg cttcgccgaa gaaaaagcgc 60
   aaggtcgaag cgtccatggc aagacctgca tttcgggcac ctcggagaga acacgtcaac 120
   ggctggaccc ctgacccaca tcggattagc aaaccctttt tcatcctggt gagctggcac 180
   ctgctgtccc gggtggtcat tgacagctcc tctggatgct tcccaggcac cagccgggac 240
   cacaccgaca agtttgccga gtgggaatgt gctgtgcagc cctacaggct gagtttcgac 300
   ctggggacca actcaatcgg atggggcctg ctgaatctgg atcgccaggg aaaaccaagg 360
   gagatccgag cactggggtc ccgcatcttc agcgacggac gggatcccca ggacaaggct 420
   tctctggctg tggcacggag actggccaga cagatgaggc gccgacggga cagatatctg 480
   actagaagga ccaggctgat gggagctctg gtgcgcttcg gcctgatgcc agcagaccct 540
   gcagctagga agcgcctgga agtggccgtc gatccatacc tggcacgaga gcgggccaca 600
   agagaaaggc tggagccctt cgaaatcggg agggccctgt ttcacctgaa ccagcgccga 660
   ggatataaac ccgtgcgcac cgccacaaag cctgatgagg aagccggcaa ggtgaaagag 720
   gctgtcgaaa ggctggaggc agcaatcgct gcagccggag cacctactct gggagcttgg 780
   ttcgcatggc gaaaaacacg aggagaaact ctgcgagcac gactggctgg gaagggaaaa 840
   gaggctgcat acccattcta tcccgcacgg agaatgctgg aggccgaatt tgacactctg 900
   tgggcagagc aggccaggca ccatccagat ctgctgaccg ccgaagctcg cgagatcctg 960
   cggcacagaa tttttcatca gcggcccctg aagccacctc cagtgggaag atgcactctg 1020
   taccctgacg atgggagagc tcctagggca ctgccaagcg ctcagaggct gcgcctgttc 1080
   caggagctgg ccagcctgag agtgatccac ctggacctgt ccgaacgccc tctgacccca 1140
   gctgagcgag atcggattgt ggcatttgtc cagggcagac cccctaaagc cggaaggaag 1200
   cctggcaaag tgcagaagag cgtcccattc gaaaagctga gggggctgct ggagctgcca 1260
   ccaggcactg ggttttctct ggagagtgac aaacggcctg aactgctggg cgacgagaca 1320
   ggcgccagaa tcgcaccagc attcggacct ggatggacag ctctgcctct ggaggaacag 1380
   gacgccctgg tggaactgct gctgacagag gcagaaccag agagggcaat tgcagctctg 1440
   actgcacgat gggctctgga cgaggcaact gcagcaaagc tggctggagc aaccctgcca 1500
   gattttcacg gacgatatgg caggcgcgca gtggctgaac tgctgcctgt cctggaacgc 1560
   gagacacgag gcgacccaga tgggagagtg aggcccatcc ggctggacga ggcagtgaaa 1620
   ctgctgagag gcgggaagga tcactcagac ttcagccggg aaggagctct gctggacgca 1680
   ctgccctact atggagccgt gctggagaga catgtcgctt ttgggacagg aaaccccgca 1740
   gatcctgagg aaaagcgggt gggaagagtc gccaatccca ctgtgcacat cgctctgaac 1800
   cagctgcggc atctggtgaa tgcaattctg gccaggcacg gccgccctga ggaaatcgtg 1860
   attgagctgg cacgggacct gaaaaggtct gccgaagatc gacggagaga ggacaagcgg 1920
   caggccgata accagaaaag aaatgaggaa cgcaagcgac tgatcctgag tctgggagag 1980
   cgcccaaccc cacgaaacct gctgaagctg cggctgtggg aggaacaggg cccagtggaa 2040
   aataggcgct gcccctactc tggggagaca attagtatga gaatgctgct gagcgagcag 2100
   gtggacatcg atcacattct gccattcagc gtgtccctgg acgattccgc tgcaaacaag 2160
   gtggtctgtc tgcgggaggc caacagaatc aagcggaata gatctccctg ggaggccttc 2220
   ggccatgaca gtgagagatg ggcagggatt ctggcacgag cagaagctct gcccaagaac 2280
   aaaaggtggc gctttgctcc tgacgcactg gagaaactgg aaggagaggg aggcctgcga     2340
   gcacgacacc tgaatgatac aaggcatctg agtcgcctgg ccgtggagta tctgcggtgc     2400
   gtctgtccta aggtgcgggt gagcccaggc cgactgactg cactgctgcg acggagatgg     2460
   ggcatcgacg ccattctggc agaagcagat ggacctccac cagaagtgcc cgcagagaca     2520
   ctggaccctt ccccagctga gaagaaccga gcagaccacc ggcaccatgc cctggatgct     2580
   gtggtcatcg gctgtattga tcgctcaatg gtgcagcgag tccagctggc cgctgcaagc     2640
   gcagaaagag aggccgctgc aagggaggac aatatcaggc gcgtgctgga gggattcaaa     2700
   gaggaacctt gggatggctt tagagctgaa ctggagcgac gggcacggac catcgtggtg     2760
   agccacagac cagaacatgg gattggggga gccctgcata aggagacagc ttacgggcct     2820
   gtggaccctc cagaggaagg attcaacctg gtggtcagga aaccaatcga cggcctgtca     2880
   aaggatgaga ttaatagcgt gcgggacccc cggctgagaa gggcactgat cgatcgcctg     2940
   gccattcgcc gacgggatgc taacgaccct gctaccgcac tggccaaagc agctgaggat     3000
   ctggcagcac agccagcctc ccgcggcatc agaagggtgc gggtcctgaa gaaagaatct     3060
   aaccccatta gggtggagca cggcgggaat ccaagtggac cccgctcagg aggccctttt     3120
   cataagctgc tgctggcagg agaggtgcac catgtggacg tcgcactgcg agcagatggc     3180
   cgccgatggg tgggacactg ggtcacactg ttcgaggcac atgggggacg gggagcagac     3240
   ggagctgcag ccccacctag actgggcgat ggggaaagat ttctgatgag gctgcacaag     3300
   ggagactgcc tgaaactgga gcataagggc agagtgaggg tcatgcaggt ggtcaaactg     3360
   gaacctagtt caaatagcgt ggtcgtggtc gagccacacc aggtgaaaac cgacagatcc     3420
   aaacatgtca agatctcttg tgatcagctg cgcgctcgag gagcacggag agtgaccgtc     3480
   gatccactgg gacgggtgag agtccacgcc ccaggagcta gggtgggaat cggaggggac     3540
   gccggacgaa ccgctatgga acccgcagag gatattagct aagaattc                  3588
<210> 132
   <211> 3234
   <212> DNA
   <213> Artificial Sequence
<220>
   <221> source
   <223> /note="Description of Artificial Sequence: Synthetic polynucleotide"
<400> 132
   accggtgcca ccatgtaccc atacgatgtt ccagattacg cttcgccgaa gaaaaagcgc       60
   aaggtcgaag cgtccatggg agagaatatg attgacgaga gtctgacctt cggcattgac      120
   ctggggattg ggagttgtgg gtgggcagtg ctgaggcggc caagcgcctt cggacggaag      180
   ggcgtgatcg agggaatggg ctcctggtgc tttgacgtcc ccgagacatc taaagaacgg 240
   actcctacca accagatccg gagatccaat agactgctga ggcgcgtgat ccgacggaga 300
   aggaaccgga tggccgctat tcgccgactg ctgcacgcag caggcctgct gccatcaacc 360
   gacagcgatg ccctgaagcg gcccggacat gatccttggg aactgagggc acgaggcctg 420
   gacaagccac tgaaacccgt ggagttcgct gtggtcctgg gccacatcgc aaagcggaga 480
   gggtttaaat ccgctgcaaa gagaaaagcc acaaacatta gctccgacga taagaaaatg 540
   ctgacagccc tggaggctac tcgagaacgg ctggggagat acaggaccgt gggagaaatg 600
   ttcgccaggg accctgattt tgcttctagg cgccgaaatc gcgagggcaa gtatgatagg 660
   accacagctc gcgacgatct ggagcacgaa gtgcacgccc tgttcgcagc tcagcggaga 720
   ctgggacagg gatttgccag tccagaactg gaggaggcct tcaccgcttc agcatttcat 780
   cagaggccca tgcaggacag cgagcgcctg gtgggattct gccctttgga gcgaaccgaa 840
   aagcgggcag ccaaactgac accctctttc gagcgctttc gactgctggc ccggctgctg 900
   aacctgagaa tcactacccc agacggagag cggcccctga cagtggatga aattgctctg 960
   gtcacccggg acctgggcaa gaccgcaaaa ctgagtatca agcgggtgag aactctgatt 1020
   ggactggagg acaatcagag gttcacaact atccgccccg aggacgaaga tcgagacatt 1080
   gtggctcgga caggcggggc aatgacaggg actgccaccc tgaggaaggc actgggagag 1140
   gccctgtgga ctgatatgca ggagcgccct gaacagctgg acgctatcgt gcaggtcctg 1200
   agcttctttg aggccaacga aacaatcact gagaagctga gggaaattgg cctgactctg 1260
   gccgtgctgg acgtcctgct gaccgcactg gatgccggag tgttcgccaa gtttaaaggc 1320
   gctgcacaca tcagcaccaa agccgctagg aatctgctgc cacatctgga gcagggcagg 1380
   cgctacgatg aggcctgcac aatggcaggg tatgaccacg cagcctcccg cctgtctcac 1440
   catggccaga tcgtggcaaa gacacagttc aacgccctgg tcactgagat cggcgaatcc 1500
   attgccaatc caatcgctcg gaaggcactg atcgaggggc tgaaacagat ttgggccatg 1560
   agaaaccact gggggctgcc cggaagtatc catgtggagc tggcccggga tgtcggcaac 1620
   tcaattgaaa agcgacggga gattgaaaag cacatcgaga aaaatactgc cctgagggct 1680
   cgcgagagaa gggaggtgca tgatctgctg gacctggaag atgtcaatgg cgacaccctg 1740
   ctgcgatacc ggctgtggaa ggagcaggga ggcaaatgcc tgtatacagg gaaggccatc 1800
   cacattcggc agatcgctgc aactgacaac tccgtgcagg tcgatcatat tctgccttgg 1860
   agccggttcg gcgacgatag ttttaacaac aagaccctgt gtctggcctc tgctaatcag 1920
   cagaagaaaa ggtcaacacc atacgagtgg ctgagcggcc agactgggga tgcatggaac 1980
   gccttcgtgc agcgcatcga gacaaataag gaactgagag ggtttaagaa aaggaactat 2040
   ctgctgaaga atgctaaaga ggcagaggaa aaattcagaa gcaggaacct gaatgacacc 2100
   agatacgccg ctaggctgtt cgcagaggcc gtgaagctgc tgtatgcctt tggggagaga 2160
   caggaaaaag ggggaaaccg ccgagtgttt actcggcctg gagcactgac cgcagcactg 2220
   agacaggctt ggggagtgga gagcctgaag aaacaggatg ggaagcgcat caatgacgat 2280
   cgacaccatg ccctggatgc tctgaccgtg gctgcagtcg acgaggccga aattcagagg 2340
   ctgacaaaat cattccacga gtgggaacag cagggcctgg ggcggcctct gcggagagtg 2400
   gagccacctt gggagagctt ccgggcagac gtcgaggcta cctaccctga agtgtttgtc 2460
   gcacggccag agaggcgccg agcaagagga gaaggccatg ccgctaccat ccggcaggtg 2520
   aaggagagag aatgcacacc aattgtgttt gagagaaagg ctgtctctag tctgaaagag 2580
   gcagacctgg aacgaatcaa agatggcgag cgcaacgaag caattgtgga ggccatcagg 2640
   agctggattg ccactggacg cccagctgat gcaccaccac gctccccccg aggcgacatc 2700
   attaccaaga tcaggctggc caccaccatc aaggcagccg tgcctgtccg cggagggacc 2760
   gcaggaaggg gagaaatggt gcgcgcagat gtgttcagca agccaaaccg gagagggaaa 2820
   gacgagtggt atctggtgcc cgtgtatcca caccagatca tgaacaggaa ggcttggccc 2880
   aaacctccaa tgcgctcaat tgtggccaat aaggatgagg acgaatggac cgaagtggga 2940
   cctgaacacc agttccggtt tagcctgtac cctagatcca atatcgagat cattaggcca 3000
   tctggagaag tgatcgaagg atatttcgtc ggcctgcatc gcaacactgg cgctctgacc 3060
   atcagtgcac acaatgatcc caagagtatc cattcaggca ttgggaccaa gacactgctg 3120
   gccatttcca aataccaggt ggacagattc ggcagaaagt ctccagtgcg caaagaggtc 3180
   cgaacttggc acggggaagc ctgtatctct cccacccccc ctggataaga attc 3234
<210> 133
   <211> 3504
   <212> DNA
   <213> Artificial Sequence
<220>
   <221> source
   <223> /note="Description of Artificial Sequence: Synthetic polynucleotide"
<400> 133
   accggtgcca ccatgtaccc atacgatgtt ccagattacg cttcgccgaa gaaaaagcgc 60
   aaggtcgaag cgtccatgag agagaacggg agtgacgaaa ggcggaggaa tatggacgaa 120
   aaaatggatt acaggattgg gctggacatc ggcatcgcat cagtggggtg ggccgtcctg 180
   cagaacaatt cagacgatga gcctgtgagg attgtcgacc tgggagtgcg cattttcgat 240
   accgcagaga tcccaaagac aggcgaaagc ctggcaggac cccggagagc agctcgaacc 300
   acaaggcgcc gactgcggag aaggaaacac cgcctggacc gaatcaagtg gctgttcgag 360
   aaccaggggc tgatcaatat tgacgatttt ctgaagagat acaacatggc cggactgcca 420
   gatgtgtacc agctgcggta tgaggctctg gacagaaaac tgaccgatga ggaactggcc 480
   caggtgctgc tgcacatcgc taagcatcgg ggcttcagaa gcaccaggaa agccgagaca 540
   gcagccaagg aaaacggggc agtgctgaag gccacagacg agaatcagaa acgaatgcag 600
   gaaaagggat acaggacagt gggcgagatg atctacctgg acgaggcctt ccggactggc 660
   tgctcttgga gtgagaaagg gtacatcctg accccccgca acaaggctga aaattatcag 720
   cacacaatgc tgcgggcaat gctggtggag gaagtcaagg agattttcag ctcccagcgc 780
   cgactgggca acgaaaaagc cactgaggaa ctggaggaaa agtacctgga gatcatgacc 840
   tcccagcgct cttttgacct ggggcctgga atgcagccag atgggaagcc ctccccttat 900
   gcaatggagg gcttctctga cagagtgggg aaatgtactt ttctggggga tcagggagag 960
   ctgaggggcg ctaaggggac ctacacagcc gaatatttcg tggctctgca gaaaatcaac 1020
   cacacaaagc tggtcaatca ggacggcgag acaaggaatt tcactgagga agagcggaga 1080
   gccctgactc tgctgctgtt tacccagaaa gaggtgaagt acgctgcagt ccgcaagaaa 1140
   ctgggcctgc ctgaggacat cctgttctac aacctgaact acaagaaggc cgctactaaa 1200
   gaagagcagc agaaggagaa ccagaatacc gaaaaagcca agtttatcgg gatgccatac 1260
   tatcacgatt acaagaaatg cctggaagag agagtgaagt atctgaccga gaacgaagtc 1320
   agggacctgt ttgatgagat cggaatgatt ctgacttgtt acaaaaatga cgattcccgc 1380
   accgaacgac tggccaagct gggactggtg cccatcgaga tggaaggcct gctggcttat 1440
   actcctacca aattccagca tctgtctatg aaggcaatgc ggaacatcat tccctttctg 1500
   gagaaaggga tgacctacga caaggcttgc gaagaggcag gatatgactt caaagccgat 1560
   agcaagggga ctaaacagaa gctgctgacc ggagagaacg tgaatcagac aatcaacgaa 1620
   attactaatc ctgtggtcaa acgctcagtg agccagacag tgaaggtcat taacgccatc 1680
   attcggactt acggcagtcc acaggctatc aatattgagc tggcaagaga aatgtcaaag 1740
   acctttgaag agaggcgcaa aatcaagggg gacatggaga aacggcagaa gaacaatgaa 1800
   gatgtgaaga aacagattca ggagctggga aaactgtctc ctacaggcca ggacatcctg 1860
   aagtacagac tgtggcagga gcagcagggg atttgtatgt atagtggaaa aaccatccca 1920
   ctggaagagc tgttcaagcc cggctacgac atcgatcaca ttctgcccta ttcaattaca 1980
   ttcgacgata gctttaggaa caaagtgctg gtcacatccc aggagaacag acagaagggc 2040
   aataggactc cttacgagta tatggggaac gacgaacagc gctggaatga gtttgaaacc 2100
   agggtgaaaa ctaccatccg cgattacaag aaacagcaga agctgctgaa gaaacatttc 2160
   tctgaagagg aaaggagtga gtttaaagaa cggaacctga cagacactaa gtacatcaca 2220
   accgtgatct acaacatgat cagacagaat ctggagatgg cccccctgaa ccgccctgaa 2280
   aagaaaaagc aggtgcgggc tgtcaatggc gcaattaccg cctacctgcg aaaacggtgg 2340
   gggctgccac agaagaatcg ggagacagac acacaccatg ctatggatgc agtggtcatc 2400
   gcctgctgta ccgacggcat gatccagaaa attagtagat acacaaaggt gagagagagg 2460
   tgctattcaa agggaacaga gttcgtcgat gcagagactg gcgaaatctt tagacccgag 2520
   gactacagca gggccgagtg ggatgaaatt ttcggcgtgc acatcccaaa gccctgggag 2580
   acatttcgcg ccgaactgga cgtccgaatg ggggacgatc caaagggatt cctggacact 2640
   catagcgatg tggctctgga gctggattat cccgagtaca tctacgaaaa cctgcggcct 2700
   atcttcgtga gcagaatgcc aaatcacaag gtcaccggag cagcccatgc tgacacaatt 2760
   cggtccccaa gacactttaa agatgagggc atcgtgctga ctaagaccgc actgaccgac 2820
   ctgaaactgg acaaggatgg ggagatcgac ggatactata acccccagtc cgatctgctg 2880
   ctgtacgaag cactgaaaaa gcagctgctg ctgtatggca atgatgccaa aaaggccttc 2940
   gctcaggact ttcataaacc caaggccgat ggaactgagg gccctgtggt caggaaggtg 3000
   aagatccaga aaaagcagac catgggagtg ttcgtcgact ctggcaacgg gattgccgag 3060
   aatggcggga tggtgcgcat cgatgtgttc cgagtcaacg gcaagtacta ttttgtgccc 3120
   gtctacaccg ctgacgtggt caaaaaggtg ctgcctaata gggccagtac agctcacaag 3180
   ccatacggcg agtggaaagt gatggaggac aaggatttcc tgtttagtct gtattcacgc 3240
   gacctgatcc atatcaagtc taaaaaggat atccctatta agatggtgaa cggaggcatg 3300
   gaggggatca aggaaaccta cgcatactat attggagccg acatcagcgc tgcaaatatc 3360
   cagggcattg cccacgattc caggtataaa ttccgcggac tgggcattca gtctctggac 3420
   gtgctggaga agtgtcagat cgatgtgctg ggacatgtca gcgtggtccg atccgaaaag 3480
   cggatgggct ttagctaaga attc 3504
<210> 134
   <211> 3243
   <212> DNA
   <213> Artificial Sequence
<220>
   <221> source
   <223> /note="Description of Artificial Sequence: Synthetic polynucleotide"
<400> 134
   accggtgcca ccatgtaccc atacgatgtt ccagattacg cttcgccgaa gaaaaagcgc 60
   aaggtcgaag cgtccatgaa aaggaactac attctggggc tggacatcgg gattacaagc 120
   gtggggtatg ggattattga ctatgaaaca agggacgtga tcgacgcagg cgtcagactg 180
   ttcaaggagg ccaacgtgga aaacaatgag ggacggagaa gcaagagggg agccaggcgc 240
   ctgaaacgac ggagaaggca cagaatccag agggtgaaga aactgctgtt cgattacaac 300
   ctgctgaccg accattctga gctgagtgga attaatcctt atgaagccag ggtgaaaggc 360
   ctgagtcaga agctgtcaga ggaagagttt tccgcagctc tgctgcacct ggctaagcgc 420
   cgaggagtgc ataacgtcaa tgaggtggaa gaggacaccg gcaacgagct gtctacaaag 480
   gaacagatct cacgcaatag caaagctctg gaagagaagt atgtcgcaga gctgcagctg 540
   gaacggctga agaaagatgg cgaggtgaga gggtcaatta ataggttcaa gacaagcgac 600
   tacgtcaaag aagccaagca gctgctgaaa gtgcagaagg cttaccacca gctggatcag 660
   agcttcatcg atacttatat cgacctgctg gagactcgga gaacctacta tgagggacca 720
   ggagaaggga gccccttcgg atggaaagac atcaaggaat ggtacgagat gctgatggga 780
   cattgcacct attttccaga agagctgaga agcgtcaagt acgcttataa cgcagatctg 840
   tacaacgccc tgaatgacct gaacaacctg gtcatcacca gggatgaaaa cgagaaactg 900
   gaatactatg agaagttcca gatcatcgaa aacgtgttta agcagaagaa aaagcctaca 960
   ctgaaacaga ttgctaagga gatcctggtc aacgaagagg acatcaaggg ctaccgggtg 1020
   acaagcactg gaaaaccaga gttcaccaat ctgaaagtgt atcacgatat taaggacatc 1080
   acagcacgga aagaaatcat tgagaacgcc gaactgctgg atcagattgc taagatcctg 1140
   actatctacc agagctccga ggacatccag gaagagctga ctaacctgaa cagcgagctg 1200
   acccaggaag agatcgaaca gattagtaat ctgaaggggt acaccggaac acacaacctg 1260
   tccctgaaag ctatcaatct gattctggat gagctgtggc atacaaacga caatcagatt 1320
   gcaatcttta accggctgaa gctggtccca aaaaaggtgg acctgagtca gcagaaagag 1380
   atcccaacca cactggtgga cgatttcatt ctgtcacccg tggtcaagcg gagcttcatc 1440
   cagagcatca aagtgatcaa cgccatcatc aagaagtacg gcctgcccaa tgatatcatt 1500
   atcgagctgg ctagggagaa gaacagcaag gacgcacaga agatgatcaa tgagatgcag 1560
   aaacgaaacc ggcagaccaa tgaacgcatt gaagagatta tccgaactac cgggaaagag 1620
   aacgcaaagt acctgattga aaaaatcaag ctgcacgata tgcaggaggg aaagtgtctg 1680
   tattctctgg aggccatccc cctggaggac ctgctgaaca atccattcaa ctacgaggtc 1740
   gatcatatta tccccagaag cgtgtccttc gacaattcct ttaacaacaa ggtgctggtc 1800
   aagcaggaag agaactctaa aaagggcaat aggactcctt tccagtacct gtctagttca 1860
   gattccaaga tctcttacga aacctttaaa aagcacattc tgaatctggc caaaggaaag 1920
   ggccgcatca gcaagaccaa aaaggagtac ctgctggaag agcgggacat caacagattc 1980
   tccgtccaga aggattttat taaccggaat ctggtggaca caagatacgc tactcgcggc 2040
   ctgatgaatc tgctgcgatc ctatttccgg gtgaacaatc tggatgtgaa agtcaagtcc 2100
   atcaacggcg ggttcacatc ttttctgagg cgcaaatgga agtttaaaaa ggagcgcaac 2160
   aaagggtaca agcaccatgc cgaagatgct ctgattatcg caaatgccga cttcatcttt 2220
   aaggagtgga aaaagctgga caaagccaag aaagtgatgg agaaccagat gttcgaagag 2280
   aagcaggccg aatctatgcc cgaaatcgag acagaacagg agtacaagga gattttcatc 2340
   actcctcacc agatcaagca tatcaaggat ttcaaggact acaagtactc tcaccgggtg 2400
   gataaaaagc ccaacagaga gctgatcaat gacaccctgt atagtacaag aaaagacgat 2460
   aaggggaata ccctgattgt gaacaatctg aacggactgt acgacaaaga taatgacaag 2520
   ctgaaaaagc tgatcaacaa aagtcccgag aagctgctga tgtaccacca tgatcctcag 2580
   acatatcaga aactgaagct gattatggag cagtacggcg acgagaagaa cccactgtat 2640
   aagtactatg aagagactgg gaactacctg accaagtata gcaaaaagga taatggcccc 2700
   gtgatcaaga agatcaagta ctatgggaac aagctgaatg cccatctgga catcacagac 2760
   gattacccta acagtcgcaa caaggtggtc aagctgtcac tgaagccata cagattcgat 2820
   gtctatctgg acaacggcgt gtataaattt gtgactgtca agaatctgga tgtcatcaaa 2880
   aaggagaact actatgaagt gaatagcaag tgctacgaag aggctaaaaa gctgaaaaag 2940
   attagcaacc aggcagagtt catcgcctcc ttttacaaca acgacctgat taagatcaat 3000
   ggcgaactgt atagggtcat cggggtgaac aatgatctgc tgaaccgcat tgaagtgaat 3060
   atgattgaca tcacttaccg agagtatctg gaaaacatga atgataagcg cccccctcga 3120
   attatcaaaa caattgcctc taagactcag agtatcaaaa agtactcaac cgacattctg 3180
   ggaaacctgt atgaggtgaa gagcaaaaag caccctcaga ttatcaaaaa gggctaagaa 3240
   ttc 3243
<210> 135
   <211> 3243
   <212> DNA
   <213> Artificial Sequence
<220>
   <221> source
   <223> /note="Description of Artificial Sequence: Synthetic polynucleotide"
<400> 135
   accggtgcca ccatgtaccc atacgatgtt ccagattacg cttcgccgaa gaaaaagcgc 60
   aaggtcgaag cgtccatgaa aaggaactac attctggggc tggacatcgg gattacaagc 120
   gtggggtatg ggattattga ctatgaaaca agggacgtga tcgacgcagg cgtcagactg 180
   ttcaaggagg ccaacgtgga aaacaatgag ggacggagaa gcaagagggg agccaggcgc 240
   ctgaaacgac ggagaaggca cagaatccag agggtgaaga aactgctgtt cgattacaac 300
   ctgctgaccg accattctga gctgagtgga attaatcctt atgaagccag ggtgaaaggc 360
   ctgagtcaga agctgtcaga ggaagagttt tccgcagctc tgctgcacct ggctaagcgc 420
   cgaggagtgc ataacgtcaa tgaggtggaa gaggacaccg gcaacgagct gtctacaaag 480
   gaacagatct cacgcaatag caaagctctg gaagagaagt atgtcgcaga gctgcagctg 540
   gaacggctga agaaagatgg cgaggtgaga gggtcaatta ataggttcaa gacaagcgac 600
   tacgtcaaag aagccaagca gctgctgaaa gtgcagaagg cttaccacca gctggatcag 660
   agcttcatcg atacttatat cgacctgctg gagactcgga gaacctacta tgagggacca 720
   ggagaaggga gccccttcgg atggaaagac atcaaggaat ggtacgagat gctgatggga 780
   cattgcacct attttccaga agagctgaga agcgtcaagt acgcttataa cgcagatctg 840
   tacaacgccc tgaatgacct gaacaacctg gtcatcacca gggatgaaaa cgagaaactg 900
   gaatactatg agaagttcca gatcatcgaa aacgtgttta agcagaagaa aaagcctaca 960
   ctgaaacaga ttgctaagga gatcctggtc aacgaagagg acatcaaggg ctaccgggtg 1020
   acaagcactg gaaaaccaga gttcaccaat ctgaaagtgt atcacgatat taaggacatc 1080
   acagcacgga aagaaatcat tgagaacgcc gaactgctgg atcagattgc taagatcctg 1140
   actatctacc agagctccga ggacatccag gaagagctga ctaacctgaa cagcgagctg 1200
   acccaggaag agatcgaaca gattagtaat ctgaaggggt acaccggaac acacaacctg 1260
   tccctgaaag ctatcaatct gattctggat gagctgtggc atacaaacga caatcagatt 1320
   gcaatcttta accggctgaa gctggtccca aaaaaggtgg acctgagtca gcagaaagag 1380
   atcccaacca cactggtgga cgatttcatt ctgtcacccg tggtcaagcg gagcttcatc 1440
   cagagcatca aagtgatcaa cgccatcatc aagaagtacg gcctgcccaa tgatatcatt 1500
   atcgagctgg ctagggagaa gaacagcaag gacgcacaga agatgatcaa tgagatgcag 1560
   aaacgaaacc ggcagaccaa tgaacgcatt gaagagatta tccgaactac cgggaaagag 1620
   aacgcaaagt acctgattga aaaaatcaag ctgcacgata tgcaggaggg aaagtgtctg 1680
   tattctctgg aggccatccc cctggaggac ctgctgaaca atccattcaa ctacgaggtc 1740
   gatcatatta tccccagaag cgtgtccttc gacaattcct ttaacaacaa ggtgctggtc 1800
   aagcaggaag agaactctaa aaagggcaat aggactcctt tccagtacct gtctagttca 1860
   gattccaaga tctcttacga aacctttaaa aagcacattc tgaatctggc caaaggaaag 1920
   ggccgcatca gcaagaccaa aaaggagtac ctgctggaag agcgggacat caacagattc 1980
   tccgtccaga aggattttat taaccggaat ctggtggaca caagatacgc tactcgcggc 2040
   ctgatgaatc tgctgcgatc ctatttccgg gtgaacaatc tggatgtgaa agtcaagtcc 2100
   atcaacggcg ggttcacatc ttttctgagg cgcaaatgga agtttaaaaa ggagcgcaac 2160
   aaagggtaca agcaccatgc cgaagatgct ctgattatcg caaatgccga cttcatcttt 2220
   aaggagtgga aaaagctgga caaagccaag aaagtgatgg agaaccagat gttcgaagag 2280
   aagcaggccg aatctatgcc cgaaatcgag acagaacagg agtacaagga gattttcatc 2340
   actcctcacc agatcaagca tatcaaggat ttcaaggact acaagtactc tcaccgggtg 2400
   gataaaaagc ccaacagaga gctgatcaat gacaccctgt atagtacaag aaaagacgat 2460
   aaggggaata ccctgattgt gaacaatctg aacggactgt acgacaaaga taatgacaag 2520
   ctgaaaaagc tgatcaacaa aagtcccgag aagctgctga tgtaccacca tgatcctcag 2580
   acatatcaga aactgaagct gattatggag cagtacggcg acgagaagaa cccactgtat 2640
   aagtactatg aagagactgg gaactacctg accaagtata gcaaaaagga taatggcccc 2700
   gtgatcaaga agatcaagta ctatgggaac aagctgaatg cccatctgga catcacagac 2760
   gattacccta acagtcgcaa caaggtggtc aagctgtcac tgaagccata cagattcgat 2820
   gtctatctgg acaacggcgt gtataaattt gtgactgtca agaatctgga tgtcatcaaa 2880
   aaggagaact actatgaagt gaatagcaag tgctacgaag aggctaaaaa gctgaaaaag 2940
   attagcaacc aggcagagtt catcgcctcc ttttacaaca acgacctgat taagatcaat 3000
   ggcgaactgt atagggtcat cggggtgaac aatgatctgc tgaaccgcat tgaagtgaat 3060
   atgattgaca tcacttaccg agagtatctg gaaaacatga atgataagcg cccccctcga 3120
   attatcaaaa caattgcctc taagactcag agtatcaaaa agtactcaac cgacattctg 3180
   ggaaacctgt atgaggtgaa gagcaaaaag caccctcaga ttatcaaaaa gggctaagaa 3240
   ttc 3243
<210> 136
   <211> 3891
   <212> DNA
   <213> Artificial Sequence
<220>
   <221> source
   <223> /note="Description of Artificial Sequence: Synthetic polynucleotide"
<400> 136
   accggtgcca ccatgtaccc atacgatgtt ccagattacg cttcgccgaa gaaaaagcgc 60
   aaggtcgaag cgtccatgaa caatagcatc aaatctaaac ctgaagtgac catcgggctg 120
   gacctgggag tgggaagcgt ggggtgggca atcgtggata acgaaacaaa catcattcac 180
   catctgggct ccaggctgtt ttctcaggcc aagactgctg aggatcggag atctttccgc 240
   ggggtgaggc gcctgatccg acggagaaaa tacaagctga aacgattcgt caatctgatt 300
   tggaagtaca acagctattt cggcttcaag aacaaagagg acatcctgaa caattatcag 360
   gagcagcaga agctgcacaa taccgtgctg aacctgaaat cagaggcact gaatgccaag 420
   atcgatccta aagcactgag ctggattctg cacgactacc tgaagaacag aggccatttt 480
   tatgaggaca atagggattt caacgtgtac ccaacaaagg agctggccaa gtacttcgat 540
   aagtacgggt actacaaggg aatcattgac agcaaggagg acaatgataa caaactggag 600
   gaagagctga caaagtacaa attctccaat aagcactggc tggaagaggt gaagaaagtc 660
   ctgtctaacc agactggcct gccagaaaag tttaaagaag agtatgagtc actgttcagc 720
   tacgtgagaa attattcaga gggcccaggg agcatcaact ctgtcagtcc ctacgggatc 780
   taccatctgg acgaaaaaga gggaaaggtg gtccagaagt acaacaacat ctgggataag 840
   acaatcggaa agtgcaacat cttccctgac gagtatagag ctcccaagaa cagtcctatc 900
   gcaatgattt tcaatgaaat caacgagctg tccacaatca ggtcatacag catctacctg 960
   actggctggt tcattaatca ggagttcaag aaagcctacc tgaacaagct gctggatctg 1020
   ctgatcaaaa ccaacggaga gaagccaatt gacgcaaggc agttcaagaa actgcgcgaa 1080
   gagacaatcg ccgaaagcat tggcaaagag acactgaagg atgtggagaa tgaagagaaa 1140
   ctggaaaagg aggaccacaa gtggaaactg aagggactga agctgaatac caacggcaaa 1200
   atccagtaca acgatctgag ctccctggct aagtttgtgc acaaactgaa gcagcatctg 1260
   aaactggatt tcctgctgga ggaccagtat gcaacactgg acaagatcaa tttcctgcag 1320
   tccctgtttg tgtacctggg caagcacctg agatattcca atagggtcga ttctgccaac 1380
   ctgaaggaat tttccgactc taacaaactg ttcgagcgca tcctgcagaa acagaaggat 1440
   gggctgttca agctgtttga acagactgac aaagacgatg agaagatcct ggcccagaca 1500
   catagtctgt caactaaggc catgctgctg gctattaccc ggatgacaaa tctggacaac 1560
   gatgaggaca accagaaaaa caatgacaag ggctggaatt ttgaggccat caaaaacttc 1620
   gatcagaagt ttatcgacat caccaagaaa aacaacaacc tgagcctgaa acagaataag 1680
   cgctacctgg acgatcgatt catcaacgat gctattctgt cccctggggt gaagcgaatc 1740
   ctgcgggagg caaccaaggt ctttaatgcc attctgaaac agttctctga agagtacgac 1800
   gtgacaaagg tggtcatcga actggctcgc gagctgagcg aagagaagga actggagaac 1860
   acaaagaact acaagaaact gatcaagaaa aacggcgaca agattagtga gggcctgaaa 1920
   gcactgggga tctcagaaga tgagatcaaa gacattctga agagtcccac taaatcatac 1980
   aagtttctgc tgtggctgca gcaggaccac atcgatcctt atagcctgaa ggagatcgcc 2040
   ttcgacgata tttttaccaa aacagaaaag ttcgagatcg accatatcat tccctacagc 2100
   atttccttcg acgattctag ttcaaacaag ctgctggtgc tggctgaaag taatcaggca 2160
   aagtcaaacc agactcctta tgagttcatc agctccggaa acgcaggcat taagtgggaa 2220
   gattacgagg cctattgccg caagttcaag gatggggact ctagtctgct ggacagcacc 2280
   cagcggtcca agaaattcgc caaaatgatg aaaaccgata cctcaagcaa gtacgacatc 2340
   ggatttctgg ctcgaaatct gaacgatact cggtacgcaa ccattgtgtt ccgggacgcc 2400
   ctggaggact atgctaataa ccacctggtc gaggacaaac ccatgtttaa ggtggtctgt 2460
   atcaatgggt ccgtgacctc tttcctgcgg aagaactttg acgattcctc ttacgccaag 2520
   aaagatagag acaagaatat ccaccatgct gtggatgcaa gtatcatctc aattttcagc 2580
   aacgagacaa agactctgtt caaccagctg actcagtttg ctgactataa actgttcaag 2640
   aacaccgatg gcagctggaa gaaaatcgac cctaagacag gggtggtcac tgaagtgacc 2700
   gacgagaatt ggaagcagat tagggtgcgc aaccaggtga gcgaaatcgc caaagtcatt 2760
   gagaagtaca tccaggatag caacatcgaa agaaaggcta ggtattcccg caaaatcgag 2820
   aataagacta acatttccct gtttaatgac accgtgtact ctgccaagaa agtcggctat 2880
   gaggatcaga tcaaaagaaa gaacctgaaa accctggaca ttcacgaatc tgctaaagag 2940
   aataagaaca gtaaagtgaa gcggcagttt gtctacagaa agctggtgaa tgtcagcctg 3000
   ctgaataacg ataagctggc agacctgttc gccgaaaaag aggatatcct gatgtatagg 3060
   gccaatccat gggtcatcaa cctggctgag cagattttca atgaatacac tgagaacaag 3120
   aaaatcaagt cccagaacgt gtttgaaaaa tatatgctgg acctgaccaa agagttcccc 3180
   gagaagttca gcgagtttct ggtgaagtcc atgctgagaa acaagaccgc catcatctac 3240
   gacgataaga aaaacattgt ccatcgaatc aaacggctga agatgctgag ttcagaactg 3300
   aaagagaata agctgtctaa cgtgatcatt aggtctaaga atcagagtgg gaccaaactg 3360
   tcataccagg atacaatcaa cagcctggcc ctgatgatta tgcgcagcat cgaccctact 3420
   gctaagaaac agtatattcg agtgccactg aataccctga acctgcacct gggagatcat 3480
   gactttgatc tgcacaatat ggatgcttac ctgaagaaac caaaattcgt gaagtatctg 3540
   aaagcaaacg aaatcggcga cgagtacaag ccctggaggg tcctgacatc tggcactctg 3600
   ctgatccata agaaggataa gaaactgatg tacatcagct ccttccagaa tctgaacgac 3660
   gtgatcgaaa ttaagaatct gatcgaaacc gagtataaag agaacgacga ttctgatagt 3720
   aagaaaaaga aaaaggcaaa ccgctttctg atgaccctga gcacaatcct gaatgactac 3780
   attctgctgg acgccaagga taacttcgac atcctggggc tgtctaaaaa tcggatcgat 3840
   gagattctga acagtaagct gggactggac aagattgtga aataagaatt c 3891
<210> 137
   <211> 3093
   <212> DNA
   <213> Artificial Sequence
<220>
   <221> source
   <223> /note="Description of Artificial Sequence: Synthetic polynucleotide"
<400> 137
   accggtgcca ccatgtaccc atacgatgtt ccagattacg cttcgccgaa gaaaaagcgc 60
   aaggtcgaag cgtccatgag gattctgggg tttgacattg gcattaacag catcgggtgg 120
   gcttttgtgg agaacgacga actgaaggac tgcggagtgc ggatcttcac aaaggccgag 180
   aacccaaaaa ataaggaaag cctggcactg ccccggagaa atgcacgcag ctccaggcgc 240
   cgactgaaac ggagaaaggc ccggctgatc gctattaaga gaatcctggc caaagagctg 300
   aagctgaact acaaggacta tgtcgcagct gatggagagc tgccaaaggc ctacgaagga 360
   tccctggcat ctgtgtacga gctgcggtat aaggccctga cacagaacct ggaaactaaa 420
   gatctggcca gagtgatcct gcacattgct aagcataggg ggtacatgaa caagaacgag 480
   aagaaatcaa acgacgctaa gaaaggaaag atcctgagcg ctctgaaaaa caatgcactg 540
   aagctggaga actaccagag cgtgggcgaa tacttctaca aggagttctt tcagaaatac 600
   aagaaaaaca caaagaactt catcaagatc cgcaacacta aggataatta caacaattgc 660
   gtgctgtcta gtgacctgga aaaagagctg aagctgatcc tggaaaaaca gaaggagttc 720
   ggctacaact actctgaaga tttcatcaac gagattctga aggtcgcctt ctttcagcgg 780
   cccctgaagg acttcagtca cctggtgggg gcctgcactt tctttgagga agagaaaagg 840
   gcctgtaaga acagctactc tgcctgggag tttgtggctc tgaccaagat cattaacgag 900
   atcaagagcc tggagaagat cagcggcgaa attgtgccaa cccagacaat caacgaggtc 960
   ctgaatctga tcctggacaa ggggtctatc acctacaaga aattcagaag ttgtatcaat 1020
   ctgcatgaga gtatcagctt caagagcctg aagtatgata aagaaaacgc cgagaatgct 1080
   aaactgatcg acttccgcaa gctggtggag tttaagaaag ccctgggagt ccacagcctg 1140
   tcccggcagg aactggatca gatctccact catatcaccc tgattaagga caacgtgaag 1200
   ctgaaaaccg tcctggagaa atacaacctg agtaatgaac agatcaacaa tctgctggaa 1260
   attgagttca acgattatat caacctgagc ttcaaggccc tgggaatgat tctgccactg 1320
   atgcgcgagg gcaaacgata cgacgaggcc tgcgagatcg ccaatctgaa acctaagacc 1380
   gtggacgaga agaaagattt cctgccagca ttttgtgatt ccattttcgc ccacgagctg 1440
   tctaaccccg tggtcaatag ggctatcagc gaataccgca aggtgctgaa cgcactgctg 1500
   aagaaatatg gaaaggtcca caaaattcat ctggagctgg ctcgcgacgt gggcctgtcc 1560
   aagaaagcac gagagaagat cgaaaaagag cagaaggaaa accaggccgt gaatgcatgg 1620
   gccctgaagg aatgcgagaa tattggcctg aaggccagcg caaagaacat cctgaaactg 1680
   aagctgtgga aagaacagaa ggagatctgt atctactccg gaaataagat ctctattgag 1740
   cacctgaaag atgaaaaggc cctggaggtg gaccatatct acccctattc taggagtttc 1800
   gacgattctt ttatcaacaa agtgctggtg ttcaccaagg aaaatcagga gaaactgaac 1860
   aagacacctt tcgaggcctt tggcaagaat attgaaaaat ggagcaagat ccagaccctg 1920
   gctcagaacc tgccatacaa gaaaaagaat aagattctgg acgagaactt caaagataag 1980
   cagcaggagg actttatctc tcgaaatctg aacgacaccc ggtatatcgc tacactgatt 2040
   gcaaaataca caaaggagta tctgaacttc ctgctgctga gcgaaaatga gaacgccaat 2100
   ctgaagagtg gcgaaaaagg gtcaaagatc cacgtgcaga ctattagcgg gatgctgacc 2160
   tccgtcctga ggcacacatg ggggtttgac aaaaaggatc gcaacaatca tctgcaccat 2220
   gcactggatg ccatcattgt ggcctacagt acaaattcaa tcattaaggc tttcagcgat 2280
   ttccggaaaa accaggagct gctgaaggcc agattctacg ctaaagaact gacttccgat 2340
   aactataaac atcaggtcaa gttctttgag cctttcaaga gttttagaga aaaaatcctg 2400
   tcaaagatcg acgagatttt cgtgtccaaa ccacctcgaa agcgagctag gcgcgcactg 2460
   cacaaggata cctttcattc tgagaacaag atcattgaca agtgcagcta caactccaag 2520
   gaaggcctgc agattgccct gagctgtgga agagtgagga aaatcggcac taagtatgtc 2580
   gagaatgata ccatcgtgag ggtcgacatt ttcaaaaagc agaacaagtt ttacgctatc 2640
   ccaatctacg caatggattt tgccctgggg atcctgccca ataagatcgt gattactgga 2700
   aaagataaga acaataaccc caaacagtgg cagaccattg acgaatcata cgagttctgc 2760
   tttagcctgt ataagaatga cctgatcctg ctgcagaaaa agaacatgca ggaacctgag 2820
   ttcgcctact ataacgattt ttcaatcagc acatcaagca tttgtgtgga gaaacacgac 2880
   aacaagttcg aaaatctgac tagcaaccag aagctgctgt tttccaatgc aaaagagggc 2940
   tctgtgaagg tcgaaagtct ggggatccag aacctgaaag tgttcgagaa gtacatcatt 3000
   acccccctgg gagataaaat taaggctgac tttcagcctc gagaaaacat cagcctgaaa 3060
   accagtaaaa agtatggcct gaggtaagaa ttc 3093
<210> 138
   <211> 4104
   <212> DNA
   <213> Homo sapiens
<220>
   <221> CDS
   <222> (1)..(4104)
<400> 138
   atg gac aag aag tac agc atc ggc ctg gac atc ggc acc aac tct gtg          48
   Met Asp Lys Lys Tyr Ser Ile Gly Leu Asp Ile Gly Thr Asn Ser Val
   1               5                   10                  15
   ggc tgg gcc gtg atc acc gac gag tac aag gtg ccc agc aag aaa ttc          96
   Gly Trp Ala Val Ile Thr Asp Glu Tyr Lys Val Pro Ser Lys Lys Phe
               20                  25                  30
   aag gtg ctg ggc aac acc gac cgg cac agc atc aag aag aac ctg atc         144
   Lys Val Leu Gly Asn Thr Asp Arg His Ser Ile Lys Lys Asn Leu Ile
           35                  40                  45
   gga gcc ctg ctg ttc gac agc ggc gaa aca gcc gag gcc acc cgg ctg        192
   Gly Ala Leu Leu Phe Asp Ser Gly Glu Thr Ala Glu Ala Thr Arg Leu
       50                  55                  60
   aag aga acc gcc aga aga aga tac acc aga cgg aag aac cgg atc tgc        240
   Lys Arg Thr Ala Arg Arg Arg Tyr Thr Arg Arg Lys Asn Arg Ile Cys
   65                  70                  75                  80
   tat ctg caa gag atc ttc agc aac gag atg gcc aag gtg gac gac agc        288
   Tyr Leu Gln Glu Ile Phe Ser Asn Glu Met Ala Lys Val Asp Asp Ser
                   85                  90                  95
   ttc ttc cac aga ctg gaa gag tcc ttc ctg gtg gaa gag gat aag aag        336
   Phe Phe His Arg Leu Glu Glu Ser Phe Leu Val Glu Glu Asp Lys Lys
               100                 105                 110
   cac gag cgg cac ccc atc ttc ggc aac atc gtg gac gag gtg gcc tac        384
   His Glu Arg His Pro Ile Phe Gly Asn Ile Val Asp Glu Val Ala Tyr
           115                 120                 125
   cac gag aag tac ccc acc atc tac cac ctg aga aag aaa ctg gtg gac        432
   His Glu Lys Tyr Pro Thr Ile Tyr His Leu Arg Lys Lys Leu Val Asp
       130                 135                 140
   agc acc gac aag gcc gac ctg cgg ctg atc tat ctg gcc ctg gcc cac        480
   Ser Thr Asp Lys Ala Asp Leu Arg Leu Ile Tyr Leu Ala Leu Ala His
   145                 150                 155                 160
   atg atc aag ttc cgg ggc cac ttc ctg atc gag ggc gac ctg aac ccc        528
   Met Ile Lys Phe Arg Gly His Phe Leu Ile Glu Gly Asp Leu Asn Pro
                   165                 170                 175
   gac aac agc gac gtg gac aag ctg ttc atc cag ctg gtg cag acc tac        576
   Asp Asn Ser Asp Val Asp Lys Leu Phe Ile Gln Leu Val Gln Thr Tyr
               180                 185                 190
   aac cag ctg ttc gag gaa aac ccc atc aac gcc agc ggc gtg gac gcc        624
   Asn Gln Leu Phe Glu Glu Asn Pro Ile Asn Ala Ser Gly Val Asp Ala
           195                 200                 205
   aag gcc atc ctg tct gcc aga ctg agc aag agc aga cgg ctg gaa aat        672
   Lys Ala Ile Leu Ser Ala Arg Leu Ser Lys Ser Arg Arg Leu Glu Asn
       210                 215                 220
   ctg atc gcc cag ctg ccc ggc gag aag aag aat ggc ctg ttc ggc aac        720
   Leu Ile Ala Gln Leu Pro Gly Glu Lys Lys Asn Gly Leu Phe Gly Asn
   225                 230                 235                 240
   ctg att gcc ctg agc ctg ggc ctg acc ccc aac ttc aag agc aac ttc        768
   Leu Ile Ala Leu Ser Leu Gly Leu Thr Pro Asn Phe Lys Ser Asn Phe
                   245                 250                 255
   gac ctg gcc gag gat gcc aaa ctg cag ctg agc aag gac acc tac gac        816
   Asp Leu Ala Glu Asp Ala Lys Leu Gln Leu Ser Lys Asp Thr Tyr Asp
               260                 265                 270
   gac gac ctg gac aac ctg ctg gcc cag atc ggc gac cag tac gcc gac        864
   Asp Asp Leu Asp Asn Leu Leu Ala Gln Ile Gly Asp Gln Tyr Ala Asp
           275                 280                 285
   ctg ttt ctg gcc gcc aag aac ctg tcc gac gcc atc ctg ctg agc gac        912
   Leu Phe Leu Ala Ala Lys Asn Leu Ser Asp Ala Ile Leu Leu Ser Asp
       290                 295                 300
   atc ctg aga gtg aac acc gag atc acc aag gcc ccc ctg agc gcc tct     960
   Ile Leu Arg Val Asn Thr Glu Ile Thr Lys Ala Pro Leu Ser Ala Ser
   305                 310                 315                 320
   atg atc aag aga tac gac gag cac cac cag gac ctg acc ctg ctg aaa    1008
   Met Ile Lys Arg Tyr Asp Glu His His Gln Asp Leu Thr Leu Leu Lys
                   325                 330                 335
   gct ctc gtg cgg cag cag ctg cct gag aag tac aaa gag att ttc ttc    1056
   Ala Leu Val Arg Gln Gln Leu Pro Glu Lys Tyr Lys Glu Ile Phe Phe
               340                 345                 350
   gac cag agc aag aac ggc tac gcc ggc tac att gac ggc gga gcc agc    1104
   Asp Gln Ser Lys Asn Gly Tyr Ala Gly Tyr Ile Asp Gly Gly Ala Ser
           355                 360                 365
   cag gaa gag ttc tac aag ttc atc aag ccc atc ctg gaa aag atg gac    1152
   Gln Glu Glu Phe Tyr Lys Phe Ile Lys Pro Ile Leu Glu Lys Met Asp
       370                 375                 380
   ggc acc gag gaa ctg ctc gtg aag ctg aac aga gag gac ctg ctg cgg    1200
   Gly Thr Glu Glu Leu Leu Val Lys Leu Asn Arg Glu Asp Leu Leu Arg
   385                 390                 395                 400
   aag cag cgg acc ttc gac aac ggc agc atc ccc cac cag atc cac ctg    1248
   Lys Gln Arg Thr Phe Asp Asn Gly Ser Ile Pro His Gln Ile His Leu
                   405                 410                 415
   gga gag ctg cac gcc att ctg cgg cgg cag gaa gat ttt tac cca ttc    1296
   Gly Glu Leu His Ala Ile Leu Arg Arg Gln Glu Asp Phe Tyr Pro Phe
               420                 425                 430
   ctg aag gac aac cgg gaa aag atc gag aag atc ctg acc ttc cgc atc    1344
   Leu Lys Asp Asn Arg Glu Lys Ile Glu Lys Ile Leu Thr Phe Arg Ile
           435                 440                 445
   ccc tac tac gtg ggc cct ctg gcc agg gga aac agc aga ttc gcc tgg    1392
   Pro Tyr Tyr Val Gly Pro Leu Ala Arg Gly Asn Ser Arg Phe Ala Trp
       450                 455                 460
   atg acc aga aag agc gag gaa acc atc acc ccc tgg aac ttc gag gaa    1440
   Met Thr Arg Lys Ser Glu Glu Thr Ile Thr Pro Trp Asn Phe Glu Glu
   465                 470                 475                 480
   gtg gtg gac aag ggc gct tcc gcc cag agc ttc atc gag cgg atg acc    1488
   Val Val Asp Lys Gly Ala Ser Ala Gln Ser Phe Ile Glu Arg Met Thr
                   485                 490                 495
   aac ttc gat aag aac ctg ccc aac gag aag gtg ctg ccc aag cac agc    1536
   Asn Phe Asp Lys Asn Leu Pro Asn Glu Lys Val Leu Pro Lys His Ser
               500                 505                 510
   ctg ctg tac gag tac ttc acc gtg tat aac gag ctg acc aaa gtg aaa    1584
   Leu Leu Tyr Glu Tyr Phe Thr Val Tyr Asn Glu Leu Thr Lys Val Lys
           515                 520                 525
   tac gtg acc gag gga atg aga aag ccc gcc ttc ctg agc ggc gag cag    1632
   Tyr Val Thr Glu Gly Met Arg Lys Pro Ala Phe Leu Ser Gly Glu Gln
       530                 535                 540
   aaa aag gcc atc gtg gac ctg ctg ttc aag acc aac cgg aaa gtg acc    1680
   Lys Lys Ala Ile Val Asp Leu Leu Phe Lys Thr Asn Arg Lys Val Thr
   545                 550                 555                 560
   gtg aag cag ctg aaa gag gac tac ttc aag aaa atc gag tgc ttc gac      1728
   Val Lys Gln Leu Lys Glu Asp Tyr Phe Lys Lys Ile Glu Cys Phe Asp
                   565                 570                 575
   tcc gtg gaa atc tcc ggc gtg gaa gat cgg ttc aac gcc tcc ctg ggc      1776
   Ser Val Glu Ile Ser Gly Val Glu Asp Arg Phe Asn Ala Ser Leu Gly
               580                 585                 590
   aca tac cac gat ctg ctg aaa att atc aag gac aag gac ttc ctg gac      1824
   Thr Tyr His Asp Leu Leu Lys Ile Ile Lys Asp Lys Asp Phe Leu Asp
           595                 600                 605
   aat gag gaa aac gag gac att ctg gaa gat atc gtg ctg acc ctg aca      1872
   Asn Glu Glu Asn Glu Asp Ile Leu Glu Asp Ile Val Leu Thr Leu Thr
       610                 615                 620
   ctg ttt gag gac aga gag atg atc gag gaa cgg ctg aaa acc tat gcc      1920
   Leu Phe Glu Asp Arg Glu Met Ile Glu Glu Arg Leu Lys Thr Tyr Ala
   625                 630                 635                 640
   cac ctg ttc gac gac aaa gtg atg aag cag ctg aag cgg cgg aga tac      1968
   His Leu Phe Asp Asp Lys Val Met Lys Gln Leu Lys Arg Arg Arg Tyr
                   645                 650                 655
   acc ggc tgg ggc agg ctg agc cgg aag ctg atc aac ggc atc cgg gac      2016
   Thr Gly Trp Gly Arg Leu Ser Arg Lys Leu Ile Asn Gly Ile Arg Asp
               660                 665                 670
   aag cag tcc ggc aag aca atc ctg gat ttc ctg aag tcc gac ggc ttc      2064
   Lys Gln Ser Gly Lys Thr Ile Leu Asp Phe Leu Lys Ser Asp Gly Phe
           675                 680                 685
   gcc aac aga aac ttc atg cag ctg atc cac gac gac agc ctg acc ttt      2112
   Ala Asn Arg Asn Phe Met Gln Leu Ile His Asp Asp Ser Leu Thr Phe
       690                 695                 700
   aaa gag gac atc cag aaa gcc cag gtg tcc ggc cag ggc gat agc ctg      2160
   Lys Glu Asp Ile Gln Lys Ala Gln Val Ser Gly Gln Gly Asp Ser Leu
   705                 710                 715                 720
   cac gag cac att gcc aat ctg gcc ggc agc ccc gcc att aag aag ggc      2208
   His Glu His Ile Ala Asn Leu Ala Gly Ser Pro Ala Ile Lys Lys Gly
                   725                 730                 735
   atc ctg cag aca gtg aag gtg gtg gac gag ctc gtg aaa gtg atg ggc      2256
   Ile Leu Gln Thr Val Lys Val Val Asp Glu Leu Val Lys Val Met Gly
               740                 745                 750
   cgg cac aag ccc gag aac atc gtg atc gcc atg gcc aga gag aac cag      2304
   Arg His Lys Pro Glu Asn Ile Val Ile Ala Met Ala Arg Glu Asn Gln
           755                 760                 765
   acc acc cag aag gga cag aag aac agc cgc gag aga atg aag cgg atc      2352
   Thr Thr Gln Lys Gly Gln Lys Asn Ser Arg Glu Arg Met Lys Arg Ile
       770                 775                 780
   gaa gag ggc atc aaa gag ctg ggc agc cag atc ctg aaa gaa cac ccc      2400
   Glu Glu Gly Ile Lys Glu Leu Gly Ser Gln Ile Leu Lys Glu His Pro
   785                 790                 795                 800
   gtg gaa aac acc cag ctg cag aac gag aag ctg tac ctg tac tac ctg        2448
   Val Glu Asn Thr Gln Leu Gln Asn Glu Lys Leu Tyr Leu Tyr Tyr Leu
                   805                 810                 815
   cag aat ggg cgg gat atg tac gtg gac cag gaa ctg gac atc aac cgg        2496
   Gln Asn Gly Arg Asp Met Tyr Val Asp Gln Glu Leu Asp Ile Asn Arg
               820                 825                 830
   ctg tcc gac tac gat gtg gac gcc atc gtg cct cag agc ttt ctg aag        2544
   Leu Ser Asp Tyr Asp Val Asp Ala Ile Val Pro Gln Ser Phe Leu Lys
           835                 840                 845
   gac gac tcc atc gac gcc aag gtg ctg acc aga agc gac aag gcc cgg        2592
   Asp Asp Ser Ile Asp Ala Lys Val Leu Thr Arg Ser Asp Lys Ala Arg
       850                 855                 860
   ggc aag agc gac aac gtg ccc tcc gaa gag gtc gtg aag aag atg aag        2640
   Gly Lys Ser Asp Asn Val Pro Ser Glu Glu Val Val Lys Lys Met Lys
   865                 870                 875                 880
   aac tac tgg cgg cag ctg ctg aac gcc aag ctg att acc cag aga aag        2688
   Asn Tyr Trp Arg Gln Leu Leu Asn Ala Lys Leu Ile Thr Gln Arg Lys
                   885                 890                 895
   ttc gac aat ctg acc aag gcc gag aga ggc ggc ctg agc gaa ctg gat        2736
   Phe Asp Asn Leu Thr Lys Ala Glu Arg Gly Gly Leu Ser Glu Leu Asp
               900                 905                 910
   aag gcc ggc ttc atc aag aga cag ctg gtg gaa acc cgg cag atc aca        2784
   Lys Ala Gly Phe Ile Lys Arg Gln Leu Val Glu Thr Arg Gln Ile Thr
           915                 920                 925
   aag cac gtg gca cag atc ctg gac tcc cgg atg aac act aag tac gac        2832
   Lys His Val Ala Gln Ile Leu Asp Ser Arg Met Asn Thr Lys Tyr Asp
       930                 935                 940
   gag aat gac aag ctg atc cgg gaa gtg aaa gtg atc acc ctg aag tcc        2880
   Glu Asn Asp Lys Leu Ile Arg Glu Val Lys Val Ile Thr Leu Lys Ser
   945                 950                 955                 960
   aag ctg gtg tcc gat ttc cgg aag gat ttc cag ttt tac aaa gtg cgc        2928
   Lys Leu Val Ser Asp Phe Arg Lys Asp Phe Gln Phe Tyr Lys Val Arg
                   965                 970                 975
   gag atc aac aac tac cac cac gcc cac gcc gcc tac ctg aac gcc gtc        2976
   Glu Ile Asn Asn Tyr His His Ala His Ala Ala Tyr Leu Asn Ala Val
               980                 985                 990
   gtg gga acc gcc ctg atc aaa aag  tac cct aag ctg gaa  agc gag ttc      3024
   Val Gly Thr Ala Leu Ile Lys Lys  Tyr Pro Lys Leu Glu  Ser Glu Phe
           995                 1000                1005
   gtg tac  ggc gac tac aag gtg  tac gac gtg cgg aag  atg atc gcc        3069
   Val Tyr  Gly Asp Tyr Lys Val  Tyr Asp Val Arg Lys  Met Ile Ala
       1010                1015                1020
   aag agc  gag cag gaa atc ggc  aag gct acc gcc aag  tac ttc ttc        3114
   Lys Ser  Glu Gln Glu Ile Gly  Lys Ala Thr Ala Lys  Tyr Phe Phe
       1025                1030                1035
   tac agc  aac atc atg aac ttt  ttc aag acc gag att  acc ctg gcc        3159
   Tyr Ser  Asn Ile Met Asn Phe  Phe Lys Thr Glu Ile  Thr Leu Ala
       1040                1045                1050
   aac ggc  gag atc cgg aag cgg  cct ctg atc gag aca  aac ggc gaa        3204
   Asn Gly  Glu Ile Arg Lys Arg  Pro Leu Ile Glu Thr  Asn Gly Glu
       1055                 1060                 1065
   acc ggg  gag atc gtg tgg gat  aag ggc cgg gat ttt  gcc acc gtg        3249
   Thr Gly  Glu Ile Val Trp Asp  Lys Gly Arg Asp Phe  Ala Thr Val
       1070                 1075                 1080
   cgg aaa  gtg ctg agc atg ccc  caa gtg aat atc gtg  aaa aag acc        3294
   Arg Lys  Val Leu Ser Met Pro  Gln Val Asn Ile Val  Lys Lys Thr
       1085                 1090                 1095
   gag gtg  cag aca ggc ggc ttc  agc aaa gag tct atc  ctg ccc aag        3339
   Glu Val  Gln Thr Gly Gly Phe  Ser Lys Glu Ser Ile  Leu Pro Lys
       1100                 1105                 1110
   agg aac  agc gat aag ctg atc  gcc aga aag aag gac  tgg gac cct        3384
   Arg Asn  Ser Asp Lys Leu Ile  Ala Arg Lys Lys Asp  Trp Asp Pro
       1115                 1120                 1125
   aag aag  tac ggc ggc ttc gac  agc ccc acc gtg gcc  tat tct gtg        3429
   Lys Lys  Tyr Gly Gly Phe Asp  Ser Pro Thr Val Ala  Tyr Ser Val
       1130                 1135                 1140
   ctg gtg  gtg gcc aaa gtg gaa  aag ggc aag tcc aag  aaa ctg aag        3474
   Leu Val  Val Ala Lys Val Glu  Lys Gly Lys Ser Lys  Lys Leu Lys
       1145                 1150                 1155
   agt gtg  aaa gag ctg ctg ggg  atc acc atc atg gaa  aga agc agc        3519
   Ser Val  Lys Glu Leu Leu Gly  Ile Thr Ile Met Glu  Arg Ser Ser
       1160                 1165                 1170
   ttc gag  aag aat ccc atc gac  ttt ctg gaa gcc aag  ggc tac aaa        3564
   Phe Glu  Lys Asn Pro Ile Asp  Phe Leu Glu Ala Lys  Gly Tyr Lys
       1175                 1180                 1185
   gaa gtg  aaa aag gac ctg atc  atc aag ctg cct aag  tac tcc ctg        3609
   Glu Val  Lys Lys Asp Leu Ile  Ile Lys Leu Pro Lys  Tyr Ser Leu
       1190                 1195                 1200
   ttc gag  ctg gaa aac ggc cgg  aag aga atg ctg gcc  tct gcc ggc        3654
   Phe Glu  Leu Glu Asn Gly Arg  Lys Arg Met Leu Ala  Ser Ala Gly
       1205                 1210                 1215
   gaa ctg  cag aag gga aac gaa  ctg gcc ctg ccc tcc  aaa tat gtg        3699
   Glu Leu  Gln Lys Gly Asn Glu  Leu Ala Leu Pro Ser  Lys Tyr Val
       1220                 1225                 1230
   aac ttc  ctg tac ctg gcc agc  cac tat gag aag ctg  aag ggc tcc        3744
   Asn Phe  Leu Tyr Leu Ala Ser  His Tyr Glu Lys Leu  Lys Gly Ser
       1235                 1240                 1245
   ccc gag  gat aat gag cag aaa  cag ctg ttt gtg gaa  cag cac aag        3789
   Pro Glu  Asp Asn Glu Gln Lys  Gln Leu Phe Val Glu  Gln His Lys
       1250                 1255                 1260
   cac tac  ctg gac gag atc atc  gag cag atc agc gag  ttc tcc aag        3834
   His Tyr  Leu Asp Glu Ile Ile  Glu Gln Ile Ser Glu  Phe Ser Lys
       1265                 1270                 1275
   aga gtg  atc ctg gcc gac gct  aat ctg gac aaa gtg  ctg tcc gcc        3879
   Arg Val  Ile Leu Ala Asp Ala  Asn Leu Asp Lys Val  Leu Ser Ala
       1280                 1285                 1290
   tac aac  aag cac cgg gat aag  ccc atc aga gag cag  gcc gag aat         3924
   Tyr Asn  Lys His Arg Asp Lys  Pro Ile Arg Glu Gln  Ala Glu Asn
       1295                 1300                 1305
   atc atc  cac ctg ttt acc ctg  acc aat ctg gga gcc  cct gcc gcc         3969
   Ile Ile  His Leu Phe Thr Leu  Thr Asn Leu Gly Ala  Pro Ala Ala
       1310                 1315                 1320
   ttc aag  tac ttt gac acc acc  atc gac cgg aag agg  tac acc agc         4014
   Phe Lys  Tyr Phe Asp Thr Thr  Ile Asp Arg Lys Arg  Tyr Thr Ser
       1325                 1330                 1335
   acc aaa  gag gtg ctg gac gcc  acc ctg atc cac cag  agc atc acc         4059
   Thr Lys  Glu Val Leu Asp Ala  Thr Leu Ile His Gln  Ser Ile Thr
       1340                 1345                 1350
   ggc ctg  tac gag aca cgg atc  gac ctg tct cag ctg  gga ggc gac         4104
   Gly Leu  Tyr Glu Thr Arg Ile  Asp Leu Ser Gln Leu  Gly Gly Asp
       1355                 1360                 1365
<210> 139
   <211> 1368
   <212> PRT
   <213> Homo sapiens
<400> 139
   Met Asp Lys Lys Tyr Ser Ile Gly Leu Asp Ile Gly Thr Asn Ser Val
   1               5                   10                  15
   Gly Trp Ala Val Ile Thr Asp Glu Tyr Lys Val Pro Ser Lys Lys Phe
               20                  25                  30
   Lys Val Leu Gly Asn Thr Asp Arg His Ser Ile Lys Lys Asn Leu Ile
           35                  40                  45
   Gly Ala Leu Leu Phe Asp Ser Gly Glu Thr Ala Glu Ala Thr Arg Leu
       50                  55                  60
   Lys Arg Thr Ala Arg Arg Arg Tyr Thr Arg Arg Lys Asn Arg Ile Cys
   65                  70                  75                  80
   Tyr Leu Gln Glu Ile Phe Ser Asn Glu Met Ala Lys Val Asp Asp Ser
                   85                  90                  95
   Phe Phe His Arg Leu Glu Glu Ser Phe Leu Val Glu Glu Asp Lys Lys
               100                 105                 110
   His Glu Arg His Pro Ile Phe Gly Asn Ile Val Asp Glu Val Ala Tyr
           115                 120                 125
   His Glu Lys Tyr Pro Thr Ile Tyr His Leu Arg Lys Lys Leu Val Asp
       130                 135                 140
   Ser Thr Asp Lys Ala Asp Leu Arg Leu Ile Tyr Leu Ala Leu Ala His
   145                 150                 155                 160
   Met Ile Lys Phe Arg Gly His Phe Leu Ile Glu Gly Asp Leu Asn Pro
                   165                 170                 175
   Asp Asn Ser Asp Val Asp Lys Leu Phe Ile Gln Leu Val Gln Thr Tyr
               180                 185                 190
   Asn Gln Leu Phe Glu Glu Asn Pro Ile Asn Ala Ser Gly Val Asp Ala
           195                 200                 205
   Lys Ala Ile Leu Ser Ala Arg Leu Ser Lys Ser Arg Arg Leu Glu Asn
       210                 215                 220
   Leu Ile Ala Gln Leu Pro Gly Glu Lys Lys Asn Gly Leu Phe Gly Asn
   225                 230                 235                 240
   Leu Ile Ala Leu Ser Leu Gly Leu Thr Pro Asn Phe Lys Ser Asn Phe
                   245                 250                 255
   Asp Leu Ala Glu Asp Ala Lys Leu Gln Leu Ser Lys Asp Thr Tyr Asp
               260                 265                 270
   Asp Asp Leu Asp Asn Leu Leu Ala Gln Ile Gly Asp Gln Tyr Ala Asp
           275                 280                 285
   Leu Phe Leu Ala Ala Lys Asn Leu Ser Asp Ala Ile Leu Leu Ser Asp
       290                 295                 300
   Ile Leu Arg Val Asn Thr Glu Ile Thr Lys Ala Pro Leu Ser Ala Ser
   305                 310                 315                 320
   Met Ile Lys Arg Tyr Asp Glu His His Gln Asp Leu Thr Leu Leu Lys
                   325                 330                 335
   Ala Leu Val Arg Gln Gln Leu Pro Glu Lys Tyr Lys Glu Ile Phe Phe
               340                 345                 350
   Asp Gln Ser Lys Asn Gly Tyr Ala Gly Tyr Ile Asp Gly Gly Ala Ser
           355                 360                 365
   Gln Glu Glu Phe Tyr Lys Phe Ile Lys Pro Ile Leu Glu Lys Met Asp
       370                 375                 380
   Gly Thr Glu Glu Leu Leu Val Lys Leu Asn Arg Glu Asp Leu Leu Arg
   385 390 395 400
   Lys Gln Arg Thr Phe Asp Asn Gly Ser Ile Pro His Gln Ile His Leu
   405 410 415
   Gly Glu Leu His Ala Ile Leu Arg Arg Gln Glu Asp Phe Tyr Pro Phe
   420 425 430
   Leu Lys Asp Asn Arg Glu Lys Ile Glu Lys Ile Leu Thr Phe Arg Ile
   435 440 445
   Pro Tyr Tyr Val Gly Pro Leu Ala Arg Gly Asn Ser Arg Phe Ala Trp
   450 455 460
   Met Thr Arg Lys Ser Glu Glu Thr Ile Thr Pro Trp Asn Phe Glu Glu
   465 470 475 480
   Val Val Asp Lys Gly Ala Ser Ala Gln Ser Phe Ile Glu Arg Met Thr
   485 490 495
   Asn Phe Asp Lys Asn Leu Pro Asn Glu Lys Val Leu Pro Lys His Ser
   500 505 510
   Leu Leu Tyr Glu Tyr Phe Thr Val Tyr Asn Glu Leu Thr Lys Val Lys
   515 520 525
   Tyr Val Thr Glu Gly Met Arg Lys Pro Ala Phe Leu Ser Gly Glu Gln
   530 535 540
   Lys Lys Ala Ile Val Asp Leu Leu Phe Lys Thr Asn Arg Lys Val Thr
   545 550 555 560
   Val Lys Gln Leu Lys Glu Asp Tyr Phe Lys Lys Ile Glu Cys Phe Asp
   565 570 575
   Ser Val Glu Ile Ser Gly Val Glu Asp Arg Phe Asn Ala Ser Leu Gly
   580 585 590
   Thr Tyr His Asp Leu Leu Lys Ile Ile Lys Asp Lys Asp Phe Leu Asp
   595 600 605
   Asn Glu Glu Asn Glu Asp Ile Leu Glu Asp Ile Val Leu Thr Leu Thr
   610 615 620
   Leu Phe Glu Asp Arg Glu Met Ile Glu Glu Arg Leu Lys Thr Tyr Ala
   625 630 635 640
   His Leu Phe Asp Asp Lys Val Met Lys Gln Leu Lys Arg Arg Arg Tyr
   645 650 655
   Thr Gly Trp Gly Arg Leu Ser Arg Lys Leu Ile Asn Gly Ile Arg Asp
   660 665 670
   Lys Gln Ser Gly Lys Thr Ile Leu Asp Phe Leu Lys Ser Asp Gly Phe
   675 680 685
   Ala Asn Arg Asn Phe Met Gln Leu Ile His Asp Asp Ser Leu Thr Phe
   690 695 700
   Lys Glu Asp Ile Gln Lys Ala Gln Val Ser Gly Gln Gly Asp Ser Leu
   705 710 715 720
   His Glu His Ile Ala Asn Leu Ala Gly Ser Pro Ala Ile Lys Lys Gly
   725 730 735
   Ile Leu Gln Thr Val Lys Val Val Asp Glu Leu Val Lys Val Met Gly
   740 745 750
   Arg His Lys Pro Glu Asn Ile Val Ile Ala Met Ala Arg Glu Asn Gln
   755 760 765
   Thr Thr Gln Lys Gly Gln Lys Asn Ser Arg Glu Arg Met Lys Arg Ile
   770 775 780
   Glu Glu Gly Ile Lys Glu Leu Gly Ser Gln Ile Leu Lys Glu His Pro
   785 790 795 800
   Val Glu Asn Thr Gln Leu Gln Asn Glu Lys Leu Tyr Leu Tyr Tyr Leu
   805 810 815
   Gln Asn Gly Arg Asp Met Tyr Val Asp Gln Glu Leu Asp Ile Asn Arg
   820 825 830
   Leu Ser Asp Tyr Asp Val Asp Ala Ile Val Pro Gln Ser Phe Leu Lys
   835 840 845
   Asp Asp Ser Ile Asp Ala Lys Val Leu Thr Arg Ser Asp Lys Ala Arg
   850 855 860
   Gly Lys Ser Asp Asn Val Pro Ser Glu Glu Val Val Lys Lys Met Lys
   865 870 875 880
   Asn Tyr Trp Arg Gln Leu Leu Asn Ala Lys Leu Ile Thr Gln Arg Lys
   885 890 895
   Phe Asp Asn Leu Thr Lys Ala Glu Arg Gly Gly Leu Ser Glu Leu Asp
               900                 905                 910
   Lys Ala Gly Phe Ile Lys Arg Gln Leu Val Glu Thr Arg Gln Ile Thr
           915                 920                 925
   Lys His Val Ala Gln Ile Leu Asp Ser Arg Met Asn Thr Lys Tyr Asp
       930                 935                 940
   Glu Asn Asp Lys Leu Ile Arg Glu Val Lys Val Ile Thr Leu Lys Ser
   945                 950                 955                 960
   Lys Leu Val Ser Asp Phe Arg Lys Asp Phe Gln Phe Tyr Lys Val Arg
                   965                 970                 975
   Glu Ile Asn Asn Tyr His His Ala His Ala Ala Tyr Leu Asn Ala Val
               980                 985                 990
   Val Gly Thr Ala Leu Ile Lys Lys  Tyr Pro Lys Leu Glu  Ser Glu Phe
           995                 1000                1005
   Val Tyr  Gly Asp Tyr Lys Val  Tyr Asp Val Arg Lys  Met Ile Ala
       1010                1015                1020
   Lys Ser  Glu Gln Glu Ile Gly  Lys Ala Thr Ala Lys  Tyr Phe Phe
       1025                1030                1035
   Tyr Ser  Asn Ile Met Asn Phe  Phe Lys Thr Glu Ile  Thr Leu Ala
       1040                1045                1050
   Asn Gly  Glu Ile Arg Lys Arg  Pro Leu Ile Glu Thr  Asn Gly Glu
       1055                1060                1065
   Thr Gly  Glu Ile Val Trp Asp  Lys Gly Arg Asp Phe  Ala Thr Val
       1070                1075                1080
   Arg Lys  Val Leu Ser Met Pro  Gln Val Asn Ile Val  Lys Lys Thr
       1085                1090                1095
   Glu Val  Gln Thr Gly Gly Phe  Ser Lys Glu Ser Ile  Leu Pro Lys
       1100                1105                1110
   Arg Asn  Ser Asp Lys Leu Ile  Ala Arg Lys Lys Asp  Trp Asp Pro
       1115                1120                1125
   Lys Lys  Tyr Gly Gly Phe Asp  Ser Pro Thr Val Ala  Tyr Ser Val
       1130                1135                1140
   Leu Val Val Ala Lys Val Glu Lys Gly Lys Ser Lys Lys Leu Lys
   1145 1150 1155
   Ser Val Lys Glu Leu Leu Gly Ile Thr Ile Met Glu Arg Ser Ser
   1160 1165 1170
   Phe Glu Lys Asn Pro Ile Asp Phe Leu Glu Ala Lys Gly Tyr Lys
   1175 1180 1185
   Glu Val Lys Lys Asp Leu Ile Ile Lys Leu Pro Lys Tyr Ser Leu
   1190 1195 1200
   Phe Glu Leu Glu Asn Gly Arg Lys Arg Met Leu Ala Ser Ala Gly
   1205 1210 1215
   Glu Leu Gln Lys Gly Asn Glu Leu Ala Leu Pro Ser Lys Tyr Val
   1220 1225 1230
   Asn Phe Leu Tyr Leu Ala Ser His Tyr Glu Lys Leu Lys Gly Ser
   1235 1240 1245
   Pro Glu Asp Asn Glu Gln Lys Gln Leu Phe Val Glu Gln His Lys
   1250 1255 1260
   His Tyr Leu Asp Glu Ile Ile Glu Gln Ile Ser Glu Phe Ser Lys
   1265 1270 1275
   Arg Val Ile Leu Ala Asp Ala Asn Leu Asp Lys Val Leu Ser Ala
   1280 1285 1290
   Tyr Asn Lys His Arg Asp Lys Pro Ile Arg Glu Gln Ala Glu Asn
   1295 1300 1305
   Ile Ile His Leu Phe Thr Leu Thr Asn Leu Gly Ala Pro Ala Ala
   1310 1315 1320
   Phe Lys Tyr Phe Asp Thr Thr Ile Asp Arg Lys Arg Tyr Thr Ser
   1325 1330 1335
   Thr Lys Glu Val Leu Asp Ala Thr Leu Ile His Gln Ser Ile Thr
   1340 1345 1350
   Gly Leu Tyr Glu Thr Arg Ile Asp Leu Ser Gln Leu Gly Gly Asp
   1355 1360 1365
<210> 140
   <211> 155
   <212> RNA
   <213> Suterella wadsworthensis
<400> 140
   guuucagugc uauaggaaac uauaggaaau caccuucggg ugagcugaaa uccccuaaag 60
   cuaagauuga auccggccac uaucuauuag uagauauccg gauauucuga uauaaaaccu 120
   cauucuuuga uuagaccaaa ggaugagguu uuuuu 155
<210> 141
   <211> 107
   <212> RNA
   <213> Treponema denticola
<400> 141
   guuugagagu uggaaacaac gaguucaaau aagaauucau caaaaucguc ccuuuuggga 60
   ccgcucauug uggagcauca aggcuuaaca ugguuaagcc uuuuuuu 107
<210> 142
   <211> 99
   <212> RNA
   <213> Filifactor alocis
<400> 142
   guuugagagu aggaaacuac acgguucaaa uaaagaauuu uucuaaucgc ccaaugggcc 60
   cauauugaua uggaugaaac ucgcuuagcg aguuuuuuu 99
<210> 143
   <211> 91
   <212> RNA
   <213> Staphylococcus pseudintermedius
<400> 143
   guuuuagcac uagaaauagu uaaguuaaaa caagcuuaaa gcgucaaugu aauauuuuau 60
   uaacacccua cugugucagu gggguuuuuu u 91
<210> 144
   <211> 94
   <212> RNA
   <213> Lactobacillus johnsonii
<400> 144
   guuuuagaug gugaaaacca gauuuaaaau caagcaaugc aucuuuugau gcaaaguuuc 60
   aauauuuguc ccacguuauc gagggacuuu uuuu 94
<210> 145
   <211> 78
   <212> RNA
   <213> Eubacterium ventriosum
<400> 145
   auuuuaguac cuggaaacag aucuacuaaa acaaggcuuu augccgaaau caagagcacc 60
   gacgggugcu cuuuuuuu 78
<210> 146
   <211> 84
   <212> RNA
   <213> Streptococcus pasteurianus
<400> 146
   guuuuuguac ucgaaagagc cuacaaagau aaggcuuuau gccgaauuca agcaccccau 60
   guuuugacau gaggugcuuu uuuu 84
<210> 147
   <211> 85
   <212> RNA
   <213> Lactobacillus farciminis
<400> 147
   guuuuuguac cuuagaaaua agaucuacaa aaauaaggau uuauuccgaa uuuaccaccu 60
   auuuuaauua auaggugguu uuuuu 85
<210> 148
   <211> 100
   <212> RNA
   <213> Bacteroides coprophilus
<400> 148
   guugugauuu gcuuucauuu gaaaaauuga agcaaaucac aauaaggauu auuccguugu 60
   gaaaacaauu aaagcggucu ugcaaaaggu cgcuuuuuuu 100
<210> 149
   <211> 102
   <212> RNA
   <213> Flaviivola taffensis
<400> 149
   guugugauuc gcuuucaaug aaaauugaag cgaaucacaa uaaggauuau uccguuguga 60
   aaacauuuac uacggggcau cgaaagacug ccucguuuuu uu 102
<210> 150
   <211> 90
   <212> RNA
   <213> Flavobacterium columnare
<400> 150
   guugugguuu gauuagaaau aaucuuauca caauaaggcu auaugccgua gacgaaaguc 60
   uuuagucccg cuucggtggg acuuuuuuuu 90
<210> 151
   <211> 78
   <212> RNA
   <213> Sphaerochaeta globus
<400> 151
   guuggggaug accgcgaaag cgauuaucuc uaauaagacu uaagucgcaa aaugcucccu 60
   auuuugggag cuuuuuuu 78
<210> 152
   <211> 85
   <212> RNA
   <213> Azospirillum sp.
<400> 152
   guugcggcug gagaaaucca gccguuaaca uguucccuuc ggggagcacg aaaugcgggg 60
   cgggccacgg uccgccccuu uuuuu 85
<210> 153
   <211> 126
   <212> RNA
   <213> Gluconacetobacter diazotrophicus
<400> 153
   gccgugguuu ccgaaaggaa accacgguaa cagaauuacc guaagguuuu uucugugaag 60
   gaucaucccu cgcuugggca accaggcggg ggaaauuccu cguucgggcc aaucagcccu 120
   uuuuuu 126
<210> 154
   <211> 82
   <212> RNA
   <213> Roseburia intestinalis
<400> 154
   guuguaauuc ccugaaaagg uuauuacaau aagguaagaa accuaaaagc ucuaauccca 60
   uucuucggaa ugggauuuuu uu 82
<210> 155
   <211> 83
   <212> RNA
   <213> Staphylococcus aureus
<400> 155
   guuuuaguac ucuggaaaca gaaucuacua aaacaaggca aaaugccgug uuuaucucgu 60
   caacuuguug gcgagauuuu uuu 83
<210> 156
   <211> 94
   <212> RNA
   <213> Mycoplasma gallisepticum
<400> 156
   guuuuagcac uguacaagaa auugucgugc uaaaauaagg cgcuguuaau gcagcugccg 60
   cauccgccag agcauuuaug cucuggcuuu uuuu 94
<210> 157
   <211> 117
   <212> RNA
   <213> Campylobacter lari
<400> 157
   guuuuagucu cugaaaagag acuaaaauaa gugguuuuug gucauccacg caggguuaca 60
   aucccuuuaa aaccauuaaa auucaaauaa acuagguugu aucaacuuag uuuuuuu 117
<210> 158
   <211> 119
   <212> RNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, u, g, unknown or other
<400> 158
   nnnnnnnnnn nnnnnnnnnn acugggguuc aggaaacuga accucaguaa gcauuggcuc 60
   guuuccaaug uugauugcuc cgccggugcu ccuuauuuuu aagggcgccg gcuuuuuuu 119
<210> 159
   <211> 1084
   <212> PRT
   <213> Corynebacterium diphtheriae
<400> 159
   Met Lys Tyr His Val Gly Ile Asp Val Gly Thr Phe Ser Val Gly Leu
   1               5                   10                  15
   Ala Ala Ile Glu Val Asp Asp Ala Gly Met Pro Ile Lys Thr Leu Ser
               20                  25                  30
   Leu Val Ser His Ile His Asp Ser Gly Leu Asp Pro Asp Glu Ile Lys
           35                  40                  45
   Ser Ala Val Thr Arg Leu Ala Ser Ser Gly Ile Ala Arg Arg Thr Arg
       50                  55                  60
   Arg Leu Tyr Arg Arg Lys Arg Arg Arg Leu Gln Gln Leu Asp Lys Phe
   65                  70                  75                  80
   Ile Gln Arg Gln Gly Trp Pro Val Ile Glu Leu Glu Asp Tyr Ser Asp
                   85                  90                  95
   Pro Leu Tyr Pro Trp Lys Val Arg Ala Glu Leu Ala Ala Ser Tyr Ile
               100                 105                 110
   Ala Asp Glu Lys Glu Arg Gly Glu Lys Leu Ser Val Ala Leu Arg His
           115                 120                 125
   Ile Ala Arg His Arg Gly Trp Arg Asn Pro Tyr Ala Lys Val Ser Ser
       130                 135                 140
   Leu Tyr Leu Pro Asp Gly Pro Ser Asp Ala Phe Lys Ala Ile Arg Glu
   145                 150                 155                 160
   Glu Ile Lys Arg Ala Ser Gly Gln Pro Val Pro Glu Thr Ala Thr Val
                   165                 170                 175
   Gly Gln Met Val Thr Leu Cys Glu Leu Gly Thr Leu Lys Leu Arg Gly
               180                 185                 190
   Glu Gly Gly Val Leu Ser Ala Arg Leu Gln Gln Ser Asp Tyr Ala Arg
           195                 200                 205
   Glu Ile Gln Glu Ile Cys Arg Met Gln Glu Ile Gly Gln Glu Leu Tyr
       210                 215                 220
   Arg Lys Ile Ile Asp Val Val Phe Ala Ala Glu Ser Pro Lys Gly Ser
   225                 230                 235                 240
   Ala Ser Ser Arg Val Gly Lys Asp Pro Leu Gln Pro Gly Lys Asn Arg
                   245                 250                 255
   Ala Leu Lys Ala Ser Asp Ala Phe Gln Arg Tyr Arg Ile Ala Ala Leu
               260                 265                 270
   Ile Gly Asn Leu Arg Val Arg Val Asp Gly Glu Lys Arg Ile Leu Ser
           275                 280                 285
   Val Glu Glu Lys Asn Leu Val Phe Asp His Leu Val Asn Leu Thr Pro
       290                 295                 300
   Lys Lys Glu Pro Glu Trp Val Thr Ile Ala Glu Ile Leu Gly Ile Asp
   305                 310                 315                 320
   Arg Gly Gln Leu Ile Gly Thr Ala Thr Met Thr Asp Asp Gly Glu Arg
                   325                 330                 335
   Ala Gly Ala Arg Pro Pro Thr His Asp Thr Asn Arg Ser Ile Val Asn
               340                 345                 350
   Ser Arg Ile Ala Pro Leu Val Asp Trp Trp Lys Thr Ala Ser Ala Leu
   355 360 365
   Glu Gln His Ala Met Val Lys Ala Leu Ser Asn Ala Glu Val Asp Asp
   370 375 380
   Phe Asp Ser Pro Glu Gly Ala Lys Val Gln Ala Phe Phe Ala Asp Leu
   385 390 395 400
   Asp Asp Asp Val His Ala Lys Leu Asp Ser Leu His Leu Pro Val Gly
   405 410 415
   Arg Ala Ala Tyr Ser Glu Asp Thr Leu Val Arg Leu Thr Arg Arg Met
   420 425 430
   Leu Ser Asp Gly Val Asp Leu Tyr Thr Ala Arg Leu Gln Glu Phe Gly
   435 440 445
   Ile Glu Pro Ser Trp Thr Pro Pro Thr Pro Arg Ile Gly Glu Pro Val
   450 455 460
   Gly Asn Pro Ala Val Asp Arg Val Leu Lys Thr Val Ser Arg Trp Leu
   465 470 475 480
   Glu Ser Ala Thr Lys Thr Trp Gly Ala Pro Glu Arg Val Ile Ile Glu
   485 490 495
   His Val Arg Glu Gly Phe Val Thr Glu Lys Arg Ala Arg Glu Met Asp
   500 505 510
   Gly Asp Met Arg Arg Arg Ala Ala Arg Asn Ala Lys Leu Phe Gln Glu
   515 520 525
   Met Gln Glu Lys Leu Asn Val Gln Gly Lys Pro Ser Arg Ala Asp Leu
   530 535 540
   Trp Arg Tyr Gln Ser Val Gln Arg Gln Asn Cys Gln Cys Ala Tyr Cys
   545 550 555 560
   Gly Ser Pro Ile Thr Phe Ser Asn Ser Glu Met Asp His Ile Val Pro
   565 570 575
   Arg Ala Gly Gln Gly Ser Thr Asn Thr Arg Glu Asn Leu Val Ala Val
   580 585 590
   Cys His Arg Cys Asn Gln Ser Lys Gly Asn Thr Pro Phe Ala Ile Trp
           595                 600                 605
   Ala Lys Asn Thr Ser Ile Glu Gly Val Ser Val Lys Glu Ala Val Glu
       610                 615                 620
   Arg Thr Arg His Trp Val Thr Asp Thr Gly Met Arg Ser Thr Asp Phe
   625                 630                 635                 640
   Lys Lys Phe Thr Lys Ala Val Val Glu Arg Phe Gln Arg Ala Thr Met
                   645                 650                 655
   Asp Glu Glu Ile Asp Ala Arg Ser Met Glu Ser Val Ala Trp Met Ala
               660                 665                 670
   Asn Glu Leu Arg Ser Arg Val Ala Gln His Phe Ala Ser His Gly Thr
           675                 680                 685
   Thr Val Arg Val Tyr Arg Gly Ser Leu Thr Ala Glu Ala Arg Arg Ala
       690                 695                 700
   Ser Gly Ile Ser Gly Lys Leu Lys Phe Phe Asp Gly Val Gly Lys Ser
   705                 710                 715                 720
   Arg Leu Asp Arg Arg His His Ala Ile Asp Ala Ala Val Ile Ala Phe
                   725                 730                 735
   Thr Ser Asp Tyr Val Ala Glu Thr Leu Ala Val Arg Ser Asn Leu Lys
               740                 745                 750
   Gln Ser Gln Ala His Arg Gln Glu Ala Pro Gln Trp Arg Glu Phe Thr
           755                 760                 765
   Gly Lys Asp Ala Glu His Arg Ala Ala Trp Arg Val Trp Cys Gln Lys
       770                 775                 780
   Met Glu Lys Leu Ser Ala Leu Leu Thr Glu Asp Leu Arg Asp Asp Arg
   785                 790                 795                 800
   Val Val Val Met Ser Asn Val Arg Leu Arg Leu Gly Asn Gly Ser Ala
                   805                 810                 815
   His Lys Glu Thr Ile Gly Lys Leu Ser Lys Val Lys Leu Ser Ser Gln
               820                 825                 830
   Leu Ser Val Ser Asp Ile Asp Lys Ala Ser Ser Glu Ala Leu Trp Cys
           835                 840                 845
   Ala Leu Thr Arg Glu Pro Gly Phe Asp Pro Lys Glu Gly Leu Pro Ala
       850                 855                 860
   Asn Pro Glu Arg His Ile Arg Val Asn Gly Thr His Val Tyr Ala Gly
   865                 870                 875                 880
   Asp Asn Ile Gly Leu Phe Pro Val Ser Ala Gly Ser Ile Ala Leu Arg
                   885                 890                 895
   Gly Gly Tyr Ala Glu Leu Gly Ser Ser Phe His His Ala Arg Val Tyr
               900                 905                 910
   Lys Ile Thr Ser Gly Lys Lys Pro Ala Phe Ala Met Leu Arg Val Tyr
           915                 920                 925
   Thr Ile Asp Leu Leu Pro Tyr Arg Asn Gln Asp Leu Phe Ser Val Glu
       930                 935                 940
   Leu Lys Pro Gln Thr Met Ser Met Arg Gln Ala Glu Lys Lys Leu Arg
   945                 950                 955                 960
   Asp Ala Leu Ala Thr Gly Asn Ala Glu Tyr Leu Gly Trp Leu Val Val
                   965                 970                 975
   Asp Asp Glu Leu Val Val Asp Thr Ser Lys Ile Ala Thr Asp Gln Val
               980                 985                 990
   Lys Ala Val Glu Ala Glu Leu Gly  Thr Ile Arg Arg Trp  Arg Val Asp
           995                 1000                1005
   Gly Phe  Phe Ser Pro Ser Lys  Leu Arg Leu Arg Pro  Leu Gln Met
       1010                 1015                 1020
   Ser Lys  Glu Gly Ile Lys Lys  Glu Ser Ala Pro Glu  Leu Ser Lys
       1025                 1030                 1035
   Ile Ile  Asp Arg Pro Gly Trp  Leu Pro Ala Val Asn  Lys Leu Phe
       1040                 1045                 1050
   Ser Asp  Gly Asn Val Thr Val  Val Arg Arg Asp Ser  Leu Gly Arg
       1055                 1060                 1065
   Val Arg  Leu Glu Ser Thr Ala  His Leu Pro Val Thr  Trp Lys Val
       1070                 1075                 1080
   Gln
<210> 160
   <211> 3336
   <212> DNA
   <213> Artificial Sequence
<220>
   <221> source
   <223> /note="Description of Artificial Sequence: Synthetic polynucleotide"
<400> 160
   accggtgcca ccatgtaccc atacgatgtt ccagattacg cttcgccgaa gaaaaagcgc 60
   aaggtcgaag cgtccatgaa gtaccatgtc ggaatcgatg tcggaacctt ttctgtgggg 120
   ctggctgcta ttgaagtgga tgacgctgga atgcctatta agaccctgag tctggtgtca 180
   cacattcatg actcaggact ggatcctgac gagatcaaga gcgctgtgac caggctggca 240
   agctccggaa tcgcccggag aacaaggcgc ctgtaccgac ggaagagaag gcgcctgcag 300
   cagctggata agttcatcca gaggcagggc tggccagtga tcgagctgga agattacagc 360
   gaccccctgt atccttggaa ggtgcgcgcc gaactggccg cttcttatat tgctgacgag 420
   aaggaacggg gggagaaact gagtgtggct ctgagacaca tcgcaaggca tcgcggatgg 480
   aggaaccctt acgccaaggt gtctagtctg tatctgccag atggcccctc agacgccttc 540
   aaggctatta gggaggaaat caaacgcgct agcggccagc ctgtgccaga gactgcaacc 600
   gtcgggcaga tggtgaccct gtgcgaactg ggcacactga agctgcgagg agagggagga 660
   gtgctgagtg cacggctgca gcagtcagat tacgcccgcg agatccagga aatttgtcga 720
   atgcaggaga tcggccagga actgtatcgc aagatcattg acgtggtgtt cgcagccgag 780
   tccccaaagg gctctgcctc aagccgggtg gggaaagatc ctctgcagcc aggaaagaac 840
   agagcactga aagccagcga cgcttttcag cgataccgga ttgctgcact gatcggcaat 900
   ctgagagtca gggtggatgg ggagaagagg attctgagcg tggaggagaa gaacctggtg 960
   ttcgaccacc tggtgaatct gactccaaag aaagagcccg aatgggtgac catcgccgaa 1020
   attctgggca tcgatcgcgg gcagctgatc ggaacagcta ctatgaccga cgatggagag 1080
   cgagcaggag cccgaccccc tacacacgat actaacagaa gtattgtgaa cagccggatc 1140
   gcaccactgg tcgactggtg gaaaacagct agcgcactgg agcagcacgc catggtgaag 1200
   gcactgtcca acgccgaagt cgacgatttt gattctcccg agggagcaaa agtgcaggca 1260
   ttctttgccg atctggacga tgacgtccac gccaagctgg acagcctgca tctgcctgtg 1320
   ggacgagcag cttactccga ggacactctg gtcagactga cccgacggat gctgagtgat 1380
   ggggtggacc tgtataccgc ccggctgcag gagttcggaa ttgaacctag ctggacccca 1440
   cccacaccaa gaatcggaga gcctgtcggc aatccagccg tcgaccgggt gctgaaaaca 1500
   gtgagcagat ggctggaatc cgcaacaaag acttggggcg ccccagagag ggtcatcatt 1560
   gagcacgtgc gcgaaggctt cgtcactgag aaacgcgctc gagaaatgga tggggacatg 1620
   agaaggcgcg cagcccggaa cgccaagctg tttcaggaga tgcaggaaaa gctgaatgtg 1680
   cagggcaaac ccagtcgagc cgatctgtgg agataccagt cagtgcagag acagaactgc 1740
   cagtgtgcct attgcgggtc cccaattacc ttttctaata gtgaaatgga ccacatcgtg 1800
   cccagagcag ggcagggatc caccaacaca agggagaatc tggtcgccgt gtgccatcgc 1860
   tgtaaccagt ctaagggcaa tacacccttc gctatttggg caaaaaacac ttctatcgaa 1920
   ggggtcagtg tgaaggaggc cgtggaacgg accagacatt gggtcactga taccggcatg 1980
   agaagcactg acttcaagaa gttcaccaag gctgtggtcg agcggtttca gagagcaaca 2040
   atggatgagg aaatcgacgc cagaagcatg gaatccgtcg cctggatggc taatgagctg 2100
   aggagccgcg tggctcagca cttcgcatcc catggaacca cagtcagggt gtaccgaggc 2160
   agcctgacag cagaggctcg acgggcatct gggatcagtg gaaagctgaa attctttgat 2220
   ggcgtgggga agtccaggct ggatagaagg caccatgcta ttgacgctgc agtgatcgca 2280
   ttcacctctg actatgtggc cgaaacactg gctgtccgct caaacctgaa acagagccag 2340
   gcccaccgac aggaggctcc tcagtggaga gagttcaccg gcaaggatgc agagcatcga 2400
   gcagcttgga gagtgtggtg ccagaagatg gaaaaactga gcgccctgct gaccgaggac 2460
   ctgcgagatg accgggtggt cgtgatgtct aacgtgcgac tgcggctggg aaatggcagt 2520
   gcccacaagg aaaccattgg caaactgtca aaggtgaaac tgtcctctca gctgtcagtc 2580
   agcgatatcg acaaagcaag ttcagaggcc ctgtggtgtg ctctgaccag agagcccgga 2640
   ttcgatccta aggaaggcct gcccgctaac cctgagagac acatcagggt gaatgggaca 2700
   catgtctacg ccggggacaa tattggactg tttccagtgt cagcaggaag catcgcactg 2760
   aggggaggat acgcagagct gggcagctcc ttccaccatg ctcgcgtgta taaaattact 2820
   tccggcaaga aacccgcatt tgccatgctg agggtgtaca ccatcgatct gctgccttat 2880
   cgcaaccagg acctgtttag cgtggaactg aagccacaga caatgtccat gaggcaggct 2940
   gagaagaaac tgcgcgacgc tctggcaact gggaatgcag aatatctggg atggctggtc 3000
   gtggatgacg agctggtcgt ggatacatct aagattgcca ctgaccaggt caaagcagtg 3060
   gaggccgaac tggggactat ccgccgatgg cgggtggatg gattcttttc cccctctaaa 3120
   ctgagactga ggcctctgca gatgtccaag gaggggatca agaaagagtc cgctcccgaa 3180
   ctgtctaaaa tcattgacag accaggatgg ctgcccgccg tgaacaagct gttctctgat 3240
   ggaaatgtca ccgtcgtgcg gagagactct ctgggacgcg tgcgactgga gagtacagcc 3300
   cacctgcctg tcacttggaa ggtgcagtaa gaattc 3336
<210> 161
   <211> 175
   <212> RNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, u, g, unknown or other
<400> 161
   nnnnnnnnnn nnnnnnnnnn guuucagugc uauaggaaac uauaggaaau caccuucggg    60
   ugagcugaaa uccccuaaag cuaagauuga auccggccac uaucuauuag uagauauccg   120
   gauauucuga uauaaaaccu cauucuuuga uuagaccaaa ggaugagguu uuuuu        175
<210> 162
   <211> 1422
   <212> PRT
   <213> Sutterella wadsworthensis
<400> 162
   Met Thr Gln Ser Glu Arg Arg Phe Ser Cys Ser Ile Gly Ile Asp Met
   1               5                   10                  15
   Gly Ala Lys Tyr Thr Gly Val Phe Tyr Ala Leu Phe Asp Arg Glu Glu
               20                  25                  30
   Leu Pro Thr Asn Leu Asn Ser Lys Ala Met Thr Leu Val Met Pro Glu
           35                  40                  45
   Thr Gly Pro Arg Tyr Val Gln Ala Gln Arg Thr Ala Val Arg His Arg
       50                  55                  60
   Leu Arg Gly Gln Lys Arg Tyr Thr Leu Ala Arg Lys Leu Ala Phe Leu
   65                  70                  75                  80
   Val Val Asp Asp Met Ile Lys Lys Gln Glu Lys Arg Leu Thr Asp Glu
                   85                  90                  95
   Glu Trp Lys Arg Gly Arg Glu Ala Leu Ser Gly Leu Leu Lys Arg Arg
               100                 105                 110
   Gly Tyr Ser Arg Pro Asn Ala Asp Gly Glu Asp Leu Thr Pro Leu Glu
           115                 120                 125
   Asn Val Arg Ala Asp Val Phe Ala Ala His Pro Ala Phe Ser Thr Tyr
       130                 135                 140
   Phe Ser Glu Val Arg Ser Leu Ala Glu Gln Trp Glu Glu Phe Thr Ala
   145 150 155 160
   Asn Ile Ser Asn Val Glu Lys Phe Leu Gly Asp Pro Asn Ile Pro Ala
   165 170 175
   Asp Lys Glu Phe Ile Glu Phe Ala Val Ala Glu Gly Leu Ile Asp Lys
   180 185 190
   Thr Glu Lys Lys Ala Tyr Gln Ser Ala Leu Ser Thr Leu Arg Ala Asn
   195 200 205
   Ala Asn Val Leu Thr Gly Leu Arg Gln Met Gly His Lys Pro Arg Ser
   210 215 220
   Glu Tyr Phe Lys Ala Ile Glu Ala Asp Leu Lys Lys Asp Ser Arg Leu
   225 230 235 240
   Ala Lys Ile Asn Glu Ala Phe Gly Gly Ala Glu Arg Leu Ala Arg Leu
   245 250 255
   Leu Gly Asn Leu Ser Asn Leu Gln Leu Arg Ala Glu Arg Trp Tyr Phe
   260 265 270
   Asn Ala Pro Asp Ile Met Lys Asp Arg Gly Trp Glu Pro Asp Arg Phe
   275 280 285
   Lys Lys Thr Leu Val Arg Ala Phe Lys Phe Phe His Pro Ala Lys Asp
   290 295 300
   Gln Asn Lys Gln His Leu Glu Leu Ile Lys Gln Ile Glu Asn Ser Glu
   305 310 315 320
   Asp Ile Ile Glu Thr Leu Cys Thr Leu Asp Pro Asn Arg Thr Ile Pro
   325 330 335
   Pro Tyr Glu Asp Gln Asn Asn Arg Arg Pro Pro Leu Asp Gln Thr Leu
   340 345 350
   Leu Leu Ser Pro Glu Lys Leu Thr Arg Gln Tyr Gly Glu Ile Trp Lys
   355 360 365
   Thr Trp Ser Ala Arg Leu Thr Ser Ala Glu Pro Thr Leu Ala Pro Ala
   370 375 380
   Ala Glu Ile Leu Glu Arg Ser Thr Asp Arg Lys Ser Arg Val Ala Val
   385 390 395 400
   Asn Gly His Glu Pro Leu Pro Thr Leu Ala Tyr Gln Leu Ser Tyr Ala
   405 410 415
   Leu Gln Arg Ala Phe Asp Arg Ser Lys Ala Leu Asp Pro Tyr Ala Leu
   420 425 430
   Arg Ala Leu Ala Ala Gly Ser Lys Ser Asn Lys Leu Thr Ser Ala Arg
   435 440 445
   Thr Ala Leu Glu Asn Cys Ile Gly Gly Gln Asn Val Lys Thr Phe Leu
   450 455 460
   Asp Cys Ala Arg Arg Tyr Tyr Arg Glu Ala Asp Asp Ala Lys Val Gly
   465 470 475 480
   Leu Trp Phe Asp Asn Ala Asp Gly Leu Leu Glu Arg Ser Asp Leu His
   485 490 495
   Pro Pro Met Lys Lys Lys Ile Leu Pro Leu Leu Val Ala Asn Ile Leu
   500 505 510
   Gln Thr Asp Glu Thr Thr Gly Gln Lys Phe Leu Asp Glu Ile Trp Arg
   515 520 525
   Lys Gln Ile Lys Gly Arg Glu Thr Val Ala Ser Arg Cys Ala Arg Ile
   530 535 540
   Glu Thr Val Arg Lys Ser Phe Gly Gly Gly Phe Asn Ile Ala Tyr Asn
   545 550 555 560
   Thr Ala Gln Tyr Arg Glu Val Asn Lys Leu Pro Arg Asn Ala Gln Asp
   565 570 575
   Lys Glu Leu Leu Thr Ile Arg Asp Arg Val Ala Glu Thr Ala Asp Phe
   580 585 590
   Ile Ala Ala Asn Leu Gly Leu Ser Asp Glu Gln Lys Arg Lys Phe Ala
   595 600 605
   Asn Pro Phe Ser Leu Ala Gln Phe Tyr Thr Leu Ile Glu Thr Glu Val
   610 615 620
   Ser Gly Phe Ser Ala Thr Thr Leu Ala Val His Leu Glu Asn Ala Trp
   625 630 635 640
   Arg Met Thr Ile Lys Asp Ala Val Ile Asn Gly Glu Thr Val Arg Ala
                   645                 650                 655
   Ala Gln Cys Ser Arg Leu Pro Ala Glu Thr Ala Arg Pro Phe Asp Gly
               660                 665                 670
   Leu Val Arg Arg Leu Val Asp Arg Gln Ala Trp Glu Ile Ala Lys Arg
           675                 680                 685
   Val Ser Thr Asp Ile Gln Ser Lys Val Asp Phe Ser Asn Gly Ile Val
       690                 695                 700
   Asp Val Ser Ile Phe Val Glu Glu Asn Lys Phe Glu Phe Ser Ala Ser
   705                 710                 715                 720
   Val Ala Asp Leu Lys Lys Asn Lys Arg Val Lys Asp Lys Met Leu Ser
                   725                 730                 735
   Glu Ala Glu Lys Leu Glu Thr Arg Trp Leu Ile Lys Asn Glu Arg Ile
               740                 745                 750
   Lys Lys Ala Ser Arg Gly Thr Cys Pro Tyr Thr Gly Asp Arg Leu Ala
           755                 760                 765
   Glu Gly Gly Glu Ile Asp His Ile Leu Pro Arg Ser Leu Ile Lys Asp
       770                 775                 780
   Ala Arg Gly Ile Val Phe Asn Ala Glu Pro Asn Leu Ile Tyr Ala Ser
   785                 790                 795                 800
   Ser Arg Gly Asn Gln Leu Lys Lys Asn Gln Arg Tyr Ser Leu Ser Asp
                   805                 810                 815
   Leu Lys Ala Asn Tyr Arg Asn Glu Ile Phe Lys Thr Ser Asn Ile Ala
               820                 825                 830
   Ala Ile Thr Ala Glu Ile Glu Asp Val Val Thr Lys Leu Gln Gln Thr
           835                 840                 845
   His Arg Leu Lys Phe Phe Asp Leu Leu Asn Glu His Glu Gln Asp Cys
       850                 855                 860
   Val Arg His Ala Leu Phe Leu Asp Asp Gly Ser Glu Ala Arg Asp Ala
   865                 870                 875                 880
   Val Leu Glu Leu Leu Ala Thr Gln Arg Arg Thr Arg Val Asn Gly Thr
                   885                 890                 895
   Gln Ile Trp Met Ile Lys Asn Leu Ala Asn Lys Ile Arg Glu Glu Leu
   900 905 910
   Gln Asn Trp Cys Lys Thr Thr Asn Asn Arg Leu His Phe Gln Ala Ala
   915 920 925
   Ala Thr Asn Val Ser Asp Ala Lys Asn Leu Arg Leu Lys Leu Ala Gln
   930 935 940
   Asn Gln Pro Asp Phe Glu Lys Pro Asp Ile Gln Pro Ile Ala Ser His
   945 950 955 960
   Ser Ile Asp Ala Leu Cys Ser Phe Ala Val Gly Ser Ala Asp Ala Glu
   965 970 975
   Arg Asp Gln Asn Gly Phe Asp Tyr Leu Asp Gly Lys Thr Val Leu Gly
   980 985 990
   Leu Tyr Pro Gln Ser Cys Glu Val Ile His Leu Gln Ala Lys Pro Gln
   995 1000 1005
   Glu Glu Lys Ser His Phe Asp Ser Val Ala Ile Phe Lys Glu Gly
   1010 1015 1020
   Ile Tyr Ala Glu Gln Phe Leu Pro Ile Phe Thr Leu Asn Glu Lys
   1025 1030 1035
   Ile Trp Ile Gly Tyr Glu Thr Leu Asn Ala Lys Gly Glu Arg Cys
   1040 1045 1050
   Gly Ala Ile Glu Val Ser Gly Lys Gln Pro Lys Glu Leu Leu Glu
   1055 1060 1065
   Met Leu Ala Pro Phe Phe Asn Lys Pro Val Gly Asp Leu Ser Ala
   1070 1075 1080
   His Ala Thr Tyr Arg Ile Leu Lys Lys Pro Ala Tyr Glu Phe Leu
   1085 1090 1095
   Ala Lys Ala Ala Leu Gln Pro Leu Ser Ala Glu Glu Lys Arg Leu
   1100 1105 1110
   Ala Ala Leu Leu Asp Ala Leu Arg Tyr Cys Thr Ser Arg Lys Ser
   1115 1120 1125
   Leu Met Ser Leu Phe Met Ala Ala Asn Gly Lys Ser Leu Lys Lys
   1130 1135 1140
   Arg Glu Asp Val Leu Lys Pro Lys Leu Phe Gln Leu Lys Val Glu
   1145 1150 1155
   Leu Lys Gly Glu Lys Ser Phe Lys Leu Asn Gly Ser Leu Thr Leu
   1160 1165 1170
   Pro Val Lys Gln Asp Trp Leu Arg Ile Cys Asp Ser Pro Glu Leu
   1175 1180 1185
   Ala Asp Ala Phe Gly Lys Pro Cys Ser Ala Asp Glu Leu Thr Ser
   1190 1195 1200
   Lys Leu Ala Arg Ile Trp Lys Arg Pro Val Met Arg Asp Leu Ala
   1205 1210 1215
   His Ala Pro Val Arg Arg Glu Phe Ser Leu Pro Ala Ile Asp Asn
   1220 1225 1230
   Pro Ser Gly Gly Phe Arg Ile Arg Arg Thr Asn Leu Phe Gly Asn
   1235 1240 1245
   Glu Leu Tyr Gln Val His Ala Ile Asn Ala Lys Lys Tyr Arg Gly
   1250 1255 1260
   Phe Ala Ser Ala Gly Ser Asn Val Asp Trp Ser Lys Gly Ile Leu
   1265 1270 1275
   Phe Asn Glu Leu Gln His Glu Asn Leu Thr Glu Cys Gly Gly Arg
   1280 1285 1290
   Phe Ile Thr Ser Ala Asp Val Thr Pro Met Ser Glu Trp Arg Lys
   1295 1300 1305
   Val Val Ala Glu Asp Asn Leu Ser Ile Trp Ile Ala Pro Gly Thr
   1310 1315 1320
   Glu Gly Arg Arg Tyr Val Arg Val Glu Thr Thr Phe Ile Gln Ala
   1325 1330 1335
   Ser His Trp Phe Glu Gln Ser Val Glu Asn Trp Ala Ile Thr Ser
   1340 1345 1350
   Pro Leu Ser Leu Pro Ala Ser Phe Lys Val Asp Lys Pro Ala Glu
   1355 1360 1365
   Phe Gln Lys Ala Val Gly Thr Glu Leu Ser Glu Leu Leu Gly Gln
   1370 1375 1380
   Pro Arg Ser Glu Ile Phe Ile Glu Asn Val Gly Asn Ala Lys His
   1385 1390 1395
   Ile Arg Phe Trp Tyr Ile Val Val Ser Ser Asn Lys Lys Met Asn
   1400 1405 1410
   Glu Ser Tyr Asn Asn Val Ser Lys Ser
   1415 1420
<210> 163
   <211> 4350
   <212> DNA
   <213> Artificial Sequence
<220>
   <221> source
   <223> /note="Description of Artificial Sequence: Synthetic polynucleotide"
<400> 163
   accggtgcca ccatgtaccc atacgatgtt ccagattacg cttcgccgaa gaaaaagcgc 60
   aaggtcgaag cgtccatgac tcagagcgag cgacgatttt cttgcagcat tggcattgac 120
   atgggggcta agtacactgg ggtgttctac gcactgttcg accgggagga actgcccaca 180
   aacctgaaca gcaaggccat gaccctggtc atgcctgaga cagggccaag atacgtgcag 240
   gcacagagaa ctgccgtcag acacaggctg cgcggacaga agagatatac cctggctagg 300
   aaactggcat ttctggtggt cgacgatatg atcaagaaac aggaaaagag gctgactgat 360
   gaggaatgga aacgaggacg ggaggccctg tccggcctgc tgaagcggag agggtactct 420
   cggcccaacg ctgacggcga agatctgacc cctctggaga atgtgagagc agacgtgttc 480
   gccgctcatc ctgccttcag cacatatttt tccgaagtgc gctctctggc tgagcagtgg 540
   gaggagttca ccgcaaacat cagcaatgtc gagaagtttc tgggcgaccc aaacatcccc 600
   gccgataaag agttcattga atttgccgtg gctgaagggc tgattgacaa gaccgagaag 660
   aaagcctacc agtcagctct gagcaccctg agggcaaacg ccaatgtgct gacaggactg 720
   cggcagatgg gccacaagcc tagatcagaa tattttaaag caatcgaggc cgacctgaag 780
   aaagatagcc gcctggccaa gattaacgaa gcattcggag gagcagagcg cctggctcga 840
   ctgctgggaa acctgtccaa tctgcagctg cgggcagaaa gatggtactt caatgccccc 900
   gacatcatga aggatagggg ctgggagcct gatcgcttca agaaaacact ggtgcgggct 960
   tttaagttct ttcacccagc aaaggaccag aacaaacagc atctggaact gatcaaacag 1020
   attgagaaca gcgaagatat cattgagact ctgtgcaccc tggacccaaa cagaaccatc 1080
   cccccttacg aggatcagaa caataggcgc ccacccctgg accagactct gctgctgagt 1140
   cccgaaaagc tgacccggca gtatggcgag atctggaaaa catggagcgc cagactgacc 1200
   tccgctgaac ccacactggc acctgcagcc gagattctgg aaagatctac cgacaggaag 1260
   agtcgcgtgg cagtcaacgg acacgagcca ctgcctacac tggcttacca gctgagttat 1320
   gcactgcaga gagccttcga caggtcaaaa gccctggatc catatgctct gagggcactg 1380
   gctgcaggct caaaaagcaa taagctgaca tccgcccgca ctgctctgga gaactgcatc 1440
   ggaggccaga atgtgaaaac cttcctggac tgtgcccgac ggtactatcg ggaagcagac 1500
   gatgccaaag tcgggctgtg gttcgacaac gccgatggac tgctggagag atctgacctg 1560
   catcctccaa tgaagaaaaa gatcctgccc ctgctggtgg ccaatattct gcagacagat 1620
   gaaaccacag gccagaagtt tctggacgag atctggcgaa aacagattaa ggggcgggaa 1680
   actgtggcta gccgatgtgc acggatcgag acagtgcgga aatccttcgg gggaggcttt 1740
   aacattgcct acaataccgc tcagtatagg gaggtgaaca agctgccccg caatgcccag 1800
   gataaagaac tgctgacaat cagagatagg gtggctgaga ctgcagactt cattgccgct 1860
   aacctggggc tgtctgacga gcagaaaaga aagttcgcca atccttttag tctggctcag 1920
   ttctacaccc tgatcgagac agaagtgtcc ggatttctgg caactaccct ggccgtccac 1980
   ctggagaacg cctggaggat gacaatcaag gatgctgtga ttaatgggga aactgtcaga 2040
   gcagcacagt gcagcaggct gcctgcagag acagctcgcc cattcgatgg actggtgaga 2100
   aggctggtcg acagacaggc ttgggagatc gcaaagaggg tgtcaactga cattcagagc 2160
   aaagtcgatt tctccaacgg catcgtggac gtcagcattt ttgtggagga aaataagttc 2220
   gagttttccg catctgtggc cgatctgaaa aagaacaaac gggtcaaaga caagatgctg 2280
   tccgaggccg aaaagctgga aaccagatgg ctgatcaaaa atgagcggat caagaaggcc 2340
   agccggggaa cttgtcccta caccggcgat aggctggctg agggggagaa aatcgaccac 2400
   attctgcccc gaagcctgat caaggatgcc cggggaattg tgtttaacgc tgagcctaat 2460
   ctgatctatg caagctcccg cggcaaccag ctgaaaaaga atcagcgata cagtctgtca 2520
   gatctgaagg ccaactatcg gaatgagatc ttcaaaacta gcaacatcgc tgcaattacc 2580
   gccgagattg aggacgtggt cactaagctg cagcagaccc atagactgaa attctttgat 2640
   ctgctgaatg agcacgaaca ggactgcgtg cggcacgccc tgttcctgga cgatggcagc 2700
   gaagctcgcg acgcagtgct ggagctgctg gcaacacagc gccgaactcg cgtcaacggg 2760
   acacagatct ggatgattaa gaacctggcc aacaagatcc gagaggaact gcagaattgg 2820
   tgtaagacaa ctaacaatag actgcacttt caggccgctg caactaacgt gtccgatgca 2880
   aagaatctga ggctgaaact ggcccagaac cagcccgact tcgagaagcc agatatccag 2940
   cccattgcca gccattccat cgacgccctg tgctctttcg ctgtggggag tgctgacgca 3000
   gaacgcgatc agaatggatt tgactacctg gatggcaaga ccgtgctggg actgtatcca 3060
   cagagctgtg aggtcattca cctgcaggcc aagccccagg aggaaaaaag tcatttcgat 3120
   tcagtggcta tctttaagga aggcatctac gcagagcagt tcctgcctat ctttaccctg 3180
   aacgaaaaga tctggattgg atatgagaca ctgaatgcca aaggcgaaag atgcggggct 3240
   attgaggtga gtggcaaaca gccaaaggag ctgctggaaa tgctggcccc cttctttaac 3300
   aagcctgtgg gcgacctgtc agcccacgct acttaccgga tcctgaaaaa gcctgcatat 3360
   gagtttctgg caaaggcagc tctgcagcca ctgagcgcag aggaaaaaag actggcagcc 3420
   ctgctggatg ctctgcgcta ctgtaccagt cgaaagtcac tgatgagcct gttcatggct 3480
   gcaaacggga aatccctgaa aaagcgggag gacgtgctga aacccaagct gttccagctg 3540
   aaggtcgagc tgaaaggcga aaagagcttc aagctgaacg ggagcctgac cctgcctgtg 3600
   aagcaggact ggctgagaat ctgcgatagc ccagaactgg cagacgcctt tggcaaaccc 3660
   tgttccgccg atgagctgac atctaagctg gctcgcattt ggaaacgacc tgtgatgcgg 3720
   gatctggctc atgcaccagt ccggagagag ttcagcctgc ccgcaatcga caacccaagt 3780
   ggagggttca ggattaggcg caccaacctg tttggcaatg agctgtacca ggtgcacgcc 3840
   atcaacgcta aaaagtatcg cggcttcgcc tccgctgggt ctaatgtcga ctggtccaag 3900
   gggatcctgt ttaacgagct gcagcatgaa aatctgaccg agtgcggagg caggttcatt 3960
   acaagcgccg atgtgactcc tatgtccgaa tggcgcaagg tggtcgcaga ggacaacctg 4020
   tctatctgga ttgctccagg gacagaagga cgacggtacg tgagggtcga gacaacattc 4080
   atccaggcca gtcactggtt tgaacagtca gtggagaatt gggccattac tagtcctctg 4140
   tcactgccag cttccttcaa ggtggacaaa ccagctgagt ttcagaaggc agtcggaacc 4200
   gagctgtcag aactgctggg ccagcccagg agcgaaatct tcattgagaa cgtgggcaat 4260
   gccaagcata tccgcttttg gtacattgtg gtgagcagca acaaaaagat gaacgagtct 4320
   tacaacaatg tgtctaagag ttaagaattc 4350
<210> 164
   <211> 112
   <212> RNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, u, g, unknown or other
<400> 164
   nnnnnnnnnn nnnnnnnnnn guuucagugg ugaaaacccu gaaaucaaca aaauuaaaga 60
   uugaaucguu uuucuaugcu cgucuuaaua gcgagcauau aacgauuuuu uu 112
<210> 165
   <211> 1372
   <212> PRT
   <213> Legionella pneumophila
<400> 165
   Met Glu Ser Ser Gln Ile Leu Ser Pro Ile Gly Ile Asp Leu Gly Gly
   1               5                   10                  15
   Lys Phe Thr Gly Val Cys Leu Ser His Leu Glu Ala Phe Ala Glu Leu
               20                  25                  30
   Pro Asn His Ala Asn Thr Lys Tyr Ser Val Ile Leu Ile Asp His Asn
           35                  40                  45
   Asn Phe Gln Leu Ser Gln Ala Gln Arg Arg Ala Thr Arg His Arg Val
       50                  55                  60
   Arg Asn Lys Lys Arg Asn Gln Phe Val Lys Arg Val Ala Leu Gln Leu
   65                  70                  75                  80
   Phe Gln His Ile Leu Ser Arg Asp Leu Asn Ala Lys Glu Glu Thr Ala
                   85                  90                  95
   Leu Cys His Tyr Leu Asn Asn Arg Gly Tyr Thr Tyr Val Asp Thr Asp
               100                 105                 110
   Leu Asp Glu Tyr Ile Lys Asp Glu Thr Thr Ile Asn Leu Leu Lys Glu
           115                 120                 125
   Leu Leu Pro Ser Glu Ser Glu His Asn Phe Ile Asp Trp Phe Leu Gln
       130                 135                 140
   Lys Met Gln Ser Ser Glu Phe Arg Lys Ile Leu Val Ser Lys Val Glu
   145                 150                 155                 160
   Glu Lys Lys Asp Asp Lys Glu Leu Lys Asn Ala Val Lys Asn Ile Lys
                   165                 170                 175
   Asn Phe Ile Thr Gly Phe Glu Lys Asn Ser Val Glu Gly His Arg His
               180                 185                 190
   Arg Lys Val Tyr Phe Glu Asn Ile Lys Ser Asp Ile Thr Lys Asp Asn
           195                 200                 205
   Gln Leu Asp Ser Ile Lys Lys Lys Ile Pro Ser Val Cys Leu Ser Asn
       210                 215                 220
   Leu Leu Gly His Leu Ser Asn Leu Gln Trp Lys Asn Leu His Arg Tyr
   225                 230                 235                 240
   Leu Ala Lys Asn Pro Lys Gln Phe Asp Glu Gln Thr Phe Gly Asn Glu
                   245                 250                 255
   Phe Leu Arg Met Leu Lys Asn Phe Arg His Leu Lys Gly Ser Gln Glu
               260                 265                 270
   Ser Leu Ala Val Arg Asn Leu Ile Gln Gln Leu Glu Gln Ser Gln Asp
           275                 280                 285
   Tyr Ile Ser Ile Leu Glu Lys Thr Pro Pro Glu Ile Thr Ile Pro Pro
       290                 295                 300
   Tyr Glu Ala Arg Thr Asn Thr Gly Met Glu Lys Asp Gln Ser Leu Leu
   305                 310                 315                 320
   Leu Asn Pro Glu Lys Leu Asn Asn Leu Tyr Pro Asn Trp Arg Asn Leu
                   325                 330                 335
   Ile Pro Gly Ile Ile Asp Ala His Pro Phe Leu Glu Lys Asp Leu Glu
               340                 345                 350
   His Thr Lys Leu Arg Asp Arg Lys Arg Ile Ile Ser Pro Ser Lys Gln
           355                 360                 365
   Asp Glu Lys Arg Asp Ser Tyr Ile Leu Gln Arg Tyr Leu Asp Leu Asn
       370                 375                 380
   Lys Lys Ile Asp Lys Phe Lys Ile Lys Lys Gln Leu Ser Phe Leu Gly
   385                 390                 395                 400
   Gln Gly Lys Gln Leu Pro Ala Asn Leu Ile Glu Thr Gln Lys Glu Met
                   405                 410                 415
   Glu Thr His Phe Asn Ser Ser Leu Val Ser Val Leu Ile Gln Ile Ala
               420                 425                 430
   Ser Ala Tyr Asn Lys Glu Arg Glu Asp Ala Ala Gln Gly Ile Trp Phe
           435                 440                 445
   Asp Asn Ala Phe Ser Leu Cys Glu Leu Ser Asn Ile Asn Pro Pro Arg
       450                 455                 460
   Lys Gln Lys Ile Leu Pro Leu Leu Val Gly Ala Ile Leu Ser Glu Asp
   465                 470                 475                 480
   Phe Ile Asn Asn Lys Asp Lys Trp Ala Lys Phe Lys Ile Phe Trp Asn
                   485                 490                 495
   Thr His Lys Ile Gly Arg Thr Ser Leu Lys Ser Lys Cys Lys Glu Ile
               500                 505                 510
   Glu Glu Ala Arg Lys Asn Ser Gly Asn Ala Phe Lys Ile Asp Tyr Glu
           515                 520                 525
   Glu Ala Leu Asn His Pro Glu His Ser Asn Asn Lys Ala Leu Ile Lys
       530                 535                 540
   Ile Ile Gln Thr Ile Pro Asp Ile Ile Gln Ala Ile Gln Ser His Leu
   545                 550                 555                 560
   Gly His Asn Asp Ser Gln Ala Leu Ile Tyr His Asn Pro Phe Ser Leu
                   565                 570                 575
   Ser Gln Leu Tyr Thr Ile Leu Glu Thr Lys Arg Asp Gly Phe His Lys
               580                 585                 590
   Asn Cys Val Ala Val Thr Cys Glu Asn Tyr Trp Arg Ser Gln Lys Thr
           595                 600                 605
   Glu Ile Asp Pro Glu Ile Ser Tyr Ala Ser Arg Leu Pro Ala Asp Ser
       610                 615                 620
   Val Arg Pro Phe Asp Gly Val Leu Ala Arg Met Met Gln Arg Leu Ala
   625                 630                 635                 640
   Tyr Glu Ile Ala Met Ala Lys Trp Glu Gln Ile Lys His Ile Pro Asp
                   645                 650                 655
   Asn Ser Ser Leu Leu Ile Pro Ile Tyr Leu Glu Gln Asn Arg Phe Glu
               660                 665                 670
   Phe Glu Glu Ser Phe Lys Lys Ile Lys Gly Ser Ser Ser Asp Lys Thr
           675                 680                 685
   Leu Glu Gln Ala Ile Glu Lys Gln Asn Ile Gln Trp Glu Glu Lys Phe
       690                 695                 700
   Gln Arg Ile Ile Asn Ala Ser Met Asn Ile Cys Pro Tyr Lys Gly Ala
   705 710 715 720
   Ser Ile Gly Gly Gln Gly Glu Ile Asp His Ile Tyr Pro Arg Ser Leu
   725 730 735
   Ser Lys Lys His Phe Gly Val Ile Phe Asn Ser Glu Val Asn Leu Ile
   740 745 750
   Tyr Cys Ser Ser Gln Gly Asn Arg Glu Lys Lys Glu Glu His Tyr Leu
   755 760 765
   Leu Glu His Leu Ser Pro Leu Tyr Leu Lys His Gln Phe Gly Thr Asp
   770 775 780
   Asn Val Ser Asp Ile Lys Asn Phe Ile Ser Gln Asn Val Ala Asn Ile
   785 790 795 800
   Lys Lys Tyr Ile Ser Phe His Leu Leu Thr Pro Glu Gln Gln Lys Ala
   805 810 815
   Ala Arg His Ala Leu Phe Leu Asp Tyr Asp Asp Glu Ala Phe Lys Thr
   820 825 830
   Ile Thr Lys Phe Leu Met Ser Gln Gln Lys Ala Arg Val Asn Gly Thr
   835 840 845
   Gln Lys Phe Leu Gly Lys Gln Ile Met Glu Phe Leu Ser Thr Leu Ala
   850 855 860
   Asp Ser Lys Gln Leu Gln Leu Glu Phe Ser Ile Lys Gln Ile Thr Ala
   865 870 875 880
   Glu Glu Val His Asp His Arg Glu Leu Leu Ser Lys Gln Glu Pro Lys
   885 890 895
   Leu Val Lys Ser Arg Gln Gln Ser Phe Pro Ser His Ala Ile Asp Ala
   900 905 910
   Thr Leu Thr Met Ser Ile Gly Leu Lys Glu Phe Pro Gln Phe Ser Gln
   915 920 925
   Glu Leu Asp Asn Ser Trp Phe Ile Asn His Leu Met Pro Asp Glu Val
   930 935 940
   His Leu Asn Pro Val Arg Ser Lys Glu Lys Tyr Asn Lys Pro Asn Ile
   945 950 955 960
   Ser Ser Thr Pro Leu Phe Lys Asp Ser Leu Tyr Ala Glu Arg Phe Ile
   965 970 975
   Pro Val Trp Val Lys Gly Glu Thr Phe Ala Ile Gly Phe Ser Glu Lys
   980 985 990
   Asp Leu Phe Glu Ile Lys Pro Ser Asn Lys Glu Lys Leu Phe Thr Leu
   995 1000 1005
   Leu Lys Thr Tyr Ser Thr Lys Asn Pro Gly Glu Ser Leu Gln Glu
   1010 1015 1020
   Leu Gln Ala Lys Ser Lys Ala Lys Trp Leu Tyr Phe Pro Ile Asn
   1025 1030 1035
   Lys Thr Leu Ala Leu Glu Phe Leu His His Tyr Phe His Lys Glu
   1040 1045 1050
   Ile Val Thr Pro Asp Asp Thr Thr Val Cys His Phe Ile Asn Ser
   1055 1060 1065
   Leu Arg Tyr Tyr Thr Lys Lys Glu Ser Ile Thr Val Lys Ile Leu
   1070 1075 1080
   Lys Glu Pro Met Pro Val Leu Ser Val Lys Phe Glu Ser Ser Lys
   1085 1090 1095
   Lys Asn Val Leu Gly Ser Phe Lys His Thr Ile Ala Leu Pro Ala
   1100 1105 1110
   Thr Lys Asp Trp Glu Arg Leu Phe Asn His Pro Asn Phe Leu Ala
   1115 1120 1125
   Leu Lys Ala Asn Pro Ala Pro Asn Pro Lys Glu Phe Asn Glu Phe
   1130 1135 1140
   Ile Arg Lys Tyr Phe Leu Ser Asp Asn Asn Pro Asn Ser Asp Ile
   1145 1150 1155
   Pro Asn Asn Gly His Asn Ile Lys Pro Gln Lys His Lys Ala Val
   1160 1165 1170
   Arg Lys Val Phe Ser Leu Pro Val Ile Pro Gly Asn Ala Gly Thr
   1175 1180 1185
   Met Met Arg Ile Arg Arg Lys Asp Asn Lys Gly Gln Pro Leu Tyr
   1190 1195 1200
   Gln Leu  Gln Thr Ile Asp Asp  Thr Pro Ser Met Gly  Ile Gln Ile
       1205                 1210                 1215
   Asn Glu  Asp Arg Leu Val Lys  Gln Glu Val Leu Met  Asp Ala Tyr
       1220                 1225                 1230
   Lys Thr  Arg Asn Leu Ser Thr  Ile Asp Gly Ile Asn  Asn Ser Glu
       1235                 1240                 1245
   Gly Gln  Ala Tyr Ala Thr Phe  Asp Asn Trp Leu Thr  Leu Pro Val
       1250                 1255                 1260
   Ser Thr  Phe Lys Pro Glu Ile  Ile Lys Leu Glu Met  Lys Pro His
       1265                 1270                 1275
   Ser Lys  Thr Arg Arg Tyr Ile  Arg Ile Thr Gln Ser  Leu Ala Asp
       1280                 1285                 1290
   Phe Ile  Lys Thr Ile Asp Glu  Ala Leu Met Ile Lys  Pro Ser Asp
       1295                 1300                 1305
   Ser Ile  Asp Asp Pro Leu Asn  Met Pro Asn Glu Ile  Val Cys Lys
       1310                 1315                 1320
   Asn Lys  Leu Phe Gly Asn Glu  Leu Lys Pro Arg Asp  Gly Lys Met
       1325                 1330                 1335
   Lys Ile  Val Ser Thr Gly Lys  Ile Val Thr Tyr Glu  Phe Glu Ser
       1340                 1345                 1350
   Asp Ser  Thr Pro Gln Trp Ile  Gln Thr Leu Tyr Val  Thr Gln Leu
       1355                 1360                 1365
   Lys Lys  Gln Pro
       1370
<210> 166
   <211> 4200
   <212> DNA
   <213> Artificial Sequence
<220>
   <221> source
   <223> /note="Description of Artificial Sequence: Synthetic polynucleotide"
<400> 166
   accggtgcca ccatgtaccc atacgatgtt ccagattacg cttcgccgaa gaaaaagcgc 60
   aaggtcgaag cgtccatgga gtcctctcag atcctgtcac ctatcggcat cgacctgggc 120
   ggaaagttta ctggagtctg cctgtcacac ctggaagcat ttgctgagct gcctaaccac 180
   gcaaatacaa agtactcagt gatcctgatt gatcataaca atttccagct gagccaggca 240
   cagcggagag ccactagaca cagggtgcgc aacaagaaaa gaaatcagtt cgtgaagagg 300
   gtcgccctgc agctgtttca gcacatcctg tcccgggacc tgaacgccaa ggaggaaacc 360
   gctctgtgcc attacctgaa caatagaggc tacacctatg tggacacaga tctggacgag 420
   tatatcaaag atgaaaccac aattaacctg ctgaaggagc tgctgccaag cgagtccgaa 480
   cataatttca ttgactggtt tctgcagaag atgcagagct ccgagttccg gaagatcctg 540
   gtgagcaaag tcgaggaaaa gaaagacgat aaagaactga agaacgccgt gaagaacatc 600
   aagaacttca tcaccggatt cgagaaaaat agcgtggaag gccaccgaca tcggaaggtc 660
   tactttgaga acatcaagtc cgatattaca aaagacaatc agctggattc tatcaagaaa 720
   aagattccta gcgtgtgtct gtccaacctg ctgggccacc tgtccaacct gcagtggaag 780
   aatctgcata ggtatctggc caaaaatcca aagcagttcg acgagcagac cttcgggaac 840
   gaatttctgc ggatgctgaa gaattttcga cacctgaaag gatctcagga gagtctggct 900
   gtgcgcaacc tgatccagca gctggaacag tctcaggatt acatcagtat cctggagaaa 960
   accccccctg aaatcaccat tccaccctat gaggcccgga caaacactgg catggaaaaa 1020
   gaccagagcc tgctgctgaa ccccgagaag ctgaacaatc tgtacccaaa ctggagaaat 1080
   ctgatccccg ggatcattga cgcccaccct ttcctggaga aggatctgga acatacaaag 1140
   ctgagagaca ggaaacgcat catttctccc agtaaacagg acgagaagcg ggatagctac 1200
   atcctgcaga gatatctgga cctgaacaaa aagatcgata agttcaagat caagaagcag 1260
   ctgagctttc tgggacaggg caaacagctg cctgctaacc tgatcgagac acagaaggag 1320
   atggaaactc acttcaattc tagtctggtg tctgtcctga tccagattgc tagtgcatac 1380
   aacaaggaga gggaagatgc cgctcagggg atctggttcg acaatgcctt ttcactgtgc 1440
   gagctgagca acatcaatcc tccacgcaaa cagaagattc tgcccctgct ggtgggagca 1500
   atcctgagcg aggacttcat taacaacaag gataagtggg ccaaattcaa gatcttttgg 1560
   aacacccaca agattgggcg aacatcactg aaaagcaagt gtaaagagat cgaggaagcc 1620
   cggaagaaca gtggaaacgc ttttaaaatc gactacgagg aagctctgaa tcacccagag 1680
   cattcaaaca acaaggccct gatcaagatc attcagacca ttcccgatat cattcaggcc 1740
   atccagtcac acctgggaca taacgacagc caggctctga tctaccacaa tcctttctca 1800
   ctgagccagc tgtatactat cctggagaca aagagggatg gctttcataa aaactgcgtg 1860
   gccgtcactt gtgaaaatta ctggcggagc cagaaaaccg agatcgaccc agaaatttcc 1920
   tatgcatcta ggctgccagc agacagtgtg cgccccttcg atggcgtcct ggcacgaatg 1980
   atgcagcggc tggcctacga gatcgccatg gctaagtggg aacagatcaa acacattcct 2040
   gataactcaa gcctgctgat cccaatctac ctggagcaga atcggttcga atttgaggag 2100
   agcttcaaga agatcaaggg gtcctctagt gacaaaaccc tggagcaggc catcgaaaag 2160
   cagaacattc agtgggagga aaagttccag agaatcatta acgcaagtat gaatatctgc 2220
   ccttacaagg gcgcctcaat tggcgggcag ggggagatcg accacatcta cccaaggtcc 2280
   ctgtctaaaa agcatttcgg cgtgatcttt aactccgaag tcaatctgat ctactgttca 2340
   agccagggga atcgcgagaa aaaggaggaa cactacctgc tggaacatct gtctccactg 2400
   tatctgaaac accagttcgg cactgacaac gtgtccgata tcaagaattt tatttctcag 2460
   aacgtcgcta atatcaaaaa gtacatttcc ttccacctgc tgaccccaga gcagcagaag 2520
   gcagcacggc acgccctgtt tctggattat gacgatgaag cattcaaaac cattacaaag 2580
   tttctgatgt ctcagcagaa ggccagagtg aacggcacac agaaattcct ggggaagcag 2640
   atcatggagt ttctgtccac tctggcagat tctaaacagc tgcagctgga gttcagcatc 2700
   aagcagatta ccgccgagga agtgcacgac catagagagc tgctgtctaa gcaggaaccc 2760
   aaactggtca agagtaggca gcagagtttc ccttcacacg ctatcgacgc aactctgacc 2820
   atgtctattg ggctgaagga gttcccacag tttagtcagg aactggataa ctcatggttt 2880
   atcaatcacc tgatgccaga cgaggtgcat ctgaaccccg tccggagcaa ggaaaagtac 2940
   aacaaaccta acatctcctc tactccactg ttcaaggatt ccctgtatgc tgagcggttc 3000
   atccccgtgt gggtcaaggg agaaaccttc gcaatcggct ttagcgagaa ggacctgttc 3060
   gagatcaagc cctccaacaa ggagaaactg tttacactgc tgaaaaccta cagtactaaa 3120
   aatcctggcg agtcactgca ggaactgcag gctaagagca aagcaaagtg gctgtacttc 3180
   ccaatcaaca aaaccctggc cctggagttc ctgcaccatt attttcacaa ggaaatcgtg 3240
   acacccgacg atactaccgt ctgccatttc atcaactcac tgagatacta cactaaaaag 3300
   gagagcatca ccgtgaaaat tctgaaggaa cccatgcctg tgctgtccgt caagttcgag 3360
   agttcaaaaa agaacgtgct gggatctttt aaacacacaa tcgccctgcc tgctactaag 3420
   gattgggaga ggctgttcaa ccatccaaat tttctggcac tgaaggccaa cccagctccc 3480
   aatcctaaag agttcaatga gttcatccgc aagtacttcc tgagcgacaa caatcccaac 3540
   tccgatatcc ctaacaatgg ccacaatatc aagccccaga aacataaggc cgtgcgaaag 3600
   gtctttagcc tgccagtgat ccccgggaac gctggaacca tgatgcgcat taggcgcaaa 3660
   gacaataagg gacagccact gtatcagctg cagacaatcg acgatactcc cagcatgggc 3720
   atccagatta acgaggatcg cctggtgaaa caggaagtcc tgatggacgc ctacaagaca 3780
   cgaaatctga gcactatcga tgggattaac aattccgagg gacaggcata tgccacattc 3840
   gacaactggc tgaccctgcc cgtgagcacc ttcaagcctg agatcatcaa gctggaaatg 3900
   aagcctcact ctaaaacccg acggtacatc agaattacac agagtctggc cgacttcatc 3960
   aaaactattg atgaggctct gatgatcaag cccagtgact caattgacga tcctctgaac 4020
   atgccaaatg agatcgtgtg taaaaacaag ctgttcggga atgaactgaa gcctagggat 4080
   ggaaaaatga agatcgtgag cactggcaag attgtcacct acgagtttga aagcgactcc 4140
   accccccagt ggatccagac cctgtatgtg acacagctga aaaagcagcc ttaagaattc 4200
<210> 167
   <211> 127
   <212> RNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, u, g, unknown or other
<400> 167
   nnnnnnnnnn nnnnnnnnnn guuugagagu uggaaacaac gaguucaaau aagaauucau 60
   caaaaucguc ccuuuuggga ccgcucauug uggagcauca aggcuuaaca ugguuaagcc 120
   uuuuuuu 127
<210> 168
   <211> 1395
   <212> PRT
   <213> Treponema denticola
<400> 168
   Met Lys Lys Glu Ile Lys Asp Tyr Phe Leu Gly Leu Asp Val Gly Thr
   1               5                   10                  15
   Gly Ser Val Gly Trp Ala Val Thr Asp Thr Asp Tyr Lys Leu Leu Lys
               20                  25                  30
   Ala Asn Arg Lys Asp Leu Trp Gly Met Arg Cys Phe Glu Thr Ala Glu
           35                  40                  45
   Thr Ala Glu Val Arg Arg Leu His Arg Gly Ala Arg Arg Arg Ile Glu
       50                  55                  60
   Arg Arg Lys Lys Arg Ile Lys Leu Leu Gln Glu Leu Phe Ser Gln Glu
   65                  70                  75                  80
   Ile Ala Lys Thr Asp Glu Gly Phe Phe Gln Arg Met Lys Glu Ser Pro
   85 90 95
   Phe Tyr Ala Glu Asp Lys Thr Ile Leu Gln Glu Asn Thr Leu Phe Asn
   100 105 110
   Asp Lys Asp Phe Ala Asp Lys Thr Tyr His Lys Ala Tyr Pro Thr Ile
   115 120 125
   Asn His Leu Ile Lys Ala Trp Ile Glu Asn Lys Val Lys Pro Asp Pro
   130 135 140
   Arg Leu Leu Tyr Leu Ala Cys His Asn Ile Ile Lys Lys Arg Gly His
   145 150 155 160
   Phe Leu Phe Glu Gly Asp Phe Asp Ser Glu Asn Gln Phe Asp Thr Ser
   165 170 175
   Ile Gln Ala Leu Phe Glu Tyr Leu Arg Glu Asp Met Glu Val Asp Ile
   180 185 190
   Asp Ala Asp Ser Gln Lys Val Lys Glu Ile Leu Lys Asp Ser Ser Leu
   195 200 205
   Lys Asn Ser Glu Lys Gln Ser Arg Leu Asn Lys Ile Leu Gly Leu Lys
   210 215 220
   Pro Ser Asp Lys Gln Lys Lys Ala Ile Thr Asn Leu Ile Ser Gly Asn
   225 230 235 240
   Lys Ile Asn Phe Ala Asp Leu Tyr Asp Asn Pro Asp Leu Lys Asp Ala
   245 250 255
   Glu Lys Asn Ser Ile Ser Phe Ser Lys Asp Asp Phe Asp Ala Leu Ser
   260 265 270
   Asp Asp Leu Ala Ser Ile Leu Gly Asp Ser Phe Glu Leu Leu Leu Lys
   275 280 285
   Ala Lys Ala Val Tyr Asn Cys Ser Val Leu Ser Lys Val Ile Gly Asp
   290 295 300
   Glu Gln Tyr Leu Ser Phe Ala Lys Val Lys Ile Tyr Glu Lys His Lys
   305 310 315 320
   Thr Asp Leu Thr Lys Leu Lys Asn Val Ile Lys Lys His Phe Pro Lys
   325 330 335
   Asp Tyr Lys Lys Val Phe Gly Tyr Asn Lys Asn Glu Lys Asn Asn Asn
   340 345 350
   Asn Tyr Ser Gly Tyr Val Gly Val Cys Lys Thr Lys Ser Lys Lys Leu
   355 360 365
   Ile Ile Asn Asn Ser Val Asn Gln Glu Asp Phe Tyr Lys Phe Leu Lys
   370 375 380
   Thr Ile Leu Ser Ala Lys Ser Glu Ile Lys Glu Val Asn Asp Ile Leu
   385 390 395 400
   Thr Glu Ile Glu Thr Gly Thr Phe Leu Pro Lys Gln Ile Ser Lys Ser
   405 410 415
   Asn Ala Glu Ile Pro Tyr Gln Leu Arg Lys Met Glu Leu Glu Lys Ile
   420 425 430
   Leu Ser Asn Ala Glu Lys His Phe Ser Phe Leu Lys Gln Lys Asp Glu
   435 440 445
   Lys Gly Leu Ser His Ser Glu Lys Ile Ile Met Leu Leu Thr Phe Lys
   450 455 460
   Ile Pro Tyr Tyr Ile Gly Pro Ile Asn Asp Asn His Lys Lys Phe Phe
   465 470 475 480
   Pro Asp Arg Cys Trp Val Val Lys Lys Glu Lys Ser Pro Ser Gly Lys
   485 490 495
   Thr Thr Pro Trp Asn Phe Phe Asp His Ile Asp Lys Glu Lys Thr Ala
   500 505 510
   Glu Ala Phe Ile Thr Ser Arg Thr Asn Phe Cys Thr Tyr Leu Val Gly
   515 520 525
   Glu Ser Val Leu Pro Lys Ser Ser Leu Leu Tyr Ser Glu Tyr Thr Val
   530 535 540
   Leu Asn Glu Ile Asn Asn Leu Gln Ile Ile Ile Asp Gly Lys Asn Ile
   545 550 555 560
   Cys Asp Ile Lys Leu Lys Gln Lys Ile Tyr Glu Asp Leu Phe Lys Lys
   565 570 575
   Tyr Lys Lys Ile Thr Gln Lys Gln Ile Ser Thr Phe Ile Lys His Glu
   580 585 590
   Gly Ile Cys Asn Lys Thr Asp Glu Val Ile Ile Leu Gly Ile Asp Lys
   595 600 605
   Glu Cys Thr Ser Ser Leu Lys Ser Tyr Ile Glu Leu Lys Asn Ile Phe
   610 615 620
   Gly Lys Gln Val Asp Glu Ile Ser Thr Lys Asn Met Leu Glu Glu Ile
   625 630 635 640
   Ile Arg Trp Ala Thr Ile Tyr Asp Glu Gly Glu Gly Lys Thr Ile Leu
   645 650 655
   Lys Thr Lys Ile Lys Ala Glu Tyr Gly Lys Tyr Cys Ser Asp Glu Gln
   660 665 670
   Ile Lys Lys Ile Leu Asn Leu Lys Phe Ser Gly Trp Gly Arg Leu Ser
   675 680 685
   Arg Lys Phe Leu Glu Thr Val Thr Ser Glu Met Pro Gly Phe Ser Glu
   690 695 700
   Pro Val Asn Ile Ile Thr Ala Met Arg Glu Thr Gln Asn Asn Leu Met
   705 710 715 720
   Glu Leu Leu Ser Ser Glu Phe Thr Phe Thr Glu Asn Ile Lys Lys Ile
   725 730 735
   Asn Ser Gly Phe Glu Asp Ala Glu Lys Gln Phe Ser Tyr Asp Gly Leu
   740 745 750
   Val Lys Pro Leu Phe Leu Ser Pro Ser Val Lys Lys Met Leu Trp Gln
   755 760 765
   Thr Leu Lys Leu Val Lys Glu Ile Ser His Ile Thr Gln Ala Pro Pro
   770 775 780
   Lys Lys Ile Phe Ile Glu Met Ala Lys Gly Ala Glu Leu Glu Pro Ala
   785 790 795 800
   Arg Thr Lys Thr Arg Leu Lys Ile Leu Gln Asp Leu Tyr Asn Asn Cys
   805 810 815
   Lys Asn Asp Ala Asp Ala Phe Ser Ser Glu Ile Lys Asp Leu Ser Gly
   820 825 830
   Lys Ile Glu Asn Glu Asp Asn Leu Arg Leu Arg Ser Asp Lys Leu Tyr
   835 840 845
   Leu Tyr Tyr Thr Gln Leu Gly Lys Cys Met Tyr Cys Gly Lys Pro Ile
       850                 855                 860
   Glu Ile Gly His Val Phe Asp Thr Ser Asn Tyr Asp Ile Asp His Ile
   865                 870                 875                 880
   Tyr Pro Gln Ser Lys Ile Lys Asp Asp Ser Ile Ser Asn Arg Val Leu
                   885                 890                 895
   Val Cys Ser Ser Cys Asn Lys Asn Lys Glu Asp Lys Tyr Pro Leu Lys
               900                 905                 910
   Ser Glu Ile Gln Ser Lys Gln Arg Gly Phe Trp Asn Phe Leu Gln Arg
           915                 920                 925
   Asn Asn Phe Ile Ser Leu Glu Lys Leu Asn Arg Leu Thr Arg Ala Thr
       930                 935                 940
   Pro Ile Ser Asp Asp Glu Thr Ala Lys Phe Ile Ala Arg Gln Leu Val
   945                 950                 955                 960
   Glu Thr Arg Gln Ala Thr Lys Val Ala Ala Lys Val Leu Glu Lys Met
                   965                 970                 975
   Phe Pro Glu Thr Lys Ile Val Tyr Ser Lys Ala Glu Thr Val Ser Met
               980                 985                 990
   Phe Arg Asn Lys Phe Asp Ile Val  Lys Cys Arg Glu Ile  Asn Asp Phe
           995                 1000                1005
   His His  Ala His Asp Ala Tyr  Leu Asn Ile Val Val  Gly Asn Val
       1010                1015                1020
   Tyr Asn  Thr Lys Phe Thr Asn  Asn Pro Trp Asn Phe  Ile Lys Glu
       1025                1030                1035
   Lys Arg  Asp Asn Pro Lys Ile  Ala Asp Thr Tyr Asn  Tyr Tyr Lys
       1040                1045                1050
   Val Phe  Asp Tyr Asp Val Lys  Arg Asn Asn Ile Thr  Ala Trp Glu
       1055                1060                1065
   Lys Gly  Lys Thr Ile Ile Thr  Val Lys Asp Met Leu  Lys Arg Asn
       1070                1075                1080
   Thr Pro  Ile Tyr Thr Arg Gln  Ala Ala Cys Lys Lys  Gly Glu Leu
   1085 1090 1095
   Phe Asn Gln Thr Ile Met Lys Lys Gly Leu Gly Gln His Pro Leu
   1100 1105 1110
   Lys Lys Glu Gly Pro Phe Ser Asn Ile Ser Lys Tyr Gly Gly Tyr
   1115 1120 1125
   Asn Lys Val Ser Ala Ala Tyr Tyr Thr Leu Ile Glu Tyr Glu Glu
   1130 1135 1140
   Lys Gly Asn Lys Ile Arg Ser Leu Glu Thr Ile Pro Leu Tyr Leu
   1145 1150 1155
   Val Lys Asp Ile Gln Lys Asp Gln Asp Val Leu Lys Ser Tyr Leu
   1160 1165 1170
   Thr Asp Leu Leu Gly Lys Lys Glu Phe Lys Ile Leu Val Pro Lys
   1175 1180 1185
   Ile Lys Ile Asn Ser Leu Leu Lys Ile Asn Gly Phe Pro Cys His
   1190 1195 1200
   Ile Thr Gly Lys Thr Asn Asp Ser Phe Leu Leu Arg Pro Ala Val
   1205 1210 1215
   Gln Phe Cys Cys Ser Asn Asn Glu Val Leu Tyr Phe Lys Lys Ile
   1220 1225 1230
   Ile Arg Phe Ser Glu Ile Arg Ser Gln Arg Glu Lys Ile Gly Lys
   1235 1240 1245
   Thr Ile Ser Pro Tyr Glu Asp Leu Ser Phe Arg Ser Tyr Ile Lys
   1250 1255 1260
   Glu Asn Leu Trp Lys Lys Thr Lys Asn Asp Glu Ile Gly Glu Lys
   1265 1270 1275
   Glu Phe Tyr Asp Leu Leu Gln Lys Lys Asn Leu Glu Ile Tyr Asp
   1280 1285 1290
   Met Leu Leu Thr Lys His Lys Asp Thr Ile Tyr Lys Lys Arg Pro
   1295 1300 1305
   Asn Ser Ala Thr Ile Asp Ile Leu Val Lys Gly Lys Glu Lys Phe
   1310 1315 1320
   Lys Ser Leu Ile Ile Glu Asn Gln Phe Glu Val Ile Leu Glu Ile
   1325 1330 1335
   Leu Lys Leu Phe Ser Ala Thr Arg Asn Val Ser Asp Leu Gln His
   1340 1345 1350
   Ile Gly Gly Ser Lys Tyr Ser Gly Val Ala Lys Ile Gly Asn Lys
   1355 1360 1365
   Ile Ser Ser Leu Asp Asn Cys Ile Leu Ile Tyr Gln Ser Ile Thr
   1370 1375 1380
   Gly Ile Phe Glu Lys Arg Ile Asp Leu Leu Lys Val
   1385 1390 1395
<210> 169
   <211> 4269
   <212> DNA
   <213> Artificial Sequence
<220>
   <221> source
   <223> /note="Description of Artificial Sequence: Synthetic polynucleotide"
<400> 169
   accggtgcca ccatgtaccc atacgatgtt ccagattacg cttcgccgaa gaaaaagcgc      60
   aaggtcgaag cgtccatgaa aaaagaaatc aaagactact tcctggggct ggatgtgggg     120
   actgggagcg tggggtgggc tgtgaccgat actgactaca aactgctgaa ggctaaccga     180
   aaagacctgt ggggcatgag atgcttcgag acagccgaaa ctgctgaggt gcggagactg     240
   cacaggggag ccaggcgccg aatcgagcgg agaaagaaac gcattaagct gctgcaggag     300
   ctgttctctc aggaaatcgc caaaaccgat gagggcttct ttcagagaat gaaggaaagc     360
   cccttttacg ctgaggacaa aacaatcctg caggaaaaca ctctgttcaa tgacaaggat     420
   tttgctgata agacttacca caaagcatat cctaccatta atcatctgat caaggcttgg     480
   attgagaaca aggtgaaacc agacccccga ctgctgtacc tggcatgtca caacatcatt     540
   aagaaaaggg gacatttcct gtttgaaggc gacttcgatt cagagaatca gtttgatacc     600
   agcatccagg cactgttcga gtatctgcgc gaggacatgg aggtggacat cgatgccgac     660
   agccagaagg tcaaagagat tctgaaggat agctccctga agaactctga aaaacagagt     720
   cggctgaata agatcctggg gctgaagcct tccgacaaac agaagaaagc catcacaaac     780
   ctgatttctg gaaacaagat caatttcgcc gatctgtacg acaatccaga tctgaaggac     840
   gctgagaaaa actcaatcag cttctccaag gacgattttg atgcactgag tgacgatctg     900
   gcctcaattc tgggcgacag ctttgaactg ctgctgaagg ccaaagctgt ctataactgc     960
   tctgtgctga gtaaggtcat cggggacgag cagtacctga gcttcgccaa ggtgaaaatc 1020
   tacgaaaagc acaaaaccga tctgacaaag ctgaaaaacg tgatcaagaa acatttcccc 1080
   aaggactaca agaaggtctt tggatacaac aagaacgaga aaaacaacaa caattactcc 1140
   ggctatgtgg gagtctgtaa gaccaagagt aagaaactga tcattaacaa ctcagtcaac 1200
   caggaagatt tctacaagtt tctgaaaact atcctgtcag ccaagagcga gatcaaggaa 1260
   gtgaatgaca tcctgaccga gattgaaact ggcacctttc tgccaaagca gatctctaaa 1320
   agtaacgcag agattcccta tcagctgagg aaaatggagc tggaaaagat cctgtccaat 1380
   gccgaaaagc acttctcttt tctgaagcag aaagacgaaa aaggactgtc acatagcgag 1440
   aagatcatta tgctgctgac cttcaagatc ccttactata ttggcccaat caacgataat 1500
   cacaagaaat tctttcccga cagatgctgg gtggtcaaga aagagaaatc cccttctggc 1560
   aagaccacac catggaactt ctttgatcat atcgacaagg aaaaaacagc agaggccttc 1620
   attacttcta ggaccaattt ttgcacttac ctggtgggag agagcgtcct gcctaagtct 1680
   agtctgctgt actccgaata taccgtgctg aacgagatca acaatctgca gatcattatc 1740
   gatggcaaga atatttgtga catcaagctg aaacagaaga tctacgagga cctgttcaag 1800
   aagtacaaga aaattaccca gaagcagatc agcaccttca tcaagcacga aggcatctgc 1860
   aacaaaaccg atgaagtgat catcctgggg attgacaagg aatgtacatc aagcctgaaa 1920
   agctacatcg agctgaaaaa cattttcggc aagcaggtgg atgagatctc cactaagaat 1980
   atgctggagg aaattatcag atgggctacc atctacgacg agggggaagg aaagaccatc 2040
   ctgaaaacaa agatcaaggc agaatacgga aagtattgct ccgacgagca gattaagaaa 2100
   atcctgaacc tgaagttctc cggctggggg cgactgtctc ggaaatttct ggagacagtg 2160
   actagtgaaa tgcccggctt ctcagaacct gtcaatatta tcaccgccat gagggagaca 2220
   cagaacaatc tgatggagct gctgtcctct gagttcacct tcaccgagaa cattaagaaa 2280
   atcaattctg gattcgaaga tgccgagaag cagtttagtt acgacggcct ggtgaaacca 2340
   ctgtttctga gtccctcagt caagaaaatg ctgtggcaga ccctgaagct ggtgaaagag 2400
   attagccata tcacacaggc cccccctaag aaaattttca tcgaaatggc aaagggggcc 2460
   gagctggaac ctgctcggac taagaccaga ctgaaaatcc tgcaggatct gtataacaat 2520
   tgtaagaacg atgctgacgc cttcagctca gagatcaaag acctgagcgg aaagattgag 2580
   aacgaagata atctgaggct gcgctccgac aagctgtacc tgtactatac tcagctgggg 2640
   aaatgcatgt attgtggaaa gccaattgag atcggccacg tgttcgatac ctcaaactac 2700
   gatattgacc atatctatcc ccagagcaag atcaaagacg atagcatttc caatcgggtg 2760
   ctggtctgca gctcctgtaa caagaacaag gaggacaagt acccactgaa atcagagatc 2820
   cagagcaagc agcgcggctt ctggaacttt ctgcagcgaa acaatttcat ttctctggag 2880
   aagctgaata gactgacaag ggccactcca atcagtgacg atgagacagc caagtttatt 2940
   gctaggcagc tggtggaaac tcgccaggct accaaggtgg ccgctaaagt cctggaaaag 3000
   atgttccccg agacaaaaat cgtgtacagc aaggccgaga ctgtctccat gttccggaac 3060
   aagtttgata tcgtgaagtg cagagaaatt aacgattttc accatgctca cgacgcatac 3120
   ctgaatatcg tggtcggcaa cgtgtataat accaagttca caaacaatcc ttggaacttt 3180
   atcaaggaga aaagagataa tccaaagatt gctgacacct acaactacta taaggtgttc 3240
   gattatgacg tcaaaaggaa caatatcaca gcatgggaga aggggaaaac tattatcacc 3300
   gtgaaagaca tgctgaagag aaacacacca atctacacta ggcaggcagc ctgtaagaaa 3360
   ggggagctgt tcaatcagac cattatgaag aaaggactgg gccagcaccc cctgaagaaa 3420
   gaaggacctt tttccaatat ctctaaatac ggcgggtata acaaggtgag cgctgcatac 3480
   tatacactga ttgagtatga ggaaaagggc aacaaaatcc gctccctgga aactattccc 3540
   ctgtacctgg tgaaagatat ccagaaggat caggacgtcc tgaagtctta tctgacagac 3600
   ctgctgggga agaaagagtt caagatcctg gtgcccaaga tcaagatcaa cagcctgctg 3660
   aagatcaatg ggtttccttg ccatattaca ggaaaaacta acgatagttt cctgctgcgc 3720
   cctgccgtgc agttttgctg ttcaaacaat gaggtcctgt acttcaagaa aattatccgg 3780
   ttttccgaaa tccgctctca gcgagagaag atcgggaaaa caattagccc atacgaggac 3840
   ctgagcttcc ggtcatatat caaggagaac ctgtggaaga aaactaagaa cgatgaaatc 3900
   ggagagaagg aattttacga cctgctgcag aagaaaaacc tggagatcta tgatatgctg 3960
   ctgactaagc acaaagacac catctacaag aaacgcccta attctgccac cattgatatc 4020
   ctggtgaagg ggaaagagaa gttcaaaagc ctgattatcg aaaaccagtt tgaagtgatc 4080
   ctggagatcc tgaagctgtt ttctgcaaca cggaatgtca gtgacctgca gcatatcgga 4140
   ggcagcaagt actccggcgt ggccaaaatc gggaacaaga tctctagtct ggataactgt 4200
   atcctgatct atcagtccat caccggcatc ttcgagaaac ggatcgacct gctgaaggtg 4260
   taagaattc 4269
<210> 170
   <211> 119
   <212> RNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, u, g, unknown or other
<400> 170
   nnnnnnnnnn nnnnnnnnnn guuugagagu aggaaacuac acgguucaaa uaaagaauuu 60
   uucuaaucgc ccaaugggcc cauauugaua uggaugaaac ucgcuuagcg aguuuuuuu 119
<210> 171
   <211> 1365
   <212> PRT
   <213> Filifactor alocis
<400> 171
   Met Thr Lys Glu Tyr Tyr Leu Gly Leu Asp Val Gly Thr Asn Ser Val
   1               5                   10                  15
   Gly Trp Ala Val Thr Asp Ser Gln Tyr Asn Leu Cys Lys Phe Lys Lys
               20                  25                  30
   Lys Asp Met Trp Gly Ile Arg Leu Phe Glu Ser Ala Asn Thr Ala Lys
           35                  40                  45
   Asp Arg Arg Leu Gln Arg Gly Asn Arg Arg Arg Leu Glu Arg Lys Lys
       50                  55                  60
   Gln Arg Ile Asp Leu Leu Gln Glu Ile Phe Ser Pro Glu Ile Cys Lys
   65                  70                  75                  80
   Ile Asp Pro Thr Phe Phe Ile Arg Leu Asn Glu Ser Arg Leu His Leu
                   85                  90                  95
   Glu Asp Lys Ser Asn Asp Phe Lys Tyr Pro Leu Phe Ile Glu Lys Asp
               100                 105                 110
   Tyr Ser Asp Ile Glu Tyr Tyr Lys Glu Phe Pro Thr Ile Phe His Leu
           115                 120                 125
   Arg Lys His Leu Ile Glu Ser Glu Glu Lys Gln Asp Ile Arg Leu Ile
       130                 135                 140
   Tyr Leu Ala Leu His Asn Ile Ile Lys Thr Arg Gly His Phe Leu Ile
   145                 150                 155                 160
   Asp Gly Asp Leu Gln Ser Ala Lys Gln Leu Arg Pro Ile Leu Asp Thr
                   165                 170                 175
   Phe Leu Leu Ser Leu Gln Glu Glu Gln Asn Leu Ser Val Ser Leu Ser
               180                 185                 190
   Glu Asn Gln Lys Asp Glu Tyr Glu Glu Ile Leu Lys Asn Arg Ser Ile
           195                 200                 205
   Ala Lys Ser Glu Lys Val Lys Lys Leu Lys Asn Leu Phe Glu Ile Ser
       210                 215                 220
   Asp Glu Leu Glu Lys Glu Glu Lys Lys Ala Gln Ser Ala Val Ile Glu
   225                 230                 235                 240
   Asn Phe Cys Lys Phe Ile Val Gly Asn Lys Gly Asp Val Cys Lys Phe
                   245                 250                 255
   Leu Arg Val Ser Lys Glu Glu Leu Glu Ile Asp Ser Phe Ser Phe Ser
               260                 265                 270
   Glu Gly Lys Tyr Glu Asp Asp Ile Val Lys Asn Leu Glu Glu Lys Val
           275                 280                 285
   Pro Glu Lys Val Tyr Leu Phe Glu Gln Met Lys Ala Met Tyr Asp Trp
       290                 295                 300
   Asn Ile Leu Val Asp Ile Leu Glu Thr Glu Glu Tyr Ile Ser Phe Ala
   305                 310                 315                 320
   Lys Val Lys Gln Tyr Glu Lys His Lys Thr Asn Leu Arg Leu Leu Arg
                   325                 330                 335
   Asp Ile Ile Leu Lys Tyr Cys Thr Lys Asp Glu Tyr Asn Arg Met Phe
               340                 345                 350
   Asn Asp Glu Lys Glu Ala Gly Ser Tyr Thr Ala Tyr Val Gly Lys Leu
           355                 360                 365
   Lys Lys Asn Asn Lys Lys Tyr Trp Ile Glu Lys Lys Arg Asn Pro Glu
       370                 375                 380
   Glu Phe Tyr Lys Ser Leu Gly Lys Leu Leu Asp Lys Ile Glu Pro Leu
   385                 390                 395                 400
   Lys Glu Asp Leu Glu Val Leu Thr Met Met Ile Glu Glu Cys Lys Asn
                   405                 410                 415
   His Thr Leu Leu Pro Ile Gln Lys Asn Lys Asp Asn Gly Val Ile Pro
               420                 425                 430
   His Gln Val His Glu Val Glu Leu Lys Lys Ile Leu Glu Asn Ala Lys
           435                 440                 445
   Lys Tyr Tyr Ser Phe Leu Thr Glu Thr Asp Lys Asp Gly Tyr Ser Val
       450                 455                 460
   Val Gln Lys Ile Glu Ser Ile Phe Arg Phe Arg Ile Pro Tyr Tyr Val
   465                 470                 475                 480
   Gly Pro Leu Ser Thr Arg His Gln Glu Lys Gly Ser Asn Val Trp Met
                   485                 490                 495
   Val Arg Lys Pro Gly Arg Glu Asp Arg Ile Tyr Pro Trp Asn Met Glu
               500                 505                 510
   Glu Ile Ile Asp Phe Glu Lys Ser Asn Glu Asn Phe Ile Thr Arg Met
           515                 520                 525
   Thr Asn Lys Cys Thr Tyr Leu Ile Gly Glu Asp Val Leu Pro Lys His
       530                 535                 540
   Ser Leu Leu Tyr Ser Lys Tyr Met Val Leu Asn Glu Leu Asn Asn Val
   545                 550                 555                 560
   Lys Val Arg Gly Lys Lys Leu Pro Thr Ser Leu Lys Gln Lys Val Phe
                   565                 570                 575
   Glu Asp Leu Phe Glu Asn Lys Ser Lys Val Thr Gly Lys Asn Leu Leu
               580                 585                 590
   Glu Tyr Leu Gln Ile Gln Asp Lys Asp Ile Gln Ile Asp Asp Leu Ser
           595                 600                 605
   Gly Phe Asp Lys Asp Phe Lys Thr Ser Leu Lys Ser Tyr Leu Asp Phe
       610                 615                 620
   Lys Lys Gln Ile Phe Gly Glu Glu Ile Glu Lys Glu Ser Ile Gln Asn
   625                 630                 635                 640
   Met Ile Glu Asp Ile Ile Lys Trp Ile Thr Ile Tyr Gly Asn Asp Lys
                   645                 650                 655
   Glu Met Leu Lys Arg Val Ile Arg Ala Asn Tyr Ser Asn Gln Leu Thr
               660                 665                 670
   Glu Glu Gln Met Lys Lys Ile Thr Gly Phe Gln Tyr Ser Gly Trp Gly
           675                 680                 685
   Asn Phe Ser Lys Met Phe Leu Lys Gly Ile Ser Gly Ser Asp Val Ser
       690                 695                 700
   Thr Gly Glu Thr Phe Asp Ile Ile Thr Ala Met Trp Glu Thr Asp Asn
   705                 710                 715                 720
   Asn Leu Met Gln Ile Leu Ser Lys Lys Phe Thr Phe Met Asp Asn Val
                   725                 730                 735
   Glu Asp Phe Asn Ser Gly Lys Val Gly Lys Ile Asp Lys Ile Thr Tyr
               740                 745                 750
   Asp Ser Thr Val Lys Glu Met Phe Leu Ser Pro Glu Asn Lys Arg Ala
           755                 760                 765
   Val Trp Gln Thr Ile Gln Val Ala Glu Glu Ile Lys Lys Val Met Gly
       770                 775                 780
   Cys Glu Pro Lys Lys Ile Phe Ile Glu Met Ala Arg Gly Gly Glu Lys
   785                 790                 795                 800
   Val Lys Lys Arg Thr Lys Ser Arg Lys Ala Gln Leu Leu Glu Leu Tyr
                   805                 810                 815
   Ala Ala Cys Glu Glu Asp Cys Arg Glu Leu Ile Lys Glu Ile Glu Asp
               820                 825                 830
   Arg Asp Glu Arg Asp Phe Asn Ser Met Lys Leu Phe Leu Tyr Tyr Thr
           835                 840                 845
   Gln Phe Gly Lys Cys Met Tyr Ser Gly Asp Asp Ile Asp Ile Asn Glu
       850                 855                 860
   Leu Ile Arg Gly Asn Ser Lys Trp Asp Arg Asp His Ile Tyr Pro Gln
   865                 870                 875                 880
   Ser Lys Ile Lys Asp Asp Ser Ile Asp Asn Leu Val Leu Val Asn Lys
                   885                 890                 895
   Thr Tyr Asn Ala Lys Lys Ser Asn Glu Leu Leu Ser Glu Asp Ile Gln
               900                 905                 910
   Lys Lys Met His Ser Phe Trp Leu Ser Leu Leu Asn Lys Lys Leu Ile
           915                 920                 925
   Thr Lys Ser Lys Tyr Asp Arg Leu Thr Arg Lys Gly Asp Phe Thr Asp
       930                 935                 940
   Glu Glu Leu Ser Gly Phe Ile Ala Arg Gln Leu Val Glu Thr Arg Gln
   945 950 955 960
   Ser Thr Lys Ala Ile Ala Asp Ile Phe Lys Gln Ile Tyr Ser Ser Glu
   965 970 975
   Val Val Tyr Val Lys Ser Ser Leu Val Ser Asp Phe Arg Lys Lys Pro
   980 985 990
   Leu Asn Tyr Leu Lys Ser Arg Arg Val Asn Asp Tyr His His Ala Lys
   995 1000 1005
   Asp Ala Tyr Leu Asn Ile Val Val Gly Asn Val Tyr Asn Lys Lys
   1010 1015 1020
   Phe Thr Ser Asn Pro Ile Gln Trp Met Lys Lys Asn Arg Asp Thr
   1025 1030 1035
   Asn Tyr Ser Leu Asn Lys Val Phe Glu His Asp Val Val Ile Asn
   1040 1045 1050
   Gly Glu Val Ile Trp Glu Lys Cys Thr Tyr His Glu Asp Thr Asn
   1055 1060 1065
   Thr Tyr Asp Gly Gly Thr Leu Asp Arg Ile Arg Lys Ile Val Glu
   1070 1075 1080
   Arg Asp Asn Ile Leu Tyr Thr Glu Tyr Ala Tyr Cys Glu Lys Gly
   1085 1090 1095
   Glu Leu Phe Asn Ala Thr Ile Gln Asn Lys Asn Gly Asn Ser Thr
   1100 1105 1110
   Val Ser Leu Lys Lys Gly Leu Asp Val Lys Lys Tyr Gly Gly Tyr
   1115 1120 1125
   Phe Ser Ala Asn Thr Ser Tyr Phe Ser Leu Ile Glu Phe Glu Asp
   1130 1135 1140
   Lys Lys Gly Asp Arg Ala Arg His Ile Ile Gly Val Pro Ile Tyr
   1145 1150 1155
   Ile Ala Asn Met Leu Glu His Ser Pro Ser Ala Phe Leu Glu Tyr
   1160 1165 1170
   Cys Glu Gln Lys Gly Tyr Gln Asn Val Arg Ile Leu Val Glu Lys
   1175 1180 1185
   Ile Lys Lys Asn Ser Leu Leu Ile Ile Asn Gly Tyr Pro Leu Arg
       1190                1195                1200
   Ile Arg Gly Glu Asn Glu Val Asp Thr Ser Phe Lys Arg Ala Ile
       1205                1210                1215
   Gln Leu Lys Leu Asp Gln Lys Asn Tyr Glu Leu Val Arg Asn Ile
       1220                1225                1230
   Glu Lys Phe Leu Glu Lys Tyr Val Glu Lys Lys Gly Asn Tyr Pro
       1235                1240                1245
   Ile Asp Glu Asn Arg Asp His Ile Thr His Glu Lys Met Asn Gln
       1250                1255                1260
   Leu Tyr Glu Val Leu Leu Ser Lys Met Lys Lys Phe Asn Lys Lys
       1265                1270                1275
   Gly Met Ala Asp Pro Ser Asp Arg Ile Glu Lys Ser Lys Pro Lys
       1280                1285                1290
   Phe Ile Lys Leu Glu Asp Leu Ile Asp Lys Ile Asn Val Ile Asn
       1295                1300                1305
   Lys Met Leu Asn Leu Leu Arg Cys Asp Asn Asp Thr Lys Ala Asp
       1310                1315                1320
   Leu Ser Leu Ile Glu Leu Pro Lys Asn Ala Gly Ser Phe Val Val
       1325                1330                1335
   Lys Lys Asn Thr Ile Gly Lys Ser Lys Ile Ile Leu Val Asn Gln
       1340                1345                1350
   Ser Val Thr Gly Leu Tyr Glu Asn Arg Arg Glu Leu
       1355                1360                1365
<210> 172
   <211> 4179
   <212> DNA
   <213> Artificial Sequence
<220>
   <221> source
   <223> /note="Description of Artificial Sequence: Synthetic polynucleotide"
<400> 172
   accggtgcca ccatgtaccc atacgatgtt ccagattacg cttcgccgaa gaaaaagcgc    60
   aaggtcgaag cgtccatgac caaggagtat tacctggggc tggatgtggg gaccaattcc   120
   gtgggatggg cagtgaccga ttctcagtac aacctgtgca agtttaagaa aaaggatatg 180
   tggggcatcc ggctgttcga aagcgccaac acagcaaagg accggagact gcagagaggg 240
   aataggcgcc gactggagcg gaaaaagcag agaattgatc tgctgcagga aatcttctcc 300
   ccagagatct gcaagattga ccccactttc tttatccgac tgaacgaatc ccggctgcac 360
   ctggaggaca agtctaacga tttcaaatac ccactgttta ttgagaagga ctattctgat 420
   atcgagtact ataaagagtt ccccaccatt tttcacctga ggaagcatct gatcgagagt 480
   gaggaaaaac aggatatccg gctgatctac ctggccctgc acaacatcat taagacccga 540
   ggacattttc tgattgacgg cgatctgcag agcgccaagc agctgaggcc catcctggat 600
   acattcctgc tgtccctgca ggaggaacag aacctgtcag tgagcctgtc cgaaaatcag 660
   aaggacgagt atgaggaaat tctgaaaaac cgcagcatcg ccaagtccga aaaagtgaaa 720
   aagctgaaga atctgtttga gatctcagac gagctggaaa aagaggagaa gaaggcccag 780
   agcgccgtga tcgagaactt ctgcaagttt atcgtgggaa ataagggcga tgtctgtaaa 840
   ttcctgcggg tgtctaagga ggaactggag attgactctt tcagtttttc agagggcaag 900
   tacgaggacg acatcgtgaa aaacctggag gaaaaagtgc ctgaaaaggt ctacctgttt 960
   gagcagatga aggcaatgta tgattggaat attctggtcg acatcctgga aaccgaggaa 1020
   tacatcagct tcgccaaagt gaagcagtat gagaaacaca agactaacct gcggctgctg 1080
   agagacatca ttctgaaata ctgcaccaag gatgagtata atcggatgtt taacgacgag 1140
   aaggaagctg gcagctacac cgcatatgtg gggaaactga aaaagaacaa caagaagtac 1200
   tggatcgaga aaaagagaaa tcccgaggag ttctacaaat ccctgggcaa gctgctggat 1260
   aaaattgagc ctctgaagga ggacctggaa gtgctgacta tgatgatcga ggagtgtaag 1320
   aaccacaccc tgctgccaat tcagaaaaat aaggacaacg gcgtgatccc ccaccaggtg 1380
   catgaggtcg aactgaaaaa gatcctggaa aatgccaaaa agtactattc cttcctgacc 1440
   gagacagaca aggatgggta ctcagtggtc cagaaaatcg agagcatttt caggtttcgc 1500
   atcccctact atgtggggcc tctgagtacc cggcaccagg aaaagggatc aaacgtgtgg 1560
   atggtcagaa aacctggcag ggaggatcgc atctacccat ggaatatgga ggaaatcatt 1620
   gactttgaga agagcaacga aaatttcatt acacggatga ctaacaaatg tacatatctg 1680
   atcggggaag atgtcctgcc caagcattct ctgctgtaca gtaaatatat ggtgctgaat 1740
   gagctgaaca atgtgaaggt cagaggaaaa aagctgccta catctctgaa acagaaggtg 1800
   ttcgaggacc tgtttgaaaa caaatccaaa gtgactggaa agaatctgct ggagtacctg 1860
   cagatccagg acaaagatat ccagattgac gatctgtctg gcttcgacaa ggacttcaag 1920
   accagcctga agagctatct ggacttcaaa aagcagattt ttggggagga aatcgagaag 1980
   gaaagcattc agaacatgat cgaagatatc attaagtgga tcaccatcta cggcaatgac 2040
   aaggagatgc tgaaacgagt gattcgggct aattatagca accagctgac agaggaacag 2100
   atgaaaaaga tcactggatt tcagtacagt ggctggggga acttctcaaa gatgtttctg 2160
   aaagggatca gcggatccga cgtgagcacc ggcgaaacat tcgacatcat taccgcaatg 2220
   tgggagacag acaacaatct gatgcagatc ctgtcaaaaa agttcacctt tatggacaac 2280
   gtcgaggact tcaacagcgg caaggtcggg aaaatcgaca agattactta cgatagcacc 2340
   gtgaaggaaa tgttcctgtc ccctgagaac aaaagggccg tctggcagac cattcaggtg 2400
   gctgaggaga tcaagaaagt gatgggctgc gagccaaaaa agatctttat tgaaatggca 2460
   cggggcgggg agaaggtgaa aaagaggaca aaatctcgca aggcccagct gctggagctg 2520
   tacgccgctt gcgaggaaga ttgtagagaa ctgatcaagg agattgagga ccgggacgag 2580
   agggacttca atagcatgaa gctgtttctg tactataccc agttcgggaa atgtatgtat 2640
   tccggcgacg acatcgatat taacgagctg attcgcggca attctaagtg ggaccgagat 2700
   cacatctacc cccagagcaa aattaaggac gattccatcg ataacctggt gctggtcaat 2760
   aagacatata atgccaaaaa gtccaatgag ctgctgtctg aggacatcca gaaaaagatg 2820
   cattcattct ggctgagcct gctgaacaaa aagctgatca ctaaaagcaa gtacgaccgc 2880
   ctgactcgaa agggcgactt taccgatgag gaactgagtg ggttcatcgc tagacagctg 2940
   gtggaaacaa ggcagtcaac taaggcaatc gccgatatct tcaagcagat ctacagctcc 3000
   gaggtggtct atgtgaagag cagcctggtg agcgacttca ggaaaaagcc actgaactac 3060
   ctgaagtctc ggagagtcaa tgattaccac catgcaaaag acgcctatct gaacattgtg 3120
   gtcgggaacg tgtacaacaa aaagtttacc agtaatccca tccagtggat gaaaaagaat 3180
   cgcgatacaa actatagcct gaacaaggtg ttcgaacacg acgtggtcat taacggagaa 3240
   gtgatctggg aaaagtgcac ataccatgag gacactaata cctatgatgg aggcactctg 3300
   gaccgaatcc ggaagattgt ggaacgcgat aacattctgt acaccgagta cgcttattgt 3360
   gagaagggcg aactgtttaa tgcaaccatc cagaacaaaa atggaaactc cacagtctct 3420
   ctgaaaaagg gcctggacgt gaaaaagtac gggggatact tcagcgccaa cacaagttac 3480
   ttctcactga tcgagtttga ggacaagaag ggggatagag caaggcacat cattggagtg 3540
   cctatctata ttgcaaacat gctggagcat tctccaagtg ccttcctgga gtactgcgaa 3600
   cagaaggggt atcagaatgt gcggattctg gtcgagaaaa tcaaaaagaa cagcctgctg 3660
   atcattaatg gataccctct gcgcattcga ggcgagaacg aagtggatac ttcctttaag 3720
   agggccatcc agctgaagct ggaccagaaa aactatgagc tggtccgcaa tatcgagaag 3780
   ttcctggaaa aatacgtgga gaaaaaggga aactatccaa ttgacgagaa tagagatcac 3840
   atcacacatg aaaagatgaa ccagctgtac gaggtgctgc tgtccaaaat gaaaaagttc 3900
   aacaagaagg gcatggccga cccctctgat aggatcgaaa agagtaagcc taaattcatc 3960
   aagctggagg acctgatcga taagattaat gtgatcaaca aaatgctgaa cctgctgcgc 4020
   tgtgacaatg atactaaggc cgacctgtct ctgattgagc tgcccaaaaa cgctgggagt 4080
   ttcgtggtca aaaagaatac catcggaaag tcaaaaatca tcctggtgaa tcagagcgtg 4140
   actggactgt acgagaatag acgggaactg taagaattc 4179
<210> 173
   <211> 111
   <212> RNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, u, g, unknown or other
<400> 173
   nnnnnnnnnn nnnnnnnnnn guuuuagcac uagaaauagu uaaguuaaaa caagcuuaaa 60
   gcgucaaugu aauauuuuau uaacacccua cugugucagu gggguuuuuu u 111
<210> 174
   <211> 1334
   <212> PRT
   <213> Staphylococcus pseudintermedius
<400> 174
   Met Gly Arg Lys Pro Tyr Ile Leu Ser Leu Asp Ile Gly Thr Gly Ser
   1               5                   10                  15
   Val Gly Tyr Ala Cys Met Asp Lys Gly Phe Asn Val Leu Lys Tyr His
               20                  25                  30
   Asp Lys Asp Ala Leu Gly Val Tyr Leu Phe Asp Gly Ala Leu Thr Ala
           35                  40                  45
   Gln Glu Arg Arg Gln Phe Arg Thr Ser Arg Arg Arg Lys Asn Arg Arg
       50                  55                  60
   Ile Lys Arg Leu Gly Leu Leu Gln Glu Leu Leu Ala Pro Leu Val Gln
   65                  70                  75                  80
   Asn Pro Asn Phe Tyr Gln Phe Gln Arg Gln Phe Ala Trp Lys Asn Asp
                   85                  90                  95
   Asn Met Asp Phe Lys Asn Lys Ser Leu Ser Glu Val Leu Ser Phe Leu
               100                 105                 110
   Gly Tyr Glu Ser Lys Lys Tyr Pro Thr Ile Tyr His Leu Gln Glu Ala
           115                 120                 125
   Leu Leu Leu Lys Asp Glu Lys Phe Asp Pro Glu Leu Ile Tyr Met Ala
       130                 135                 140
   Leu Tyr His Leu Val Lys Tyr Arg Gly His Phe Leu Phe Asp His Leu
   145                 150                 155                 160
   Lys Ile Glu Asn Leu Thr Asn Asn Asp Asn Met His Asp Phe Val Glu
                   165                 170                 175
   Leu Ile Glu Thr Tyr Glu Asn Leu Asn Asn Ile Lys Leu Asn Leu Asp
               180                 185                 190
   Tyr Glu Lys Thr Lys Val Ile Tyr Glu Ile Leu Lys Asp Asn Glu Met
           195                 200                 205
   Thr Lys Asn Asp Arg Ala Lys Arg Val Lys Asn Met Glu Lys Lys Leu
       210                 215                 220
   Glu Gln Phe Ser Ile Met Leu Leu Gly Leu Lys Phe Asn Glu Gly Lys
   225                 230                 235                 240
   Leu Phe Asn His Ala Asp Asn Ala Glu Glu Leu Lys Gly Ala Asn Gln
                   245                 250                 255
   Ser His Thr Phe Ala Asp Asn Tyr Glu Glu Asn Leu Thr Pro Phe Leu
               260                 265                 270
   Thr Val Glu Gln Ser Glu Phe Ile Glu Arg Ala Asn Lys Ile Tyr Leu
           275                 280                 285
   Ser Leu Thr Leu Gln Asp Ile Leu Lys Gly Lys Lys Ser Met Ala Met
       290                 295                 300
   Ser Lys Val Ala Ala Tyr Asp Lys Phe Arg Asn Glu Leu Lys Gln Val
   305                 310                 315                 320
   Lys Asp Ile Val Tyr Lys Ala Asp Ser Thr Arg Thr Gln Phe Lys Lys
                   325                 330                 335
   Ile Phe Val Ser Ser Lys Lys Ser Leu Lys Gln Tyr Asp Ala Thr Pro
               340                 345                 350
   Asn Asp Gln Thr Phe Ser Ser Leu Cys Leu Phe Asp Gln Tyr Leu Ile
           355                 360                 365
   Arg Pro Lys Lys Gln Tyr Ser Leu Leu Ile Lys Glu Leu Lys Lys Ile
       370                 375                 380
   Ile Pro Gln Asp Ser Glu Leu Tyr Phe Glu Ala Glu Asn Asp Thr Leu
   385                 390                 395                 400
   Leu Lys Val Leu Asn Thr Thr Asp Asn Ala Ser Ile Pro Met Gln Ile
                   405                 410                 415
   Asn Leu Tyr Glu Ala Glu Thr Ile Leu Arg Asn Gln Gln Lys Tyr His
               420                 425                 430
   Ala Glu Ile Thr Asp Glu Met Ile Glu Lys Val Leu Ser Leu Ile Gln
           435                 440                 445
   Phe Arg Ile Pro Tyr Tyr Val Gly Pro Leu Val Asn Asp His Thr Ala
       450                 455                 460
   Ser Lys Phe Gly Trp Met Glu Arg Lys Ser Asn Glu Ser Ile Lys Pro
   465                 470                 475                 480
   Trp Asn Phe Asp Glu Val Val Asp Arg Ser Lys Ser Ala Thr Gln Phe
                   485                 490                 495
   Ile Arg Arg Met Thr Asn Lys Cys Ser Tyr Leu Ile Asn Glu Asp Val
               500                 505                 510
   Leu Pro Lys Asn Ser Leu Leu Tyr Gln Glu Met Glu Val Leu Asn Glu
           515                 520                 525
   Leu Asn Ala Thr Gln Ile Arg Leu Gln Thr Asp Pro Lys Asn Arg Lys
       530                 535                 540
   Tyr Arg Met Met Pro Gln Ile Lys Leu Phe Ala Val Glu His Ile Phe
   545                 550                 555                 560
   Lys Lys Tyr Lys Thr Val Ser His Ser Lys Phe Leu Glu Ile Met Leu
                   565                 570                 575
   Asn Ser Asn His Arg Glu Asn Phe Met Asn His Gly Glu Lys Leu Ser
               580                 585                 590
   Ile Phe Gly Thr Gln Asp Asp Lys Lys Phe Ala Ser Lys Leu Ser Ser
           595                 600                 605
   Tyr Gln Asp Met Thr Lys Ile Phe Gly Asp Ile Glu Gly Lys Arg Ala
   610 615 620
   Gln Ile Glu Glu Ile Ile Gln Trp Ile Thr Ile Phe Glu Asp Lys Lys
   625 630 635 640
   Ile Leu Val Gln Lys Leu Lys Glu Cys Tyr Pro Glu Leu Thr Ser Lys
   645 650 655
   Gln Ile Asn Gln Leu Lys Lys Leu Asn Tyr Ser Gly Trp Gly Arg Leu
   660 665 670
   Ser Glu Lys Leu Leu Thr His Ala Tyr Gln Gly His Ser Ile Ile Glu
   675 680 685
   Leu Leu Arg His Ser Asp Glu Asn Phe Met Glu Ile Leu Thr Asn Asp
   690 695 700
   Val Tyr Gly Phe Gln Asn Phe Ile Lys Glu Glu Asn Gln Val Gln Ser
   705 710 715 720
   Asn Lys Ile Gln His Gln Asp Ile Ala Asn Leu Thr Thr Ser Pro Ala
   725 730 735
   Leu Lys Lys Gly Ile Trp Ser Thr Ile Lys Leu Val Arg Glu Leu Thr
   740 745 750
   Ser Ile Phe Gly Glu Pro Glu Lys Ile Ile Met Glu Phe Ala Thr Glu
   755 760 765
   Asp Gln Gln Lys Gly Lys Lys Gln Lys Ser Arg Lys Gln Leu Trp Asp
   770 775 780
   Asp Asn Ile Lys Lys Asn Lys Leu Lys Ser Val Asp Glu Tyr Lys Tyr
   785 790 795 800
   Ile Ile Asp Val Ala Asn Lys Leu Asn Asn Glu Gln Leu Gln Gln Glu
   805 810 815
   Lys Leu Trp Leu Tyr Leu Ser Gln Asn Gly Lys Cys Met Tyr Ser Gly
   820 825 830
   Gln Ser Ile Asp Leu Asp Ala Leu Leu Ser Pro Asn Ala Thr Lys His
   835 840 845
   Tyr Glu Val Asp His Ile Phe Pro Arg Ser Phe Ile Lys Asp Asp Ser
   850 855 860
   Ile Asp Asn Lys Val Leu Val Ile Lys Lys Met Asn Gln Thr Lys Gly
   865 870 875 880
   Asp Gln Val Pro Leu Gln Phe Ile Gln Gln Pro Tyr Glu Arg Ile Ala
   885 890 895
   Tyr Trp Lys Ser Leu Asn Lys Ala Gly Leu Ile Ser Asp Ser Lys Leu
   900 905 910
   His Lys Leu Met Lys Pro Glu Phe Thr Ala Met Asp Lys Glu Gly Phe
   915 920 925
   Ile Gln Arg Gln Leu Val Glu Thr Arg Gln Ile Ser Val His Val Arg
   930 935 940
   Asp Phe Leu Lys Glu Glu Tyr Pro Asn Thr Lys Val Ile Pro Met Lys
   945 950 955 960
   Ala Lys Met Val Ser Glu Phe Arg Lys Lys Phe Asp Ile Pro Lys Ile
   965 970 975
   Arg Gln Met Asn Asp Ala His His Ala Ile Asp Ala Tyr Leu Asn Gly
   980 985 990
   Val Val Tyr His Gly Ala Gln Leu Ala Tyr Pro Asn Val Asp Leu Phe
   995 1000 1005
   Asp Phe Asn Phe Lys Trp Glu Lys Val Arg Glu Lys Trp Lys Ala
   1010 1015 1020
   Leu Gly Glu Phe Asn Thr Lys Gln Lys Ser Arg Glu Leu Phe Phe
   1025 1030 1035
   Phe Lys Lys Leu Glu Lys Met Glu Val Ser Gln Gly Glu Arg Leu
   1040 1045 1050
   Ile Ser Lys Ile Lys Leu Asp Met Asn His Phe Lys Ile Asn Tyr
   1055 1060 1065
   Ser Arg Lys Leu Ala Asn Ile Pro Gln Gln Phe Tyr Asn Gln Thr
   1070 1075 1080
   Ala Val Ser Pro Lys Thr Ala Glu Leu Lys Tyr Glu Ser Asn Lys
   1085 1090 1095
   Ser Asn  Glu Val Val Tyr Lys  Gly Leu Thr Pro Tyr  Gln Thr Tyr
       1100                 1105                 1110
   Val Val  Ala Ile Lys Ser Val  Asn Lys Lys Gly Lys  Glu Lys Met
       1115                 1120                 1125
   Glu Tyr  Gln Met Ile Asp His  Tyr Val Phe Asp Phe  Tyr Lys Phe
       1130                 1135                 1140
   Gln Asn  Gly Asn Glu Lys Glu  Leu Ala Leu Tyr Leu  Ala Gln Arg
       1145                 1150                 1155
   Glu Asn  Lys Asp Glu Val Leu  Asp Ala Gln Ile Val  Tyr Ser Leu
       1160                 1165                 1170
   Asn Lys  Gly Asp Leu Leu Tyr  Ile Asn Asn His Pro  Cys Tyr Phe
       1175                 1180                 1185
   Val Ser  Arg Lys Glu Val Ile  Asn Ala Lys Gln Phe  Glu Leu Thr
       1190                 1195                 1200
   Val Glu  Gln Gln Leu Ser Leu  Tyr Asn Val Met Asn  Asn Lys Glu
       1205                 1210                 1215
   Thr Asn  Val Glu Lys Leu Leu  Ile Glu Tyr Asp Phe  Ile Ala Glu
       1220                 1225                 1230
   Lys Val  Ile Asn Glu Tyr His  His Tyr Leu Asn Ser  Lys Leu Lys
       1235                 1240                 1245
   Glu Lys  Arg Val Arg Thr Phe  Phe Ser Glu Ser Asn  Gln Thr His
       1250                 1255                 1260
   Glu Asp  Phe Ile Lys Ala Leu  Asp Glu Leu Phe Lys  Val Val Thr
       1265                 1270                 1275
   Ala Ser  Ala Thr Arg Ser Asp  Lys Ile Gly Ser Arg  Lys Asn Ser
       1280                 1285                 1290
   Met Thr  His Arg Ala Phe Leu  Gly Lys Gly Lys Asp  Val Lys Ile
       1295                 1300                 1305
   Ala Tyr  Thr Ser Ile Ser Gly  Leu Lys Thr Thr Lys  Pro Lys Ser
       1310                 1315                 1320
   Leu Phe  Lys Leu Ala Glu Ser  Arg Asn Glu Leu
       1325                 1330
<210> 175
   <211> 4086
   <212> DNA
   <213> Artificial Sequence
<220>
   <221> source
   <223> /note="Description of Artificial Sequence: Synthetic polynucleotide"
<400> 175
   accggtgcca ccatgtaccc atacgatgtt ccagattacg cttcgccgaa gaaaaagcgc 60
   aaggtcgaag cgtccatggg gaggaaacct tacattctgt ctctggatat tggaactggg 120
   tccgtcggct acgcttgcat ggataaagga ttcaacgtgc tgaagtacca cgacaaagat 180
   gccctgggag tgtatctgtt cgacggcgct ctgactgcac aggagcggag acagtttagg 240
   acctccaggc gccgaaagaa ccggagaatc aaacgcctgg gcctgctgca ggaactgctg 300
   gcacccctgg tgcagaaccc taatttctac cagtttcagc ggcagttcgc ctggaagaac 360
   gacaatatgg attttaagaa caagagcctg tctgaggtgc tgagcttcct gggatatgaa 420
   tccaagaaat accctaccat ctaccacctg caggaggctc tgctgctgaa agacgagaag 480
   tttgatccag aactgatcta catggcactg tatcatctgg tgaaatacag aggccacttt 540
   ctgttcgatc atctgaagat cgagaacctg actaacaatg acaatatgca cgatttcgtg 600
   gagctgattg aaacctatga gaacctgaac aatatcaagc tgaatctgga ctacgagaaa 660
   accaaagtga tctatgagat tctgaaagac aacgaaatga ctaagaatga tagagccaaa 720
   agggtcaaga acatggagaa gaaactggaa cagttctcta tcatgctgct ggggctgaag 780
   ttcaatgagg gaaaactgtt taaccacgcc gataatgctg aggaactgaa gggggctaac 840
   cagagccata catttgcaga caactacgag gaaaatctga ctcccttcct gaccgtggaa 900
   cagtcagagt ttattgaaag ggccaacaaa atctatctga gcctgactct gcaggatatc 960
   ctgaagggca agaaatcaat ggctatgagc aaagtggccg cttacgacaa gttcagaaat 1020
   gagctgaaac aggtgaagga cattgtctat aaggctgatt ctaccaggac acagttcaag 1080
   aaaatctttg tgagctccaa gaaaagtctg aagcagtacg acgcaactcc caacgatcag 1140
   accttctcta gtctgtgcct gtttgaccag tacctgattc gcccaaagaa acagtatagc 1200
   ctgctgatca aggagctgaa gaaaatcatt ccccaggact ccgaactgta ctttgaggca 1260
   gaaaatgata ccctgctgaa ggtgctgaac accacagaca atgctagcat ccctatgcag 1320
   attaacctgt acgaggcaga aaccatcctg cgaaatcagc agaaatatca cgccgagatc 1380
   acagatgaga tgattgaaaa ggtgctgtct ctgatccagt tccgcattcc atactatgtg 1440
   gggcccctgg tcaacgacca tacagccagt aagtttggat ggatggagcg caaaagtaac 1500
   gaatcaatca agccttggaa tttcgacgag gtggtcgatc gaagtaaatc agccactcag 1560
   tttattaggc gcatgaccaa caagtgttcc tacctgatca atgaggatgt gctgccaaaa 1620
   aactctctgc tgtatcagga gatggaagtc ctgaacgaac tgaatgccac acagatcagg 1680
   ctgcagactg acccaaaaaa ccgcaagtac cgaatgatgc cccagattaa gctgttcgct 1740
   gtggagcaca tctttaagaa atataaaacc gtcagccatt ccaagttcct ggaaattatg 1800
   ctgaacagca atcacaggga gaactttatg aatcatggag aaaagctgag tatcttcggc 1860
   acacaggacg ataagaaatt tgcatcaaag ctgtcaagct accaggacat gactaaaatc 1920
   ttcggggata ttgagggaaa gcgcgcccag attgaggaaa tcattcagtg gatcaccatt 1980
   tttgaggaca agaaaatcct ggtgcagaag ctgaaagagt gctatcctga actgacatcc 2040
   aagcagatca accagctgaa gaaactgaat tactctggct gggggaggct gagtgagaag 2100
   ctgctgactc acgcctatca gggccatagc atcattgaac tgctgcgcca ctccgatgag 2160
   aatttcatgg aaattctgac caacgacgtg tacgggttcc agaattttat caaagaggaa 2220
   aaccaggtcc agagcaataa gatccagcat caggatattg ccaacctgac tacctctccc 2280
   gctctgaaga aaggcatctg gagtacaatt aagctggtgc gggagctgac ttccattttc 2340
   ggggagcctg aaaagatcat tatggagttt gctaccgagg accagcagaa aggcaagaaa 2400
   cagaaatcaa gaaagcagct gtgggacgat aacatcaaga aaaataagct gaaaagcgtg 2460
   gacgagtaca aatatatcat tgatgtcgcc aataagctga acaatgagca gctgcagcag 2520
   gaaaaactgt ggctgtacct gagccagaac ggcaagtgta tgtatagcgg gcagtccatc 2580
   gacctggatg ccctgctgtc ccccaatgct accaagcact acgaggtgga tcatattttc 2640
   cctcggagct tcatcaagga cgatagcatt gacaacaagg tgctggtcat caagaaaatg 2700
   aatcagacaa agggcgatca ggtgcccctg cagttcattc agcagcctta cgagagaatc 2760
   gcatattgga agagcctgaa caaagccggg ctgatctctg atagtaaact gcacaagctg 2820
   atgaaaccag agttcacagc tatggacaag gaaggcttca tccagcggca gctggtggag 2880
   actagacaga tcagcgtgca tgtccgggat tttctgaaag aggaataccc taataccaaa 2940
   gtgatcccaa tgaaggccaa aatggtgagc gagttccgga agaaatttga catcccaaag 3000
   attagacaga tgaacgacgc acaccatgcc atcgatgctt acctgaatgg cgtggtctat 3060
   cacggggcac agctggccta ccccaacgtg gacctgtttg atttcaattt taagtgggag 3120
   aaagtccgag aaaagtggaa agccctggga gagttcaaca caaagcagaa atctcgggaa 3180
   ctgttctttt tcaagaaact ggagaagatg gaagtgtccc agggcgagcg gctgatctct 3240
   aagatcaagc tggacatgaa ccacttcaag atcaactact ccagaaagct ggccaacatc 3300
   cctcagcagt tttataatca gaccgcagtg tctccaaaga cagccgagct gaaatacgaa 3360
   tctaacaaga gtaatgaggt ggtctataag ggactgacac cataccagac ttatgtggtc 3420
   gccatcaaga gcgtgaacaa gaaaggcaag gagaaaatgg aataccagat gatcgaccac 3480
   tacgtgttcg atttttataa attccagaac ggcaatgaga aggaactggc tctgtacctg 3540
   gcacagaggg agaacaagga cgaagtgctg gatgctcaga ttgtctatag tctgaataag 3600
   ggggatctgc tgtacatcaa caatcatccc tgctatttcg tgtcacgcaa agaggtcatc 3660
   aacgcaaagc agtttgagct gaccgtggaa cagcagctgt ctctgtacaa cgtgatgaac 3720
   aacaaggaga caaatgtcga aaagctgctg atcgagtatg acttcattgc cgagaaagtg 3780
   atcaacgaat accaccatta tctgaatagc aagctgaaag aaaagcgagt ccggaccttt 3840
   ttctcagaga gcaaccagac acacgaggac ttcatcaagg ccctggacga gctgtttaag 3900
   gtggtcaccg catccgccac aaggtctgat aaaatcggga gtcgcaagaa cagcatgact 3960
   catcgagcct tcctgggaaa aggcaaggac gtgaagattg cttacacctc catctctgga 4020
   ctgaaaacaa ctaaacctaa gagtctgttt aagctggccg agtcaagaaa cgaactgtaa 4080
   gaattc 4086
<210> 176
   <211> 114
   <212> RNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, u, g, unknown or other
<400> 176
   nnnnnnnnnn nnnnnnnnnn guuuuagaug gugaaaacca gauuuaaaau caagcaaugc 60
   aucuuuugau gcaaaguuuc aauauuuguc ccacguuauc gagggacuuu uuuu 114
<210> 177
   <211> 1375
   <212> PRT
   <213> Lactobacillus johnsonii
<400> 177
   Met Thr Lys Ile Lys Asp Asp Tyr Ile Val Gly Leu Asp Ile Gly Thr
   1               5                   10                  15
   Asp Ser Cys Gly Trp Val Ala Met Asn Ser Asn Asn Asp Ile Leu Lys
               20                  25                  30
   Leu Gln Gly Lys Thr Ala Ile Gly Ser Arg Leu Phe Glu Gly Gly Lys
           35                  40                  45
   Ser Ala Ala Glu Arg Arg Leu Phe Arg Thr Thr His Arg Arg Ile Lys
       50                  55                  60
   Arg Arg Arg Trp Arg Leu Lys Leu Leu Glu Glu Phe Phe Asp Pro Tyr
   65                  70                  75                  80
   Met Ala Glu Val Asp Pro Tyr Phe Phe Ala Arg Leu Lys Glu Ser Gly
                   85                  90                  95
   Leu Ser Pro Leu Asp Lys Arg Lys Thr Val Ser Ser Ile Val Phe Pro
               100                 105                 110
   Thr Ser Ala Glu Asp Lys Lys Phe Tyr Asp Asp Tyr Pro Thr Ile Tyr
           115                 120                 125
   His Leu Arg Tyr Lys Leu Met Thr Glu Asp Glu Lys Phe Asp Leu Arg
       130                 135                 140
   Glu Val Tyr Leu Ala Ile His His Ile Ile Lys Tyr Arg Gly Asn Phe
   145                 150                 155                 160
   Leu Tyr Asn Thr Ser Val Lys Asp Phe Lys Ala Ser Lys Ile Asp Val
                   165                 170                 175
   Lys Ser Ser Ile Glu Lys Leu Asn Glu Leu Tyr Glu Asn Leu Gly Leu
               180                 185                 190
   Asp Leu Asn Val Glu Phe Asn Ile Ser Asn Thr Ala Glu Ile Glu Lys
           195                 200                 205
   Val Leu Lys Asp Lys Gln Ile Phe Lys Arg Asp Lys Val Lys Lys Ile
       210                 215                 220
   Ala Glu Leu Phe Ala Ile Lys Thr Asp Asn Lys Glu Gln Ser Lys Arg
   225                 230                 235                 240
   Ile Lys Asp Ile Ser Lys Gln Val Ala Asn Ala Val Leu Gly Tyr Lys
                   245                 250                 255
   Thr Arg Phe Asp Thr Ile Ala Leu Lys Glu Ile Ser Lys Asp Glu Leu
               260                 265                 270
   Ser Asp Trp Asn Phe Lys Leu Ser Asp Ile Asp Ala Asp Ser Lys Phe
           275                 280                 285
   Glu Ala Leu Met Gly Asn Leu Asp Glu Asn Glu Gln Ala Ile Leu Leu
   290 295 300
   Thr Ile Lys Glu Leu Phe Asn Glu Val Thr Leu Asn Gly Ile Val Glu
   305 310 315 320
   Asp Gly Asn Thr Leu Ser Glu Ser Met Ile Asn Lys Tyr Asn Asp His
   325 330 335
   Arg Asp Asp Leu Lys Leu Leu Lys Glu Val Ile Glu Asn His Ile Asp
   340 345 350
   Arg Lys Lys Ala Lys Glu Leu Ala Leu Ala Tyr Asp Leu Tyr Val Asn
   355 360 365
   Asn Arg His Gly Gln Leu Leu Gln Ala Lys Lys Lys Leu Gly Lys Ile
   370 375 380
   Lys Pro Arg Ser Lys Glu Asp Phe Tyr Lys Val Val Asn Lys Asn Leu
   385 390 395 400
   Asp Asp Ser Arg Ala Ser Lys Glu Ile Lys Lys Lys Ile Glu Leu Asp
   405 410 415
   Ser Phe Met Pro Lys Gln Arg Thr Asn Ala Asn Gly Val Ile Pro Tyr
   420 425 430
   Gln Leu Gln Gln Leu Glu Leu Asp Lys Ile Ile Glu Asn Gln Ser Lys
   435 440 445
   Tyr Tyr Pro Phe Leu Lys Glu Ile Asn Pro Val Ser Ser His Leu Lys
   450 455 460
   Glu Ala Pro Tyr Lys Leu Asp Glu Leu Ile Arg Phe Arg Val Pro Tyr
   465 470 475 480
   Tyr Val Gly Pro Leu Ile Ser Pro Asn Glu Ser Thr Lys Asp Ile Gln
   485 490 495
   Thr Lys Lys Asn Gln Asn Phe Ala Trp Met Ile Arg Lys Glu Glu Gly
   500 505 510
   Arg Ile Thr Pro Trp Asn Phe Asp Gln Lys Val Asp Arg Ile Glu Ser
   515 520 525
   Ala Asn Lys Phe Ile Lys Arg Met Thr Thr Lys Asp Thr Tyr Leu Phe
   530 535 540
   Gly Glu Asp Val Leu Pro Ala Asn Ser Leu Leu Tyr Gln Lys Phe Thr
   545                 550                 555                 560
   Val Leu Asn Glu Leu Asn Asn Ile Arg Ile Asn Gly Lys Arg Ile Ser
                   565                 570                 575
   Val Asp Leu Lys Gln Glu Ile Tyr Glu Asn Leu Phe Lys Lys His Thr
               580                 585                 590
   Thr Val Thr Val Lys Lys Leu Glu Asn Tyr Leu Lys Glu Asn His Asn
           595                 600                 605
   Leu Val Lys Val Glu Ile Lys Gly Leu Ala Asp Glu Lys Lys Phe Asn
       610                 615                 620
   Ser Gly Leu Thr Thr Tyr Asn Arg Phe Lys Asn Leu Asn Ile Phe Asp
   625                 630                 635                 640
   Asn Gln Ile Asp Asp Leu Lys Tyr Arg Asn Asp Phe Glu Lys Ile Ile
                   645                 650                 655
   Glu Trp Ser Thr Ile Phe Glu Asp Lys Ser Ile Tyr Lys Glu Lys Leu
               660                 665                 670
   Arg Ser Ile Asp Trp Leu Asn Glu Lys Gln Ile Asn Ala Leu Ser Asn
           675                 680                 685
   Ile Arg Leu Gln Gly Trp Gly Arg Leu Ser Lys Lys Leu Leu Ala Gln
       690                 695                 700
   Leu His Asp His Asn Gly Gln Thr Ile Ile Glu Gln Leu Trp Asp Ser
   705                 710                 715                 720
   Gln Asn Asn Phe Met Gln Ile Val Thr Gln Ala Asp Phe Lys Asp Ala
                   725                 730                 735
   Ile Ala Lys Ala Asn Gln Asn Leu Leu Val Ala Thr Ser Val Glu Asp
               740                 745                 750
   Ile Leu Asn Asn Ala Tyr Thr Ser Pro Ala Asn Lys Lys Ala Ile Arg
           755                 760                 765
   Gln Val Ile Lys Val Val Asp Asp Ile Val Lys Ala Ala Ser Gly Lys
       770                 775                 780
   Val Pro Lys Gln Ile Ala Ile Glu Phe Thr Arg Asp Ala Asp Glu Asn
   785                 790                 795                 800
   Pro Lys Arg Ser Gln Thr Arg Gly Ser Lys Leu Gln Lys Val Tyr Lys
   805 810 815
   Asp Leu Ser Thr Glu Leu Ala Ser Lys Thr Ile Ala Glu Glu Leu Asn
   820 825 830
   Glu Ala Ile Lys Asp Lys Lys Leu Val Gln Asp Lys Tyr Tyr Leu Tyr
   835 840 845
   Phe Met Gln Leu Gly Arg Asp Ala Tyr Thr Gly Glu Pro Ile Asn Ile
   850 855 860
   Asp Glu Ile Gln Lys Tyr Asp Ile Asp His Ile Leu Pro Gln Ser Phe
   865 870 875 880
   Ile Lys Asp Asp Ala Leu Asp Asn Arg Val Leu Val Ser Arg Ala Val
   885 890 895
   Asn Asn Gly Lys Ser Asp Asn Val Pro Val Lys Leu Phe Gly Asn Glu
   900 905 910
   Met Ala Ala Asn Leu Gly Met Thr Ile Arg Lys Met Trp Glu Glu Trp
   915 920 925
   Lys Asn Ile Gly Leu Ile Ser Lys Thr Lys Tyr Asn Asn Leu Leu Thr
   930 935 940
   Asp Pro Asp His Ile Asn Lys Tyr Lys Ser Ala Gly Phe Ile Arg Arg
   945 950 955 960
   Gln Leu Val Glu Thr Ser Gln Ile Ile Lys Leu Val Ser Thr Ile Leu
   965 970 975
   Gln Ser Arg Tyr Pro Asn Thr Glu Ile Ile Thr Val Lys Ala Lys Tyr
   980 985 990
   Asn His Tyr Leu Arg Glu Lys Phe Asp Leu Tyr Lys Ser Arg Glu Val
   995 1000 1005
   Asn Asp Tyr His His Ala Ile Asp Ala Tyr Leu Ser Ala Ile Cys
   1010 1015 1020
   Gly Asn Leu Leu Tyr Gln Asn Tyr Pro Asn Leu Arg Pro Phe Phe
   1025 1030 1035
   Val Tyr Gly Gln Tyr Lys Lys Phe Ser Ser Asp Pro Asp Lys Glu
   1040 1045 1050
   Lys Ala Ile Phe Asn Lys Thr Arg Lys Phe Ser Phe Ile Ser Gln
   1055 1060 1065
   Leu Leu Lys Asn Lys Ser Glu Asn Ser Lys Glu Ile Ala Lys Lys
   1070 1075 1080
   Leu Lys Arg Ala Tyr Gln Phe Lys Tyr Met Leu Val Ser Arg Glu
   1085 1090 1095
   Thr Glu Thr Arg Asp Gln Glu Met Phe Lys Met Thr Val Tyr Pro
   1100 1105 1110
   Arg Phe Ser His Asp Thr Val Lys Ala Pro Arg Asn Leu Ile Pro
   1115 1120 1125
   Lys Lys Met Gly Met Ser Pro Asp Ile Tyr Gly Gly Tyr Thr Asn
   1130 1135 1140
   Asn Ser Asp Ala Tyr Met Val Ile Val Arg Ile Asp Lys Lys Lys
   1145 1150 1155
   Gly Thr Glu Tyr Lys Ile Leu Gly Ile Pro Thr Arg Glu Leu Val
   1160 1165 1170
   Asn Leu Lys Lys Ala Glu Lys Glu Asp His Tyr Lys Ser Tyr Leu
   1175 1180 1185
   Lys Glu Ile Leu Thr Pro Arg Ile Leu Tyr Asn Lys Asn Gly Lys
   1190 1195 1200
   Arg Asp Lys Lys Ile Thr Ser Phe Glu Ile Val Lys Ser Lys Ile
   1205 1210 1215
   Pro Tyr Lys Gln Val Ile Gln Asp Gly Asp Lys Lys Phe Met Leu
   1220 1225 1230
   Gly Ser Ser Thr Tyr Val Tyr Asn Ala Lys Gln Leu Thr Leu Ser
   1235 1240 1245
   Thr Glu Ser Met Lys Ala Ile Thr Asn Asn Phe Asp Lys Asp Ser
   1250 1255 1260
   Asp Glu Asn Asp Ala Leu Ile Lys Ala Tyr Asp Glu Ile Leu Asp
   1265 1270 1275
   Lys Val Asp Lys Tyr Leu Pro Leu Phe Asp Ile Asn Lys Phe Arg
   1280 1285 1290
   Glu Lys Leu His Ser Gly Arg Glu Lys Phe Ile Lys Leu Ser Leu
   1295 1300 1305
   Glu Asp Lys Lys Asp Thr Ile Leu Lys Val Leu Glu Gly Leu His
   1310 1315 1320
   Asp Asn Ala Val Met Thr Lys Ile Pro Thr Ile Gly Leu Ser Thr
   1325 1330 1335
   Pro Leu Gly Phe Met Gln Phe Pro Asn Gly Val Ile Leu Ser Glu
   1340 1345 1350
   Asn Ala Lys Leu Ile Tyr Gln Ser Pro Thr Gly Leu Phe Lys Lys
   1355 1360 1365
   Ser Val Lys Ile Ser Asp Leu
   1370 1375
<210> 178
   <211> 4209
   <212> DNA
   <213> Artificial Sequence
<220>
   <221> source
   <223> /note="Description of Artificial Sequence: Synthetic polynucleotide"
<400> 178
   accggtgcca ccatgtaccc atacgatgtt ccagattacg cttcgccgaa gaaaaagcgc      60
   aaggtcgaag cgtccatgac aaaaatcaaa gacgactaca tcgtgggact ggacatcggc     120
   acagactcct gcgggtgggt ggctatgaac agcaataatg acattctgaa actgcagggc     180
   aagaccgcaa tcgggtcacg cctgttcgag ggagggaaga gcgcagctga acggagactg     240
   tttcgcacca cacacaggcg catcaaacga cggagatggc gactgaagct gctggaggag     300
   ttcttcgacc cctacatggc agaggtggat ccttatttct ttgcccggct gaaggaatct     360
   ggcctgagtc cactggacaa aagaaagacc gtgagctcca ttgtgttccc cacatccgcc     420
   gaggataaga agttctacga cgattaccct acaatctacc atctgaggta taaactgatg     480
   actgaggacg aaaagttcga tctgcgcgaa gtgtacctgg ctatccacca tatcattaag     540
   taccgaggaa acttcctgta taataccagt gtgaaagact tcaaggcatc aaagatcgat     600
   gtcaaatcta gtatcgagaa gctgaacgag ctgtatgaaa atctgggcct ggacctgaac     660
   gtggagttca acattagcaa tactgccgag atcgaaaagg tgctgaaaga caagcagatc     720
   ttcaagcggg ataaagtcaa gaaaattgcc gagctgtttg ctatcaaaac cgacaacaag     780
   gaacagagca agagaatcaa agatatttcc aaacaggtgg ccaatgctgt cctggggtac 840
   aagaccaggt tcgacacaat cgctctgaaa gagatttcca aggacgaact gtctgattgg 900
   aacttcaaac tgtcagacat cgatgcagac agcaagtttg aggccctgat gggaaacctg 960
   gatgagaatg aacaggccat cctgctgact attaaggagc tgtttaacga agtgaccctg 1020
   aatggaattg tcgaggacgg caacaccctg agcgaatcca tgatcaacaa gtacaatgat 1080
   caccgggacg atctgaagct gctgaaagaa gtgatcgaaa atcatattga cagaaagaaa 1140
   gccaaggagc tggcactggc ctacgatctg tatgtcaaca ataggcacgg acagctgctg 1200
   caggctaaga aaaagctggg caaaatcaag ccccgctcta aggaggactt ctacaaagtg 1260
   gtcaacaaga atctggacga ttcacgggca agcaaggaga tcaaaaagaa aattgaactg 1320
   gacagcttta tgcctaagca gagaaccaac gccaatggcg tgatcccata ccagctgcag 1380
   cagctggagc tggataagat catcgaaaac cagtctaagt actatccatt cctgaaggag 1440
   attaatcccg tgtcaagcca cctgaaagag gccccctata agctggacga actgatccga 1500
   tttcgggtgc cttactatgt cggccccctg atttctccta acgagagtac caaggatatc 1560
   cagacaaaga aaaaccagaa tttcgcctgg atgattcgca aagaggaagg gcgaatcaca 1620
   ccttggaact ttgaccagaa ggtggatcga attgagagcg ccaataagtt catcaaacgg 1680
   atgactacca aggacactta cctgtttggg gaggatgtgc tgccagctaa cagcctgctg 1740
   tatcagaagt tcaccgtcct gaacgaactg aacaacatcc ggattaatgg aaaaagaatc 1800
   tccgtggacc tgaagcagga gatctacgaa aacctgttta agaaacacac aactgtgacc 1860
   gtcaagaaac tggagaatta tctgaaggaa aaccataatc tggtgaaagt cgagatcaag 1920
   gggctggccg atgaaaagaa attcaacagc ggactgacca catacaatag attcaagaac 1980
   ctgaacatct ttgacaacca gattgacgat ctgaagtaca ggaacgattt cgagaagatc 2040
   atcgaatggt ctacaatttt tgaggacaag agtatctaca aagaaaagct gaggagcatc 2100
   gattggctga acgagaagca gattaacgct ctgtctaata tcagactgca ggggtgggga 2160
   aggctgagta agaaactgct ggcacagctg cacgaccata atggccagac catcattgag 2220
   cagctgtggg attcccagaa caatttcatg cagattgtga cacaggccga ctttaaagat 2280
   gctatcgcaa aggccaacca gaatctgctg gtggctacct cagtcgagga cattctgaac 2340
   aatgcataca caagccccgc aaacaagaaa gccatcagac aggtcatcaa ggtggtcgac 2400
   gatatcgtga aggcagcctc cggaaaggtc ccaaaacaga tcgccattga gttcactagg 2460
   gatgctgacg aaaatcccaa gagaagtcag accaggggct caaagctgca gaaagtgtac 2520
   aaggacctga gcactgagct ggcctccaag accattgctg aggaactgaa cgaagcaatc 2580
   aaagacaaga aactggtgca ggataagtac tatctgtact ttatgcagct ggggcgggac 2640
   gcctatacag gagagcctat caatatcgat gaaatccaga agtacgatat cgaccacatt 2700
   ctgccacagt ctttcatcaa ggacgatgcc ctggacaaca gggtgctggt gagccgggct 2760
   gtgaacaatg gcaaatctga taatgtgcct gtcaagctgt ttggcaacga gatggctgca 2820
   aatctgggga tgactatcag gaaaatgtgg gaggaatgga agaacatcgg cctgattagc 2880
   aaaacaaagt acaacaatct gctgactgat cccgaccaca ttaacaagta taagagtgcc 2940
   gggttcatca ggcgccagct ggtggagaca tcacagatca tcaagctggt gagcactatc 3000
   ctgcagagtc gctaccctaa cactgaaatc attaccgtga aggctaagta caatcattat 3060
   ctgcgggaga aatttgacct gtataagagc agagaagtca acgactacca ccatgctatt 3120
   gatgcatatc tgtccgccat ctgcggaaat ctgctgtacc agaactatcc aaatctgcgg 3180
   cccttctttg tgtacggcca gtataagaaa ttctcctctg atcctgacaa agagaaggcc 3240
   atttttaaca aaacccgcaa gttctccttt atctctcagc tgctgaaaaa caagagtgag 3300
   aacagcaagg aaatcgctaa gaaactgaaa cgggcatacc agttcaagta tatgctggtg 3360
   tctcgagaga ctgaaacccg ggaccaggag atgttcaaaa tgaccgtgta cccccggttc 3420
   agccacgata cagtcaaggc tcctaggaac ctgattccaa agaaaatggg catgtcccct 3480
   gacatctacg gaggctatac aaacaattct gacgcataca tggtcatcgt ccgcattgat 3540
   aagaaaaagg gaactgagta taagatcctg ggcattccaa cccgggaact ggtgaatctg 3600
   aaaaaggccg agaaggagga ccattacaaa agctatctga aggagatcct gacaccaagg 3660
   attctgtaca acaaaaatgg gaagcgcgat aaaaagatca cttccttcga aattgtgaaa 3720
   tctaagatcc cctataagca ggtcatccag gatggggaca aaaagtttat gctgggaagt 3780
   tcaacatacg tgtataacgc aaagcagctg acactgagca ctgagtccat gaaagccatc 3840
   actaacaatt tcgataagga cagcgatgag aacgacgctc tgattaaggc atacgatgaa 3900
   atcctggaca aagtggataa gtatctgcca ctgttcgaca tcaacaagtt ccgggagaag 3960
   ctgcacagtg ggcgagaaaa gttcatcaag ctgagcctgg aggacaaaaa ggataccatc 4020
   ctgaaagtgc tggaaggact gcatgataac gctgtcatga caaagatccc tactattggc 4080
   ctgtccacac cactggggtt catgcagttt cccaacggcg tgattctgag cgagaatgcc 4140
   aaactgatct accagtcccc caccgggctg ttcaaaaagt cagtgaagat cagcgacctg 4200
   taagaattc 4209
<210> 179
   <211> 178
   <212> RNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, u, g, unknown or other
<400> 179
   nnnnnnnnnn nnnnnnnnnn guuguagcuc ccauucucga aagagaaccg uugcuacaau 60
   aaggccgucu gaaaagaugu gccgcaacgc ucugccccuu aaagcuucug cuuuaagggg 120
   caucguuuau uucgguuaaa aaugccgucu gaaaccgguu uuuagguuuc agacggca 178
<210> 180
   <211> 1082
   <212> PRT
   <213> Neisseria cinerea
<400> 180
   Met Ala Ala Phe Lys Pro Asn Pro Met Asn Tyr Ile Leu Gly Leu Asp
   1               5                   10                  15
   Ile Gly Ile Ala Ser Val Gly Trp Ala Ile Val Glu Ile Asp Glu Glu
               20                  25                  30
   Glu Asn Pro Ile Arg Leu Ile Asp Leu Gly Val Arg Val Phe Glu Arg
           35                  40                  45
   Ala Glu Val Pro Lys Thr Gly Asp Ser Leu Ala Ala Ala Arg Arg Leu
       50                  55                  60
   Ala Arg Ser Val Arg Arg Leu Thr Arg Arg Arg Ala His Arg Leu Leu
   65                  70                  75                  80
   Arg Ala Arg Arg Leu Leu Lys Arg Glu Gly Val Leu Gln Ala Ala Asp
                   85                  90                  95
   Phe Asp Glu Asn Gly Leu Ile Lys Ser Leu Pro Asn Thr Pro Trp Gln
               100                 105                 110
   Leu Arg Ala Ala Ala Leu Asp Arg Lys Leu Thr Pro Leu Glu Trp Ser
           115                 120                 125
   Ala Val Leu Leu His Leu Ile Lys His Arg Gly Tyr Leu Ser Gln Arg
       130                 135                 140
   Lys Asn Glu Gly Glu Thr Ala Asp Lys Glu Leu Gly Ala Leu Leu Lys
   145                 150                 155                 160
   Gly Val Ala Asp Asn Thr His Ala Leu Gln Thr Gly Asp Phe Arg Thr
   165 170 175
   Pro Ala Glu Leu Ala Leu Asn Lys Phe Glu Lys Glu Ser Gly His Ile
   180 185 190
   Arg Asn Gln Arg Gly Asp Tyr Ser His Thr Phe Asn Arg Lys Asp Leu
   195 200 205
   Gln Ala Glu Leu Asn Leu Leu Phe Glu Lys Gln Lys Glu Phe Gly Asn
   210 215 220
   Pro His Val Ser Asp Gly Leu Lys Glu Gly Ile Glu Thr Leu Leu Met
   225 230 235 240
   Thr Gln Arg Pro Ala Leu Ser Gly Asp Ala Val Gln Lys Met Leu Gly
   245 250 255
   His Cys Thr Phe Glu Pro Thr Glu Pro Lys Ala Ala Lys Asn Thr Tyr
   260 265 270
   Thr Ala Glu Arg Phe Val Trp Leu Thr Lys Leu Asn Asn Leu Arg Ile
   275 280 285
   Leu Glu Gln Gly Ser Glu Arg Pro Leu Thr Asp Thr Glu Arg Ala Thr
   290 295 300
   Leu Met Asp Glu Pro Tyr Arg Lys Ser Lys Leu Thr Tyr Ala Gln Ala
   305 310 315 320
   Arg Lys Leu Leu Asp Leu Asp Asp Thr Ala Phe Phe Lys Gly Leu Arg
   325 330 335
   Tyr Gly Lys Asp Asn Ala Glu Ala Ser Thr Leu Met Glu Met Lys Ala
   340 345 350
   Tyr His Ala Ile Ser Arg Ala Leu Glu Lys Glu Gly Leu Lys Asp Lys
   355 360 365
   Lys Ser Pro Leu Asn Leu Ser Pro Glu Leu Gln Asp Glu Ile Gly Thr
   370 375 380
   Ala Phe Ser Leu Phe Lys Thr Asp Glu Asp Ile Thr Gly Arg Leu Lys
   385 390 395 400
   Asp Arg Val Gln Pro Glu Ile Leu Glu Ala Leu Leu Lys His Ile Ser
   405 410 415
   Phe Asp Lys Phe Val Gln Ile Ser Leu Lys Ala Leu Arg Arg Ile Val
   420 425 430
   Pro Leu Met Glu Gln Gly Asn Arg Tyr Asp Glu Ala Cys Thr Glu Ile
   435 440 445
   Tyr Gly Asp His Tyr Gly Lys Lys Asn Thr Glu Glu Lys Ile Tyr Leu
   450 455 460
   Pro Pro Ile Pro Ala Asp Glu Ile Arg Asn Pro Val Val Leu Arg Ala
   465 470 475 480
   Leu Ser Gln Ala Arg Lys Val Ile Asn Gly Val Val Arg Arg Tyr Gly
   485 490 495
   Ser Pro Ala Arg Ile His Ile Glu Thr Ala Arg Glu Val Gly Lys Ser
   500 505 510
   Phe Lys Asp Arg Lys Glu Ile Glu Lys Arg Gln Glu Glu Asn Arg Lys
   515 520 525
   Asp Arg Glu Lys Ser Ala Ala Lys Phe Arg Glu Tyr Phe Pro Asn Phe
   530 535 540
   Val Gly Glu Pro Lys Ser Lys Asp Ile Leu Lys Leu Arg Leu Tyr Glu
   545 550 555 560
   Gln Gln His Gly Lys Cys Leu Tyr Ser Gly Lys Glu Ile Asn Leu Gly
   565 570 575
   Arg Leu Asn Glu Lys Gly Tyr Val Glu Ile Asp His Ala Leu Pro Phe
   580 585 590
   Ser Arg Thr Trp Asp Asp Ser Phe Asn Asn Lys Val Leu Ala Leu Gly
   595 600 605
   Ser Glu Asn Gln Asn Lys Gly Asn Gln Thr Pro Tyr Glu Tyr Phe Asn
   610 615 620
   Gly Lys Asp Asn Ser Arg Glu Trp Gln Glu Phe Lys Ala Arg Val Glu
   625 630 635 640
   Thr Ser Arg Phe Pro Arg Ser Lys Lys Gln Arg Ile Leu Leu Gln Lys
   645 650 655
   Phe Asp Glu Asp Gly Phe Lys Glu Arg Asn Leu Asn Asp Thr Arg Tyr
   660 665 670
   Ile Asn Arg Phe Leu Cys Gln Phe Val Ala Asp His Met Leu Leu Thr
   675 680 685
   Gly Lys Gly Lys Arg Arg Val Phe Ala Ser Asn Gly Gln Ile Thr Asn
   690 695 700
   Leu Leu Arg Gly Phe Trp Gly Leu Arg Lys Val Arg Ala Glu Asn Asp
   705 710 715 720
   Arg His His Ala Leu Asp Ala Val Val Val Ala Cys Ser Thr Ile Ala
   725 730 735
   Met Gln Gln Lys Ile Thr Arg Phe Val Arg Tyr Lys Glu Met Asn Ala
   740 745 750
   Phe Asp Gly Lys Thr Ile Asp Lys Glu Thr Gly Glu Val Leu His Gln
   755 760 765
   Lys Ala His Phe Pro Gln Pro Trp Glu Phe Phe Ala Gln Glu Val Met
   770 775 780
   Ile Arg Val Phe Gly Lys Pro Asp Gly Lys Pro Glu Phe Glu Glu Ala
   785 790 795 800
   Asp Thr Pro Glu Lys Leu Arg Thr Leu Leu Ala Glu Lys Leu Ser Ser
   805 810 815
   Arg Pro Glu Ala Val His Lys Tyr Val Thr Pro Leu Phe Ile Ser Arg
   820 825 830
   Ala Pro Asn Arg Lys Met Ser Gly Gln Gly His Met Glu Thr Val Lys
   835 840 845
   Ser Ala Lys Arg Leu Asp Glu Gly Ile Ser Val Leu Arg Val Pro Leu
   850 855 860
   Thr Gln Leu Lys Leu Lys Asp Leu Glu Lys Met Val Asn Arg Glu Arg
   865 870 875 880
   Glu Pro Lys Leu Tyr Glu Ala Leu Lys Ala Arg Leu Glu Ala His Lys
   885 890 895
   Asp Asp Pro Ala Lys Ala Phe Ala Glu Pro Phe Tyr Lys Tyr Asp Lys
   900 905 910
   Ala Gly Asn Arg Thr Gln Gln Val Lys Ala Val Arg Val Glu Gln Val
   915 920 925
   Gln Lys Thr Gly Val Trp Val His Asn His Asn Gly Ile Ala Asp Asn
       930                 935                 940
   Ala Thr Ile Val Arg Val Asp Val Phe Glu Lys Gly Gly Lys Tyr Tyr
   945                 950                 955                 960
   Leu Val Pro Ile Tyr Ser Trp Gln Val Ala Lys Gly Ile Leu Pro Asp
                   965                 970                 975
   Arg Ala Val Val Gln Gly Lys Asp Glu Glu Asp Trp Thr Val Met Asp
               980                 985                 990
   Asp Ser Phe Glu Phe Lys Phe Val  Leu Tyr Ala Asn Asp  Leu Ile Lys
           995                 1000                 1005
   Leu Thr  Ala Lys Lys Asn Glu  Phe Leu Gly Tyr Phe  Val Ser Leu
       1010                 1015                 1020
   Asn Arg  Ala Thr Gly Ala Ile  Asp Ile Arg Thr His  Asp Thr Asp
       1025                 1030                 1035
   Ser Thr  Lys Gly Lys Asn Gly  Ile Phe Gln Ser Val  Gly Val Lys
       1040                 1045                 1050
   Thr Ala  Leu Ser Phe Gln Lys  Tyr Gln Ile Asp Glu  Leu Gly Lys
       1055                 1060                 1065
   Glu Ile  Arg Pro Cys Arg Leu  Lys Lys Arg Pro Pro  Val Arg
       1070                 1075                 1080
<210> 181
   <211> 3330
   <212> DNA
   <213> Artificial Sequence
<220>
   <221> source
   <223> /note="Description of Artificial Sequence: Synthetic polynucleotide"
<400> 181
   accggtgcca ccatgtaccc atacgatgtt ccagattacg cttcgccgaa gaaaaagcgc      60
   aaggtcgaag cgtccatggc tgccttcaaa cctaatccta tgaactacat cctgggcctg     120
   gacattggaa tcgcttctgt cgggtgggct atcgtggaaa tcgacgagga agagaaccct     180
   atcagactga ttgatctggg agtcagagtg tttgaaaggg cagaggtgcc aaagaccggc     240
   gactccctgg ccgctgcacg gagactggct cggtctgtca ggcgcctgac acgacggaga     300
   gcacacaggc tgctgcgagc taggcgcctg ctgaagagag agggcgtgct gcaggccgct      360
   gacttcgatg aaaacggcct gatcaagagc ctgcccaata ctccttggca gctgagagca      420
   gccgctctgg acaggaagct gaccccactg gagtggtctg ccgtgctgct gcacctgatc      480
   aagcatcgcg gctacctgag tcagcgaaaa aatgaagggg agacagcaga taaggagctg      540
   ggagcactgc tgaaaggagt ggccgacaac actcatgctc tgcagaccgg cgattttagg      600
   acacccgctg agctggcact gaataagttc gaaaaagaga gtggacacat tcgaaaccag      660
   cggggcgact attcacatac cttcaaccgc aaggatctgc aggccgagct gaatctgctg      720
   tttgaaaagc agaaagagtt cgggaatccc cacgtgtccg acgggctgaa agaaggaatc      780
   gagacactgc tgatgactca gaggcctgca ctgtctggcg atgccgtgca gaagatgctg      840
   gggcattgca cctttgaacc aacagagccc aaggcagcca aaaacaccta cacagccgag      900
   aggttcgtgt ggctgacaaa gctgaacaat ctgcgcatcc tggaacaggg cagtgagcgg      960
   cccctgactg acaccgaaag agccacactg atggatgagc cttacaggaa gtctaaactg     1020
   acttatgccc aggctcgcaa gctgctggac ctggacgata ctgccttctt taagggcctg     1080
   aggtacggga aagataatgc agaagccagc accctgatgg agatgaaggc ctatcacgct     1140
   atctcccgcg ccctggaaaa agagggcctg aaggacaaga aatctcccct gaacctgagt     1200
   cctgaactgc aggatgagat tgggaccgct tttagcctgt tcaagactga cgaggatatc     1260
   accggacgcc tgaaagaccg agtgcagccc gaaattctgg aggcactgct gaagcacatc     1320
   agttttgata aattcgtgca gatttcactg aaggccctgc gacggatcgt ccctctgatg     1380
   gagcagggca atcggtacga cgaggcctgc accgagatct acggagatca ttatggcaag     1440
   aaaaacacag aagagaaaat ctatctgccc cctattcctg ccgacgagat ccggaatcca     1500
   gtggtcctga gagctctgtc acaggcaaga aaagtgatca acggagtggt cagaaggtac     1560
   ggcagccctg ctaggatcca cattgaaacc gcacgcgaag tgggaaagtc ctttaaagac     1620
   cgcaaggaaa tcgagaagcg acaggaagag aatagaaaag atagggaaaa gtctgctgca     1680
   aaattcaggg agtactttcc aaacttcgtg ggcgaaccca agagtaaaga catcctgaag     1740
   ctgcgcctgt acgagcagca gcacgggaag tgtctgtata gcggaaaaga aattaacctg     1800
   ggccggctga atgaaaaggg ctatgtggag atcgatcatg cactgccctt ttccagaaca     1860
   tgggacgatt ctttcaacaa taaggtcctg gctctgggga gcgagaacca gaacaaggga     1920
   aatcagactc cttacgaata tttcaacggg aaggacaata gccgagaatg gcaggagttt     1980
   aaagcccgcg tggagacaag ccggttccca cgaagcaaga aacagcggat tctgctgcag     2040
   aagtttgacg aagatggatt caaagagaga aacctgaatg acacccggta catcaacaga     2100
   tttctgtgcc agttcgtggc tgatcacatg ctgctgaccg gaaagggcaa acgccgagtc     2160
   tttgcaagca acggccagat cacaaatctg ctgaggggct tctgggggct gcggaaggtg     2220
   agagccgaga atgaccgcca ccatgcactg gatgccgtgg tcgtggcttg ttccactatt 2280
   gcaatgcagc agaagatcac caggtttgtg cgctataaag agatgaacgc cttcgacgga 2340
   aagacaattg ataaagaaac tggcgaggtg ctgcaccaga aggcacattt tcctcagcca 2400
   tgggagttct tcgcccagga agtgatgatc cgggtctttg ggaagcctga cggaaaacca 2460
   gagttcgaag aggccgatac cccagaaaag ctgcggacac tgctggctga aaaactgagc 2520
   tccagacccg aggcagtgca caagtacgtc acccccctgt tcattagcag ggcccctaat 2580
   cgcaaaatgt ccgggcaggg acatatggag actgtgaaat cagctaagcg gctggacgaa 2640
   ggcatcagcg tgctgagagt cccactgacc cagctgaagc tgaaagatct ggagaagatg 2700
   gtgaaccggg aaagagagcc caagctgtat gaagctctga aagcaagact ggaggcccac 2760
   aaggacgatc cagctaaagc atttgccgag cccttctaca aatatgacaa ggccggcaat 2820
   cggacacagc aggtgaaggc tgtcagagtg gagcaggtcc agaaaactgg ggtctgggtg 2880
   cacaaccata atggaattgc cgacaacgct acaatcgtcc gggtggatgt gttcgagaaa 2940
   ggcgggaagt actatctggt gcctatctac tcctggcagg tcgccaaggg aatcctgcca 3000
   gatagagctg tcgtgcaggg caaagacgaa gaggattgga ctgtgatgga cgattctttc 3060
   gagtttaagt tcgtcctgta cgcaaacgac ctgatcaagc tgacagccaa gaaaaatgaa 3120
   tttctggggt atttcgtgtc actgaacagg gcaactggag ccatcgatat tcgcacacat 3180
   gacactgata gcaccaaggg aaaaaacggc atctttcagt ctgtgggcgt caagaccgcc 3240
   ctgagtttcc agaaatatca gattgacgaa ctggggaagg agatccgacc ctgtcggctg 3300
   aagaaacgac cacccgtgcg gtaagaattc 3330
<210> 182
   <211> 145
   <212> RNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, u, g, unknown or other
<400> 182
   nnnnnnnnnn nnnnnnnnnn gcugcggauu gcgggaaauc gcuuuucgca agcaaauuga 60
   ccccuugugc gggcucggca ucccaagguc agcugccggu uauuaucgaa aaggcccacc 120
   gcaagcagcg cgugggccuu uuuuu 145
<210> 183
   <211> 1037
   <212> PRT
   <213> Parvibaculum lavamentivorans
<400> 183
   Met Glu Arg Ile Phe Gly Phe Asp Ile Gly Thr Thr Ser Ile Gly Phe
   1               5                   10                  15
   Ser Val Ile Asp Tyr Ser Ser Thr Gln Ser Ala Gly Asn Ile Gln Arg
               20                  25                  30
   Leu Gly Val Arg Ile Phe Pro Glu Ala Arg Asp Pro Asp Gly Thr Pro
           35                  40                  45
   Leu Asn Gln Gln Arg Arg Gln Lys Arg Met Met Arg Arg Gln Leu Arg
       50                  55                  60
   Arg Arg Arg Ile Arg Arg Lys Ala Leu Asn Glu Thr Leu His Glu Ala
   65                  70                  75                  80
   Gly Phe Leu Pro Ala Tyr Gly Ser Ala Asp Trp Pro Val Val Met Ala
                   85                  90                  95
   Asp Glu Pro Tyr Glu Leu Arg Arg Arg Gly Leu Glu Glu Gly Leu Ser
               100                 105                 110
   Ala Tyr Glu Phe Gly Arg Ala Ile Tyr His Leu Ala Gln His Arg His
           115                 120                 125
   Phe Lys Gly Arg Glu Leu Glu Glu Ser Asp Thr Pro Asp Pro Asp Val
       130                 135                 140
   Asp Asp Glu Lys Glu Ala Ala Asn Glu Arg Ala Ala Thr Leu Lys Ala
   145                 150                 155                 160
   Leu Lys Asn Glu Gln Thr Thr Leu Gly Ala Trp Leu Ala Arg Arg Pro
                   165                 170                 175
   Pro Ser Asp Arg Lys Arg Gly Ile His Ala His Arg Asn Val Val Ala
               180                 185                 190
   Glu Glu Phe Glu Arg Leu Trp Glu Val Gln Ser Lys Phe His Pro Ala
           195                 200                 205
   Leu Lys Ser Glu Glu Met Arg Ala Arg Ile Ser Asp Thr Ile Phe Ala
       210                 215                 220
   Gln Arg Pro Val Phe Trp Arg Lys Asn Thr Leu Gly Glu Cys Arg Phe
   225                 230                 235                 240
   Met Pro Gly Glu Pro Leu Cys Pro Lys Gly Ser Trp Leu Ser Gln Gln
                   245                 250                 255
   Arg Arg Met Leu Glu Lys Leu Asn Asn Leu Ala Ile Ala Gly Gly Asn
               260                 265                 270
   Ala Arg Pro Leu Asp Ala Glu Glu Arg Asp Ala Ile Leu Ser Lys Leu
           275                 280                 285
   Gln Gln Gln Ala Ser Met Ser Trp Pro Gly Val Arg Ser Ala Leu Lys
       290                 295                 300
   Ala Leu Tyr Lys Gln Arg Gly Glu Pro Gly Ala Glu Lys Ser Leu Lys
   305                 310                 315                 320
   Phe Asn Leu Glu Leu Gly Gly Glu Ser Lys Leu Leu Gly Asn Ala Leu
                   325                 330                 335
   Glu Ala Lys Leu Ala Asp Met Phe Gly Pro Asp Trp Pro Ala His Pro
               340                 345                 350
   Arg Lys Gln Glu Ile Arg His Ala Val His Glu Arg Leu Trp Ala Ala
           355                 360                 365
   Asp Tyr Gly Glu Thr Pro Asp Lys Lys Arg Val Ile Ile Leu Ser Glu
       370                 375                 380
   Lys Asp Arg Lys Ala His Arg Glu Ala Ala Ala Asn Ser Phe Val Ala
   385                 390                 395                 400
   Asp Phe Gly Ile Thr Gly Glu Gln Ala Ala Gln Leu Gln Ala Leu Lys
                   405                 410                 415
   Leu Pro Thr Gly Trp Glu Pro Tyr Ser Ile Pro Ala Leu Asn Leu Phe
               420                 425                 430
   Leu Ala Glu Leu Glu Lys Gly Glu Arg Phe Gly Ala Leu Val Asn Gly
           435                 440                 445
   Pro Asp Trp Glu Gly Trp Arg Arg Thr Asn Phe Pro His Arg Asn Gln
       450                 455                 460
   Pro Thr Gly Glu Ile Leu Asp Lys Leu Pro Ser Pro Ala Ser Lys Glu
   465                 470                 475                 480
   Glu Arg Glu Arg Ile Ser Gln Leu Arg Asn Pro Thr Val Val Arg Thr
                   485                 490                 495
   Gln Asn Glu Leu Arg Lys Val Val Asn Asn Leu Ile Gly Leu Tyr Gly
               500                 505                 510
   Lys Pro Asp Arg Ile Arg Ile Glu Val Gly Arg Asp Val Gly Lys Ser
           515                 520                 525
   Lys Arg Glu Arg Glu Glu Ile Gln Ser Gly Ile Arg Arg Asn Glu Lys
       530                 535                 540
   Gln Arg Lys Lys Ala Thr Glu Asp Leu Ile Lys Asn Gly Ile Ala Asn
   545                 550                 555                 560
   Pro Ser Arg Asp Asp Val Glu Lys Trp Ile Leu Trp Lys Glu Gly Gln
                   565                 570                 575
   Glu Arg Cys Pro Tyr Thr Gly Asp Gln Ile Gly Phe Asn Ala Leu Phe
               580                 585                 590
   Arg Glu Gly Arg Tyr Glu Val Glu His Ile Trp Pro Arg Ser Arg Ser
           595                 600                 605
   Phe Asp Asn Ser Pro Arg Asn Lys Thr Leu Cys Arg Lys Asp Val Asn
       610                 615                 620
   Ile Glu Lys Gly Asn Arg Met Pro Phe Glu Ala Phe Gly His Asp Glu
   625                 630                 635                 640
   Asp Arg Trp Ser Ala Ile Gln Ile Arg Leu Gln Gly Met Val Ser Ala
                   645                 650                 655
   Lys Gly Gly Thr Gly Met Ser Pro Gly Lys Val Lys Arg Phe Leu Ala
               660                 665                 670
   Lys Thr Met Pro Glu Asp Phe Ala Ala Arg Gln Leu Asn Asp Thr Arg
           675                 680                 685
   Tyr Ala Ala Lys Gln Ile Leu Ala Gln Leu Lys Arg Leu Trp Pro Asp
       690                 695                 700
   Met Gly Pro Glu Ala Pro Val Lys Val Glu Ala Val Thr Gly Gln Val
   705                 710                 715                 720
   Thr Ala Gln Leu Arg Lys Leu Trp Thr Leu Asn Asn Ile Leu Ala Asp
                   725                 730                 735
   Asp Gly Glu Lys Thr Arg Ala Asp His Arg His His Ala Ile Asp Ala
               740                 745                 750
   Leu Thr Val Ala Cys Thr His Pro Gly Met Thr Asn Lys Leu Ser Arg
           755                 760                 765
   Tyr Trp Gln Leu Arg Asp Asp Pro Arg Ala Glu Lys Pro Ala Leu Thr
       770                 775                 780
   Pro Pro Trp Asp Thr Ile Arg Ala Asp Ala Glu Lys Ala Val Ser Glu
   785                 790                 795                 800
   Ile Val Val Ser His Arg Val Arg Lys Lys Val Ser Gly Pro Leu His
                   805                 810                 815
   Lys Glu Thr Thr Tyr Gly Asp Thr Gly Thr Asp Ile Lys Thr Lys Ser
               820                 825                 830
   Gly Thr Tyr Arg Gln Phe Val Thr Arg Lys Lys Ile Glu Ser Leu Ser
           835                 840                 845
   Lys Gly Glu Leu Asp Glu Ile Arg Asp Pro Arg Ile Lys Glu Ile Val
       850                 855                 860
   Ala Ala His Val Ala Gly Arg Gly Gly Asp Pro Lys Lys Ala Phe Pro
   865                 870                 875                 880
   Pro Tyr Pro Cys Val Ser Pro Gly Gly Pro Glu Ile Arg Lys Val Arg
                   885                 890                 895
   Leu Thr Ser Lys Gln Gln Leu Asn Leu Met Ala Gln Thr Gly Asn Gly
               900                 905                 910
   Tyr Ala Asp Leu Gly Ser Asn His His Ile Ala Ile Tyr Arg Leu Pro
           915                 920                 925
   Asp Gly Lys Ala Asp Phe Glu Ile Val Ser Leu Phe Asp Ala Ser Arg
       930                 935                 940
   Arg Leu Ala Gln Arg Asn Pro Ile Val Gln Arg Thr Arg Ala Asp Gly
   945                 950                 955                 960
   Ala Ser Phe Val Met Ser Leu Ala Ala Gly Glu Ala Ile Met Ile Pro
                   965                 970                 975
   Glu Gly Ser Lys Lys Gly Ile Trp Ile Val Gln Gly Val Trp Ala Ser
   980 985 990
   Gly Gln Val Val Leu Glu Arg Asp Thr Asp Ala Asp His Ser Thr Thr
   995 1000 1005
   Thr Arg Pro Met Pro Asn Pro Ile Leu Lys Asp Asp Ala Lys Lys
   1010 1015 1020
   Val Ser Ile Asp Pro Ile Gly Arg Val Arg Pro Ser Asn Asp
   1025 1030 1035
<210> 184
   <211> 3195
   <212> DNA
   <213> Artificial Sequence
<220>
   <221> source
   <223> /note="Description of Artificial Sequence: Synthetic polynucleotide"
<400> 184
   accggtgcca ccatgtaccc atacgatgtt ccagattacg cttcgccgaa gaaaaagcgc 60
   aaggtcgaag cgtccatgga gaggattttc ggctttgaca tcggcacaac aagtatcgga 120
   ttcagcgtga ttgattacag tagcacccag tccgcaggca acatccagag gctgggcgtg 180
   cgcattttcc ctgaggcaag ggacccagat gggacccccc tgaaccagca gcggagacag 240
   aaacgcatga tgaggcgcca gctgcgacgg agaaggattc gccgaaaggc actgaatgag 300
   acactgcacg aagccggctt tctgccagct tacgggtctg cagattggcc cgtggtcatg 360
   gccgacgagc cttatgaact gcggagaagg ggactggagg aaggcctgag tgcttacgag 420
   ttcggacggg caatctatca tctggcccag caccggcatt ttaaaggcag agaactggag 480
   gaatccgata cacccgaccc tgatgtggac gatgagaagg aagccgctaa cgagagagca 540
   gccactctga aggccctgaa aaatgaacag accacactgg gagcatggct ggcccgccga 600
   cccccttctg accgcaagcg aggaatccac gcccatagga acgtggtcgc tgaggagttc 660
   gagcgcctgt gggaagtgca gtccaagttt caccccgctc tgaaatctga ggaaatgcgg 720
   gcaagaatca gtgatacaat tttcgcccag aggcctgtgt tttggcgcaa gaacactctg 780
   ggagagtgca gattcatgcc tggcgaacca ctgtgtccca aggggtcctg gctgtctcag 840
   cagcggagaa tgctggagaa actgaacaat ctggctatcg caggcgggaa tgctaggcca 900
   ctggatgcag aggaacgcga cgccattctg agtaagctgc agcagcaggc cagcatgtcc 960
   tggccaggcg tgcggtcagc tctgaaggca ctgtacaaac agagaggcga gcccggggct 1020
   gaaaagagcc tgaaattcaa cctggagctg ggaggcgaat ccaagctgct gggaaatgcc 1080
   ctggaggcta aactggcaga tatgtttggc cctgactggc cagctcaccc ccgaaagcag 1140
   gagatccggc acgcagtgca tgaacggctg tgggctgcag attacggcga gacacccgac 1200
   aagaaaagag tcatcattct gtccgagaag gatcgaaaag ctcatcggga agccgctgca 1260
   aactctttcg tggcagactt tggaattact ggcgagcagg cagctcagct gcaggccctg 1320
   aagctgccaa ccggctggga accttatagc atcccagcac tgaacctgtt cctggccgag 1380
   ctggaaaagg gggagaggtt tggagccctg gtgaatggac ctgattggga aggctggagg 1440
   cgcacaaact tcccccaccg caatcagcct actggggaga tcctggacaa gctgccaagt 1500
   cccgcctcaa aagaggaaag ggaacgcatt agccagctgc gcaacccaac cgtggtccga 1560
   acacagaatg agctgagaaa ggtggtcaac aatctgatcg ggctgtatgg aaaacccgat 1620
   cgaatccgga ttgaagtggg ccgggacgtc gggaagtcca aaagagaaag ggaggaaatc 1680
   cagtctggca ttcgacggaa cgagaagcag agaaagaaag ccactgaaga tctgatcaaa 1740
   aacggaattg ctaatcctag ccgggacgat gtggagaagt ggatcctgtg gaaagagggc 1800
   caggaaagat gcccatacac cggcgaccag attggcttca atgccctgtt tagagaaggc 1860
   agatatgagg tggaacacat ctggcctcgc tctcgaagtt ttgataacag cccaaggaat 1920
   aagacactgt gtcgcaaaga cgtgaacatc gagaagggaa ataggatgcc tttcgaggca 1980
   tttggccatg acgaagatcg gtggagcgcc atccagatta gactgcaggg catggtgtca 2040
   gccaaagggg gaactgggat gagccccgga aaggtcaaac gcttcctggc taagaccatg 2100
   cctgaggatt ttgcagcccg gcagctgaac gacacaagat acgctgcaaa gcagatcctg 2160
   gcccagctga aaaggctgtg gccagacatg ggacctgagg ctccagtgaa ggtcgaagca 2220
   gtgactggac aggtcaccgc ccagctgcgc aaactgtgga ctctgaacaa tattctggct 2280
   gacgatgggg agaaaaccag agcagatcac aggcaccatg ccatcgacgc tctgacagtg 2340
   gcctgcactc atcctggaat gaccaacaag ctgagcaggt attggcagct gcgcgacgat 2400
   ccacgagcag agaagccagc tctgactcca ccctgggata ccatccgcgc cgacgctgag 2460
   aaagccgtgt ctgaaattgt ggtcagtcac cgggtgagaa agaaagtcag cggcccactg 2520
   cataaggaga ctacctacgg cgatacaggg actgacatta agaccaaatc cggcacatat 2580
   agacagttcg tgaccaggaa gaaaatcgag tcactgagca agggggagct ggatgaaatt 2640
   cgcgaccccc gaatcaaaga aattgtggca gctcacgtcg caggacgagg aggcgacccc 2700
   aagaaggcct tccctccata cccctgtgtg tctcccggag gccctgagat ccggaaggtc 2760
   agactgacca gtaaacagca gctgaacctg atggcccaga cagggaatgg atacgctgac 2820
   ctgggctcca accaccatat cgcaatctac cggctgcccg atgggaaggc cgacttcgag 2880
   attgtgtcac tgtttgatgc tagcagaagg ctggcacaga gaaatccaat cgtgcagagg 2940
   acacgagcag acggagccag cttcgtcatg tccctggcag ccggagaggc catcatgatt 3000
   cccgaaggct caaagaaagg gatctggatt gtgcagggag tctgggcaag cggacaggtg 3060
   gtcctggaga gggacaccga tgctgaccac tctacaacta cccgccctat gccaaacccc 3120
   atcctgaagg acgatgccaa gaaagtgagt atcgatccta ttggccgagt ccggccatca 3180
   aatgactaag aattc 3195
<210> 185
   <211> 103
   <212> RNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, u, g, unknown or other
<400> 185
   nnnnnnnnnn nnnnnnnnnn guuuuaguac ucuggaaaca gaaucuacua aaacaaggca 60
   aaaugccgug uuuaucucgu caacuuguug gcgagauuuu uuu 103
<210> 186
   <211> 1053
   <212> PRT
   <213> Staphylococcus aureus
<400> 186
   Met Lys Arg Asn Tyr Ile Leu Gly Leu Asp Ile Gly Ile Thr Ser Val
   1               5                   10                  15
   Gly Tyr Gly Ile Ile Asp Tyr Glu Thr Arg Asp Val Ile Asp Ala Gly
               20                  25                  30
   Val Arg Leu Phe Lys Glu Ala Asn Val Glu Asn Asn Glu Gly Arg Arg
           35                  40                  45
   Ser Lys Arg Gly Ala Arg Arg Leu Lys Arg Arg Arg Arg His Arg Ile
       50                  55                  60
   Gln Arg Val Lys Lys Leu Leu Phe Asp Tyr Asn Leu Leu Thr Asp His
   65                  70                  75                  80
   Ser Glu Leu Ser Gly Ile Asn Pro Tyr Glu Ala Arg Val Lys Gly Leu
                   85                  90                  95
   Ser Gln Lys Leu Ser Glu Glu Glu Phe Ser Ala Ala Leu Leu His Leu
               100                 105                 110
   Ala Lys Arg Arg Gly Val His Asn Val Asn Glu Val Glu Glu Asp Thr
           115                 120                 125
   Gly Asn Glu Leu Ser Thr Lys Glu Gln Ile Ser Arg Asn Ser Lys Ala
       130                 135                 140
   Leu Glu Glu Lys Tyr Val Ala Glu Leu Gln Leu Glu Arg Leu Lys Lys
   145                 150                 155                 160
   Asp Gly Glu Val Arg Gly Ser Ile Asn Arg Phe Lys Thr Ser Asp Tyr
               165                 170                 175
   Val Lys Glu Ala Lys Gln Leu Leu Lys Val Gln Lys Ala Tyr His Gln
           180                 185                 190
   Leu Asp Gln Ser Phe Ile Asp Thr Tyr Ile Asp Leu Leu Glu Thr Arg
           195                 200                 205
   Arg Thr Tyr Tyr Glu Gly Pro Gly Glu Gly Ser Pro Phe Gly Trp Lys
       210                 215                 220
   Asp Ile Lys Glu Trp Tyr Glu Met Leu Met Gly His Cys Thr Tyr Phe
   225                 230                 235                 240
   Pro Glu Glu Leu Arg Ser Val Lys Tyr Ala Tyr Asn Ala Asp Leu Tyr
               245                 250                 255
   Asn Ala Leu Asn Asp Leu Asn Asn Leu Val Ile Thr Arg Asp Glu Asn
           260                 265                 270
   Glu Lys Leu Glu Tyr Tyr Glu Lys Phe Gln Ile Ile Glu Asn Val Phe
           275                 280                 285
   Lys Gln Lys Lys Lys Pro Thr Leu Lys Gln Ile Ala Lys Glu Ile Leu
       290                 295                 300
   Val Asn Glu Glu Asp Ile Lys Gly Tyr Arg Val Thr Ser Thr Gly Lys
   305                 310                 315                 320
   Pro Glu Phe Thr Asn Leu Lys Val Tyr His Asp Ile Lys Asp Ile Thr
               325                 330                 335
   Ala Arg Lys Glu Ile Ile Glu Asn Ala Glu Leu Leu Asp Gln Ile Ala
           340                 345                 350
   Lys Ile Leu Thr Ile Tyr Gln Ser Ser Glu Asp Ile Gln Glu Glu Leu
   355 360 365
   Thr Asn Leu Asn Ser Glu Leu Thr Gln Glu Glu Ile Glu Gln Ile Ser
   370 375 380
   Asn Leu Lys Gly Tyr Thr Gly Thr His Asn Leu Ser Leu Lys Ala Ile
   385 390 395 400
   Asn Leu Ile Leu Asp Glu Leu Trp His Thr Asn Asp Asn Gln Ile Ala
   405 410 415
   Ile Phe Asn Arg Leu Lys Leu Val Pro Lys Lys Val Asp Leu Ser Gln
   420 425 430
   Gln Lys Glu Ile Pro Thr Thr Leu Val Asp Asp Phe Ile Leu Ser Pro
   435 440 445
   Val Val Lys Arg Ser Phe Ile Gln Ser Ile Lys Val Ile Asn Ala Ile
   450 455 460
   Ile Lys Lys Tyr Gly Leu Pro Asn Asp Ile Ile Ile Glu Leu Ala Arg
   465 470 475 480
   Glu Lys Asn Ser Lys Asp Ala Gln Lys Met Ile Asn Glu Met Gln Lys
   485 490 495
   Arg Asn Arg Gln Thr Asn Glu Arg Ile Glu Glu Ile Ile Arg Thr Thr
   500 505 510
   Gly Lys Glu Asn Ala Lys Tyr Leu Ile Glu Lys Ile Lys Leu His Asp
   515 520 525
   Met Gln Glu Gly Lys Cys Leu Tyr Ser Leu Glu Ala Ile Pro Leu Glu
   530 535 540
   Asp Leu Leu Asn Asn Pro Phe Asn Tyr Glu Val Asp His Ile Ile Pro
   545 550 555 560
   Arg Ser Val Ser Phe Asp Asn Ser Phe Asn Asn Lys Val Leu Val Lys
   565 570 575
   Gln Glu Glu Asn Ser Lys Lys Gly Asn Arg Thr Pro Phe Gln Tyr Leu
   580 585 590
   Ser Ser Ser Asp Ser Lys Ile Ser Tyr Glu Thr Phe Lys Lys His Ile
   595 600 605
   Leu Asn Leu Ala Lys Gly Lys Gly Arg Ile Ser Lys Thr Lys Lys Glu
       610                 615                 620
   Tyr Leu Leu Glu Glu Arg Asp Ile Asn Arg Phe Ser Val Gln Lys Asp
   625                 630                 635                 640
   Phe Ile Asn Arg Asn Leu Val Asp Thr Arg Tyr Ala Thr Arg Gly Leu
                   645                 650                 655
   Met Asn Leu Leu Arg Ser Tyr Phe Arg Val Asn Asn Leu Asp Val Lys
               660                 665                 670
   Val Lys Ser Ile Asn Gly Gly Phe Thr Ser Phe Leu Arg Arg Lys Trp
           675                 680                 685
   Lys Phe Lys Lys Glu Arg Asn Lys Gly Tyr Lys His His Ala Glu Asp
       690                 695                 700
   Ala Leu Ile Ile Ala Asn Ala Asp Phe Ile Phe Lys Glu Trp Lys Lys
   705                 710                 715                 720
   Leu Asp Lys Ala Lys Lys Val Met Glu Asn Gln Met Phe Glu Glu Lys
                   725                 730                 735
   Gln Ala Glu Ser Met Pro Glu Ile Glu Thr Glu Gln Glu Tyr Lys Glu
               740                 745                 750
   Ile Phe Ile Thr Pro His Gln Ile Lys His Ile Lys Asp Phe Lys Asp
           755                 760                 765
   Tyr Lys Tyr Ser His Arg Val Asp Lys Lys Pro Asn Arg Glu Leu Ile
       770                 775                 780
   Asn Asp Thr Leu Tyr Ser Thr Arg Lys Asp Asp Lys Gly Asn Thr Leu
   785                 790                 795                 800
   Ile Val Asn Asn Leu Asn Gly Leu Tyr Asp Lys Asp Asn Asp Lys Leu
                   805                 810                 815
   Lys Lys Leu Ile Asn Lys Ser Pro Glu Lys Leu Leu Met Tyr His His
               820                 825                 830
   Asp Pro Gln Thr Tyr Gln Lys Leu Lys Leu Ile Met Glu Gln Tyr Gly
           835                 840                 845
   Asp Glu Lys Asn Pro Leu Tyr Lys Tyr Tyr Glu Glu Thr Gly Asn Tyr
       850                 855                 860
   Leu Thr Lys Tyr Ser Lys Lys Asp Asn Gly Pro Val Ile Lys Lys Ile
   865 870 875 880
   Lys Tyr Tyr Gly Asn Lys Leu Asn Ala His Leu Asp Ile Thr Asp Asp
   885 890 895
   Tyr Pro Asn Ser Arg Asn Lys Val Val Lys Leu Ser Leu Lys Pro Tyr
   900 905 910
   Arg Phe Asp Val Tyr Leu Asp Asn Gly Val Tyr Lys Phe Val Thr Val
   915 920 925
   Lys Asn Leu Asp Val Ile Lys Lys Glu Asn Tyr Tyr Glu Val Asn Ser
   930 935 940
   Lys Cys Tyr Glu Glu Ala Lys Lys Leu Lys Lys Ile Ser Asn Gln Ala
   945 950 955 960
   Glu Phe Ile Ala Ser Phe Tyr Asn Asn Asp Leu Ile Lys Ile Asn Gly
   965 970 975
   Glu Leu Tyr Arg Val Ile Gly Val Asn Asn Asp Leu Leu Asn Arg Ile
   980 985 990
   Glu Val Asn Met Ile Asp Ile Thr Tyr Arg Glu Tyr Leu Glu Asn Met
   995 1000 1005
   Asn Asp Lys Arg Pro Pro Arg Ile Ile Lys Thr Ile Ala Ser Lys
   1010 1015 1020
   Thr Gln Ser Ile Lys Lys Tyr Ser Thr Asp Ile Leu Gly Asn Leu
   1025 1030 1035
   Tyr Glu Val Lys Ser Lys Lys His Pro Gln Ile Ile Lys Lys Gly
   1040 1045 1050
<210> 187
   <211> 3243
   <212> DNA
   <213> Artificial Sequence
<220>
   <221> source
   <223> /note="Description of Artificial Sequence: Synthetic polynucleotide"
<400> 187
   accggtgcca ccatgtaccc atacgatgtt ccagattacg cttcgccgaa gaaaaagcgc 60
   aaggtcgaag cgtccatgaa aaggaactac attctggggc tggacatcgg gattacaagc 120
   gtggggtatg ggattattga ctatgaaaca agggacgtga tcgacgcagg cgtcagactg 180
   ttcaaggagg ccaacgtgga aaacaatgag ggacggagaa gcaagagggg agccaggcgc 240
   ctgaaacgac ggagaaggca cagaatccag agggtgaaga aactgctgtt cgattacaac 300
   ctgctgaccg accattctga gctgagtgga attaatcctt atgaagccag ggtgaaaggc 360
   ctgagtcaga agctgtcaga ggaagagttt tccgcagctc tgctgcacct ggctaagcgc 420
   cgaggagtgc ataacgtcaa tgaggtggaa gaggacaccg gcaacgagct gtctacaaag 480
   gaacagatct cacgcaatag caaagctctg gaagagaagt atgtcgcaga gctgcagctg 540
   gaacggctga agaaagatgg cgaggtgaga gggtcaatta ataggttcaa gacaagcgac 600
   tacgtcaaag aagccaagca gctgctgaaa gtgcagaagg cttaccacca gctggatcag 660
   agcttcatcg atacttatat cgacctgctg gagactcgga gaacctacta tgagggacca 720
   ggagaaggga gccccttcgg atggaaagac atcaaggaat ggtacgagat gctgatggga 780
   cattgcacct attttccaga agagctgaga agcgtcaagt acgcttataa cgcagatctg 840
   tacaacgccc tgaatgacct gaacaacctg gtcatcacca gggatgaaaa cgagaaactg 900
   gaatactatg agaagttcca gatcatcgaa aacgtgttta agcagaagaa aaagcctaca 960
   ctgaaacaga ttgctaagga gatcctggtc aacgaagagg acatcaaggg ctaccgggtg 1020
   acaagcactg gaaaaccaga gttcaccaat ctgaaagtgt atcacgatat taaggacatc 1080
   acagcacgga aagaaatcat tgagaacgcc gaactgctgg atcagattgc taagatcctg 1140
   actatctacc agagctccga ggacatccag gaagagctga ctaacctgaa cagcgagctg 1200
   acccaggaag agatcgaaca gattagtaat ctgaaggggt acaccggaac acacaacctg 1260
   tccctgaaag ctatcaatct gattctggat gagctgtggc atacaaacga caatcagatt 1320
   gcaatcttta accggctgaa gctggtccca aaaaaggtgg acctgagtca gcagaaagag 1380
   atcccaacca cactggtgga cgatttcatt ctgtcacccg tggtcaagcg gagcttcatc 1440
   cagagcatca aagtgatcaa cgccatcatc aagaagtacg gcctgcccaa tgatatcatt 1500
   atcgagctgg ctagggagaa gaacagcaag gacgcacaga agatgatcaa tgagatgcag 1560
   aaacgaaacc ggcagaccaa tgaacgcatt gaagagatta tccgaactac cgggaaagag 1620
   aacgcaaagt acctgattga aaaaatcaag ctgcacgata tgcaggaggg aaagtgtctg 1680
   tattctctgg aggccatccc cctggaggac ctgctgaaca atccattcaa ctacgaggtc 1740
   gatcatatta tccccagaag cgtgtccttc gacaattcct ttaacaacaa ggtgctggtc 1800
   aagcaggaag agaactctaa aaagggcaat aggactcctt tccagtacct gtctagttca 1860
   gattccaaga tctcttacga aacctttaaa aagcacattc tgaatctggc caaaggaaag 1920
   ggccgcatca gcaagaccaa aaaggagtac ctgctggaag agcgggacat caacagattc 1980
   tccgtccaga aggattttat taaccggaat ctggtggaca caagatacgc tactcgcggc 2040
   ctgatgaatc tgctgcgatc ctatttccgg gtgaacaatc tggatgtgaa agtcaagtcc 2100
   atcaacggcg ggttcacatc ttttctgagg cgcaaatgga agtttaaaaa ggagcgcaac 2160
   aaagggtaca agcaccatgc cgaagatgct ctgattatcg caaatgccga cttcatcttt 2220
   aaggagtgga aaaagctgga caaagccaag aaagtgatgg agaaccagat gttcgaagag 2280
   aagcaggccg aatctatgcc cgaaatcgag acagaacagg agtacaagga gattttcatc 2340
   actcctcacc agatcaagca tatcaaggat ttcaaggact acaagtactc tcaccgggtg 2400
   gataaaaagc ccaacagaga gctgatcaat gacaccctgt atagtacaag aaaagacgat 2460
   aaggggaata ccctgattgt gaacaatctg aacggactgt acgacaaaga taatgacaag 2520
   ctgaaaaagc tgatcaacaa aagtcccgag aagctgctga tgtaccacca tgatcctcag 2580
   acatatcaga aactgaagct gattatggag cagtacggcg acgagaagaa cccactgtat 2640
   aagtactatg aagagactgg gaactacctg accaagtata gcaaaaagga taatggcccc 2700
   gtgatcaaga agatcaagta ctatgggaac aagctgaatg cccatctgga catcacagac 2760
   gattacccta acagtcgcaa caaggtggtc aagctgtcac tgaagccata cagattcgat 2820
   gtctatctgg acaacggcgt gtataaattt gtgactgtca agaatctgga tgtcatcaaa 2880
   aaggagaact actatgaagt gaatagcaag tgctacgaag aggctaaaaa gctgaaaaag 2940
   attagcaacc aggcagagtt catcgcctcc ttttacaaca acgacctgat taagatcaat 3000
   ggcgaactgt atagggtcat cggggtgaac aatgatctgc tgaaccgcat tgaagtgaat 3060
   atgattgaca tcacttaccg agagtatctg gaaaacatga atgataagcg cccccctcga 3120
   attatcaaaa caattgcctc taagactcag agtatcaaaa agtactcaac cgacattctg 3180
   ggaaacctgt atgaggtgaa gagcaaaaag caccctcaga ttatcaaaaa gggctaagaa 3240
   ttc 3243
<210> 188
   <211> 114
   <212> RNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, u, g, unknown or other
<400> 188
   nnnnnnnnnn nnnnnnnnnn guuuuagcac uguacaagaa auugucgugc uaaaauaagg 60
   cgcuguuaau gcagcugccg cauccgccag agcauuuaug cucuggcuuu uuuu 114
<210> 189
   <211> 1269
   <212> PRT
   <213> Mycoplasma gallisepticum
<400> 189
   Met Asn Asn Ser Ile Lys Ser Lys Pro Glu Val Thr Ile Gly Leu Asp
   1               5                   10                  15
   Leu Gly Val Gly Ser Val Gly Trp Ala Ile Val Asp Asn Glu Thr Asn
               20                  25                  30
   Ile Ile His His Leu Gly Ser Arg Leu Phe Ser Gln Ala Lys Thr Ala
           35                  40                  45
   Glu Asp Arg Arg Ser Phe Arg Gly Val Arg Arg Leu Ile Arg Arg Arg
       50                  55                  60
   Lys Tyr Lys Leu Lys Arg Phe Val Asn Leu Ile Trp Lys Tyr Asn Ser
   65                  70                  75                  80
   Tyr Phe Gly Phe Lys Asn Lys Glu Asp Ile Leu Asn Asn Tyr Gln Glu
                   85                  90                  95
   Gln Gln Lys Leu His Asn Thr Val Leu Asn Leu Lys Ser Glu Ala Leu
               100                 105                 110
   Asn Ala Lys Ile Asp Pro Lys Ala Leu Ser Trp Ile Leu His Asp Tyr
           115                 120                 125
   Leu Lys Asn Arg Gly His Phe Tyr Glu Asp Asn Arg Asp Phe Asn Val
       130                 135                 140
   Tyr Pro Thr Lys Glu Leu Ala Lys Tyr Phe Asp Lys Tyr Gly Tyr Tyr
   145                 150                 155                 160
   Lys Gly Ile Ile Asp Ser Lys Glu Asp Asn Asp Asn Lys Leu Glu Glu
                   165                 170                 175
   Glu Leu Thr Lys Tyr Lys Phe Ser Asn Lys His Trp Leu Glu Glu Val
               180                 185                 190
   Lys Lys Val Leu Ser Asn Gln Thr Gly Leu Pro Glu Lys Phe Lys Glu
           195                 200                 205
   Glu Tyr Glu Ser Leu Phe Ser Tyr Val Arg Asn Tyr Ser Glu Gly Pro
       210                 215                 220
   Gly Ser Ile Asn Ser Val Ser Pro Tyr Gly Ile Tyr His Leu Asp Glu
   225                 230                 235                 240
   Lys Glu Gly Lys Val Val Gln Lys Tyr Asn Asn Ile Trp Asp Lys Thr
                   245                 250                 255
   Ile Gly Lys Cys Asn Ile Phe Pro Asp Glu Tyr Arg Ala Pro Lys Asn
               260                 265                 270
   Ser Pro Ile Ala Met Ile Phe Asn Glu Ile Asn Glu Leu Ser Thr Ile
           275                 280                 285
   Arg Ser Tyr Ser Ile Tyr Leu Thr Gly Trp Phe Ile Asn Gln Glu Phe
       290                 295                 300
   Lys Lys Ala Tyr Leu Asn Lys Leu Leu Asp Leu Leu Ile Lys Thr Asn
   305                 310                 315                 320
   Gly Glu Lys Pro Ile Asp Ala Arg Gln Phe Lys Lys Leu Arg Glu Glu
                   325                 330                 335
   Thr Ile Ala Glu Ser Ile Gly Lys Glu Thr Leu Lys Asp Val Glu Asn
               340                 345                 350
   Glu Glu Lys Leu Glu Lys Glu Asp His Lys Trp Lys Leu Lys Gly Leu
           355                 360                 365
   Lys Leu Asn Thr Asn Gly Lys Ile Gln Tyr Asn Asp Leu Ser Ser Leu
       370                 375                 380
   Ala Lys Phe Val His Lys Leu Lys Gln His Leu Lys Leu Asp Phe Leu
   385                 390                 395                 400
   Leu Glu Asp Gln Tyr Ala Thr Leu Asp Lys Ile Asn Phe Leu Gln Ser
                   405                 410                 415
   Leu Phe Val Tyr Leu Gly Lys His Leu Arg Tyr Ser Asn Arg Val Asp
               420                 425                 430
   Ser Ala Asn Leu Lys Glu Phe Ser Asp Ser Asn Lys Leu Phe Glu Arg
           435                 440                 445
   Ile Leu Gln Lys Gln Lys Asp Gly Leu Phe Lys Leu Phe Glu Gln Thr
       450                 455                 460
   Asp Lys Asp Asp Glu Lys Ile Leu Ala Gln Thr His Ser Leu Ser Thr
   465 470 475 480
   Lys Ala Met Leu Leu Ala Ile Thr Arg Met Thr Asn Leu Asp Asn Asp
   485 490 495
   Glu Asp Asn Gln Lys Asn Asn Asp Lys Gly Trp Asn Phe Glu Ala Ile
   500 505 510
   Lys Asn Phe Asp Gln Lys Phe Ile Asp Ile Thr Lys Lys Asn Asn Asn
   515 520 525
   Leu Ser Leu Lys Gln Asn Lys Arg Tyr Leu Asp Asp Arg Phe Ile Asn
   530 535 540
   Asp Ala Ile Leu Ser Pro Gly Val Lys Arg Ile Leu Arg Glu Ala Thr
   545 550 555 560
   Lys Val Phe Asn Ala Ile Leu Lys Gln Phe Ser Glu Glu Tyr Asp Val
   565 570 575
   Thr Lys Val Val Ile Glu Leu Ala Arg Glu Leu Ser Glu Glu Lys Glu
   580 585 590
   Leu Glu Asn Thr Lys Asn Tyr Lys Lys Leu Ile Lys Lys Asn Gly Asp
   595 600 605
   Lys Ile Ser Glu Gly Leu Lys Ala Leu Gly Ile Ser Glu Asp Glu Ile
   610 615 620
   Lys Asp Ile Leu Lys Ser Pro Thr Lys Ser Tyr Lys Phe Leu Leu Trp
   625 630 635 640
   Leu Gln Gln Asp His Ile Asp Pro Tyr Ser Leu Lys Glu Ile Ala Phe
   645 650 655
   Asp Asp Ile Phe Thr Lys Thr Glu Lys Phe Glu Ile Asp His Ile Ile
   660 665 670
   Pro Tyr Ser Ile Ser Phe Asp Asp Ser Ser Ser Asn Lys Leu Leu Val
   675 680 685
   Leu Ala Glu Ser Asn Gln Ala Lys Ser Asn Gln Thr Pro Tyr Glu Phe
   690 695 700
   Ile Ser Ser Gly Asn Ala Gly Ile Lys Trp Glu Asp Tyr Glu Ala Tyr
   705 710 715 720
   Cys Arg Lys Phe Lys Asp Gly Asp Ser Ser Leu Leu Asp Ser Thr Gln
   725 730 735
   Arg Ser Lys Lys Phe Ala Lys Met Met Lys Thr Asp Thr Ser Ser Lys
   740 745 750
   Tyr Asp Ile Gly Phe Leu Ala Arg Asn Leu Asn Asp Thr Arg Tyr Ala
   755 760 765
   Thr Ile Val Phe Arg Asp Ala Leu Glu Asp Tyr Ala Asn Asn His Leu
   770 775 780
   Val Glu Asp Lys Pro Met Phe Lys Val Val Cys Ile Asn Gly Ser Val
   785 790 795 800
   Thr Ser Phe Leu Arg Lys Asn Phe Asp Asp Ser Ser Tyr Ala Lys Lys
   805 810 815
   Asp Arg Asp Lys Asn Ile His His Ala Val Asp Ala Ser Ile Ile Ser
   820 825 830
   Ile Phe Ser Asn Glu Thr Lys Thr Leu Phe Asn Gln Leu Thr Gln Phe
   835 840 845
   Ala Asp Tyr Lys Leu Phe Lys Asn Thr Asp Gly Ser Trp Lys Lys Ile
   850 855 860
   Asp Pro Lys Thr Gly Val Val Thr Glu Val Thr Asp Glu Asn Trp Lys
   865 870 875 880
   Gln Ile Arg Val Arg Asn Gln Val Ser Glu Ile Ala Lys Val Ile Glu
   885 890 895
   Lys Tyr Ile Gln Asp Ser Asn Ile Glu Arg Lys Ala Arg Tyr Ser Arg
   900 905 910
   Lys Ile Glu Asn Lys Thr Asn Ile Ser Leu Phe Asn Asp Thr Val Tyr
   915 920 925
   Ser Ala Lys Lys Val Gly Tyr Glu Asp Gln Ile Lys Arg Lys Asn Leu
   930 935 940
   Lys Thr Leu Asp Ile His Glu Ser Ala Lys Glu Asn Lys Asn Ser Lys
   945 950 955 960
   Val Lys Arg Gln Phe Val Tyr Arg Lys Leu Val Asn Val Ser Leu Leu
   965 970 975
   Asn Asn Asp Lys Leu Ala Asp Leu Phe Ala Glu Lys Glu Asp Ile Leu
   980 985 990
   Met Tyr Arg Ala Asn Pro Trp Val Ile Asn Leu Ala Glu Gln Ile Phe
   995 1000 1005
   Asn Glu Tyr Thr Glu Asn Lys Lys Ile Lys Ser Gln Asn Val Phe
   1010 1015 1020
   Glu Lys Tyr Met Leu Asp Leu Thr Lys Glu Phe Pro Glu Lys Phe
   1025 1030 1035
   Ser Glu Phe Leu Val Lys Ser Met Leu Arg Asn Lys Thr Ala Ile
   1040 1045 1050
   Ile Tyr Asp Asp Lys Lys Asn Ile Val His Arg Ile Lys Arg Leu
   1055 1060 1065
   Lys Met Leu Ser Ser Glu Leu Lys Glu Asn Lys Leu Ser Asn Val
   1070 1075 1080
   Ile Ile Arg Ser Lys Asn Gln Ser Gly Thr Lys Leu Ser Tyr Gln
   1085 1090 1095
   Asp Thr Ile Asn Ser Leu Ala Leu Met Ile Met Arg Ser Ile Asp
   1100 1105 1110
   Pro Thr Ala Lys Lys Gln Tyr Ile Arg Val Pro Leu Asn Thr Leu
   1115 1120 1125
   Asn Leu His Leu Gly Asp His Asp Phe Asp Leu His Asn Met Asp
   1130 1135 1140
   Ala Tyr Leu Lys Lys Pro Lys Phe Val Lys Tyr Leu Lys Ala Asn
   1145 1150 1155
   Glu Ile Gly Asp Glu Tyr Lys Pro Trp Arg Val Leu Thr Ser Gly
   1160 1165 1170
   Thr Leu Leu Ile His Lys Lys Asp Lys Lys Leu Met Tyr Ile Ser
   1175 1180 1185
   Ser Phe Gln Asn Leu Asn Asp Val Ile Glu Ile Lys Asn Leu Ile
   1190 1195 1200
   Glu Thr Glu Tyr Lys Glu Asn Asp Asp Ser Asp Ser Lys Lys Lys
       1205                1210                1215
   Lys Lys  Ala Asn Arg Phe Leu  Met Thr Leu Ser Thr  Ile Leu Asn
       1220                1225                1230
   Asp Tyr  Ile Leu Leu Asp Ala  Lys Asp Asn Phe Asp  Ile Leu Gly
       1235                1240                1245
   Leu Ser  Lys Asn Arg Ile Asp  Glu Ile Leu Asn Ser  Lys Leu Gly
       1250                1255                1260
   Leu Asp  Lys Ile Val Lys
       1265
<210> 190
   <211> 3891
   <212> DNA
   <213> Artificial Sequence
<220>
   <221> source
   <223> /note="Description of Artificial Sequence: Synthetic polynucleotide"
<400> 190
   accggtgcca ccatgtaccc atacgatgtt ccagattacg cttcgccgaa gaaaaagcgc      60
   aaggtcgaag cgtccatgaa caatagcatc aaatctaaac ctgaagtgac catcgggctg     120
   gacctgggag tgggaagcgt ggggtgggca atcgtggata acgaaacaaa catcattcac     180
   catctgggct ccaggctgtt ttctcaggcc aagactgctg aggatcggag atctttccgc     240
   ggggtgaggc gcctgatccg acggagaaaa tacaagctga aacgattcgt caatctgatt     300
   tggaagtaca acagctattt cggcttcaag aacaaagagg acatcctgaa caattatcag     360
   gagcagcaga agctgcacaa taccgtgctg aacctgaaat cagaggcact gaatgccaag     420
   atcgatccta aagcactgag ctggattctg cacgactacc tgaagaacag aggccatttt     480
   tatgaggaca atagggattt caacgtgtac ccaacaaagg agctggccaa gtacttcgat     540
   aagtacgggt actacaaggg aatcattgac agcaaggagg acaatgataa caaactggag     600
   gaagagctga caaagtacaa attctccaat aagcactggc tggaagaggt gaagaaagtc     660
   ctgtctaacc agactggcct gccagaaaag tttaaagaag agtatgagtc actgttcagc     720
   tacgtgagaa attattcaga gggcccaggg agcatcaact ctgtcagtcc ctacgggatc     780
   taccatctgg acgaaaaaga gggaaaggtg gtccagaagt acaacaacat ctgggataag     840
   acaatcggaa agtgcaacat cttccctgac gagtatagag ctcccaagaa cagtcctatc     900
   gcaatgattt tcaatgaaat caacgagctg tccacaatca ggtcatacag catctacctg     960
   actggctggt tcattaatca ggagttcaag aaagcctacc tgaacaagct gctggatctg    1020
   ctgatcaaaa ccaacggaga gaagccaatt gacgcaaggc agttcaagaa actgcgcgaa 1080
   gagacaatcg ccgaaagcat tggcaaagag acactgaagg atgtggagaa tgaagagaaa 1140
   ctggaaaagg aggaccacaa gtggaaactg aagggactga agctgaatac caacggcaaa 1200
   atccagtaca acgatctgag ctccctggct aagtttgtgc acaaactgaa gcagcatctg 1260
   aaactggatt tcctgctgga ggaccagtat gcaacactgg acaagatcaa tttcctgcag 1320
   tccctgtttg tgtacctggg caagcacctg agatattcca atagggtcga ttctgccaac 1380
   ctgaaggaat tttccgactc taacaaactg ttcgagcgca tcctgcagaa acagaaggat 1440
   gggctgttca agctgtttga acagactgac aaagacgatg agaagatcct ggcccagaca 1500
   catagtctgt caactaaggc catgctgctg gctattaccc ggatgacaaa tctggacaac 1560
   gatgaggaca accagaaaaa caatgacaag ggctggaatt ttgaggccat caaaaacttc 1620
   gatcagaagt ttatcgacat caccaagaaa aacaacaacc tgagcctgaa acagaataag 1680
   cgctacctgg acgatcgatt catcaacgat gctattctgt cccctggggt gaagcgaatc 1740
   ctgcgggagg caaccaaggt ctttaatgcc attctgaaac agttctctga agagtacgac 1800
   gtgacaaagg tggtcatcga actggctcgc gagctgagcg aagagaagga actggagaac 1860
   acaaagaact acaagaaact gatcaagaaa aacggcgaca agattagtga gggcctgaaa 1920
   gcactgggga tctcagaaga tgagatcaaa gacattctga agagtcccac taaatcatac 1980
   aagtttctgc tgtggctgca gcaggaccac atcgatcctt atagcctgaa ggagatcgcc 2040
   ttcgacgata tttttaccaa aacagaaaag ttcgagatcg accatatcat tccctacagc 2100
   atttccttcg acgattctag ttcaaacaag ctgctggtgc tggctgaaag taatcaggca 2160
   aagtcaaacc agactcctta tgagttcatc agctccggaa acgcaggcat taagtgggaa 2220
   gattacgagg cctattgccg caagttcaag gatggggact ctagtctgct ggacagcacc 2280
   cagcggtcca agaaattcgc caaaatgatg aaaaccgata cctcaagcaa gtacgacatc 2340
   ggatttctgg ctcgaaatct gaacgatact cggtacgcaa ccattgtgtt ccgggacgcc 2400
   ctggaggact atgctaataa ccacctggtc gaggacaaac ccatgtttaa ggtggtctgt 2460
   atcaatgggt ccgtgacctc tttcctgcgg aagaactttg acgattcctc ttacgccaag 2520
   aaagatagag acaagaatat ccaccatgct gtggatgcaa gtatcatctc aattttcagc 2580
   aacgagacaa agactctgtt caaccagctg actcagtttg ctgactataa actgttcaag 2640
   aacaccgatg gcagctggaa gaaaatcgac cctaagacag gggtggtcac tgaagtgacc 2700
   gacgagaatt ggaagcagat tagggtgcgc aaccaggtga gcgaaatcgc caaagtcatt 2760
   gagaagtaca tccaggatag caacatcgaa agaaaggcta ggtattcccg caaaatcgag 2820
   aataagacta acatttccct gtttaatgac accgtgtact ctgccaagaa agtcggctat 2880
   gaggatcaga tcaaaagaaa gaacctgaaa accctggaca ttcacgaatc tgctaaagag 2940
   aataagaaca gtaaagtgaa gcggcagttt gtctacagaa agctggtgaa tgtcagcctg 3000
   ctgaataacg ataagctggc agacctgttc gccgaaaaag aggatatcct gatgtatagg 3060
   gccaatccat gggtcatcaa cctggctgag cagattttca atgaatacac tgagaacaag 3120
   aaaatcaagt cccagaacgt gtttgaaaaa tatatgctgg acctgaccaa agagttcccc 3180
   gagaagttca gcgagtttct ggtgaagtcc atgctgagaa acaagaccgc catcatctac 3240
   gacgataaga aaaacattgt ccatcgaatc aaacggctga agatgctgag ttcagaactg 3300
   aaagagaata agctgtctaa cgtgatcatt aggtctaaga atcagagtgg gaccaaactg 3360
   tcataccagg atacaatcaa cagcctggcc ctgatgatta tgcgcagcat cgaccctact 3420
   gctaagaaac agtatattcg agtgccactg aataccctga acctgcacct gggagatcat 3480
   gactttgatc tgcacaatat ggatgcttac ctgaagaaac caaaattcgt gaagtatctg 3540
   aaagcaaacg aaatcggcga cgagtacaag ccctggaggg tcctgacatc tggcactctg 3600
   ctgatccata agaaggataa gaaactgatg tacatcagct ccttccagaa tctgaacgac 3660
   gtgatcgaaa ttaagaatct gatcgaaacc gagtataaag agaacgacga ttctgatagt 3720
   aagaaaaaga aaaaggcaaa ccgctttctg atgaccctga gcacaatcct gaatgactac 3780
   attctgctgg acgccaagga taacttcgac atcctggggc tgtctaaaaa tcggatcgat 3840
   gagattctga acagtaagct gggactggac aagattgtga aataagaatt c 3891
<210> 191
   <211> 137
   <212> RNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, u, g, unknown or other
<400> 191
   nnnnnnnnnn nnnnnnnnnn guuuuagucu cugaaaagag acuaaaauaa gugguuuuug 60
   gucauccacg cagguuuaca aucccuuuaa aaccauuaaa auucaaauaa acuagguugu 120
   aucaacuuag uuuuuuu 137
<210> 192
   <211> 1003
   <212> PRT
   <213> Campylobacter lari
<400> 192
   Met Arg Ile Leu Gly Phe Asp Ile Gly Ile Asn Ser Ile Gly Trp Ala
   1               5                   10                  15
   Phe Val Glu Asn Asp Glu Leu Lys Asp Cys Gly Val Arg Ile Phe Thr
               20                  25                  30
   Lys Ala Glu Asn Pro Lys Asn Lys Glu Ser Leu Ala Leu Pro Arg Arg
           35                  40                  45
   Asn Ala Arg Ser Ser Arg Arg Arg Leu Lys Arg Arg Lys Ala Arg Leu
       50                  55                  60
   Ile Ala Ile Lys Arg Ile Leu Ala Lys Glu Leu Lys Leu Asn Tyr Lys
   65                  70                  75                  80
   Asp Tyr Val Ala Ala Asp Gly Glu Leu Pro Lys Ala Tyr Glu Gly Ser
                   85                  90                  95
   Leu Ala Ser Val Tyr Glu Leu Arg Tyr Lys Ala Leu Thr Gln Asn Leu
               100                 105                 110
   Glu Thr Lys Asp Leu Ala Arg Val Ile Leu His Ile Ala Lys His Arg
           115                 120                 125
   Gly Tyr Met Asn Lys Asn Glu Lys Lys Ser Asn Asp Ala Lys Lys Gly
       130                 135                 140
   Lys Ile Leu Ser Ala Leu Lys Asn Asn Ala Leu Lys Leu Glu Asn Tyr
   145                 150                 155                 160
   Gln Ser Val Gly Glu Tyr Phe Tyr Lys Glu Phe Phe Gln Lys Tyr Lys
                   165                 170                 175
   Lys Asn Thr Lys Asn Phe Ile Lys Ile Arg Asn Thr Lys Asp Asn Tyr
               180                 185                 190
   Asn Asn Cys Val Leu Ser Ser Asp Leu Glu Lys Glu Leu Lys Leu Ile
           195                 200                 205
   Leu Glu Lys Gln Lys Glu Phe Gly Tyr Asn Tyr Ser Glu Asp Phe Ile
       210                 215                 220
   Asn Glu Ile Leu Lys Val Ala Phe Phe Gln Arg Pro Leu Lys Asp Phe
   225                 230                 235                 240
   Ser His Leu Val Gly Ala Cys Thr Phe Phe Glu Glu Glu Lys Arg Ala
   245 250 255
   Cys Lys Asn Ser Tyr Ser Ala Trp Glu Phe Val Ala Leu Thr Lys Ile
   260 265 270
   Ile Asn Glu Ile Lys Ser Leu Glu Lys Ile Ser Gly Glu Ile Val Pro
   275 280 285
   Thr Gln Thr Ile Asn Glu Val Leu Asn Leu Ile Leu Asp Lys Gly Ser
   290 295 300
   Ile Thr Tyr Lys Lys Phe Arg Ser Cys Ile Asn Leu His Glu Ser Ile
   305 310 315 320
   Ser Phe Lys Ser Leu Lys Tyr Asp Lys Glu Asn Ala Glu Asn Ala Lys
   325 330 335
   Leu Ile Asp Phe Arg Lys Leu Val Glu Phe Lys Lys Ala Leu Gly Val
   340 345 350
   His Ser Leu Ser Arg Gln Glu Leu Asp Gln Ile Ser Thr His Ile Thr
   355 360 365
   Leu Ile Lys Asp Asn Val Lys Leu Lys Thr Val Leu Glu Lys Tyr Asn
   370 375 380
   Leu Ser Asn Glu Gln Ile Asn Asn Leu Leu Glu Ile Glu Phe Asn Asp
   385 390 395 400
   Tyr Ile Asn Leu Ser Phe Lys Ala Leu Gly Met Ile Leu Pro Leu Met
   405 410 415
   Arg Glu Gly Lys Arg Tyr Asp Glu Ala Cys Glu Ile Ala Asn Leu Lys
   420 425 430
   Pro Lys Thr Val Asp Glu Lys Lys Asp Phe Leu Pro Ala Phe Cys Asp
   435 440 445
   Ser Ile Phe Ala His Glu Leu Ser Asn Pro Val Val Asn Arg Ala Ile
   450 455 460
   Ser Glu Tyr Arg Lys Val Leu Asn Ala Leu Leu Lys Lys Tyr Gly Lys
   465 470 475 480
   Val His Lys Ile His Leu Glu Leu Ala Arg Asp Val Gly Leu Ser Lys
   485 490 495
   Lys Ala Arg Glu Lys Ile Glu Lys Glu Gln Lys Glu Asn Gln Ala Val
   500 505 510
   Asn Ala Trp Ala Leu Lys Glu Cys Glu Asn Ile Gly Leu Lys Ala Ser
   515 520 525
   Ala Lys Asn Ile Leu Lys Leu Lys Leu Trp Lys Glu Gln Lys Glu Ile
   530 535 540
   Cys Ile Tyr Ser Gly Asn Lys Ile Ser Ile Glu His Leu Lys Asp Glu
   545 550 555 560
   Lys Ala Leu Glu Val Asp His Ile Tyr Pro Tyr Ser Arg Ser Phe Asp
   565 570 575
   Asp Ser Phe Ile Asn Lys Val Leu Val Phe Thr Lys Glu Asn Gln Glu
   580 585 590
   Lys Leu Asn Lys Thr Pro Phe Glu Ala Phe Gly Lys Asn Ile Glu Lys
   595 600 605
   Trp Ser Lys Ile Gln Thr Leu Ala Gln Asn Leu Pro Tyr Lys Lys Lys
   610 615 620
   Asn Lys Ile Leu Asp Glu Asn Phe Lys Asp Lys Gln Gln Glu Asp Phe
   625 630 635 640
   Ile Ser Arg Asn Leu Asn Asp Thr Arg Tyr Ile Ala Thr Leu Ile Ala
   645 650 655
   Lys Tyr Thr Lys Glu Tyr Leu Asn Phe Leu Leu Leu Ser Glu Asn Glu
   660 665 670
   Asn Ala Asn Leu Lys Ser Gly Glu Lys Gly Ser Lys Ile His Val Gln
   675 680 685
   Thr Ile Ser Gly Met Leu Thr Ser Val Leu Arg His Thr Trp Gly Phe
   690 695 700
   Asp Lys Lys Asp Arg Asn Asn His Leu His His Ala Leu Asp Ala Ile
   705 710 715 720
   Ile Val Ala Tyr Ser Thr Asn Ser Ile Ile Lys Ala Phe Ser Asp Phe
   725 730 735
   Arg Lys Asn Gln Glu Leu Leu Lys Ala Arg Phe Tyr Ala Lys Glu Leu
   740 745 750
   Thr Ser Asp Asn Tyr Lys His Gln Val Lys Phe Phe Glu Pro Phe Lys
           755                 760                 765
   Ser Phe Arg Glu Lys Ile Leu Ser Lys Ile Asp Glu Ile Phe Val Ser
       770                 775                 780
   Lys Pro Pro Arg Lys Arg Ala Arg Arg Ala Leu His Lys Asp Thr Phe
   785                 790                 795                 800
   His Ser Glu Asn Lys Ile Ile Asp Lys Cys Ser Tyr Asn Ser Lys Glu
                   805                 810                 815
   Gly Leu Gln Ile Ala Leu Ser Cys Gly Arg Val Arg Lys Ile Gly Thr
               820                 825                 830
   Lys Tyr Val Glu Asn Asp Thr Ile Val Arg Val Asp Ile Phe Lys Lys
           835                 840                 845
   Gln Asn Lys Phe Tyr Ala Ile Pro Ile Tyr Ala Met Asp Phe Ala Leu
       850                 855                 860
   Gly Ile Leu Pro Asn Lys Ile Val Ile Thr Gly Lys Asp Lys Asn Asn
   865                 870                 875                 880
   Asn Pro Lys Gln Trp Gln Thr Ile Asp Glu Ser Tyr Glu Phe Cys Phe
                   885                 890                 895
   Ser Leu Tyr Lys Asn Asp Leu Ile Leu Leu Gln Lys Lys Asn Met Gln
               900                 905                 910
   Glu Pro Glu Phe Ala Tyr Tyr Asn Asp Phe Ser Ile Ser Thr Ser Ser
           915                 920                 925
   Ile Cys Val Glu Lys His Asp Asn Lys Phe Glu Asn Leu Thr Ser Asn
       930                 935                 940
   Gln Lys Leu Leu Phe Ser Asn Ala Lys Glu Gly Ser Val Lys Val Glu
   945                 950                 955                 960
   Ser Leu Gly Ile Gln Asn Leu Lys Val Phe Glu Lys Tyr Ile Ile Thr
                   965                 970                 975
   Pro Leu Gly Asp Lys Ile Lys Ala Asp Phe Gln Pro Arg Glu Asn Ile
               980                 985                 990
   Ser Leu Lys Thr Ser Lys Lys Tyr  Gly Leu Arg
           995                 1000
<210> 193
   <211> 3093
   <212> DNA
   <213> Artificial Sequence
<220>
   <221> source
   <223> /note="Description of Artificial Sequence: Synthetic polynucleotide"
<400> 193
   accggtgcca ccatgtaccc atacgatgtt ccagattacg cttcgccgaa gaaaaagcgc       60
   aaggtcgaag cgtccatgag gattctgggg tttgacattg gcattaacag catcgggtgg      120
   gcttttgtgg agaacgacga actgaaggac tgcggagtgc ggatcttcac aaaggccgag      180
   aacccaaaaa ataaggaaag cctggcactg ccccggagaa atgcacgcag ctccaggcgc      240
   cgactgaaac ggagaaaggc ccggctgatc gctattaaga gaatcctggc caaagagctg      300
   aagctgaact acaaggacta tgtcgcagct gatggagagc tgccaaaggc ctacgaagga      360
   tccctggcat ctgtgtacga gctgcggtat aaggccctga cacagaacct ggaaactaaa      420
   gatctggcca gagtgatcct gcacattgct aagcataggg ggtacatgaa caagaacgag      480
   aagaaatcaa acgacgctaa gaaaggaaag atcctgagcg ctctgaaaaa caatgcactg      540
   aagctggaga actaccagag cgtgggcgaa tacttctaca aggagttctt tcagaaatac      600
   aagaaaaaca caaagaactt catcaagatc cgcaacacta aggataatta caacaattgc      660
   gtgctgtcta gtgacctgga aaaagagctg aagctgatcc tggaaaaaca gaaggagttc      720
   ggctacaact actctgaaga tttcatcaac gagattctga aggtcgcctt ctttcagcgg      780
   cccctgaagg acttcagtca cctggtgggg gcctgcactt tctttgagga agagaaaagg      840
   gcctgtaaga acagctactc tgcctgggag tttgtggctc tgaccaagat cattaacgag      900
   atcaagagcc tggagaagat cagcggcgaa attgtgccaa cccagacaat caacgaggtc      960
   ctgaatctga tcctggacaa ggggtctatc acctacaaga aattcagaag ttgtatcaat     1020
   ctgcatgaga gtatcagctt caagagcctg aagtatgata aagaaaacgc cgagaatgct     1080
   aaactgatcg acttccgcaa gctggtggag tttaagaaag ccctgggagt ccacagcctg     1140
   tcccggcagg aactggatca gatctccact catatcaccc tgattaagga caacgtgaag     1200
   ctgaaaaccg tcctggagaa atacaacctg agtaatgaac agatcaacaa tctgctggaa     1260
   attgagttca acgattatat caacctgagc ttcaaggccc tgggaatgat tctgccactg     1320
   atgcgcgagg gcaaacgata cgacgaggcc tgcgagatcg ccaatctgaa acctaagacc     1380
   gtggacgaga agaaagattt cctgccagca ttttgtgatt ccattttcgc ccacgagctg     1440
   tctaaccccg tggtcaatag ggctatcagc gaataccgca aggtgctgaa cgcactgctg     1500
   aagaaatatg gaaaggtcca caaaattcat ctggagctgg ctcgcgacgt gggcctgtcc      1560
   aagaaagcac gagagaagat cgaaaaagag cagaaggaaa accaggccgt gaatgcatgg      1620
   gccctgaagg aatgcgagaa tattggcctg aaggccagcg caaagaacat cctgaaactg      1680
   aagctgtgga aagaacagaa ggagatctgt atctactccg gaaataagat ctctattgag      1740
   cacctgaaag atgaaaaggc cctggaggtg gaccatatct acccctattc taggagtttc      1800
   gacgattctt ttatcaacaa agtgctggtg ttcaccaagg aaaatcagga gaaactgaac      1860
   aagacacctt tcgaggcctt tggcaagaat attgaaaaat ggagcaagat ccagaccctg      1920
   gctcagaacc tgccatacaa gaaaaagaat aagattctgg acgagaactt caaagataag      1980
   cagcaggagg actttatctc tcgaaatctg aacgacaccc ggtatatcgc tacactgatt      2040
   gcaaaataca caaaggagta tctgaacttc ctgctgctga gcgaaaatga gaacgccaat      2100
   ctgaagagtg gcgaaaaagg gtcaaagatc cacgtgcaga ctattagcgg gatgctgacc      2160
   tccgtcctga ggcacacatg ggggtttgac aaaaaggatc gcaacaatca tctgcaccat      2220
   gcactggatg ccatcattgt ggcctacagt acaaattcaa tcattaaggc tttcagcgat      2280
   ttccggaaaa accaggagct gctgaaggcc agattctacg ctaaagaact gacttccgat      2340
   aactataaac atcaggtcaa gttctttgag cctttcaaga gttttagaga aaaaatcctg      2400
   tcaaagatcg acgagatttt cgtgtccaaa ccacctcgaa agcgagctag gcgcgcactg      2460
   cacaaggata cctttcattc tgagaacaag atcattgaca agtgcagcta caactccaag      2520
   gaaggcctgc agattgccct gagctgtgga agagtgagga aaatcggcac taagtatgtc      2580
   gagaatgata ccatcgtgag ggtcgacatt ttcaaaaagc agaacaagtt ttacgctatc      2640
   ccaatctacg caatggattt tgccctgggg atcctgccca ataagatcgt gattactgga      2700
   aaagataaga acaataaccc caaacagtgg cagaccattg acgaatcata cgagttctgc      2760
   tttagcctgt ataagaatga cctgatcctg ctgcagaaaa agaacatgca ggaacctgag      2820
   ttcgcctact ataacgattt ttcaatcagc acatcaagca tttgtgtgga gaaacacgac      2880
   aacaagttcg aaaatctgac tagcaaccag aagctgctgt tttccaatgc aaaagagggc      2940
   tctgtgaagg tcgaaagtct ggggatccag aacctgaaag tgttcgagaa gtacatcatt      3000
   acccccctgg gagataaaat taaggctgac tttcagcctc gagaaaacat cagcctgaaa      3060
   accagtaaaa agtatggcct gaggtaagaa ttc                                    3093
<210> 194
   <211> 103
   <212> RNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, u, g, unknown or other
<400> 194
   nnnnnnnnnn nnnnnnnnnn guuuuagagc uagaaauagc aaguuaaaau aaggcuaguc        60
   cguuaucaac uugaaaaagu ggcaccgagu cggugcuuuu uuu                        103
<210> 195
   <211> 1368
   <212> PRT
   <213> Streptococcus pyogenes
<400> 195
<210> 196
   <211> 4218
   <212> DNA
   <213> Artificial Sequence
<220>
   <221> source
   <223> /note="Description of Artificial Sequence: Synthetic polynucleotide"
<400> 196
   accggtgcca ccatgtaccc atacgatgtt ccagattacg cttcgccgaa gaaaaagcgc    60
   aaggtcgaag cgtccgacaa gaagtacagc atcggcctgg acatcggcac caactctgtg   120
   ggctgggccg tgatcaccga cgagtacaag gtgcccagca agaaattcaa ggtgctgggc   180
   aacaccgacc ggcacagcat caagaagaac ctgatcggag ccctgctgtt cgacagcggc   240
   gaaacagccg aggccacccg gctgaagaga accgccagaa gaagatacac cagacggaag   300
   aaccggatct gctatctgca agagatcttc agcaacgaga tggccaaggt ggacgacagc   360
   ttcttccaca gactggaaga gtccttcctg gtggaagagg ataagaagca cgagcggcac   420
   cccatcttcg gcaacatcgt ggacgaggtg gcctaccacg agaagtaccc caccatctac   480
   cacctgagaa agaaactggt ggacagcacc gacaaggccg acctgcggct gatctatctg   540
   gccctggccc acatgatcaa gttccggggc cacttcctga tcgagggcga cctgaacccc   600
   gacaacagcg acgtggacaa gctgttcatc cagctggtgc agacctacaa ccagctgttc   660
   gaggaaaacc ccatcaacgc cagcggcgtg gacgccaagg ccatcctgtc tgccagactg   720
   agcaagagca gacggctgga aaatctgatc gcccagctgc ccggcgagaa gaagaatggc   780
   ctgttcggca acctgattgc cctgagcctg ggcctgaccc ccaacttcaa gagcaacttc   840
   gacctggccg aggatgccaa actgcagctg agcaaggaca cctacgacga cgacctggac   900
   aacctgctgg cccagatcgg cgaccagtac gccgacctgt ttctggccgc caagaacctg   960
   tccgacgcca tcctgctgag cgacatcctg agagtgaaca ccgagatcac caaggccccc  1020
   ctgagcgcct ctatgatcaa gagatacgac gagcaccacc aggacctgac cctgctgaaa  1080
   gctctcgtgc ggcagcagct gcctgagaag tacaaagaga ttttcttcga ccagagcaag  1140
   aacggctacg ccggctacat tgacggcgga gccagccagg aagagttcta caagttcatc  1200
   aagcccatcc tggaaaagat ggacggcacc gaggaactgc tcgtgaagct gaacagagag  1260
   gacctgctgc ggaagcagcg gaccttcgac aacggcagca tcccccacca gatccacctg  1320
   ggagagctgc acgccattct gcggcggcag gaagattttt acccattcct gaaggacaac  1380
   cgggaaaaga tcgagaagat cctgaccttc cgcatcccct actacgtggg ccctctggcc  1440
   aggggaaaca gcagattcgc ctggatgacc agaaagagcg aggaaaccat caccccctgg  1500
   aacttcgagg aagtggtgga caagggcgct tccgcccaga gcttcatcga gcggatgacc  1560
   aacttcgata agaacctgcc caacgagaag gtgctgccca agcacagcct gctgtacgag  1620
   tacttcaccg tgtataacga gctgaccaaa gtgaaatacg tgaccgaggg aatgagaaag  1680
   cccgccttcc tgagcggcga gcagaaaaag gccatcgtgg acctgctgtt caagaccaac  1740
   cggaaagtga ccgtgaagca gctgaaagag gactacttca agaaaatcga gtgcttcgac  1800
   tccgtggaaa tctccggcgt ggaagatcgg ttcaacgcct ccctgggcac ataccacgat  1860
   ctgctgaaaa ttatcaagga caaggacttc ctggacaatg aggaaaacga ggacattctg 1920
   gaagatatcg tgctgaccct gacactgttt gaggacagag agatgatcga ggaacggctg 1980
   aaaacctatg cccacctgtt cgacgacaaa gtgatgaagc agctgaagcg gcggagatac 2040
   accggctggg gcaggctgag ccggaagctg atcaacggca tccgggacaa gcagtccggc 2100
   aagacaatcc tggatttcct gaagtccgac ggcttcgcca acagaaactt catgcagctg 2160
   atccacgacg acagcctgac ctttaaagag gacatccaga aagcccaggt gtccggccag 2220
   ggcgatagcc tgcacgagca cattgccaat ctggccggca gccccgccat taagaagggc 2280
   atcctgcaga cagtgaaggt ggtggacgag ctcgtgaaag tgatgggccg gcacaagccc 2340
   gagaacatcg tgatcgaaat ggccagagag aaccagacca cccagaaggg acagaagaac 2400
   agccgcgaga gaatgaagcg gatcgaagag ggcatcaaag agctgggcag ccagatcctg 2460
   aaagaacacc ccgtggaaaa cacccagctg cagaacgaga agctgtacct gtactacctg 2520
   cagaatgggc gggatatgta cgtggaccag gaactggaca tcaaccggct gtccgactac 2580
   gatgtggacc atatcgtgcc tcagagcttt ctgaaggacg actccatcga caacaaggtg 2640
   ctgaccagaa gcgacaagaa ccggggcaag agcgacaacg tgccctccga agaggtcgtg 2700
   aagaagatga agaactactg gcggcagctg ctgaacgcca agctgattac ccagagaaag 2760
   ttcgacaatc tgaccaaggc cgagagaggc ggcctgagcg aactggataa ggccggcttc 2820
   atcaagagac agctggtgga aacccggcag atcacaaagc acgtggcaca gatcctggac 2880
   tcccggatga acactaagta cgacgagaat gacaagctga tccgggaagt gaaagtgatc 2940
   accctgaagt ccaagctggt gtccgatttc cggaaggatt tccagtttta caaagtgcgc 3000
   gagatcaaca actaccacca cgcccacgac gcctacctga acgccgtcgt gggaaccgcc 3060
   ctgatcaaaa agtaccctaa gctggaaagc gagttcgtgt acggcgacta caaggtgtac 3120
   gacgtgcgga agatgatcgc caagagcgag caggaaatcg gcaaggctac cgccaagtac 3180
   ttcttctaca gcaacatcat gaactttttc aagaccgaga ttaccctggc caacggcgag 3240
   atccggaagc ggcctctgat cgagacaaac ggcgaaaccg gggagatcgt gtgggataag 3300
   ggccgggatt ttgccaccgt gcggaaagtg ctgagcatgc cccaagtgaa tatcgtgaaa 3360
   aagaccgagg tgcagacagg cggcttcagc aaagagtcta tcctgcccaa gaggaacagc 3420
   gataagctga tcgccagaaa gaaggactgg gaccctaaga agtacggcgg cttcgacagc 3480
   cccaccgtgg cctattctgt gctggtggtg gccaaagtgg aaaagggcaa gtccaagaaa 3540
   ctgaagagtg tgaaagagct gctggggatc accatcatgg aaagaagcag cttcgagaag 3600
   aatcccatcg actttctgga agccaagggc tacaaagaag tgaaaaagga cctgatcatc 3660
   aagctgccta agtactccct gttcgagctg gaaaacggcc ggaagagaat gctggcctct 3720
   gccggcgaac tgcagaaggg aaacgaactg gccctgccct ccaaatatgt gaacttcctg 3780
   tacctggcca gccactatga gaagctgaag ggctcccccg aggataatga gcagaaacag     3840
   ctgtttgtgg aacagcacaa gcactacctg gacgagatca tcgagcagat cagcgagttc     3900
   tccaagagag tgatcctggc cgacgctaat ctggacaaag tgctgtccgc ctacaacaag     3960
   caccgggata agcccatcag agagcaggcc gagaatatca tccacctgtt taccctgacc     4020
   aatctgggag cccctgccgc cttcaagtac tttgacacca ccatcgaccg gaagaggtac     4080
   accagcacca aagaggtgct ggacgccacc ctgatccacc agagcatcac cggcctgtac     4140
   gagacacgga tcgacctgtc tcagctggga ggcgacagcc ccaagaagaa gagaaaggtg     4200
   gaggccagct aagaattc                                                   4218
<210> 197
   <211> 116
   <212> RNA
   <213> Artificial Sequence
<220>
   <221> source
   <223> /note="Description of Artificial Sequence: Synthetic polynucleotide"
<220>
   <221> modified_base
   <222> (1)..(20)
   <223> a, c, u, g, unknown or other
<400> 197
   nnnnnnnnnn nnnnnnnnnn guuuuuguac ucucaagauu uagaaacuug cagaagcuac       60
   aaagauaagg cuucaugccg aaaucaacac ccugucauuu uauggcaggg uguuuu          116
<210> 198
   <211> 1120
   <212> PRT
   <213> Streptococcus thermophilus
<400> 198
   Met Ser Asp Leu Val Leu Gly Leu Asp Ile Gly Ile Gly Ser Val Gly
   1               5                   10                  15
   Val Gly Ile Leu Asn Lys Val Thr Gly Glu Ile Ile His Lys Asn Ser
               20                  25                  30
   Arg Ile Phe Pro Ala Ala Gln Ala Glu Asn Asn Leu Val Arg Arg Thr
           35                  40                  45
   Asn Arg Gln Gly Arg Arg Leu Ala Arg Arg Lys Lys His Arg Arg Val
       50                  55                  60
   Arg Leu Asn Arg Leu Phe Glu Glu Ser Gly Leu Ile Thr Asp Phe Thr
   65 70 75 80
   Lys Ile Ser Ile Asn Leu Asn Pro Tyr Gln Leu Arg Val Lys Gly Leu
   85 90 95
   Thr Asp Glu Leu Ser Asn Glu Glu Leu Phe Ile Ala Leu Lys Asn Met
   100 105 110
   Val Lys His Arg Gly Ile Ser Tyr Leu Asp Asp Ala Ser Asp Asp Gly
   115 120 125
   Asn Ser Ser Val Gly Asp Tyr Ala Gln Ile Val Lys Glu Asn Ser Lys
   130 135 140
   Gln Leu Glu Thr Lys Thr Pro Gly Gln Ile Gln Leu Glu Arg Tyr Gln
   145 150 155 160
   Thr Tyr Gly Gln Leu Arg Gly Asp Phe Thr Val Glu Lys Asp Gly Lys
   165 170 175
   Lys His Arg Leu Ile Asn Val Phe Pro Thr Ser Ala Tyr Arg Ser Glu
   180 185 190
   Ala Leu Arg Ile Leu Gln Thr Gln Gln Glu Phe Asn Pro Gln Ile Thr
   195 200 205
   Asp Glu Phe Ile Asn Arg Tyr Leu Glu Ile Leu Thr Gly Lys Arg Lys
   210 215 220
   Tyr Tyr His Gly Pro Gly Asn Glu Lys Ser Arg Thr Asp Tyr Gly Arg
   225 230 235 240
   Tyr Arg Thr Ser Gly Glu Thr Leu Asp Asn Ile Phe Gly Ile Leu Ile
   245 250 255
   Gly Lys Cys Thr Phe Tyr Pro Asp Glu Phe Arg Ala Ala Lys Ala Ser
   260 265 270
   Tyr Thr Ala Gln Glu Phe Asn Leu Leu Asn Asp Leu Asn Asn Leu Thr
   275 280 285
   Val Pro Thr Glu Thr Lys Lys Leu Ser Lys Glu Gln Lys Asn Gln Ile
   290 295 300
   Ile Asn Tyr Val Lys Asn Glu Lys Ala Met Gly Pro Ala Lys Leu Phe
   305 310 315 320
   Lys Tyr Ile Ala Lys Leu Leu Ser Cys Asp Val Ala Asp Ile Lys Gly
                   325                 330                 335
   Tyr Arg Ile Asp Lys Ser Gly Lys Ala Glu Ile His Thr Phe Glu Ala
               340                 345                 350
   Tyr Arg Lys Met Lys Thr Leu Glu Thr Leu Asp Ile Glu Gln Met Asp
           355                 360                 365
   Arg Glu Thr Leu Asp Lys Leu Ala Tyr Val Leu Thr Leu Asn Thr Glu
       370                 375                 380
   Arg Glu Gly Ile Gln Glu Ala Leu Glu His Glu Phe Ala Asp Gly Ser
   385                 390                 395                 400
   Phe Ser Gln Lys Gln Val Asp Glu Leu Val Gln Phe Arg Lys Ala Asn
                   405                 410                 415
   Ser Ser Ile Phe Gly Lys Gly Trp His Asn Phe Ser Val Lys Leu Met
               420                 425                 430
   Met Glu Leu Ile Pro Glu Leu Tyr Glu Thr Ser Glu Glu Gln Met Thr
           435                 440                 445
   Ile Leu Thr Arg Leu Gly Lys Gln Lys Thr Thr Ser Ser Ser Asn Lys
       450                 455                 460
   Thr Lys Tyr Ile Asp Glu Lys Leu Leu Thr Glu Glu Ile Tyr Asn Pro
   465                 470                 475                 480
   Val Val Ala Lys Ser Val Arg Gln Ala Ile Lys Ile Val Asn Ala Ala
                   485                 490                 495
   Ile Lys Glu Tyr Gly Asp Phe Asp Asn Ile Val Ile Glu Met Ala Arg
               500                 505                 510
   Glu Thr Asn Glu Asp Asp Glu Lys Lys Ala Ile Gln Lys Ile Gln Lys
           515                 520                 525
   Ala Asn Lys Asp Glu Lys Asp Ala Ala Met Leu Lys Ala Ala Asn Gln
       530                 535                 540
   Tyr Asn Gly Lys Ala Glu Leu Pro His Ser Val Phe His Gly His Lys
   545                 550                 555                 560
   Gln Leu Ala Thr Lys Ile Arg Leu Trp His Gln Gln Gly Glu Arg Cys
                   565                 570                 575
   Leu Tyr Thr Gly Lys Thr Ile Ser Ile His Asp Leu Ile Asn Asn Ser
               580                 585                 590
   Asn Gln Phe Glu Val Asp His Ile Leu Pro Leu Ser Ile Thr Phe Asp
           595                 600                 605
   Asp Ser Leu Ala Asn Lys Val Leu Val Tyr Ala Thr Ala Asn Gln Glu
       610                 615                 620
   Lys Gly Gln Arg Thr Pro Tyr Gln Ala Leu Asp Ser Met Asp Asp Ala
   625                 630                 635                 640
   Trp Ser Phe Arg Glu Leu Lys Ala Phe Val Arg Glu Ser Lys Thr Leu
                   645                 650                 655
   Ser Asn Lys Lys Lys Glu Tyr Leu Leu Thr Glu Glu Asp Ile Ser Lys
               660                 665                 670
   Phe Asp Val Arg Lys Lys Phe Ile Glu Arg Asn Leu Val Asp Thr Arg
           675                 680                 685
   Tyr Ala Ser Arg Val Val Leu Asn Ala Leu Gln Glu His Phe Arg Ala
       690                 695                 700
   His Lys Ile Asp Thr Lys Val Ser Val Val Arg Gly Gln Phe Thr Ser
   705                 710                 715                 720
   Gln Leu Arg Arg His Trp Gly Ile Glu Lys Thr Arg Asp Thr Tyr His
                   725                 730                 735
   His His Ala Val Asp Ala Leu Ile Ile Ala Ala Ser Ser Gln Leu Asn
               740                 745                 750
   Leu Trp Lys Lys Gln Lys Asn Thr Leu Val Ser Tyr Ser Glu Asp Gln
           755                 760                 765
   Leu Leu Asp Ile Glu Thr Gly Glu Leu Ile Ser Asp Asp Glu Tyr Lys
       770                 775                 780
   Glu Ser Val Phe Lys Ala Pro Tyr Gln His Phe Val Asp Thr Leu Lys
   785                 790                 795                 800
   Ser Lys Glu Phe Glu Asp Ser Ile Leu Phe Ser Tyr Gln Val Asp Ser
                   805                 810                 815
   Lys Phe Asn Arg Lys Ile Ser Asp Ala Thr Ile Tyr Ala Thr Arg Gln
               820                 825                 830
   Ala Lys Val Gly Lys Asp Lys Ala Asp Glu Thr Tyr Val Leu Gly Lys
   835 840 845
   Ile Lys Asp Ile Tyr Thr Gln Asp Gly Tyr Asp Ala Phe Met Lys Ile
   850 855 860
   Tyr Lys Lys Asp Lys Ser Lys Phe Leu Met Tyr Arg His Asp Pro Gln
   865 870 875 880
   Thr Phe Glu Lys Val Ile Glu Pro Ile Leu Glu Asn Tyr Pro Asn Lys
   885 890 895
   Gln Ile Asn Glu Lys Gly Lys Glu Val Pro Cys Asn Pro Phe Leu Lys
   900 905 910
   Tyr Lys Glu Glu His Gly Tyr Ile Arg Lys Tyr Ser Lys Lys Gly Asn
   915 920 925
   Gly Pro Glu Ile Lys Ser Leu Lys Tyr Tyr Asp Ser Lys Leu Gly Asn
   930 935 940
   His Ile Asp Ile Thr Pro Lys Asp Ser Asn Asn Lys Val Val Leu Gln
   945 950 955 960
   Ser Val Ser Pro Trp Arg Ala Asp Val Tyr Phe Asn Lys Thr Thr Gly
   965 970 975
   Lys Tyr Glu Ile Leu Gly Leu Lys Tyr Ala Asp Leu Gln Phe Glu Lys
   980 985 990
   Gly Thr Gly Thr Tyr Lys Ile Ser Gln Glu Lys Tyr Asn Asp Ile Lys
   995 1000 1005
   Lys Lys Glu Gly Val Asp Ser Asp Ser Glu Phe Lys Phe Thr Leu
   1010 1015 1020
   Tyr Lys Asn Asp Leu Leu Leu Val Lys Asp Thr Glu Thr Lys Glu
   1025 1030 1035
   Gln Gln Leu Phe Arg Phe Leu Ser Arg Thr Met Pro Lys Gln Lys
   1040 1045 1050
   His Tyr Val Glu Leu Lys Pro Tyr Asp Lys Gln Lys Phe Glu Gly
   1055 1060 1065
   Gly Glu Ala Leu Ile Lys Val Leu Gly Asn Val Ala Asn Ser Gly
   1070 1075 1080
   Gln Cys Lys Lys Gly Leu Gly Lys Ser Asn Ile Ser Ile Tyr Lys
   1085 1090 1095
   Val Arg Thr Asp Val Leu Gly Asn Gln His Ile Ile Lys Asn Glu
   1100 1105 1110
   Gly Asp Lys Pro Lys Leu Met
   1115 1120
<210> 199
   <211> 3477
   <212> DNA
   <213> Artificial Sequence
<220>
   <221> source
   <223> /note="Description of Artificial Sequence: Synthetic polynucleotide"
<400> 199
   accggtgcca ccatgtaccc atacgatgtt ccagattacg cttcgccgaa gaaaaagcgc        60
   aaggtcgaag cgtcctccga cctggtactt ggactggata ttggtatcgg ttcggtggga       120
   gtcggaatcc tcaacaaggt cacgggggag atcattcaca agaactcgcg gatcttcccc       180
   gcagctcagg ctgagaacaa cttggtgcgg agaacgaata ggcagggcag gcgactggcg       240
   aggaggaaga aacacaggag agtccgattg aaccggctgt tcgaggagtc cggtttgatc       300
   accgacttta cgaaaatctc gattaacctt aatccctatc agcttcgggt gaaaggcctg       360
   acagacgaac tttcgaatga ggaacttttc atcgcgctga aaaacatggt caagcacaga       420
   gggatttcct acctcgatga cgcctcggat gacggaaatt cctcagtagg agattatgca       480
   cagatcgtga aagagaactc aaagcaactg gaaacaaaga caccggggca gatccaactt       540
   gaaagatacc agacatacgg acagctcaga ggagatttta cggtggagaa ggacggtaaa       600
   aagcacagac tcattaacgt atttcccacg tcggcgtaca gatccgaagc gctccgcatc       660
   cttcagactc aacaggagtt caacccgcaa attactgatg agttcatcaa ccgctatttg       720
   gaaatcttga ccggaaagcg caagtattat catgggccgg gtaatgagaa atccagaaca       780
   gattacggcc gatacagaac ttcgggggaa accttggata acatctttgg tattttgatt       840
   ggaaagtgca ccttttaccc ggacgagttt cgagcggcca aggcgtcata cacagcacaa       900
   gagtttaatc tcttgaatga tttgaacaac ttgacggtcc ccacggagac aaagaagctc       960
   tccaaagagc aaaagaacca aatcatcaac tacgtcaaga acgagaaggc tatggggcca      1020
   gcgaagctgt tcaagtatat cgctaaactt ctcagctgtg atgtggcgga catcaaaggg      1080
   taccgaatcg acaagtcggg aaaagcggaa attcacacgt ttgaagcata tcgaaagatg      1140
   aaaacgttgg aaacactgga cattgagcag atggaccggg aaacgctcga caaactggca 1200
   tacgtgctca cgttgaatac tgaacgagag ggaatccaag aggcccttga acatgagttc 1260
   gccgatggat cgttcagcca gaagcaggtc gacgaacttg tgcaattccg caaggcgaat 1320
   agctccatct tcgggaaggg atggcacaac ttttcggtca aactcatgat ggagttgatc 1380
   ccagaacttt atgagacttc ggaggagcaa atgacgatct tgacgcgctt ggggaaacag 1440
   aaaacgacaa gctcatcgaa caaaactaag tacattgatg agaaattgct gacggaagaa 1500
   atctataatc cggtagtagc gaaatcggta agacaagcga tcaaaatcgt gaacgcggcg 1560
   atcaaggaat atggtgactt tgataacatc gtaattgaaa tggctagaga gacgaacgaa 1620
   gatgacgaga aaaaggcaat ccagaagatc cagaaggcca acaaggatga aaaagatgca 1680
   gcgatgctta aagcggccaa ccaatacaat ggaaaggcgg agctgcccca ttcagtgttt 1740
   cacggtcata aacagttggc gaccaagatc cgactctggc atcagcaggg tgagcggtgt 1800
   ctctacaccg gaaagactat ctccatccat gacttgatta acaattcgaa ccagtttgaa 1860
   gtggatcata ttctgcccct gtcaatcacc tttgacgact cgcttgcgaa caaggtgctc 1920
   gtgtacgcaa cggcaaatca ggagaaaggc cagcggactc cgtatcaggc gctcgactca 1980
   atggacgatg cgtggtcatt ccgggagctg aaggcgttcg tacgcgagag caagacactg 2040
   agcaacaaaa agaaagagta tctgctgaca gaggaggaca tctcgaaatt cgatgtcagg 2100
   aagaagttca tcgagcggaa tcttgtcgac actcgctacg cttccagagt agtactgaac 2160
   gcgctccagg aacactttag agcgcacaaa attgacacga aggtgtcagt ggtgagaggg 2220
   cagttcacat cccaactccg ccgacattgg ggcatcgaaa agacgcggga cacatatcac 2280
   catcatgcgg tggacgcgct gattattgcc gcttcgtccc agttgaatct ctggaaaaag 2340
   cagaagaaca cgctggtgtc gtattcggag gatcagcttt tggacatcga aaccggggag 2400
   ctgatttccg acgatgaata caaagaatcg gtgtttaagg caccatatca gcatttcgtg 2460
   gacacgctga agagcaaaga gtttgaggac agcatcctct tttcgtacca agtggactcg 2520
   aagtttaatc gcaagatttc agacgccaca atctacgcga cgaggcaggc gaaggtgggc 2580
   aaagataaag cagatgaaac ctacgtcctt ggtaaaatca aggacatcta cactcaggac 2640
   gggtacgatg cgttcatgaa aatctacaag aaggataagt cgaagtttct catgtaccgc 2700
   cacgatccac agactttcga aaaagtcatt gagcctattt tggagaacta ccctaacaag 2760
   caaatcaacg agaaagggaa agaagtcccg tgcaacccct ttctgaagta caaggaagag 2820
   cacggttata tccgcaaata ctcgaagaaa ggaaatgggc ctgagattaa gtcgcttaag 2880
   tattacgact caaagttggg taaccacatc gacattaccc cgaaagactc caacaacaaa 2940
   gtcgtgttgc agtccgtctc gccctggcga gcagatgtgt attttaataa gacgaccggc 3000
   aaatatgaga tccttggact caaatacgca gaccttcaat tcgaaaaggg gacgggcact 3060
   tataagattt cacaagagaa gtacaacgac atcaagaaaa aggaaggggt cgattcagat 3120
   tcggagttca aattcaccct ctacaaaaac gacctcctgc ttgtgaagga cacagaaacg 3180
   aaggagcagc agctctttcg gttcctctca cgcacgatgc ccaaacaaaa acattacgtc 3240
   gaacttaaac cttacgataa gcaaaagttt gaagggggag aggcactgat caaagtattg 3300
   ggtaacgtag ccaatagcgg acagtgtaag aaagggctgg gaaagtccaa tatctcgatc 3360
   tataaagtac gaacagatgt attgggaaac cagcatatca tcaaaaatga gggggataaa 3420
   cccaaactcg atttcagccc caagaagaag agaaaggtgg aggccagcta agaattc 3477
<210> 200
   <211> 27
   <212> DNA
   <213> Artificial Sequence
<220>
   <221> source
   <223> /note="Description of Artificial Sequence: Synthetic oligonucleotide"
<400> 200
   gcccgggtgg aactggtagc catgaat 27
<210> 201
   <211> 26
   <212> DNA
   <213> Artificial Sequence
<220>
   <221> source
   <223> /note="Description of Artificial Sequence: Synthetic oligonucleotide"
<400> 201
   gttgaagatg aagcccagag cggagt 26
<210> 202
   <211> 27
   <212> DNA
   <213> Artificial Sequence
<220>
   <221> source
   <223> /note="Description of Artificial Sequence: Synthetic oligonucleotide"
<400> 202
   gcttccgacg aggtggccat caaggat 27
<210> 203
   <211> 27
   <212> DNA
   <213> Artificial Sequence
<220>
   <221> source
   <223> /note="Description of Artificial Sequence: Synthetic oligonucleotide"
<400> 203
   gcaccatctc tccgtggtac cccgggt 27
<210> 204
   <211> 26
   <212> DNA
   <213> Artificial Sequence
<220>
   <221> source
   <223> /note="Description of Artificial Sequence: Synthetic oligonucleotide"
<400> 204
   ggtggaactg gtagccatga atgaga 26
<210> 205
   <211> 27
   <212> DNA
   <213> Artificial Sequence
<220>
   <221> source
   <223> /note="Description of Artificial Sequence: Synthetic oligonucleotide"
<400> 205
   gccatgaatg agaccgaccc aaagagc 27
<210> 206
   <211> 27
   <212> DNA
   <213> Artificial Sequence
<220>
   <221> source
   <223> /note="Description of Artificial Sequence: Synthetic oligonucleotide"
<400> 206
   gcatcctcgt gggcacttcc gacgagg 27
<210> 207
   <211> 27
   <212> DNA
   <213> Artificial Sequence
<220>
   <221> source
   <223> /note="Description of Artificial Sequence: Synthetic oligonucleotide"
<400> 207
   gcagagcgga gtgctgttct cccaagt 27
<210> 208
   <211> 26
   <212> DNA
   <213> Artificial Sequence
<220>
   <221> source
   <223> /note="Description of Artificial Sequence: Synthetic oligonucleotide"
<400> 208
   ggtcggtctc attcatggct accagt 26
<210> 209
   <211> 26
   <212> DNA
   <213> Artificial Sequence
<220>
   <221> source
   <223> /note="Description of Artificial Sequence: Synthetic oligonucleotide"
<400> 209
   gcaataaaag gtgctattgc tatagt 26
<210> 210
   <211> 26
   <212> DNA
   <213> Artificial Sequence
<220>
   <221> source
   <223> /note="Description of Artificial Sequence: Synthetic oligonucleotide"
<400> 210
   gtactcaacc aagtcattct gagaat 26
<210> 211
   <211> 26
   <212> DNA
   <213> Artificial Sequence
<220>
   <221> source
   <223> /note="Description of Artificial Sequence: Synthetic oligonucleotide"
<220>
   <221> modified_base
   <222> (1)..(7)
   <223> a, c, t, g, unknown or other
<400> 211
   nnnnnnngta ctcaaccaag tcattc 26
<210> 212
   <211> 1121
   <212> PRT
   <213> Streptococcus thermophilus
<400> 212
   Met Ser Asp Leu Val Leu Gly Leu Asp Ile Gly Ile Gly Ser Val Gly
   1               5                   10                  15
   Val Gly Ile Leu Asn Lys Val Thr Gly Glu Ile Ile His Lys Asn Ser
               20                  25                  30
   Arg Ile Phe Pro Ala Ala Gln Ala Glu Asn Asn Leu Val Arg Arg Thr
           35                  40                  45
   Asn Arg Gln Gly Arg Arg Leu Ala Arg Arg Lys Lys His Arg Arg Val
       50                  55                  60
   Arg Leu Asn Arg Leu Phe Glu Glu Ser Gly Leu Ile Thr Asp Phe Thr
   65                  70                  75                  80
   Lys Ile Ser Ile Asn Leu Asn Pro Tyr Gln Leu Arg Val Lys Gly Leu
                   85                  90                  95
   Thr Asp Glu Leu Ser Asn Glu Glu Leu Phe Ile Ala Leu Lys Asn Met
               100                 105                 110
   Val Lys His Arg Gly Ile Ser Tyr Leu Asp Asp Ala Ser Asp Asp Gly
           115                 120                 125
   Asn Ser Ser Val Gly Asp Tyr Ala Gln Ile Val Lys Glu Asn Ser Lys
       130                 135                 140
   Gln Leu Glu Thr Lys Thr Pro Gly Gln Ile Gln Leu Glu Arg Tyr Gln
   145                 150                 155                 160
   Thr Tyr Gly Gln Leu Arg Gly Asp Phe Thr Val Glu Lys Asp Gly Lys
                   165                 170                 175
   Lys His Arg Leu Ile Asn Val Phe Pro Thr Ser Ala Tyr Arg Ser Glu
               180                 185                 190
   Ala Leu Arg Ile Leu Gln Thr Gln Gln Glu Phe Asn Pro Gln Ile Thr
           195                 200                 205
   Asp Glu Phe Ile Asn Arg Tyr Leu Glu Ile Leu Thr Gly Lys Arg Lys
       210                 215                 220
   Tyr Tyr His Gly Pro Gly Asn Glu Lys Ser Arg Thr Asp Tyr Gly Arg
   225                 230                 235                 240
   Tyr Arg Thr Ser Gly Glu Thr Leu Asp Asn Ile Phe Gly Ile Leu Ile
                   245                 250                 255
   Gly Lys Cys Thr Phe Tyr Pro Asp Glu Phe Arg Ala Ala Lys Ala Ser
               260                 265                 270
   Tyr Thr Ala Gln Glu Phe Asn Leu Leu Asn Asp Leu Asn Asn Leu Thr
           275                 280                 285
   Val Pro Thr Glu Thr Lys Lys Leu Ser Lys Glu Gln Lys Asn Gln Ile
       290                 295                 300
   Ile Asn Tyr Val Lys Asn Glu Lys Ala Met Gly Pro Ala Lys Leu Phe
   305                 310                 315                 320
   Lys Tyr Ile Ala Lys Leu Leu Ser Cys Asp Val Ala Asp Ile Lys Gly
                   325                 330                 335
   Tyr Arg Ile Asp Lys Ser Gly Lys Ala Glu Ile His Thr Phe Glu Ala
               340                 345                 350
   Tyr Arg Lys Met Lys Thr Leu Glu Thr Leu Asp Ile Glu Gln Met Asp
           355                 360                 365
   Arg Glu Thr Leu Asp Lys Leu Ala Tyr Val Leu Thr Leu Asn Thr Glu
       370                 375                 380
   Arg Glu Gly Ile Gln Glu Ala Leu Glu His Glu Phe Ala Asp Gly Ser
   385                 390                 395                 400
   Phe Ser Gln Lys Gln Val Asp Glu Leu Val Gln Phe Arg Lys Ala Asn
                   405                 410                 415
   Ser Ser Ile Phe Gly Lys Gly Trp His Asn Phe Ser Val Lys Leu Met
               420                 425                 430
   Met Glu Leu Ile Pro Glu Leu Tyr Glu Thr Ser Glu Glu Gln Met Thr
           435                 440                 445
   Ile Leu Thr Arg Leu Gly Lys Gln Lys Thr Thr Ser Ser Ser Asn Lys
       450                 455                 460
   Thr Lys Tyr Ile Asp Glu Lys Leu Leu Thr Glu Glu Ile Tyr Asn Pro
   465                 470                 475                 480
   Val Val Ala Lys Ser Val Arg Gln Ala Ile Lys Ile Val Asn Ala Ala
                   485                 490                 495
   Ile Lys Glu Tyr Gly Asp Phe Asp Asn Ile Val Ile Glu Met Ala Arg
               500                 505                 510
   Glu Thr Asn Glu Asp Asp Glu Lys Lys Ala Ile Gln Lys Ile Gln Lys
           515                 520                 525
   Ala Asn Lys Asp Glu Lys Asp Ala Ala Met Leu Lys Ala Ala Asn Gln
       530                 535                 540
   Tyr Asn Gly Lys Ala Glu Leu Pro His Ser Val Phe His Gly His Lys
   545                 550                 555                 560
   Gln Leu Ala Thr Lys Ile Arg Leu Trp His Gln Gln Gly Glu Arg Cys
                   565                 570                 575
   Leu Tyr Thr Gly Lys Thr Ile Ser Ile His Asp Leu Ile Asn Asn Ser
               580                 585                 590
   Asn Gln Phe Glu Val Asp His Ile Leu Pro Leu Ser Ile Thr Phe Asp
           595                 600                 605
   Asp Ser Leu Ala Asn Lys Val Leu Val Tyr Ala Thr Ala Asn Gln Glu
       610                 615                 620
   Lys Gly Gln Arg Thr Pro Tyr Gln Ala Leu Asp Ser Met Asp Asp Ala
   625                 630                 635                 640
   Trp Ser Phe Arg Glu Leu Lys Ala Phe Val Arg Glu Ser Lys Thr Leu
                   645                 650                 655
   Ser Asn Lys Lys Lys Glu Tyr Leu Leu Thr Glu Glu Asp Ile Ser Lys
               660                 665                 670
   Phe Asp Val Arg Lys Lys Phe Ile Glu Arg Asn Leu Val Asp Thr Arg
           675                 680                 685
   Tyr Ala Ser Arg Val Val Leu Asn Ala Leu Gln Glu His Phe Arg Ala
       690                 695                 700
   His Lys Ile Asp Thr Lys Val Ser Val Val Arg Gly Gln Phe Thr Ser
   705                 710                 715                 720
   Gln Leu Arg Arg His Trp Gly Ile Glu Lys Thr Arg Asp Thr Tyr His
               725                 730                 735
   His His Ala Val Asp Ala Leu Ile Ile Ala Ala Ser Ser Gln Leu Asn
           740                 745                 750
   Leu Trp Lys Lys Gln Lys Asn Thr Leu Val Ser Tyr Ser Glu Asp Gln
       755                 760                 765
   Leu Leu Asp Ile Glu Thr Gly Glu Leu Ile Ser Asp Asp Glu Tyr Lys
   770                 775                 780
   Glu Ser Val Phe Lys Ala Pro Tyr Gln His Phe Val Asp Thr Leu Lys
   785                 790                 795                 800
   Ser Lys Glu Phe Glu Asp Ser Ile Leu Phe Ser Tyr Gln Val Asp Ser
                   805                 810                 815
   Lys Phe Asn Arg Lys Ile Ser Asp Ala Thr Ile Tyr Ala Thr Arg Gln
               820                 825                 830
   Ala Lys Val Gly Lys Asp Lys Ala Asp Glu Thr Tyr Val Leu Gly Lys
           835                 840                 845
   Ile Lys Asp Ile Tyr Thr Gln Asp Gly Tyr Asp Ala Phe Met Lys Ile
       850                 855                 860
   Tyr Lys Lys Asp Lys Ser Lys Phe Leu Met Tyr Arg His Asp Pro Gln
   865                 870                 875                 880
   Thr Phe Glu Lys Val Ile Glu Pro Ile Leu Glu Asn Tyr Pro Asn Lys
                   885                 890                 895
   Gln Ile Asn Glu Lys Gly Lys Glu Val Pro Cys Asn Pro Phe Leu Lys
               900                 905                 910
   Tyr Lys Glu Glu His Gly Tyr Ile Arg Lys Tyr Ser Lys Lys Gly Asn
           915                 920                 925
   Gly Pro Glu Ile Lys Ser Leu Lys Tyr Tyr Asp Ser Lys Leu Gly Asn
       930                 935                 940
   His Ile Asp Ile Thr Pro Lys Asp Ser Asn Asn Lys Val Val Leu Gln
   945                 950                 955                 960
   Ser Val Ser Pro Trp Arg Ala Asp Val Tyr Phe Asn Lys Thr Thr Gly
                   965                 970                 975
   Lys Tyr Glu Ile Leu Gly Leu Lys Tyr Ala Asp Leu Gln Phe Glu Lys
   980 985 990
   Gly Thr Gly Thr Tyr Lys Ile Ser Gln Glu Lys Tyr Asn Asp Ile Lys
   995 1000 1005
   Lys Lys Glu Gly Val Asp Ser Asp Ser Glu Phe Lys Phe Thr Leu
   1010 1015 1020
   Tyr Lys Asn Asp Leu Leu Leu Val Lys Asp Thr Glu Thr Lys Glu
   1025 1030 1035
   Gln Gln Leu Phe Arg Phe Leu Ser Arg Thr Met Pro Lys Gln Lys
   1040 1045 1050
   His Tyr Val Glu Leu Lys Pro Tyr Asp Lys Gln Lys Phe Glu Gly
   1055 1060 1065
   Gly Glu Ala Leu Ile Lys Val Leu Gly Asn Val Ala Asn Ser Gly
   1070 1075 1080
   Gln Cys Lys Lys Gly Leu Gly Lys Ser Asn Ile Ser Ile Tyr Lys
   1085 1090 1095
   Val Arg Thr Asp Val Leu Gly Asn Gln His Ile Ile Lys Asn Glu
   1100 1105 1110
   Gly Asp Lys Pro Lys Leu Asp Phe
   1115 1120
<210> 213
   <211> 20
   <212> DNA
   <213> Corynebacter diphtheriae
<400> 213
   aaaagggaat aagggcgaca 20
<210> 214
   <211> 27
   <212> DNA
   <213> Sutterella wadsworthensis
<220>
   <221> modified_base
   <222> (1)..(1)
   <223> a, c, t, g, unknown or other
<220>
   <221> modified_base
   <222> (4)..(7)
   <223> a, c, t, g, unknown or other
<400> 214
   nggnnnncat tctgagaata gtgtatg 27
<210> 215
   <211> 20
   <212> DNA
   <213> Sutterella wadsworthensis
<400> 215
   gcgaccgagt tgctcttgcc 20
<210> 216
   <211> 27
   <212> DNA
   <213> Legionella pneumophila
<220>
   <221> modified_base
   <222> (1)..(1)
   <223> a, c, t, g, unknown or other
<220>
   <221> modified_base
   <222> (4)..(7)
   <223> a, c, t, g, unknown or other
<400> 216
   nggnnnncat tctgagaata gtgtatg 27
<210> 217
   <211> 20
   <212> DNA
   <213> Legionella pneumophila
<400> 217
   gcgaccgagt tgctcttgcc 20
<210> 218
   <211> 27
   <212> DNA
   <213> Filifactor alocis
<220>
   <221> modified_base
   <222> (1)..(3)
   <223> a, c, t, g, unknown or other
<400> 218
   nnnaagcttg agtactcacc agtcaca 27
<210> 219
   <211> 20
   <212> DNA
   <213> Filifactor alocis
<400> 219
   ctcttccttt ttcaatatta 20
<210> 220
   <211> 27
   <212> DNA
   <213> Lactobacillus johnsonii
<220>
   <221> modified_base
   <222> (1)..(2)
   <223> a, c, t, g, unknown or other
<220>
   <221> modified_base
   <222> (6)..(7)
   <223> a, c, t, g, unknown or other
<400> 220
   nnaaanntaa atgcttcaat aatattg 27
<210> 221
   <211> 20
   <212> DNA
   <213> Lactobacillus johnsonii
<400> 221
   aaagggaata agggcgacac 20
<210> 222
   <211> 27
   <212> DNA
   <213> Parvibaculum lavamentivorans
<220>
   <221> modified_base
   <222> (1)..(3)
   <223> a, c, t, g, unknown or other
<220>
   <221> modified_base
   <222> (7)..(7)
   <223> a, c, t, g, unknown or other
<400> 222
   nnncatnaaa ggaagagtat gagtatt 27
<210> 223
   <211> 20
   <212> DNA
   <213> Staphylococcus aureus
<400> 223
   ctattctcag aatgacttgg 20
<210> 224
   <211> 27
   <212> DNA
   <213> Mycoplasma gallisepticum
<220>
   <221> modified_base
   <222> (1)..(2)
   <223> a, c, t, g, unknown or other
<220>
   <221> modified_base
   <222> (5)..(5)
   <223> a, c, t, g, unknown or other
<400> 224
   nnganggagt cacagaaaag catctta 27
<210> 225
   <211> 20
   <212> DNA
   <213> Mycoplasma gallisepticum
<400> 225
   gtttctgggt gagcaaaaac 20
<210> 226
   <211> 27
   <212> DNA
   <213> Campylobacter lari
<220>
   <221> modified_base
   <222> (1)..(2)
   <223> a, c, t, g, unknown or other
<220>
   <221> modified_base
   <222> (6)..(6)
   <223> a, c, t, g, unknown or other
<400> 226
   nngggnagaa ggcaaaatgc cgcaaaa 27
<210> 227
   <211> 20
   <212> DNA
   <213> Campylobacter lari
<400> 227
   ggtattatcc cgtattgacg 20
<210> 228
   <211> 20
   <212> DNA
   <213> Unknown
<220>
   <221> source
   <223> /note="Description of Unknown: SpCas9 oligonucleotide"
<400> 228
   gggactcaac caagtcattc 20